(19) Europäisches Patentamt
European Patent Office
Office européen des brevets

(11) EP 4 674 841 A2

(12) EUROPEAN PATENT APPLICATION

(43) Date of publication:
**07.01.2026 Bulletin 2026/02**

(21) Application number: **25217560.9**

(22) Date of filing: **14.02.2023**

(51) International Patent Classification (IPC):
***C07D 241/40*** *(2006.01)*

(52) Cooperative Patent Classification (CPC):
**A61K 31/517; A61K 31/4545; A61K 31/506; A61K 31/519; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.02.2022 GB 202202070**
**06.09.2022 GB 202212967**

(62) Document number(s) of the earlier application(s) in accordance with Art. 76 EPC:
**23705256.8 / 4 444 305**

(71) Applicant: **Duke Street Bio Limited**
**London W1U 3EH (GB)**

(72) Inventors:
- **COWLEY, Phillip Martin**
**London, W1U 3EH (GB)**
- **WISE, Alan**
**London, W1U 3EH (GB)**
- **HILL, Christopher**
**London, W1U 3EH (GB)**

(74) Representative: **Page White Farrer**
**Bedford House**
**21a John Street**
**London WC1N 2BF (GB)**

Remarks:
•The complete document including Reference Table(s) and the Sequence Listing(s) can be downloaded from the EPO website
•This application was filed on 21-11-2025 as a divisional application to the application mentioned under INID code 62.

# (54) PHARMACEUTICAL COMPOUND

(57) A PARP1 inhibitor compound for use in medicine has a formula selected from:

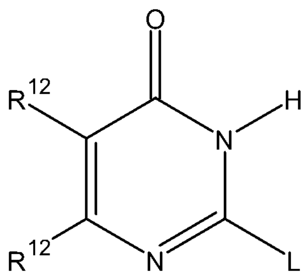
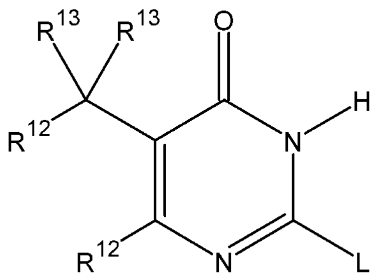
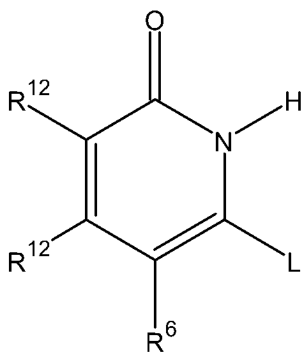

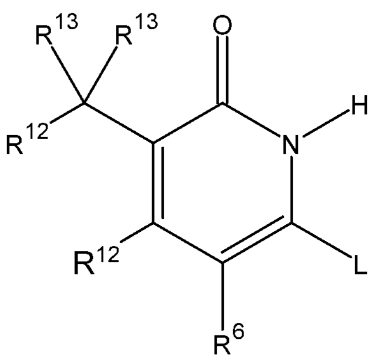
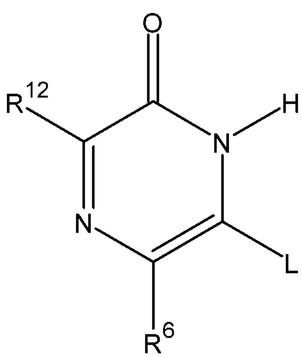
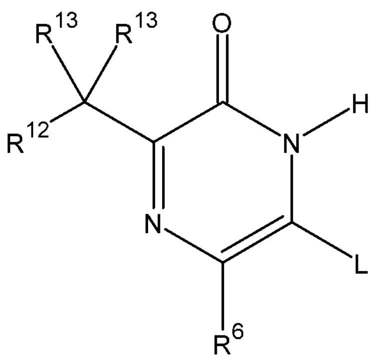

EP 4 674 841 A2

wherein $R^6$, $R^{12}$, $R^{13}$, and L are organic groups as defined herein. The compound is useful for treating, for example, a cancer. Also provided are compositions and kits including such compounds.

## Description

The present invention relates to PARP1 inhibitor compounds, and in particular to PARP1 inhibitor compounds for use in medicine. The inhibitors of the invention may be used in pharmaceutical compositions, and in particular pharmaceutical compositions for treating a cancer. The invention also relates to methods of manufacture of such inhibitors, and methods of treatment using such inhibitors.

## Background to the Invention

The family of poly(ADP-ribose) polymerases (PARPs) consists of 17 PARP proteins that catalyse the transfer of ADP-ribose to target proteins, a posttranslational process termed PARylation. Target protein modification by PARylation causes significant changes to function and as such PARPs play an important role in many cellular processes such as chromatin remodelling, transcription, replication, recombination, cell cycle progression and DNA damage repair (Kamaletdinova, T. et al. Cell. 2019; 8: 1625). PARP1 and 2 are the most widely studied PARP enzymes, primarily due to their role in DNA damage repair, in particular in the base excision repair (BER) process of DNA single-strand breaks (Ngoi, YL. et al. Cancer J. 2021; 27: 521-528). PARP1 is activated by DNA damage breaks, and the subsequent PARylation of target proteins leads to recruitment of additional factors that initiate repair of DNA lesions. Auto-PARylation of PARP triggers the release of bound PARP from the DNA allowing other DNA repair proteins access to complete lesion repair. This highlights the critical role PARP plays in enabling a cancer cell to repair DNA damage caused by exogenous agents such as radiation therapy and chemotherapeutic agents. Hence, inhibition of PARP enzymes has been utilised as a strategy to selectively kill cancer cells that harbour genetic defects in complementary DNA damage repair pathways (Farmer, H. et al. Nature. 2005; 434: 917-921). This synthetic lethality approach has been demonstrated successfully in tumours with epigenetic modifications or deleterious mutations in BRCA1 and BRCA2, two functionally redundant tumour suppressor proteins involved in DNA double-strand break (DSB) repair by homologous recombination (HR) (Lord, CJ. and Ashworth, A. Science. 2017; 355: 1152-1158). Such tumours with HR deficiency (HRD) are dependent on PARP function for survival - following PARP inhibition in these tumours, DSB breaks will be processed by alternative error-prone repair pathways leading to genomic instability and cancer cell death.

The inhibition of PARP can trap the inactivated PARP at the sites of DNA damage. This leads to replication fork stalling and subsequent collapse in S-phase when the fork reaches the site of the trapped PARP, resulting in the generation of genotoxic DNA double-strand breaks. It is believed that this PARP1-DNA trapping can lead to the selective death of cancer cells harbouring HRD (Farmer, H. et al. Nature. 2005; 434: 917-921.

This strategy has led to the successful approval of several PARP inhibitors for the treatment of cancers with HRD, such as in BRCA1/2-mutated breast, ovarian and prostate cancer, as well as in ovarian and prostate cancer harbouring genomic consequences of HRD, and ovarian cancer in the maintenance setting where platinum sensitivity acts as a surrogate for HRD (Fong, PC. et al. N. Engl. J. Med. 2009; 361: 123-134).

It has recently been shown that genomic instability, in the form of unrepaired DNA double-strand breaks or micronuclei disruption can trigger innate immune system activation via the cytosolic DNA sensor cyclic GMP-AMP synthase (cGAS), leading to generation of cyclic guanosine monophosphate-adenosine monophosphate (cGAMP) and induction of dimerization of Stimulator of interferon genes (STING). STING subsequently translocates from the endoplasmic reticulum to the Golgi where it recruits and activates TANK-binding kinase 1 (TBK1). TBK1 phosphorylates interferon regulatory transcription factor 3 (IRF3) which drives the production of type 1 interferons and supports the induction of an adaptive immune response (Zhu, Y. et al. Mol. Cancer. 2019, 18: 152).

For example, PARP inhibitor-induced STING pathway activation and anti-tumour immune responses have been demonstrated in multiple tumour models, providing rationale for exploiting combinations of PARP inhibitors with immunotherapies for improved therapeutic efficacy (Sen, T. et al. Cancer Discov. 2019; 9: 646-661). For example, the PARP inhibitor Olaparib was also recently shown to induce synthetic lethal effects in combination with a synthetic cyclic dinucleotide STING agonist in DNA damage repair deficient cancer cells and a BRCA-deficient breast cancer model (Pantelidou, C. et al. 2021: bioRxiv).

Overall, modulation of nucleic acid sensing pathways via multiple mechanisms has been shown to promote anti-tumour efficacy in a variety of cell and animal models thus demonstrating therapeutic potential for augmenting efficacy of immunotherapies and overcoming resistance to immune checkpoint blockade through use of PARP inhibitors. There are numerous clinical trials ongoing combining PARP inhibitors with immunotherapies (reviewed in Chabanon, RM, et al. Nat. Rev. Cancer. 2021; 21: 701-717).

Recently, PARP1 has also been shown to bind the Epstein Barr Virus (EBV) genome and that PARP1 inhibition can alter EBV chromatin structure and latent gene expression (Morgan, SM. et al. Nat. Commun. 2022; 13: 187). Hence, PARP1 inhibitors may play a role in cancers where EBV plays a contributing role such as Burkitt's lymphoma, Hodgkin's lymphoma, nasopharyngeal and gastrointestinal cancers. Interestingly, EBV has also been shown to be a causative factor in multiple sclerosis (MS) whereby EBV infection greatly increases the risk of subsequent MS (Bjomevik, K. et al. Science

(2021); 375: 296-301).

First-generation PARP inhibitors generally demonstrate non-selective activity at PARP1 and 2. Haematological toxicities such as anaemia, neutropenia and thrombocytopenia are associated with clinical use of these molecules which restricts their use in combination with cytotoxic chemotherapies and other targeted agents due to dose-limiting cytopenias (LaFargue, CJ. et al. Lancet Oncol. 2019, 20, c15-c28). Evidence from pre-clinical mouse studies strongly suggests that PARP2 inhibition is a major driver of these haematological toxicities, with PARP2 being particularly linked to erythrogenesis in mice (Farrés, J. et al. Blood. 2013; 122: 44-54). In addition, PARP2 function has been shown to be dispensable for anti-tumour activity in HRD mouse cancer models (Ronson, G E. et al. Nat. Commun. 2018, 9: 746). Taken together, these data suggest an unmet medical need for the development of inhibitors with improved selectivity for PARP1 over PARP2 and other PARPs, thus providing expanded therapeutic utility (1) as single agents and (2) in combination with other anti-cancer agents.

To date, two PARP1-selective inhibitors, AZD5305 and AZD9574, have entered clinical development. AZD5305 was described as a potent PARP1 inhibitor and trapper with 500-fold selectivity over PARP2 and less off-target activity against secondary pharmacology targets than first-generation PARP inhibitors (Johannes, JW. et al. J. Med. Chem. 2021; 64: 14498-14512). Importantly, significantly less haematotoxicity was observed for AZD5305 in rodent models than with first-generation PARP inhibitors, confirming the reported pathogenic role of PARP2 in haematologic toxicity (Illuzzi, G. et al. Clin. Cancer Res. 2022; CCR-22-0301).

Having regard to the above, it is an aim of the present invention to provide PARP1 inhibitors, and in particular PARP1 inhibitors for use in medicine. It is a further aim to provide pharmaceutical compositions comprising such inhibitors, and in particular to provide compounds and pharmaceutical compositions for treating a cancer. It is also an aim to provide methods of synthesis of the compounds.

## Summary of the Invention

Accordingly, the present invention provides a PARP1 inhibitor compound for use in medicine, which compound comprises the following structure:

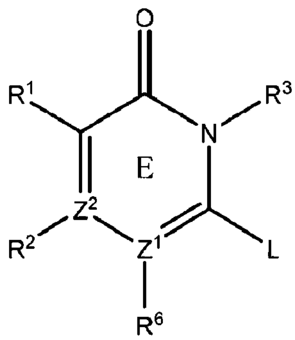

wherein $R^1$ is selected from H and a substituted or unsubstituted organic group; $R^2$ may be present or absent and is independently selected from H and a substituted or unsubstituted organic group; $R^1$ and $R^2$ may together form a ring; $R^3$ is independently selected from H and a substituted or unsubstituted organic group; $R^6$ may be present or absent and is independently selected from H and a substituted or unsubstituted organic group; $Z^1$ and $Z^2$ are independently selected from C and N; and L comprises a group having the following structure:

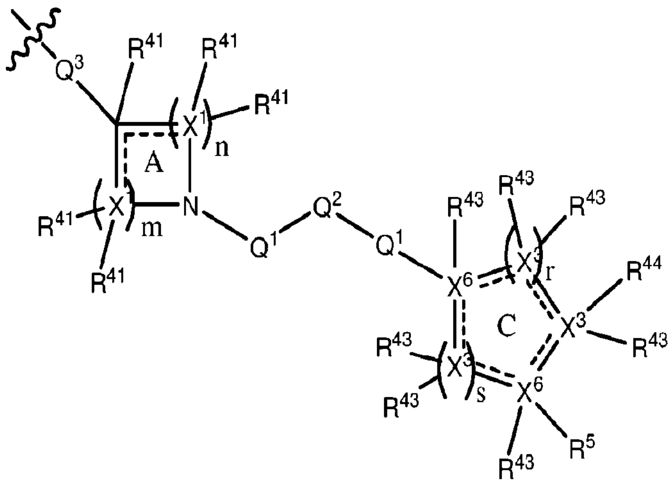

wherein, n is a number independently selected from 0, 1, 2, 3, 4, 5 and 6; m is a number independently selected from 0, 1, 2, 3, 4, 5 and 6; and m + n is a number selected from 2, 3, 4, 5, and 6; preferably wherein both n and m are at least 1;

wherein, r is a number independently selected from 0, 1, 2, 3, 4, and 5; s is a number independently selected from 0, 1, 2, 3, 4, and 5, preferably from 1, 2, 3, 4 and 5; and r + s is a number selected from 1, 2, 3, 4, and 5; preferably wherein both r and s are at least 1;

and wherein, each $X^1$ may be the same or different and is independently selected from C, N, O and S; each $X^3$ may be the same or different and is independently selected from C, N, O and S, preferably wherein at least one $X^3$ is independently N; each $X^6$ may be the same or different and is independently selected from C and N; each $R^{41}$ may be the same or different, and may be present or absent, and is selected from H and a substituted or unsubstituted organic group; each $R^{43}$ may be the same or different, and may be present or absent, and is selected from H and a substituted or unsubstituted organic group; and $R^{44}$ may be present or absent, and is selected from H and a substituted or unsubstituted organic group;

and wherein, the dotted lines indicate that: the ring A may comprise single bonds, or a combination of both single and double bonds, and may be aliphatic or aromatic; and independently the ring C may comprise single bonds, or a combination of both single and double bonds, and may be aliphatic or aromatic;

and wherein, $R^5$ is selected from H and a substituted or unsubstituted organic group and is preferably a substituted or unsubstituted organic group;

and wherein each $Q^1$ may be the same or different, and may be present or absent, and comprises a group independently selected from the following structures:

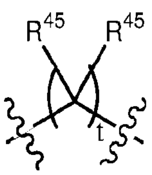

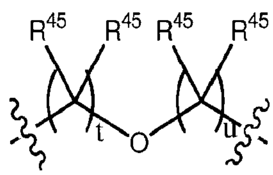

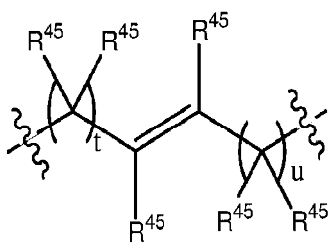

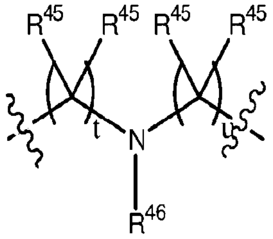

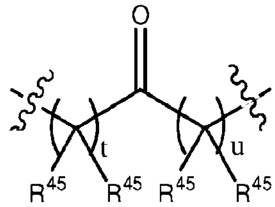

wherein t is independently a number selected from 0, 1, 2, 3, 4 and 5; u is independently a number selected from 0, 1, 2, 3, 4 and 5; and t + u is a number selected from 0, 1, 2, 3, 4, 5 and 6 (preferably a number selected from 0, 1, 2, and 3); each $R^{45}$ may be the same or different and is independently selected from H and a substituted or unsubstituted organic group; and $R^{46}$ is selected from H and a substituted or unsubstituted organic group;

and wherein $Q^2$ may be present or absent and is a group having the following structure:

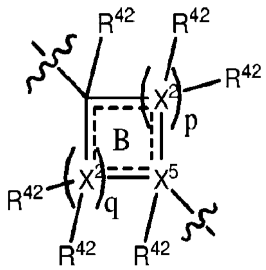

wherein, p is a number independently selected from 0, 1, 2, 3, 4, 5 and 6; q is a number independently selected from 0, 1, 2, 3, 4, 5 and 6; and p + q is a number selected from 2, 3, 4, 5, and 6;

and wherein, each $X^2$ may be the same or different and is independently selected from C, N, O and S; $X^5$ is independently selected from C and N; each $R^{42}$ may be the same or different, and may be present or absent, and is selected from H and a substituted or unsubstituted organic group;

and wherein, the dotted lines indicate that: the ring B may comprise single bonds, or a combination of both single and double bonds, and may be aliphatic or aromatic;

and wherein $Q^3$ may be present or absent and comprises a group independently selected from the following structures:

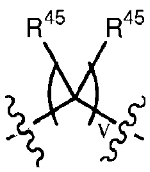

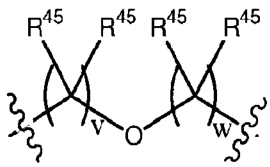

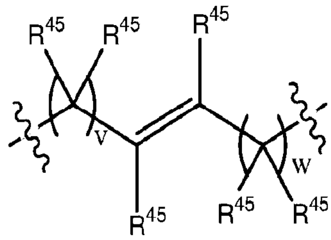

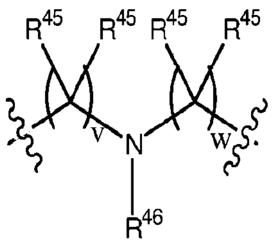

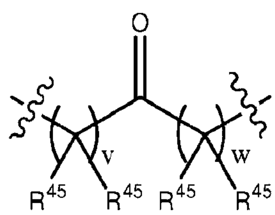

wherein v is independently a number selected from 0, 1, 2, 3, 4 and 5; w is independently a number selected from 0, 1,

2, 3, 4 and 5; and v + w is a number selected from 0, 1, 2, 3, 4, 5 and 6 (preferably a number selected from 0, 1, and 2).

In the context of the present invention, the $Z^1$, $Z^2$, $X^1$, $X^2$, $X^3$, $X^4$, $X^5$ and $X^6$ atoms maintain their normal valency and the number of substituents attached to these will depend on their nature and the number of other bonds they possess. Maintaining the valency means ensuring that an atom has its normal (typically most common) valency in organic compounds (i.e. 2 for oxygen and divalent sulphur, 3 for nitrogen and 4 for carbon). Nitrogen atoms may, in some instances, have 4 bonds, but in such cases they are typically positively charged such that the compound may have a counter-ion. Such compounds are also considered to be part of the invention, and in these cases, due to the positive charge, it will be clear that the nitrogen atom still maintains its normal valency of 3. For the avoidance of doubt, where the number of R groups may vary according to the choice of X group, it may vary as follows.

$R^6$ is absent when $Z^1$ is N. $R^2$ is absent when $Z^2$ is N. Each $R^{41}$ may be the same or different, provided that for each $X^1$: $R^{41}$ is absent when $X^1$ is O or divalent S; $R^{41}$ is absent when $X^1$ is N and is double bonded to an adjacent atom; one $R^{41}$ is present when $X^1$ is N and is not double bonded to an adjacent atom; one $R^{41}$ is present when $X^1$ is C and is double bonded to an adjacent atom; and two $R^{41}$ are present when $X^1$ is C and is not double bonded to an adjacent atom. Each $R^{43}$ may be the same or different, provided that for each $X^3$: $R^{43}$ is absent when $X^3$ is O or divalent S; $R^{43}$ is absent when $X^3$ is N and is double bonded to an adjacent atom; one $R^{43}$ is present when $X^3$ is N and is not double bonded to an adjacent atom; one $R^{43}$ is present when $X^3$ is C and is double bonded to an adjacent atom; and two $R^{43}$ are present when $X^3$ is C and is not double bonded to an adjacent atom. For each $X^6$: $R^{43}$ is absent when $X^6$ is N or is C and is double bonded to an adjacent atom; and one $R^{43}$ is present when $X^6$ is C and not double bonded to an adjacent atom. For the $X^3$ with which it is associated: $R^{44}$ is absent when $X^3$ is O or divalent S; $R^{44}$ is absent when $X^3$ is N and is double bonded to an adjacent atom; one $R^{44}$ is present when $X^3$ is N and is not double bonded to an adjacent atom; one $R^{44}$ is present when $X^3$ is C and is double bonded to an adjacent atom; and one $R^{44}$ is present when $X^3$ is C and the C is attached to $R^{43}$ and not double bonded to an adjacent atom. Each $R^{42}$ may be the same or different, provided that for each $X^2$: $R^{42}$ is absent when $X^2$ is O or divalent S; $R^{42}$ is absent when $X^2$ is N and is double bonded to an adjacent atom; one $R^{42}$ is present when $X^2$ is N and is not double bonded to an adjacent atom; one $R^{42}$ is present when $X^2$ is C and is double bonded to an adjacent atom; and two $R^{42}$ are present when $X^2$ is C and is not double bonded to an adjacent atom. For $X^5$: $R^{42}$ is absent when $X^5$ is N or is C and is double bonded to an adjacent atom; and one $R^{42}$ is present when $X^5$ is C and not double bonded to an adjacent atom. Each $R^{11}$ may be the same or different, provided that for each $X^4$: $R^{11}$ is absent when $X^4$ is O or divalent S; $R^{11}$ is absent when $X^4$ is N and is double bonded to an adjacent atom; one $R^{11}$ is present when $X^4$ is N and is not double bonded to an adjacent atom; one $R^{11}$ is present when $X^4$ is C and is double bonded to an adjacent atom; and two $R^{11}$ are present when $X^4$ is C and is not double bonded to an adjacent atom. Each $R^{14}$ bonded to N may be the same or different, provided that $R^{14}$ is absent when the N is double bonded to an adjacent atom.

In these compounds, and elsewhere herein, in some embodiments any R group may form a ring with any other R group on an adjacent and/or proximal atom, although in most embodiments this is not preferred, except where explicitly stated. Thus, in some embodiments the following substituents may together form a ring: $R^5$ with $R^{44}$; $R^{11}$ with another $R^{11}$; $R^{13}$ with another $R^{13}$ $R^{41}$ with another $R^{41}$; $R^{42}$ with another $R^{42}$; $R^{43}$ with another $R^{43}$; $R^{45}$ with another $R^{45}$. In the context of the present invention, an adjacent and/or proximal atom may mean another atom directly bonded to an atom (adjacent) or may be two atoms with only a single atom in between (proximal), or may mean two atoms close enough sterically to be capable of forming a ring (proximal). Preferably R groups attached to the same atom do not together form a ring, although this is not excluded.

In the present context the invention includes compounds in which a single R group on an atom, or two R groups on the same atom, form a group which is double bonded to that atom. Accordingly, an R group, or two R groups attached to the same atom, may together form a =O group, or a $=C(R')_2$ group (wherein each R' group is the same or different and is H or an organic group, preferably H or a straight or branched $C_1$-$C_6$ alkyl group). This is more typical in cases where the R groups are attached to a C atom, such that together they form a C=O group or a $C=C(R')_2$ group. Thus is some cases a C ring atom in a ring may comprise a =O group, as may an X, and/or one or more of $R^{11}$, $R^{41}$, $R^{42}$, $R^{43}$, $R^{44}$, and $R^{45}$.

In the present context a part of any structure present in brackets may be repeated the number of times given by the numbers next to the brackets (whether regular brackets or square brackets). For example, in the case of $(C(R))_{0,1,2}$ or $[C(R)]_{0,1,2}$ the C-R group may be absent, present once i.e. -C(R)-; or present twice i.e. -C(R)-C(R)-.

In the context of the present invention, a compound is considered to be a PARP1 inhibitor if its presence is capable of preventing or reducing the ability of immobilised PARP1 to undergo auto-poly-ADP ribosylation (AutoPARylation) following incubation with biotinylated-NAD+ as compared to the same process in its absence. Typically, the compound is considered to be a PARP1 inhibitor if it has an $IC_{50}$ < 10 $\mu$M in a suitable assay. A suitable assay may be conducted using 2 nM PARP1, 2 $\mu$M biotin-$NAD^+$ assay solution in 20 mM HEPES (pH 7.5), 100 mM NaCl, 2 mM DTT, 0.1 % BSA (w/v), 0.02 % Tween (v/v) assay buffer. PARylation may take place for 2 h at room temperature and may be detected using a dissociation-enhanced lanthanide fluorescence immunoassay (DELFIA) readout. A particularly suitable assay is described in the Examples below. Preferably, the compound has an $IC_{50}$ < 1 $\mu$M, more preferably < 100 nM and most preferably < 10 nM in the PARP1 inhibitor assay.

A compound is also considered to be a selective PARP1 inhibitor if its presence is capable of displacing or reducing the ability of a high affinity Cy5 fluorescent dye-labelled chemical probe to bind to PARP1 whilst displacing the same chemical probe at PARP2 with at least 10-fold weaker activity. Typically, the compound is considered to be a selective PARP1 inhibitor if it has an $IC_{50}$ < 10 $\mu$M in this assay at PARP1 with at least 10-fold selectivity preference over PARP2. A suitable such assay may be conducted for 1 h at room temperature using 10 nM PARP1 or PARP2, Tb-cryptate antibody and PARP1/2 binding probe in 20 mM HEPES (pH 7.5), 100 mM NaCl, 2 mM DTT, 0.1 % BSA (w/v), 0.02 % Tween (v/v) assay buffer. Probe binding displacement may be detected using homogeneous time-resolved fluorescence. A particularly suitable assay is described in the Examples below. Preferably the selectivity preference of PARP1 over PARP2 is at least 50-fold, more preferably at least 100-fold.

The compound is also considered to be a selective PARP1 inhibitor if it has an $IC_{50}$ < 10 $\mu$M at PARP1 with at least 10-fold selectivity preference over PARP2 in NanoBRET assays demonstrating cellular target engagement. These assays are based on bioluminescence resonance energy transfer (BRET) between a Nano-luc-tagged protein (eg PARP1 or PARP2) and a fluorescent group on a high affinity $NAD^+$ competitive binding probe. Such cellular probe displacement assays can be utilised to measure inhibitor affinities and selectivity ratios at PARP1 and 2. A particularly suitable assay is described in the Examples below. Preferably the selectivity preference of PARP1 over PARP2 is at least 50-fold, more preferably at least 100-fold.

In all of the embodiments of this invention (both above and below herein), the substituents (each of the R groups) are not especially limited, provided that they do not prevent the PARP1 inhibitory function from occurring. In all of the embodiments mentioned in connection with this invention, both above and in the following, the substituents are selected from H and an organic group. Thus, both above and in the following, the terms 'substituent' and 'organic group' are not especially limited and may be any functional group or any atom, especially any functional group or atom common in organic chemistry. Thus, 'substituent' and 'organic group' may have any of the following meanings.

The organic group may comprise any one or more atoms from any of groups IIIA, IVA, VA, VIA or VIIA of the Periodic Table, such as a B, Si, N, P, O, or S atom (e.g. OH, OR, $NH_2$, NHR, $NR_2$, SH, SR, $SO_2R$, $SO_3H$, $PO_4H_2$) or a halogen atom (e.g. F, Cl, Br or I) where R is a linear or branched lower hydrocarbon (1-6 C atoms) or a linear or branched higher hydrocarbon (7 C atoms or more, e.g. 7-40 C atoms).

The organic group preferably comprises a hydrocarbon group. The hydrocarbon group may comprise a straight chain, a branched chain or a cyclic group. Independently, the hydrocarbon group may comprise an aliphatic or an aromatic group. Also independently, the hydrocarbon group may comprise a saturated or unsaturated group.

When the hydrocarbon comprises an unsaturated group, it may comprise one or more alkene functionalities and/or one or more alkyne functionalities. When the hydrocarbon comprises a straight or branched chain group, it may comprise one or more primary, secondary and/or tertiary alkyl groups.

When the hydrocarbon comprises a cyclic group it may comprise an aromatic ring, a non-aromatic ring, an aliphatic ring, a heterocyclic group, and/or fused ring derivatives of these groups. The ring may be fully saturated, partially saturated, or fully unsaturated. The cyclic group may thus comprise a benzene, naphthalene, anthracene, phenanthrene, phenalene, biphenylene, pentalene, indene, as-indacene, s-indacene, acenaphthylene, fluorene, fluoranthene, acephenanthrylene, azulene, heptalene, pyrrole, pyrazole, imidazole, 1,2,3-triazole, 1,2,4-triazole, tetrazole, pyrrolidine, furan, oxetane, tetrahydrofuran, 2-aza-tetrahydrofuran, 3-aza-tetrahydrofuran, oxazole, isoxazole, furazan, 1,2,4-oxadiazol, 1,3,4-oxadiazole, thiophene, isothiazole, thiazole, thiolane, pyridine, pyridazine, pyrimidine, pyrazine, piperidine, 2-azapiperidine, 3-azapiperidine, piperazine, pyran, tetrahydropyran, 2-azapyran, 3-azapyran, 4-azapyran, 2-aza-tetrahydropyran, 3-aza-tetrahydropyran, morpholine, thiopyran, 2-azathiopyran, 3-azathiopyran, 4-azathiopyran, thiane, indole, indazole, benzimidazole, 4-azaindole, 5-azaindole, 6-azaindole, 7-azaindole, isoindole, 4-azaisoindole, 5-azaisoindole, 6-azaisoindole, 7-azaisoindole, indolizine, 1-azaindolizine, 2-azaindolizine, 3-azaindolizine, 5-azaindolizine, 6-azaindolizine, 7-azaindolizine, 8-azaindolizine, 9-azaindolizine, purine, carbazole, carboline, benzofuran, isobenzofuran, benzothiophene, isobenzothiophene, quinoline, cinnoline, quinazoline, quinoxaline, 5-azaquinoline, 6-azaquinoline, 7-azaquinoline, isoquinoline, phthalazine, 6-azaisoquinoline, 7-azaisoquinoline, pteridine, chromene, isochromene, acridine, phenanthridine, perimidine, phenanthroline, phenoxazine, xanthene, phenoxanthiin, and/or thianthrene, as well as regioisomers of the above groups. These groups may generally be attached at any point in the group, and also may be attached at a hetero-atom or at a carbon atom. In some instances particular attachment points are preferred, such as at 1-yl, 2-yl and the like, and these are specified explicitly where appropriate. All tautomeric ring forms are included in these definitions. For example pyrrole is intended to include 1H-pyrrole, 2H-pyrrole and 3H-pyrrole.

The number of carbon atoms in the hydrocarbon group is not especially limited, but preferably the hydrocarbon group comprises from 1-40 C atoms. The hydrocarbon group may thus be a lower hydrocarbon (1-6 C atoms) or a higher hydrocarbon (7 C atoms or more, e.g. 7-40 C atoms). The lower hydrocarbon group may be a methyl, ethyl, propyl, butyl, pentyl or hexyl group or regioisomers of these, such as isopropyl, isobutyl, tert-butyl, etc. The number of atoms in the ring of the cyclic group is not especially limited, but preferably the ring of the cyclic group comprises from 3-10 atoms, such as 3, 4, 5, 6, 7, 8, 9 or 10 atoms.

The groups comprising heteroatoms described above, as well as any of the other groups defined above, may

comprise one or more heteroatoms from any of groups IIIA, IVA, VA, VIA or VIIA of the Periodic Table, such as a B, Si, N, P, O, or S atom or a halogen atom (e.g. F, Cl, Br or I). Thus, the substituent may comprise one or more of any of the common functional groups in organic chemistry, such as hydroxy groups, carboxylic acid groups, ester groups, ether groups, aldehyde groups, ketone groups, amine groups, amide groups, imine groups, thiol groups, thioether groups, sulphate groups, sulphonic acid groups, sulphonyl groups, and phosphate groups etc. The substituent may also comprise derivatives of these groups, such as carboxylic acid anhydrides and carboxylic acid halides.

In addition, any substituent may comprise a combination of two or more of the substituents and/or functional groups defined above.

The rings A, B (when present) and C of the compounds of the present invention form a bicyclic or tricyclic ring structure (which may comprise further fused rings when the substituents on either ring themselves form a ring). Each of rings A, B, C and D are not necessarily limited, provided that they do not prevent the PARP1 inhibitory function from occurring. Rings A, B, C and D may independently be comprised of an aromatic ring, a non-aromatic ring, an aliphatic ring, and/or a heterocyclic group. The rings may be fully saturated, partially saturated, or fully unsaturated. Each ring may thus independently comprise a benzene, naphthalene, anthracene, phenanthrene, phenalene, biphenylene, pentalene, indene, as-indacene, s-indacene, acenaphthylene, fluorene, fluoranthene, acephenanthrylene, azulene, heptalene, pyrrole, pyrazole, imidazole, 1,2,3-triazole, 1,2,4-triazole, tetrazole, pyrrolidine, oxetane, furan, tetrahydrofuran, 2-aza-tetrahydrofuran, 3-aza-tetrahydrofuran, oxazole, isoxazole, furazan, 1,2,4-oxadiazol, 1,3,4-oxadiazole, thiophene, isothiazole, thiazole, thiolane, pyridine, pyridazine, pyrimidine, pyrazine, piperidine, 2-azapiperidine, 3-azapiperidine, piperazine, pyran, tetrahydropyran, 2-azapyran, 3-azapyran, 4-azapyran, 2-aza-tetrahydropyran, 3-aza-tetrahydropyran, morpholine, thiopyran, 2-azathiopyran, 3-azathiopyran, 4-azathiopyran, thiane, indole, indazole, benzimidazole, 4-azaindole, 5-azaindole, 6-azaindole, 7-azaindole, isoindole, 4-azaisoindole, 5-azaisoindole, 6-azaisoindole, 7-azaisoindole, indolizine, 1-azaindolizine, 2-azaindolizine, 3-azaindolizine, 5-azaindolizine, 6-azaindolizine, 7-azaindolizine, 8-azaindolizine, 9-azaindolizine, purine, carbazole, carboline, benzofuran, isobenzofuran, benzothiophene, isobenzothiophene, quinoline, cinnoline, quinazoline, quinoxaline, 5-azaquinoline, 6-azaquinoline, 7-azaquinoline, isoquinoline, phthalazine, 6-azaisoquinoline, 7-azaisoquinoline, pteridine, chromene, isochromene, acridine, phenanthridine, perimidine, phenanthroline, phenoxazine, xanthene, phenoxanthiin, and/or thianthrene, as well as regioisomers of the above groups. These rings may generally be substituted at any point in the group, and also may be substituted at a hetero-atom or at a carbon atom. All tautomeric ring forms are included in these definitions. For example, pyrrole is intended to include 1*H*-pyrrole, 2*H*-pyrrole and 3*H*-pyrrole.

In typical embodiments, the invention provides a compound as defined above, wherein $Q^3$ is absent or is a group independently selected from the following structures:

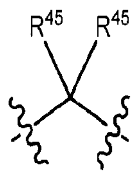
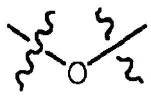
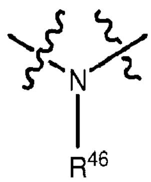
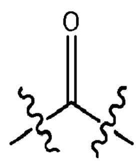

wherein each $R^{45}$ may be the same or different and is independently selected from H and a substituted or unsubstituted organic group; and $R^{46}$ is selected from H and a substituted or unsubstituted organic group. It is preferred that $Q^3$ is absent or is

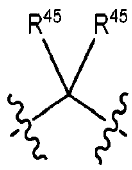 or 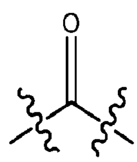,

such as a $-CH_2-$ group.

In typical embodiments, the invention provides a compound as defined above, wherein $Q^2$ is present and each $Q^1$ is independently absent or is a group independently selected from the following structures:

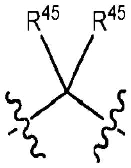 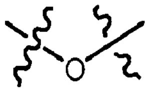 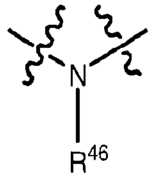 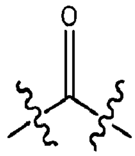

wherein each $R^{45}$ may be the same or different and is independently selected from H and a substituted or unsubstituted organic group; and $R^{46}$ is selected from H and a substituted or unsubstituted organic group. It is preferred that $Q^1$ is absent or is

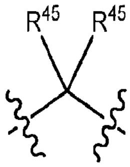 or 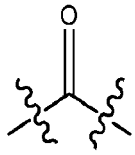,

such as a $-CH_2-$ group, particularly when $Q^2$ is present.

The $Q^1$ group bound to ring A is preferably

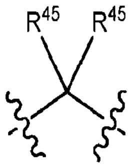 or 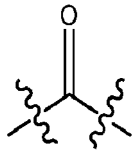,

such as a $-CH_2-$ group.

In some embodiments, the invention provides a compound as defined above, wherein the group L comprises a group having the following structure:

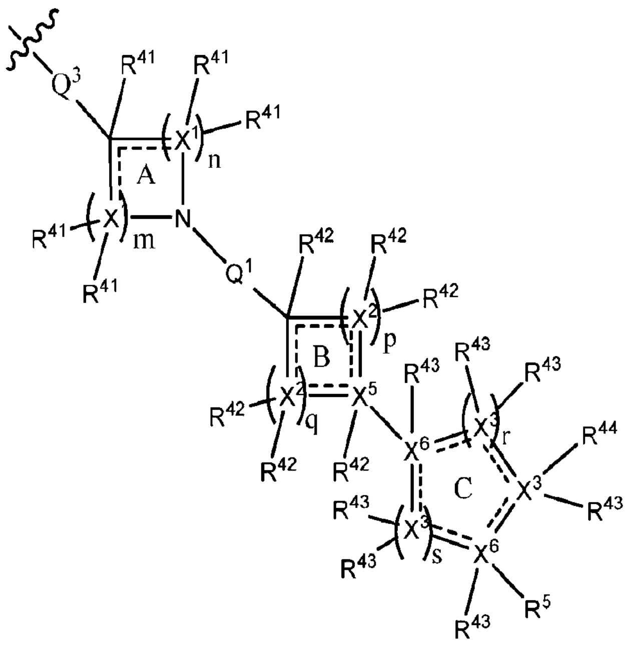

wherein $Q^1$ is absent or is a group as defined above, $Q^3$ is absent or is a group as defined above, and n, m, p, q, r, s, $X^1$, $X^2$, $X^3$, $X^5$, $X^6$, $R^{41}$, $R^{42}$, $R^{43}$, $R^{44}$, $R^5$, and rings A, B, and C are as defined above. It is preferred that $X^5$ is N with no $R^{42}$ substituent.

In some embodiments, the group L comprises a group having the following structure:

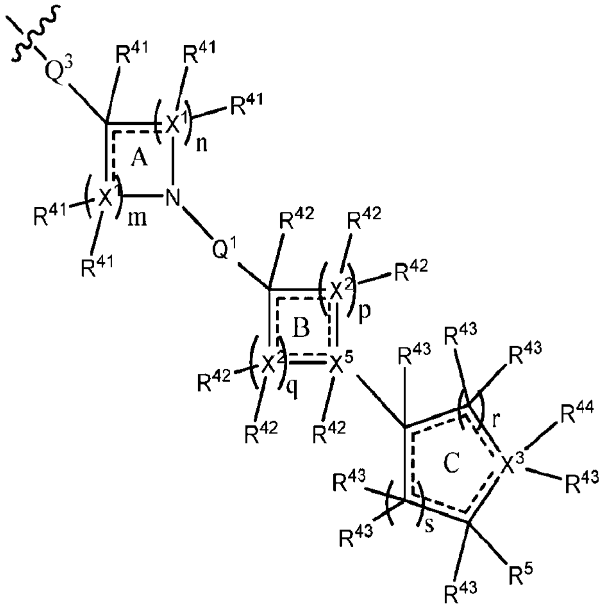

wherein n, m, p, q, r, s, $X^1$, $X^2$, X3, $X^5$, $R^{41}$, $R^{42}$, $R^{43}$, $R^{44}$, $R^5$, $Q^1$, $Q^3$ and rings A, B, and C are as defined above. It is preferred that $X^5$ is N with no $R^{42}$ substituent.

In some embodiments, the group L comprises a group having the following structure:

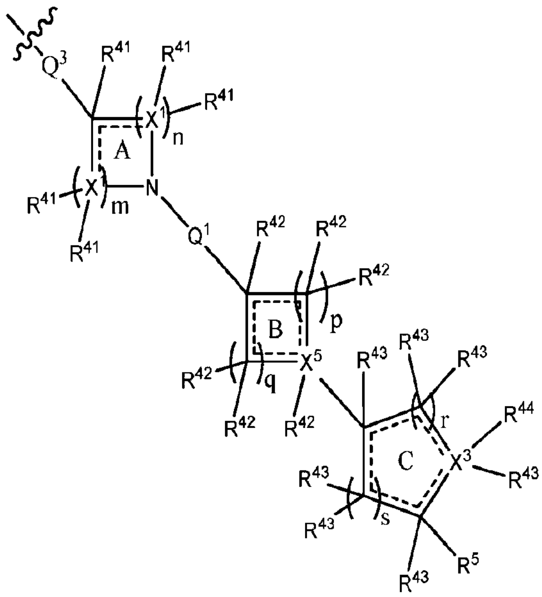

wherein n, m, p, q, r, s, $X^1$, $X^3$, $x^5$, $R^{41}$, $R^{42}$, $R^{43}$, $R^{44}$, $R^5$, $Q^1$, $Q^3$ and rings A, B, and C are as defined above. It is preferred that $X^5$ is N with no $R^{42}$ substituent.

In some embodiments, the group L comprises a group having the following structure:

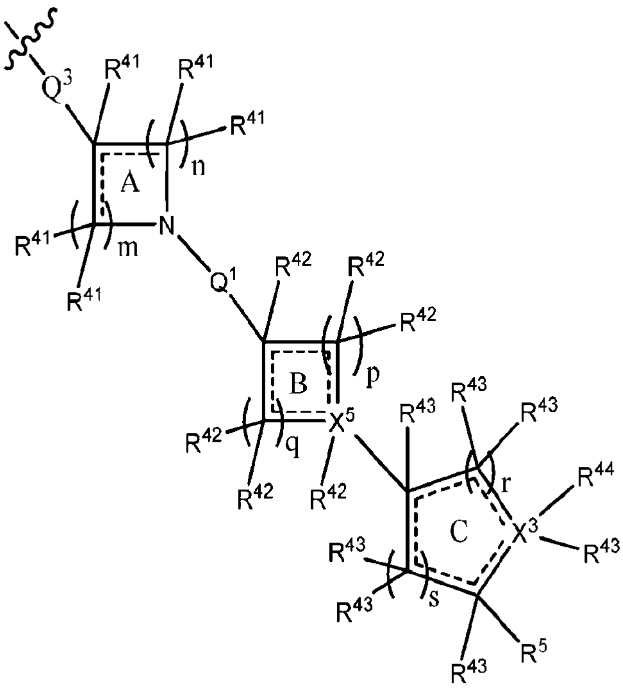

wherein n, m, p, q, r, s, $X^3$, $X^5$, $R^{41}$, $R^{42}$, $R^{43}$, $R^{44}$, $R^5$, and rings A, B, and C are as defined above. It is preferred that $X^3$ is N with no $R^{42}$ substituent.

In some embodiments, the group L comprises a group having the following structure:

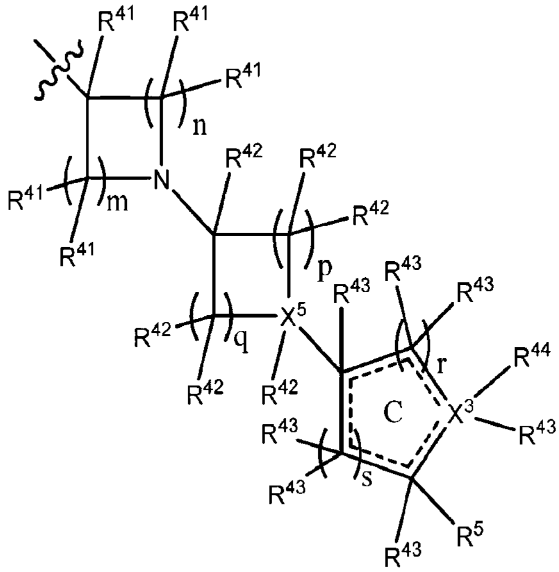

wherein n, m, p, q, r, s, $X^3$, $X^5$, $R^{41}$, $R^{42}$, $R^{43}$, $R^{44}$, $R^5$, and ring C, are as defined above. It is preferred that $X^5$ is N with no $R^{42}$ substituent.

In some embodiments, the group L comprises a group having the following structure:

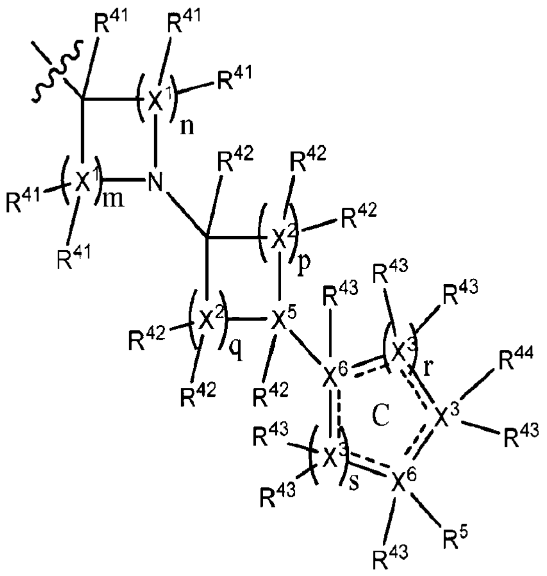

wherein n, m, p, q, r, s, $X^1$, $X^2$, $X^3$, $X^5$, $X^6$, $R^{41}$, $R^{42}$, $R^{43}$, $R^{44}$, $R^5$, and ring C, are as defined above. It is preferred that $X^5$ is N with no $R^{42}$ substituent.

In some embodiments, the group L comprises a group selected from the following structures:

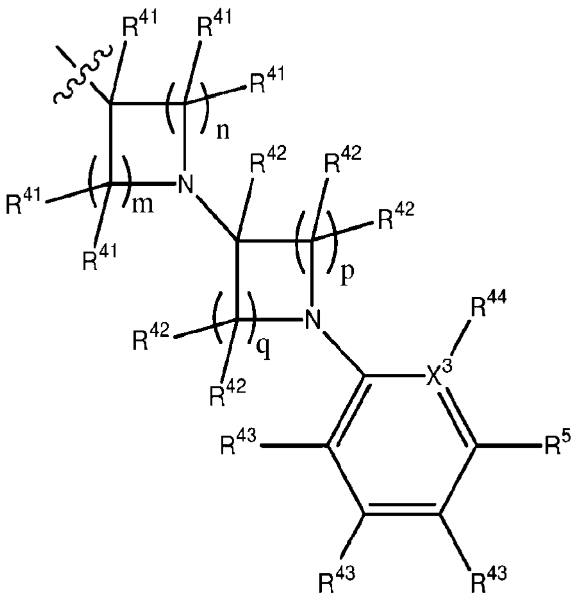

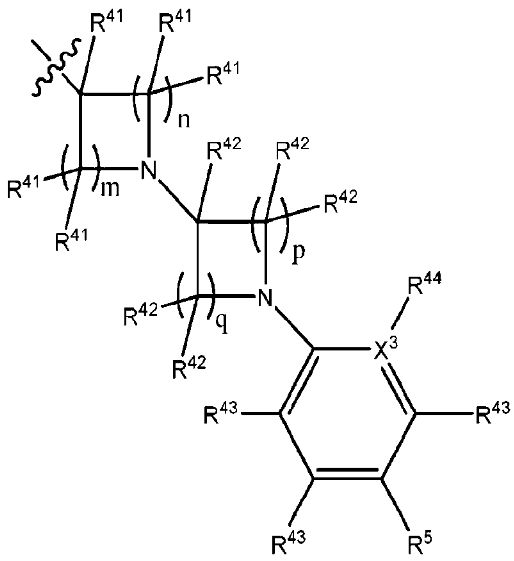

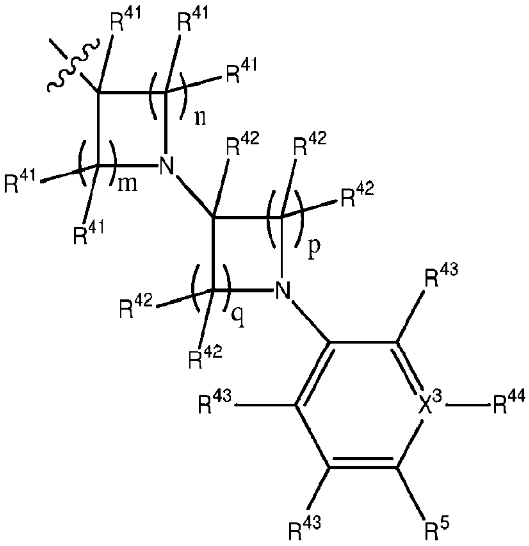

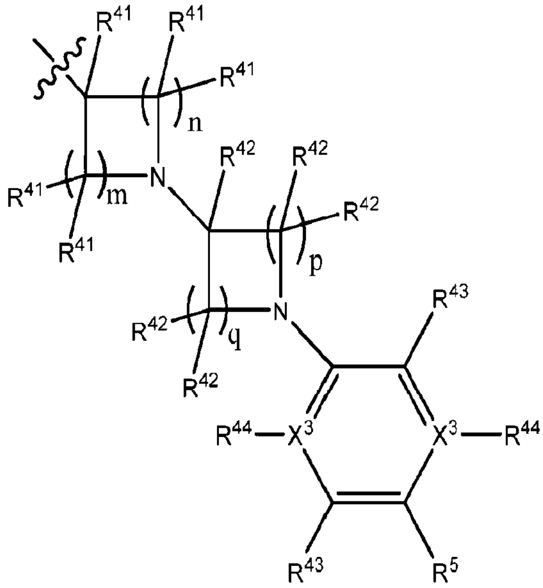

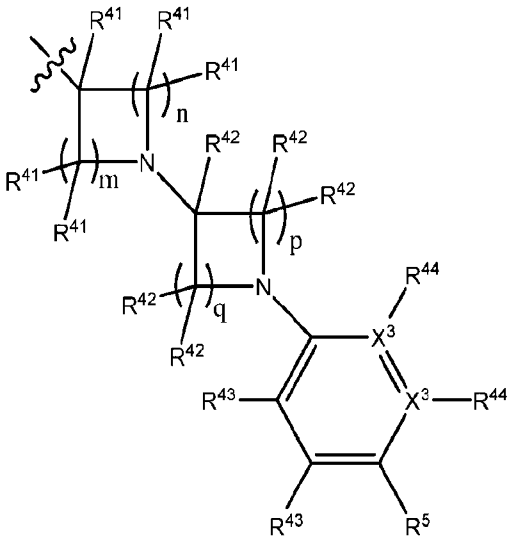

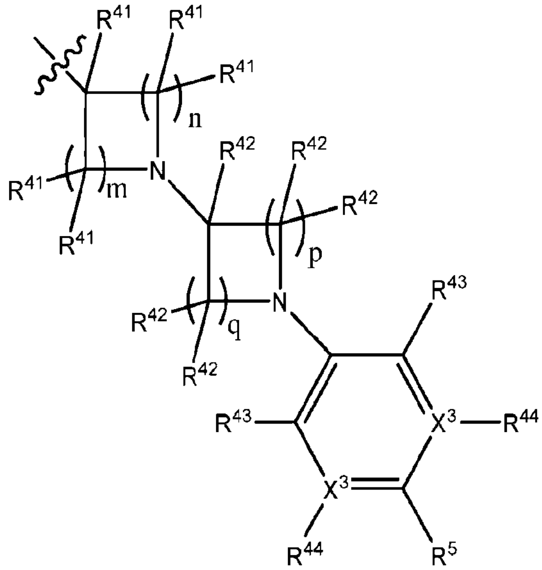

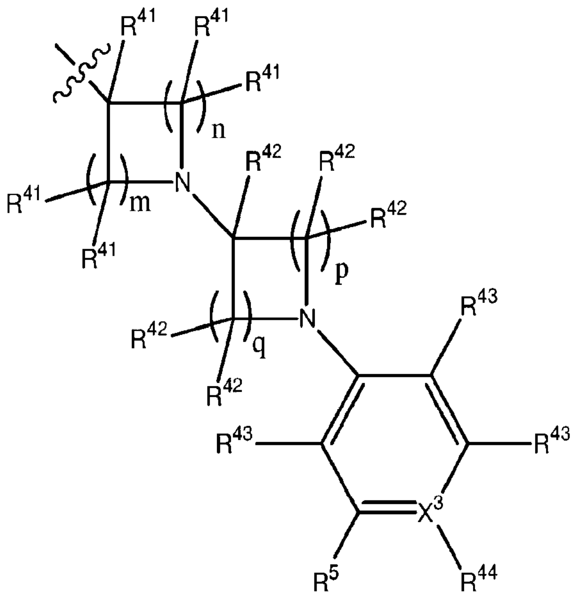

wherein n, m, p, q, $X^3$, $R^{41}$, $R^{42}$, $R^{43}$, $R^{44}$, and $R^5$, are as defined above.

In these structures for the L group it is preferred that m is selected from 1 or 2, n is selected from 2 or 3, p is selected from 1, 2 or 3 (more preferably for 2 or 3) and q is selected from 1 or 2.

In some embodiments, group L comprises a group having any the following structures:

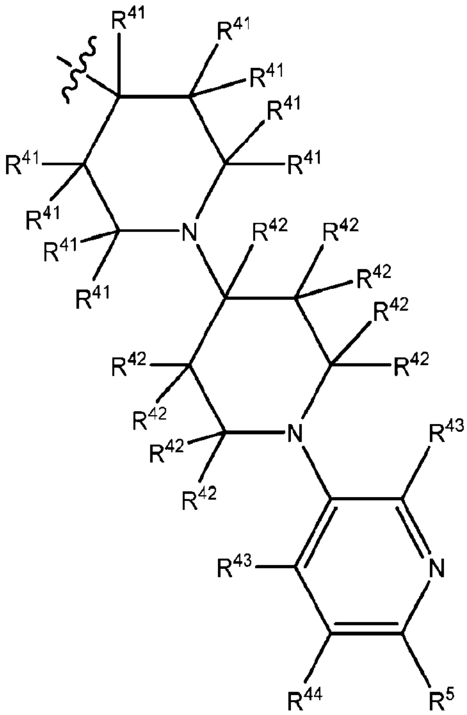

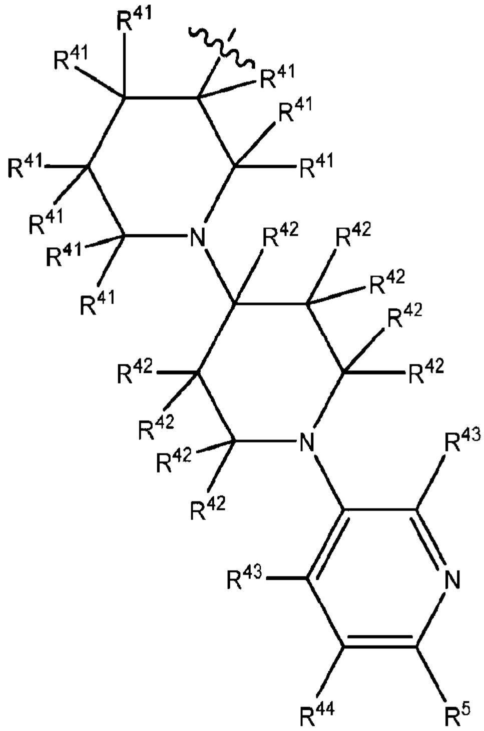

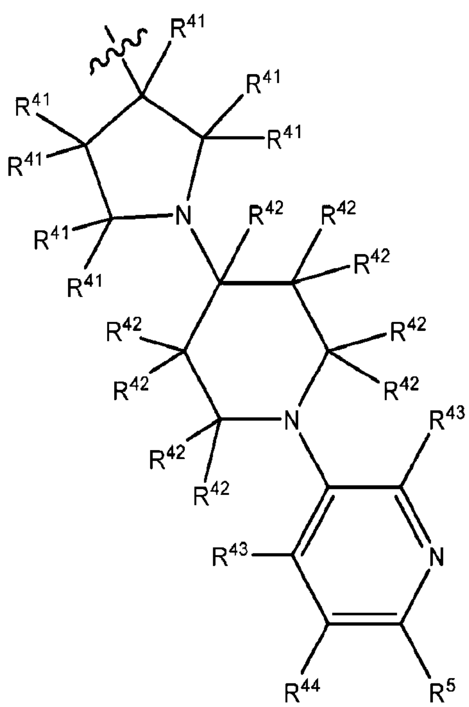

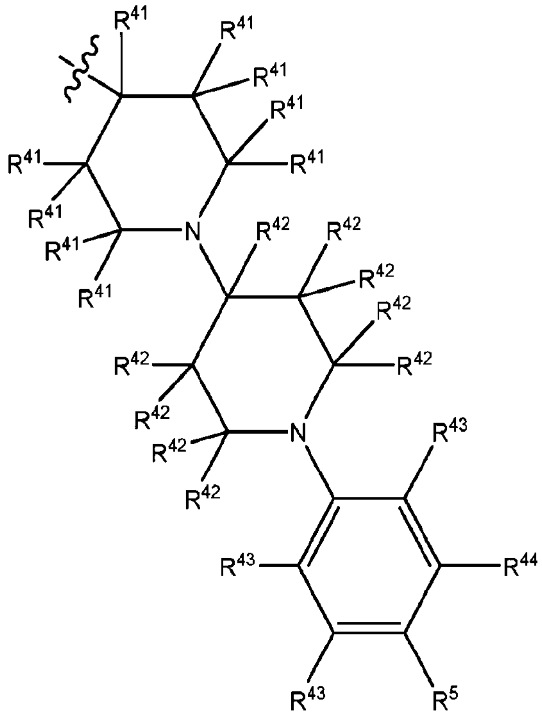

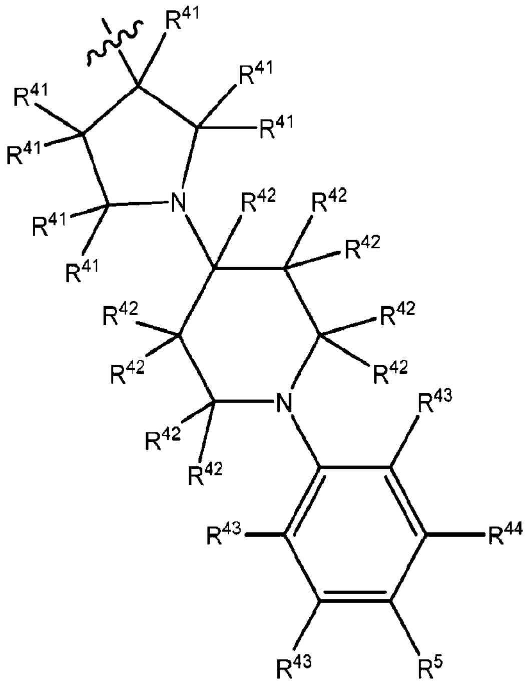

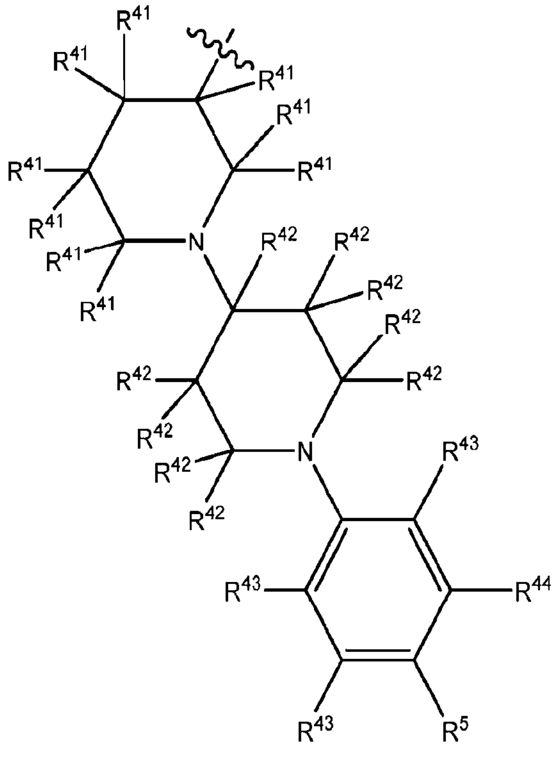

wherein, $R^{41}$, $R^{42}$, $R^{43}$, $R^{44}$,and $R^5$ are as defined above.

Typically, $R^{41}$, $R^{42}$, $R^{43}$, $R^{44}$ and $R^{45}$ are each independently selected from H and a group selected from the following groups:

- deuterium;
- a halogen (such as -F, -Cl, -Br and -I);
- a nitrile group;
- a substituted or unsubstituted linear or branched $C_1$-$C_6$ alkyl group (such as Me, Et, Pr, i-Pr, n-Bu, i-Bu, t-Bu, pentyl and hexyl);

- a substituted or unsubstituted linear or branched $C_1$-$C_6$ alkyl-aryl group (such as -$CH_2$Ph, - $CH_2$(2,3 or 4)F-Ph, -$CH_2$(2,3 or 4)Cl-Ph, -$CH_2$(2,3 or 4)Br-Ph, -$CH_2$(2,3 or 4)I-Ph, - $CH_2CH_2$Ph, -$CH_2CH_2CH_2$Ph, -$CH_2CH_{2C}H_2CH_2$Ph, -$CH_2CH_2CH_2CH_2CH_2$Ph, and -$CH_2CH_2CH_2CH_2CH_2CH_2$Ph);
- a substituted or unsubstituted linear or branched $C_1$-$C_6$ halogenated alkyl group (such as -$CH_2$F, -$CH_2$Cl, -$CH_2$Br, -$CH_2$I, -$CF_3$, -$CCl_3$ -$CBr_3$, -$CI_3$, -$CH_2CF_3$, -$CH_2CCl_3$, -$CH_2CBr_3$, and -$CH_2CI_3$);
- -$NH_2$ or a substituted or unsubstituted linear or branched primary secondary or tertiary $C_1$-$C_6$ amine group (such as -NMeH, -$NMe_2$, -NEtH, -NEtMe, -$NEt_2$, -NPrH, -NPrMe, -NPrEt, -$NPr_2$, -NBuH, -NBuMe, -NBuEt, -$CH_2$-$NH_2$, -$CH_2$-NMeH, -$CH_2$-$NMe_2$, -$CH_2$-NEtH, -$CH_2$-NEtMe, -$CH_2$-$NEt_2$, -$CH_2$-NPrH, -$CH_2$-NPrMe, and -$CH_2$-NPrEt);
- a substituted or unsubstituted amino-aryl group (such as -NH-Ph, -NH-(2,3 or 4)F-Ph, -NH-(2,3 or 4)Cl-Ph, -NH-(2,3 or 4)Br-Ph, -NH-(2,3 or 4)I-Ph, -NH-(2,3 or 4)Me-Ph, -NH-(2,3 or 4)Et-Ph, -NH-(2,3 or 4)Pr-Ph, -NH-(2.3 or 4)Bu-Ph, NH-(2,3 or 4)OMe-Ph, -NH-(2,3 or 4)OEt-Ph, -NH-(2,3 or 4)OPr-Ph, -NH-(2,3 or 4)OBu-Ph, -NH-2,(3,4,5 or 6)$F_2$-Ph, -NH-2,(3,4,5 or 6)$Cl_2$-Ph, -NH-2,(3,4,5 or 6)$Br_2$-Ph, -NH-2,(3,4,5 or 6)$I_2$-Ph, -NH-2,(3,4,5 or 6)$Me_2$-Ph, -NH-2,(3,4,5 or 6)$Et_2$-Ph, -NH-2,(3,4,5, or 6)$Pr_2$-Ph, -NH-2,(3,4,5 or 6)$Bu_2$-Ph,
- a substituted or unsubstituted cyclic amine or amido group (such as pyrrolidin-1-yl, pyrrolidin-2-yl, pyrrolidin-3-yl, piperidin-1-yl, piperidin-2-yl, piperidin-3-yl, piperidin-4-yl, morpholin-2-yl, morpholin-3-yl, morpholin-4-yl, 2-keto-pyrrolidinyl, 3-keto-pyrrolidinyl, 2-keto-piperidinyl, 3-keto-piperidinyl, and 4-keto-piperidinyl);
- a substituted or unsubstituted cyclic $C_3$-$C_8$ alkyl group (such as cyclopropyl, cyclobutyl, cyclopentyl, cyclohexyl, cycloheptyl and cyclooctyl);
- an -OH group or a substituted or unsubstituted linear or branched $C_1$-$C_6$ alcohol group (such as -$CH_2$OH, -$CH_2CH_2$OH, -$CH(CH_3)CH_2$OH, -$C(CH_3)_2$OH, -$CH_2CH_2CH_2$OH, - $CH_2CH_2CH_2CH_2$OH, -$CH(CH_3)CH_2CH_2$OH, -$CH(CH_3)CH(CH_3)$OH, -$CH(CH_2CH_3)CH_2$OH, -$C(CH_3)_2CH_2$OH, -$CH_2CH_2CH_2CH_2CH_2$OH, and -$CH_2CH_2CH_2CH_2CH_2CH_2$OH); - a substituted or unsubstituted linear or branched $C_1$-$C_6$ carboxylic acid group (such as -COOH, -$CH_2$COOH, -$CH_2CH_2$COOH, -$CH_2CH_2CH_2$COOH, -$CH_2CH_2CH_2CH_2$COOH, and -$CH_2CH_2CH_2CH_2CH_2$COOH);
- a substituted or unsubstituted linear or branched carbonyl group (such as -(CO)Me, -(CO)Et, -(CO)Pr, -(CO)iPr, -(CO)nBu, -(CO)iBu, -(CO)tBu, -(CO)Ph, -(CO)$CH_2$Ph, -(CO)$CH_2$OH, -(CO)$CH_2OCH_3$, -(CO)$CH_2NH_2$, -(CO)$CH_2$NHMe, -(CO)$CH_2NMe_2$, -(CO)-cyclopropyl, -(CO)-1,3-epoxypropan-2-yl; -(CO)$NH_2$, -(CO)NHMe, -(CO)$NMe_2$, -(CO)NHEt, -(CO)$NEt_2$, -(CO)-pyrollidine-N-yl, -(CO)-morpholine-N-yl, -(CO)-piperazine-N-yl, -(CO)-N-methyl-piperazine-N-yl, -(CO)$NHCH_2CH_2$OH, -(CO)$NHCH_2CH_2$OMe, -(CO)$NHCH_2CH_2NH_2$, -(CO)$NHCH_2CH_2$NHMe, and -(CO)$NHCH_2CH_2NMe_2$;
- a substituted or unsubstituted linear or branched $C_1$-$C_6$ carboxylic acid ester group (such as -COOMe, -COOEt, -COOPr, -COO-i-Pr, -COO-n-Bu, -COO-i-Bu, -COO-t-Bu, -$CH_2$COOMe, -$CH_2CH_2$COOMe, -$CH_2CH_2CH_2$COOMe, and -$CH_2CH_2CH_2CH_2$COOMe);
- a substituted or unsubstituted linear or branched $C_1$-$C_6$ amide group (such as -CO-$NH_2$, - CO-NMeH, -CO-$NMe_2$, -CO-NEtH, -CO-NEtMe, -CO-$NEt_2$, -CO-NPrH, -CO-NPrMe, and - CO-NPrEt);
- a substituted or unsubstituted linear or branched $C_1$-$C_7$ amino carbonyl group (such as -NH-CO-Me, -NH-CO-Et, -NH-CO-Pr, -NH-CO-Bu, -NH-CO-pentyl, -NH-CO-hexyl, -NH-CO-Ph, -NMe-CO-Me, -NMe-CO-Et, -NMe-CO-Pr, -NMe-CO-Bu, -NMe-CO-pentyl, -NMe-CO-hexyl, -NMe-CO-Ph;
- a substituted or unsubstituted linear or branched $C_1$-$C_7$ alkoxy or aryloxy group (such as - OMe, -OEt, -OPr, -O-i-Pr, -O-n-Bu, -O-i-Bu, -O-t-Bu, -O-pentyl, -O-hexyl, -$OCH_2$F, -$OCHF_2$, -$OCF_3$, -$OCH_2$Cl, -$OCHCl_2$, -$OCCl_3$, -O-Ph, -O-$CH_2$-Ph, -O-$CH_2$-(2,3 or 4)-F-Ph, -O-$CH_2$-(2,3 or 4)-Cl-Ph, -$CH_2$OMe, -$CH_2$OEt, -$CH_2$OPr, -$CH_2$OBu, -$CH_2CH_2$OMe, -$CH_2CH_2CH_2$OMe, -$CH_2CH_2CH_2CH_2$OMe, and -$CH_2CH_2CH_2CH_2CH_2$OMe);
- a substituted or unsubstituted linear or branched aminoalkoxy group (such as - $OCH_2NH_2$, -$OCH_2$NHMe, -$OCH_2NMe_2$, -$OCH_2$NHEt, -$OCH_2NEt_2$, -$OCH_2CH_2NH_2$, -$OCH_2$C $H_2$NHMe, -$OCH_2CH_2NMe_2$, -$OCH_2CH_2$NHEt, and -$OCH_2CH_2NEt_2$;
- a substituted or unsubstituted sulphonyl group (such as -$SO_2$Me, -$SO_2$Et, -$SO_2$Pr, -$SO_2$iPr, - $SO_2$Ph, -$SO_2$-(2,3 or 4)-F-Ph, -$SO_2$-cyclopropyl, -$SO_2CH_2CH_2OCH_3$), -$SO_2NH_2$, -$SO_2$NHMe, -$SO_2NMe_2$, -$SO_2$NHEt, -$SO_2NEt_2$, -SO2-pyrrolidine-N-yl, -$SO_2$-morpholine-N-yl, -$SO_2NHCH_2$OMe, and -$SO_2NHCH_2CH_2$OMe;
- a substituted or unsubstituted aminosulphonyl group (such as -$NHSO_2$Me, - $NHSO_2$Et, - $NHSO_2$Pr, - $NHSO_2$iPr, - $NHSO_2$Ph, - $NHSO_2$-(2,3 or 4)-F-Ph, - $NHSO_2$-cyclopropyl, - $NHSO_2CH_2CH_2OCH_3$);
- a substituted or unsubstituted aromatic group (such as Ph-, 2-F-Ph-, 3-F-Ph-, 4-F-Ph-, 2-Cl-Ph-, 3-Cl-Ph-, 4-Cl-Ph-, 2-Br-Ph-, 3-Br-Ph-, 4-Br-Ph-, 2-1-Ph-, 3-I-Ph, 4-1-Ph-, 2,(3,4,5 or 6)-$F_2$-Ph-, 2,(3,4,5 or 6)-$Cl_2$-Ph-, 2,(3,4,5 or 6)-$Br_2$-Ph-, 2,(3,4,5 or 6)-$I_2$-Ph-, 2,(3,4,5 or 6)-$Me_2$-Ph-, 2,(3,4,5 or 6)-$Et_2$-Ph-, 2.(3,4,5 or 6)-$Pr_2$-Ph-, 2,(3,4,5 or 6)-$Bu_2$-Ph-, 2,(3,4,5 or 6)-$(CN)_2$-Ph-, 2,(3,4,5 or 6)-$(NO_2)_2$-Ph-, 2,(3,4,5 or 6)-$(NH_2)_2$-Ph-, 2,(3,4,5 or 6)-$(MeO)_2$-Ph-, 2,(3,4,5 or 6)-$(CF_3)_2$-Ph-, 3,(4 or 5)-$F_2$-Ph-, 3,(4 or 5)-$Cl_2$-Ph-, 3,(4 or 5)-$Br_2$-Ph-, 3,(4 or 5)-$I_2$-Ph-, 3,(4 or 5)-$Me_2$-Ph-, 3,(4 or 5)-$Et_2$-Ph-, 3,(4 or 5)-$Pr_2$-Ph-, 3,(4 or 5)-$Bu_2$-Ph-, 3,(4 or 5)-$(CN)_2$-Ph-, 3,(4 or 5)-$(NO_2)_2$-Ph-, 3,(4 or 5)-$(NH_2)_2$-Ph-, 3,(4 or 5)-$(MeO)_2$-Ph-, 3,(4 or 5)-$(CF_3)_2$-Ph-, 2-Me-Ph-, 3-Me-Ph-, 4-Me-Ph-, 2-Et-Ph-, 3-Et-Ph-, 4-Et-Ph-, 2-Pr-Ph-, 3-Pr-Ph-, 4-Pr-Ph-, 2-Bu-Ph-, 3-Bu-Ph-, 4-Bu-Ph-, 2-(CN)-Ph-, 3-(CN)-Ph-, 4-(CN)-Ph-,

$2-(NO_2)$-Ph-, $3-(NO_2)$-Ph- , $4-(NO_2)$-Ph-, $2-(NH_2)$-Ph-, $3-(NH_2)$-Ph-, $4-(NH_2)$-Ph-, 2-MeO-Ph-, 3-MeO-Ph-, 4-MeO-Ph-, $2-(NH_2-CO)$-Ph-, $3-(NH_2-CO)$-Ph-, $4-(NH_2-CO)$-Ph-, $2-CF_3$-Ph-, $3-CF_3$-Ph-, $4-CF_3$-Ph-, $2-CF_3O$-Ph-, $3-CF_3O$-Ph-, and $4-CF_3O$-Ph-);

- a saturated or unsaturated, substituted or unsubstituted, heterocyclic group including an aromatic heterocyclic group and/or a non-aromatic heterocyclic group (such as pyrrole-1-yl, pyrrole-2-yl, pyrrole-3-yl, pyrazole-1-yl, pyrazole-3-yl, pyrazole-4-yl, pyrazole-5-yl, imidazole-1-yl, imidazole-2-yl, imidazole-4-yl, imidazole-5-yl, 1,2,3-triazole-1-yl, 1,2,3-triazole-4-yl, 1,2,3-triazole-5-yl, 1,2,4-triazole-1-yl, 1,2,4-triazole-3-yl, 1,2,4-triazole-5-yl, pyridin-2-yl, pyridin-3-yl, pyridin-4-yl, pyridazine-3-yl, pyridazine-4-yl, pyrimidin-2-yl, pyrimidin-4-yl, pyrimidin-5-yl, pyrimidin-6-yl, pyrazine-2-yl, pyrrolidine-1-yl, pyrrolidine-2-yl, pyrrolidine-3-yl, piperidine-1-yl, piperidine-2-yl, piperidine-3-yl, piperidine-4-yl, 2-azapiperidine-1-yl, 2-azapiperidine-3-yl, 2-azapiperidine-4-yl, 3-azapiperidine-1-yl, 3-azapiperidine-2-yl, 3-azapiperidine-4-yl, 3-azapiperidine-5-yl, piperazine-1-yl, piperazine-2-yl, furan-2-yl, furan-3-yl, pyran-2-yl, pyran-3-yl, pyran-4-yl, 2-azapyran-2-yl, 2-azapyran-3-yl, 2-azapyran-4-yl, 2-azapyran-5-yl, 2-azapyran-6-yl, 3-azapyran-2-yl, 3-azapyran-4-yl, 3-azapyran-5-yl, 3-azapyran-6-yl, 4-azapyran-2-yl, 4-azapyran-3-yl, 4-azapyran-4-yl, 4-azapyran-5-yl, 4-azapyran-6-yl, oxetan-2-yl, oxetan-3-yl, tetrahydrofuran-2-yl, tetrahydrofuran-3-yl, 2-aza-tetrahydrofuran-2-yl, 2-aza-tetrahydrofuran-3-yl, 2-aza-tetrahydrofuran-4-yl, 2-aza-tetrahydrofuran-5-yl, 3-aza-tetrahydrofuran-2-yl, 3-aza-tetrahydrofuran-3-yl, 3-aza-tetrahydrofuran-4-yl, 3-aza-tetrahydrofuran-5-yl, tetrahydropyran-2-yl, tetrahydropyran-3-yl, tetrahydropyran-4-yl, 2-aza-tetrahydropyran-2-yl, 2-aza-tetrahydropyran-3-yl, 2-aza-tetrahydropyran-4-yl, 2-aza-tetrahydropyran-5-yl, 2-aza-tetrahydropyran-6-yl, 3-aza-tetrahydropyran-2-yl, 3-aza-tetrahydropyran-3-yl, 3-aza-tetrahydropyran-4-yl, 3-aza-tetrahydropyran-5-yl, 3-aza-tetrahydropyran-6-yl, morpholine-2-yl, morpholine-3-yl, morpholine-4-yl, thiophen-2-yl, thiophen-3-yl, isothiazole-3-yl, isothiazole-4-yl, isothiazole-5-yl, thiazole-2-yl, thiazole-4-yl, thiazole-5-yl, thiopyran-2-yl, thiopyran-3-yl, thiopyran-4-yl, 2-azathiopyran-2-yl, 2-azathiopyran-3-yl, 2-azathiopyran-4-yl, 2-azathiopyran-5-yl, 2-azathiopyran-6-yl, 3-azathiopyran-2-yl, 3-azathiopyran-4-yl, 3-azathiopyran-5-yl, 3-azathiopyran-6-yl, 4-azathiopyran-2-yl, 4-azathiopyran-3-yl, 4-azathiopyran-4-yl, 4-azathiopyran-5-yl, 4-azathiopyran-6-yl, thiolane-2-yl, thiolane-3-yl, thiane-2-yl, thiane-3-yl, thiane-4-yl, oxazol-2-yl, oxazol-4-yl, oxazol-5-yl, isoxazol-3-yl, isoxazol-4-yl, isoxazol-5-yl, furazan-3-yl, (1,3,4-oxadiazol)-2-yl, (1,3,4-oxadiazol)-5-yl, (1,2,4-oxadiazol)-3-yl, (1,2,4-oxadiazol)-5-yl; and tetrazole-1-yl, tetrazole-2-yl, tetrazole-5-yl);
- where there are two R groups attached to the same atom, they may together form a group which is double bonded to that atom, (such as a carbonyl group (=O) or an alkene group ($=C(R')_2$) wherein each R' group is the same or different and is H or an organic group, preferably H or a straight or branched $C_1$-$C_6$ alkyl group).

In some arrangements, a pair of $R^{41}$ groups attached to different atoms may together form a ring with ring A atoms, and/or a pair of $R^{42}$ groups attached to different atoms may together form a ring with ring B atoms, optionally wherein each of the pair of $R^{41}$ groups and/or pair of $R^{42}$ groups independently comprises $(X^7)_{1\text{ or }2}$, wherein each $X^7$ may be the same or different and is independently selected from C, N, O and S; and wherein each $X^7$ is independently unsubstituted or (i) independently substituted with H or an organic group selected from a $C_1$-$C_6$ alkyl group, a $C_1$-$C_6$ halogenated alkyl group, a halogen such as F, or hydroxyl, when $X^7$ is C; and (ii) independently substituted with H or an organic group selected from a $C_1$-$C_6$ alkyl group or a $C_1$-$C_6$ amide group, when $X^7$ is N.

In some arrangements, groups $R^5$ and $R^{44}$ may together form a ring with atoms $X^6$ and $X^3$ of ring C to which they are attached, optionally wherein the $R^5$ and $R^{44}$ groups together comprise $(X^8)_{3,\ 4\text{ or }5}$, wherein each $X^8$ may be the same or different and is independently selected from C, N, O and S; and wherein each $X^8$ is independently unsubstituted or (i) independently substituted with H or an organic group selected from a $C_1$-$C_6$ alkyl group, a $C_1$-$C_6$ halogenated alkyl group, a halogen such as F, or hydroxyl, when $X^8$ is C; and (ii) independently substituted with H or an organic group selected from a $C_1$-$C_6$ alkyl group, a $C_1$-$C_6$ halogenated alkyl group, or a $C_1$-$C_6$ amide group, when $X^8$ is N.

It is preferred that $R^{41}$, $R^{42}$, $R^{43}$ and $R^{44}$ are each independently selected from H, deuterium, a halogen (such as -F, -Cl, -Br, and -I, preferably F or Cl), a nitrile group, a substituted or unsubstituted $C_1$-$C_6$ alkyl group, a substituted or unsubstituted linear or branched $C_1$-$C_6$ halogenated alkyl group, (preferably $CF_3$ or $CHF_2$), a cyclopropyl group, an -OH group or a substituted or unsubstituted linear or branched $C_1$-$C_6$ alcohol group, a substituted or unsubstituted linear or branched $C_1$-$C_3$ amino carbonyl group (such as -NH-CO-Me), an $-NH_2$ group or a substituted or unsubstituted $C_1$-$C_6$ amino group and a substituted or unsubstituted $C_1$-$C_6$ alkoxy group; wherein, when a pair of $R^{41}$ groups attached to different atoms together forms a ring with ring A atoms, and/or a pair of $R^{42}$ groups attached to different atoms together forms a ring with ring B atoms, each of the pair of $R^{41}$ groups and/or pair of $R^{42}$ groups independently comprises $-CH_2-$ or $-CH_2CH_2-$; and wherein, when groups $R^5$ and $R^{44}$ together form a ring with atoms of ring C, $R^5$ and $R^{44}$ together comprise -CH=CH-CH=CH- or -NH-CO-NH-.

It is preferred that $R^{45}$ is selected from H, a halogen (such as -F, -Cl, -Br, and -I, preferably - F), a substituted or unsubstituted $C_1$-$C_6$ alkyl group, a substituted or unsubstituted linear or branched $C_1$-$C_6$ halogenated alkyl group (preferably $CF_3$), an $-NH_2$ group or a substituted or unsubstituted $C_1$-$C_6$ amino group, an -OH group or a substituted or unsubstituted linear or branched $C_1$-$C_6$ alcohol group and a substituted or unsubstituted $C_1$-$C_6$ alkoxy group.

Typically, $R^{46}$ is selected from H and a group selected from the following groups:

- a substituted or unsubstituted linear or branched $C_1$-$C_6$ alkyl group (such as Me, Et, Pr, i-Pr, n-Bu, i-Bu, t-Bu, pentyl and hexyl);
- a substituted or unsubstituted linear or branched $C_1$-$C_6$ alkyl-aryl group (such as $-CH_2Ph$, - $CH_2$(2,3 or 4)F-Ph, $-CH_2$(2,3 or 4)Cl-Ph, $-CH_2$(2,3 or 4)Br-Ph, $-CH_2$(2,3 or 4)I-Ph, - $CH_2CH_2Ph$, $-CH_2CH_2CH_2Ph$, $-CH_2CH_2CH_2CH_2Ph$, $-CH_2CH_2CH_2CH_2CH_2Ph$, and $-CH_2CH_2CH_2CH_2CH_2CH_2Ph$);
- a substituted or unsubstituted linear or branched $C_1$-$C_6$ halogenated alkyl group (such as $-CH_2F$, and $-CH_2CF_3$);
- a substituted or unsubstituted cyclic amine or amido group (such as pyrrolidin-3-yl, piperidin-3-yl, piperidin-4-yl, 2-keto-pyrrolidinyl, 3-keto-pyrrolidinyl, 2-keto-piperidinyl, 3-keto-piperidinyl, and 4-keto-piperidinyl);
- a substituted or unsubstituted cyclic $C_3$-$C_8$ alkyl group (such as cyclopropyl, cyclobutyl, cyclopentyl, cyclohexyl, cycloheptyl and cyclooctyl);
- a substituted or unsubstituted linear or branched $C_2$-$C_6$ alcohol group (such as $-CH_2CH_2OH$, $-CH(CH_3)CH_2OH$, $-C(CH_3)_2OH$, $-CH_2CH_2CH_2OH$, - $CH_2CH_2CH_2CH_2OH$, $-CH(CH_3)CH_2CH_2OH$, $-CH(CH_3)CH(CH_3)OH$, $-CH(CH_2CH_3)CH_2OH$, $-C(CH_3)_2CH_2OH$, $-CH_2CH_2CH_2CH_2CH_2OH$, and $-CH_2CH_2CH_2CH_2CH_2CH_2OH$);
- a substituted or unsubstituted linear or branched $C_2$-$C_6$ carboxylic acid group (such as - $CH_2COOH$, $-CH_2CH_2COOH$, $-CH_2CH_2CH_2COOH$, $-CH_2CH_2CH_2CH_2COOH$, and $-CH_2CH_2CH_2CH_2CH_2COOH$);
- a substituted or unsubstituted linear or branched carbonyl group (such as -(CO)Me, -(CO)Et, -(CO)Pr, -(CO)-i_Pr, -(CO)-n-Bu, -(CO)-i-Bu, -(CO)-t-Bu, -(CO)Ph, -( CO)$CH_2$Ph, -(CO)$CH_2$OH, -(CO)$CH_2OCH_3$, -(CO)$CH_2NH_2$, -(CO) $CH_2$NHMe, -(CO)$CH_2NMe_2$, -(CO)-cyclopropyl, -(CO)-1,3-epoxypropan-2-yl: -(CO)$NH_2$, -(CO)NHMe, -(CO)$NMe_2$, -(CO)NHEt, -(CO)$NEt_2$, -(CO)-pyrollidine-N-yl, -(CO)-morpholine-N-yl, -(CO)-piperazine-N-yl, -(CO)-N-methyl-piperazine-N-yl, -(CO)NH$CH_2CH_2$OH, -(CO)NH$CH_2CH_2$OMe, -(CO)NH$CH_2CH_2NH_2$, -(CO)NH$CH_2CH_2$NHMe, and -(CO)NH$CH_2CH_2NMe_2$;
- a substituted or unsubstituted linear or branched $C_1$-$C_6$ carboxylic acid ester group (such as -COOMe, -COOEt, -COOPr, -COO-i-Pr, -COO-n-Bu, -COO-i-Bu, -COO-t-Bu, $-CH_2$COOMe, $-CH_2CH_2$COOMe, $-CH_2CH_2CH_2$COOMe, and $-CH_2CH_2CH_2CH_2$COOMe);
- a substituted or unsubstituted linear or branched $C_1$-$C_6$ amide group (such as -CO-$NH_2$, - CO-NMeH, -CO-$NMe_2$, -CO-NEtH, -CO-NEtMe, -CO-$NEt_2$, -CO-NPrH, -CO-NPrMe, and - CO-NPrEt);
- a substituted or unsubstituted sulphonyl group (such as $-SO_2$Me, $-SO_2$Et, $-SO_2$Pr, $-SO_2$iPr, - $SO_2$Ph, $-SO_2$-(2,3 or 4)-F-Ph, $-SO_2$-cyclopropyl, $-SO_2CH_2CH_2OCH_3$), $-SO_2NH_2$, $-SO_2$NHMe, $-SO_2NMe_2$, $-SO_2$NHEt, $-SO_2NEt_2$, $-SO_2$-pyrrolidine-N-yl, $-SO_2$-morpholine-N-yl, $-SO_2$NH$CH_2$OMe, and $-SO_2$NH$CH_2CH_2$OMe;
- a substituted or unsubstituted aromatic group (such as Ph-, 2-F-Ph-, 3-F-Ph-, 4-F-Ph-, 2-Cl-Ph-, 3-Cl-Ph-, 4-Cl-Ph-, 2-Br-Ph-, 3-Br-Ph-, 4-Br-Ph-, 2-1-Ph-, 3-I-Ph, 4-I-Ph-, 2,(3,4.5 or 6)-$F_2$-Ph-, 2,(3,4,5 or 6)-$Cl_2$-Ph-, 2,(3,4,5 or 6)-$Br_2$-Ph-, 2,(3,4,5 or 6)-$I_2$-Ph-, 2,(3,4,5 or 6)-$Me_2$-Ph-, 2,(3,4,5 or 6)-$Et_2$-Ph-, 2,(3,4,5 or 6)-$Pr_2$-Ph-, 2,(3,4,5 or 6)-$Bu_2$-Ph-, 2,(3,4,5 or 6)-$(CN)_2$-Ph-, 2,(3,4,5 or 6)-$(NO_2)_2$-Ph-, 2,(3,4,5 or 6)-$(NH_2)_2$-Ph-, 2,(3,4,5 or 6)-$(MeO)_2$-Ph-, 2,(3,4,5 or 6)-$(CF_3)_2$-Ph-, 3,(4 or 5)-$F_2$-Ph-, 3,(4 or 5)-$Cl_2$-Ph-, 3,(4 or 5)-$Br_2$-Ph-, 3,(4 or 5)-$I_2$-Ph-, 3,(4 or 5)-$Me_2$-Ph-, 3,(4 or 5)-$Et_2$-Ph-, 3,(4 or 5)-$Pr_2$-Ph-, 3,(4 or 5)-$Bu_2$-Ph-, 3,(4 or 5)-$(CN)_2$-Ph-, 3,(4 or 5)-$(NO_2)_2$-Ph-, 3,(4 or 5)-$(NH_2)_2$-Ph-, 3,(4 or 5)-$(MeO)_2$-Ph-, 3,(4 or 5)-$(CF_3)_2$-Ph-, 2-Me-Ph-, 3-Me-Ph-, 4-Me-Ph-, 2-Et-Ph-, 3-Et-Ph-, 4-Et-Ph-, 2-Pr-Ph-, 3-Pr-Ph-, 4-Pr-Ph-, 2-Bu-Ph-, 3-Bu-Ph-, 4-Bu-Ph-, 2-(CN)-Ph-, 3-(CN)-Ph-, 4-(CN)-Ph-, 2-$(NO_2)$-Ph-, 3-$(NO_2)$-Ph- , 4-$(NO_2)$-Ph-, 2-$(NH_2)$-Ph-, 3-$(NH_2)$-Ph-, 4-$(NH_2)$-Ph-, 2-MeO-Ph-, 3-MeO-Ph-, 4-MeO-Ph-, 2-$(NH_2$-CO)-Ph-, 3-$(NH_2$-CO)-Ph-, 4-$(NH_2$-CO)-Ph-, 2-$CF_3$-Ph-, 3-$CF_3$-Ph-, 4-$CF_3$-Ph-, 2-$CF_3$O-Ph-, 3-$CF_3$O-Ph-, and 4-$CF_3$O-Ph-); and
- a substituted or unsubstituted saturated or unsaturated, substituted or unsubstituted, heterocyclic group including an aromatic heterocyclic group and/or a non-aromatic heterocyclic group (such as pyrrole-2-yl, pyrrole-3-yl, pyrazole-3-yl, pyrazole-4-yl, pyrazole-5-yl, imidazole-2-yl, imidazole-4-yl, imidazole-5-yl, 1,2,3-triazole-4-yl, 1,2,3-triazole-5-yl, 1,2,4-triazole-3-yl, 1,2,4-triazole-5-yl, pyridin-2-yl, pyridin-3-yl, pyridin-4-yl, pyridazine-3-yl, pyridazine-4-yl, pyrimidin-2-yl, pyrimidin-4-yl, pyrimidin-5-yl, pyrimidin-6-yl, pyrazine-2-yl, pyrrolidine-2-yl, pyrrolidine-3-yl, piperidine-2-yl, piperidine-3-yl, piperidine-4-yl, 2-azapiperidine-3-yl, 2-azapiperidine-4-yl, 3-azapiperidine-2-yl, 3-azapiperidine-4-yl, 3-azapiperidine-5-yl, piperazine-2-yl, furan-2-yl, furan-3-yl, pyran-2-yl, pyran-3-yl, pyran-4-yl, 2-azapyran-3-yl, 2-azapyran-4-yl, 2-azapyran-5-yl, 2-azapyran-6-yl, 3-azapyran-2-yl, 3-azapyran-4-yl, 3-azapyran-5-yl, 3-azapyran-6-yl, 4-azapyran-2-yl, 4-azapyran-3-yl, 4-azapyran-5-yl, 4-azapyran-6-yl, oxctan-3-yl, tetrahydrofuran-2-yl, tetrahydrofuran-3-yl, 2-aza-tetrahydrofuran-3-yl, 2-aza-tetrahydrofuran-4-yl, 2-aza-tetrahydrofuran-5-yl, 3-aza-tetrahydrofuran-2-yl, 3-aza-tetrahydrofuran-4-yl, 3-aza-tetrahydrofuran-5-yl, tetrahydropyran-2-yl, tetrahydropyran-3-yl, tetrahydropyran-4-yl, 2-aza-tetrahydropyran-3-yl, 2-aza-tetrahydropyran-4-yl, 2-aza-tetrahydropyran-5-yl, 2-aza-tetrahydropyran-6-yl, 3-aza-tetrahydropyran-2-yl, 3-aza-tetrahydropyran-4-yl, 3-aza-tetrahydropyran-5-yl, 3-aza-tetrahydropyran-6-yl, morpholine-2-yl, morpholine-3-yl, thiophen-2-yl, thiophen-3-yl, isothiazole-3-yl, isothiazole-4-yl, isothiazole-5-yl, thiazole-2-yl, thiazole-4-yl, thiazole-5-yl, thiopyran-2-yl, thiopyran-3-yl, thiopyran-4-yl, 2-azathiopyran-3-yl, 2-azathiopyran-4-yl, 2-azathiopyran-5-yl, 2-azathiopyran-6-yl, 3-azathiopyran-2-yl, 3-azathiopyran-4-yl, 3-azathiopyran-5-yl, 3-azathiopyran-6-yl, 4-azathiopyran-2-yl, 4-azathiopyran-3-yl, 4-azathiopyran-5-yl, 4-azathiopyran-6-yl, thiolane-2-yl, thiolane-3-yl, thiane-2-yl, thiane-3-yl, thiane-4-yl, oxazol-2-yl, oxazol-4-yl, oxazol-5-yl, isoxazol-3-yl, isoxazol-4-yl, isoxazol-5-yl, furazan-3-yl, (1,3,4-oxadiazol)-2-yl, (1,3,4-oxadiazol)-5-yl, (1,2,4-oxadiazol)-3-yl, (1,2,4-oxadiazol)-5-yl; and tetrazole-5-yl).

It is preferred that $R^{46}$ is selected from H, a substituted or unsubstituted $C_1$-$C_6$ alkyl group or a substituted or unsubstituted linear or branched $C_1$-$C_6$ halogenated alkyl group.

Ring A of the group L preferably comprises any of the following structures:

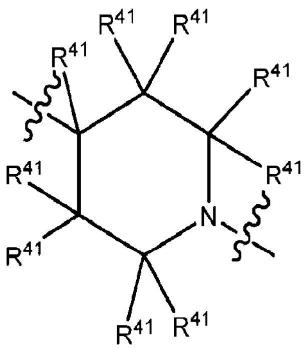
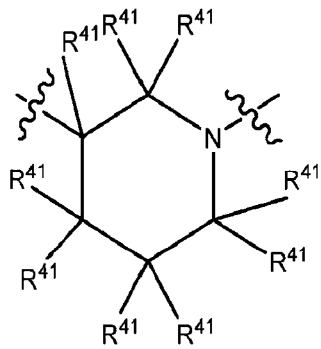
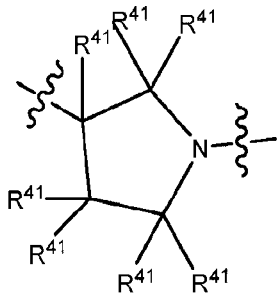

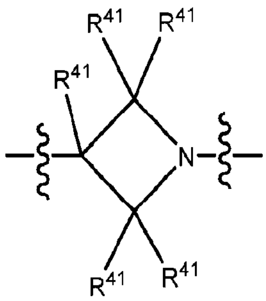
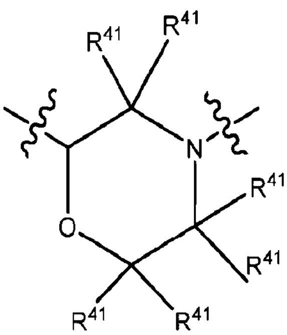
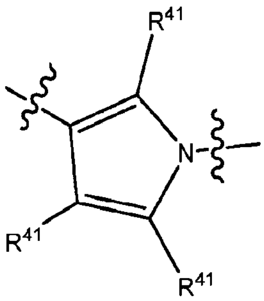
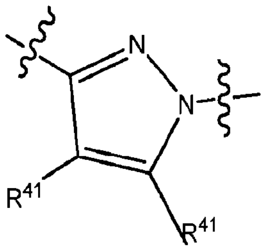

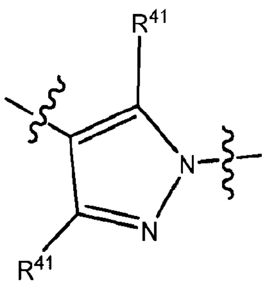
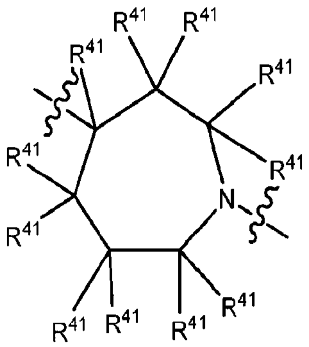
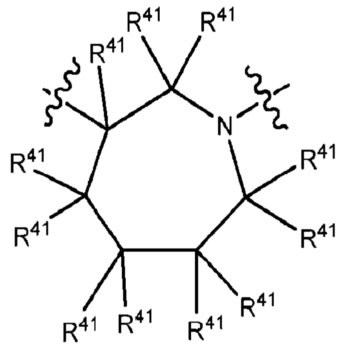

wherein $R^{41}$ is as defined herein.

More preferably, ring A of the group L comprises

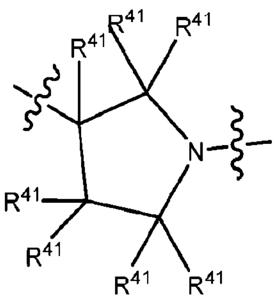

.

Still more preferably, ring A of the group L comprises any of the following structures:

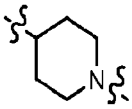 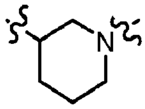 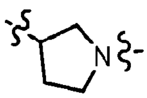 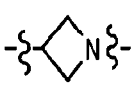 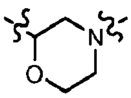 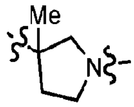 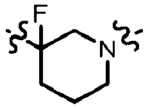

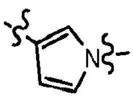 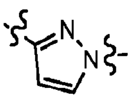 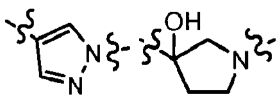 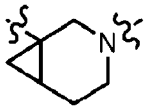 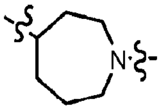 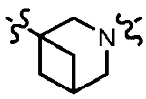

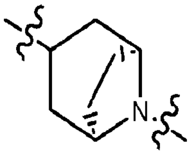 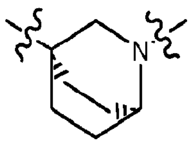 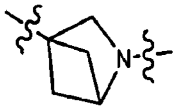 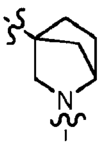 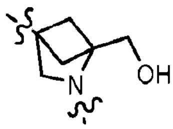 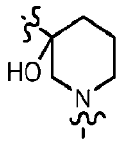

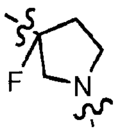 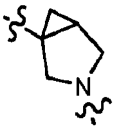 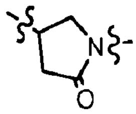 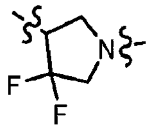 .

Particularly preferably, ring A of the group L comprises

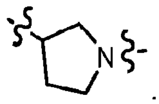 .

In one arrangement, $Q^2$ of the group L is present and comprises any of the following structures:

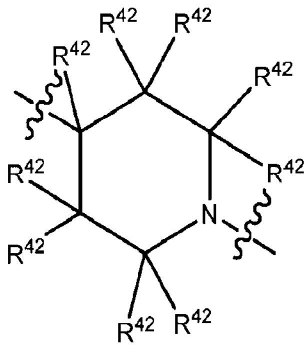 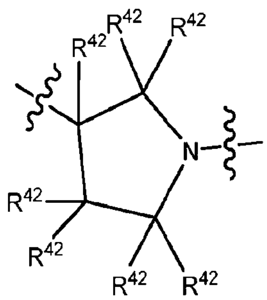 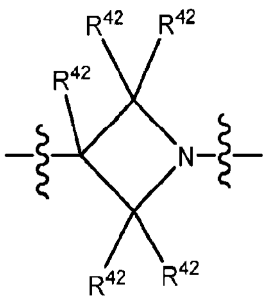

wherein $R^{42}$ is as defined herein.

More preferably, $Q^2$ of the group L comprises any of the following structures:

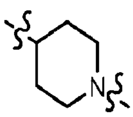 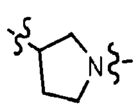 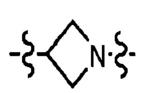 .

Alternatively, group $Q^2$ of the group L is absent and only one $Q^1$ group is present. In this arrangement the $Q^1$ group typically separates ring A from ring C by 3 or 4 atoms in the linear direction and may comprise any of the following structures:

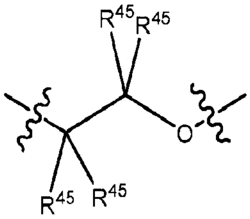
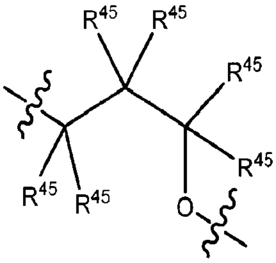
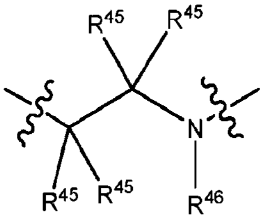
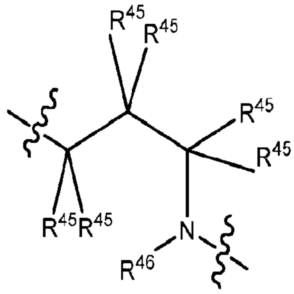

wherein $R^{45}$ and $R^{46}$ are as defined herein.

In this arrangement, the $Q^1$ group preferably comprises any of the following structures:

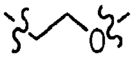
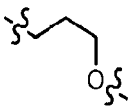
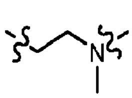
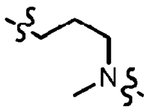

Preferably, ring C of the group L comprises any of the following structures:

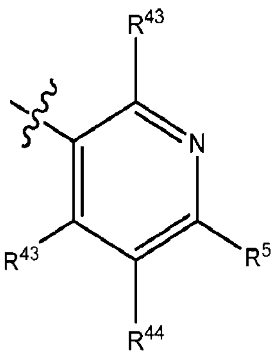
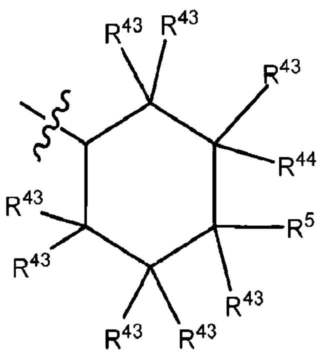
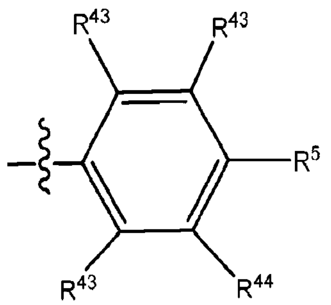
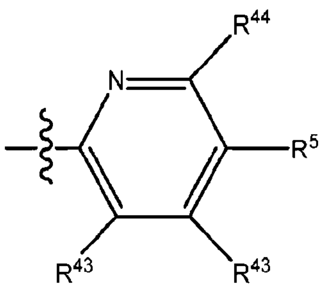
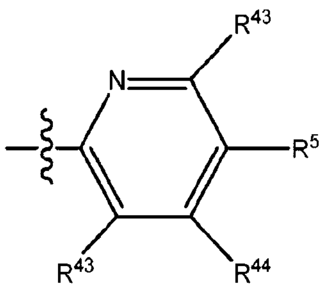
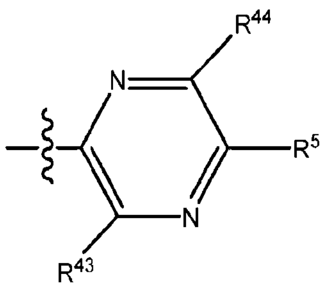
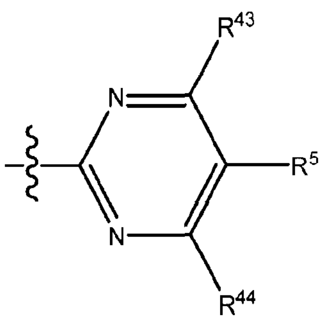
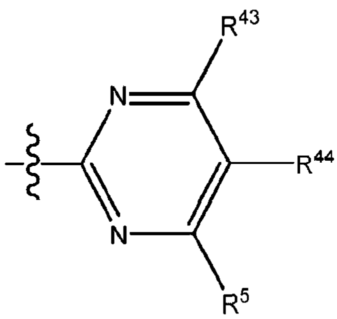
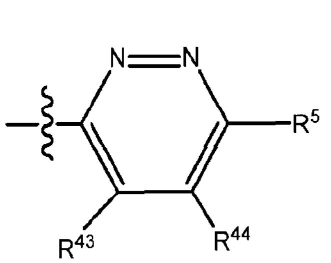

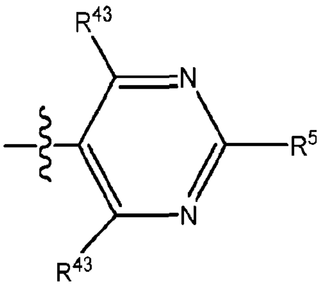
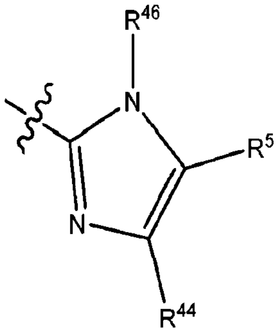
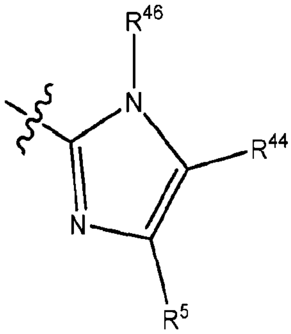
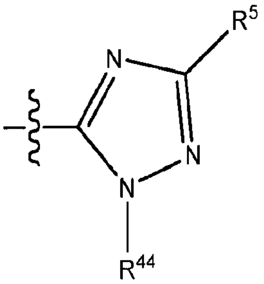
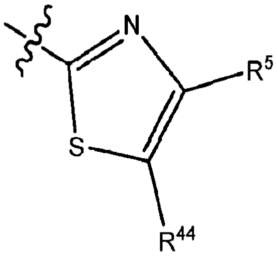
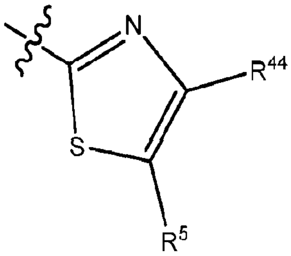
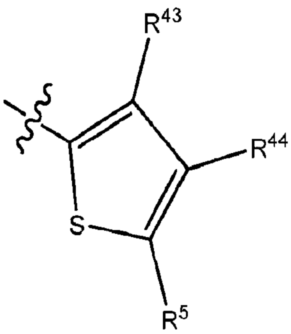
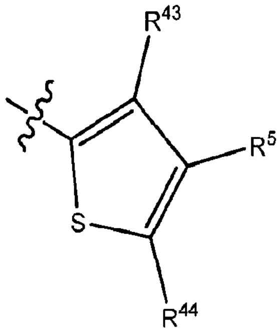
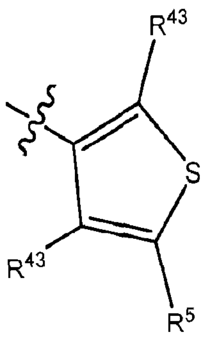
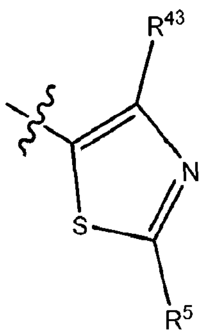

wherein $R^5$, $R^{43}$, $R^{44}$ and $R^{46}$ are as defined herein.

More preferably, ring C of the group L comprises any of the following structures:

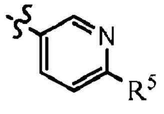
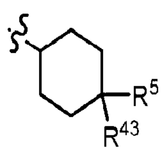
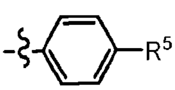
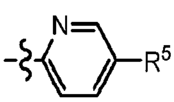
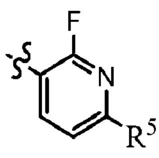
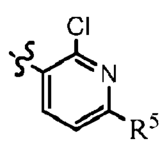

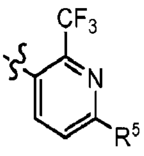 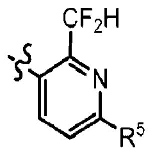 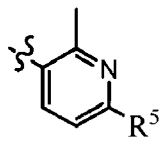 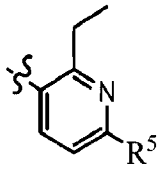 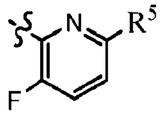 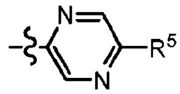

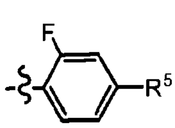 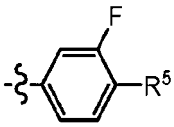

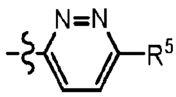 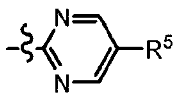 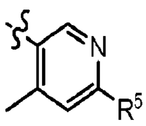 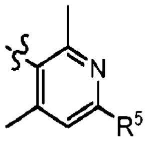

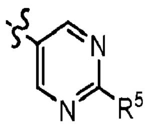 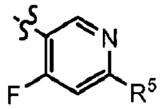 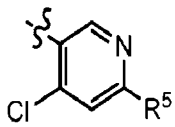 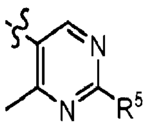

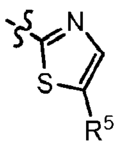 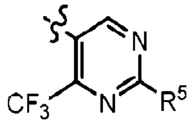 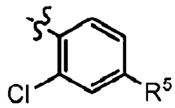 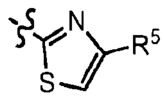 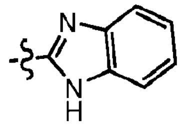

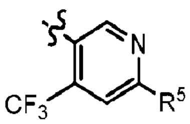 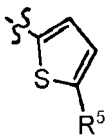 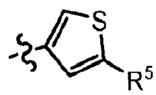 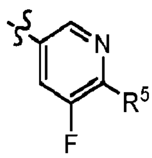 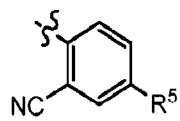

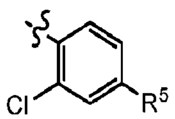 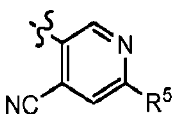 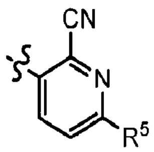 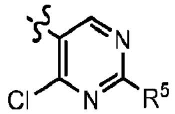 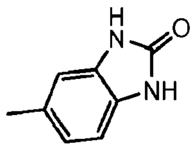 ,

wherein $R^5$ and $R^{43}$ are as defined herein.

In some arrangements, $R^5$ is a substituted or unsubstituted organic group.

Preferably, the group $R^5$ is not MeO, and is more preferably selected from H, -F, -Cl, -Br, -I, - CN, $-CONR^{51}R^{51}$, $-NR^{51}COR^{52}$, $-SO_2NR^{51}R^{51}$, $-NR^{51}SO_2R^{53}$, $-O-CR^{52}R^{52}R^{52}$, $- CR^{52}R^{52}NR^{51}R^{51}$ and any of the following structures:

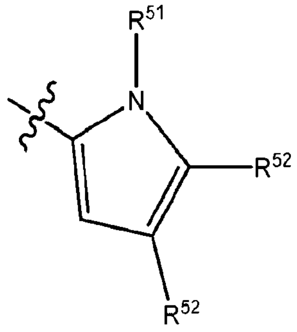 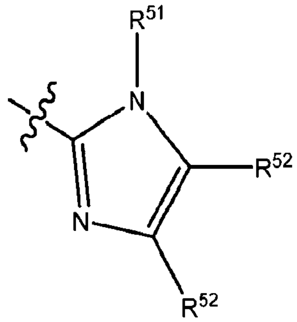 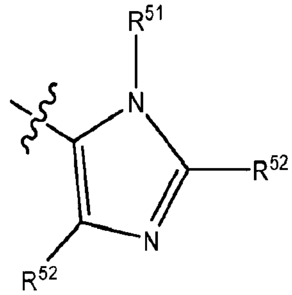

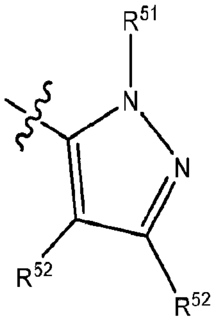 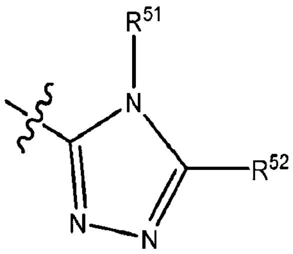 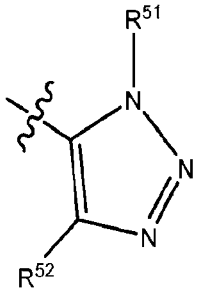

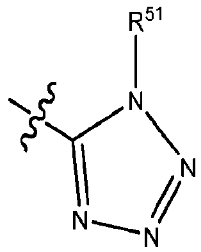 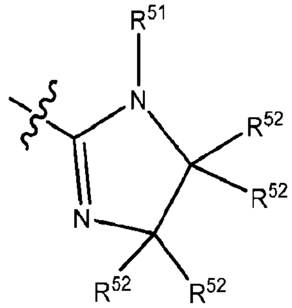

wherein, each of $R^{51}$, $R^{52}$ and $R^{53}$ may be the same or different and are independently selected from H and a substituted or unsubstituted organic group. More preferably, the group $R^5$ is - $CONR^{51}R^{51}$.

Still more preferably, the group $R^5$ is selected from -F, -Cl, -CN, $-CONH_2$, -CONHMe, - CONHEt, $-CONMe_2$, -CONHCOMe, $-CONHCH_2-CH_2OMe$, $-CONH-CH_2-CH_2F$, $-CONH-CH_2-CF_3$, $-CONH-CH_2-CHF_2$, $-OCHF_2$, -NHCOMe, $-NHSO_2Me$, $-SO_2NHMe$, - $CONHSO_2Me$,

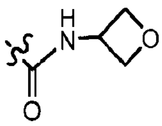 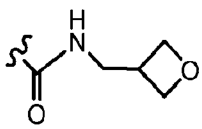 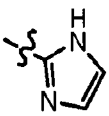 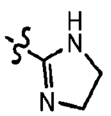 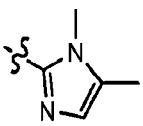 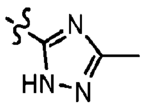

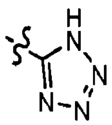 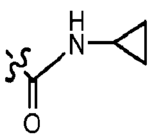 and 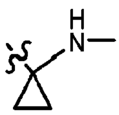 .

A particularly preferred group $R^5$ is -CONHMe, especially where ring C of the group L comprises

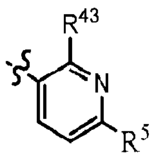,

preferably

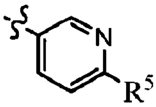.

In some embodiments, the group L of the compound according to the invention comprises a group having any the following structures:

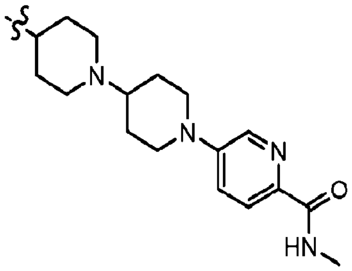

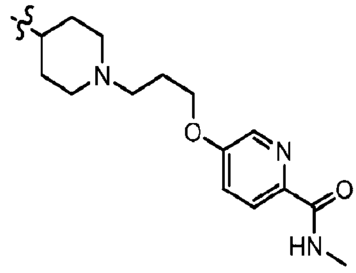

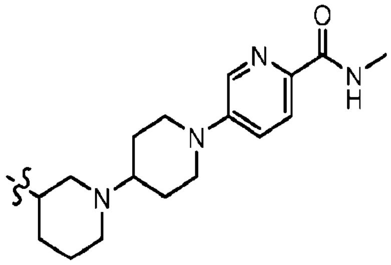

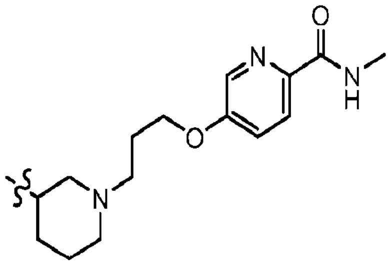

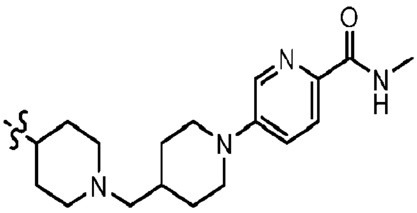

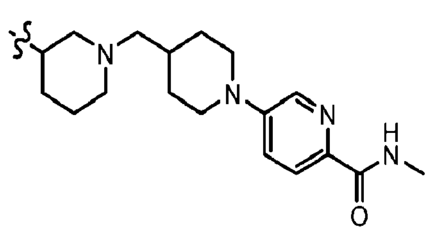

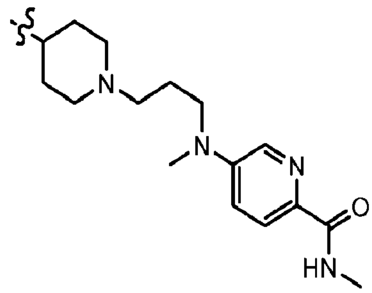

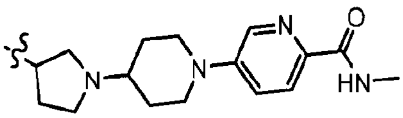

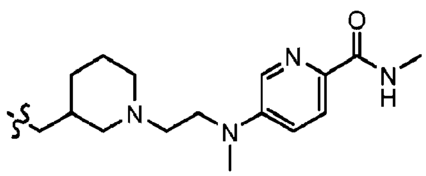

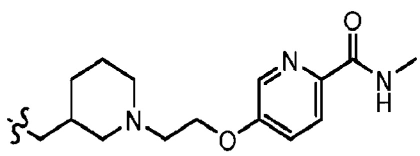

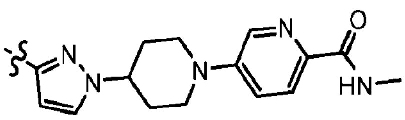

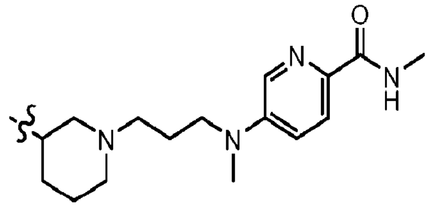

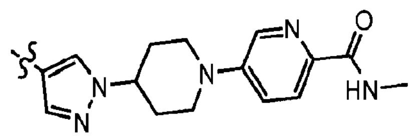

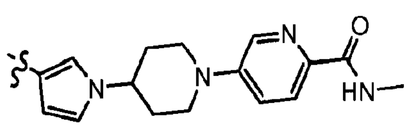

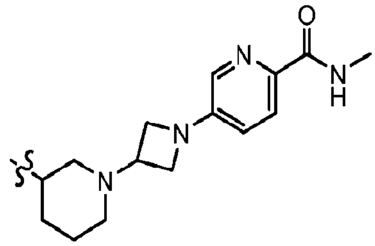

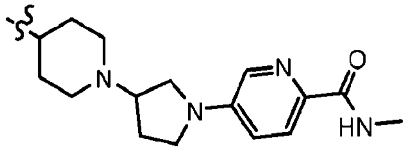

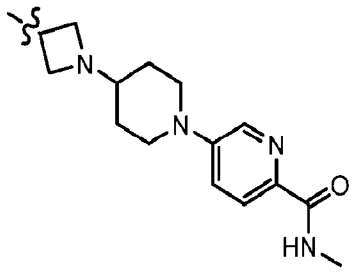

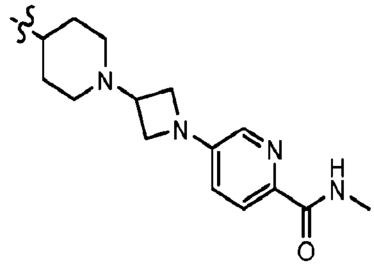

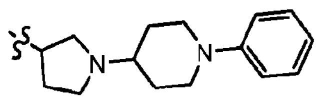

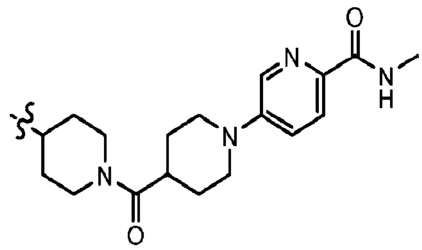

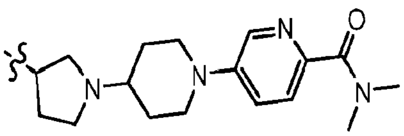

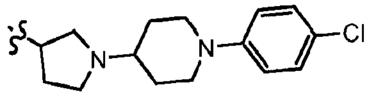

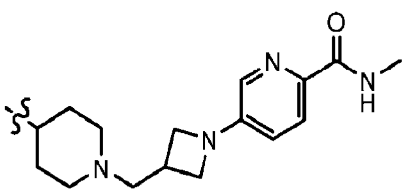

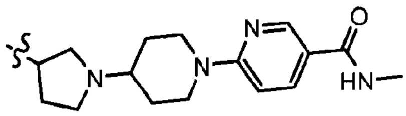

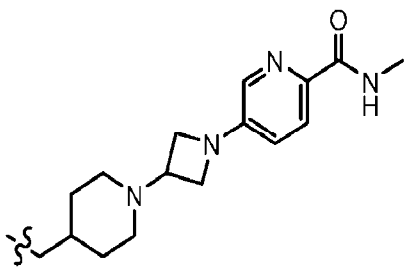

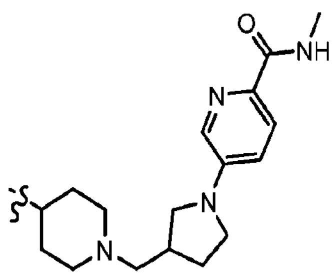

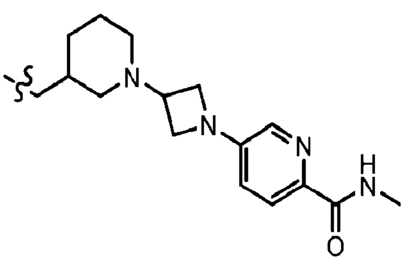

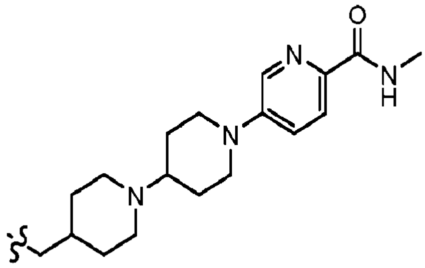

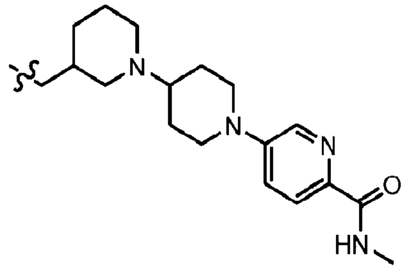

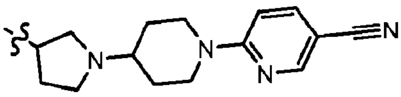

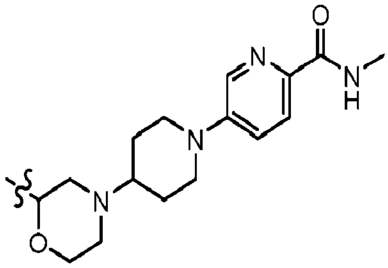

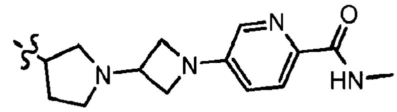

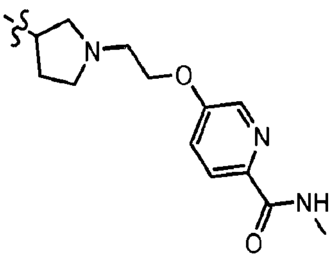

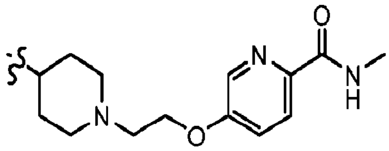

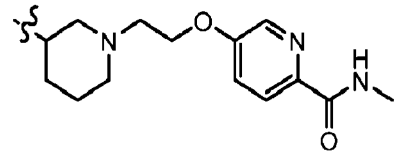

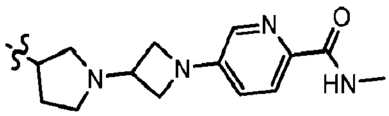

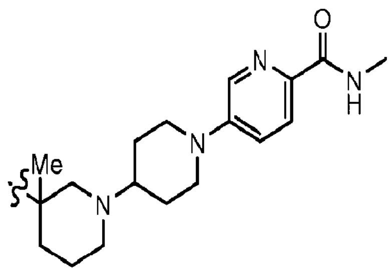

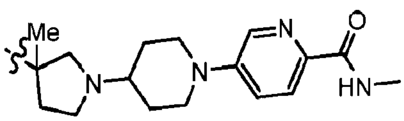

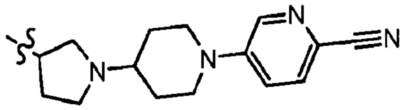

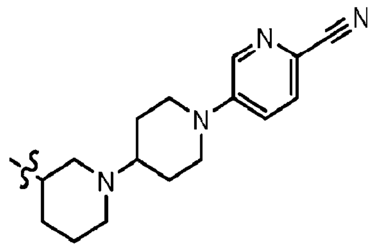

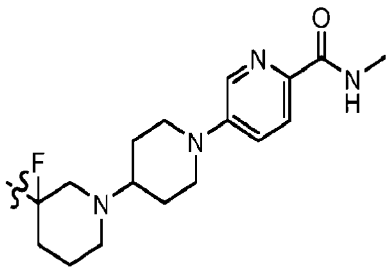

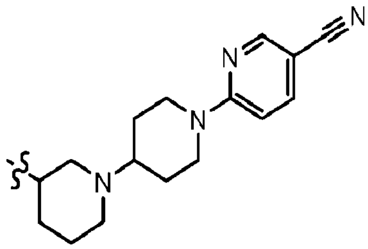

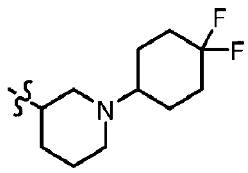

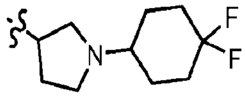

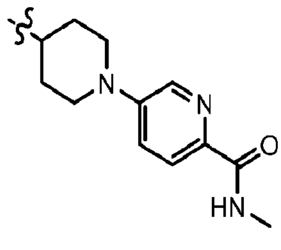

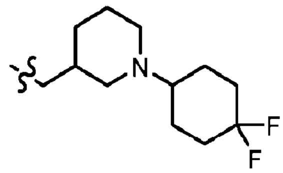

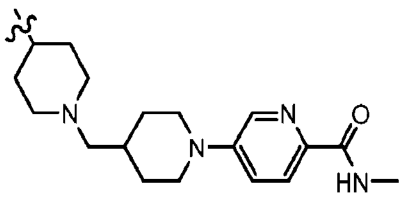

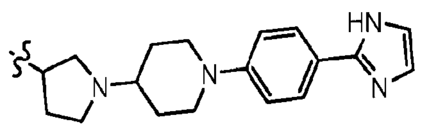

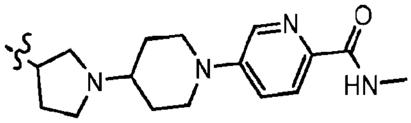

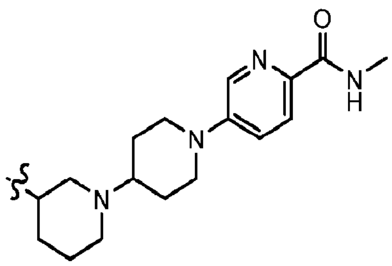

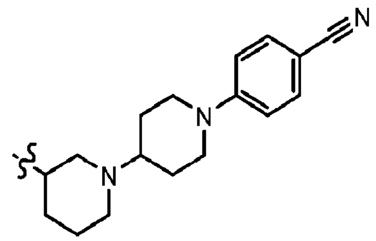

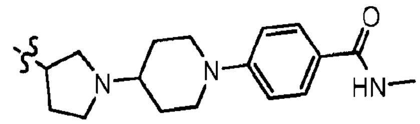

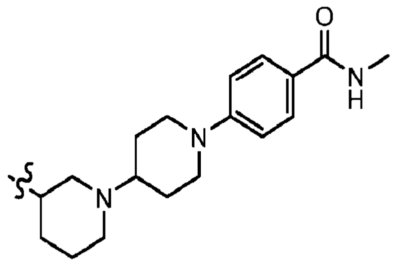

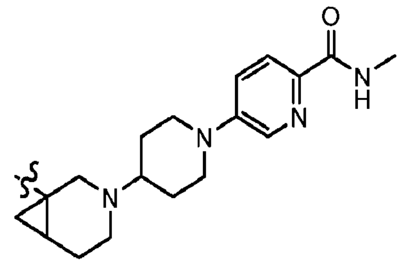

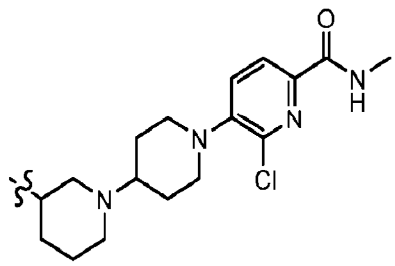

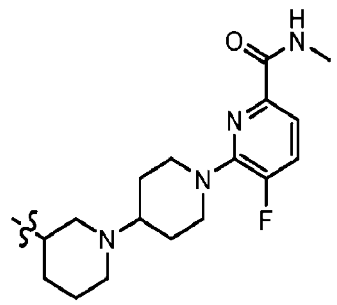

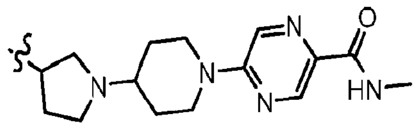

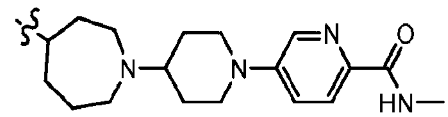

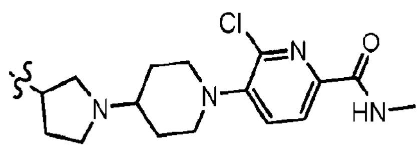

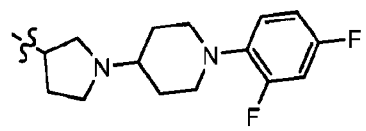

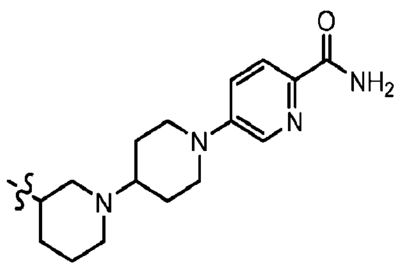

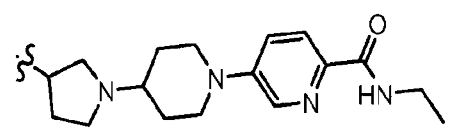

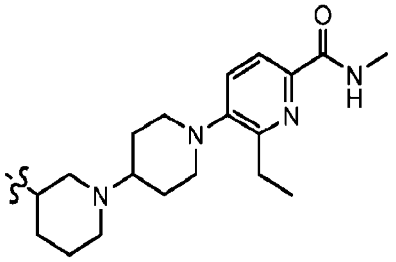

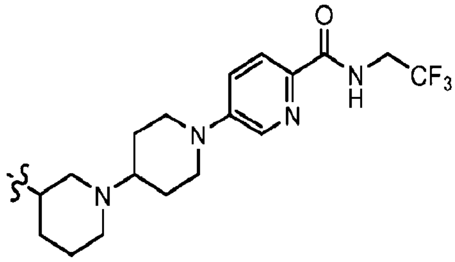

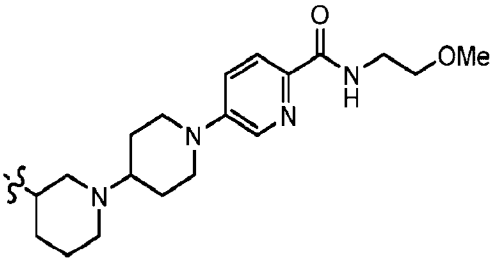

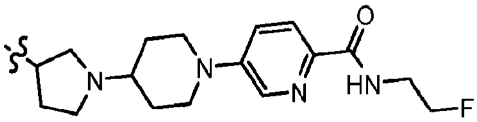

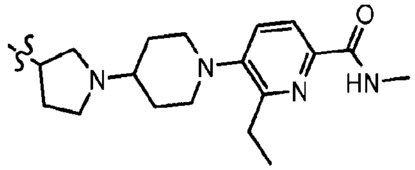

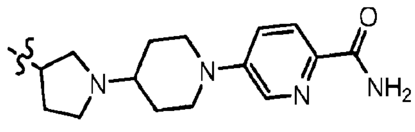

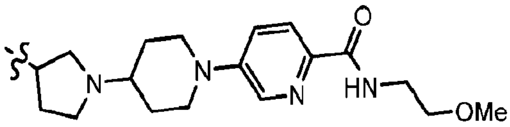

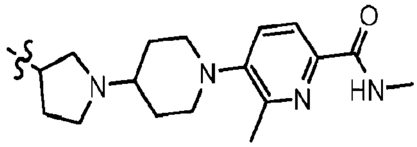

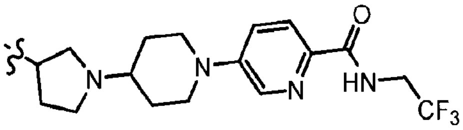

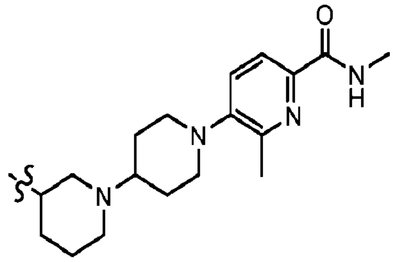

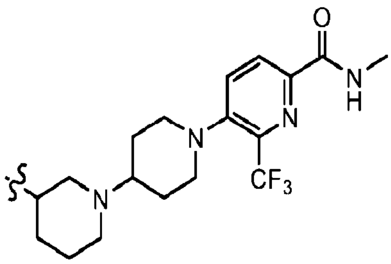

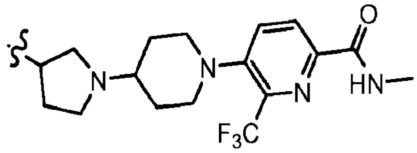

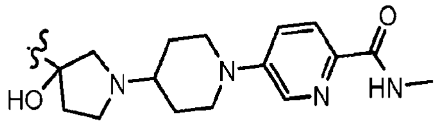

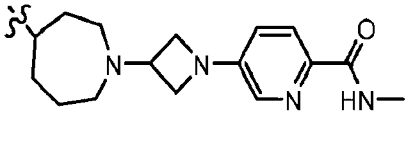

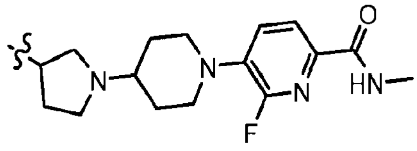

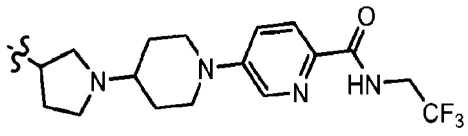

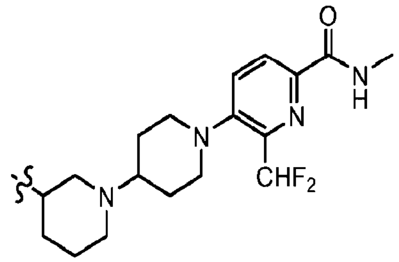

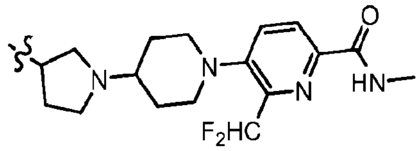

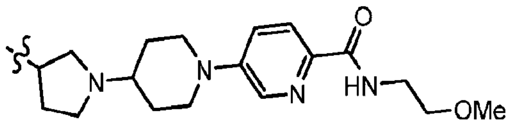

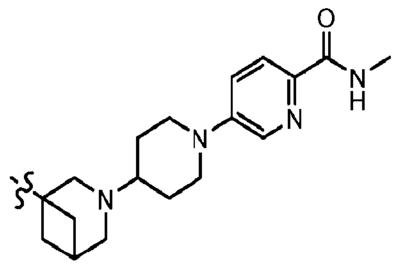

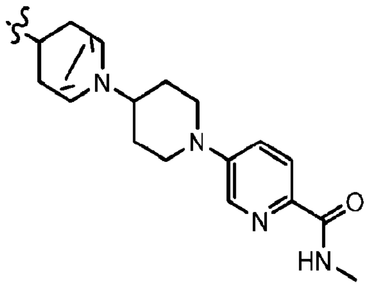

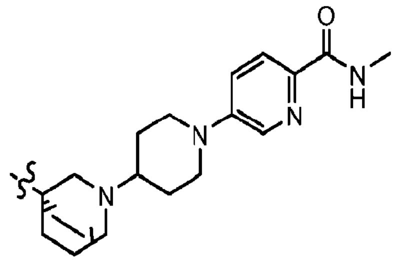

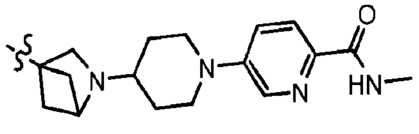

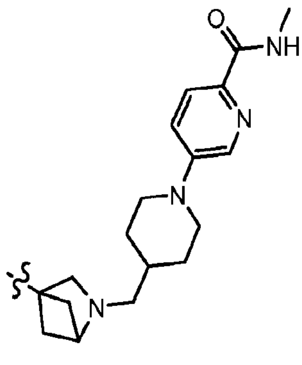

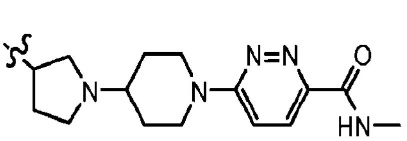

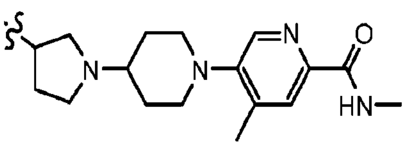

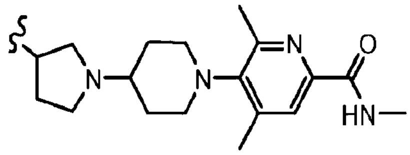

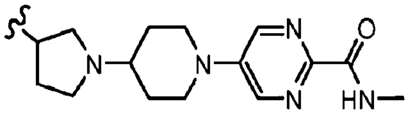

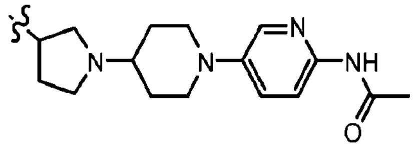

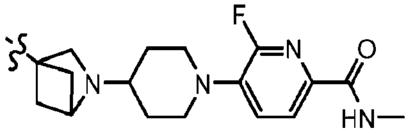

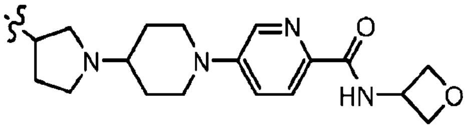

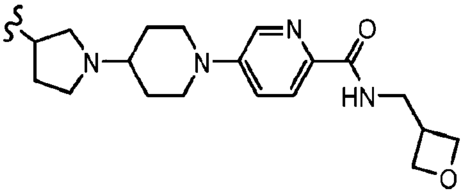

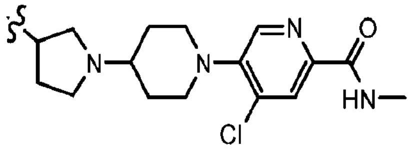

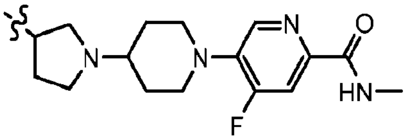

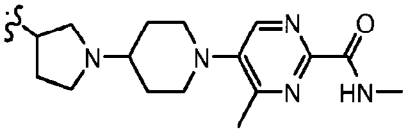

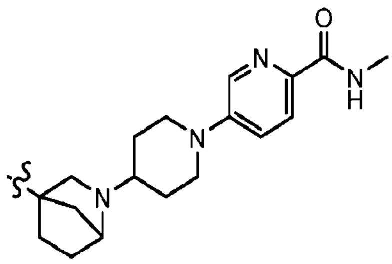

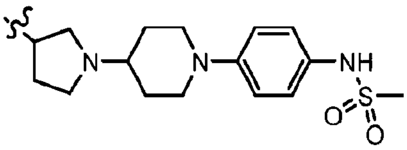

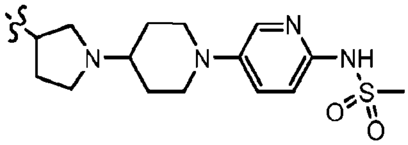

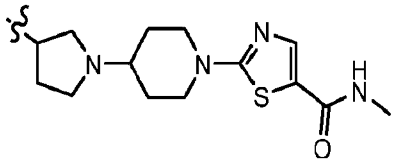

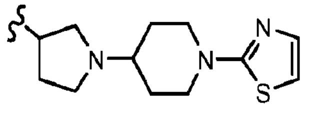

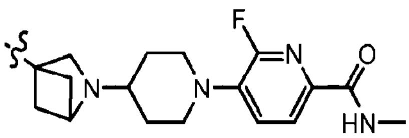

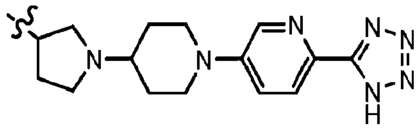

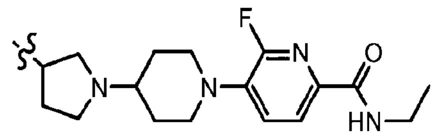
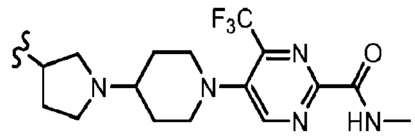
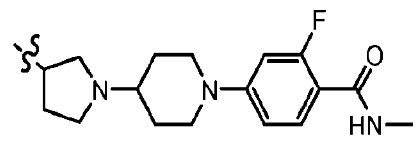
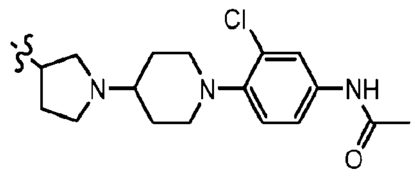
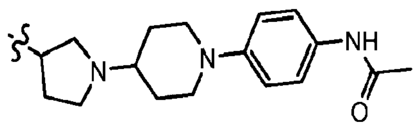
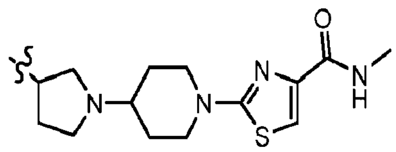
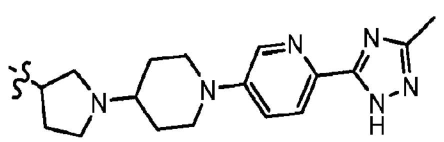
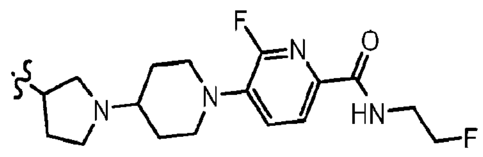
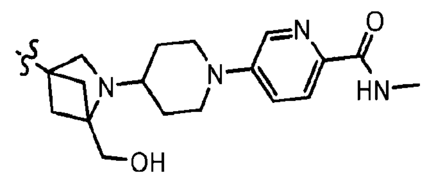
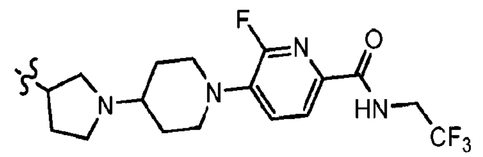
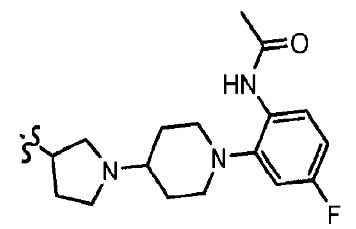
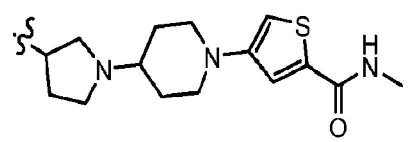
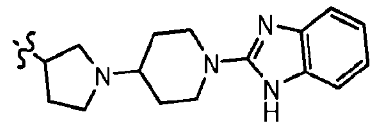
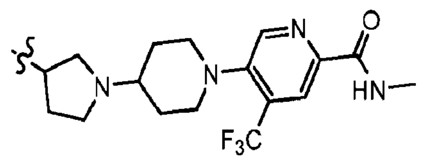
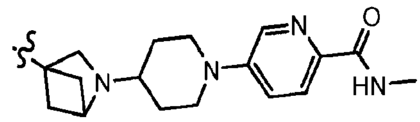
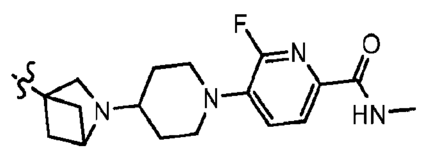
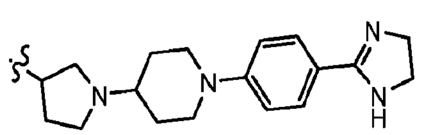
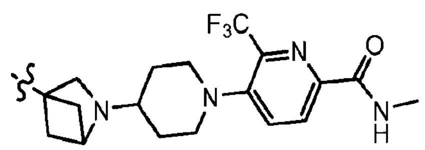

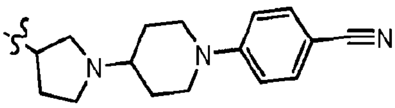

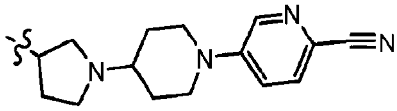

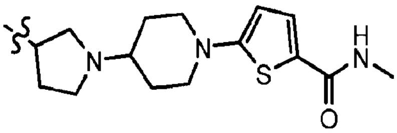

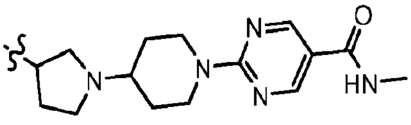

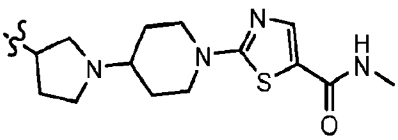

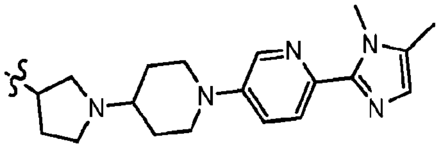

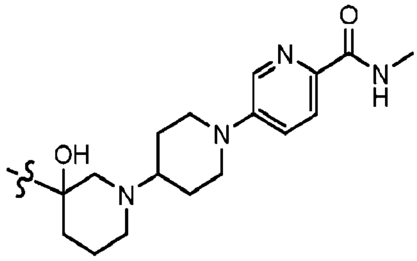

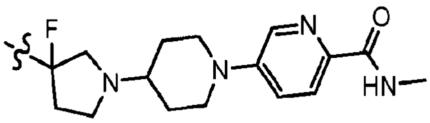

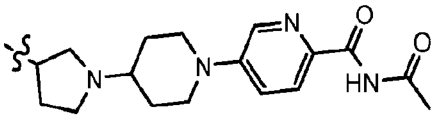

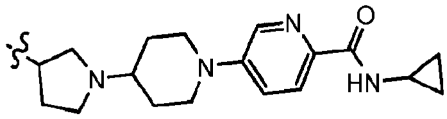

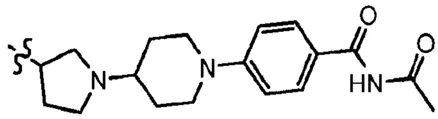

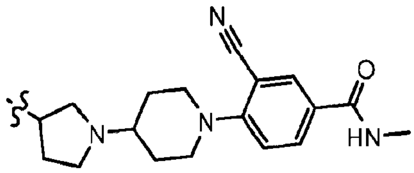

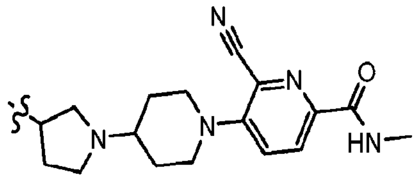

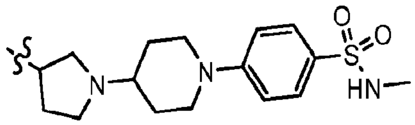

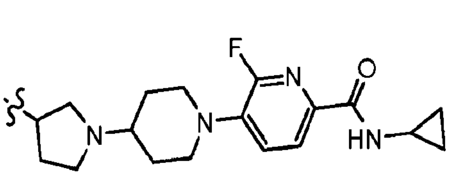

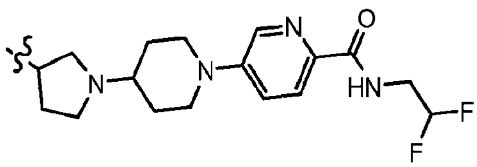

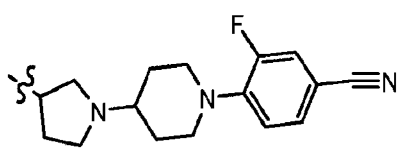

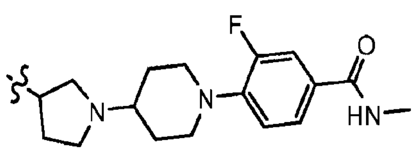

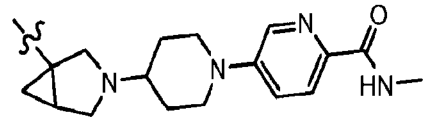

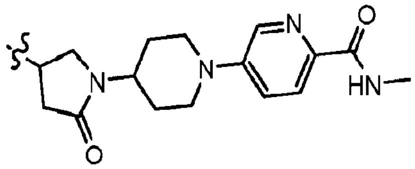

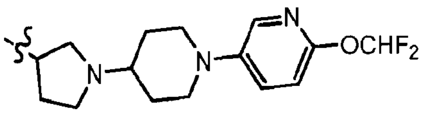

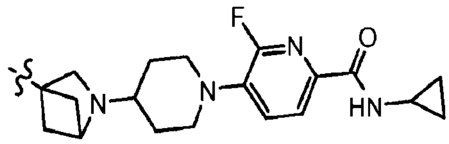

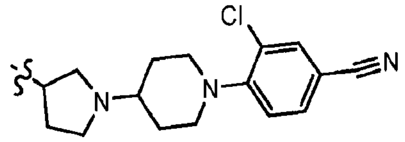

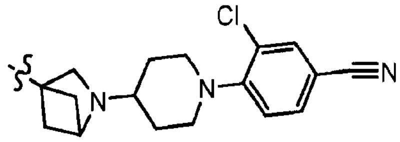

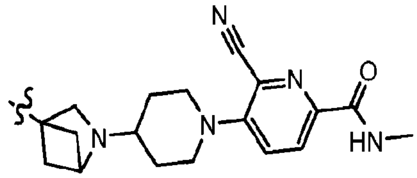

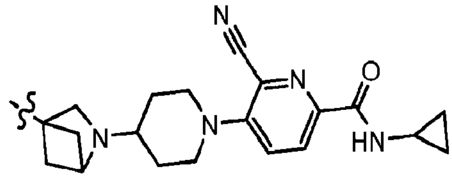

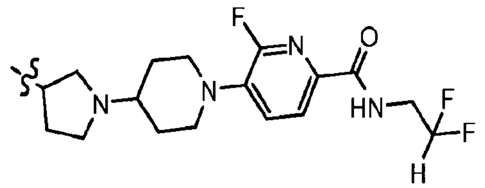

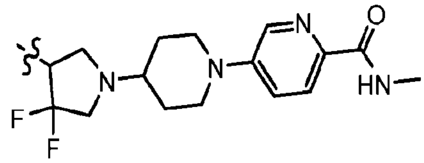

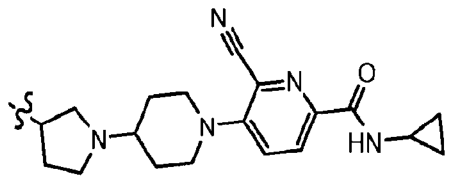

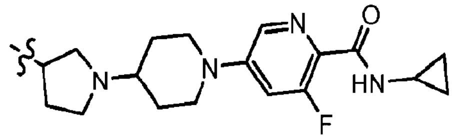

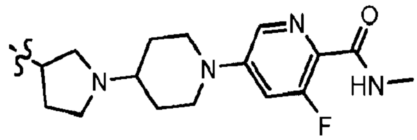

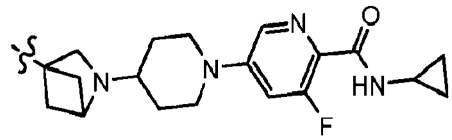

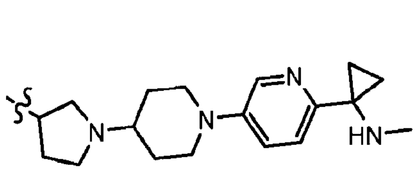

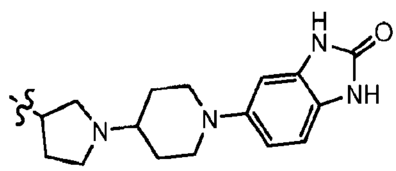

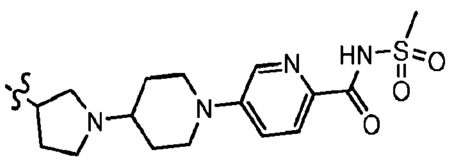

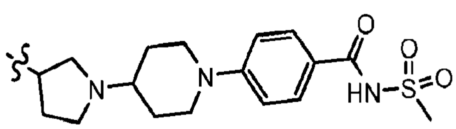

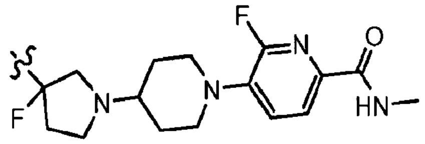

.

In some embodiments, a compound is provided which comprises any of the following structures:

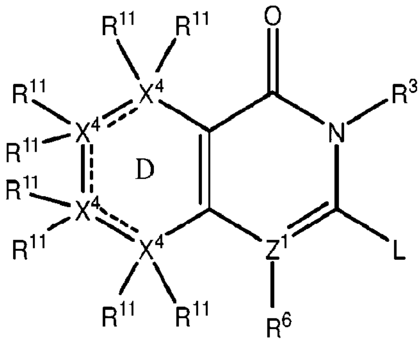

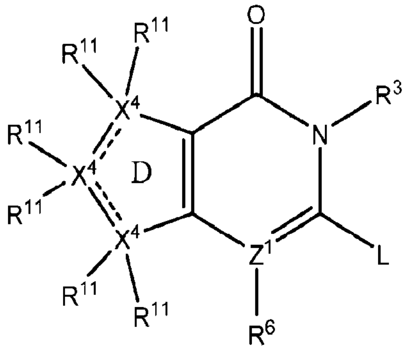

wherein, the dotted lines indicate that the ring D may comprise single bonds, or a combination of both single and double bonds, and may be aliphatic or aromatic; each $X^4$ may be the same or different and is independently selected from C, N, O and S; each $R^{11}$ may be the same or different and is independently selected from H and a substituted or unsubstituted organic group; and wherein $Z^1$, $R^3$, $R^6$, and L are as defined herein. In some embodiments, one $X^4$ of the ring D is N. It is preferred that at least one $X^4$ is C.

Preferably, the compound comprises any of the following structures:

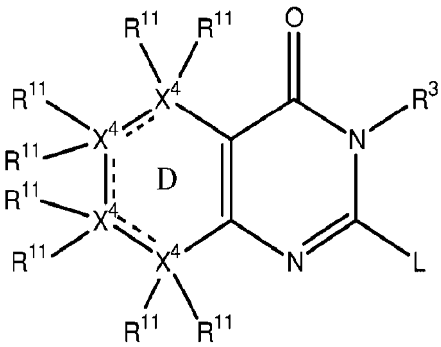

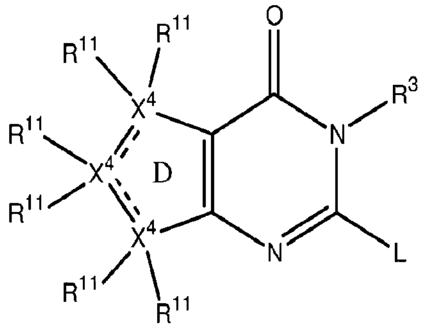

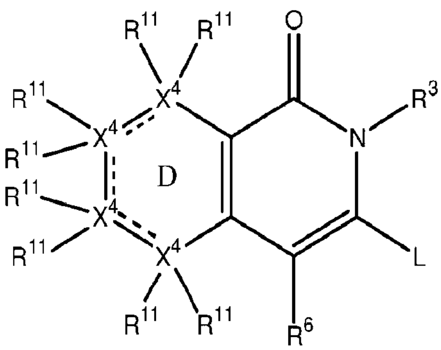

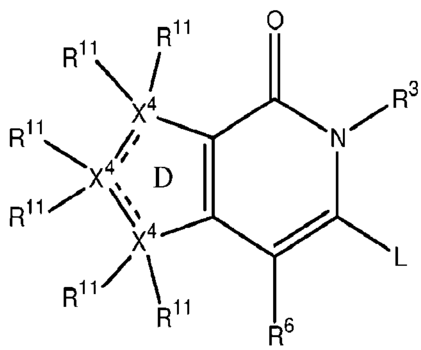

wherein the ring D, $X^4$, $R^{11}$, $R^3$, $R^6$, and L are as defined herein.

More preferably, the compound comprises any of the following structures:

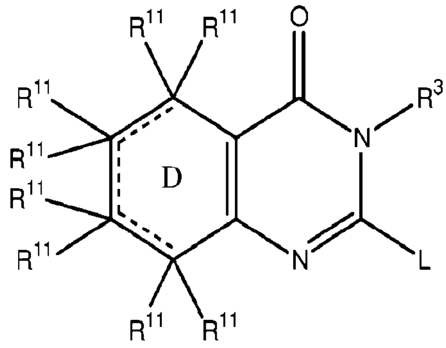

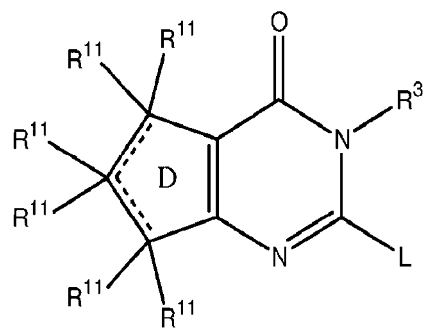

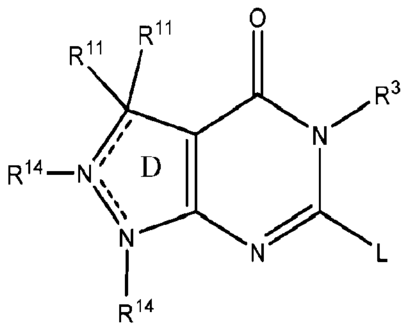

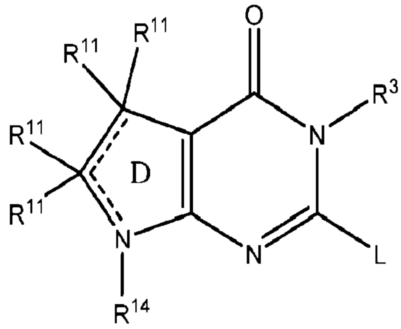

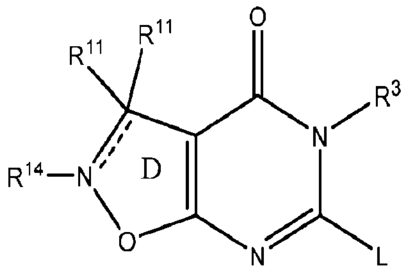

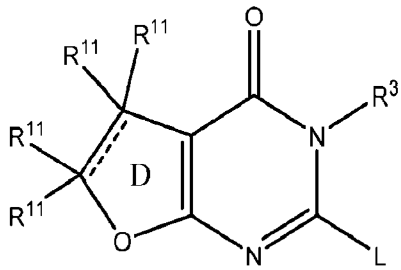

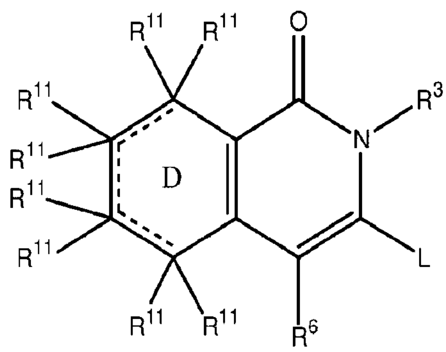

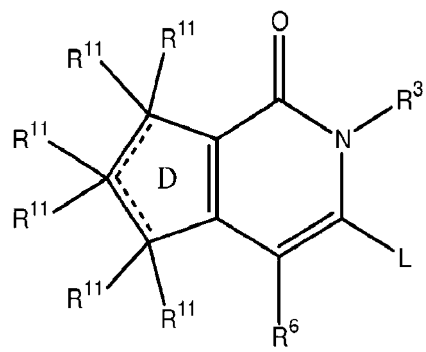

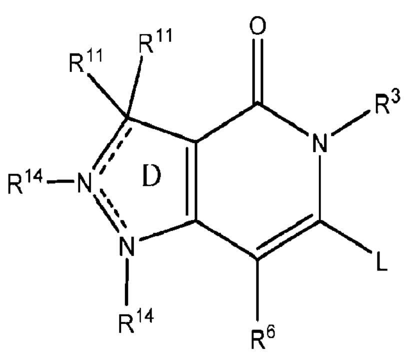

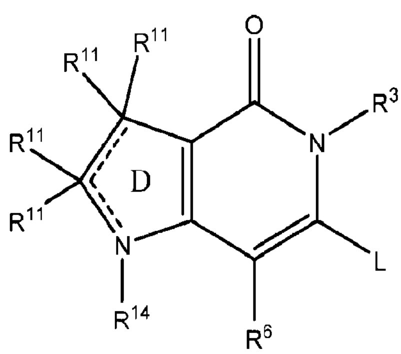

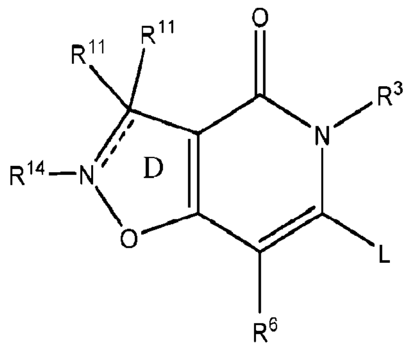

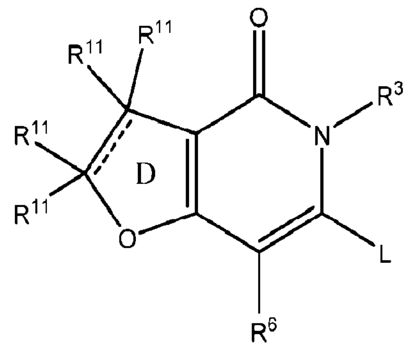

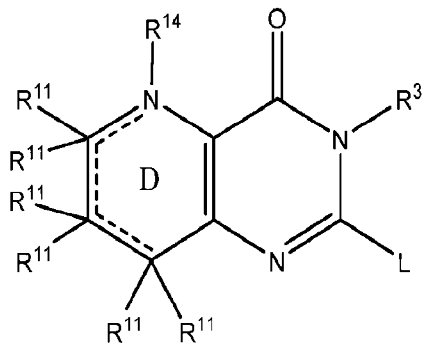

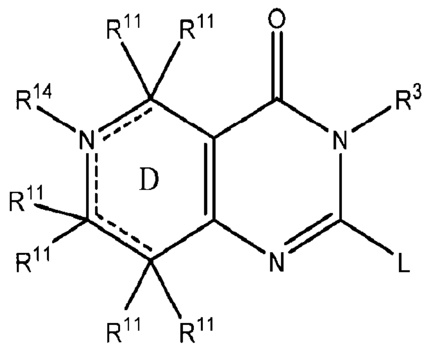

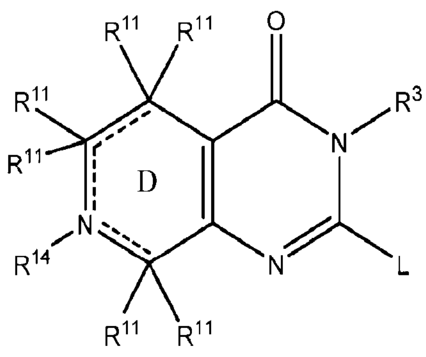

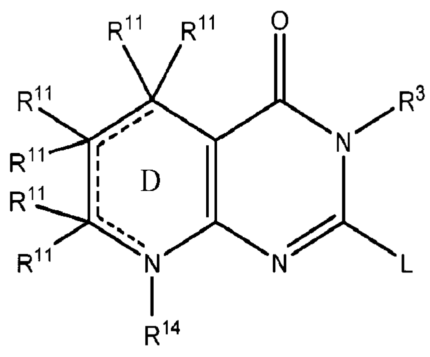

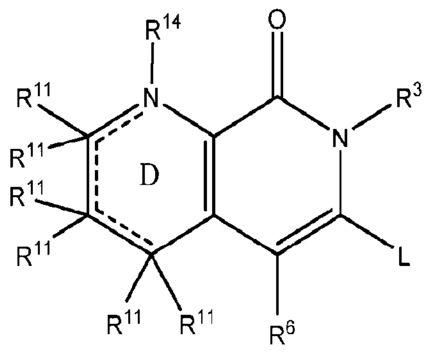

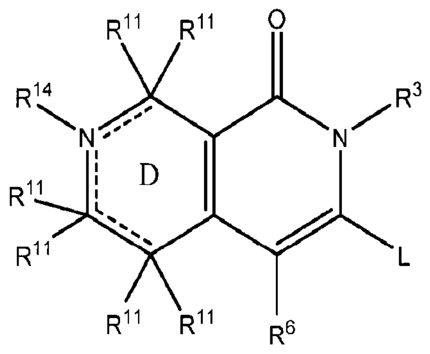

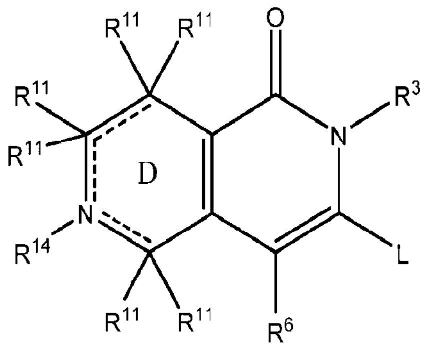

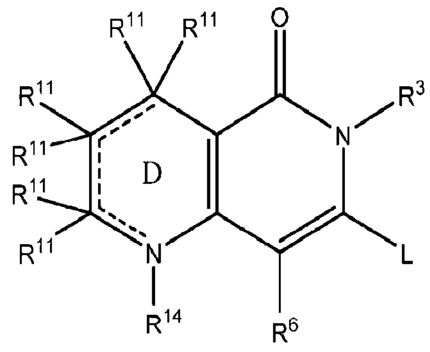

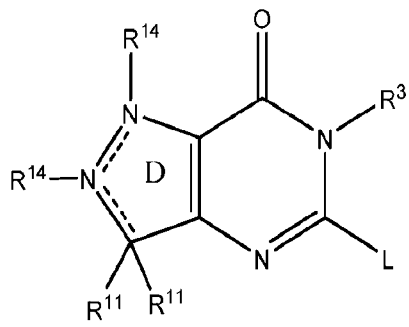

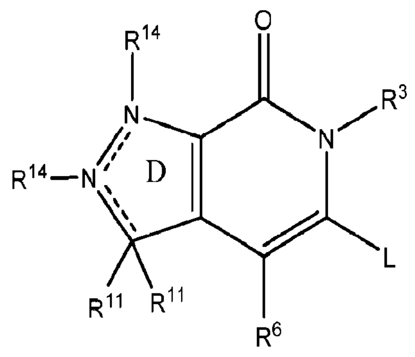

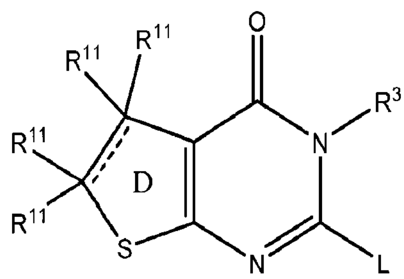

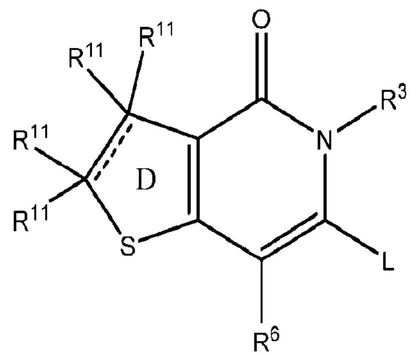

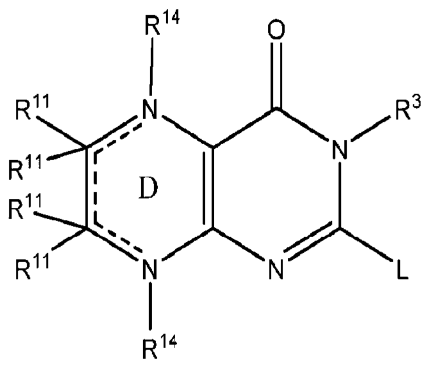

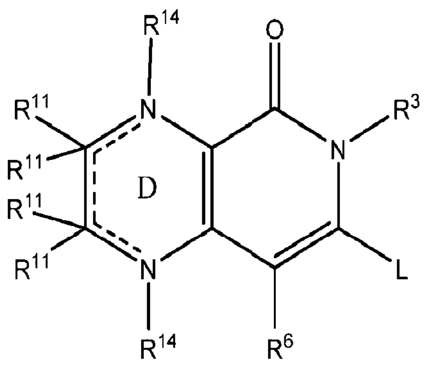

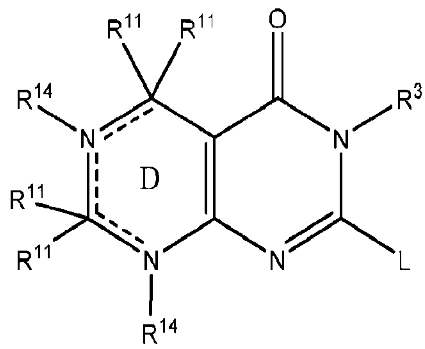

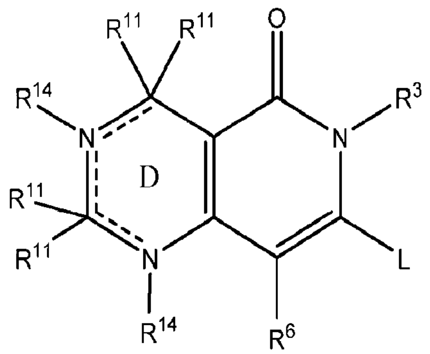

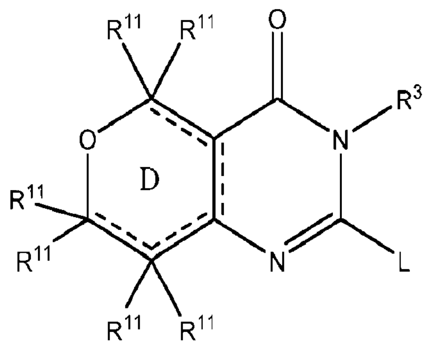

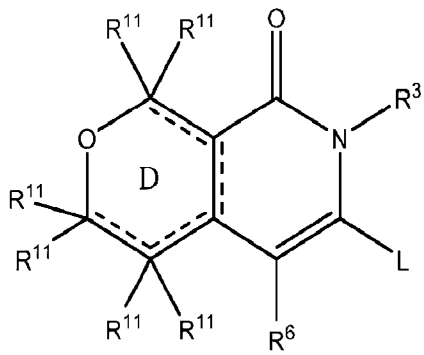

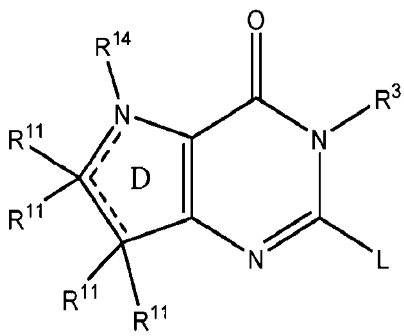

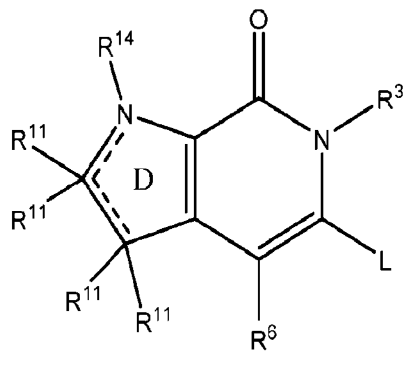

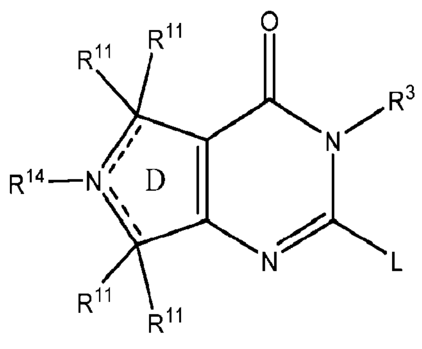

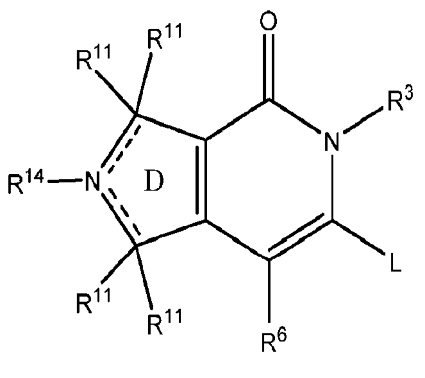

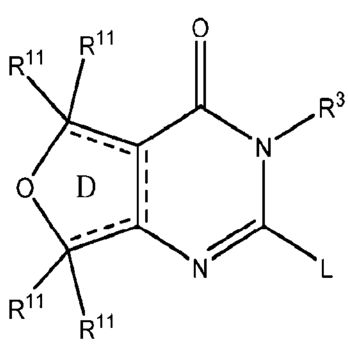

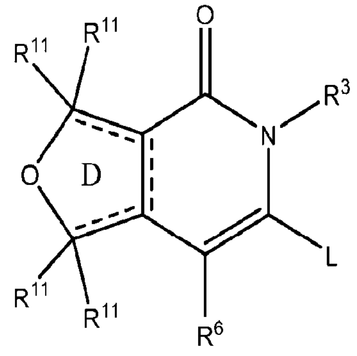

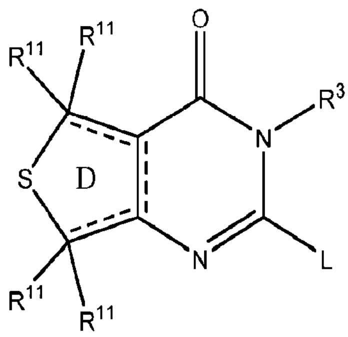

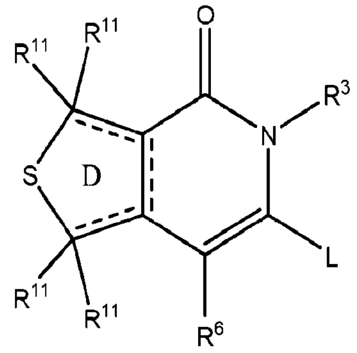

wherein $R^{14}$ is selected from H, $C_1$ to $C_3$ alkyl or $C_1$ to $C_3$ fluoroalkyl; and wherein $R^{11}$, ring D, $R^3$, $R^6$, and L are as defined herein.

Still more preferably, the compound comprises any of the following structures:

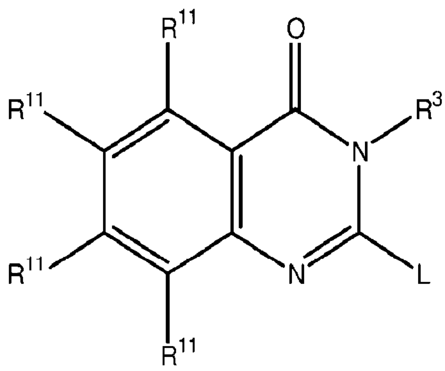

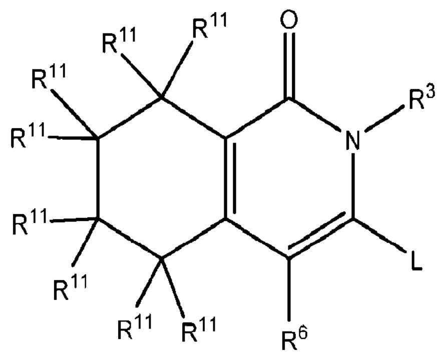

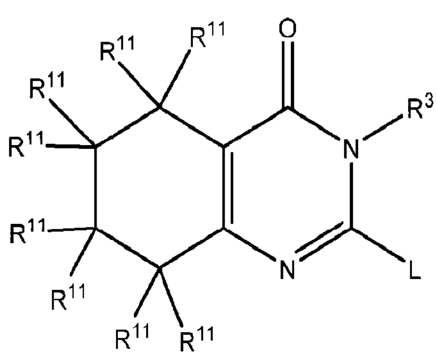

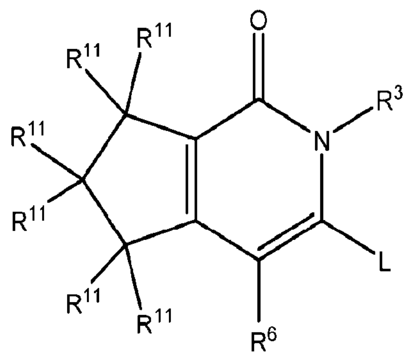

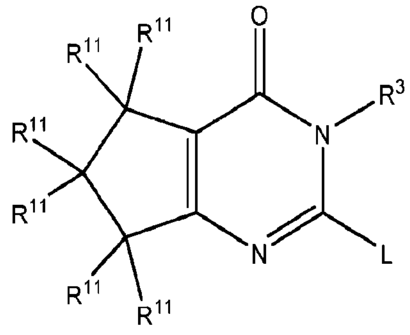

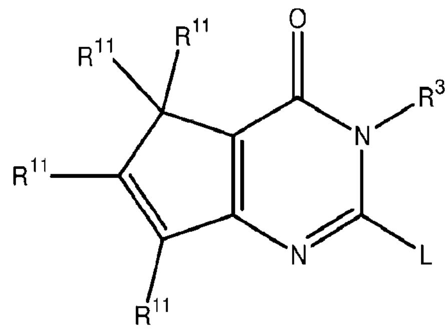

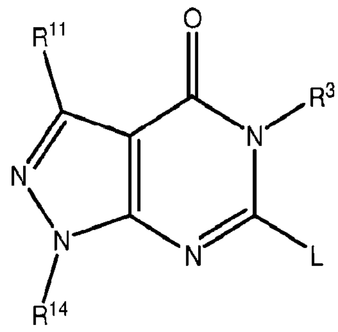

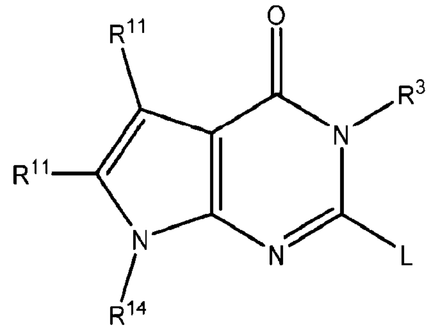

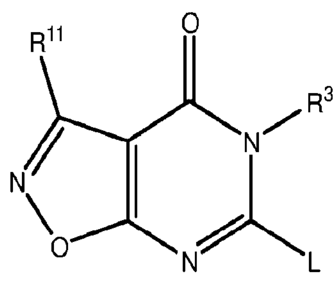

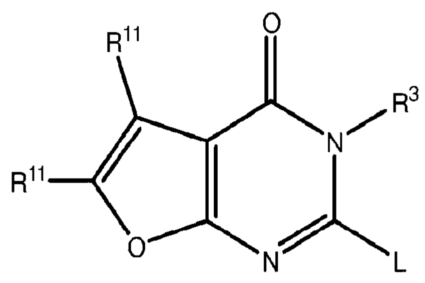

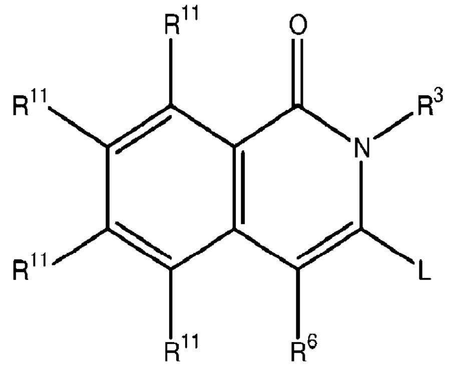

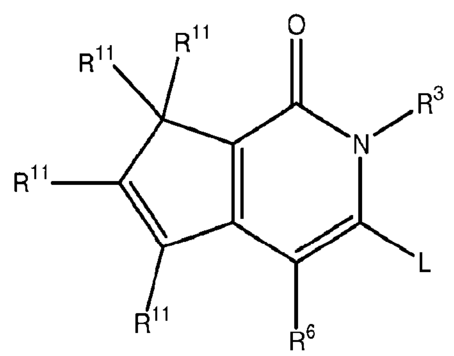

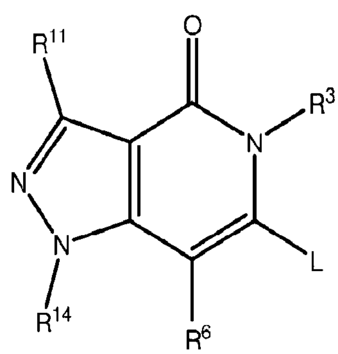

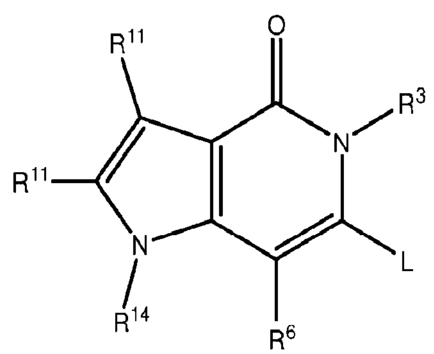

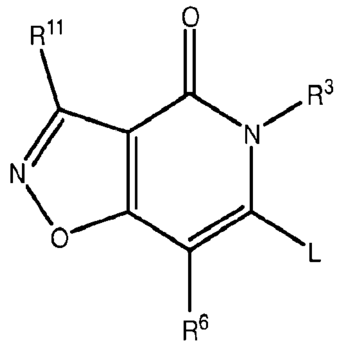

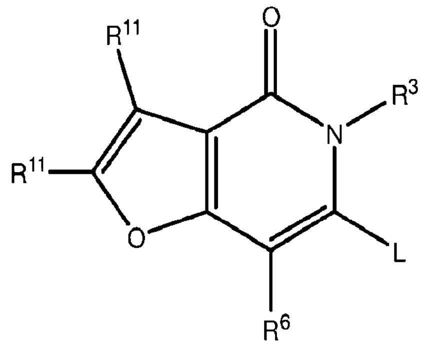

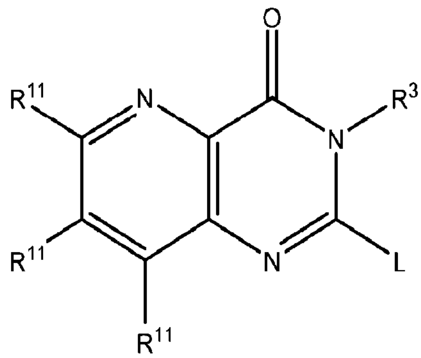

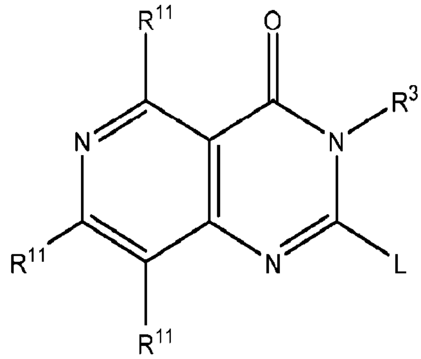

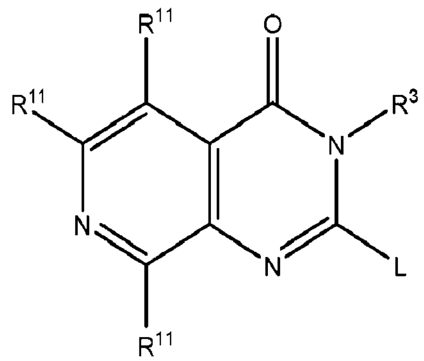

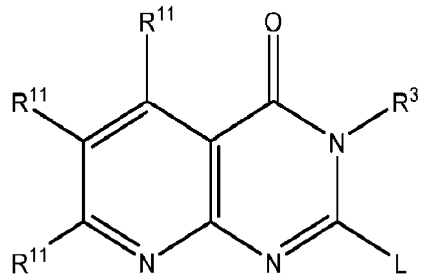

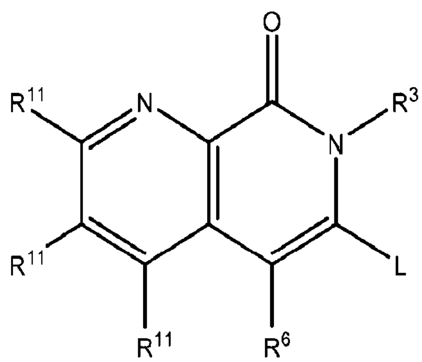

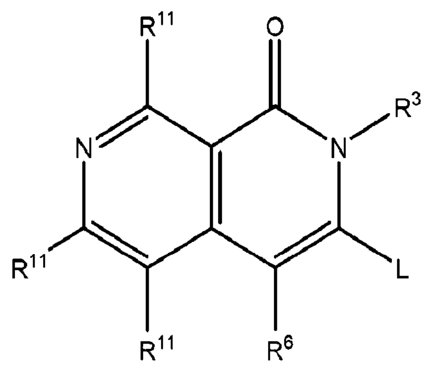

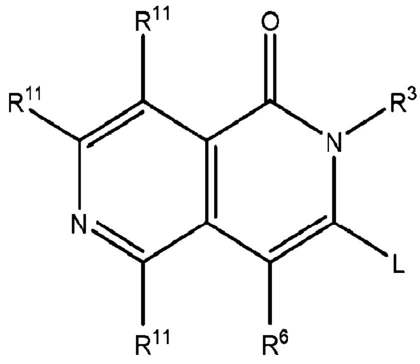

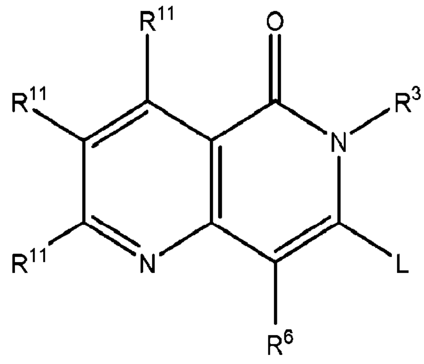

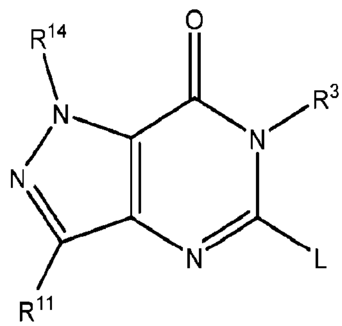

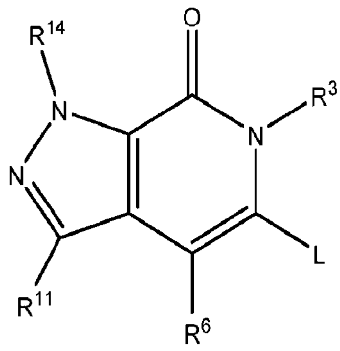

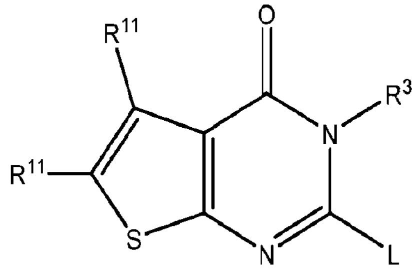

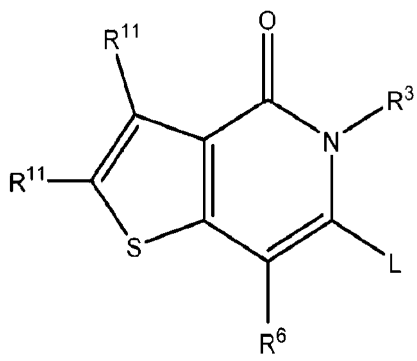

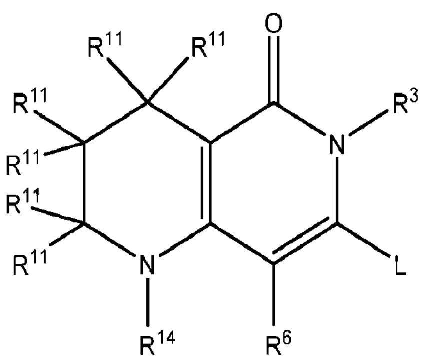

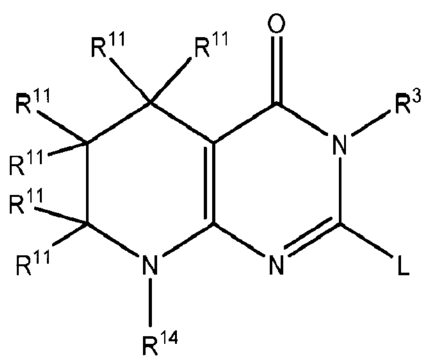

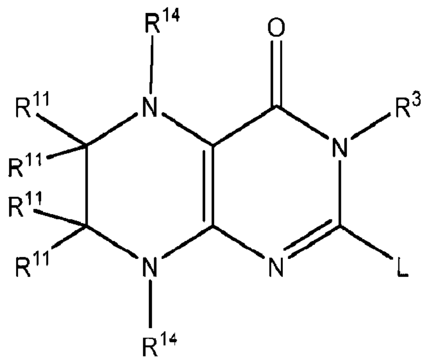

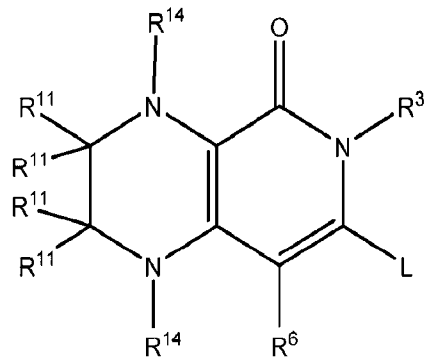

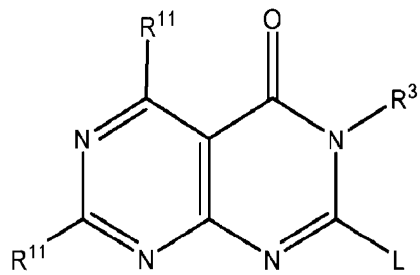

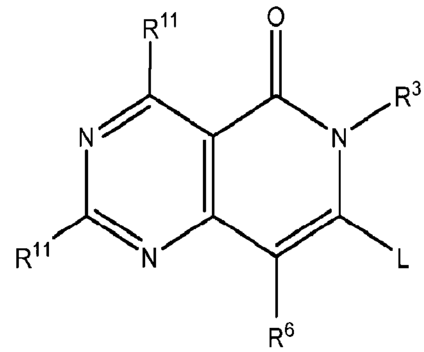

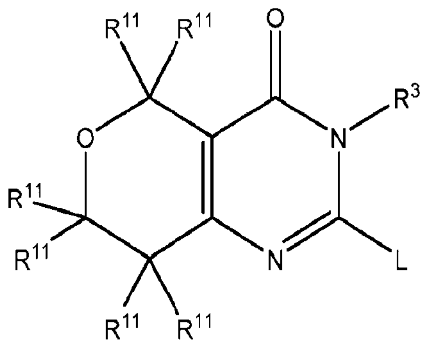

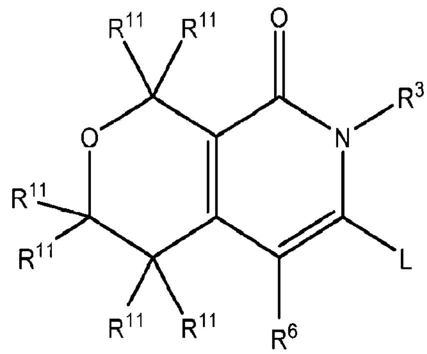

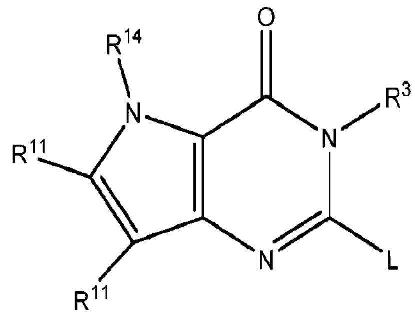

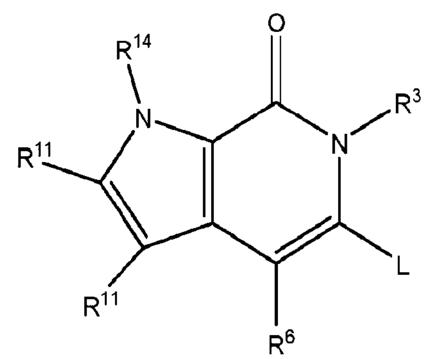

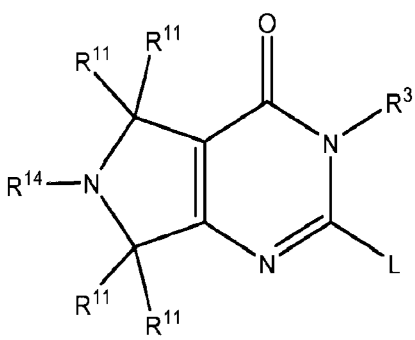

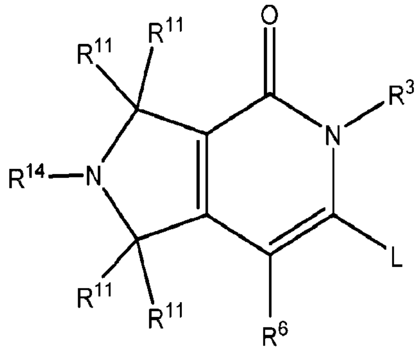

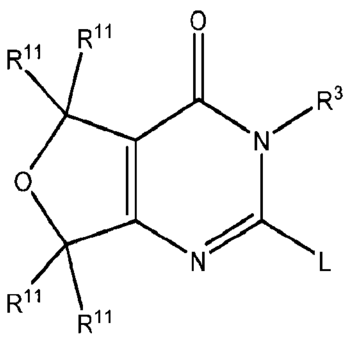

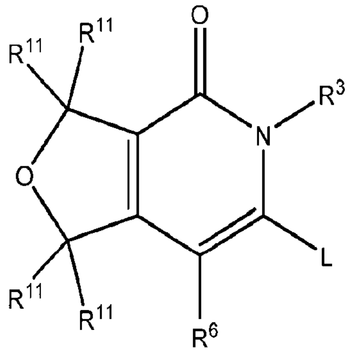

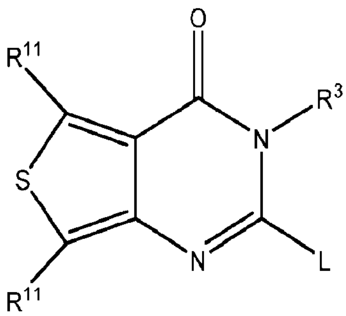

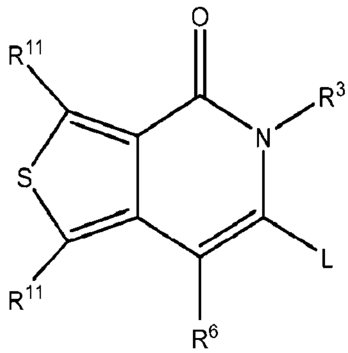

wherein $R^3$, $R^{11}$, $R^{14}$, $R^6$, and L are as defined herein.

Each $R^{11}$ may independently be selected from H, a halogen, a nitrile group, a linear or branched $C_1$-$C_3$ alkyl group, a linear or branched $C_1$-$C_3$ halogenated alkyl group (preferably fluoroalkyl), an -OH group, a linear or branched $C_1$-$C_3$ alcohol group, a halogenated (preferably fluoro-) linear or branched $C_1$-$C_3$ alcohol group, $-NH_2$, a linear or branched primary secondary or tertiary $C_1$-$C_3$ amine group, a halogenated (preferably fluoro-) linear or branched primary, secondary or tertiary $C_1$-$C_3$ amine group, a linear or branched $C_1$-$C_3$ alkoxy group, a linear or branched $C_1$-$C_3$ halogenated alkoxy group (preferably fluoroalkoxy), and/or a pair of $R^{11}$ groups attached to the same atom forming =O; and independently, and/or when a pair of $R^{11}$ groups attached to different atoms together forms a ring with ring D atoms, the pair of $R^{11}$ groups comprises $-CH_2CH_2CH_2-$.

It is preferred that each $R^{11}$ is independently selected from H, Cl, F, $CHF_2$, $CF_3$, $CH_3$, OH, $CH_3O$, and $NH_2$, and/or a pair of $R^{11}$ groups attached to the same atom forming =O; and independently, when a pair of $R^{11}$ groups attached to different atoms together forms a ring with ring D atoms, the pair of $R^{11}$ groups comprises $-CH_2CH_2CH_2-$.

In some embodiments, a compound is provided which comprises any of the following structures:

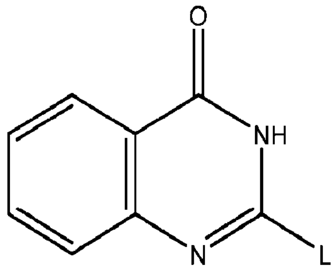

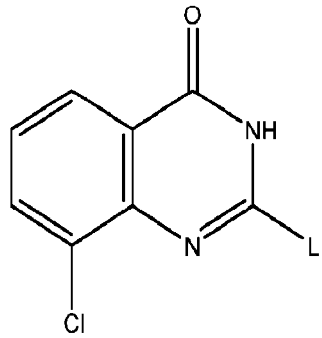

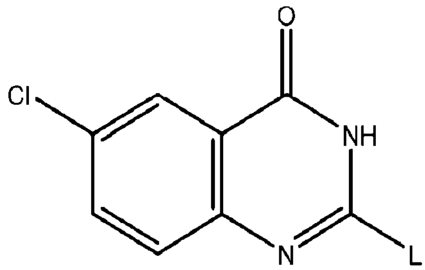

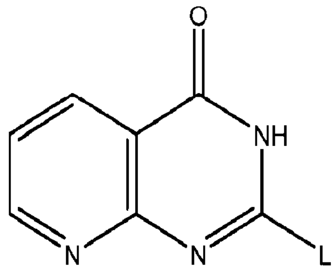

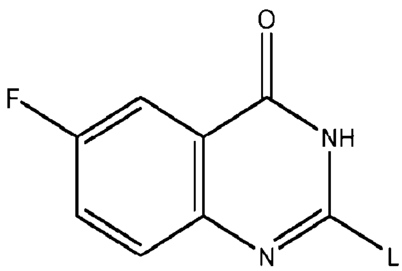

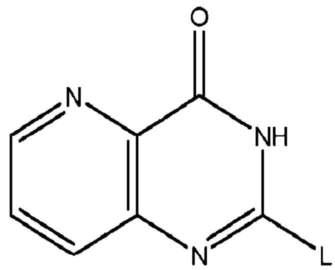

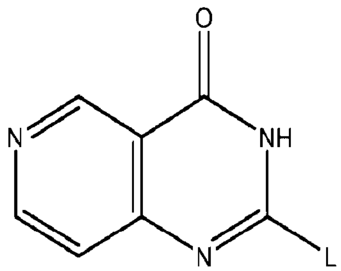

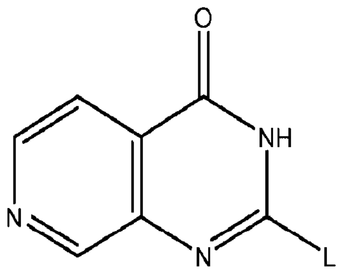

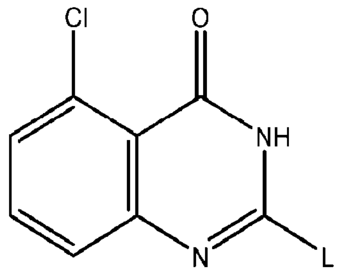

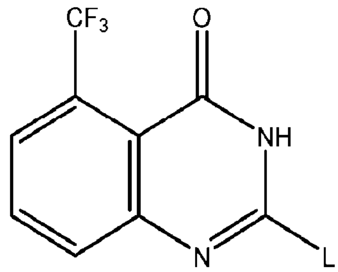

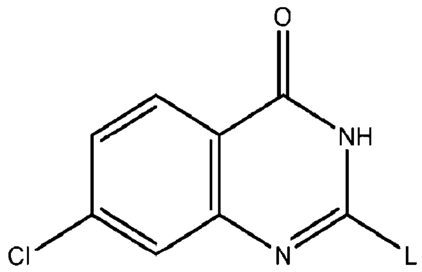
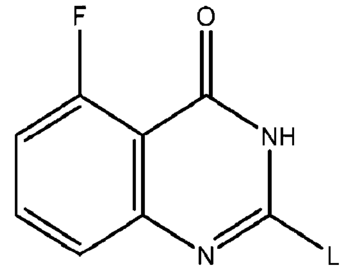
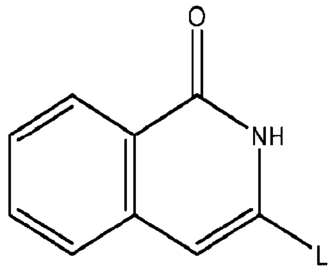
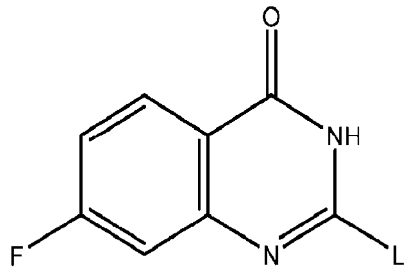
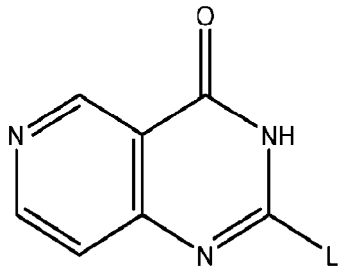
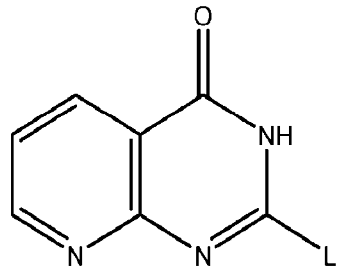
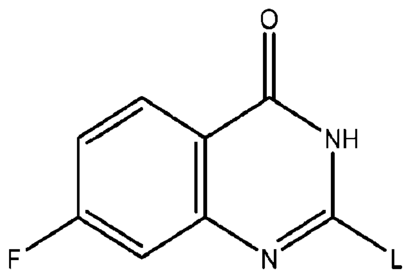
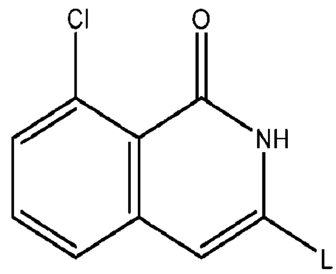
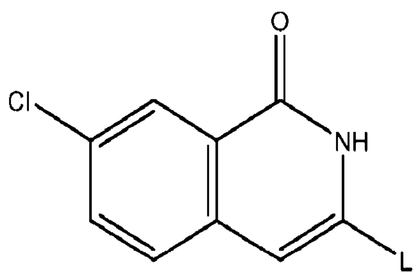
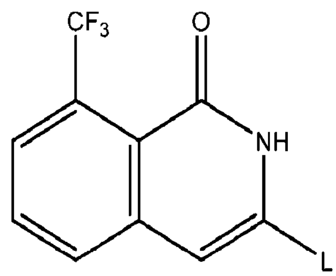

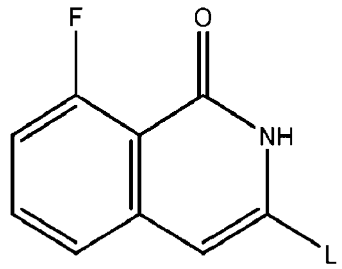

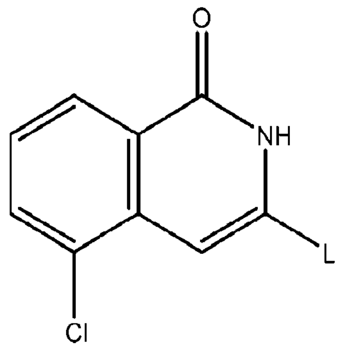

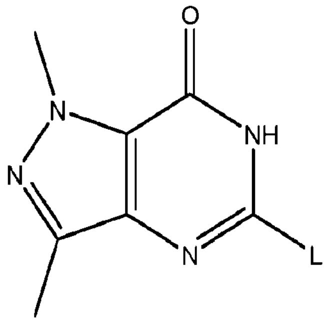

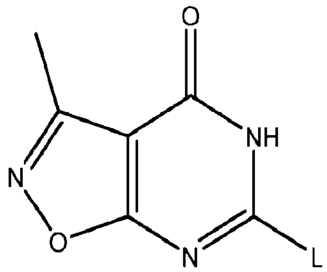

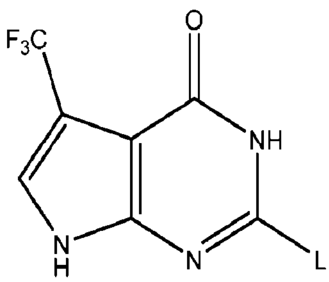

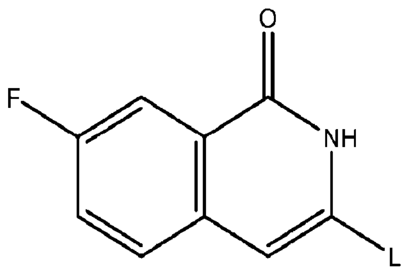

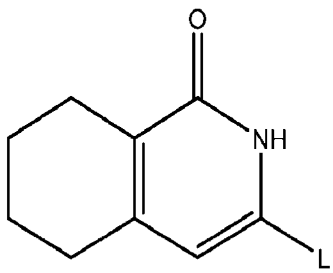

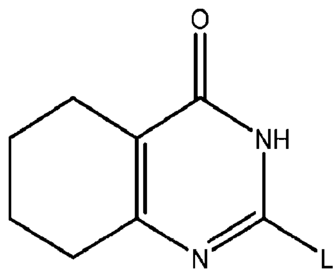

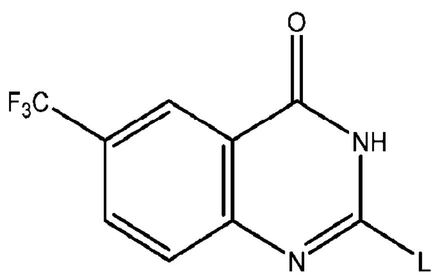

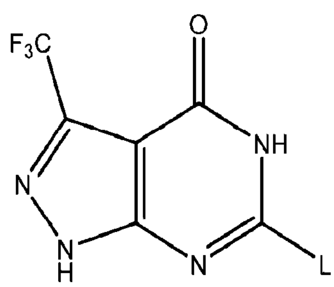

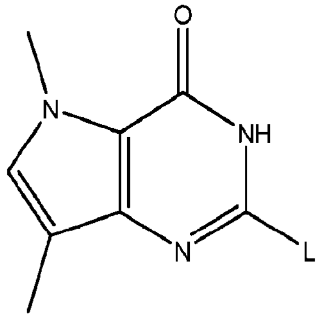

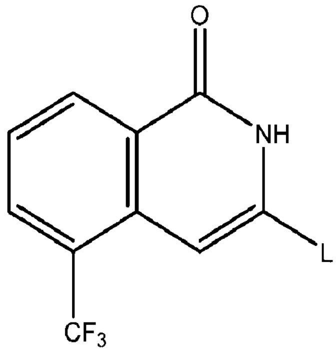

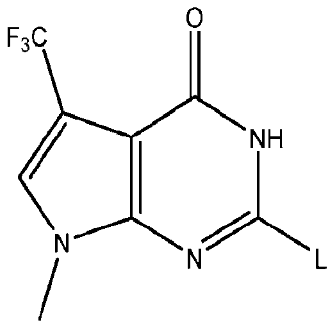

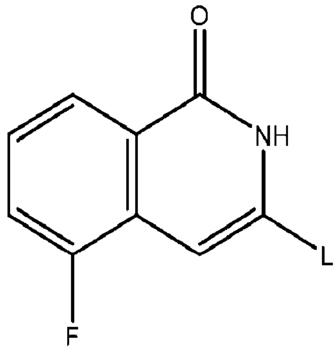

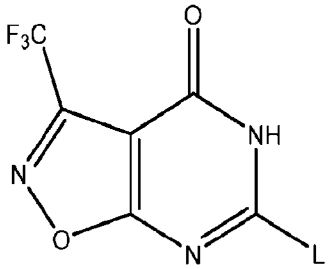

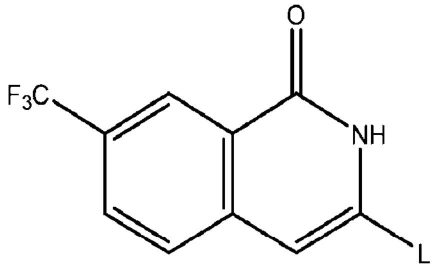

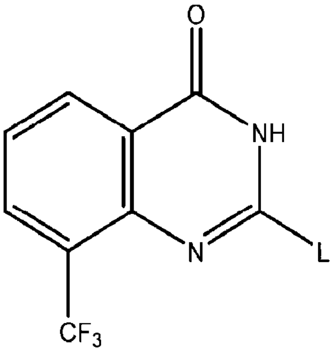

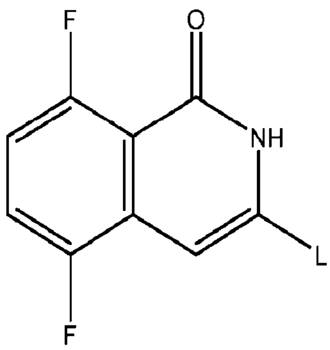

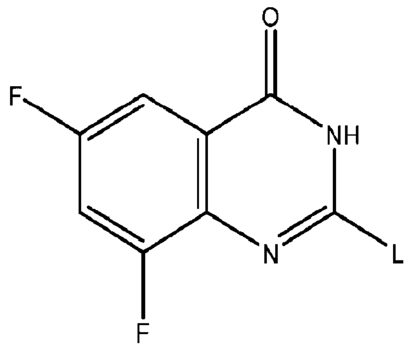

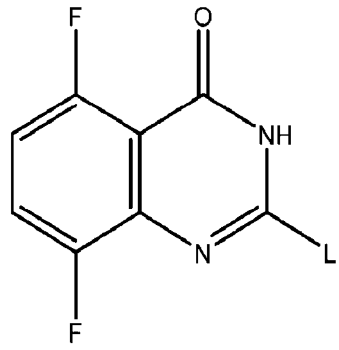

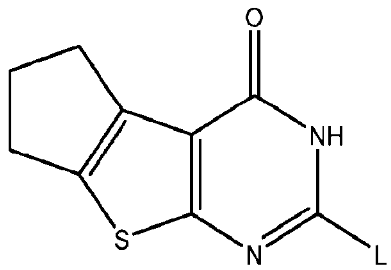

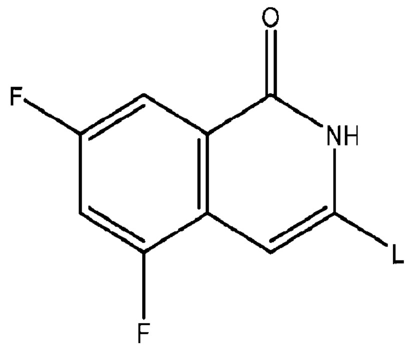

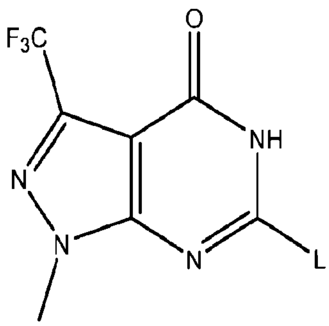

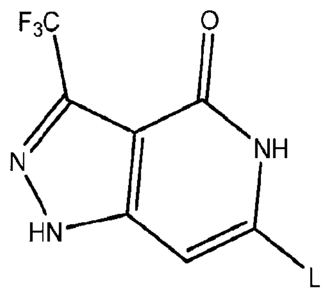

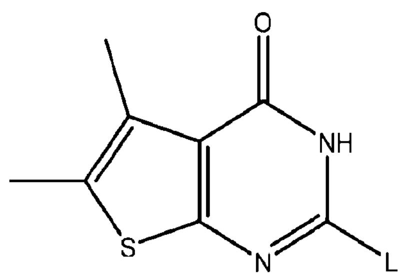

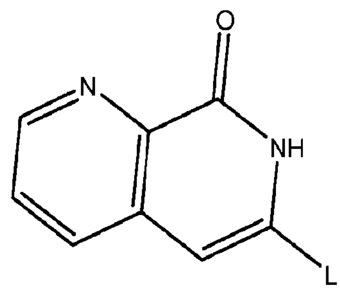

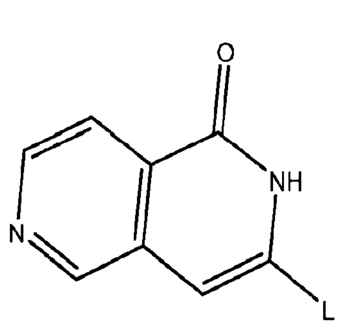

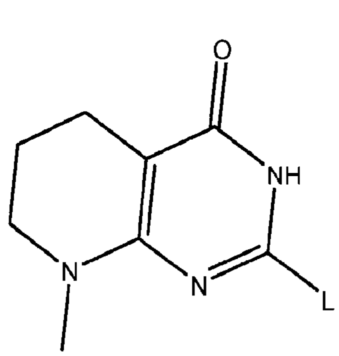

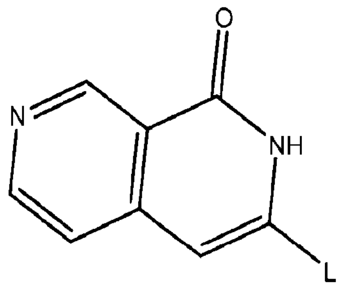

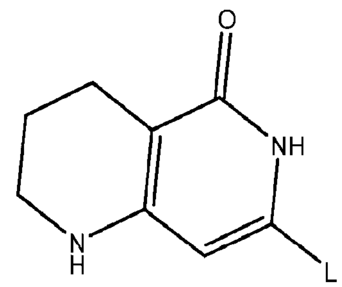

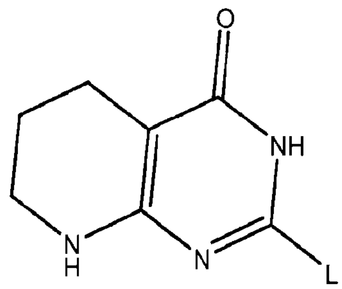

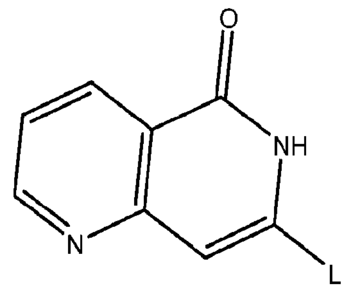

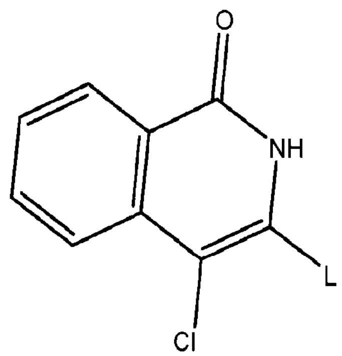

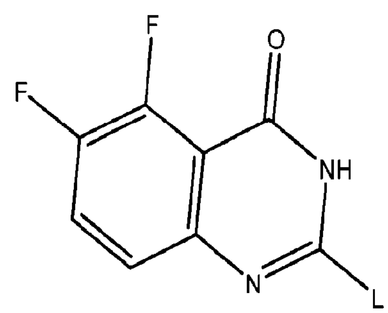

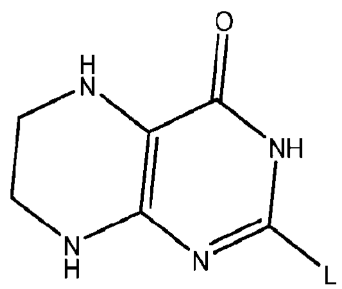

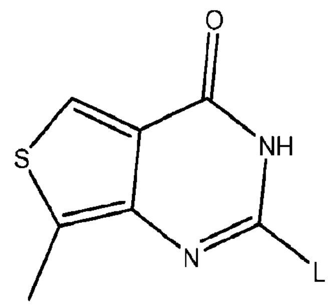

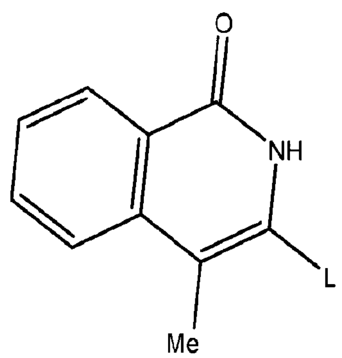

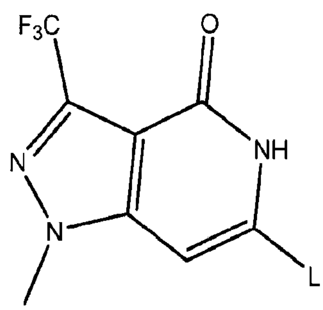

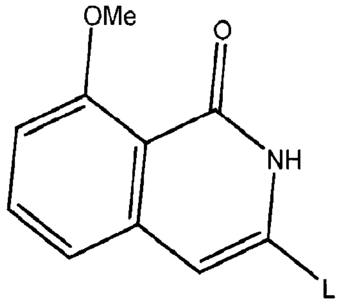
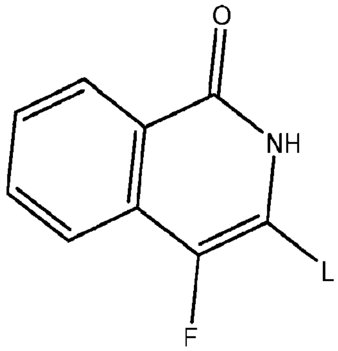
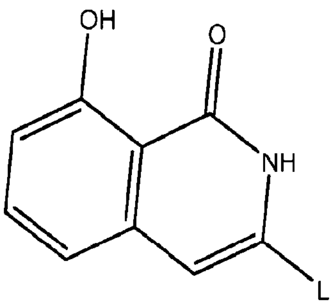
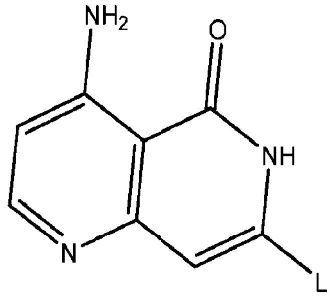
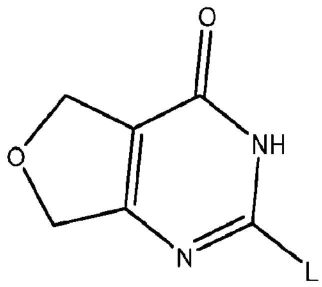
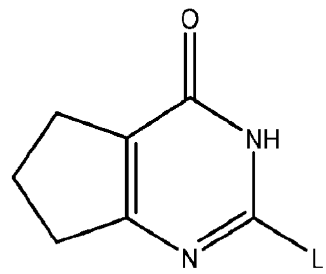
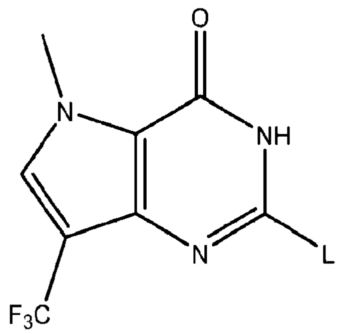
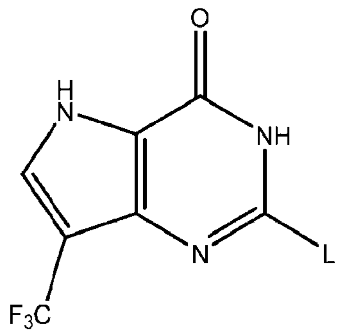
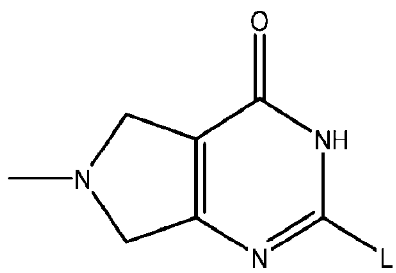
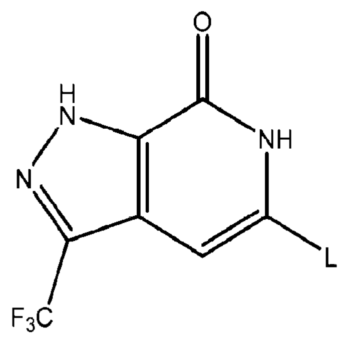

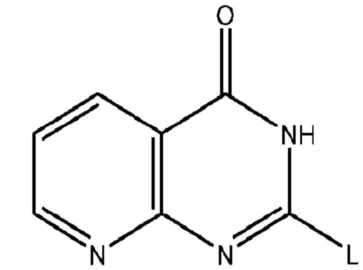

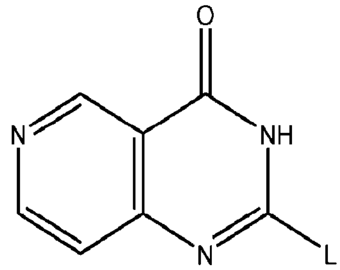

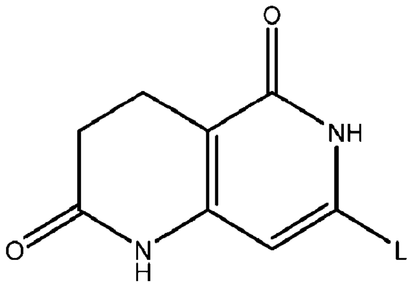

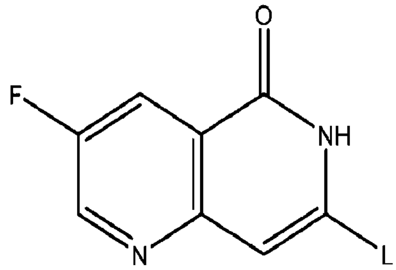

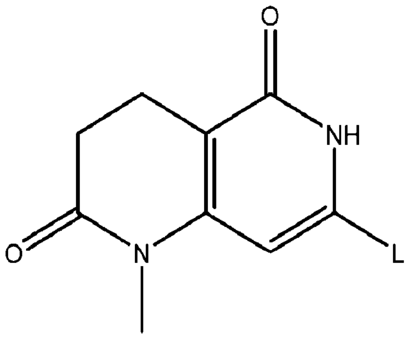

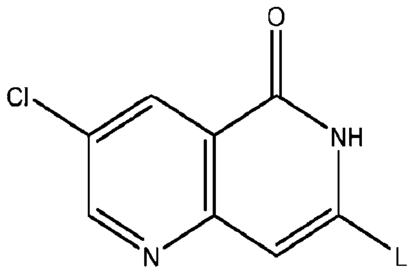

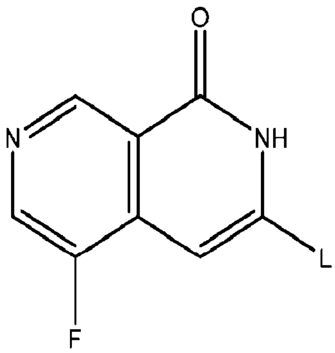

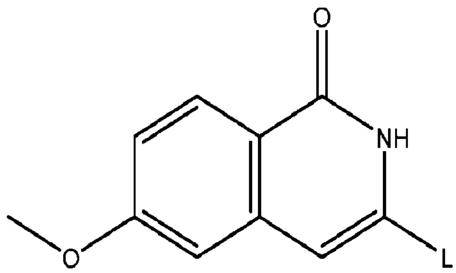

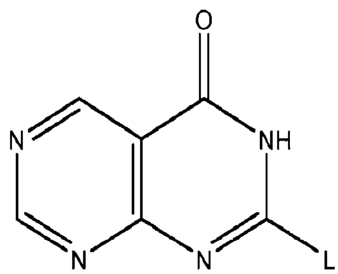

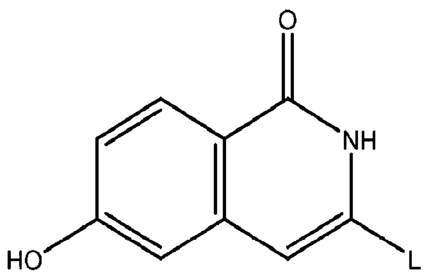

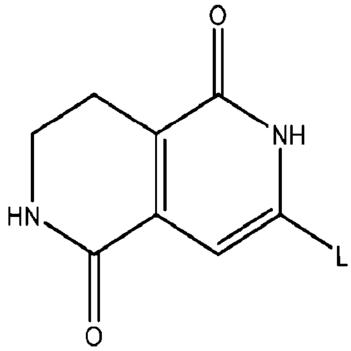

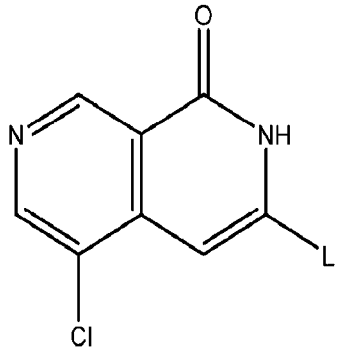

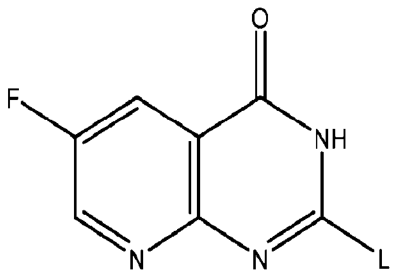

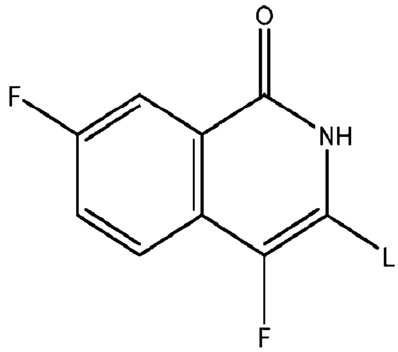

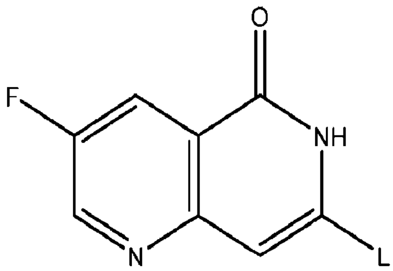

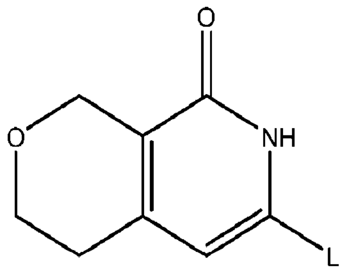

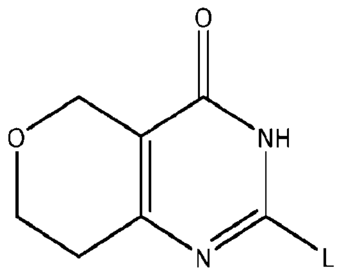

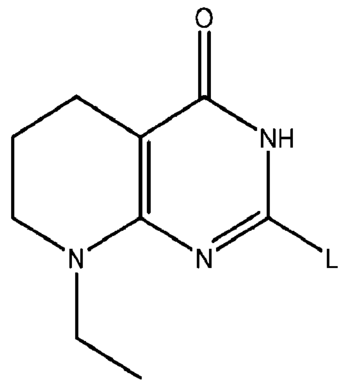

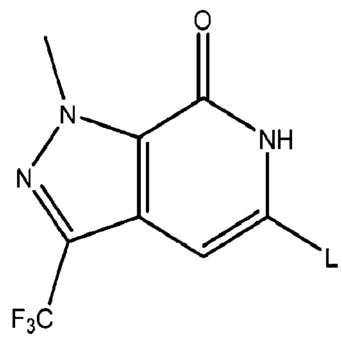

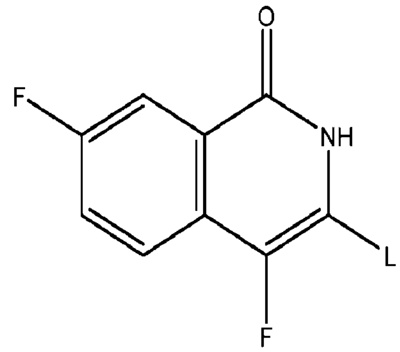

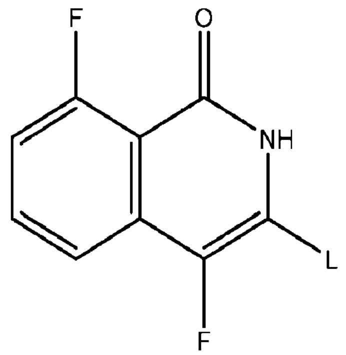

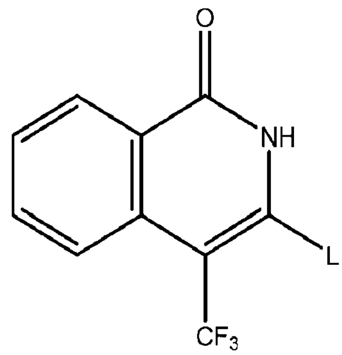

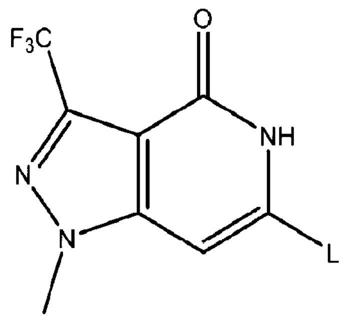

wherein L is as defined herein.

In some embodiments, a compound is provided which comprises any of the following structures:

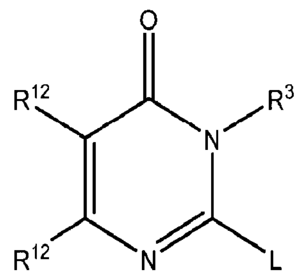

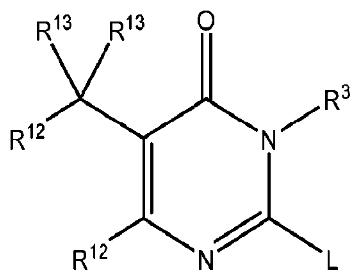

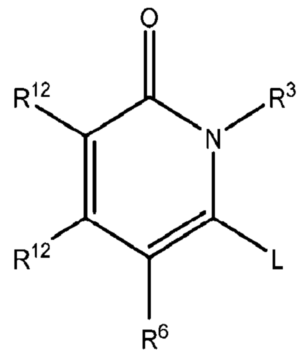
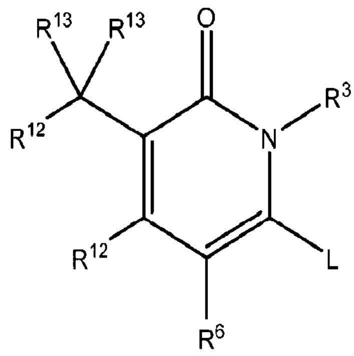

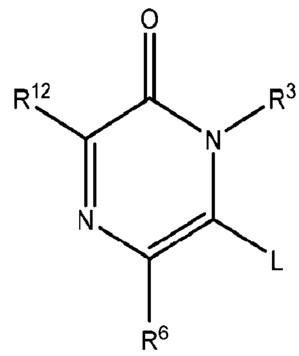
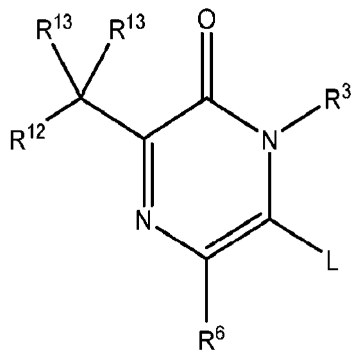

Wherein each $R^{12}$ is independently selected from H and a substituted or unsubstituted organic group, preferably a lower ($C_1$ to $C_6$) alkyl, alkoxy or haloalkyl group, a substituted or unsubstituted $C_3$ to $C_6$ cycloalkyl or heterocyclic group, and a halogen group, wherein at least one $R^{12}$ is not H; and wherein each $R^{13}$ may be the same or different and is independently selected from H and a substituted or unsubstituted organic group; and wherein $R^3$, $R^6$, and L are as defined herein.

At least one $R^{12}$ is preferably selected from $-CH_3$, $-CH_2CH_3$, $-CH_2CH_2CH_3$, $-CH_2Cl$, $-CHCl_2$, $-CCl_3$, $-CH_2F$, $-CHF_2$, $-CF_3$, -Cl, -F, -Cl, $-CH_2CF_3$, $-CH_2CH_2F$, $-CH_2CH_2OH$, methoxy, methoxymethyl, methoxyethyl, isopropyl, cyclopropyl or cyclopropylmethyl.

$R^{13}$ is preferably selected from H, F, $C_1$ to $C_3$ alkyl or $C_1$ to $C_3$ fluoroalkyl.

$R^3$ is preferably H.

$R^6$ is preferably selected from H, halogen, $C_1$ to $C_3$ alkyl, $C_1$ to $C_3$ haloalkyl, $C_1$ to $C_3$ alcohol or $C_1$ to $C_3$ aminooalkyl.

In some embodiments, a compound is provided which comprises any of the following structures:

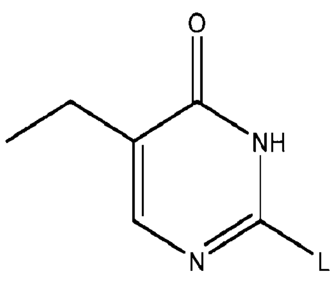
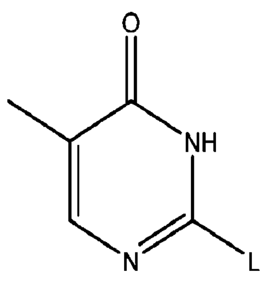
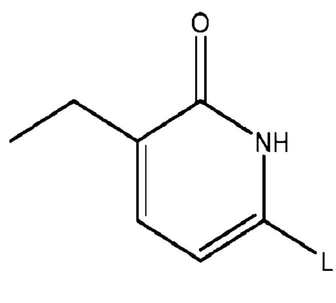

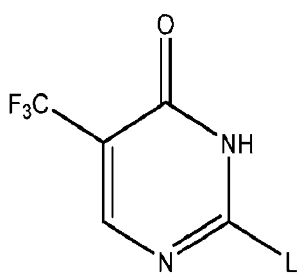
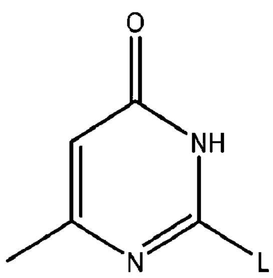
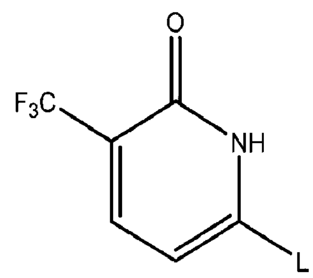

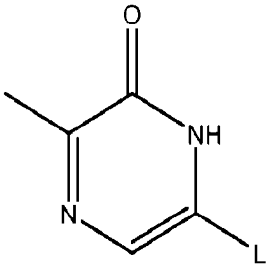
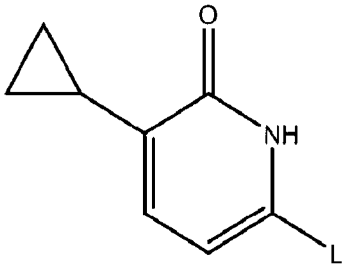
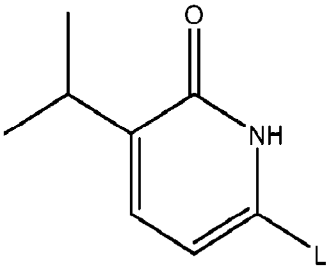

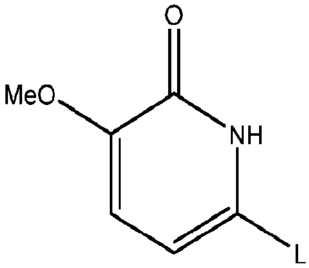
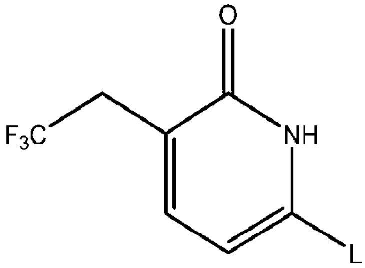
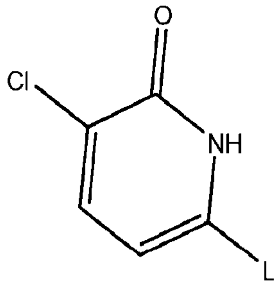

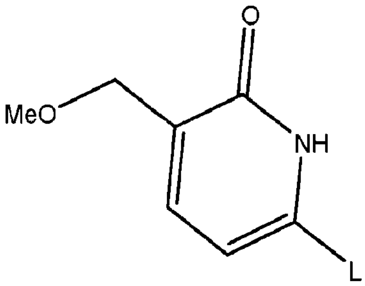
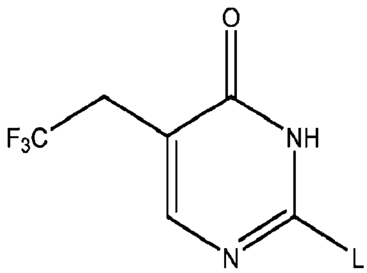

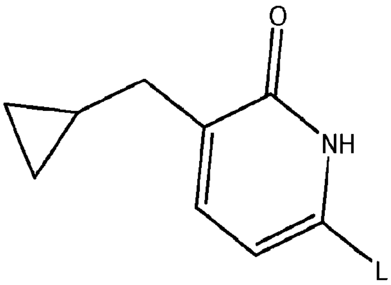
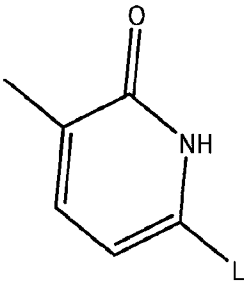
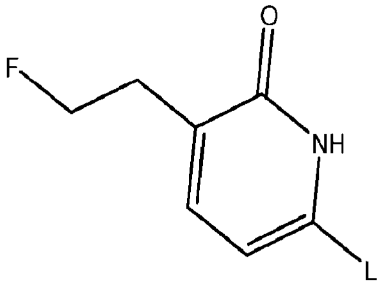

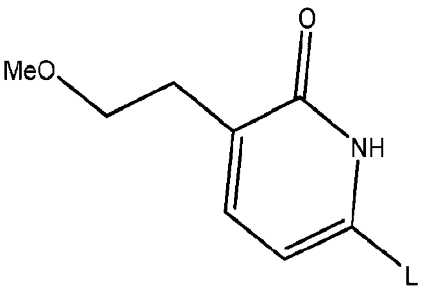
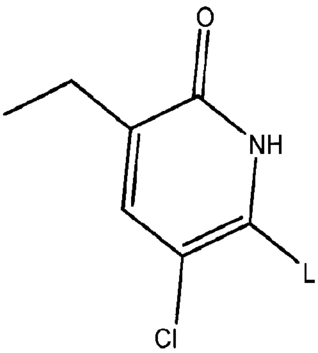
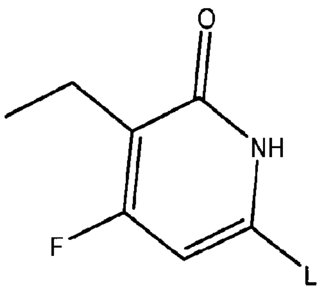

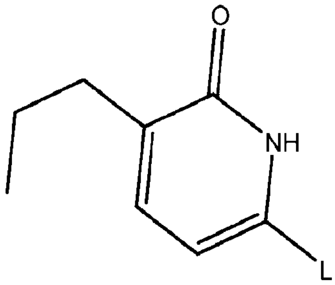

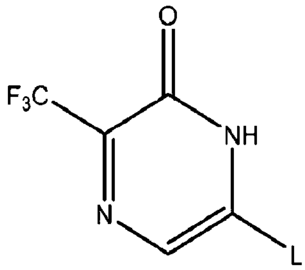

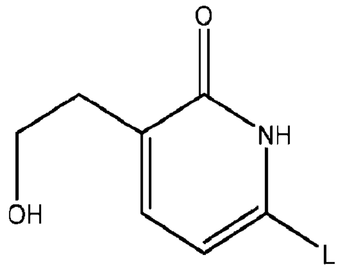

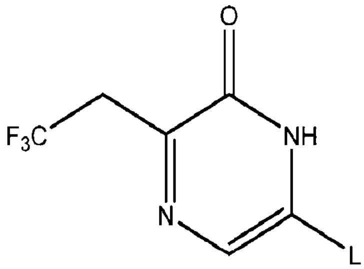

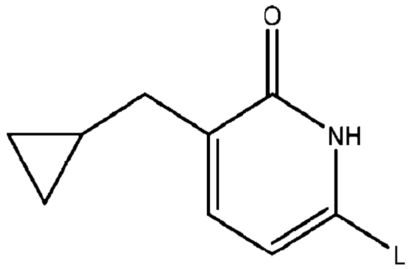

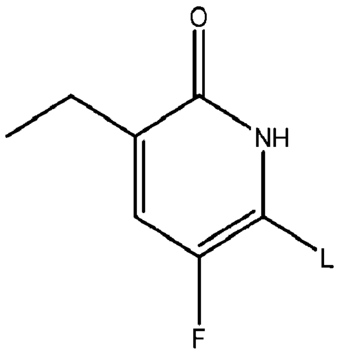

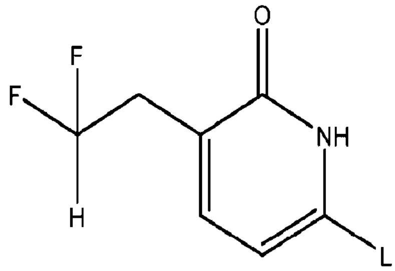

wherein L is as defined herein.

In some embodiments, the present invention provides a PARP1 inhibitor compound which comprises a formula selected from one of the following:

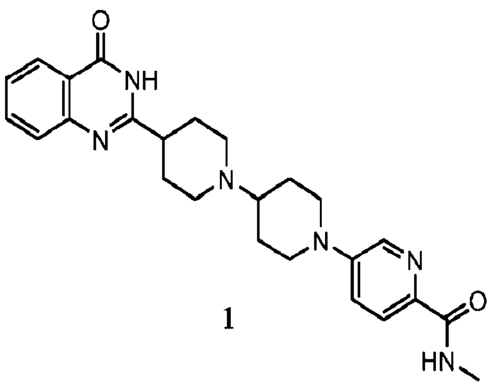

1

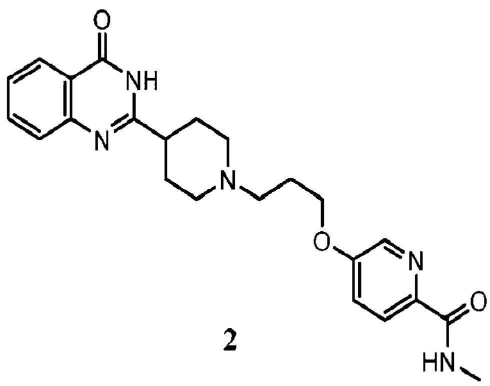

2

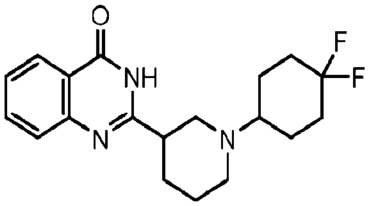

3

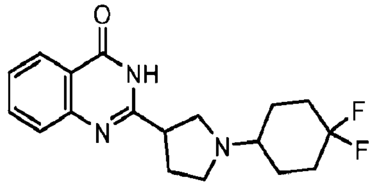

4

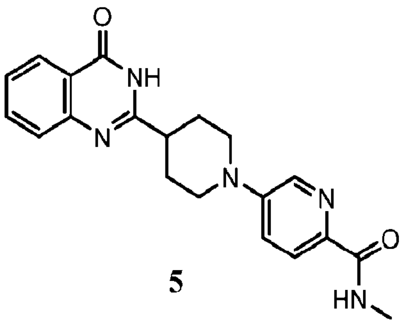

5

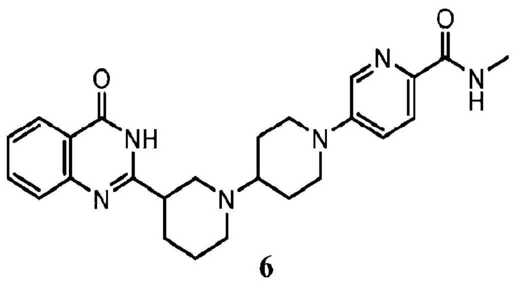

6

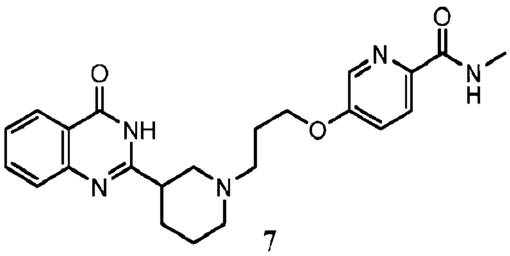

7

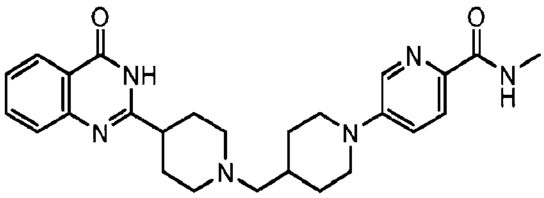

8

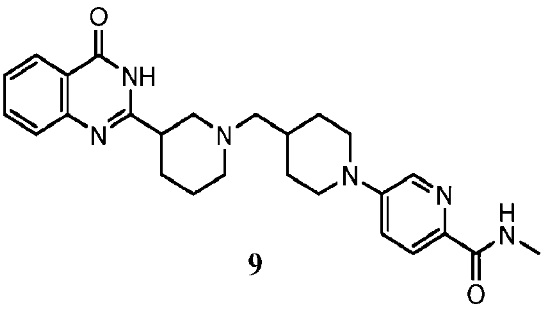

9

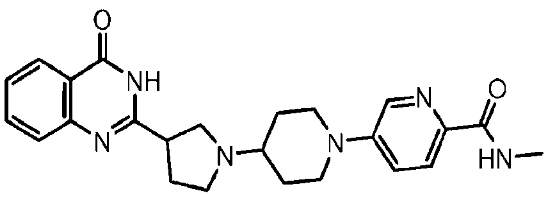

10

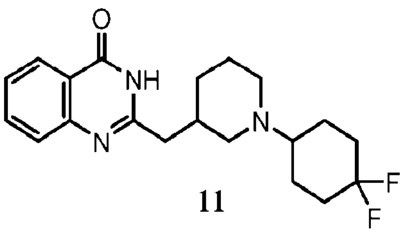

11

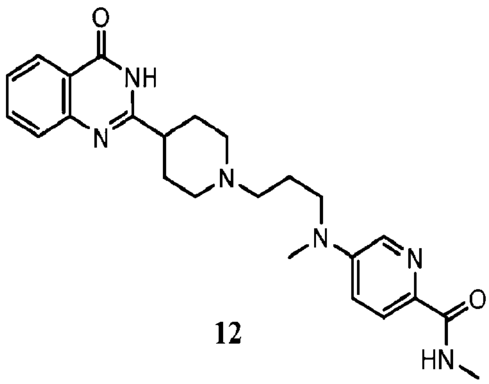

12

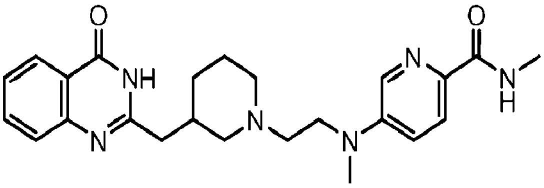

13

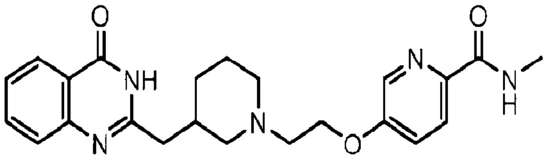

14

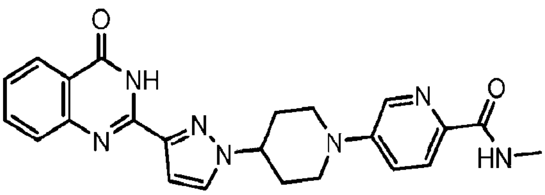

15

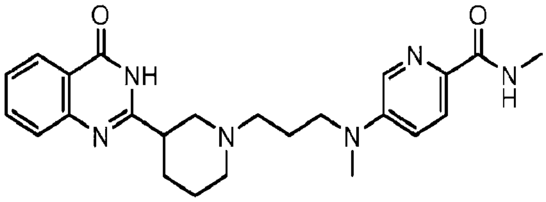

16

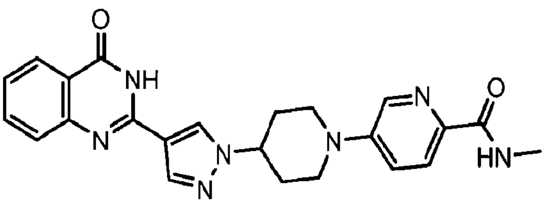

17

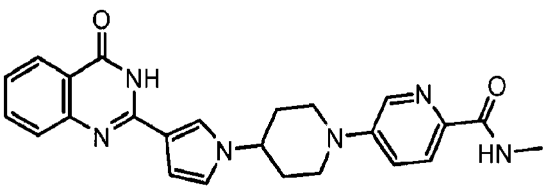

18

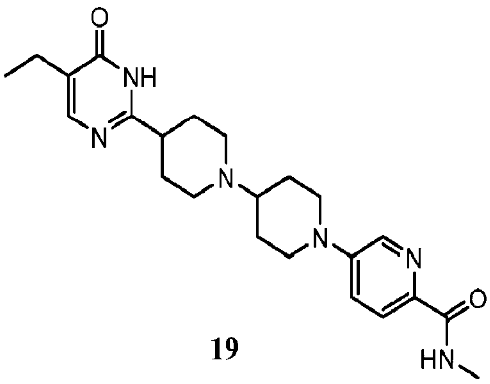

19

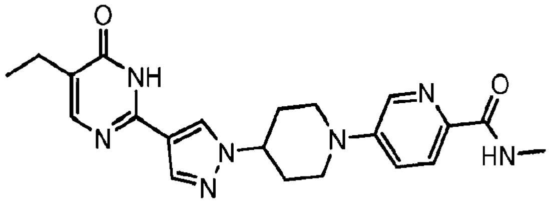

20

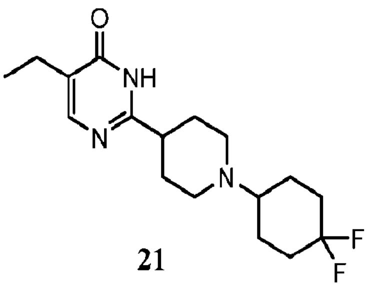

21

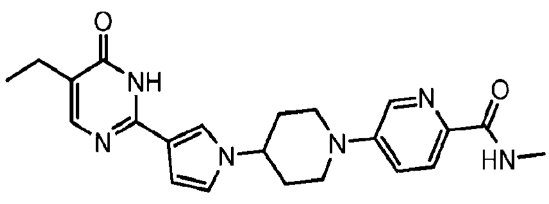

22

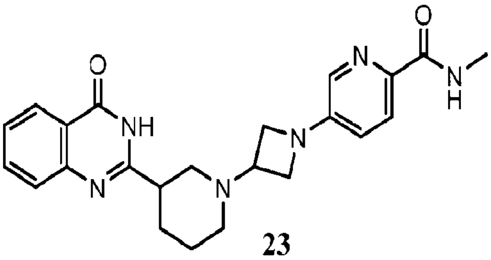

**23**

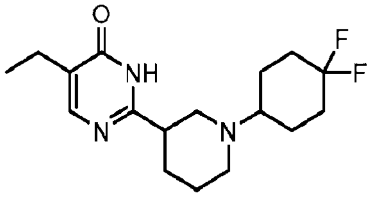

**24**

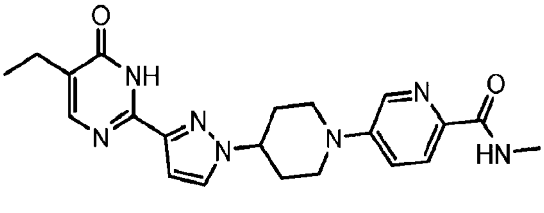

**25**

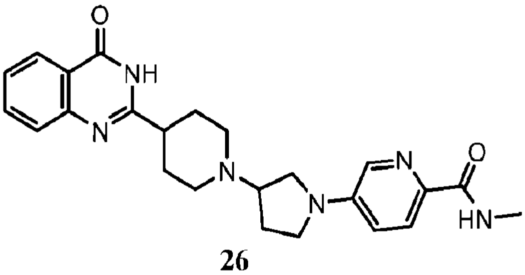

**26**

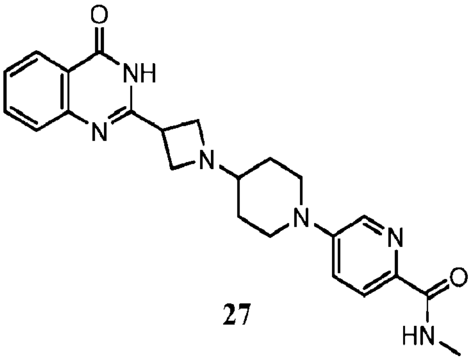

**27**

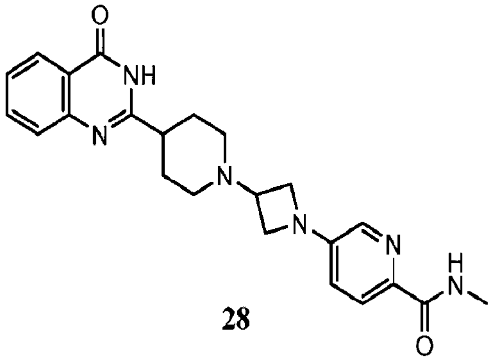

**28**

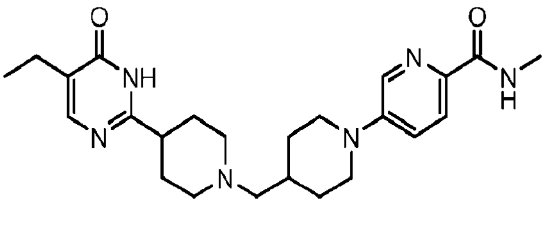

**29**

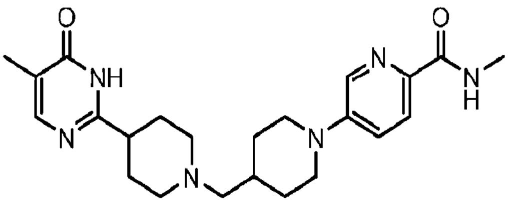

**30**

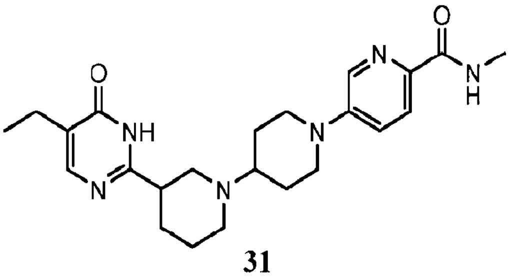

**31**

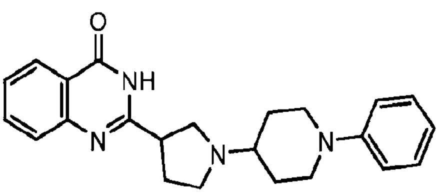

**32**

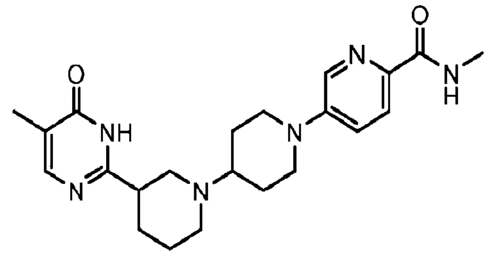

33

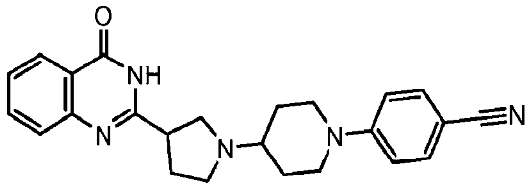

34

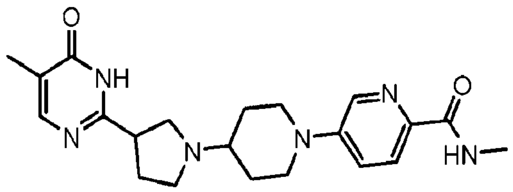

35

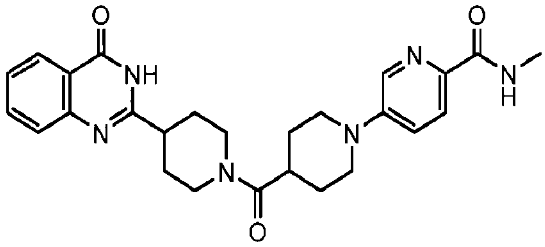

36

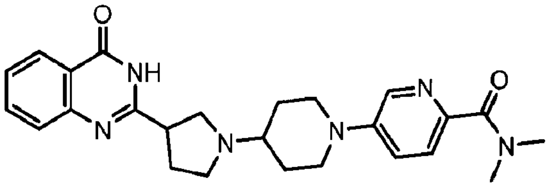

37

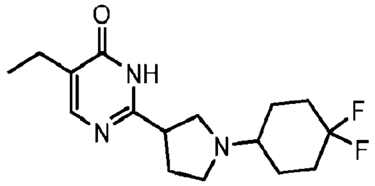

38

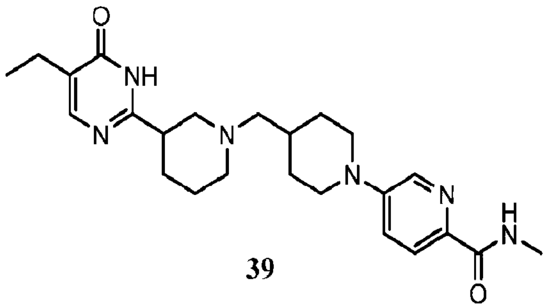

39

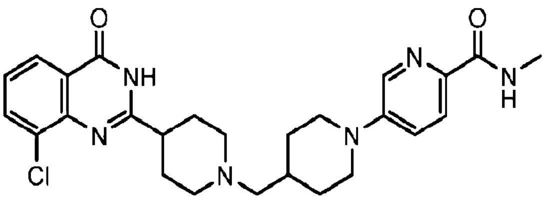

40

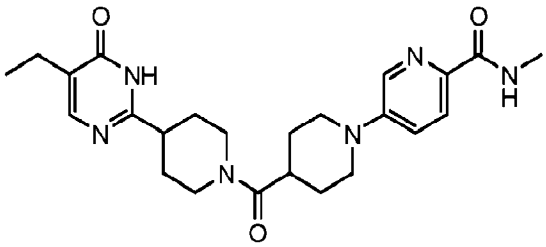

41

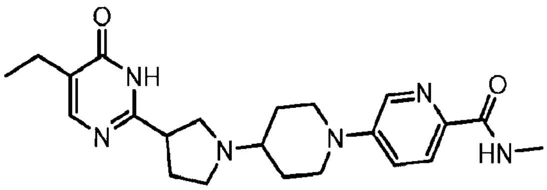

42

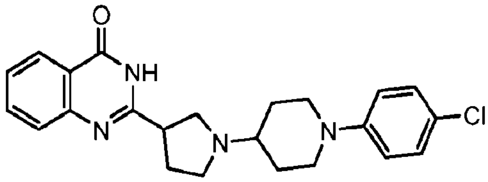

43

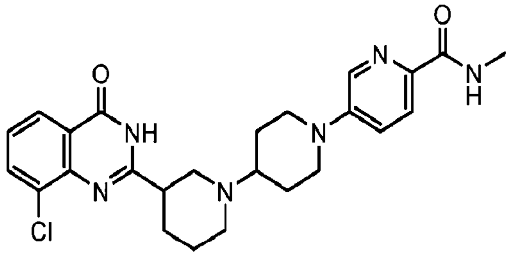

44

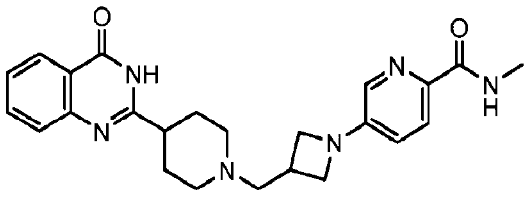

45

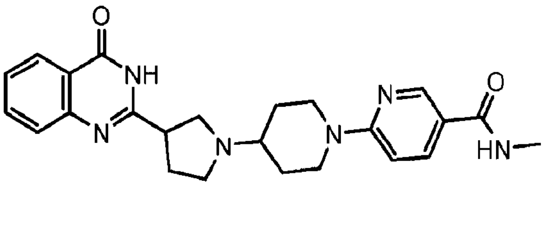

46

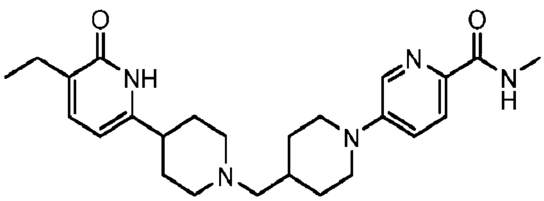

47

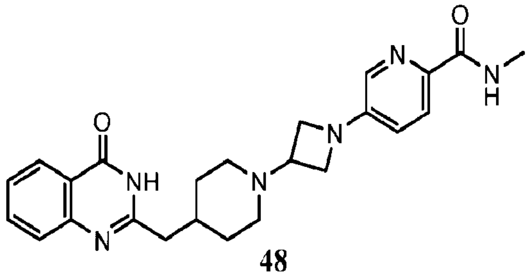

48

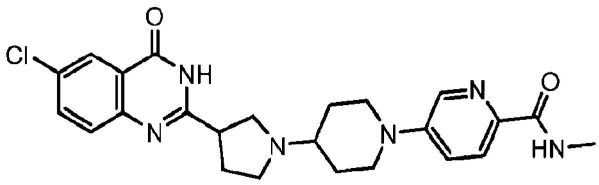

49

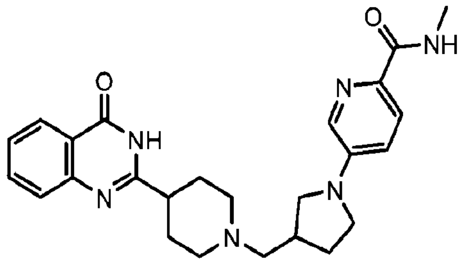

50

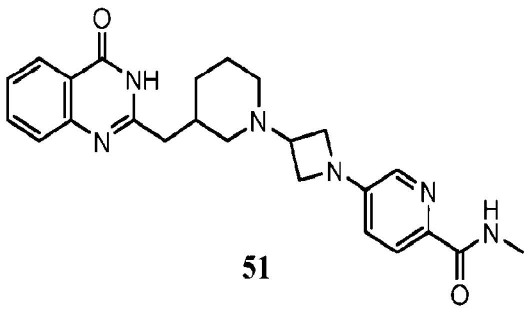

51

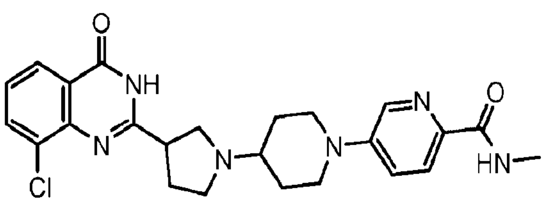

52

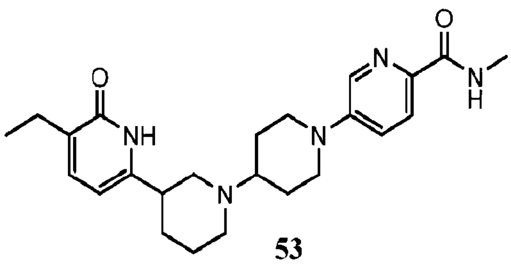

53

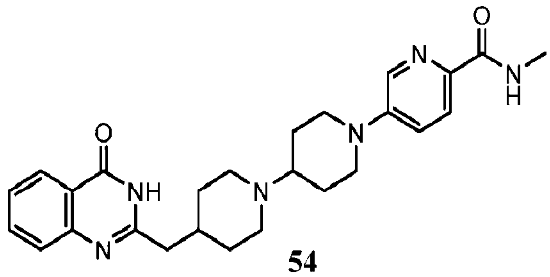

54

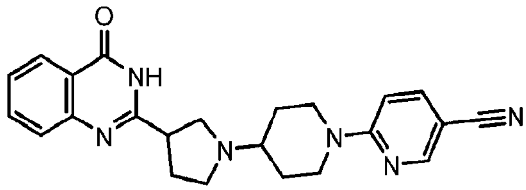

55

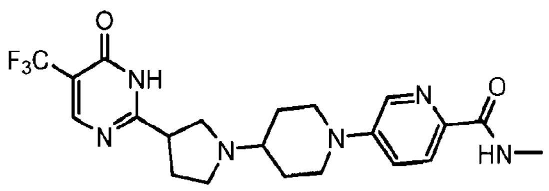

56

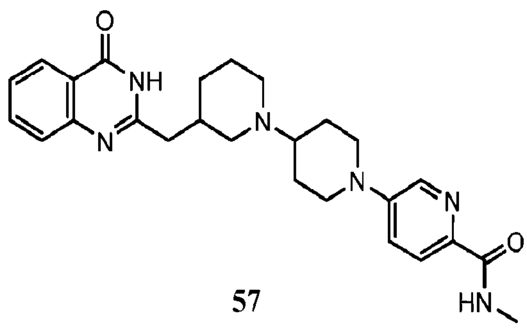

57

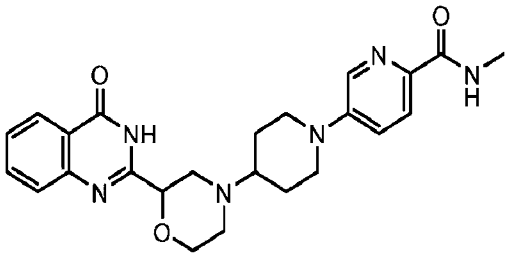

58

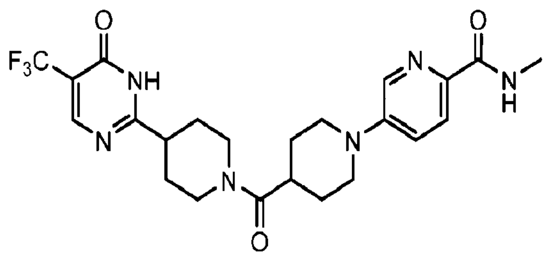

59

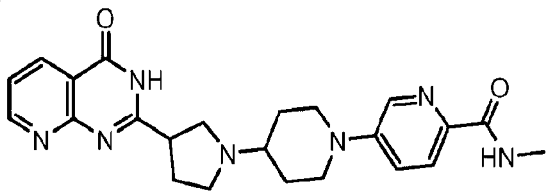

60

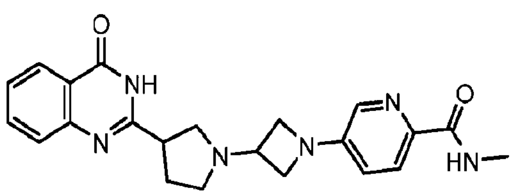

61

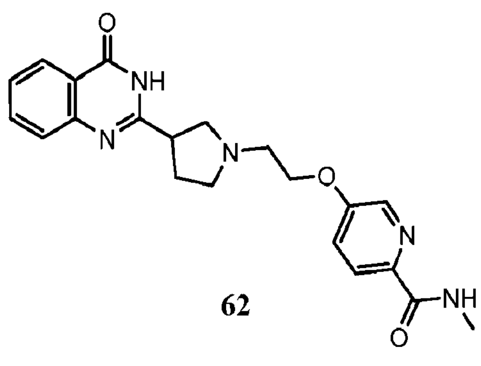

62

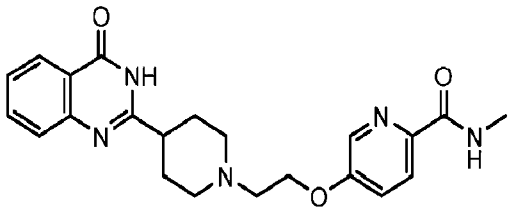

63

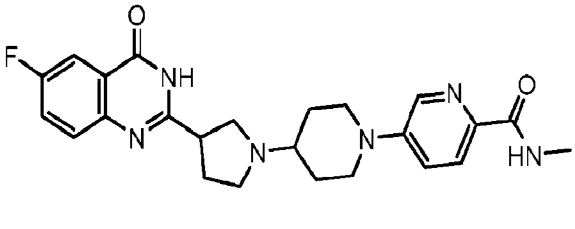

64

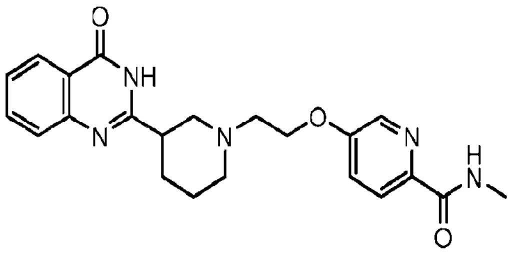

65

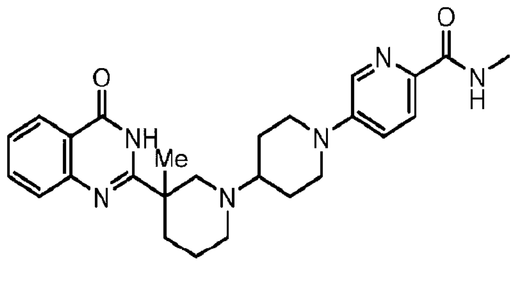

66

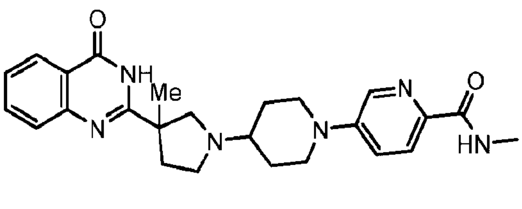

67

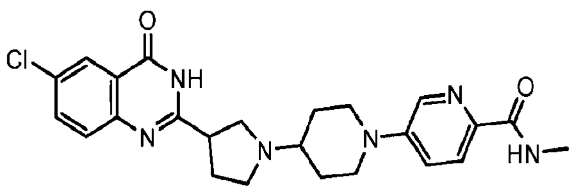

68

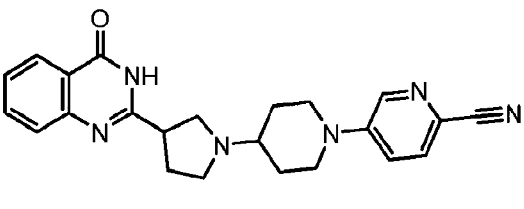

69

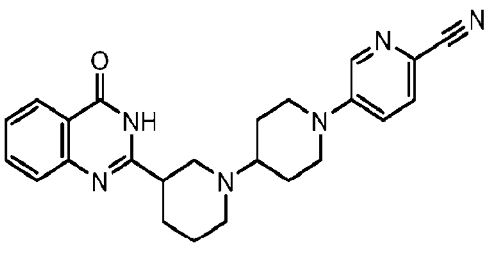

70

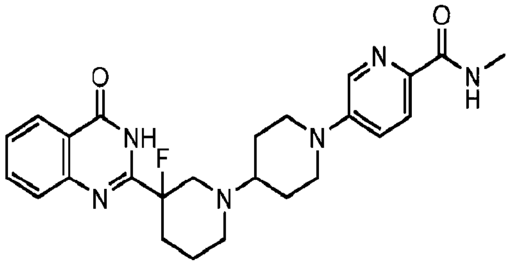

71

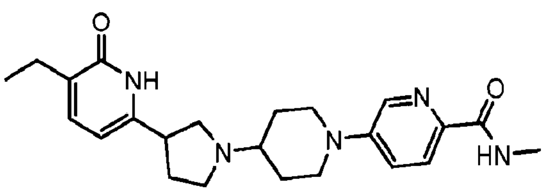

72

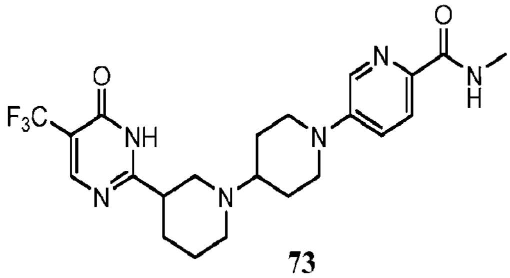

73

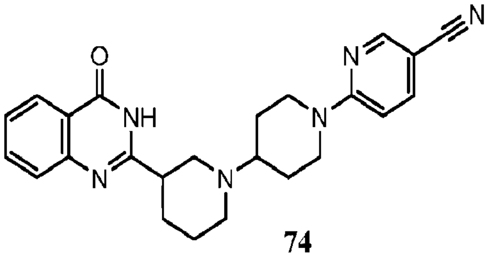

74

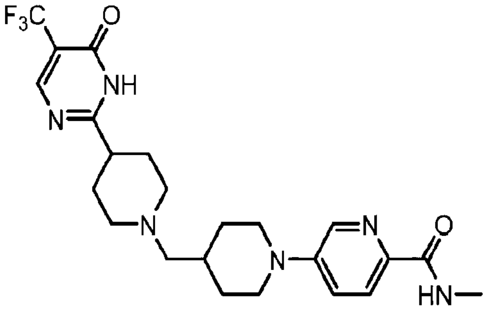

75

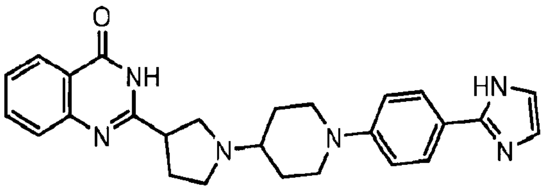

76

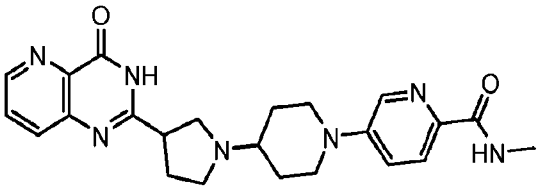

77

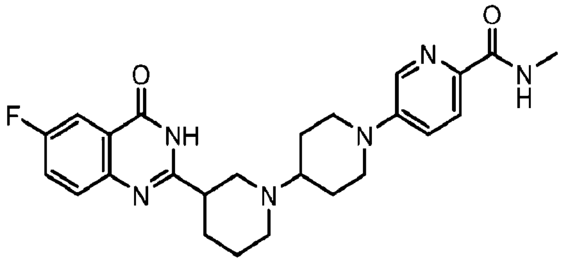

78

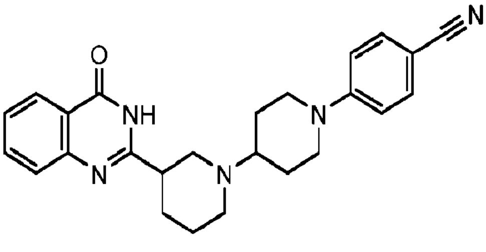

79

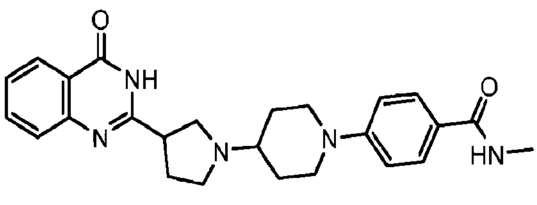

80

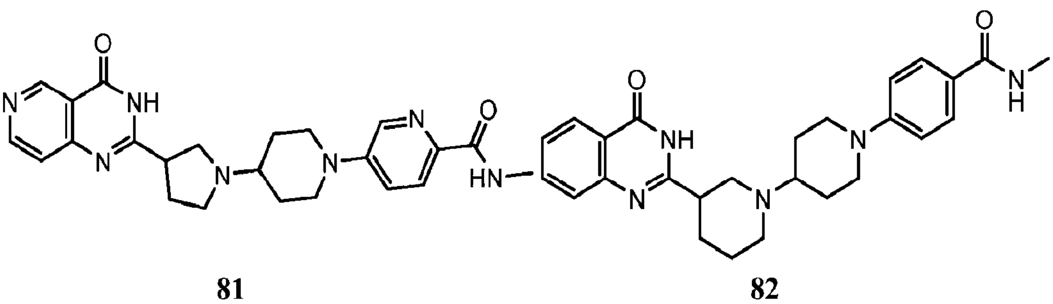

81

82

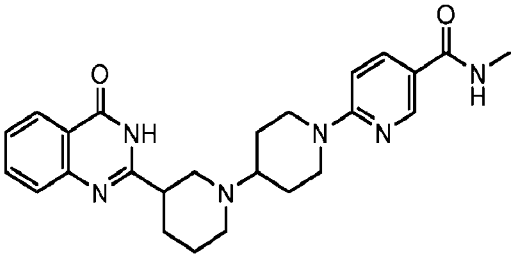

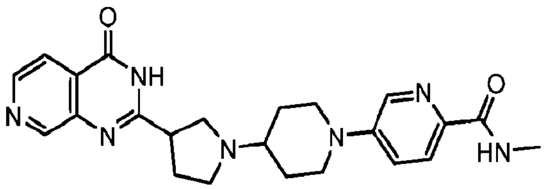

83

84

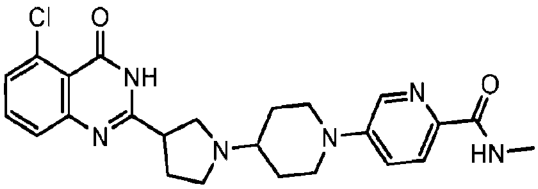

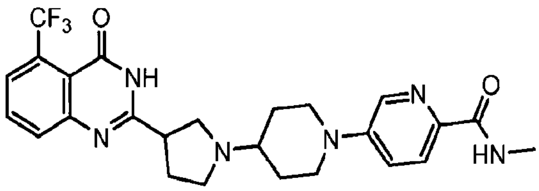

85

86

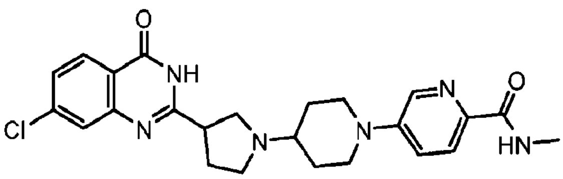

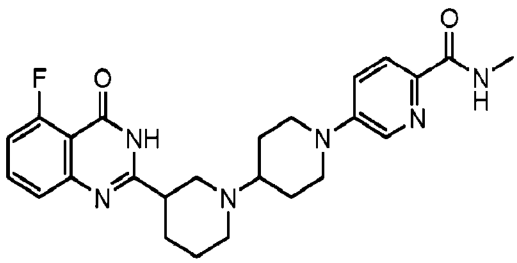

87

88

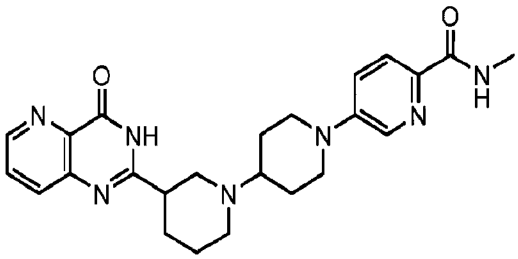

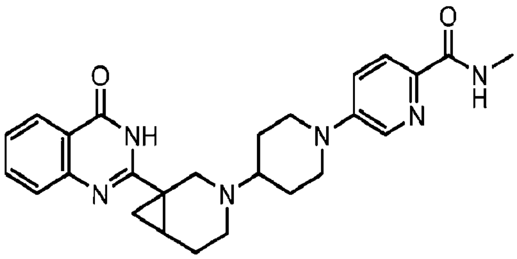

89

90

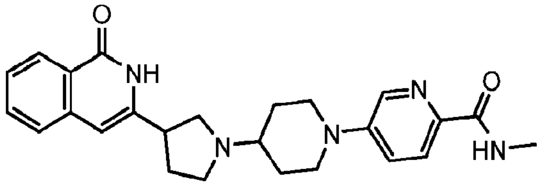

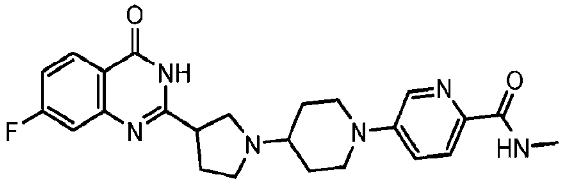

91

92

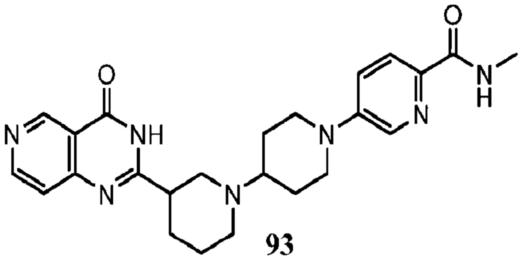

93

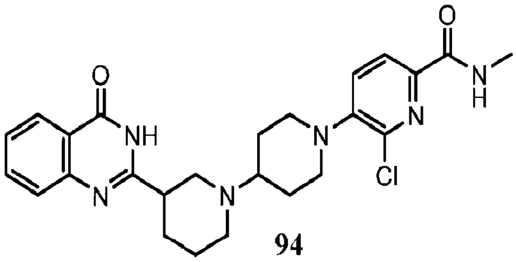

94

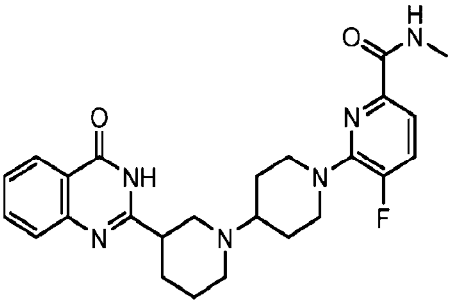

95

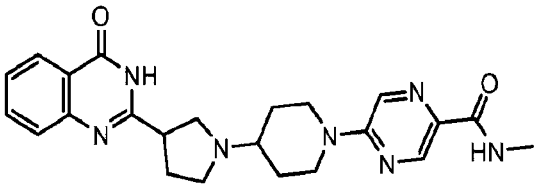

96

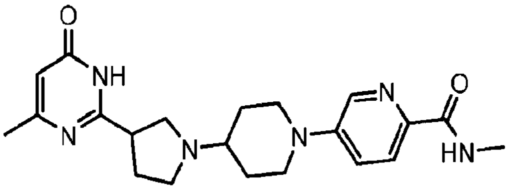

97

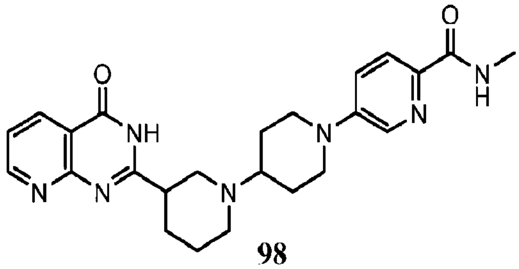

98

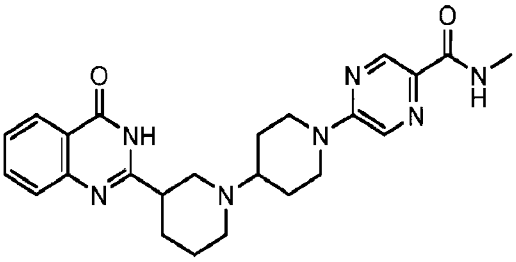

99

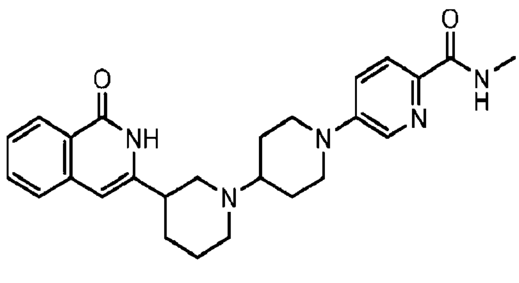

100

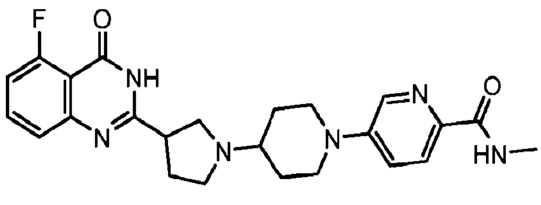

101

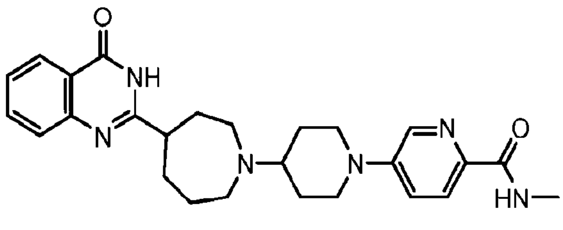

102

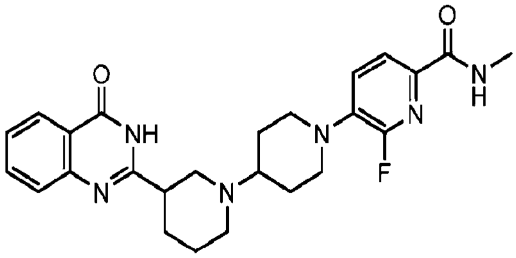

**103**

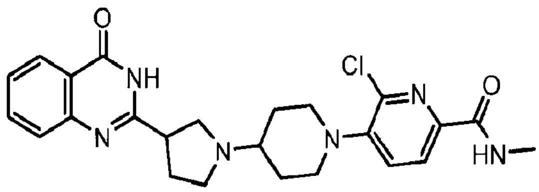

**104**

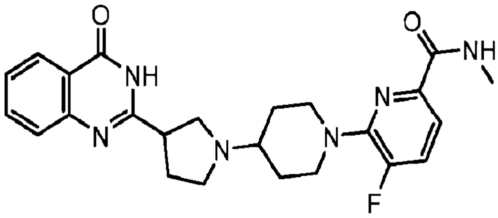

**105**

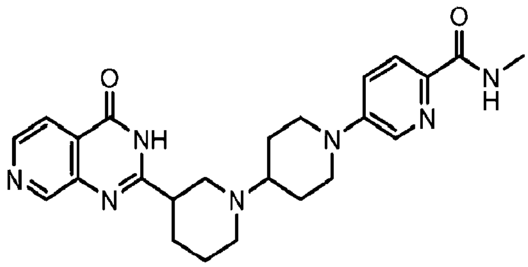

**106**

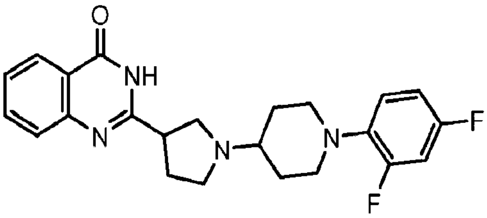

**107**

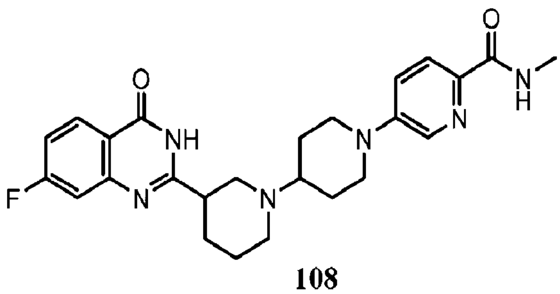

**108**

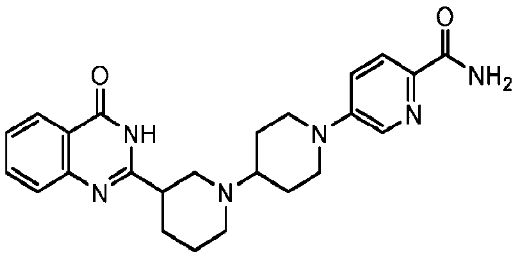

**109**

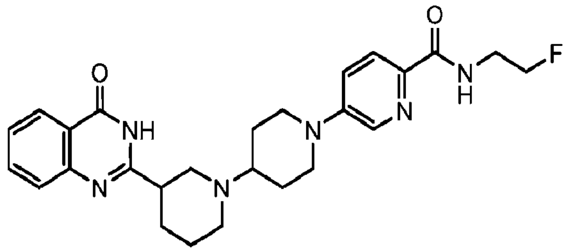

**110**

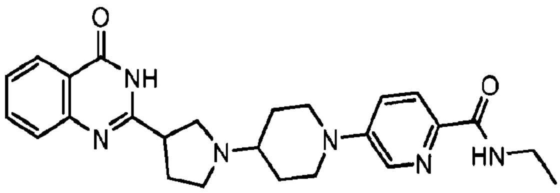

**111**

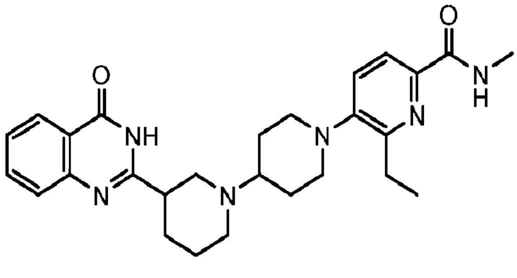

**112**

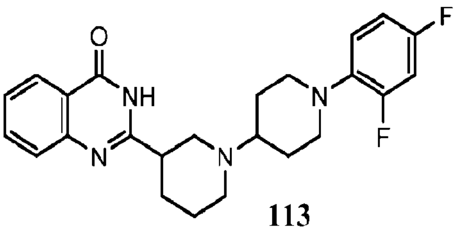

113

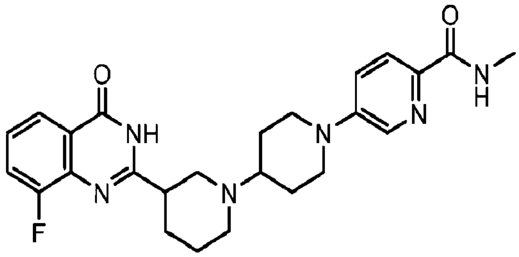

114

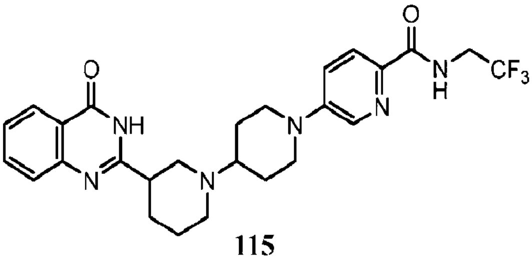

115

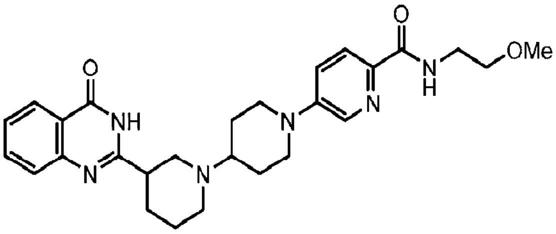

116

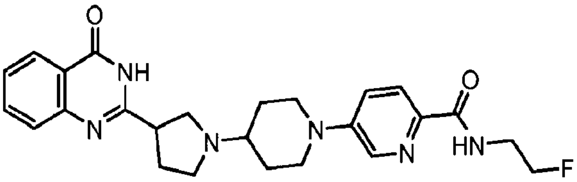

117

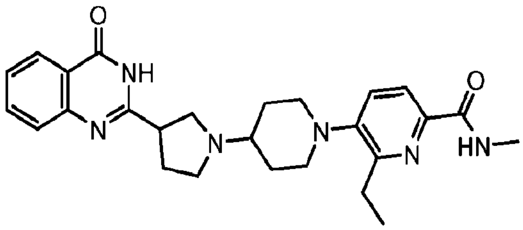

118

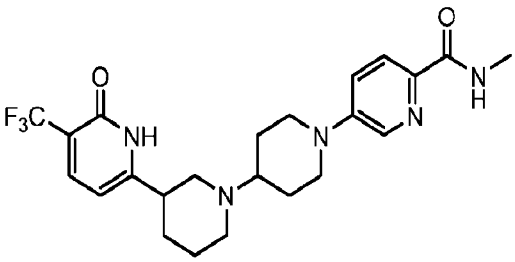

119

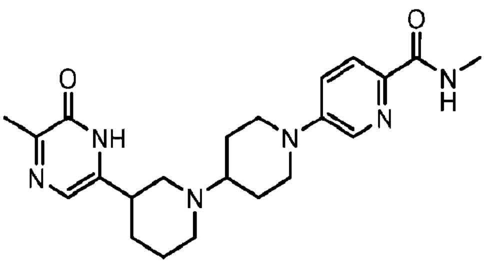

120

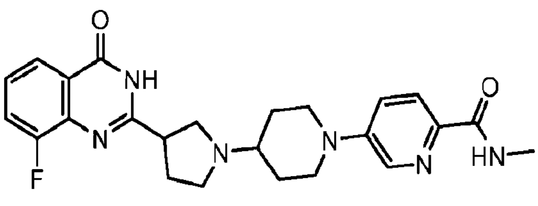

121

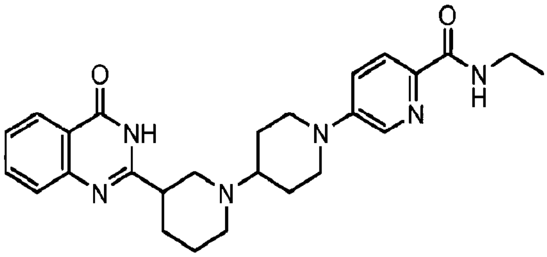

122

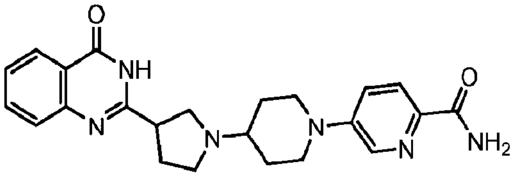

123

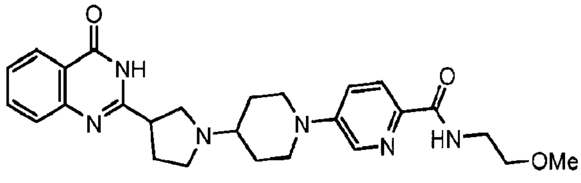

124

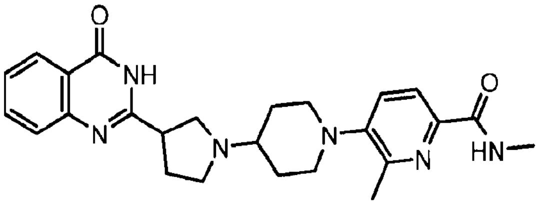

125

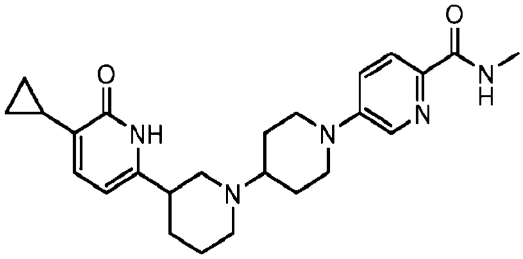

126

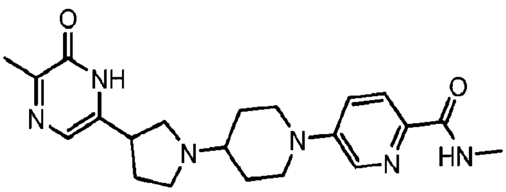

127

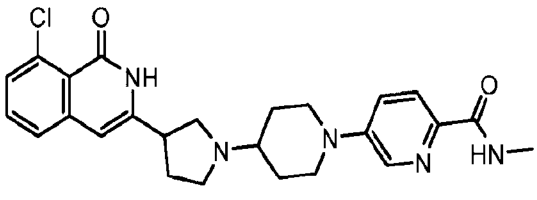

128

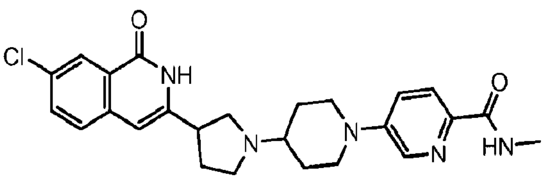

129

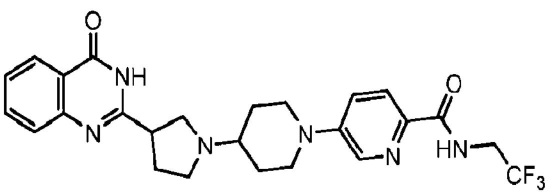

130

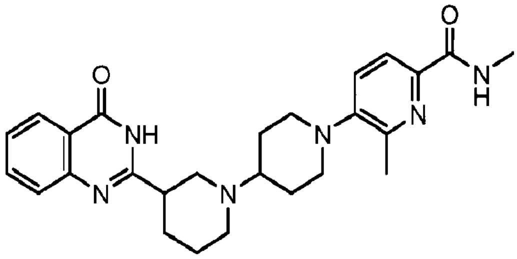

131

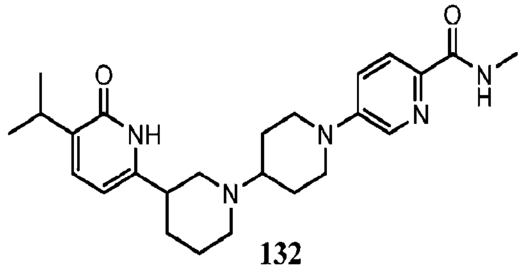

132

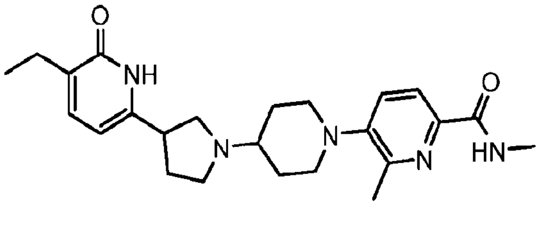

133

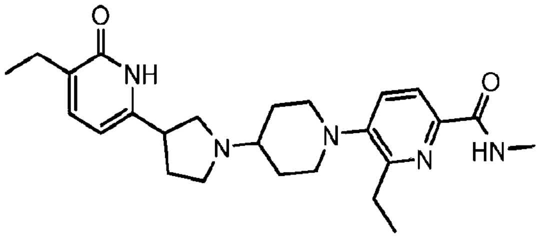

134

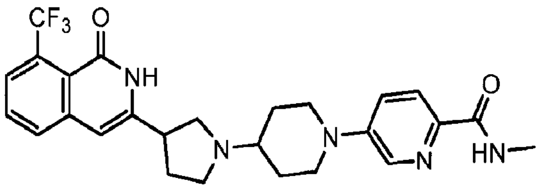

135

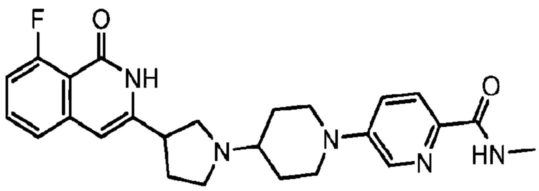

136

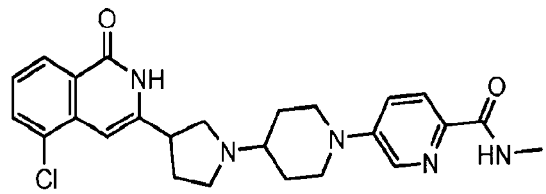

137

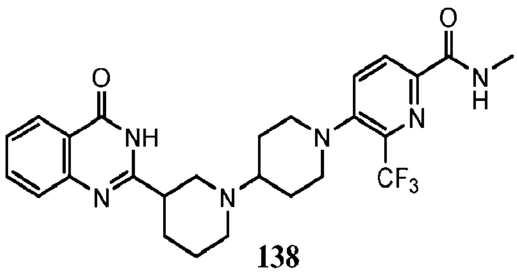

138

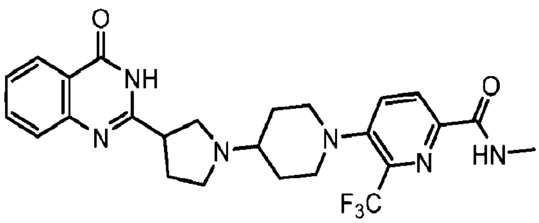

139

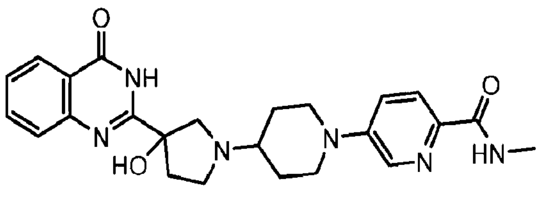

140

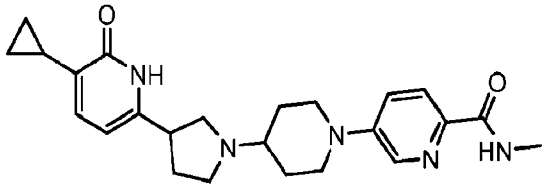

141

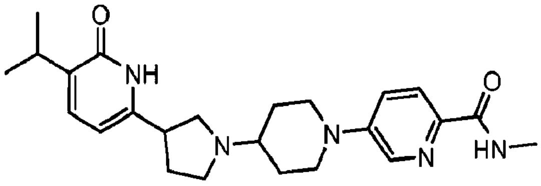

142

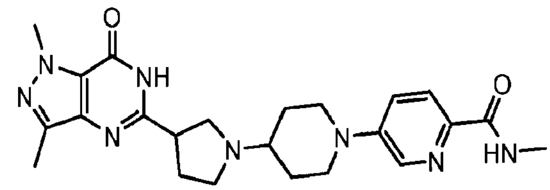

143

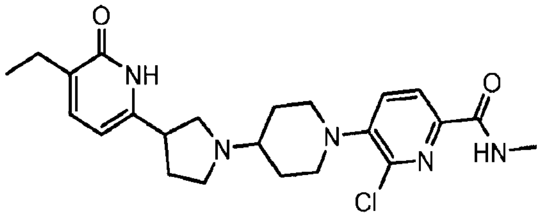

144

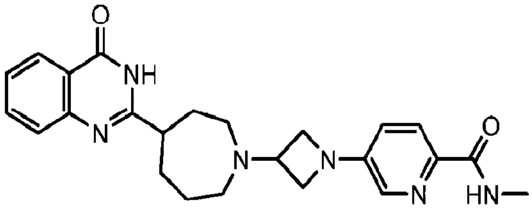

145

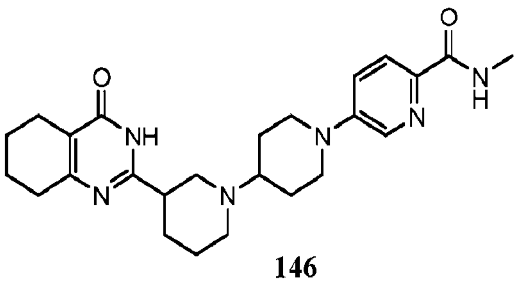

146

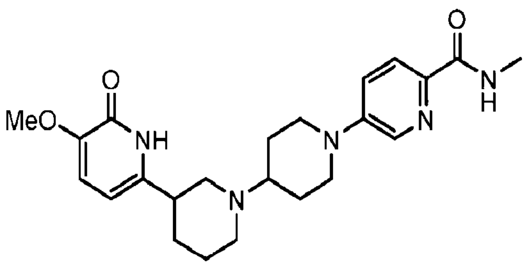

147

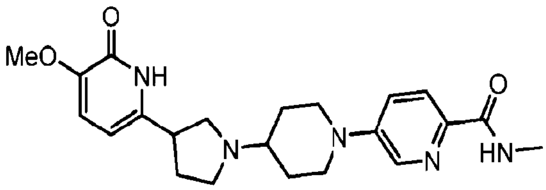

148

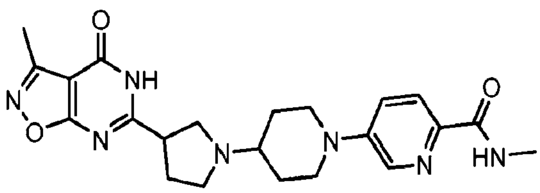

149

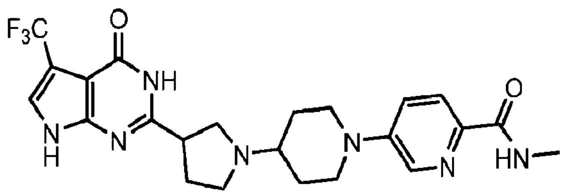

150

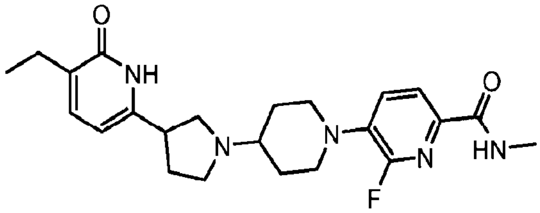

151

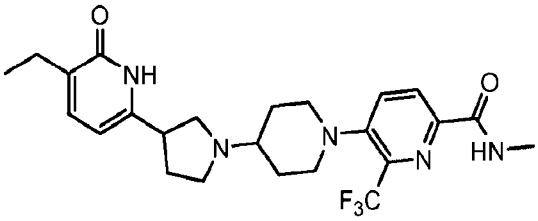

152

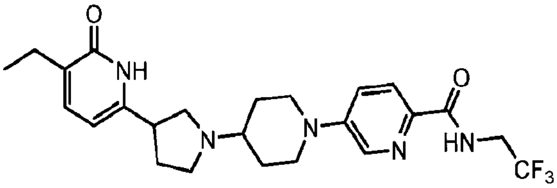

153

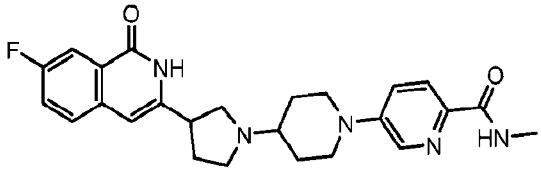

154

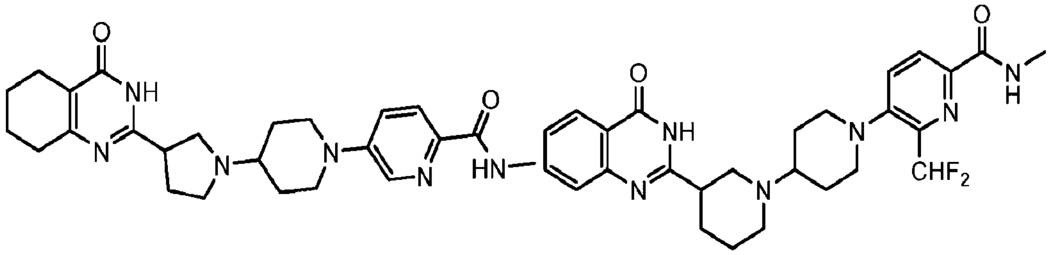

155

156

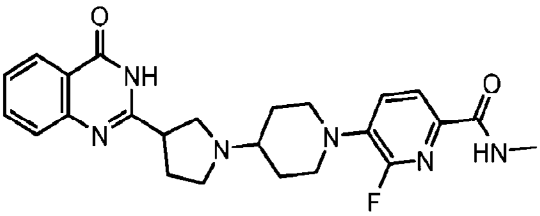

157

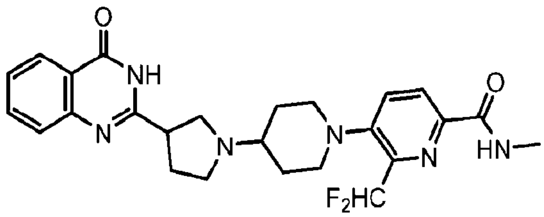

158

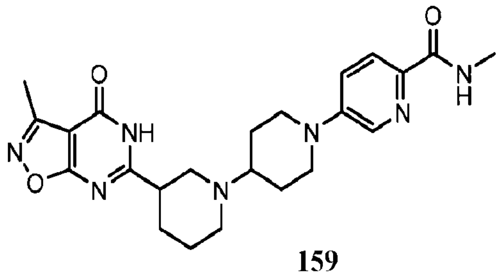

159

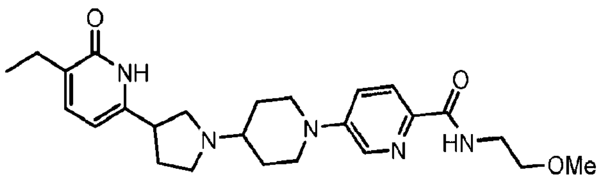

160

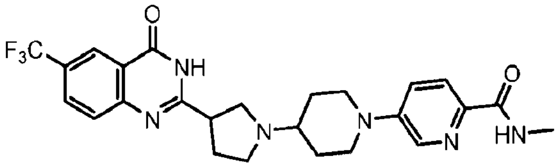

161

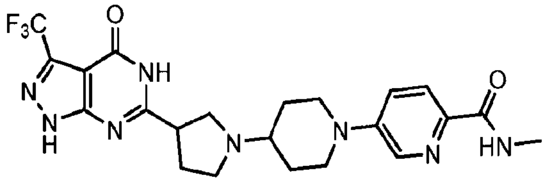

162

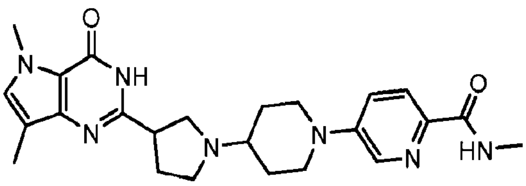

163

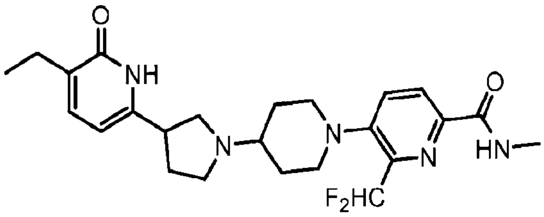

164

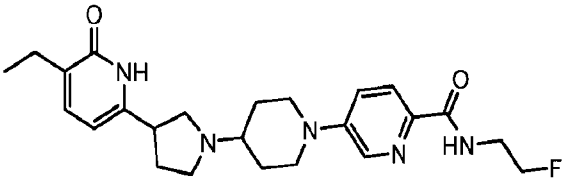

165

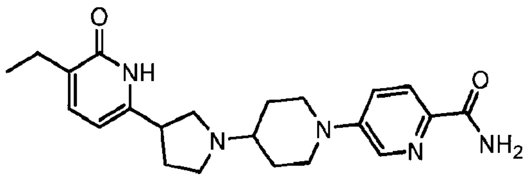

166

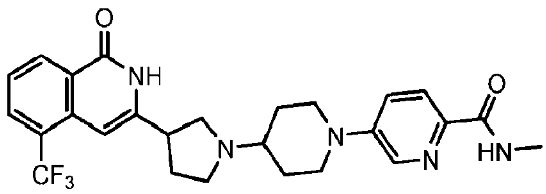

167

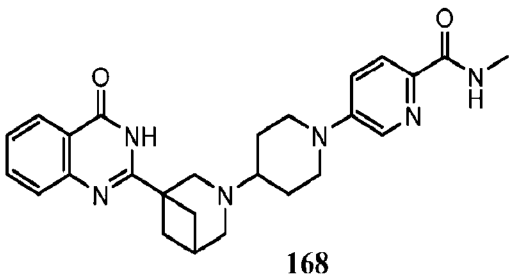

168

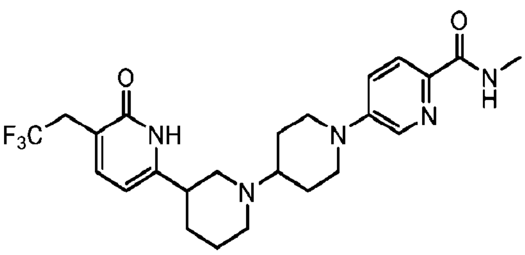

169

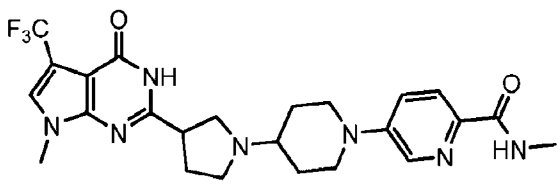

170

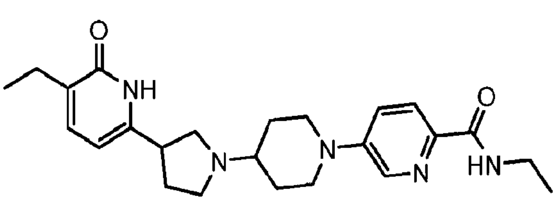

171

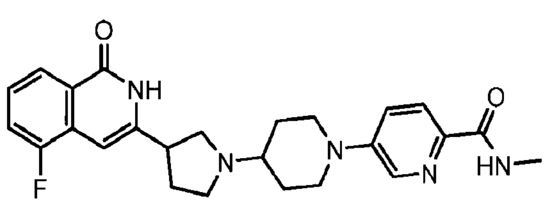

172

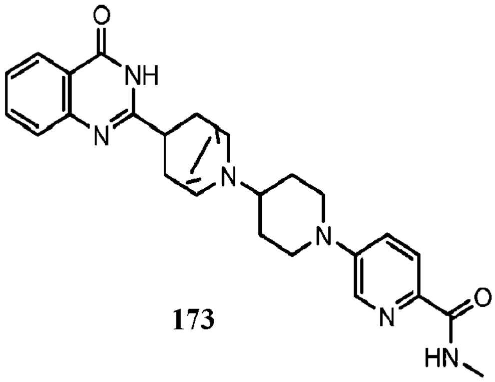

173

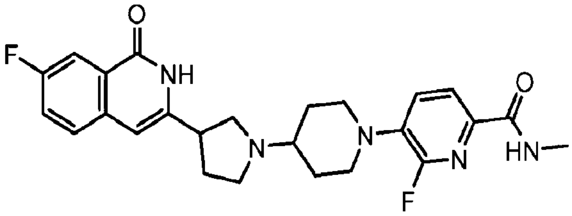

174

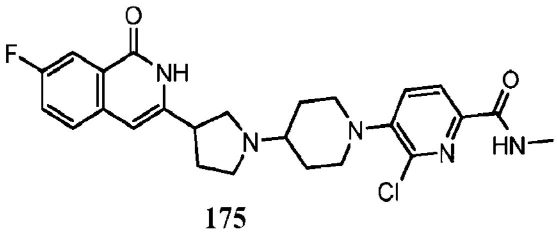

175

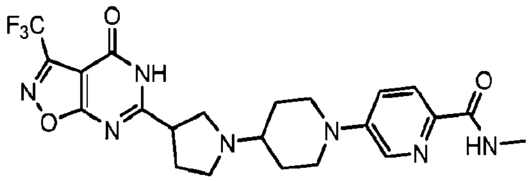

176

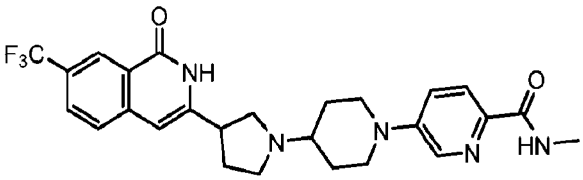

177

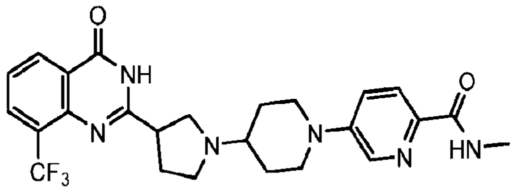

178

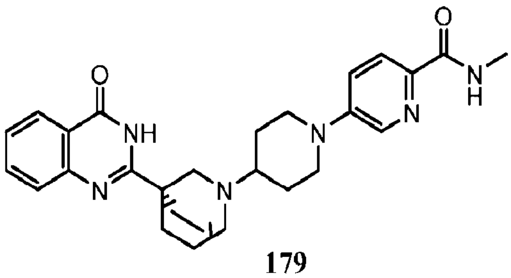

179

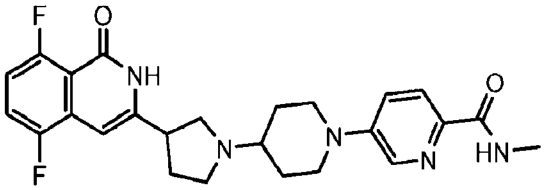

180

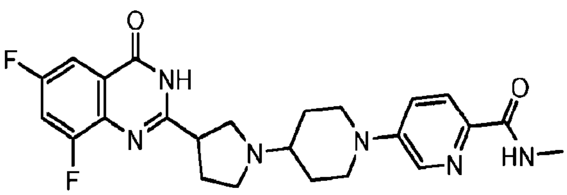

181

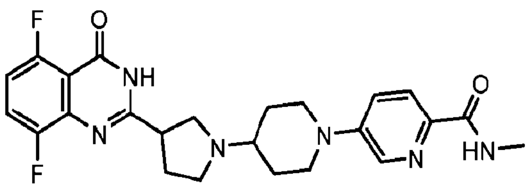

182

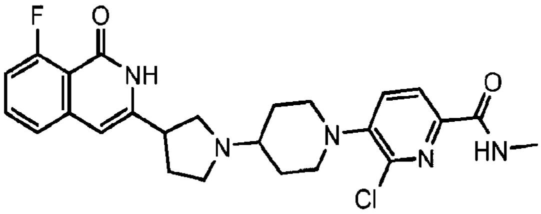

183

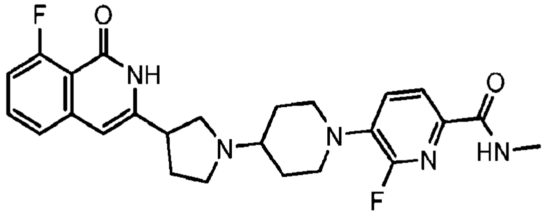

184

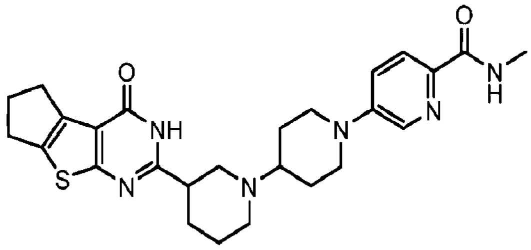

185

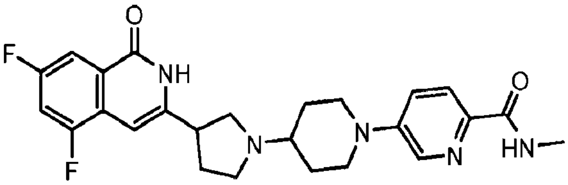

186

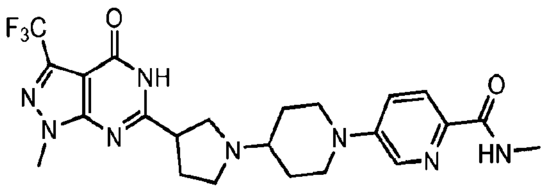

187

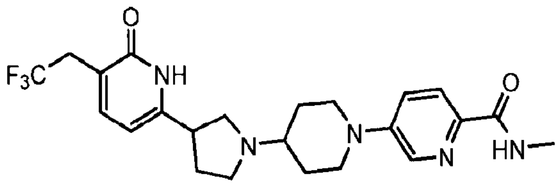

188

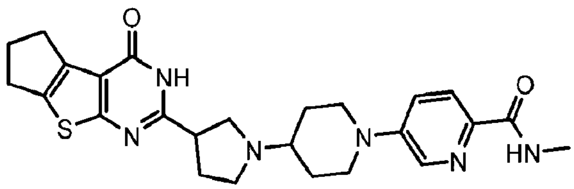

189

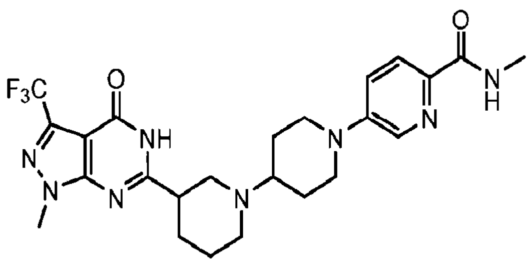

190

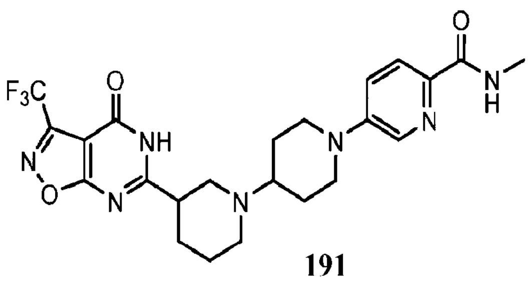

191

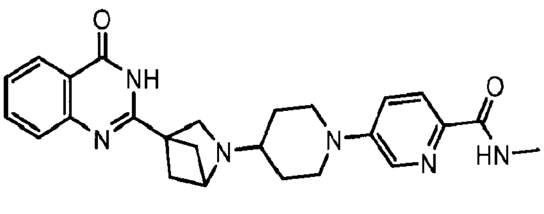

192

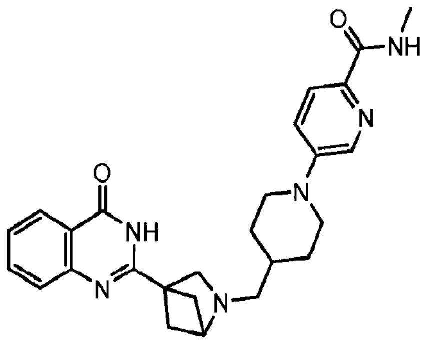

193

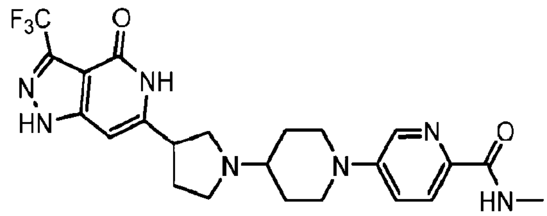

194

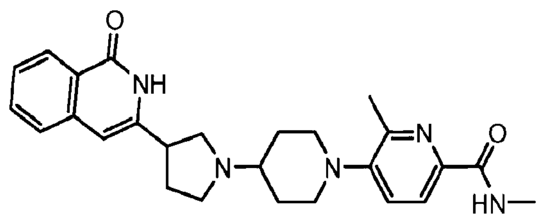

195

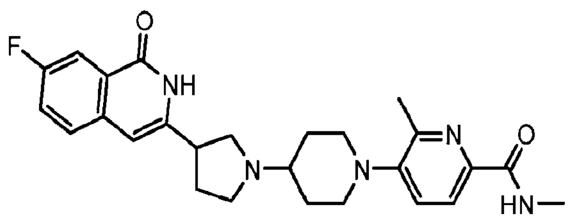

196

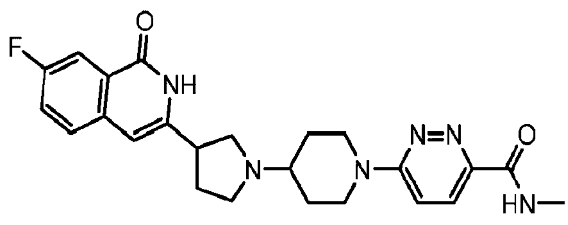

197

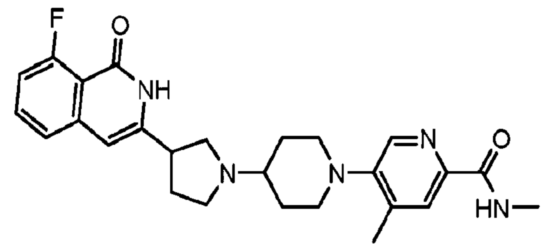

198

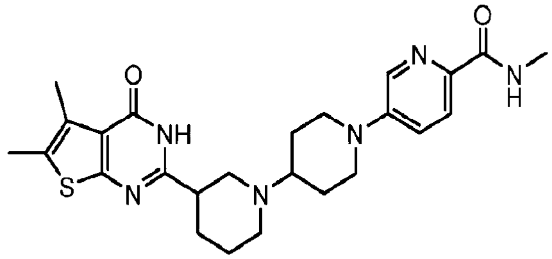

199

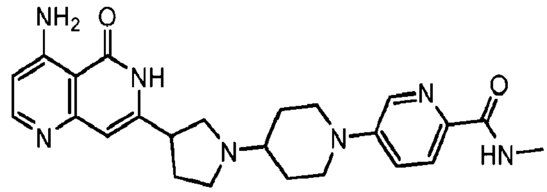

200

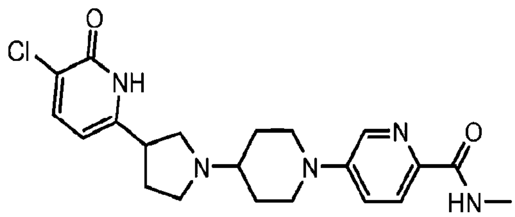

201

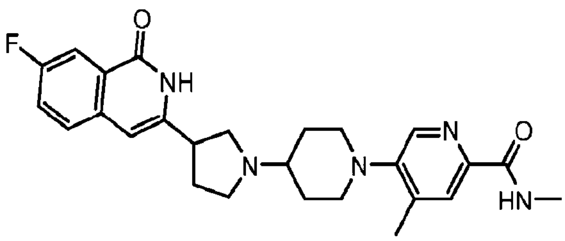

202

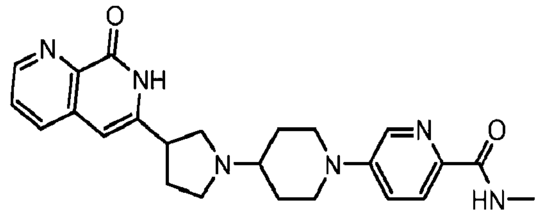

203

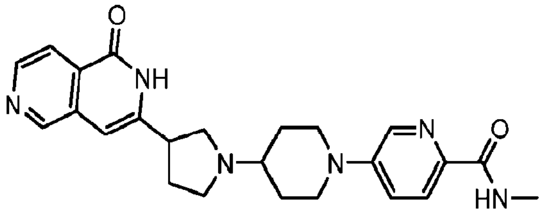

204

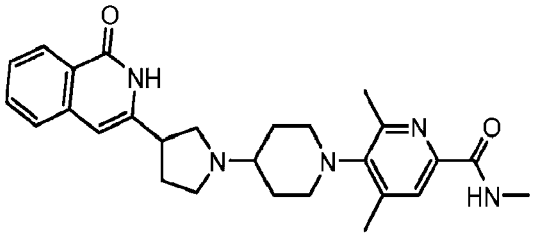

205

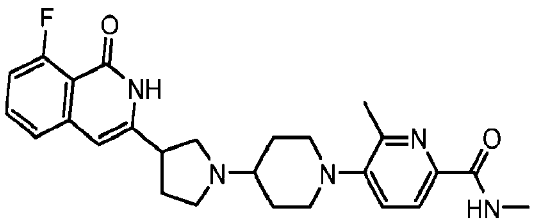

206

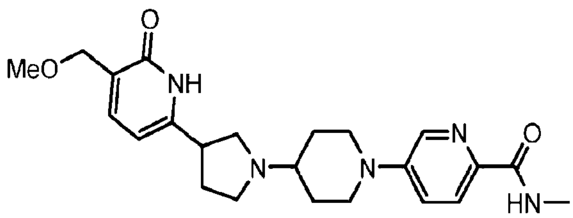

207

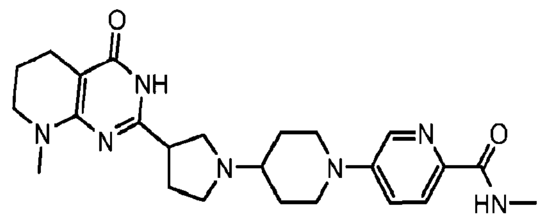

208

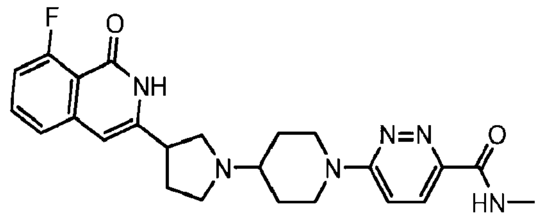

**209**

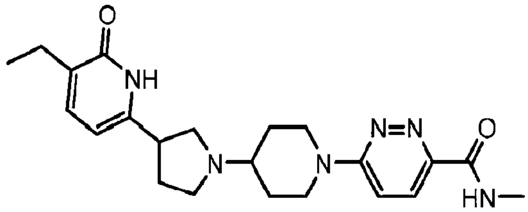

**210**

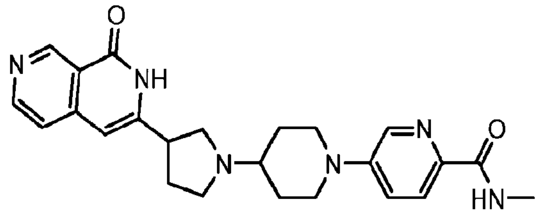

**211**

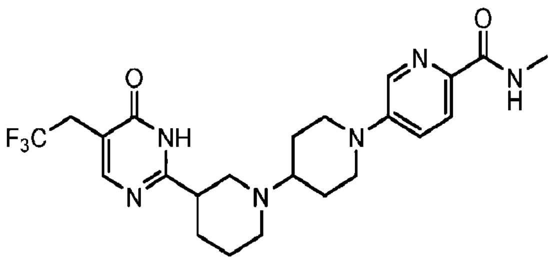

**212**

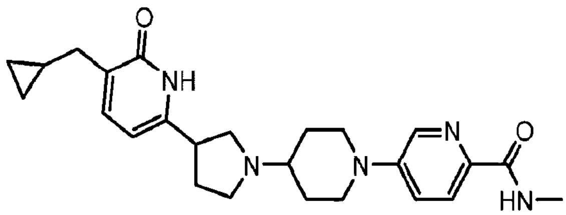

**213**

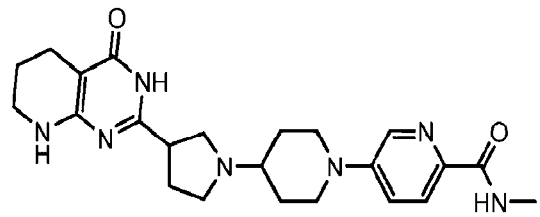

**214**

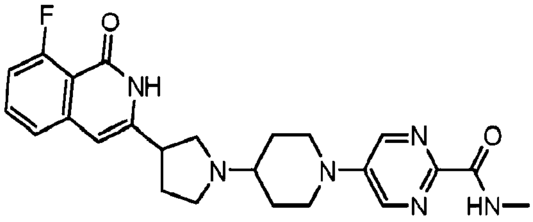

215

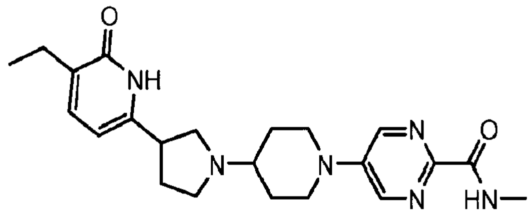

216

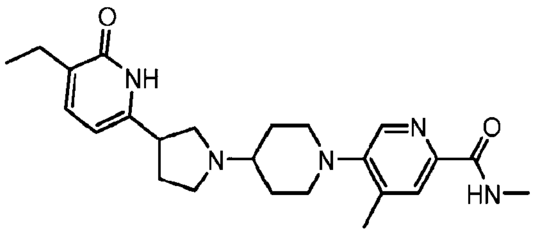

217

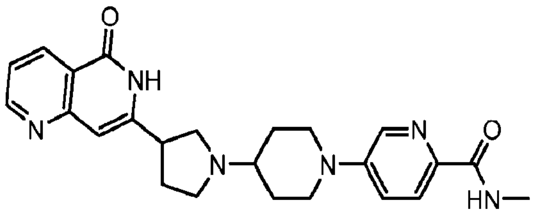

218

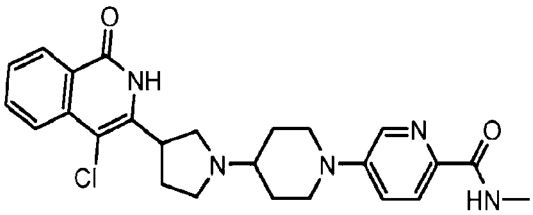

219

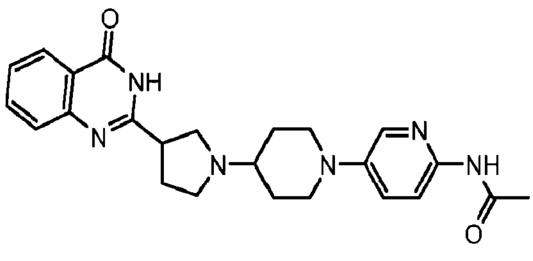

220

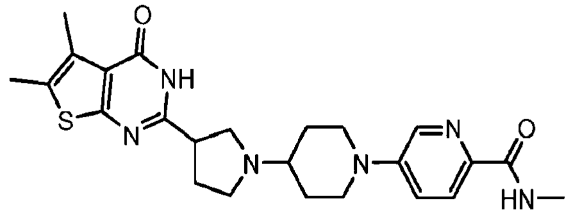

**221**

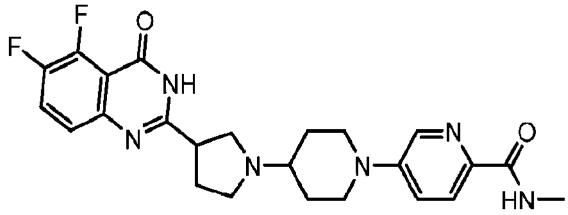

**222**

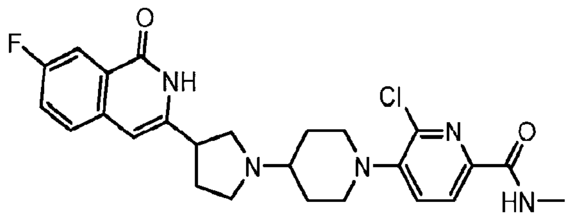

**223**

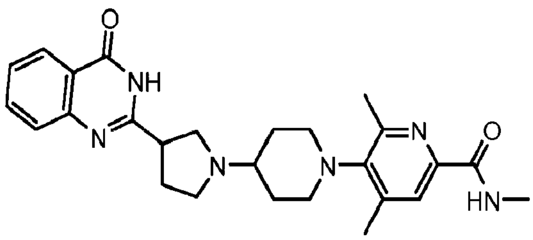

**224**

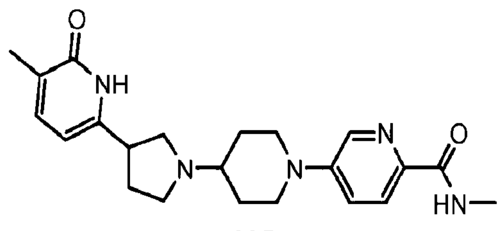

**225**

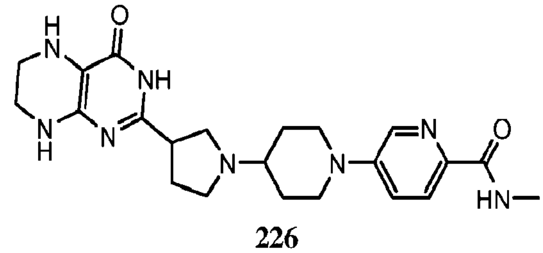

**226**

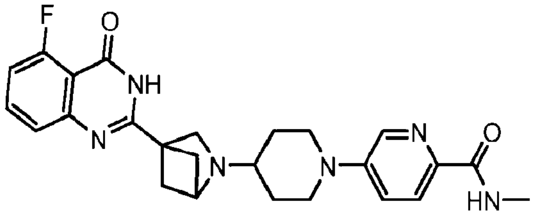

227

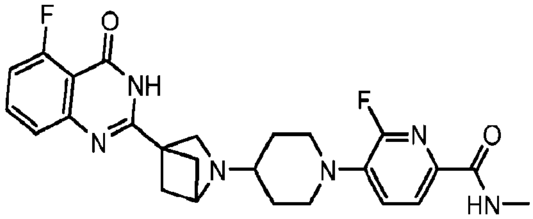

228

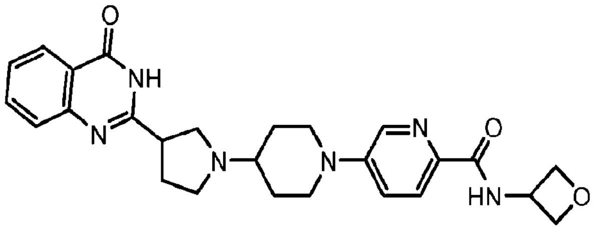

229

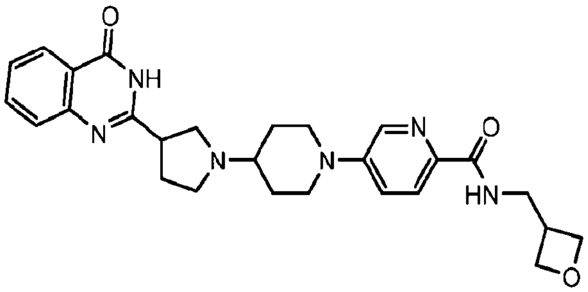

230

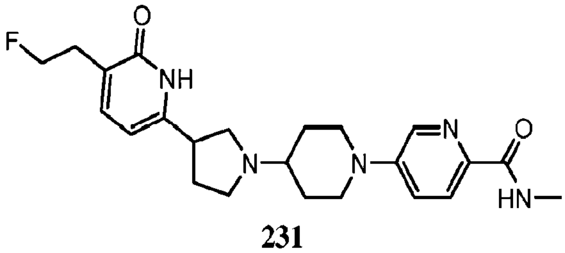

231

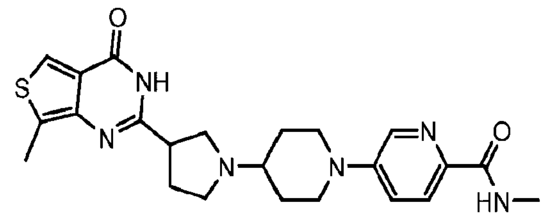

232

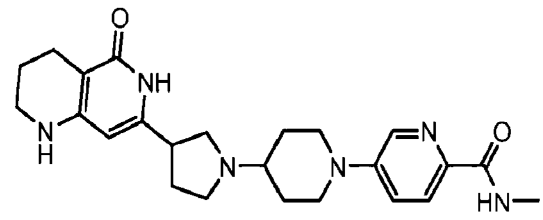

233

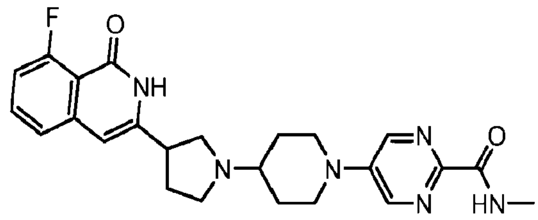

234

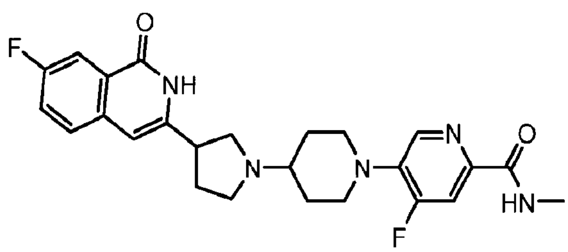

235

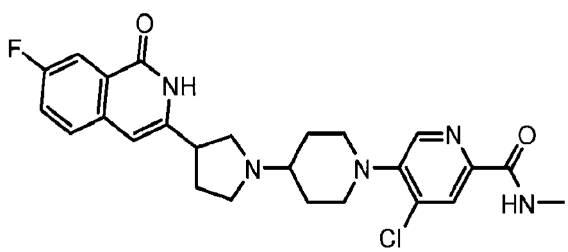

236

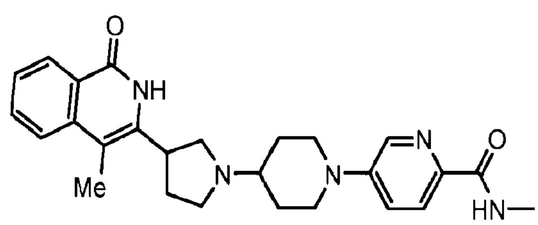

237

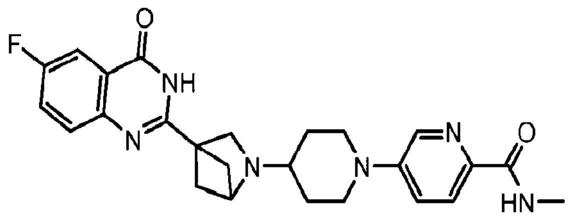

238

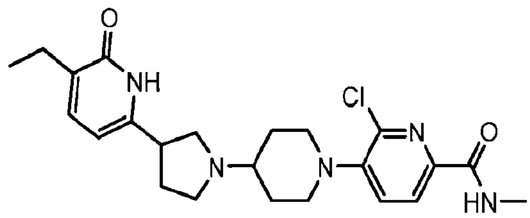

239

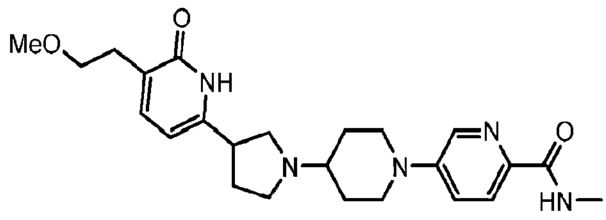

240

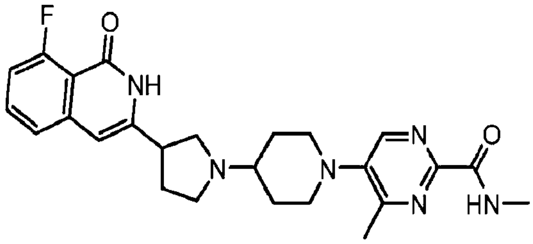

241

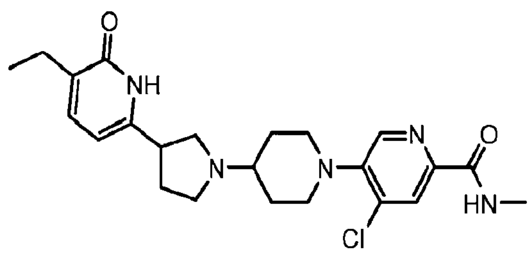

242

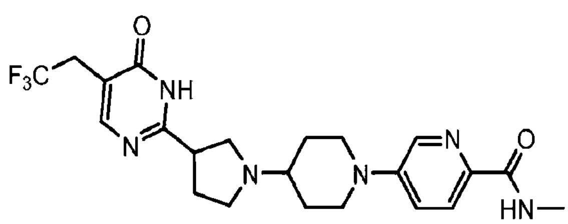

243

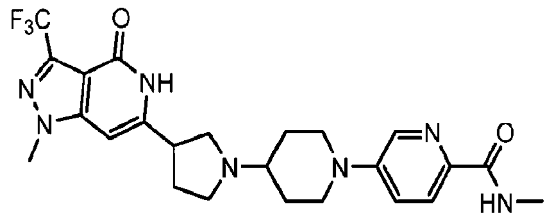

244

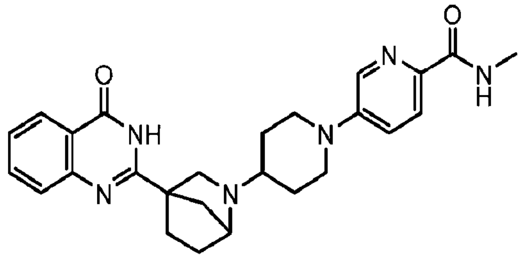

245

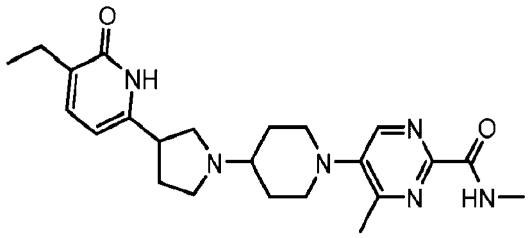

246

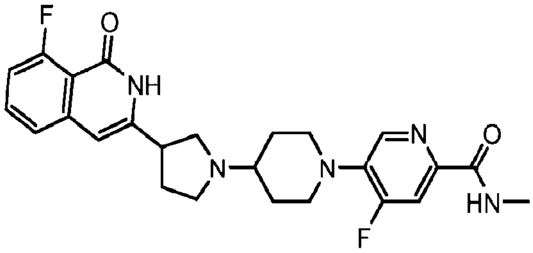

247

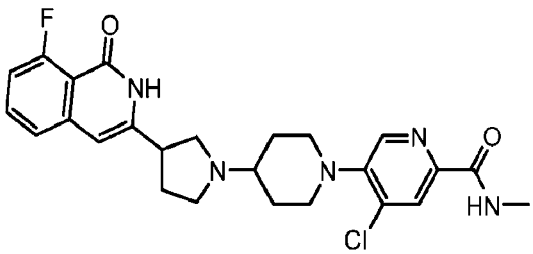

248

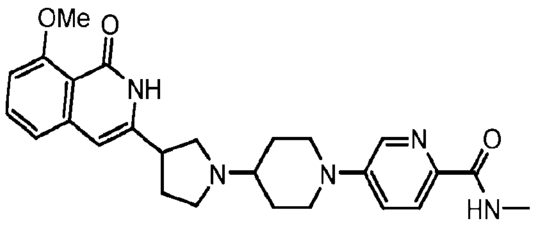

249

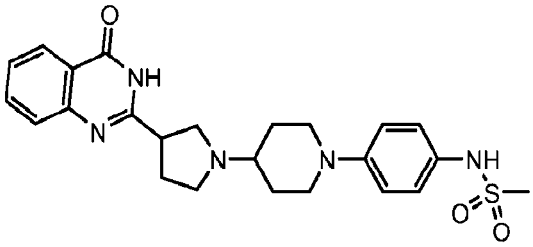

250

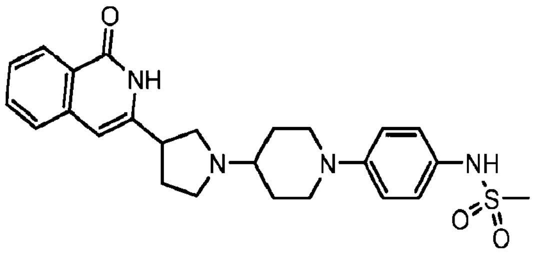

251

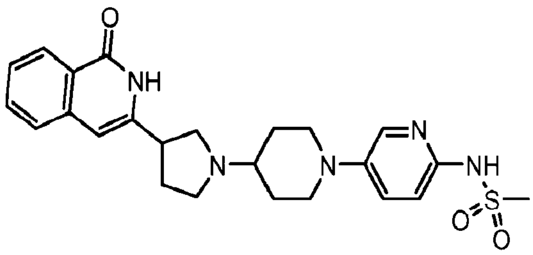

252

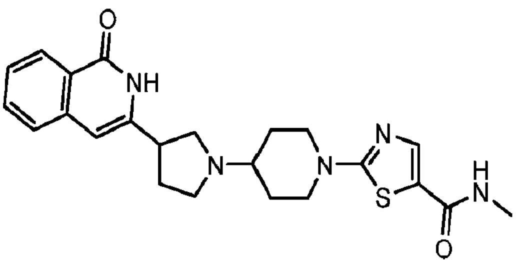

253

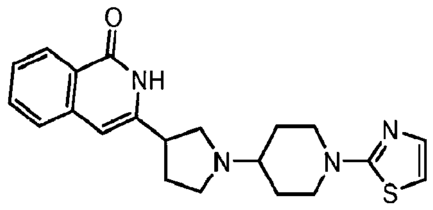

254

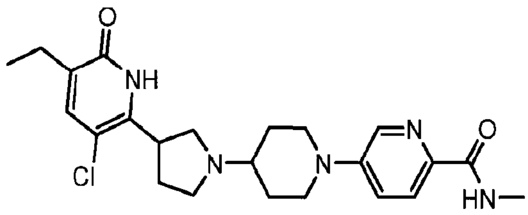

255

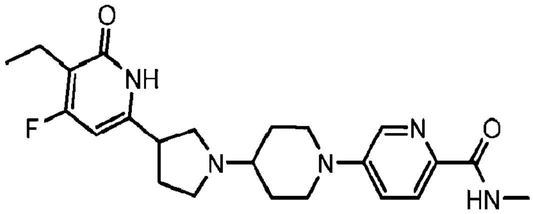

256

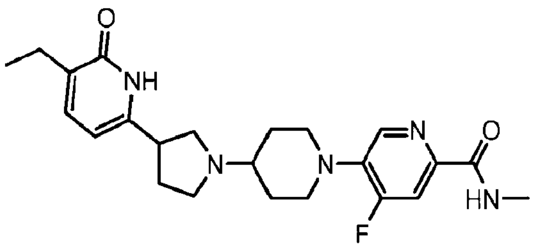

257

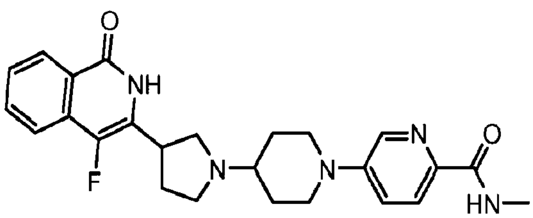

258

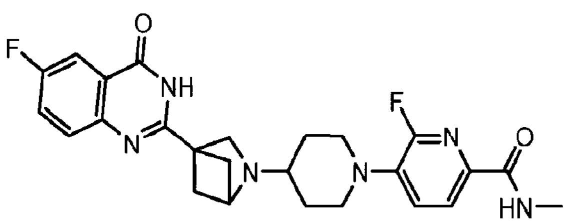

259

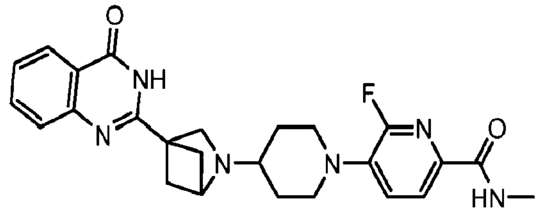

260

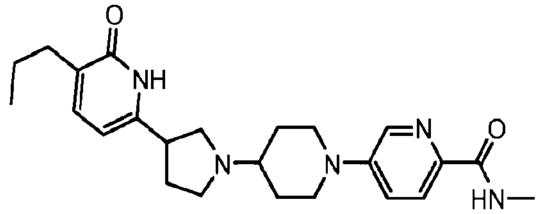

261

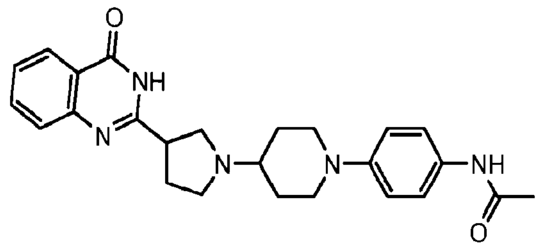

262

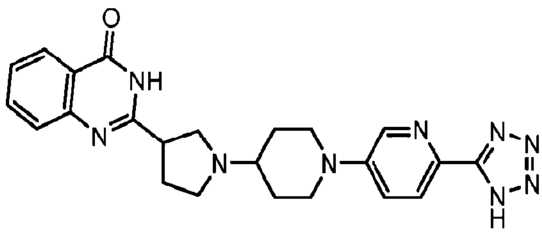

263

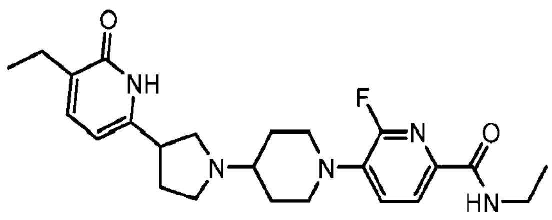

264

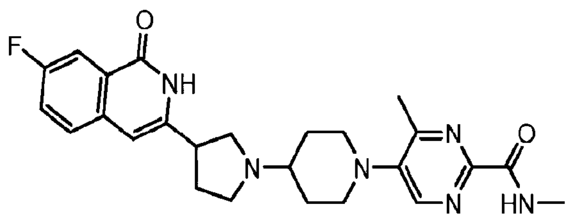

265

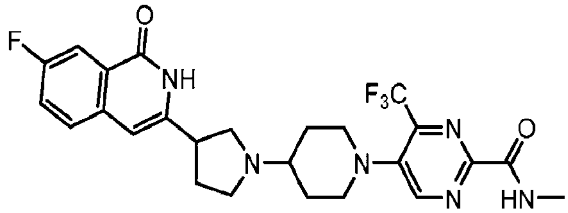

266

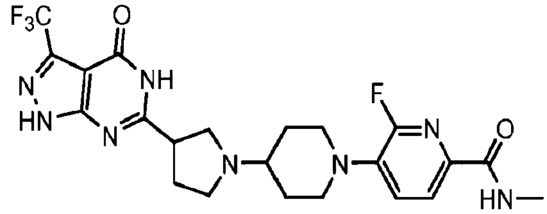

267

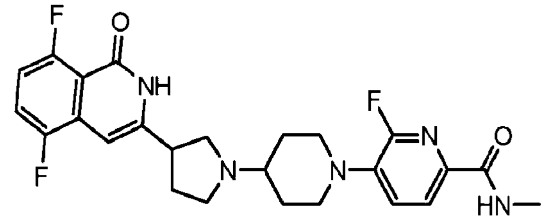

268

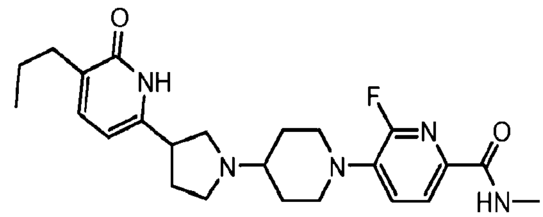

269

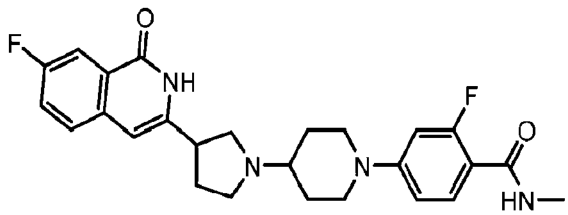

270

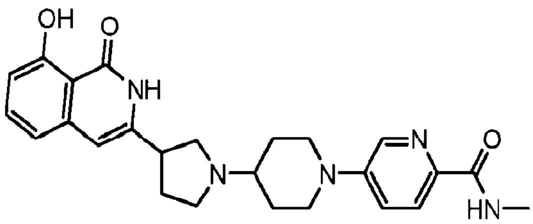

271

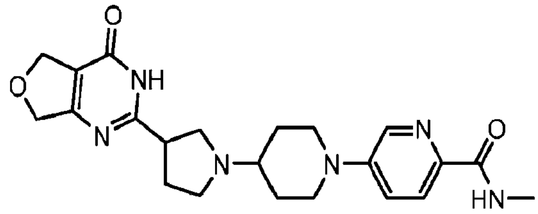

272

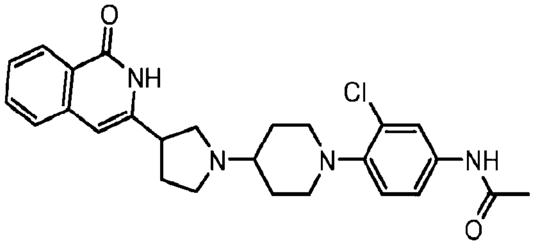

273

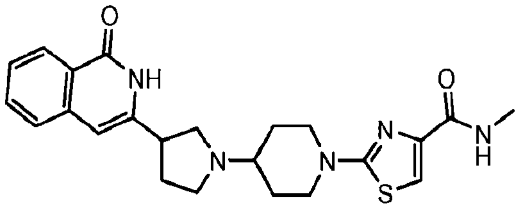

274

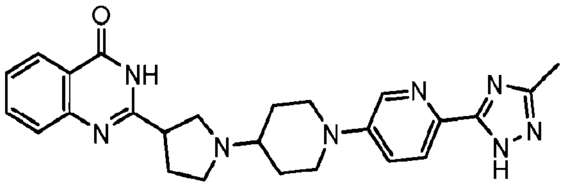

275

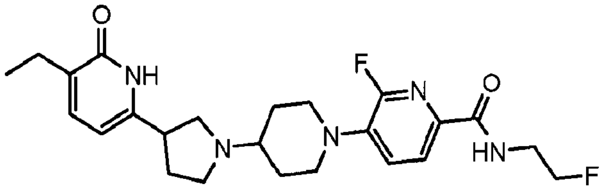

276

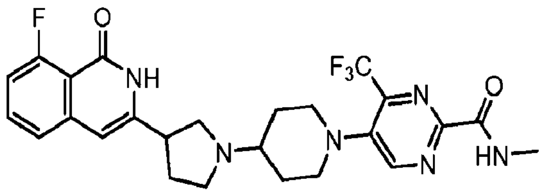

277

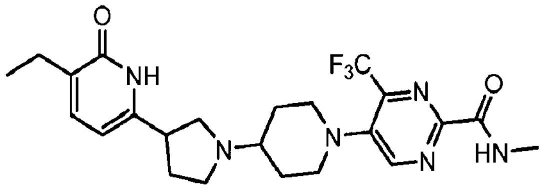

278

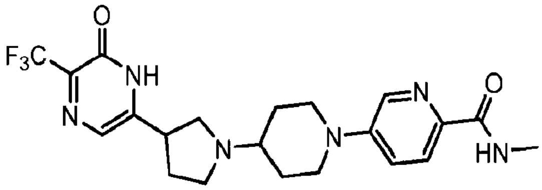

279

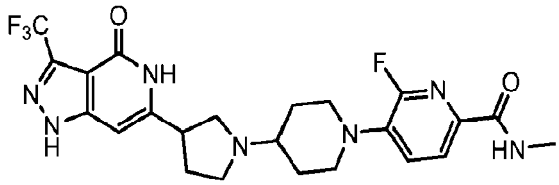

280

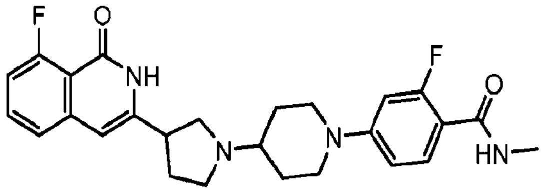

281

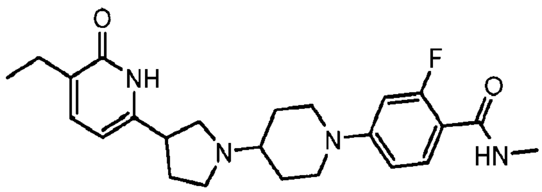

282

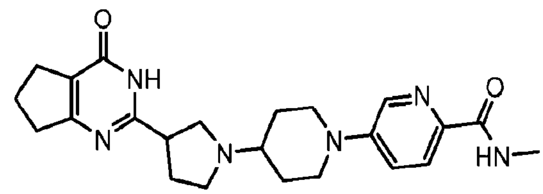

283

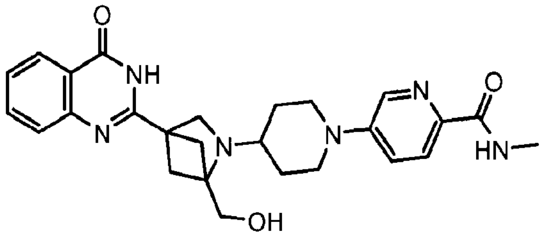

**284**

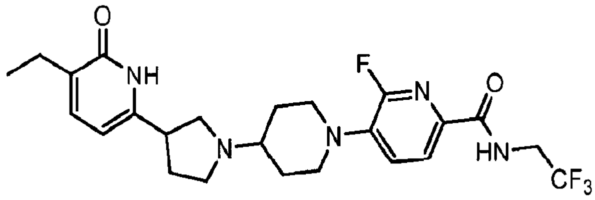

**285**

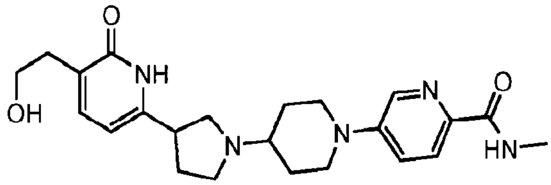

**286**

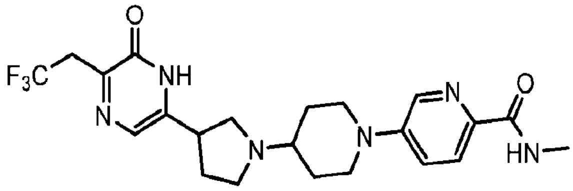

**287**

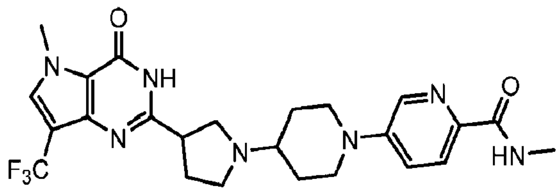

**288**

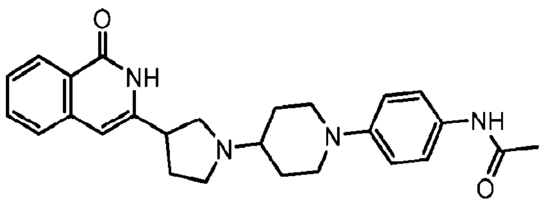

**289**

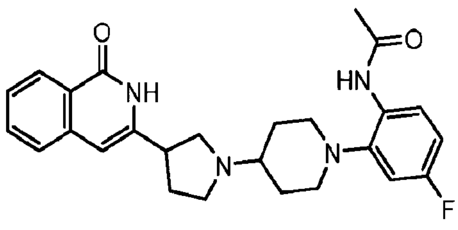

290

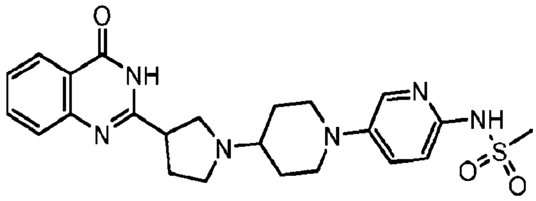

291

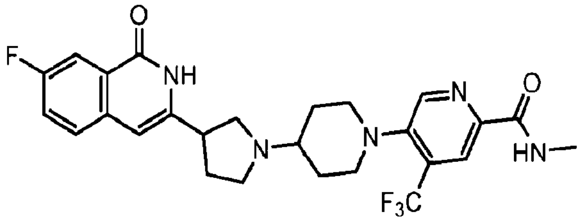

292

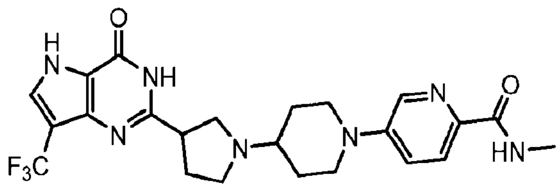

293

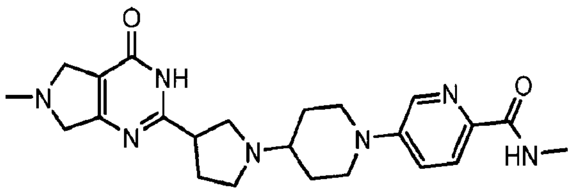

294

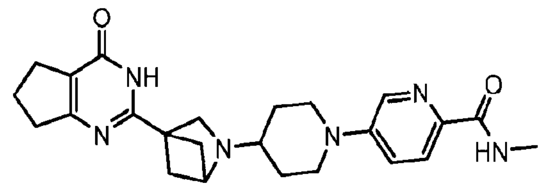

295

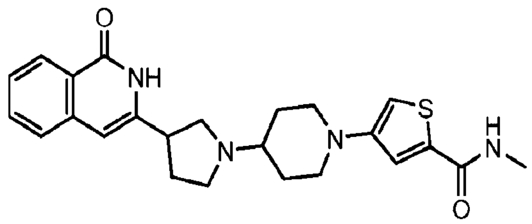

296

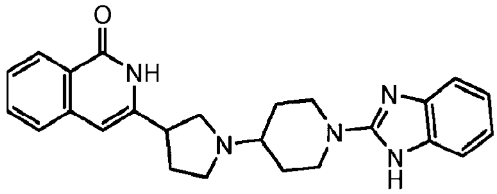

297

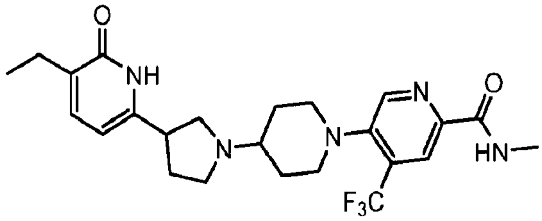

298

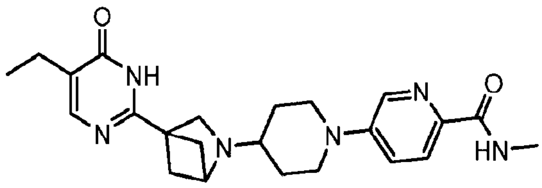

299

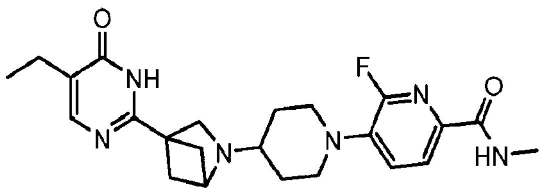

300

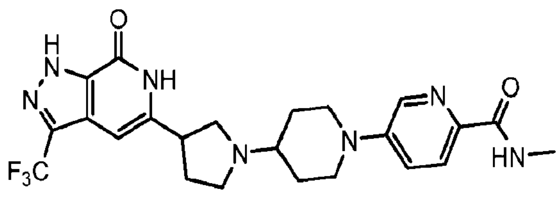

301

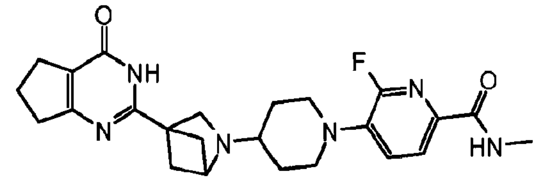

302

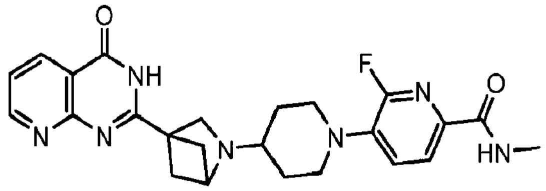

303

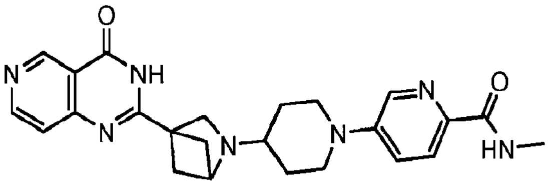

304

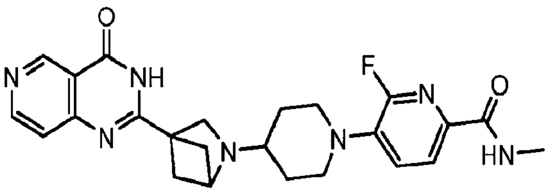

305

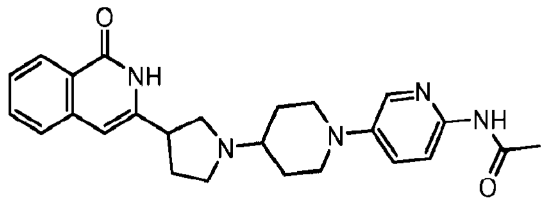

306

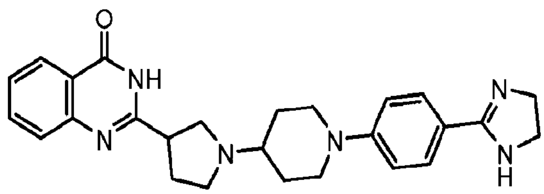

307

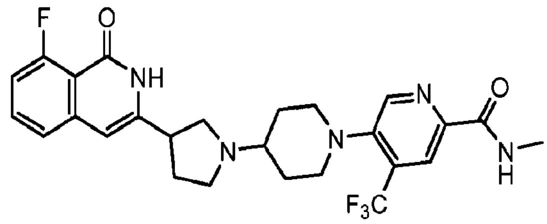

308

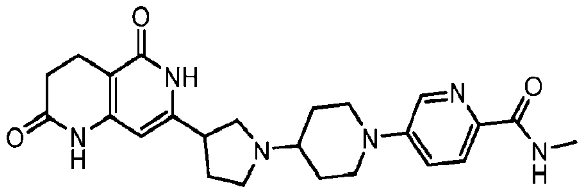

309

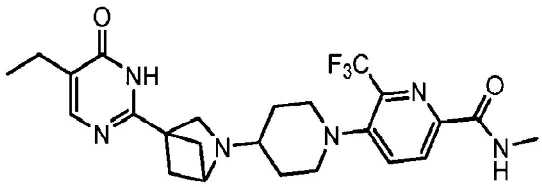

310

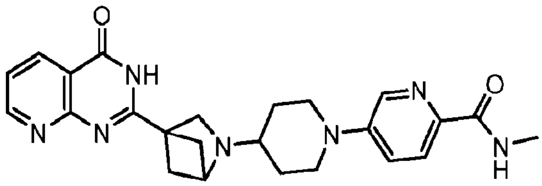

311

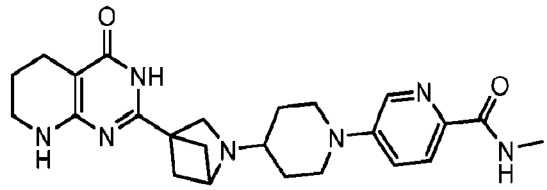

312

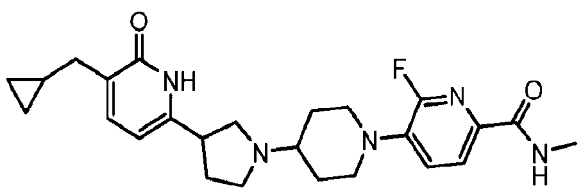

313

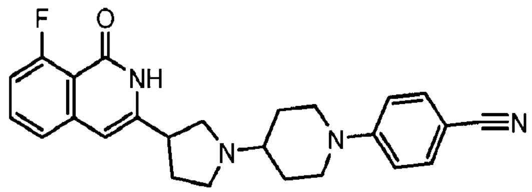

314

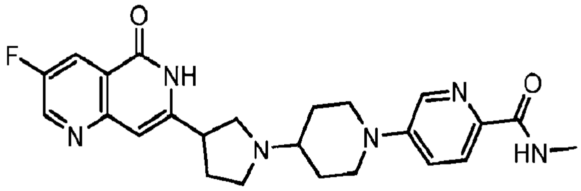

315

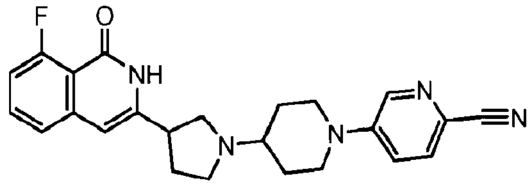

316

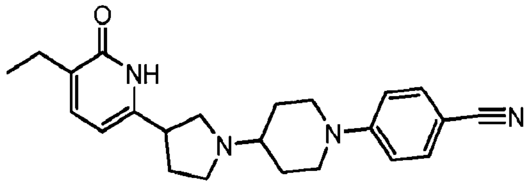

317

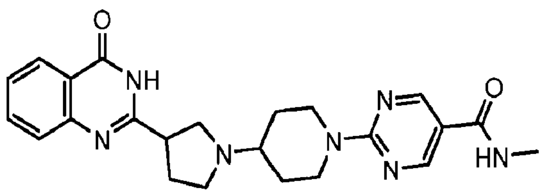

318

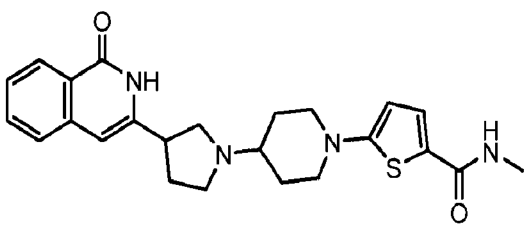

319

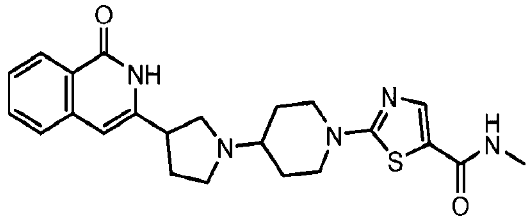

320

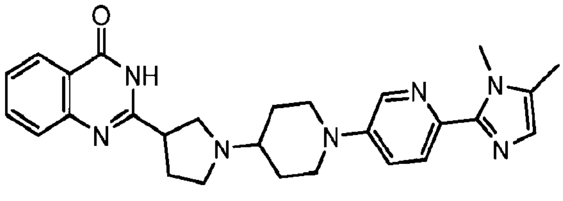

321

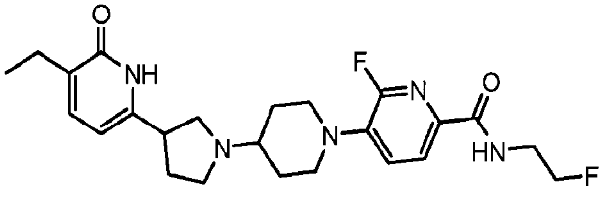

322

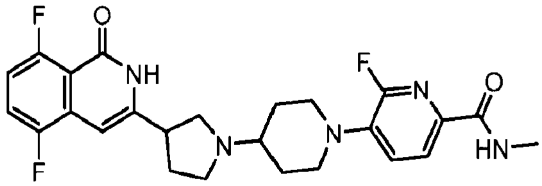

323

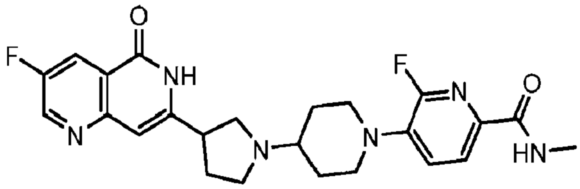

324

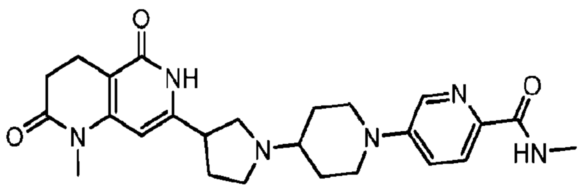

325

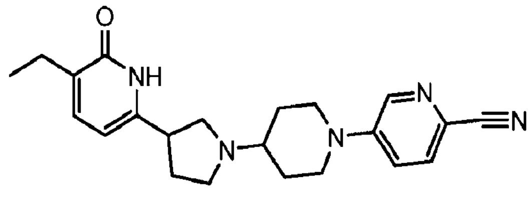

326

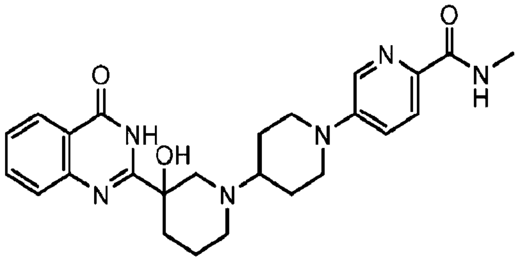

327

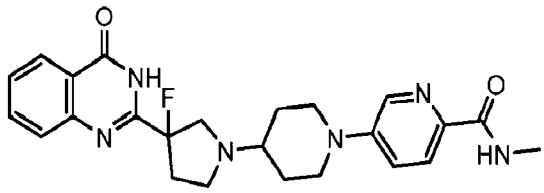

328

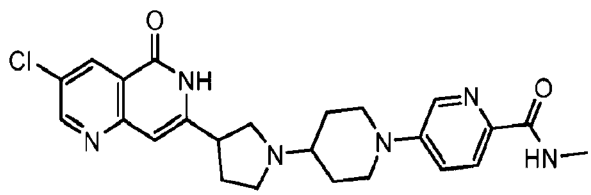

329

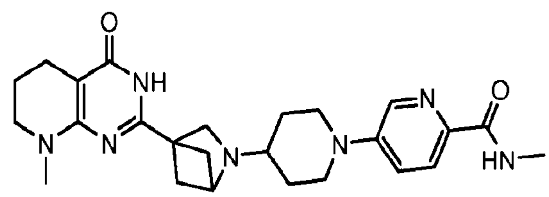

330

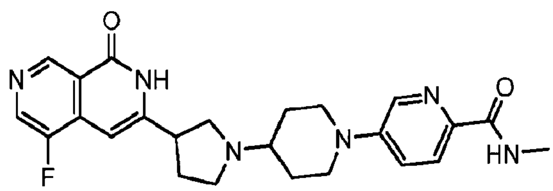

331

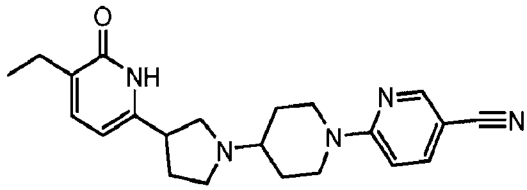

332

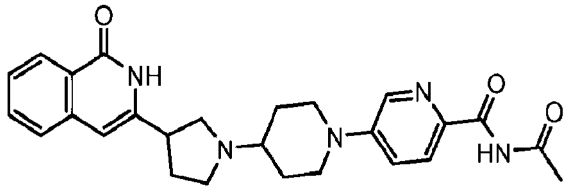

333

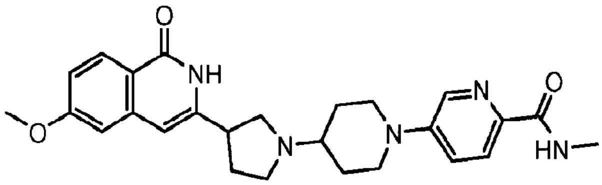

334

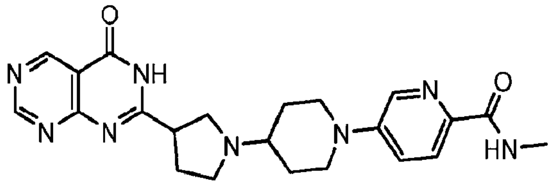

335

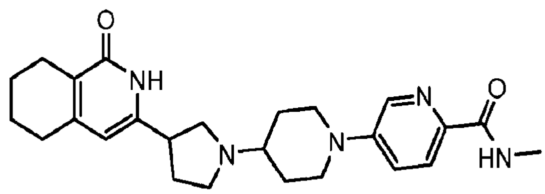

336

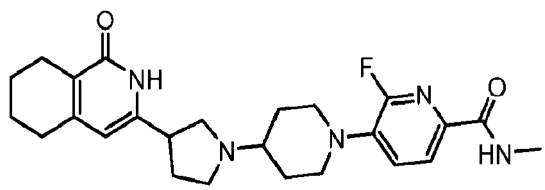

337

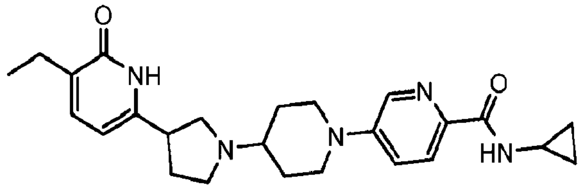

338

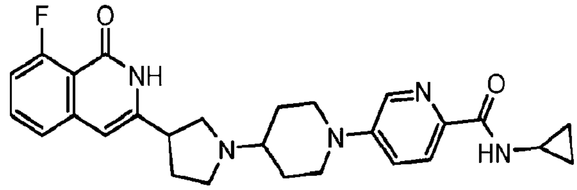

339

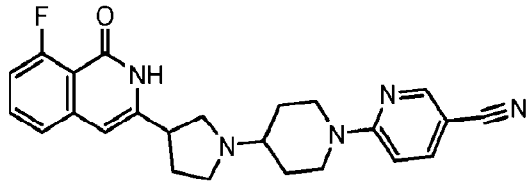

340

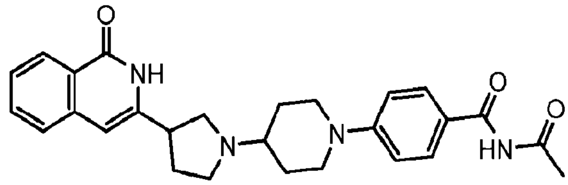

341

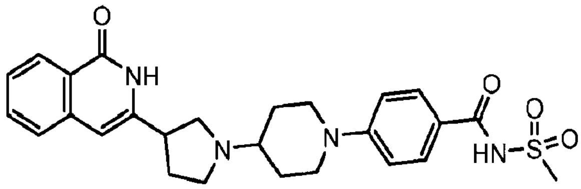

342

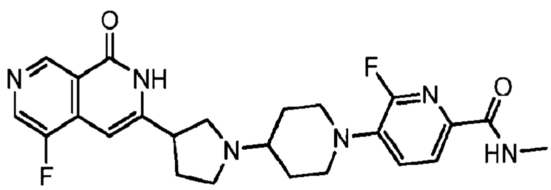

343

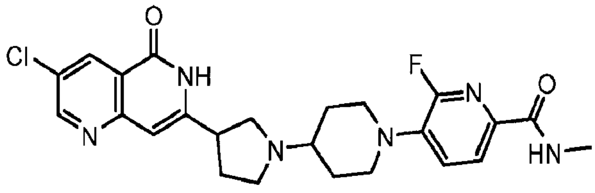

344

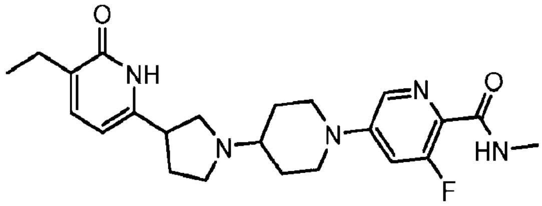

345

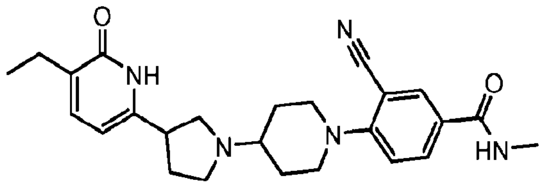

346

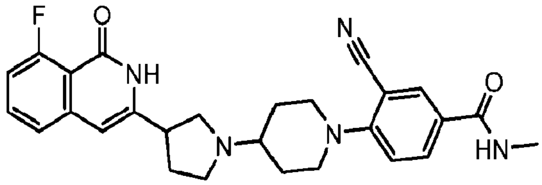

347

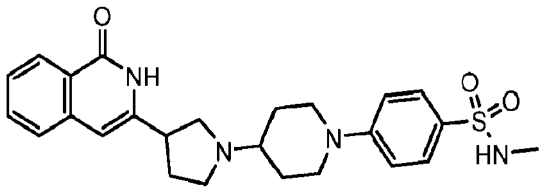

348

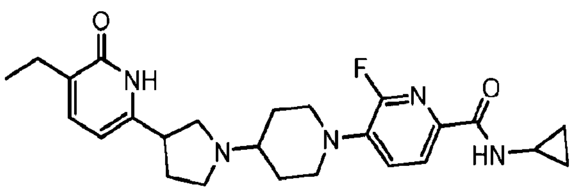

349

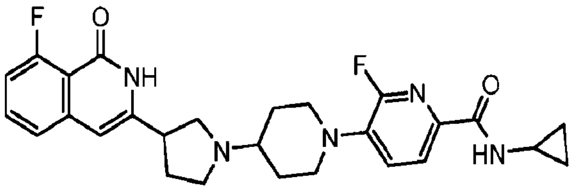

350

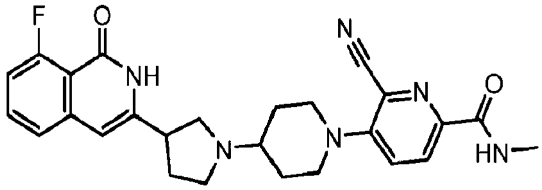

351

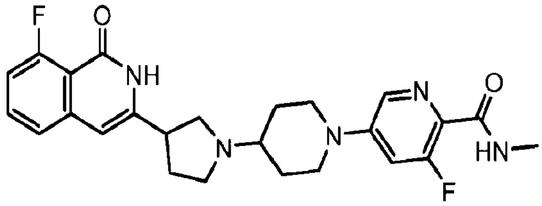

352

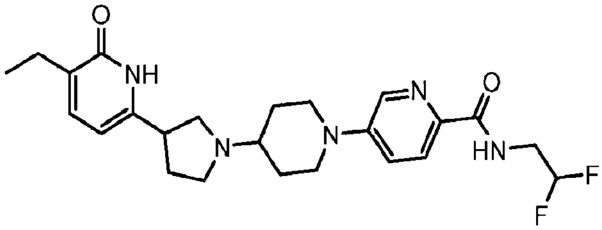

353

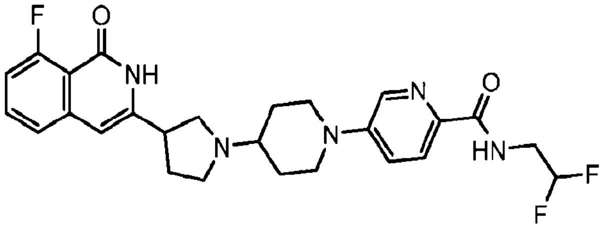

354

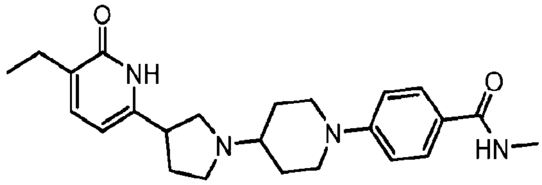

355

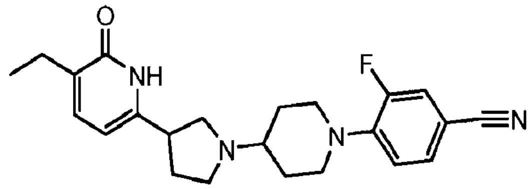

356

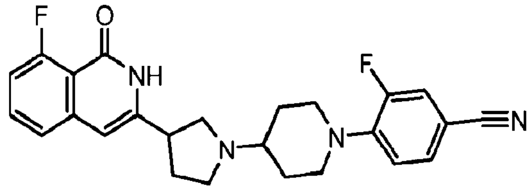

357

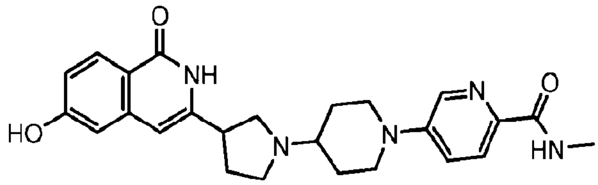

358

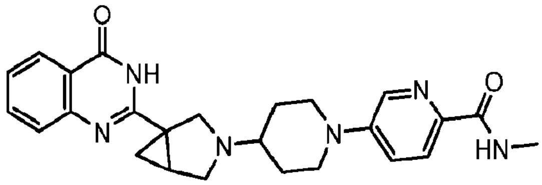

359

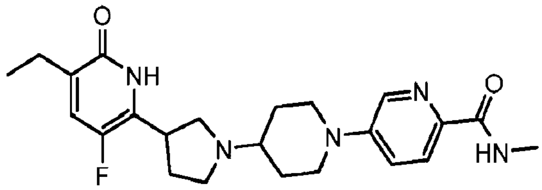

360

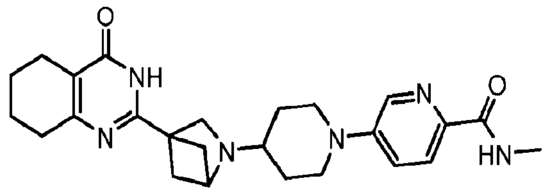

361

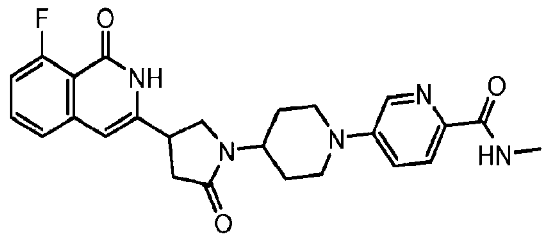

**362**

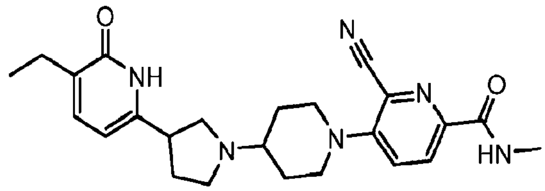

**363**

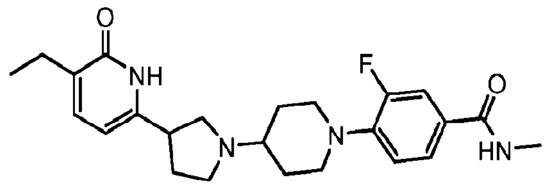

**364**

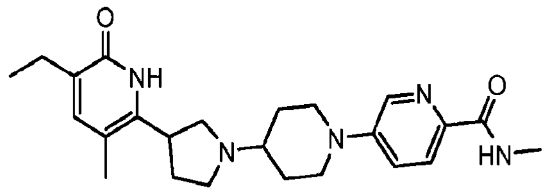

**365**

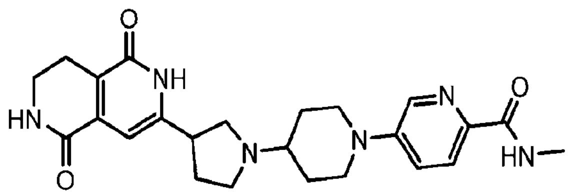

**366**

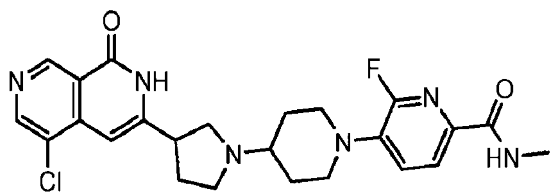

**367**

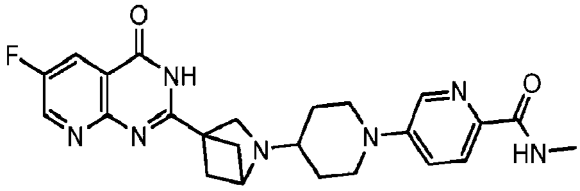

368

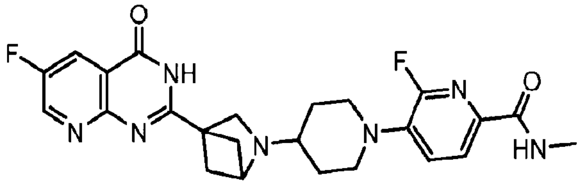

369

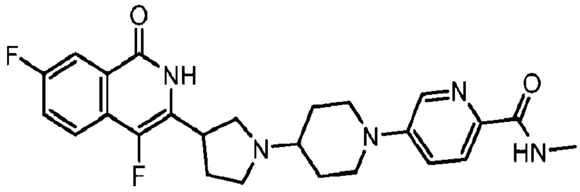

370

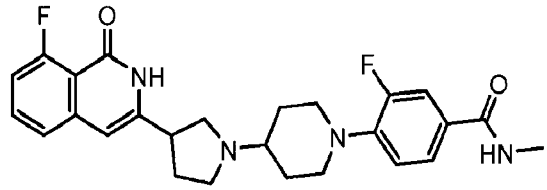

371

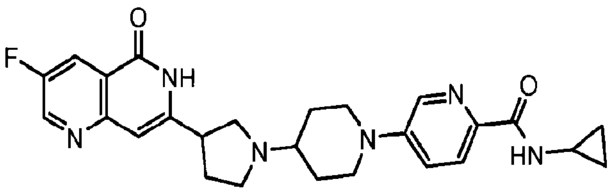

372

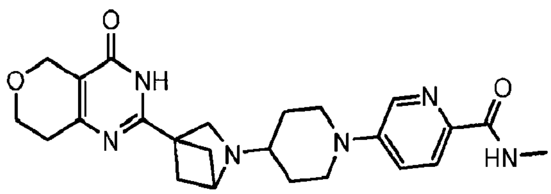

373

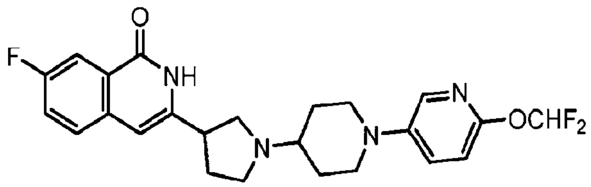

374

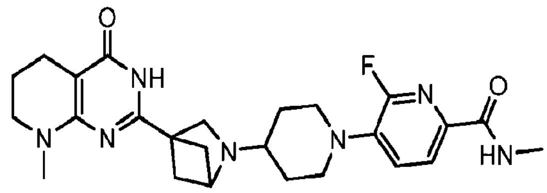

375

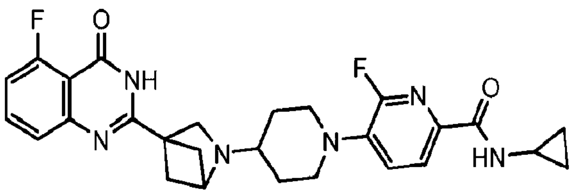

376

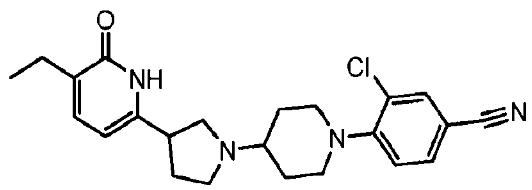

377

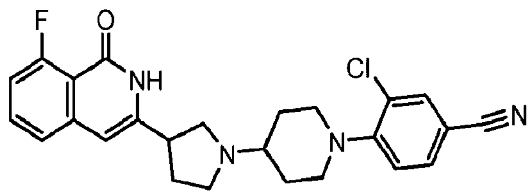

378

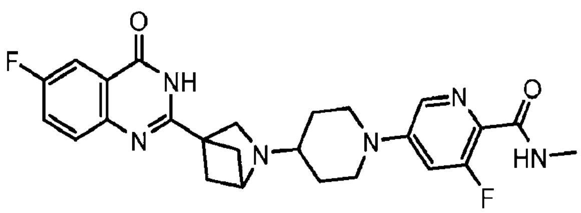

379

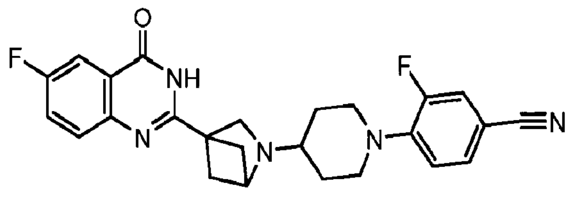

380

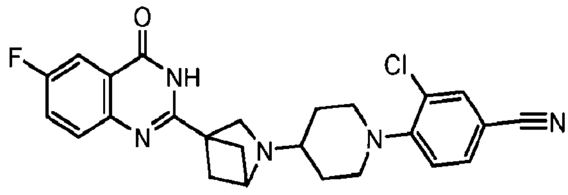

381

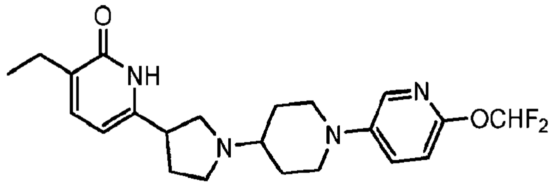

382

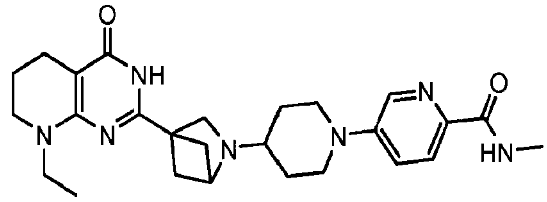

383

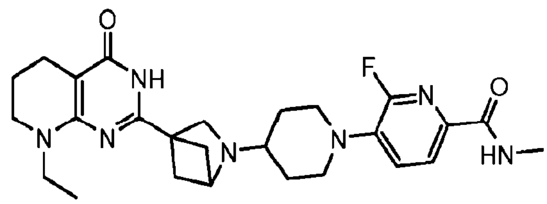

384

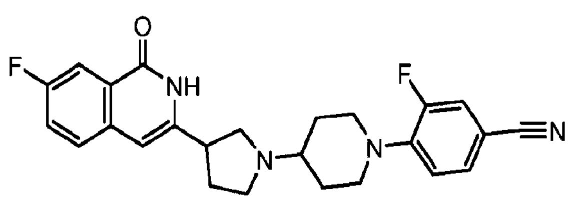

385

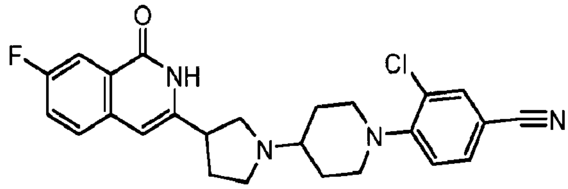

386

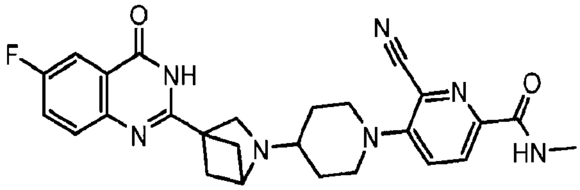

387

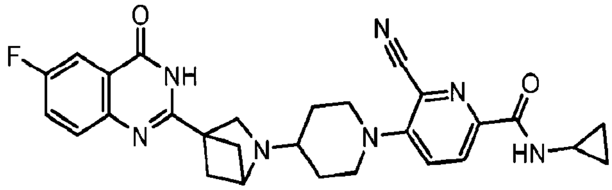

388

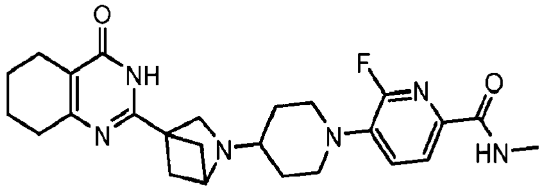

389

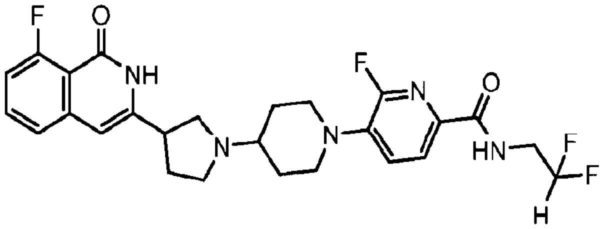

390

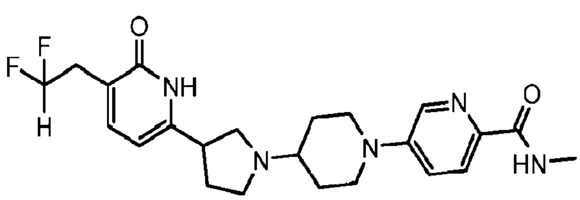

391

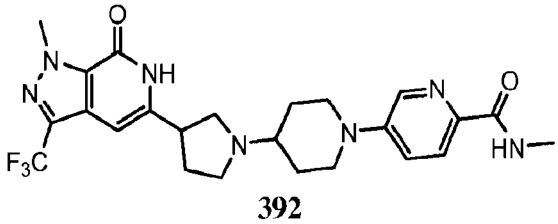

**392**

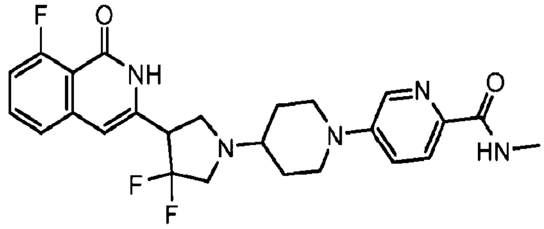

**393**

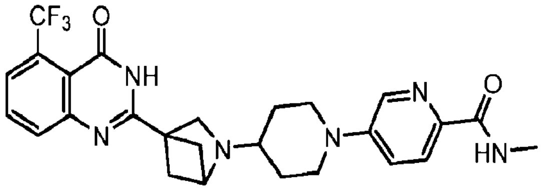

**394**

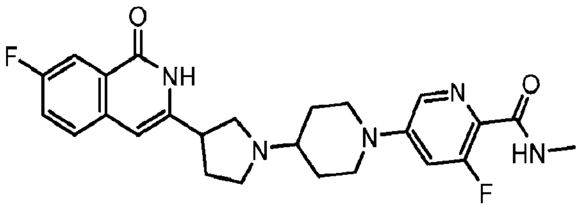

**395**

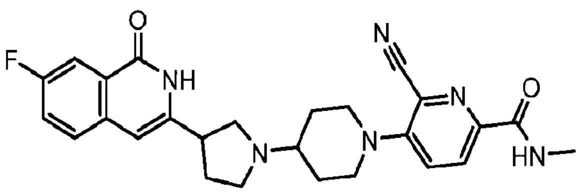

**396**

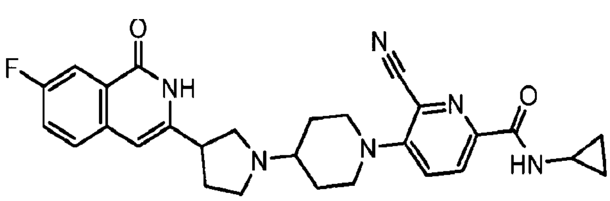

**397**

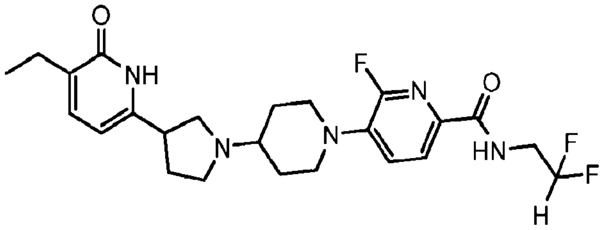

398

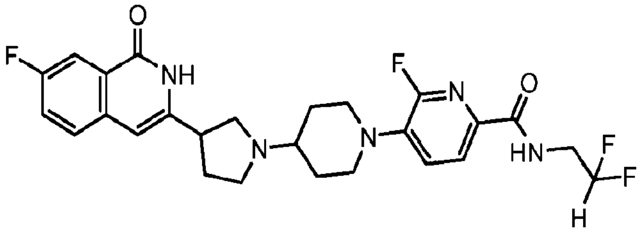

399

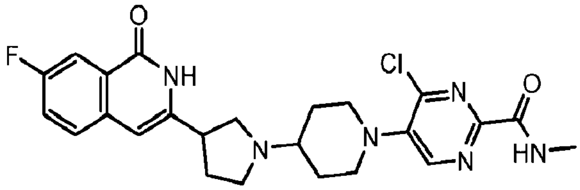

400

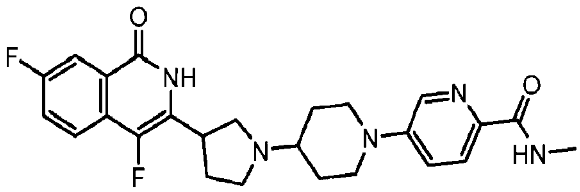

401

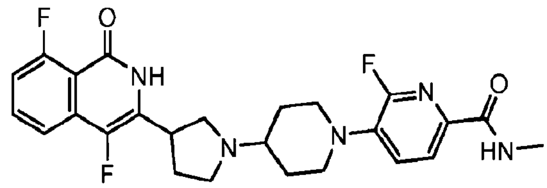

402

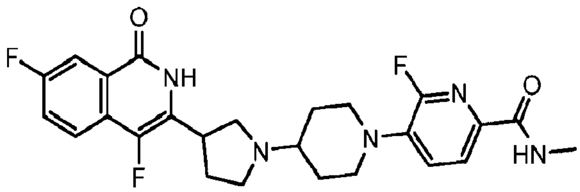

403

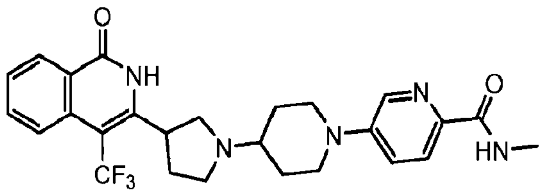

404

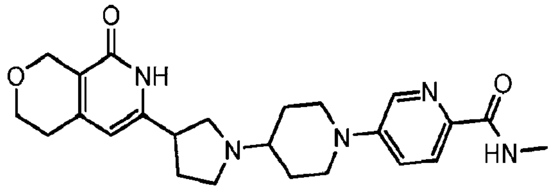

405

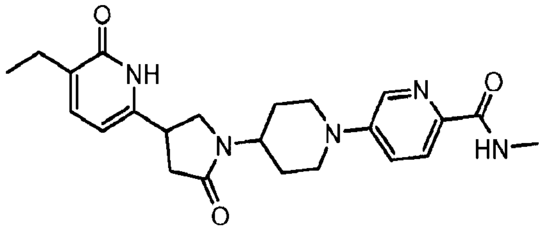

406

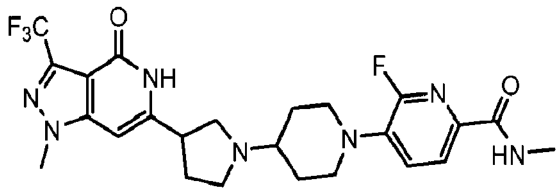

407

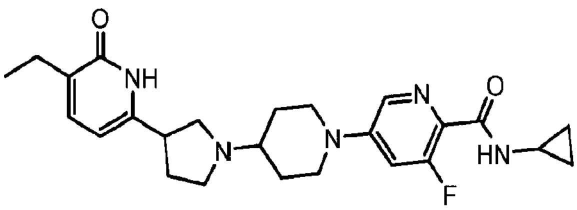

408

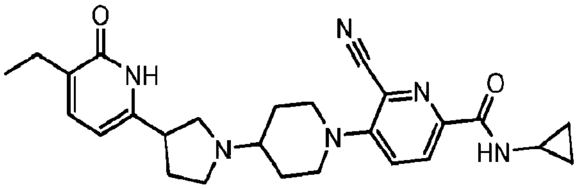

409

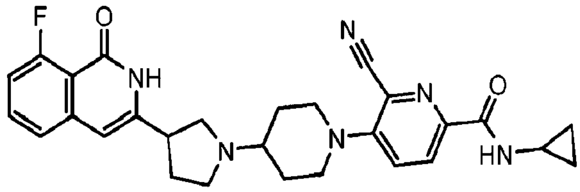

410

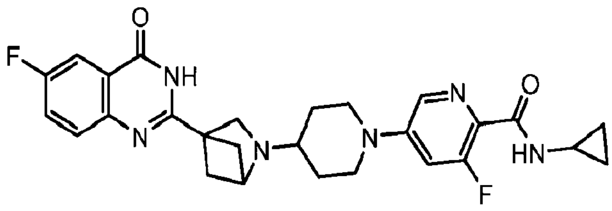

411

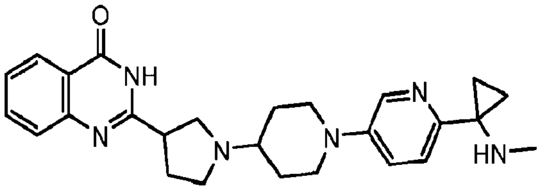

412

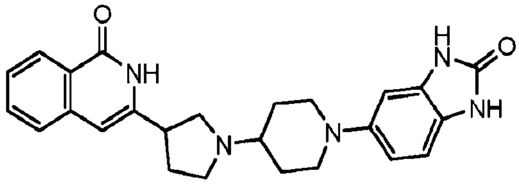

413

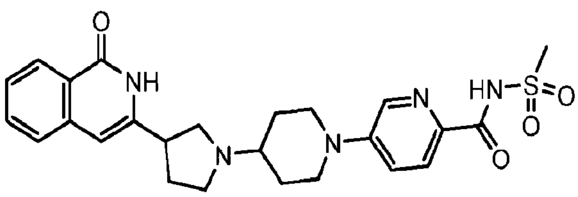

414

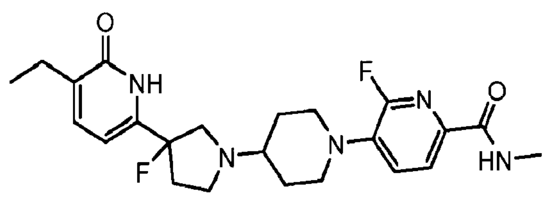

415

The compounds of the present invention have been described in detail above in terms of their structures. For the avoidance of doubt, any compounds for use in the invention may comprise compounds or compositions in accordance with their structure as follows:

- an isolated enantiomer, or
- a mixture of two or more enantiomers, or

- a mixture of two or more diastereomers, and/or epimers, or
- a racemic mixture, or
- one or more tautomers;

of each structure.

For the above numbered compounds, compounds 1, 2, 5, 8, 12, 15, 17 to 22, 25, 27 to 30, 36, 40, 41, 45, 47, 48, 54, 59, 63, 75, 168, 173, 179, 192, 193, 227, 228, 238, 259, 260, 284, 295, 299, 300, 302-305, 310-312, 330, 361, 368, 369, 373, 375, 376, 379-381, 383, 384, 387-389, 394 and 411 are achiral. The remaining compounds represent more than one enantiomeric structure which may have PARP1 inhibitory activity as a racemic mixture and/or as a separated enantiomer(s). In the examples below, a compound with a suffix "a" (eg 10a) represents an enantiomer eluted as a first fraction when a racemic mixture of the two enantiomers is applied to a Daicel CHIRALPAK chiral chromatography column. In the examples below, a compound with a suffix "b" (eg 10b) represents an enantiomer eluted as a second fraction when a racemic mixture of the two enantiomers is applied to a Daicel CHIRALPAK chiral chromatography column. In the examples below, a compound with no suffix represents either an achiral compound or a racemic mixture of the enantiomers. In the examples below, a compound which bears a suffix "rac" represents a racemic mixture of the enantiomers.

The compounds described herein may be provided for use in medicine. In the context of the present invention, the medicinal use is not especially limited, provided that it is a use which is facilitated by the PARP1 inhibitory effect of the compound. Thus, the compounds of the invention may be for use in any disease, condition or disorder that may be prevented, ameliorated or treated using a PARP1 inhibitor. Typically, this comprises a disease condition and/or a disorder selected from: a cancer, it is not especially limited, provided that the cancer is one which may be treated, prevented or ameliorated by using a PARP1 inhibitor. Thus the cancer may be a cancer selected from: a solid or liquid tumour including cancer of the eye, brain (such as gliomas, glioblastomas, medullablastomas, craniopharyngioma, ependymoma, and astrocytoma), spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer , an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes (PHTS) (such as Lhermitte-Duclos disease, Cowden syndrome, Proteus syndrome, and Proteus-like syndrome), leukaemias and lymphomas (such as acute lymphoblastic leukaemia, chronic lymphocytic leukaemia, acute myelogenous leukaemia, chronic myelogenous leukaemia, hairy cell leukaemia, T-cell prolymphocytic leukemia (T-PLL), large granular lymphocytic leukemia, adult T-cell leukemia, juvenile myelomonocytic leukaemia, Hodgkin lymphoma, non-Hodgkin lymphoma, mantle lymphoma, follicular lymphoma, primary effusion lymphoma, AIDS-related lymphoma, Hodgkin lymphoma, diffuse B cell lymphoma, Burkitt lymphoma, and cutaneous T-cell lymphoma). In addition, the compounds described herein may be of use in cancers where EBV plays a contributing role such as Burkitt's lymphoma, Hodgkin's lymphoma, nasopharyngeal and gastrointestinal cancers.

The compounds described herein may be provided for use in for treating a cancer which is deficient in DNA damage response repair pathways, in particular in Homologous Recombination dependent DNA DSB DNA repair activity. Components of HR dependent DNA DSB repair pathways and other DNA damage response pathways include but are not limited to the following proteins: ATM, ATR, ERCC1, XRCC1, XRCC2, XRCC3, RAD51, RAD51L1, RAD51C, RAD51D, RAD51L3, DMC1, RAD52, RAD54L, RAD54B, RAD50, MRE11A, NBS1, BRCA1, BRCA2, FANCP (SLX4), FEN1, PALB2, PBRM1, SMARCA4, ARID1A, ARID1B, FANCD2, BLM. Other components involved in HR dependent DNA DSB repair include regulatory factors such as ESMY (Hughes-Davies, L. et al. Cell. 2003; 115: 523-535). A cancer which is deficient in HR-dependent DNA DSB repair typically becomes dependent on alternative DSB pathway repair mechanisms. Such cancers include but are not limited to cancers of the ovary, prostate, breast, lung, gastrointestine, blood and pancreas.

In some embodiments, the cancer cells may have a BRCA1 and/or BRCA2 deficient phenotype ic they may be deficient in BRCA1 and/or 2 expression and function by means of mutation, polymorphism or epigenetic silencing in the encoding nucleic acids or by means of amplification, polymorphism, mutation in a gene encoding a regulatory factor eg, the ESMY gene which encodes a BRCA2 regulatory factor (Hughes-Davies, L. et al. Cell. 2003; 115: 523-535). Amplification of the ESMY gene is associated with breast and ovarian cancer. Carriers of mutations in the tumour suppressor BRCA1 and/or BRCA2 genes are known to have an elevated risk of developing certain cancers including ovarian, prostate and breast. Wild-type alleles of BRCA1 and/or BRCA2 are frequently lost in tumours of heterozygous carriers (Jasin, M. et al. Oncogene. 2002; 21: 8981-93) and their detection, as a means of patient selection, is well known in the art (Radice, PJ. et al. Exp. Clin. Cancer. Res. 2002; 21: 9-12; Chappnis, PO and Foulkes WO. Cancer Treat Res. 2002; 107: 29-59).

In some embodiments, the compounds described herein are selective PARP1 inhibitors, as defined above. Selective inhibition of PARP1 over PARP2 reduces PARP2 associated side effects including one or more haematological toxicities such as anaemia, neutropenia and thrombocytopenia. This enables treatment of cancer patients with reduced

haematological side effects. This also enables higher doses of PARP1 inhibitors to be administered to patients and for such inhibitors to be administered in combination with chemotherapeutic agents.

The present invention also provides a pharmaceutical composition comprising a compound as defined above. Whilst the pharmaceutical composition is not especially limited, typically the composition further comprises a pharmaceutically acceptable additive and/or excipient. In the pharmaceutical composition, the compound as defined above may be present in the form described above, but may alternatively be in a form suitable for improving bioavailability, solubility, and/or activity, and/or may be in a form suitable for improving formulation. Thus, the compound may be in the form of a pharmaceutically acceptable salt, hydrate, acid, ester, or other alternative suitable form. Typically, the composition is for treating a disease, condition or disorder as defined above. In some instances, the compound may be present in the composition as a pharmaceutically acceptable salt, or other alternative form of the compound, in order to ameliorate pharmaceutical formulation.

In some embodiments the pharmaceutical composition is a composition for treating a cancer, further comprising a further agent for treating cancer. The further agent for treating cancer is not especially limited, provided that it affords some utility for cancer treatment. However, typically the further agent for treating cancer is selected from ionising radiation, chemotherapeutic agents such as anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, immunotherapeutic agents, hormone-deprivation therapies, proapoptotic agents and cell cycle signalling inhibitors. An immunotherapeutic agent may consist of but is not limited to an anti-tumour vaccine, an oncolytic virus, an immune stimulatory antibody such as anti-CTLA4, anti-PD1, anti-PDL-1, anti-OX40, anti-41BB, anti-CD27, anti-CD40, anti-LAG3, anti-TIM3, and anti-GITR, a pattern recognition receptor agonist such as a STING, TLR-9 or RIG-I Helicase agonist, an IDO or TDO inhibitor, a novel adjuvant, a peptide, a cytokine, a chimeric antigen receptor T cell therapy (CAR-T), a small molecule immune modulator, tumour microenvironment modulators, and anti-angiogenic agents.

In still further embodiments the invention provides a pharmaceutical kit for treating a cancer, which pharmaceutical kit comprises:

(a) a compound as defined above; and

(b) a further agent for treating cancer; preferably wherein the further agent for treating cancer is selected from ionising radiation, chemotherapeutic agents such as anti-microtubule agents, platinum coordination complexes, alkylating agents, antibiotic agents, topoisomerase II inhibitors, antimetabolites, topoisomerase I inhibitors, senolytic agents, hormones and hormone analogues, hormone-deprivation therapies, signal transduction pathway inhibitors, other DNA damage repair pathway inhibitors, non-receptor tyrosine kinase angiogenesis inhibitors, antibody-drug conjugates, immunotherapeutic agents, proapoptotic agents and cell cycle signalling inhibitors;

wherein the compound and the further agent are suitable for administration simultaneously, sequentially or separately.

Further provided by the invention is a method of treating a disease and/or a condition and/or a disorder, which method comprises administering to a patient (or subject) a compound, or a composition, or a kit as defined above. The method is typically a method for treating any disease condition or disorder mentioned herein. In typical embodiments, the method is a method for treating a cancer. Preferably such a method comprises administering to a patient (or subject) a compound or a composition as defined above and a further agent for treating cancer as defined above. The compound or composition and the further agent may be administered simultaneously, sequentially or separately, depending upon the agents and patients involved, and the type of cancer indicated.

Typically, in all embodiments of the invention, both above and below, the patient (or subject) is an animal, typically a mammal, including canines, equines and felines, and more typically a human.

Further provided by the invention is a method of synthesis of a compound as defined above, which method comprises conducting a reaction between (i) a first reactant comprising ring E bearing a portion of substituent group L and (ii) a second reactant comprising the remainder of substituent group L, so as to form the PARP1 inhibitor compound.

Typically, in one method of synthesis, the first reactant comprises ring E and ring A, and the second reactant comprises a Q1 or Q2 precursor bearing a reactive group, which method comprises joining the N atom of ring A to the Q1 or Q2 precursor. In this method, the reactive group of the Q1 or Q2 precursor may comprise a carbonyl group, an alkyl halide or an alkyl sulfonate. Typically, the reaction comprises alkylation, reductive amination or amide formation so as to form group L.

Typically, in another method of synthesis, the first reactant comprises ring E, ring A, and at least one of Q1 and Q2, and the second reactant comprises a ring C derivative bearing a leaving group such as a halide or sulfonate. In this method, the reaction comprises a nucleophilic substitution reaction, such as a nucleophilic aromatic substitution reaction, so as to form group L.

The skilled person may select the reaction conditions in these methods with reference to known synthesis

techniques depending on the appropriate starting materials. In some embodiments, the method comprises one or more additional steps. Exemplary synthesis methodology is shown in the Examples herein

Typically, the above formulae (and all formulae herein) are shown in non-stereoisomeric form. For the avoidance of doubt, throughout the present disclosure a single formula is intended to represent all possible stereoisomers of a particular structure, including all possible isolated enantiomers corresponding to the formula, all possible mixtures of enantiomers corresponding to the formula, all possible mixtures of diastereomers corresponding to the formula, all possible mixtures of epimers corresponding to the formula and all possible racemic mixtures corresponding to the formula. In addition to this, the above formulae (and all formulae herein) are intended to represent all tautomeric forms equivalent to the corresponding formula.

The term "comprises" as used throughout the description and claims herein means "includes or consists of". The term denotes the inclusion of at least the features following the term and does not exclude the inclusion of other features which have not been explicitly mentioned. The term may also denote an entity which consists only of the features following the term.

## Detailed description of the invention

The invention will now be described in more detail, by way of example only, with reference to the following specific embodiments.

EXAMPLES

***Exemplary syntheses of compounds of the invention***

The compounds of the invention may be synthesised using readily available starting materials and known reactions. Exemplary syntheses of three compounds are shown below:

***Example 1 - Synthesis of Compound 42***

**INT-4 Synthesis**

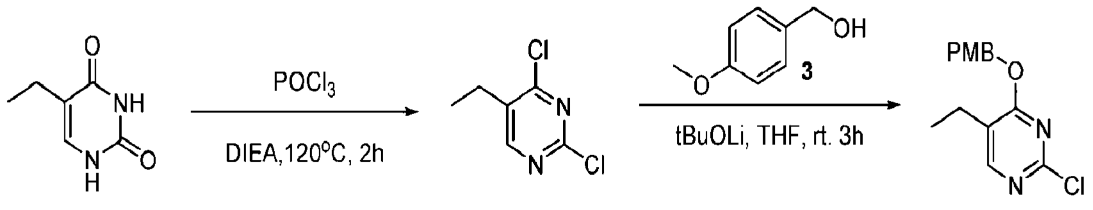


1 2 INT-4

***Preparation of 2,4-dichloro-5-ethylpyrimidine (2)***

To a solution of 5-ethyl-1,3-dihydropyrimidine-2,4-dione 1 (10.00 g, 0.07 mol) in $POCl_3$ (55.00 g, 0.36 mol) at 0 °C and added DIEA (23.00 g, 0.18 mol). Then the mixture was stirred for 2 h at 120 °C. The hot reaction mixture was poured into ice water and the aqueous layer was extracted with ethyl acetate (50 mL × 3). The combined organic layers were dried over $Na_2SO_4$. The reaction mixture was concentrated under reduced pressure. The residue was purified by flash chromatography (eluting with PE/EA = 100 : 0 to 92 : 8) to give 2,4-dichloro-5-ethylpyrimidine **2** (4.80 g, 38% yield ) as a white solid.
LCMS (ESI) calcd for C6H6Cl2N2 [M + H] $^+$ m/z 176.99, found 177.00

***Preparation of 2-chloro-5-ethyl-4-(2-methoxy-5-methylphenoxy)pyrimidine (INT-4)***

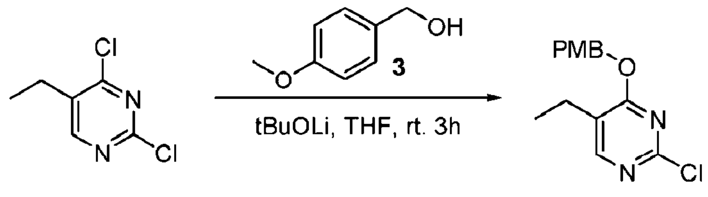


2 INT-4

To a solution of (4-methoxyphenyl) methanol **3** (4.54 g, 32.90 mmol) in THF (20 mL) was added tBuOLi (2.30 g, 28.75 mmol) at 70 °C for 15 min. Then 2,4-dichloro-5-ethylpyrimidine 2 (4.80 g, 27.27 mmol) was added to the mixture at 0 °C. The mixture was stirred for 3 h at room temperature. The reaction mixture was concentrated under reduced pressure. The residue was purified by flash chromatography (eluting with DCM/MeOH = 100 : 0 to 93 : 7) to give 2-chloro-5-ethyl-4-(2-methoxy-5-methylphenoxy)pyrimidine **INT-4** (4.00 g, 53 % yield ) as a white solid.

LCMS (ESI) calcd for C14H15ClN2O2 [M + H] + m/z 279.08, found 279.15.

**Compound 42 synthesis**

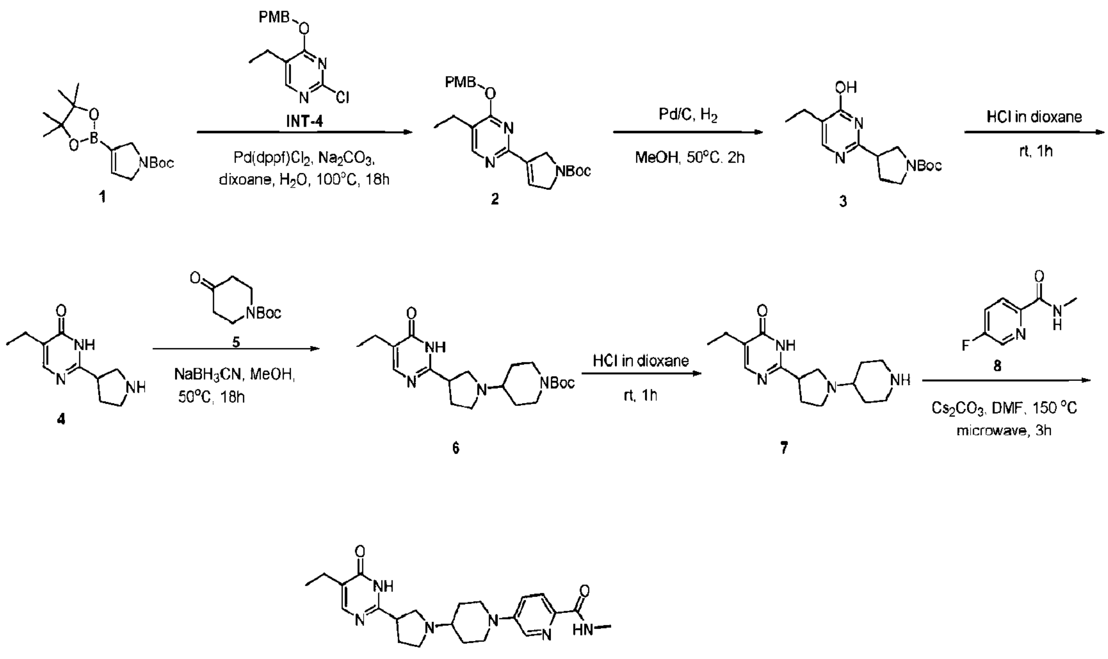


Compound 42

***Preparation of tert-butyl 3-(5-ethyl-4-((4-methoxybenzyl)oxy)pyrimidin-2-yl)-2,5-dihydro-1H-pyrrole-1-carboxylate (2)***

To a solution tert-butyl 3-(4,4,5,5-tetramethyl-1,3,2-dioxaborolan-2-yl)-2,5-dihydropyrrole-1-carboxylate **1** (500 mg, 1.69 mmol) in 1,4-dioxane/$H_2O$=4:1 (15 mL) was added 2-chloro-5-ethyl-4-(2-methoxy-5-methylphenoxy)pyrimidine **INT-4** (377.72 mg, 1.76 mmol) and $Na_2CO_3$ (189.11 mg, 3.01 mmol), then Pd(dppf)$Cl_2$ (247.89 mg, 0.34 mmol) was added at room temperature. The reaction mixture was refluxed under nitrogen at 100 °C and stirred for 18 h. After cooling to room temperature, the reaction mixture was concentrated under reduced pressure. The residue was purified by flash chromatography (eluting with petroleum ether/ethyl acetate = 100 : 0 to 85 : 15) to give tert-butyl 3-(5-ethyl-4-((4-methoxybenzyl)oxy)pyrimidin-2-yl)-2,5-dihydro-1H-pyrrole-1-carboxylate 2 (250 mg, 31% yield ) as a colorless oil. LCMS (ESI) calcd for C23H29N3O4 [M + H] + m/z 412.22, found 412.28.

***Preparation of tert-butyl 3-(5-ethyl-4-hydroxypyrimidin-2-yl)pyrrolidine-1-carboxylate (3)***

To a solution 3-(5-ethyl-4-((4-methoxybenzyl)oxy)pyrimidin-2-yl)-2,5-dihydro-1H-pyrrole-1-carboxylate **2** (250

mg, 0.61 mmol) in MeOH (15 mL) was added Pd/C (97 mg, 0.91 mmol) at room temperature. The reaction mixture was stirred for 2h at 50 °C under hydrogen. After cooling to room temperature, the mixture was filtered through a Celite pad and the filtrate was concentrated to give tert-butyl 3-(5-ethyl-4-hydroxypyrimidin-2-yl)pyrrolidine-1-carboxylate **3** (150 mg, 80% yield ) as a colourless oil.

LCMS (ESI) calcd for C15H23N3O3 $[M + H]^+$ m/z 294.17, found 294.19.

***Preparation of 5-ethyl-2-(pyrrolidin-3-yl)-3H-pyrimidin-4-one (4)***

To a solution 3-(5-ethyl-4-hydroxypyrimidin-2-yl)pyrrolidine-1-carboxylate **3** (150 mg, 0.51 mmol) was added HCl in dioxane (4 M, 10 mL) at room temperature. The reaction mixture was stirred for 1 h at room temperature. The reaction mixture was concentrated under reduced pressure to give 5-ethyl-2-(pyrrolidin-3-yl)-3H-pyrimidin-4-one **4** (100 mg, 94% yield) as a white solid.

LCMS (ESI) calcd for C10H15N3O $[M + H]^+$ m/z 194.12, found 194.19.

***Preparation of tert-butyl 4-[3-(5-ethyl-4-oxo-3H-pyrimidin-2-yl)pyrrolidin-1-yl]piperidine-1-carboxylate (6)***

To a solution 5-ethyl-2-(pyrrolidin-3-yl)-3H-pyrimidin-4-one **4** (100 mg, 0.52 mmol) in MeOH (15 mL) was added tert-butyl 4-oxopiperidine-1-carboxylate **5** (124.48 mg, 0.61 mmol) and $NaBH_3CN$ (145 mg, 3.11 mmol). The reaction mixture was refluxed under nitrogen at 50 °C and stirred for 18 h. After cooling to room temperature, the reaction mixture was concentrated under reduced pressure. The residue was purified by flash chromatography (eluting with DCM/MeOH = 100 : 0 to 95 : 5) to give tert-butyl 4-[3-(5-ethyl-4-oxo-3H-pyrimidin-2-yl)pyrrolidin-1-yl]piperidine-1-carboxylate **6** (120 mg, 55% yield ) as a white solid.

LCMS (ESI) calcd for C20H32N4O3 $[M + H]^+$ m/z 377.25, found 377.30.

***Preparation of 5-ethyl-2-[1-(piperidin-4-yl)pyrrolidin-3-yl]-3H-pyrimidin-4-one (7)***

Tert-butyl 4-[3-(5-ethyl-4-oxo-3H-pyrimidin-2-yl)pyrrolidin-1-yl]piperidine-1-carboxylate **6** (120 mg, 0.32 mmol) was added to HCl in dioxane (4 M, 10 mL) at room temperature. The reaction mixture was stirred for 1 h at room temperature, then concentrated under reduced pressure to give 5-ethyl-2-[1-(piperidin-4-yl)pyrrolidin-3-yl]-3H-pyrimidin-4-one **7** (90 mg, 92% yield) as a white solid.

LCMS (ESI) calcd for C15H24N4O $[M + H]^+$ m/z 277.20, found 277.24.

***Preparation of 5-{4-[3-(5-ethyl-4-oxo-3H-pyrimidin-2-yl)pyrrolidin-1-yl]piperidin-1-yl}-N-methylpyridine-2-carboxamide (Compound 42)***

To a solution 5-ethyl-2-[1-(piperidin-4-yl)pyrrolidin-3-yl]-3H-pyrimidin-4-one **7** (90 mg, 0.33 mmol) in DMF (10 mL) was added 5-fluoro-N-methylpyridine-2-carboxamide **8** (104 mg, 0.68 mmol) and $Cs_2CO_3$ (884 mg, 2.71 mmol). The reaction mixture was irradiated in a microwave reactor at 150 °C for 3 h. The reaction mixture was concentrated under reduced pressure. The residue was purified by flash chromatography (eluting with DCM/MeOH = 100 : 0 to 95 : 5) to give 5-{4-[3-(5-ethyl-4-oxo-3H-pyrimidin-2-yl)pyrrolidin-1-yl]piperidin-1-yl}-N-methylpyridine-2-carboxamide **Compound 42** (20 mg, 95% purity, 20% yield ) as a white solid.

$^1$H NMR (400 MHz, DMSO) δ 12.16 (s, 1 H), 8.42-8.38 (m, 1 H), 8.28 (d, *J* = 2.4 Hz, 1 H), 7.83 (d, *J* = 8.8 Hz, 1 H), 7.73 (s, 1 H), 7.42-7.39 (dd, *J* = 8.8, 2.8 Hz, 1 H), 3.87 (d, *J* = 12.8 Hz, 2 H), 3.29-3.25 (m, 2 H), 3.17-3.13 (m, 1 H), 2.94-2.74 (m, 8 H), 2.34 (q, *J* = 7.2 Hz, 2 H), 2.18-1.94 (m, 4 H), 1.56-1.47 (m, 2 H), 1.08 (t, *J* = 7.6 Hz, 3 H).

LCMS (ESI) calcd for C22H30N6O2 $[M + H]^+$ m/z 411.24, found 411.33.

***<u>Example 2 - Synthesis of Compound 53</u>***

**Synthesis of intermediate INT:**

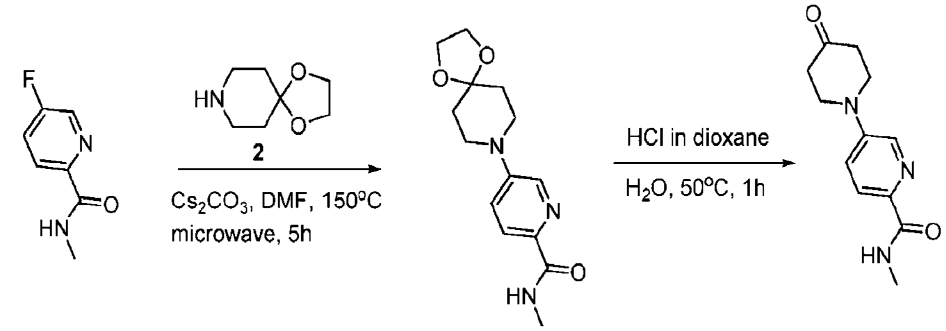


1 3 INT

***Preparation of 5-{1,4-dioxa-8-azaspiro[4.5]decan-8-yl}-N-methylpyridine-2-carboxamide (3)***

To a solution of 5-fluoro-N-methylpyridine-2-carboxamide **1** (1.00 g, 6.50 mmol) in DMF (15 mL) was added 1,4-dioxa-8-azaspiro[4.5]decane **2** (1.40 g, 9.75 mmol), then $Cs_2CO_3$ (2.12 g, 6.50 mmol) was added at room temperature. The reaction mixture was stirred at 150 °C using microwave for 5 h. After cooling to room temperature, the reaction mixture was concentrated under reduced pressure. The residue was purified by flash chromatography (eluting with DCM/MeOH = 100 : 0 to 97 : 3) to give 5-{ 1,4-dioxa-8-azaspiro[4.5]decan-8-yl}-N-methylpyridine-2-carboxamide 3 (1.50 g, 76% yield ) as a white solid.

LCMS (ESI) calcd for C14H19N3O3 [M + H] + m/z 278.14, found 278.14.

***Preparation of N-methyl-5-(4-oxopiperidin-1-yl)picolinamide (INT)***

To a solution of 5-{ 1,4-dioxa-8-azaspiro[4.5]decan-8-yl}-N-methylpyridine-2-carboxamide 3 (1.50 g, 5.40 mmol) in $H_2O$ (10 mL) was added HCl in 1,4-dioxane (4 M, 20 mL) at room temperature. The reaction mixture was stirred at 50 °C for 1 h. After cooling to room temperature, the reaction mixture was concentrated under reduced pressure. The residue was adjusted pH > 7 using $NaHCO_3$ solution, then extracted with EtOAc (50 mL x 3), the organic phase dried by $Na_2SO_4$ and concentated to give N-methyl-5-(4-oxopiperidin-1-yl)picolinamide INT (1.50 g, 76% yield ) as a yellow solid.

LCMS (ESI) calcd for C12H15N3O2 [M + H] + m/z 234.12, found 234.18.

<u>**Synthesis of Compound 53**</u>

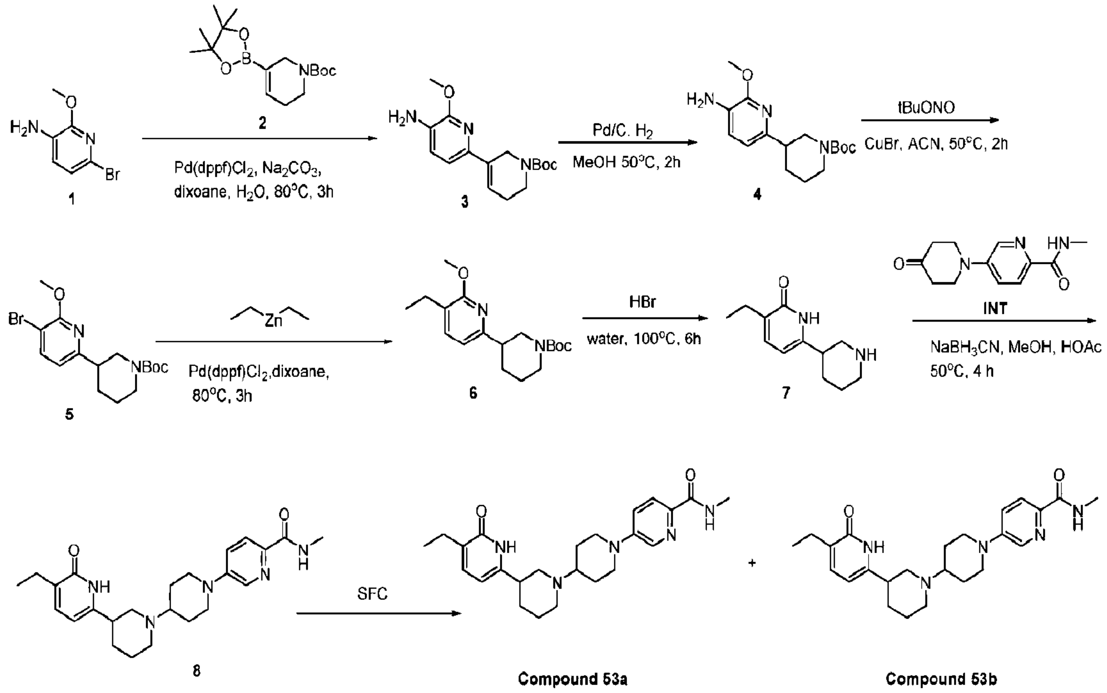


1 2 3 4 5 6 7 8

Compound 53a + Compound 53b

***Preparation of tert-butyl 3-(5-amino-6-methoxypyridin-2-yl)-5,6-dihydro-2H-pyridine-1-carboxylate (3)***

To a solution of 6-bromo-2-methoxypyridin-3-amine **1** (2.00 g, 9.90 mmol) in 1,4-dioxane/H2O=4:1 (45 mL) was added tert-butyl 3-(4,4,5,5-tetramethyl-1,3,2-dioxaborolan-2-yl)-5,6-dihydro-2H-pyridine-1-carboxylate 2 (3.98 g, 12.87 mmol), then Pd(dppf)$Cl_2$ (0.72 g, 0.99 mmol) and $Na_2CO_3$ (3.12 g, 99.00 mmol) were added at room temperature. The reaction mixture was stirred under nitrogen at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-amino-6-methoxypyridin-2-yl)-5,6-dihydro-2H-pyridine-1-carboxylate 3 (3.00 g, 89% yield ) as a white solid.

LCMS (ESI) calcd for C16H23N3O3 [M + H] $^+$ m/z 306.17, found 306.19.

***Preparation of tert-butyl 3-(5-amino-6-methoxypyridin-2-yl)piperidine-1-carboxylate (4)***

To a solution of tert-butyl 3-(5-amino-6-methoxypyridin-2-yl)-5,6-dihydro-2H-pyridine-1-carboxylate **3** (2.80 g, 9.20 mmol) in MeOH (50 mL) was added Pd/C (0.78 g, 7.36 mmol) at room temperature. The reaction mixture was stirred under hydrogen at 50 °C for 2 h. After cooling to room temperature, the reaction mixture was filtered, the solution was concentrated under reduced pressure to give product of tert-butyl 3-(5-amino-6-methoxypyridin-2-yl)piperidine-1-carboxylate 4 (2.80 g, 89% yield ) as a white solid.

LCMS (ESI) calcd for C16H25N3O3 [M + H] $^+$ m/z 308.19, found 308.19.

***Preparation of tert-butyl 3-(5-bromo-6-methoxypyridin-2-yl)piperidine-1-carboxylate (5)***

To a solution of tert-butyl 3-(5-amino-6-methoxypyridin-2-yl)piperidine-1-carboxylate 4 (2.80 g, 9.10 mmol) in ACN (50 mL) was added tert-Butyl nitrite (2.82 g, 27.30 mmol), stirred for 15 minutes, then CuBr (5.22 g, 36.40 mmol) was added at room temperature. The reaction mixture was stirred at 50 °C for 2 h. After cooling to room temperature, the reaction mixture was concentrated under reduced pressure. The residue was purified by flash chromatography (eluting with PE/EA = 100 : 0 to 85 : 15) to give tert-butyl tert-butyl 3-(5-bromo-6-methoxypyridin-2-yl)piperidine-1-carboxylate 5 (1.10 g, 30% yield ) as a white solid.

LCMS (ESI) calcd for C16H23BrN2O3 [M + H] $^+$ m/z 371.09, found 371.15.

***Preparation of tert-butyl 3-(5-ethyl-6-methoxypyridin-2-yl)piperidine-1-carboxylate (6)***

To a solution of tert-butyl 3-(5-bromo-6-methoxypyridin-2-yl)piperidine-1-carboxylate 5 (1.10 g, 2.96 mmol) in 1,4-dioxane (65 mL) was added Pd(dppf)$Cl_2$ (219 mg, 0.30 mmol). Then $Et_2Zn$ (1M, 11.84 mL, 11.84 mml) was added at room temperature. The reaction mixture under nitrogen was stirred at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-ethyl-6-methoxypyridin-2-yl)piperidine-1-carboxylate 6 (700 mg, 67% yield ) as a white solid.

LCMS (ESI) calcd for C18H28N2O3 [M + H] * m/z 321.21, found 321.30.

***Preparation of 3-ethyl-6-(piperidin-3-yl)-1H-pyridin-2-one (7)***

To a solution of tert-butyl 3-(5-ethyl-6-methoxypyridin-2-yl)piperidine-1-carboxylate 6 (200 mg, 0.62 mmol) in HBr in water (48%, 6 mL). The reaction mixture was stirred at 100 °C for 6 h. After cooling to room temperature, the reaction mixture was concentrated under reduced pressure to give 3-ethyl-6-(piperidin-3-yl)-1H-pyridin-2-one 7 (80 mg, 56% yield) as a yellow solid.

LCMS (ESI) calcd for C12H18N2O [M + H] $^+$ m/z 207.14, found 206.95.

***Preparation of 5-{4-[3-(5-ethyl-6-oxo-1H-pyridin-2-yl)piperidin-1-yl]piperidin-1-yl}-N-methylpyridine-2-carboxamide (8)***

To a solution of 3-ethyl-6-(piperidin-3-yl)-1H-pyridin-2-one 7 (80 mg, 0.38 mmol) in MeOH (10 mL) was added N-methyl-5-(4-oxopiperidin-1-yl)pyridine-2-carboxamide INT (89 mg, 0.38 mmol). Then two drops of acetic acid and $NaBH_3CN$ (24 mg, 0.38mml) was added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 95 : 5) to give 5-{4-[3-(5-ethyl-6-oxo-1H-pyridin-2-yl) piperidin-1-yl]piperidin-1-yl}-N-methylpyridine-2-carboxamide **8** (48 mg, 31% yield ) as a white solid.

LCMS (ESI) calcd for C24H33N5O2 [M + H] $^+$ m/z 424.26, found 424.37.

***Preparation of 5-{4-[3-(5-ethyl-6-oxo-1H-pyridin-2-yl)piperidin-1-yl]piperidin-1-yl}-N-methylpyridine-2-carboxamide (Compound 53a and Compound 53b)***

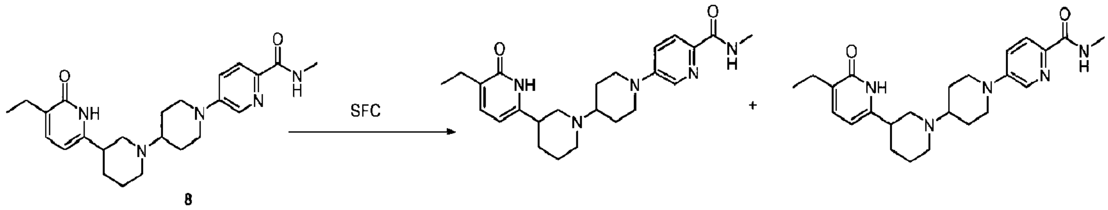


8

Compound 53a

Compound 53b

5-{4-[3-(5-ethyl-6-oxo-1H-pyridin-2-yl)piperidin-1-yl]piperidin-1-yl}-N-methylpyridine-2-carboxamide 8 was separated by SFC (Column: Daicel CHIRALPAK AD-H 250 mm × 20 mm I.D., 5 μmm; Mobile phase: $CO_2$/MeOH[0.1% ($NH_3$)] = 75/25) and concentrated under reduced pressure to afford the first fraction as **Compound 53a** (7.16 mg, 95% purity, ee%: 100, white solid) and the second fraction as **Compound 53b** (6.57 mg, 99% purity, ee%: 100, white solid)

***Compound 53a***

$^1$H NMR (400 MHz, DMSO) δ 11.48 (s, 1 H), 8.39 (q, *J* = 4.8 Hz, 1 H), 8.26 (d, *J* = 2.8 Hz, 1 H), 7.81 (d, *J* = 8.8 Hz, 1 H), 7.40 (dd, *J* = 8.8, 2.8 Hz, 1 H), 7.17 (d, *J* = 6.8 Hz, 1 H), 5.98 (d, *J* = 7.2 Hz, 1 H), 3.97 (d, *J* = 12.4 Hz, 2 H), 2.91-2.77 (m, 7 H), 2.67-2.54 (m, 2 H), 2.36-2.28 (m, 4 H), 1.83-1.81 (m, 3 H), 1.68-1.65 (m,1 H), 1.59-1.40(m, 4 H), 1.05 (t, *J* = 7.6 Hz, 3 H).

***Compound 53b***

$^1$H NMR (400 MHz, DMSO) δ 11.48 (s, 1 H), 8.39 (q, *J* = 4.4 Hz, 1 H), 8.26 (d, *J* = 2.8 Hz, 1 H), 7.81 (d, *J* = 8.8 Hz, 1 H), 7.40 (dd, *J* = 9.2, 3.2 Hz, 1 H), 7.17 (d, *J* = 7.2 Hz, 1 H), 5.98 (d, *J* = 6.8 Hz, 1 H), 3.97 (d, *J* = 12.4 Hz, 2 H), 2.91-2.77 (m, 7 H), 2.66-2.54 (m, 2 H), 2.36-2.25 (m, 4 H), 1.83-1.80 (m, 3 H), 1.67-1.64 (m, 1 H), 1.59-1.36 (m, 4 H), 1.05 (t, *J* = 7.6 Hz, 3 H).

***Example 3 - Synthesis of Compound 2***

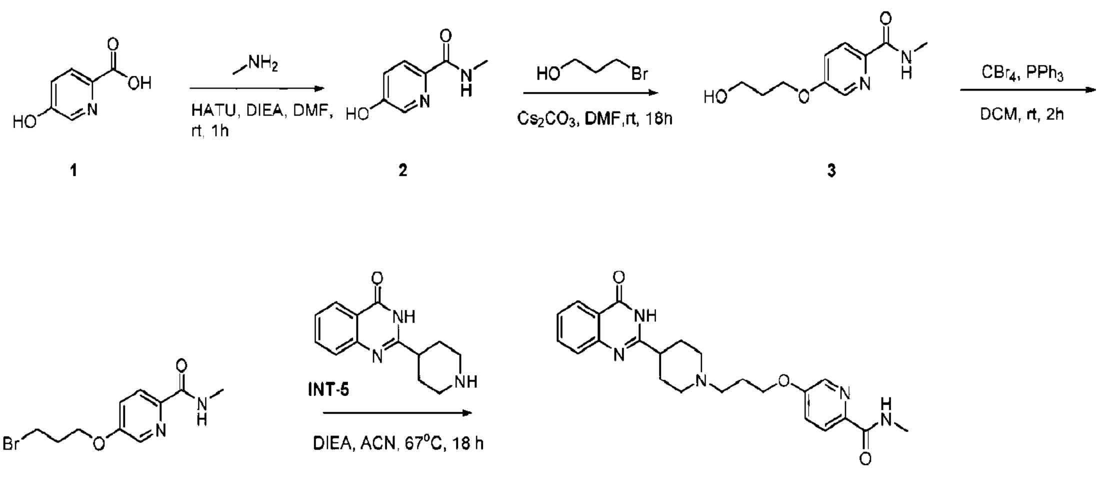


1

2

3

INT-4-1

Compound 2

***Preparation of 5-hydroxy-N-methylpicolinamide (2)***

To a solution of 5-hydroxypicolinic acid 1 (4.00 g, 28.72 mmol) in DMF (15 mL) was added HATU (12.55 g, 33.01 mmol) and DIEA (9.28 g, 71.80 mmol). Then methanamine (2M, 15 mL, 30.00 mmol) was added at room temperature. Then the mixture was stirred for 1 h at room temperature. The reaction mixture was concentrated under reduced pressure.

The residue was purified by flash chromatography (eluting with DCM/MeOH = 100 : 0 to 93 : 7) to give 5-***hydroxy-N-methylpicolinamide*** **2** (2.00 g, 46 % yield) as a white solid.
LCMS (ESI) calcd for C7H8N2O2 [M + H] + m/z 153.06, found 153.10

***Preparation of 5-(3-hydroxypropoxy)-N-methylpicolinamide (3)***

To a solution of 5-hydroxy-N-methylpicolinamide **2** (1.00 g, 6.61 mmol) in DMF (10 mL) was added 3-bromopropan-1-ol (2.75 g, 19.58 mmol) and $Cs_2CO_3$ (3.23 g, 9.93 mmol) at room temperature. Then the mixture was stirred for 18 h at room temperature. The reaction mixture was filtered to give 5-(3-hydroxypropoxy)-N-methylpicolinamide **3** (1.00 g, 65 % yield) as an oil.

LCMS (ESI) calcd for C10H14N2O3 [M + H] + m/z 211.10, found 211.05.

***Preparation of 5-(3-bromopropoxy)-N-methylpicolinamide (INT-4-1)***

To a solution of 5-(3-hydroxypropoxy)-N-methylpicolinamide 3 (250.00 mg, 1.19 mmol) in DCM (15 mL) was added $CBr_4$ (788.74 mg, 2.38 mmol). Then $PPh_3$ (623.83 mg, 2.38 mmol) was added at room temperature. Then the mixture was stirred for 2 h at room temperature. The reaction mixture was concentrated under reduced pressure. The residue was purified by flash chromatography (eluting with DCM/MeOH = 100 : 0 to 95 : 5) to give 5-(3-bromopropoxy)-N-methylpicolinamide **INT-4-1** (300 mg, 69% yield) as a white sold.

LCMS (ESI) calcd for C10H13BrN2O2 [M + H] + m/z 273.02, found 274.85.

***Preparation of N-methyl-5-(3-(4-(4-oxo-3,4-dihydroquinazolin-2-yl)piperidin-1-yl)propoxy)picolinamide (Compound 2)***

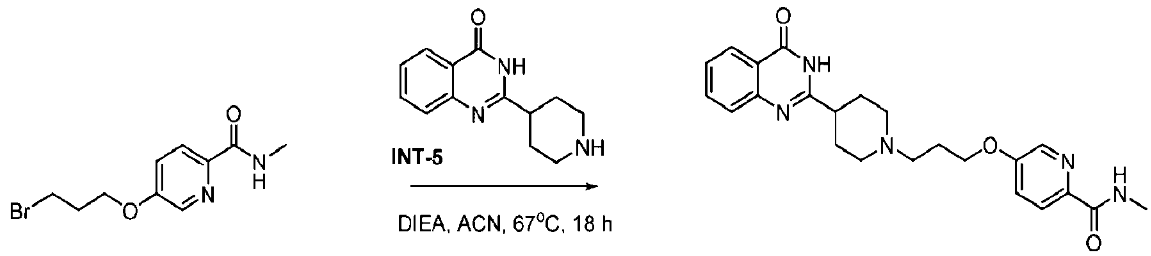


**INT-4-1** **Compound 2**

To a solution of 5-(3-bromopropoxy)-N-methylpicolinamide **INT-4-1** (150.00 mg, 0.55 mmol) in ACN (10mL) was added 2-(piperidin-4-yl)-3H-quinazolin-4-one **INT-5** (151.10 mg, 0.66 mmol). Then DIEA (212.94 mg, 1.65 mmol) was added at room temperature. Then the mixture was stirred for 18 h at 67 °C. The reaction mixture was concentrated under reduced pressure. The residue was purified by flash chromatography (eluting with DCM/MeOH = 100 : 0 to 92 : 8) to give N-methyl-5-(3-(4-(4-oxo-3,4-dihydroquinazolin-2-yl)piperidin-1-yl)propoxy)picolinamide **Compound 2** (100 mg, 78% yield) as a white sold.

1H NMR (400 MHz, DMSO) δ 12.15 (s, 1 H), 8.57-8.54 (m, 1 H), 8.29 (d, *J* = 2.8 Hz, 1 H), 8.08 (d, *J* = 8.0 Hz, 1 H), 7.98 (d, *J* = 8.8 Hz, 1 H), 7.78 (t, *J* = 7.6 Hz,1 H), 7.60 (d, *J* = 8.0 Hz, 1 H), 7.54 (dd, *J* = 8.8, 2.8 Hz, 1 H), 7.47 (t, *J* = 7.6 Hz, 1 H), 4.18 (t, *J* = 5.6 Hz, 2 H), 3.10-2.86 (m, 2 H), 2.79 (d, *J* = 4.8 Hz, 3 H), 2.65-2.53 (m, 1 H), 2.49-2.41 (m, 2 H), 1.94 (s, 8 H).

LCMS (ESI) calcd for C23H27N5O3 [M + H] + m/z 422.21, found 422.30.

***<u>Example 4- Synthesis of Compound 6</u>***

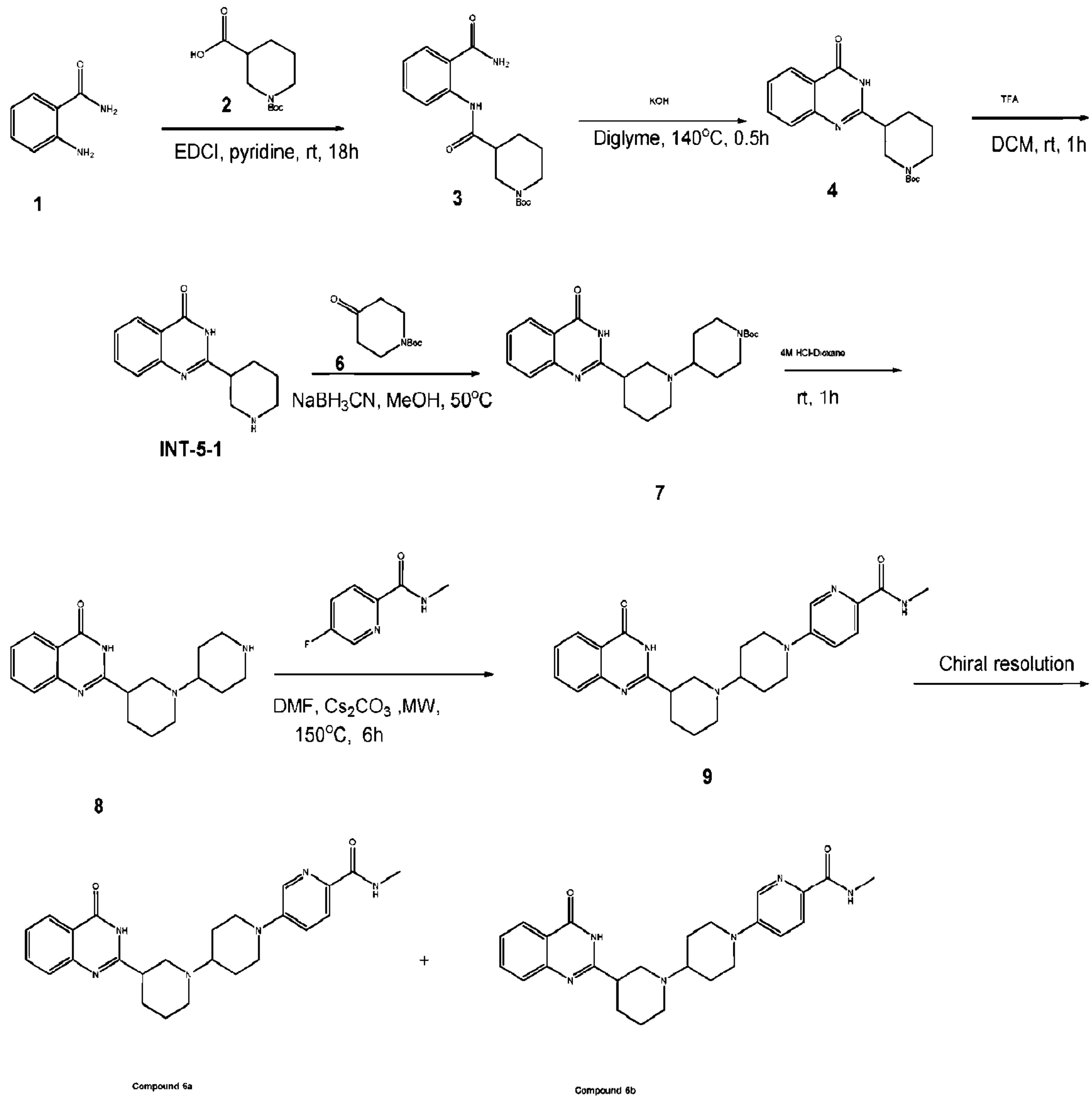


***Preparation of tert-butyl 3-((2-carbamoylphenyl)carbamoyl)piperidine-1-carboxylate (3)***

To a solution of 2-aminobenzamide **1** (6.00 g, 44.10 mmol) and 1-(tert-butoxycarbonyl)piperidine-3-carboxylic acid **2** (10.11 g, 44.10 mmol) in pyridine (50 mL) was added EDCI (8.45 g, 44.10 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over $Na_2SO_4$, concentrated under reduced pressure to give product of tert-butyl 3-((2-carbamoylphenyl)carbamoyl)piperidine-1-carboxylate 3 (12.00 g, 79% yield) as a white solid

LCMS (ESI) calcd for C18H25N3O4 $[M - H]^-$ m/z 346.18, found 346.20.

***Preparation of tert-butyl 3-(4-oxo-3,4-dihydroquinazolin-2-yl)piperidine-1-carboxylate (4)***

To the solution of tert-butyl 3-((2-carbamoylphenyl)carbamoyl)piperidine-1-carboxylate 3 (12.00 g, 34.50 mmol) in Diglyme (80 mL) was added KOH (2.13 g, 37.95 mmol), heated to 140 °C stirred for 2 h, cooled to 0 °C. ice-water (100 mL) added then adjusted pH < 7 using 1M HCl solution, precipitate formed, filtered, the filter cake washed with ice-water (50 mL*3), then dried to give product of tert-butyl 3-(4-oxo-3,4-dihydroquinazolin-2-yl)piperidine-1-carboxylate **4** (11.00 g, 97% yield) as a white solid .

LCMS (ESI) calcd for C18H23N3O3 $[M + H]^+$ m/z 330.17, found 330.20.

***Preparation of 2-(piperidin-3-yl)quinazolin-4(3H)-one (INT-5-1)***

To the solution of tert-butyl 3-(4-oxo-3,4-dihydroquinazolin-2-yl)piperidine-1-carboxylate **4** (3.30 g, 10.00 mmol) in DCM (10 mL) were added TFA (10 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-3-yl) quinazolin-4(3H)-one **INT-5-1** (1.50 g, 66% yield) as a white solid .

LCMS (ESI) calcd for C13H15N3O [M + H] $^+$ m/z 230.12, found 230.00.

***Preparation of tert-butyl 3-(4-oxo-3,4-dihydroquinazolin-2-yl)-[1,4'-bipiperidine]-1'-carboxylate (7)***

To a solution of 2-(piperidin-3-yl)quinazolin-4(3H)-one **INT-5-1** (460 mg, 2.01 mmol) in MeOH (15 mL) was added tert-butyl 4-oxopiperidine-1-carboxylate **6** (8.45 g, 44.10 mmol), then two drops of acetic acid and $NaBH_3CN$ (189.11 mg, 3.01 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 93 : 7) to give tert-butyl 3-(4-oxo-3,4-dihydroquinazolin-2-yl)-[1,4'-bipiperidine]-1'-carboxylate 7 (300 mg, 36% yield ) as a white solid.

$^1$H NMR (400 MHz, DMSO) δ 12.40 (s, 1 H), 8.11 (d, *J* = 7.2 Hz, 1 H), 7.81 (s, 1 H), 7.62 (d, *J* = 7.8 Hz, 1 H), 7.51 (s, 1 H), 4.10-3.99 (m, 2 H), 3.53-3.44 (m, 1H), 3.16 -3.04 (m, 1 H), 2.94-2.62 (m, 4 H), 1.99 (d, *J* = *2.4* Hz, 4 H), 1.91 (d, *J* = 2.4 Hz, 2 H), 1.82-1.53 (m, 4 H), 1.41 (s, 9 H).

***Preparation of 2-([1,4'-bipiperidin]-3-yl)quinazolin-4(3H)-one (8)***

Tert-butyl 3-(4-oxo-3,4-dihydroquinazolin-2-yl)-[1,4'-bipiperidine]-1'-carboxylate 7 (300 mg, 0.65 mmol) was added to HCl in dioxane (4 M, 10 mL), stirred for 1 h at room temperature, the reaction mixture was concentrated under reduced pressure to give 2-([1,4'-bipiperidin]-3-yl)quinazolin-4(3H)-one **8** (200 mg, 88% yield ) as a white solid.

LCMS (ESI) calcd for C18H24N4O [M + H] $^+$ m/z 313.20, found 313.15.

***Preparation of N-methyl-5-(3-(4-oxo-3,4-dihydroquinazolin-2-yl)-(1,4'-bipiperidin]-1'-yl)picolinamide (9)***

To a solution 2-([1,4'-bipiperidin]-3-yl)quinazolin-4(3H)-one **8** (200.00 mg, 0.64 mmol) in DMF (10 mL) was added $Cs_2CO_3$ (1042.94 mg, 3.20 mmol) and 5-fluoro-N-methylpicolinamide (148.02 mg, 0.96 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to N-methyl-5-(3-(4-oxo-3,4-dihydroquinazolin-2-yl)-[1,4'-bipiperidin]-1'-yl)picolinamide 9 (100 mg, 95% purity, 33% yield ) as a yellow solid.
LCMS (ESI) calcd for C25H30N6O2 [M + H] $^+$ m/z 447.24, found 447.20

***Chiral resolution of N-methyl-5-(3-(4-oxo-3,4-dihydroquinazolin-2-yl)-[1,4'-bipiperidin]-1'-yl)picolinamide (Compound 6a & Compound 6b)***

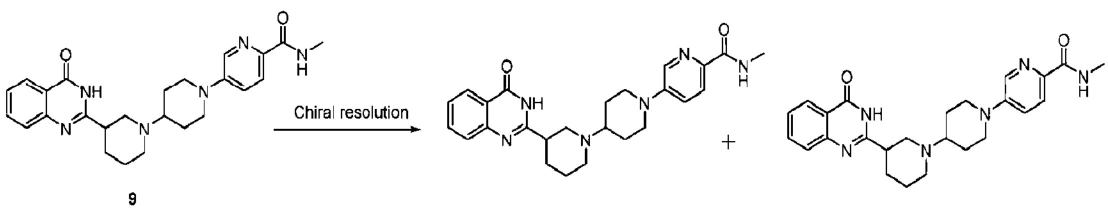


9

6a

+

6b

Compound 9 was separated by SFC (Column: Daicel CHIRALPAK OJ -H 250 mm × 20 mm I.D., 5 μmm; Mobile phase: $CO_2$/MeOH[0.1%($NH_3$)] = 70/30) and concentrated under reduced pressure to afford the first fraction as **Compound 6a** (40.6 mg, 100% purity, ee%: 100, white solid) and the second fraction as **Compound 6b** (39.5 mg, 99% purity, ee%: 100, white solid)

**Compound 6a**

$^1$H NMR (400 MHz, DMSO) δ 12.25 (s, 1 H), 8.40 (q, *J* = 4.8 Hz, 1 H), 8.29 (d, *J* = 2.8 Hz, 1 H), 8.10 (dd, *J* = 7.6, 1.6 Hz, 1 H), 7.85-7.78 (m, 2 H), 7.63 (d, *J* = 8.0 Hz, 1 H), 7.49 (t, *J* = 7.4 Hz, 1 H), 7.42 (dd, *J* = 8.8, 2.8 Hz, 1 H), 3.99 (d, *J* = 12.8 Hz, 2 H), 3.08 (d, *J* = 8.8 Hz, 1 H), 2.87-2.81 (m, 4 H), 2.80 (d, *J* = 4.8 Hz, 3 H), 2.61-2.55 (m, 2 H), 2.32 (t, *J* = 9.8 Hz, 1 H), 1.98-1.96 (m, 1 H), 1.87 (d, *J* = 12.4 Hz, 2 H), 1.78-1.75 (m, 1 H), 1.69-1.55 (m, 4 H).
LCMS (ESI) calcd for C25H30N6O2 [M + H] $^+$ m/z 447.24, found 447.

**Compound 6b**

$^1$H NMR (400 MHz, DMSO) δ 12.25 (s, 1 H), 8.39 (q, *J* = 4.8 Hz, 1 H), 8.29 (d, *J* = 2.8 Hz, 1 H), 8.10 (dd, *J* = 7.6, 1.6 Hz, 1 H), 7.84-7.78 (m, 2 H), 7.63 (d, *J* = 8.0 Hz, 1 H), 7.50- 7.47 (m, 1 H), 7.42 (dd, *J* = 8.8, 2.8 Hz, 1 H), 3.99 (d, *J* = 12.8 Hz, 2 H), 3.08 (d, *J* = 8.8 Hz, 1 H), 2.87-2.81 (m, 4 H), 2.80 (d, *J* = 4.8 Hz, 3 H), 2.61-2.55 (m, 2 H), 2.31 (t, *J* = 9.6 Hz, 1 H), 1.98-1.96 (m, 1 H), 1.87 (d, *J* = 12.4 Hz, 2 H), 1.78-1.75 ( m, 1 H), 1.63-1.55 (m, 4 H).
LCMS (ESI) calcd for C25H30N6O2 [M + H] $^+$ m/z 447.24, found 447.

***Example 5 - Synthesis of Compound 8***

**Synthesis of INT-5**

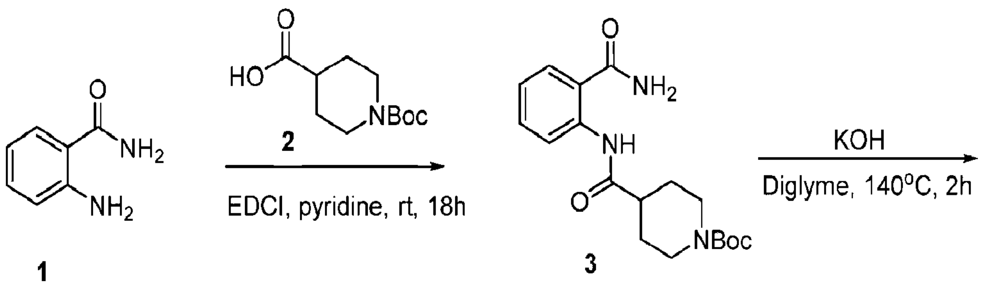


**1** **3**

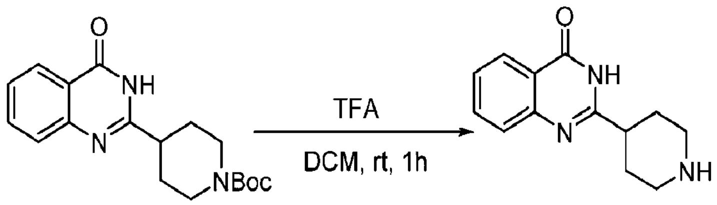


**4** **INT-5**

***Preparation of tert-butyl 4-((2-carbamoylphenyl)carbamoyl)piperidine-1-carboxylate (3)***

To a solution of 2-aminobenzamide **1** (6.00 g, 44.10 mmol) and 1-(tert-butoxycarbonyl)piperidine-4-carboxylic acid 2 (10.11 g, 44.10 mmol) in pyridine (50 mL) was added EDCI (8.45 g, 44.10 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over $Na_2SO_4$, concentrated under reduced pressure to give product of tert-butyl 4-((2-carbamoylphenyl)carbamoyl)piperidine-1-carboxylate 3 (12.00 g, 79% yield) as a white solid
LCMS (ESI) calcd for C18H25N3O4 [M + Na] $^+$ m/z 370.42, found 370.15.

***Preparation of tert-butyl 4-(4-oxo-3,4-dihydroquinazolin-2-yl)piperidine-1-carboxylate (4)***

To the solution of tert-butyl 4-((2-carbamoylphenyl)carbamoyl)piperidine-1-carboxylate **3** (12.00 g, 34.50 mmol)

in Diglyme (80 mL) was added KOH (2.13 g, 37.95 mmol), heated to 140 °C stirred for 2 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 4-(4-oxo-3,4-dihydroquinazolin-2-yl)piperidine-1-carboxylate **4** (11.00 g, 97% yield) as a white solid .

LCMS (ESI) calcd for C18H23N3O3 [M + H] $^+$ m/z 330.17, found 330.20.

***Preparation of 2-(piperidin-4-yl)quinazolin-4(3H)-one (INT-5)***

To the solution of tert-butyl 4-(4-oxo-3,4-dihydroquinazolin-2-yl)piperidine-1-carboxylate **4** (3.30 g, 10.00 mmol) in DCM (10 mL) were added TFA (10 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-4-yl) quinazolin-4(3H)-one INT-5 (1.50 g, 66% yield) as a white solid .

LCMS (ESI) calcd for C13H15N3O [M + H] $^+$ m/z 230.12, found 230.20.

**Compound 8 Synthesis**

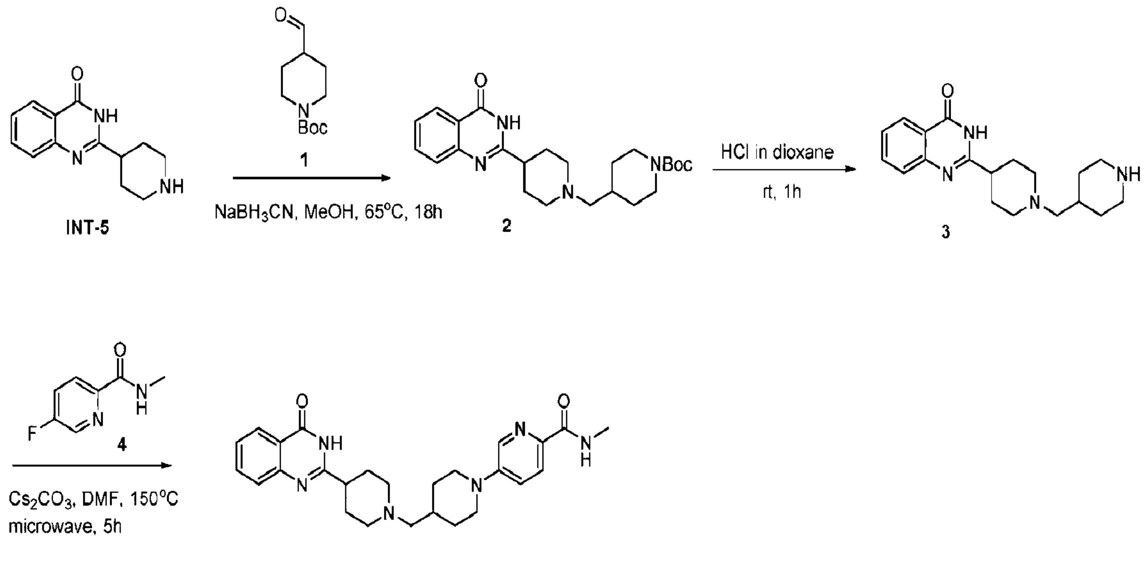


**Compound 8**

***Preparation of tert-butyl 4-formylpiperidine-1-carboxylate (2)***

To a solution of tert-butyl 4-formylpiperidine-1-carboxylate 1 (837.00 mg, 3.85 mmol) in MeOH (50 mL) was added 2-(piperidin-4-yl) quinazolin-4(3H)-one INT-5 (300.00 mg, 1.31 mmol). Then two drops of acetic acid and $NaBH_3CN$ (400.00 mg, 6.37 mmol) were added at room temperature. Then the mixture was stirred for 18 h at 65 °C. After cooling to room temperature, the reaction mixture was concentrated under reduced pressure. The residue was purified by flash chromatography (eluting with DCM/McOH = 100 : 0 to 92 : 8) to give tert-butyl 4-((4-(4-oxo-3,4-dihydroquinazolin-2-yl) piperidin-1-yl)methyl)piperidine-1-carboxylate **2** (500.00 mg, 90% yield ) as a white solid.

LCMS (ESI) calcd for C24H34N4O3 [M + H] $^+$ m/z 427.26, found 427.30.

***Preparation of tert-butyl 4-((4-(4-oxo-3,4-dihydroquinazolin-2-yl)piperidin-1-yl)methyl)piperidine-1-carboxylate (3)***

To a solution of tert-butyl 4-((4-(4-oxo-3,4-dihydroquinazolin-2-yl)piperidin-1-yl)methyl)piperidine-1-carboxylate **2** (500.00 mg, 1.17 mmol) in MeOH (5 mL) was added 4M HCl-Dioxane (10 mL) at room temperature. The reaction mixture stirred at room temperature for 1 h. The reaction mixture was concentrated under reduced pressure to give 2-(1-(piperidin-4-ylmethyl) piperidin-4-yl) quinazolin-4(3H)-one **3** (350.00 mg, 87% yield) as a white solid. LCMS (ESI) calcd for Cl9H26N4O [M + H] $^+$ m/z 327.21, found 327.15.

***Preparation of N-methyl-5-(4-((4-(4-oxo-3,4-dihydroquinazolin-2-yl)piperidin-1-yl)methyl)piperidin-1-yl)picolinamide (Compound 8)***

To a solution of 2-(1-(piperidin-4-ylmethyl)piperidin-4-yl)quinazolin-4(3H)-one **3** (300.00 mg, 0.92 mmol) in DMF (5 mL) was added 5-fluoro-N-methylpicolinamide **4** (354.14 mg, 2.30 mmol). Then $Cs_2CO_3$ (2994.20 mg, 9.19 mmol) was added at room temperature. The reaction mixture was irradiated in a microwave reactor at 150 °C for 5 h. the reaction mixture was concentrated under reduced pressure. The residue was purified by prep-HPLC (columns: Gemini 5 um C18 150 × 21.2 mm, mobile phase: ACN - $H_2O$ (0.1% FA), gradient: 15-25) to give N-methyl-5-(4-((4-(4-oxo-3,4-dihydroquinazolin-2-yl)piperidin- 1-yl)methyl)piperidin-1-yl)picolinamide **Compound 8** (100 mg, 98% purity, 24% yield) as a white sold.

LCMS (ESI) calcd for C26H32N6O2 [M + H] $^+$ m/z 461.26, found 461.20.

1H NMR (400 MHz, DMSO) δ 12.13 (s, 1 H), 8.37 (d, *J* = 4.8 Hz, 1 H), 8.26 (d, *J* = 2.8 Hz, 1 H), 8.08 (d, *J* = 7.6 Hz, 1 H), 7.82-7.76 (m, 2 H), 7.60 (d, *J* = 8.0 Hz, 1 H), 7.50-7.35 (m, 2 H), 3.91 (d, *J* = 12.4 Hz, 2 H), 2.95 (d, *J* = 10.8 Hz, 2 H), 2.89-2.75 (m, 5 H), 2.62-2.53 (m, 1 H), 2.18 (d, *J* = 6.4 Hz, 2 H), 1.98-1.73 (m, 9 H), 1.23-1.17 (m, 2 H).

***<u>Example 6 - Synthesis of Compound 10</u>***

**Compound 10 Synthesis**

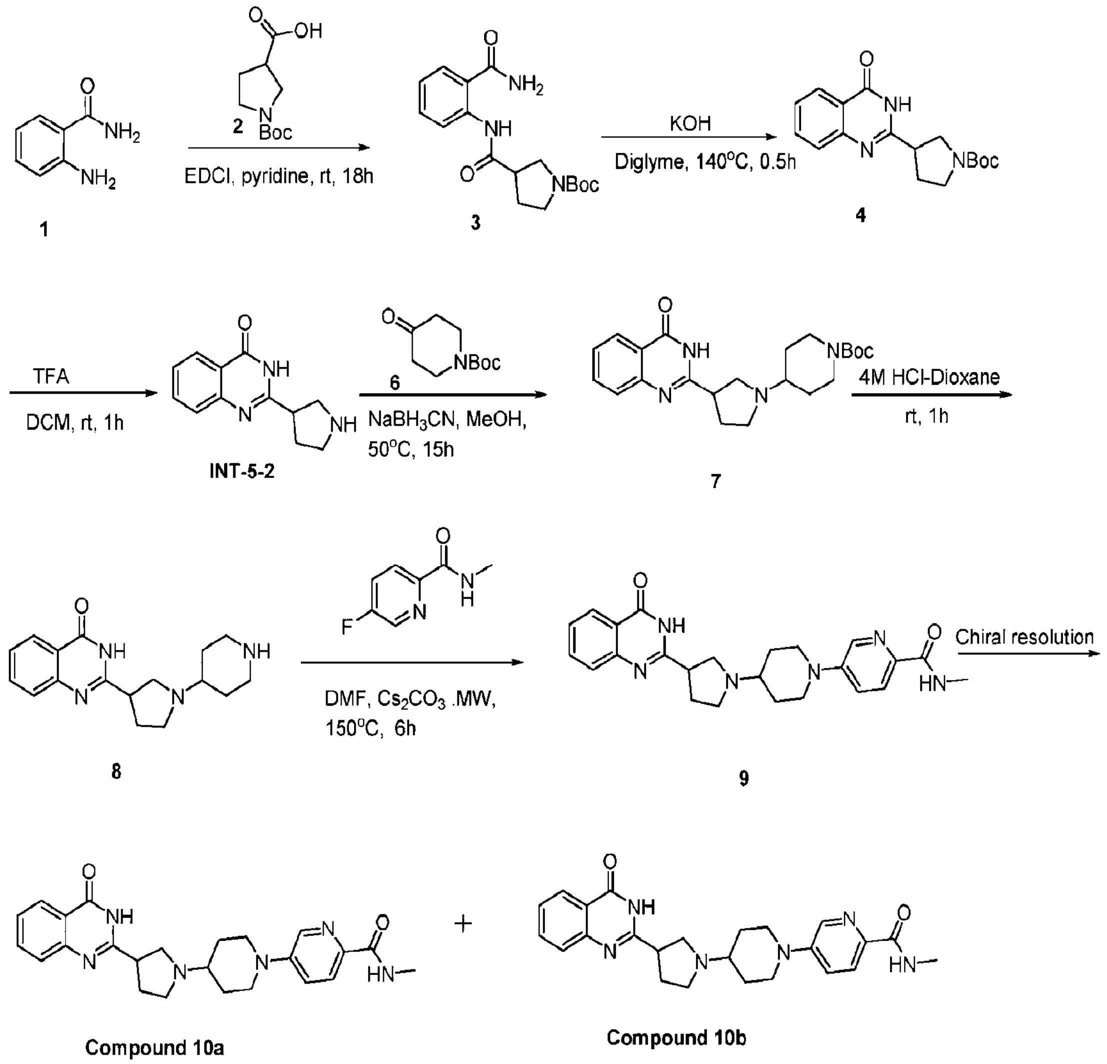


1 3 4

INT-5-2 7

8 9

Compound 10a + Compound 10b

***Preparation of tert-butyl 3-((2-carbamoylphenyl)carbamoyl)pyrrolidine-1-carboxylate (3)***

To a solution of 2-aminobenzamide **1** (6.00 g, 44.10 mmol) and 1-(tert-butoxycarbonyl)pyrrolidine-3-carboxylic acid 2 (9.49 g, 44.10 mmol) in pyridine (50 mL) was added EDCI (8.45 g, 44.10 mmol). then the mixture was stirred for 18 h at room temperature. The reaction mixture was quenched with water and the aqueous layer was extracted with EtOAc (50 mL x 3). The combined organic layers was washed by 1M HCl solution and brine, dried over $Na_2SO_4$, concentrated under reduced pressure to give tert-butyl 3-((2-carbamoylphenyl)carbamoyl)pyrrolidine-1-carboxylate 3 (12.00 g, 77% yield) as a white solid LCMS (ESI) calcd for C17H23N3O4 [M + Na] $^+$ m/z 356.17, found 356.00.

***Preparation of tert-butyl 3-(4-oxo-3,4-dihydroquinazolin-2-yl)pyrrolidine-1-carboxylate (4)***

To the solution of tert-butyl 3-((2-carbamoylphenyl)carbamoyl)pyrrolidine-1-carboxylate 3 (12.00 g, 36.04 mmol) in Diglyme (80 mL) was added KOH (2.42 g, 43.25 mmol), heated to 140 °C stirred for 0.5 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 3-(4-oxo-3,4-dihydroquinazolin-2-yl)pyrrolidine-1-carboxylate **4** (10.00 g, 83% yield) as a white solid .

LCMS (ESI) calcd for C17H21N3O3 [M + H] $^+$ m/z 316.16, found 316.05.

***Preparation of 2-(pyrrolidin-3-yl)quinazolin-4(3H)-one (INT-5-2)***

To the solution of tert-butyl 3-(4-oxo-3,4-dihydroquinazolin-2-yl)pyrrolidine-1-carboxylate **4** (10.00 g, 31.75 mmol) in DCM (20 mL) was added TFA (20 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(pyrrolidin-3-yl)quinazolin-4(3H)-one **INT-5-2** (8.00 g, 80% yield) as a white solid .

1H NMR (400 MHz, DMSO-$d_6$, ppm) $\delta$ 8.14-8.07 (m, 1 H), 7.87-7.76 (m, 1 H), 7.64 (d, $J$ = 8.0 Hz, 1 H), 7.57-7.44 (m, 1 H), 3.76-3.61 (m, 1 H), 3.61-3.49 (m, 2 H),3.43-3.33 (m, 1 H), 3.29 (dt, $J$ = 11.4, 5.6 Hz, 1 H), 2.37 (td, $J$ = 14.0, 7.1 Hz, 1 H), 2.21 (td, $J$ = 13.5, 7.2 Hz, 1 H).

LCMS (ESI) calcd for C12H13N3O [M - H] $^-$ m/z 216.11, found 215.95.

***Preparation of tert-butyl 4-(3-(4-oxo-3,4-dihydroquinazolin-2-yl)pyrrolidin-1-yl)piperidine-1-carboxylate (7)***

To a solution of 2-(pyrrolidin-3-yl)quinazolin-4(3H)-one **INT-5-2** (600.00 mg, 2.79 mmol) in MeOH (30 mL) was added tert-butyl 4-oxopiperidine-1-carboxylate **6** (1.67 g, 8.36 mmol), then two drops of acetic acid and $NaBH_3CN$ (1.05 g, 16.72 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 93 : 7) to give tert-butyl 4-(3-(4-oxo-3,4-dihydroquinazolin-2-yl) pyrrolidin-1-yl)piperidine-1-carboxylate 7 (400 mg, 34% yield ) as a white solid.

LCMS (ESI) calcd for C22H30N4O3 [M + H] $^+$ m/z 399.23, found 399.30.

***Preparation of 2-(1-(piperidin-4-yl)pyrrolidin-3-yl)quinazolin-4(3H)-one (8)***

Tert-butyl 4-(3-(4-oxo-3,4-dihydroquinazolin-2-yl)pyrrolidin-1-yl)piperidine-1-carboxylate **7** (400 mg, 0.81 mmol) was added to HCl in dioxane (4 M, 10 mL), stirred for 1 h at room temperature, the reaction mixture was concentrated under reduced pressure to give 2-(1-(piperidin-4-yl)pyrrolidin-3-yl)quinazolin-4(3H)-one **8** (200.00 mg, 79% yield ) as a white solid.

LCMS (ESI) calcd for C17H22N4O [M + H] $^+$ m/z 299.18, found 299.25.

***Preparation of N-methyl-5-(4-(3-(4-oxo-3,4-dihydroquinazolin-2-yl)pyrrolidin-1-yl)piperidin-1-yl)picolinamide (9)***

To a solution 2-(1-(piperidin-4-yl)pyrrolidin-3-yl)quinazolin-4(3H)-one **8** (200.00 mg, 0.67 mmol) in DMF (10 mL) was added $Cs_2CO_3$ (2.18 g, 6.70 mmol) and 5-fluoro-N-methylpicolinamide **9** (432.44 mg, 2.01 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to N-methyl-5-(4-(3-(4-oxo-3,4-dihydroquinazolin-2-yl)pyrrolidin-1-yl)piperidin-1-yl)picolinamide 9 (100 mg, 95% purity, 33 % yield) as a yellow solid.

LCMS (ESI) calcd for C24H28N6O2 [M + H] $^+$ m/z 433.23, found 433.25.

***Chiral resolution of N-methyl-5-(4-(3-(4-oxo-3,4-dihydroquinazolin-2-yl)pyrrolidin-1-yl)piperidin-1-yl)picolinamide (9)***

Compound 9 was separated by SFC (Column: Daicel CHIRALPAK OJ -H 250 mm × 20 mm I.D., 5 μmm; Mobile phase: $CO_2$/MeOH[0.1%($NH_3$)] = 70/30) and concentrated under reduced pressure to afford the first fraction as **Compound 10a** (16.4. mg, 100% purity, ee%: 100, off-white solid) and the second fraction as **Compound 10b** (12.3 mg, 100% purity, ee%: 100, off-white solid)

**Compound 10a**

$^1$H NMR (400 MHz, DMSO-$d_6$, ppm) δ 12.16 (s, 1 H), 8.40-8.37 (m, 1 H), 8.27 (d, *J* = 2.8 Hz, 1 H), 8.07 (dd, *J* = 7.6, 1.6 Hz, 1 H), 7.82-7.75 (m, 2 H), 7.61 (d, *J* = 8.0 Hz, 1 H), 7.48-7.44 (m, 1 H), 7.40 (dd, *J* = 8.8, 2.8 Hz, 1 H), 3.82 (d, *J* = 12.8 Hz, 2 H), 3.07 (t, *J* = 8.6 Hz, 2 H),
**[0195]** 2.95 (t, *J* = 10.8 Hz, 2 H), 2.86-2.79 (m, 2 H), 2.78-2.77 (m, 3 H), 2.69-2.63 (m, 1 H), 2.33-2.32 (m, 1H), 2.20-2.13 (m, 2 H), 1.96-1.93 (m, 2 H), 1.58-1.47 (m, 2 H).
LCMS (ESI) calcd for C24H28N6O2 [M + H] $^+$ m/z 433.23, found 433.20.

**Compound 10b**

$^1$H NMR (400 MHz, DMSO-$d_6$, ppm) δ 8.39-8.38 (m, 1 H), 8.28 (d, *J* = 3.2 Hz, 1 H), 8.08 (dd, *J* = 7.6, 1.6 Hz, 1 H), 7.83-7.76 (m, 2 H), 7.61 (d, *J* = 8.0 Hz, 1 H), 7.47 (t, *J* = 7.6 Hz, 1 H), 7.40 (dd, *J* = 8.8, 2.8 Hz, 1 H), 3.83 (d, *J* = 12.8 Hz, 2 H), 3.09 (d, *J* = 8.4 Hz, 2 H), 2.95 (t, *J* = 12.4 Hz, 2 H), 2.88-2.79 (m, 2 H), 2.79 (d, *J* = 4.8 Hz, 3 H), 2.68-2.66 (m, 1 H), 2.39-2.31 (m, 1H), 2.21-2.14 (m, 2 H), 1.97-1.94 (m, 2 H), 1.59-1.47 (m, 2 H).
LCMS (ESI) calcd for C24H28N6O2 [M + H] $^+$ m/z 433.23, found 433.20.

***Example 7 - Synthesis of Compound 14***

**Preparation of INT5-3**

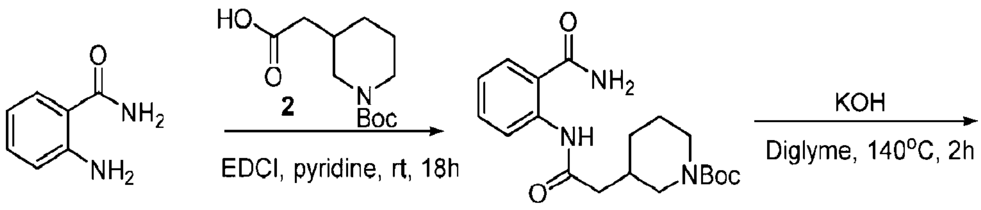


1 3

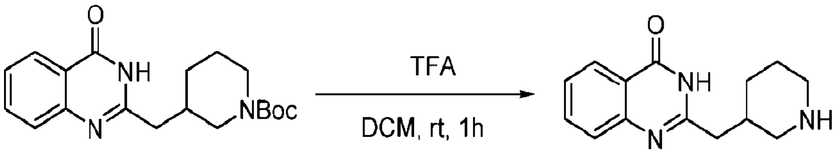


4 INT5-3

***Preparation of tert-butyl 3-(2-((2-carbamoylphenyl)amino)-2-oxoethyl)piperidine-1-carboxylate (3)***

To a solution of 2-aminobenzamide 1 (2.80 g, 20.60 mmol) and 2-(1-(tert-butoxycarbonyl) piperidin-3-yl)acetic acid **2** (5.00 g, 20.60 mmol) in pyridine (50 mL) was added EDCI (3.95 g, 20.60 mmol). then the mixture was stirred for 18 h at room temperature. The reaction mixture was quenched with water, the aqueous layer was extracted with EtOAc (50 mL x 3). The combined organic layers was washed with 1M HCl solution and brine, dried over $Na_2SO_4$, concentrated under reduced pressure to give product of tert-butyl 3-(2-((2-carbamoylphenyl)amino)-2-oxoethyl)piperidine-1-carboxylate 3 (7.00 g, 94% yield) as a white solid.

LCMS (ESI) calcd for C19H27N3O4 [M + H] + m/z 362.20, found 362.25.

***Preparation of tert-butyl 3-((4-oxo-3,4-dihydroquinazolin-2-yl)methyl)piperidine-1-carboxylate (4)***

To the solution of tert-butyl 3-(2-((2-carbamoylphenyl)amino)-2-oxoethyl)piperidine-1-carboxylate **3** (7.60 g, 21.00 mmol) in Diglyme (80 mL) was added KOH (1.18 g, 21.00 mmol), heated to 140 °C stirred for 2 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 3-((4-oxo-3,4-dihydroquinazolin-2-yl)methyl)piperidine-1-carboxylate **4** (6.80 g, 94% yield) as a white solid .

LCMS (ESI) calcd for C19H25N3O3 [M + H] + m/z 344.19, found 344.15.

***Preparation of 2-(piperidin-3-ylmethyl)quinazolin-4(3H)-one (INT-5-3)***

To a solution of tert-butyl 3-((4-oxo-3,4-dihydroquinazolin-2-yl)methyl)piperidine-1-carboxylate **4** (2.00 g, 5.80 mmol) in DCM (10 mL) were added TFA (10 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-3-ylmethyl)quinazolin-4(3H)-one **INT-5-3** (1.20 g, 85% yield) as a white solid .

LCMS (ESI) calcd for C14H17N3O [M + H] + m/z 244.14, found 244.20.

**Compound 14 Synthesis**

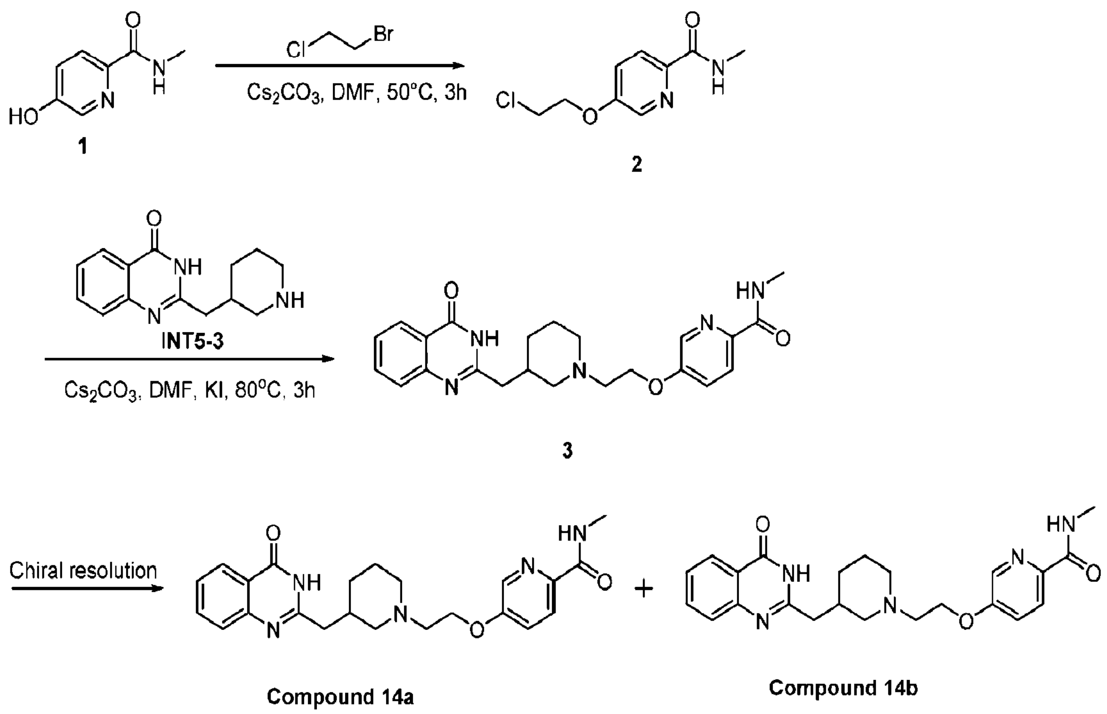


1

2

3

Compound 14a

Compound 14b

***Preparation of tert-butyl 4-((2-carbamoylphenyl)carbamoyl)piperidine-1-carboxylate (2)***

To a solution of 5-hydroxy-N-methylpicolinamide **1** (500.00 mg, 3.29 mmol) and $Cs_2CO_3$ (3.22 g, 9.87 mmol) in DMF (15 mL) was added 1-bromo-2-chloroethane **2** (943.64 mg, 6.58 mmol). then the mixture was stirred for 3 h at 50 °C. The reaction mixture was quenched with water, aqueous layer was extracted with EtOAc (50 mL x 3), the combined organic layers was concentrated under reduced pressure. the residue was purified by flash chromatography (eluting with PE/EtOAc = 100 : 0 to 80 : 20) to give tert-butyl 4-((2-carbamoylphenyl)carbamoyl)piperidine-1-carboxylate 2 (500.00 mg, 71% yield) as a colorless oil.

LCMS (ESI) calcd for C9H11ClN2O2 [M + H] + m/z 215.05, found 215.00.

***Preparation of N-methyl-5-(2-(3-((4-oxo-3,4-dihydroquinazolin-2-yl)methyl)piperidin-1-yl)ethoxy)picolinamide (3)***

To a solution of tert-butyl 4-((2-carbamoylphenyl)carbamoyl)piperidine-1-carboxylate **2** (500.00 mg, 2.33 mmol) and $Cs_2CO_3$ (2.28 g, 6.99 mmol) in DMF (15 mL) were added 2-(piperidin-3-ylmethyl)quinazolin-4(3H)-one **INT5-3** (566.91 mg, 2.33 mmol) and KI (1.16 g, 6.99 mmol). then the mixture was stirred for 3 h at 80 °C. The reaction mixture was quenched with water, aqueous layer was extracted with EtOAc (50 mL x 3), the combined organic layers was concentrated under reduced pressure. the residue was purified by flash chromatography (eluting with DCM/ MeOH = 100 : 0 to 92 : 8) to give N-methyl-5-(2-(3-((4-oxo-3,4-dihydroquinazolin-2-yl)methyl)piperidin-1-yl)ethoxy)picolinamide **3** (200.00 mg, 20% yield ) as a yellow solid.
LCMS (ESI) calcd for C23H27N5O3 [M + H] + m/z 422.21, found 422.10.

***Chiral resolution of N-methyl-5-(2-(3-((4-oxo-3,4-dihydroquinazolin-2-yl)methyl)piperidin-1-yl)ethoxy)picolinamide (Compound 14)***

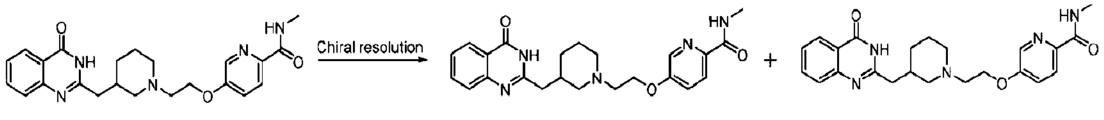


3 Compound 14a Compound 14b

Compound 3 was separated by SFC (Column: Daicel CHIRALPAK OJ -H 250 mm × 20 mm I.D., 5 μmm; Mobile phase: $CO_2$/MeOH[0.1%(FA)] = 70/30) and concentrated under reduced pressure to afford the first fraction as **Compound 14a** (31.6 mg, 100% purity, ee%: 100, white solid) and the second fraction as **Compound 14b** (28.1 mg, 99% purity, ee%: 99, white solid)

**Compound 14a**

$^1$H NMR (400 MHz, DMSO-$d_6$, ppm) δ 12.15 (s, 1 H), 8.53 (q, *J* = 9.6, 4.8 Hz, 1 H), 8.23 (d, *J* = 2.8 Hz, 1 H), 8.07 (dd, *J* = 8.0, 1.2 Hz, 1 H), 7.92 (d, *J* = 8.8 Hz, 1 H), 7.79-7.73 (m, 1 H), 7.56 (d, *J* = 8.0 Hz, 1 H), 7.49-7.42 (m, 2 H), 4.18 (t, *J* = 5.6 Hz, 2 H), 2.90-2.81 (m, 2 H), 2.79 (d, *J* = 4.8 Hz, 3 H), 2.73-2.65 (m, 2 H), 2.55-2.51 (m,2 H), 2.17-2.00 (m, 2 H), 1.93-1.85 (m, 1 H), 1.70-1.59 (m, 2 H), 1.51-1.38 (m, 1 H), 1.02-0.99 (m, 1 H).
LCMS (ESI) calcd for C23H27N5O3 [M + H] + m/z 422.21, found 422.25.

**Compound 14b**

$^1$H NMR (400 MHz, DMSO-$d_6$, ppm) δ 12.15 (s, 1 H), 8.53 (q, *J* = 9.6, 4.8 Hz, 1 H), 8.23 (d, *J* = 2.8 Hz, 1 H), 8.07 (dd, *J* = 8.0, 1.2 Hz, 1 H), 7.92 (d, *J* = 8.8 Hz, 1 H), 7.79-7.73 (m, 1 H), 7.56 (d, *J* = 8.0 Hz, 1 H), 7.49-7.42 (m, 2 H), 4.18 (t, *J* = 5.6 Hz, 2 H), 2.90-2.81 (m, 2 H), 2.79 (d, *J* = 4.8 Hz, 3 H), 2.73-2.65 (m, 2 H), 2.55-2.51 (m,2 H), 2.17-2.00 (m, 2 H), 1.93-1.85 (m, 1 H), 1.70-1.59 (m, 2 H), 1.51-1.38 (m, 1 H), 1.02-0.99 (m, 1 H).
LCMS (ESI) calcd for C23H27N5O3 [M + H] + m/z 422.21, found 422.25.

*Example 8 - Synthesis of Compound 17*

Compound 17 **Synthesis**

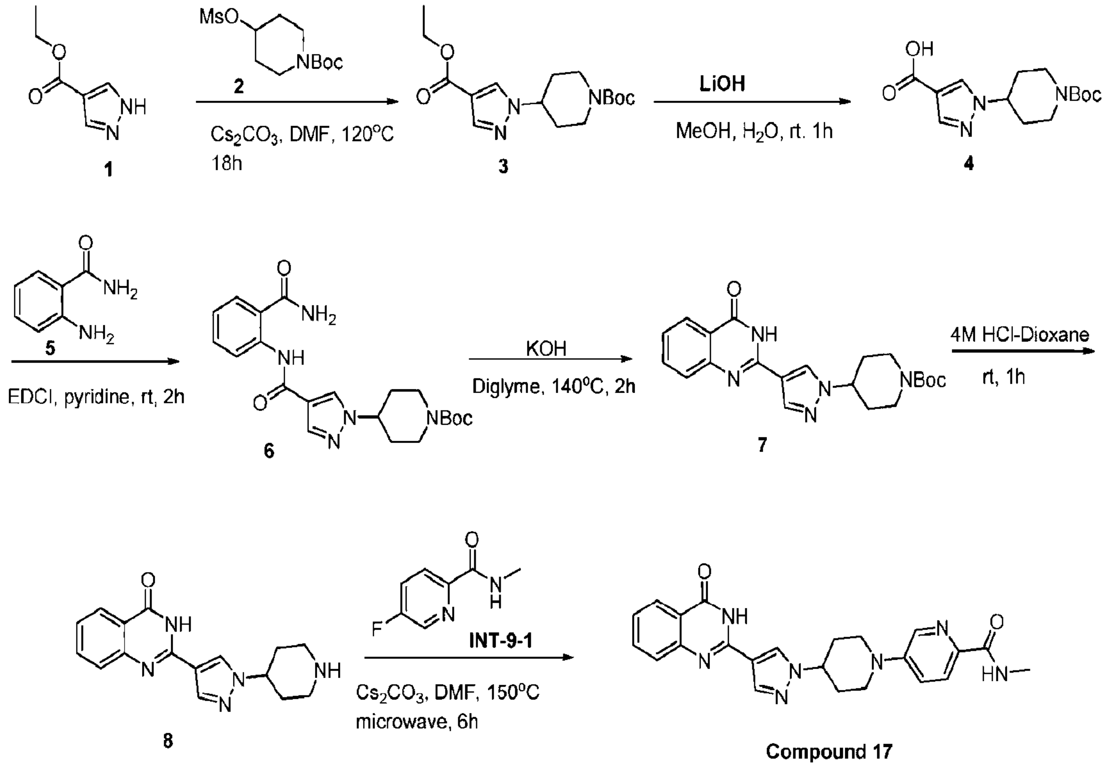


***Preparation of ert-butyl 4-(4-(ethoxycarbonyl)-1H-pyrazol-1-yl)piperidine-1-carboxylate (3)***

To a solution of ethyl 1H-pyrazole-4-carboxylate 1 (2.00 g, 14.30 mmol) and tert-butyl 4-((methylsulfonyl)oxy) piperidine-1-carboxylate 2 (4.99 g, 17.87 mmol) in DMF (60 mL) was added $Cs_2CO_3$ (10.02 g, 30.74 mmol). Then the mixture was stirred for 18 h at 120 °C. The reaction mixture was quenched with water, the aqueous layer was extracted with EtOAc (200 mL x 3). The combined organic layer was washed with brine, dried over $Na_2SO_4$, concentrated under reduced pressure. The residue was purified by flash chromatography (eluting with DCM/MeOH = 100 : 0 to 95 : 5) to give to give product of tert-butyl 4-(4-(ethoxycarbonyl)-1H-pyrazol-1-yl)piperidine-1-carboxylate 3 (2.00 g, 41% yield) as a white solid .

LCMS (ESI) calcd for $C_{16}H_{25}N_3O_4$ $[M + H]^+$ m/z 324.18, found 324.20.

***Preparation of 1-(1-(tert-butoxycarbonyl)piperidin-4-yl)-1H-pyrazole-4-carboxylic acid (4)***

To a solution of tert-butyl 4-(4-(ethoxycarbonyl)-1H-pyrazol-1-yl)piperidine-1-carboxylate **3** (1.50 g, 4.80 mmol) in MeOH/$H_2O$ = 1: 1 (40 mL) was added LiOH (460.00 mg, 19.20 mmol). The mixture was stirred at rt for 1 h. The reaction mixture was concentrated under reduced pressure at 40 °C to obtain residue. To this was added water and acidified with 1 M aqueous HCl to adjust pH= 4 ~ 5 at 0 °C, the aqueous layer was extracted with EtOAc (200 mL x 3). The combined organic layer was washed with brine, dried over $Na_2SO_4$, concentrated under reduced pressure. The residue was purified by flash chromatography (eluting with DCM/MeOH = 100 : 0 to 95 : 5) to give 1-(1-(tert-butoxycarbonyl)piperidin-4-yl)-1H-pyrazole-4-carboxylic acid 4 (1.00 g, 67% yield) as a white solid .

LCMS (ESI) calcd for $C_{14}H_{21}N_3O_4$ $[M - 56 + H]^+$ m/z 240.15, found 240.20.

$^1H$ NMR (400 MHz, DMSO-$d_6$, ppm) δ 12.29 (s, 1 H), 8.30 (s, 1H), 7.81 (s, 1 H), 4.52-4.30 (m, 1 H), 4.03 (d, *J* = 11.2 Hz, 2 H), 2.88 (s, 2 H), 1.99 (d, *J* = 12.4 Hz, 2 H), 1.78 (dt, *J* = 12.4, 8.0 Hz, 2 H), 1.41 (s, 9 H).

***Preparation of tert-butyl 4-(4-((2-carbamoylphenyl)carbamoyl)-1H-pyrazol-1-yl)piperidine-1-carboxylate (6)***

To a solution of 2-aminobenzamide **5** (299.66 mg, 2.20 mmol) and 1-(1-(tert-butoxycarbonyl)piperidin-4-yl)-1H-pyrazole-4-carboxylic acid **4** (650.00 mg, 2.20 mmol) in pyridine (25 mL) was added EDCI (421.91 mg, 2.20 mmol). then the mixture was stirred for 12 h at room temperature. The reaction mixture was quenched with water, the aqueous layer was extracted with EtOAc (50 mL x 3). The combined organic layers was washed by 1M HCl solution and brine, dried over $Na_2SO_4$, concentrated under reduced pressure to give product of tert-butyl 4-(4-((2-carbamoylphenyl)carbamoyl)-1H-pyrazol-1-yl)piperidine-1-carboxylate **6** (720.00 mg, 71% yield) as a white solid.

LCMS (ESI) calcd for $C_{21}H_{27}N_5O_4$ $[M + H]^+$ m/z 414.21, found 414.25.

***Preparation of tert-butyl 4-(4-(4-oxo-3,4-dihydroquinazolin-2-yl)-1H-pyrazol-1-yl)piperidine-1-carboxylate (7)***

To the solution of tert-butyl 4-(4-((2-carbamoylphenyl)carbamoyl)-1H-pyrazol-1-yl)piperidine-1-carboxylate **6** (700.00 mg, 1.69 mmol) in Diglyme (15 mL) was added KOH (113.99 mg, 2.03 mmol), heated to 140 °C stirred for 2 h, cooled to 0 °C, ice-water (10 mL) added then adjusted pH < 7 using 1M HCl solution, precipitate formed, filtered, the filter cake washed with ice-water (50 mL*3), then dried to give product of tert-butyl 4-(4-(4-oxo-3,4-dihydroquinazolin-2-yl)-1H-pyrazol-1-yl)piperidine-1-carboxylate **7** (600 mg, 67% yield) as a white solid .

LCMS (ESI) calcd for $C_{21}H_{25}N_5O_3$ [M + H] $^+$ m/z 396.20, found 396.25.

***Preparation of 2-(1-(piperidin-4-yl)-1H-pyrazol-4-yl)quinazolin-4(3H)-one (8)***

To a solution of tert-butyl 4-(4-(4-oxo-3,4-dihydroquinazolin-2-yl)-1H-pyrazol-1-yl)piperidine-1-carboxylate **7** (350.00 mg, 0.88 mmol) at room temperature was added HCl-Dioxane (4 M, 40 mL). The reaction mixture was stirred at room temperature for 1h. After reaction completed, the mixture was concentrated under reduced pressure to obtain crude 2-(1-(piperidin-4-yl)-1H-pyrazol-4-yl)quinazolin-4(3H)-one 8 (200 mg, 73% yield) as a white solid. LCMS (ESI) calcd for $C_{16}H_{17}N_5O$ [M + H] $^+$ m/z 296.14, found 296.25.

***Preparation of N-methyl-5-(4-(4-(4-oxo-3,4-dihydroquinazolin-2-yl)-1H-pyrazol-1-yl)piperidin-1-yl)picolinamide (Compound 17)***

To a solution 2-(1-(piperidin-4-yl)-1H-pyrazol-4-yl)quinazolin-4(3H)-one **8** (200.00 mg, 0.68 mmol) in DMF (10 mL) was added $Cs_2CO_3$ (2.21 g, 6.77 mmol) and 5-fluoro-N-methylpicolinamide **INT-9-1** (260.96 mg, 1.69 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to give crude product which was purified by prep-HPLC (Gemini 5um C18 150 × 21.2 mm, mobile phase: ACN - $H_2O$ (0.1% FA), gradient: 25 - 75) to give N-methyl-5-(4-(4-(4-oxo-3,4-dihydroquinazolin-2-yl)-1H-pyrazol-1-yl)piperidin-1-yl)picolinamide **Compound 17** (30 mg, 98% purity, 9% yield) as a white solid.

$^1$H NMR (400 MHz, DMSO-$d_6$, ppm) δ 12.30 (s, 1 H), 8.67 (s, 1 H), 8.43-8.40 (m, 1 H), 8.35 (d, *J* = 2.8 Hz, 1 H), 8.26 (s, 1 H), 8.09 (d, *J* = 8.0 Hz, 1 H), 7.85 (d, *J* = 8.8 Hz, 1 H), 7.80-7.76 (m, 1 H), 7.61 (d, *J* = 8.0 Hz, 1 H), 7.50-7.42 (m, 2 H), 4.56-4.53 (m, 1 H), 4.08 (d, *J* = 12.8 Hz, 2 H), 3.10 (t, *J* = 11.6, 10.8 Hz, 2 H), 2.79 (d, *J* = 4.8 Hz, 3 H), 2.19-2.17 (m, 2 H), 2.06-1.98 (m, 2 H).

LCMS (ESI) calcd for $C_{23}H_{23}N_7O_2$ [M + H] $^+$ m/z 430.19, found 430.25.

***<u>Example 9 - Synthesis of Compound 40</u>***

**Compound 40 Synthesis**

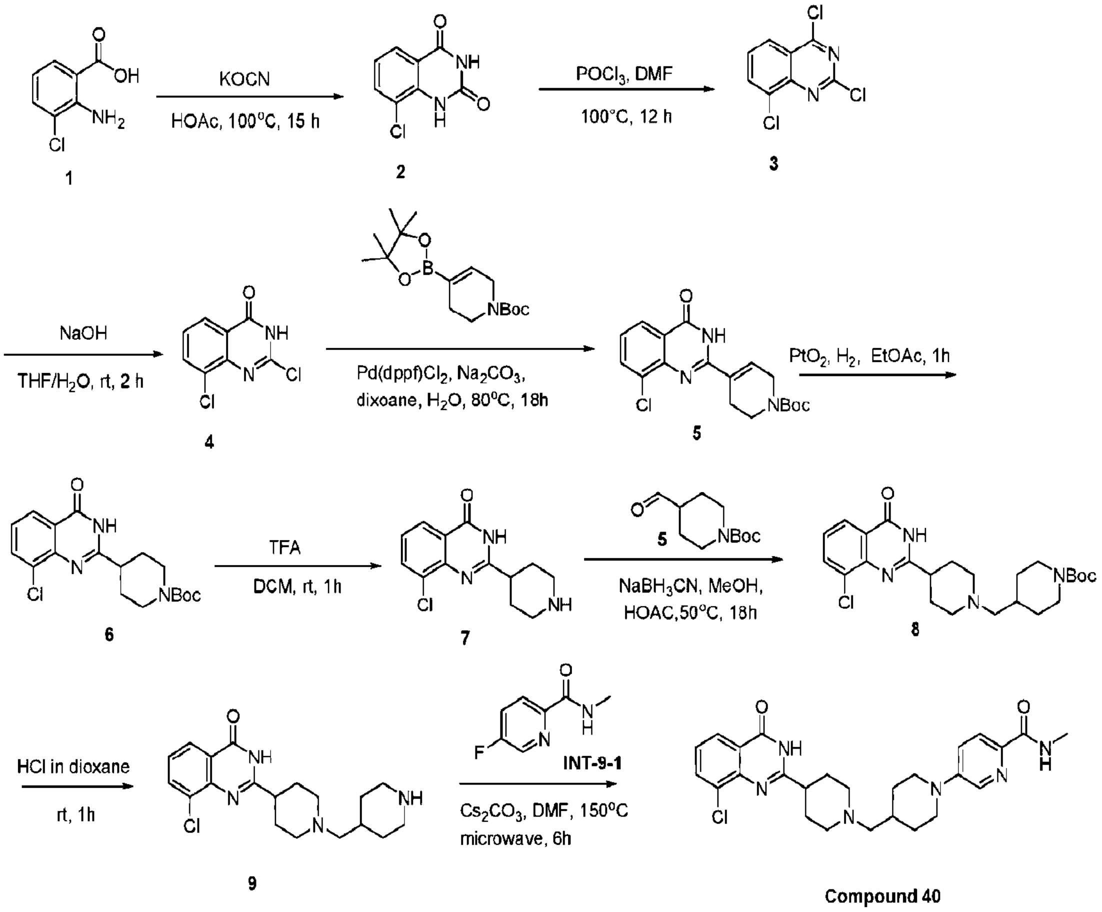


***Preparation of 8-chloroquinazoline-2,4(1H,3H)-dione (2)***

To a solution of 2-amino-3-chlorobenzoic acid 1 (10.00 g, 58.30 mmol) in HOAC (100 mL) was added KOCN (14.19 g, 174.90 mmol) slowly at room temperature. The mixture was heated to 100 °C stirred for 15 hours, cooled to 0 °C, ice-water (30 mL) added. The precipitate was collected by filtration, washed with petroleum ether:EtOAc = 5:1 (100 mL), filtered to provide 8-chloroquinazoline-2,4(1H,3H)-dione 2 (5.00 g, 40% yield) as a white solid which was used directly in next step without further purification.

LCMS (ESI) calcd for $C_8H_5ClN_2O_2$ [M + H] $^+$ m/z 197.00, found 196.95.

***Preparation of 2,4,8-trichloroquinazoline (3)***

To a solution of 8-chloroquinazoline-2,4(1H,3H)-dione 2 (5.00 g, 25.40 mmol) in $POCl_3$ (45 mL) was added DMF (3 mL) slowly at room temperature. The mixture was heated to 100 °C for 12 hours. The resulting mixture was diluted with ice water (200 mL) and extracted with EtOAc (400 mL x 3). The combine organic phases were washed with brine, dried over sodium sulfate, concentrated and purified by silica gel column chromatography (eluting with EtOAc/petroleum ether, 100: 0 to 50 :50) to give 2,4,8-trichloroquinazoline 3 (2.00 g, 32% yield) as a white solid.

LCMS (ESI) calcd for $CsH_3Cl_3N_2$ [M + H] $^+$ m/z 232.94, found 232.90.

***Preparation of 2,8-dichloroquinazolin-4(3H)-one (4)***

To a solution of 2,4,8-trichloroquinazoline 3 (2.00 g, 8.60 mmol) in THF/$H_2O$ = 1:1 (200 mL) was added NaOH (690 mg, 17.20 mmol). The mixture was stirred at room temperature for 2 h. The reaction mixture was concentrated under reduced pressure at 40 °C to obtain residue. To this was added water and acidified with 1 M aqueous HCl to adjust pH 4 ~ 5 at 0 °C. The mixture was extracted with EtOAc (200 mL x 3). The combined organic phases were washed with brine (100 mL × 3), dried over anhydrous sodium sulfate and concentrated in vacuo. The residue was purified by Flash chromatography (eluent: DCM/MeOH =100: 0 to 95 :5) to afford 2,8-dichloroquinazolin-4(3H)-one **4** (1.60 g, 82% yield) as a white solid.

LCMS (ESI) calcd for $C_8H_4C_{12}N_2O$ [M + H] $^+$ m/z 214.97, found 214.95.

***Preparation of tert-butyl 4-(8-chloro-4-oxo-3,4-dihydroquinazolin-2-yl)-3,6-dihydropyridine-1(2H)-carboxylate (5)***

To a solution of 2,8-dichloroquinazolin-4(3H)-one **4** (400 mg, 1.86 mmol) in Dioxane/$H_2O$=10:1 (40 mL) were added tert-butyl 4-(4,4,5,5-tetramethyl-1,3,2-dioxaborolan-2-yl)-3,6-dihydropyridine-1(2H)-carboxylate (690 mg, 2.23 mmol), Pd(dppf)$Cl_2$ (50 mg, 0.07 mmol) and $Na_2CO_3$ (591 mg, 5.58 mmol) successively at room temperature. The reaction mixture was stirred at 80 °C for 18 h under $N_2$. The mixture was diluted with water (50 mL) and extracted with EtOAc (100 mL × 3). The combined organic layer was washed with brine (100 mL × 2), dried over $Na_2SO_4$, filtered and concentrated in vacuo to get crude product, which was purified by flash column chromatography (PE/EtOAc = 100: 0 to 20: 80) to afford tert-butyl 4-(8-chloro-4-oxo-3,4-dihydroquinazolin-2-yl)-3,6-dihydropyridine-1(2H)-carboxylate 5 (550 mg, 77% yield) as a yellow solid.

LCMS (ESI) calcd for $C_{18}H_{20}ClN_3O_3$ [M + H] $^+$ m/z 362.12, found 362.10.

***Preparation of tert-butyl 4-(8-chloro-4-oxo-3,4-dihydroquinazolin-2-yl)piperidine-1-carboxylate (6)***

To a solution of tert-butyl 4-(8-chloro-4-oxo-3,4-dihydroquinazolin-2-yl)-3,6-dihydropyridine-1(2H)-carboxylate 5 (550 mg, 1.52 mmol) in EtOAc (200 mL) was added $PtO_2$ (110 mg). The mixture was evacuated and backfilled with hydrogen three times and then charged with hydrogen. The resulting mixture was stirred at room temperature for 1 hour. Then the mixture was filtered through celite and concentrated under vacuum to give crude product, which was purified by flash column chromatography (PE/EtOAc = 100: 0 to 50: 50) to afford tert-butyl 4-(8-chloro-4-oxo-3,4-dihydroquinazolin-2-yl)piperidine-1-carboxylate 6 (400 mg, 65% yield) as a yellow solid.

LCMS (ESI) calcd for $C_{18}H_{22}ClN_3O_3$ [M + H] $^+$ m/z 364.13, found 364.10.

***Preparation of 8-chloro-2-(piperidin-4-yl)quinazolin-4(3H)-one (7)***

To a solution of tert-butyl 4-(8-chloro-4-oxo-3,4-dihydroquinazolin-2-yl)piperidine-1-carboxylate **6** (400 mg, 1.21 mmol) in DCM (5 mL) was added TFA (2 mL) , stirred for 1 h at room temperature, the mixture was concentrated under reduced pressure to give crude product, ice-water (2 mL) added then adjusted pH > 7 using ammonium hydroxide solution, precipitate formed, filtered, the filter cake washed with ice-water (5 mL*3), then dried to give product of 8-chloro-2-(piperidin-4-yl)quinazolin-4(3H)-one **7** (300 mg, 89% yield) as a white solid .

LCMS (ESI) calcd for $C_{13}H_{14}ClN_3O$ [M + H] $^+$ m/z 264.08, found 264.10.

***Preparation of tert-butyl 4-((4-(8-chloro-4-oxo-3,4-dihydroquinazolin-2-yl)piperidin-1-yl)methyl)piperidine-1-carboxylate (8)***

To a solution of 8-chloro-2-(piperidin-4-yl)quinazolin-4(3H)-one **7** (300 mg, 1.14 mmol) in MeOH (60 mL) was added tert-butyl 4-oxopiperidine-1-carboxylate (727 mg, 3.41 mmol), then two drops of acetic acid and $NaBH_3CN$ (428 mg, 6.82 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 90 : 10) to give tert-butyl 4-((4-(8-chloro-4-oxo-3,4-dihydroquinazolin-2-yl)piperidin-1-yl)methyl)piperidine-1-carboxylate 8 (300 mg, 51% yield ) as a white solid.

LCMS (ESI) calcd for $C_{24}H_{33}ClN_4O_3$ [M + H] $^+$ m/z 361.17, found 361.05.

***Preparation of 8-chloro-2-(1-(piperidin-4-ylmethyl)piperidin-4-yl)quinazolin-4(3H)-one (9)***

Tert-butyl 4-((4-(8-chloro-4-oxo-3,4-dihydroquinazolin-2-yl)piperidin-1-yl)methyl)piperidine-1-carboxylate **8** (300 mg, 0.65 mmol) was added to HCl in dioxane (4 M, 30 mL), stirred for 1 h at room temperature, the reaction mixture was concentrated under reduced pressure to give 8-chloro-2-(1-(piperidin-4-ylmethyl)piperidin-4-yl)quinazolin-4(3H)-one **9** (200 mg, 81% yield ) as a white solid.

LCMS (ESI) calcd for $C_{19}H_{25}ClN_4O$ [M + H] $^+$ m/z 361.17, found 361.05.

***Preparation of 5-(4-((4-(8-chloro-4-oxo-3,4-dihydroquinazolin-2-yl)piperidin-1-yl)methyl)piperidin-1-yl)-N-methylpicolinamide (Compound 40)***

To a solution 8-chloro-2-(1-(piperidin-4-ylmethyl)piperidin-4-yl)quinazolin-4(3H)-one 9 (150 mg, 0.42 mmol) in DMF (10 mL) were added $Cs_2CO_3$ (1.35 g, 4.16 mmol) and 5-fluoro-N-methylpicolinamide **INT-9-1** (160 mg, 1.03 mmol). The mixture was stirred for 6 h under 150 °C using microwave. The reaction mixture was concentrated under reduced pressure. The residue was purified by flash chromatography (eluting with DCM/MeOH = 100 : 0 to 90 : 10) to give crude

5-(4-((4-(8-chloro-4-oxo-3,4-dihydroquinazolin-2-yl)piperidin-1-yl)methyl)piperidin-1-yl)-N-methylpicolinamide **Compound 40** as a yellow solid. The crude product was purified by prep-HPLC (Gemini 5um C18 150 × 21.2 mm, mobile phase: ACN - $H_2O$ (0.1% FA), gradient: 20 - 80) to give 5-(4-((4-(8-chloro-4-oxo-3,4-dihydroquinazolin-2-yl)piperidin-1-yl)methyl)piperidin-1-yl)-N-methylpicolinamide **Compound 40** (24.2 mg, 96% purity, 11% yield) as a white solid.

$^1$H NMR (400 MHz, DMSO-$d_6$, ppm) δ 12.39 (s, 1 H), 8.40-8.37 (m, 1 H), 8.26 (d, $J$ = 2.8 Hz, 1 H), 8.04 (dd, $J$ = 7.6, 1.2 Hz, 1 H), 7.94 (dd, $J$ = 7.8, 1.2 Hz, 1 H), 7.81 (d, $J$ = 8.8 Hz, 1 H), 7.46-7.36 (m, 2 H), 3.91 (d, $J$ = 12.8Hz, 2 H), 2.97-2.94 (m, 2 H), 2.86-2.81 (m, 2 H), 2.77 (d, $J$ = 4.8 Hz, 3 H), 2.65-2.53 (m, 1 H), 2.18 (d, $J$ = 6.8 Hz, 2 H), 1.97-1.80 (m, 9 H), 1.23-1.17 (m, 2 H).

LCMS (ESI) calcd for $C_{26}H_{31}ClN_6O_2$ [M + H] $^+$ m/z 495.22, found 495.15.

***Example 10 - Synthesis of Compound 52***

**Compound 52 synthesis**

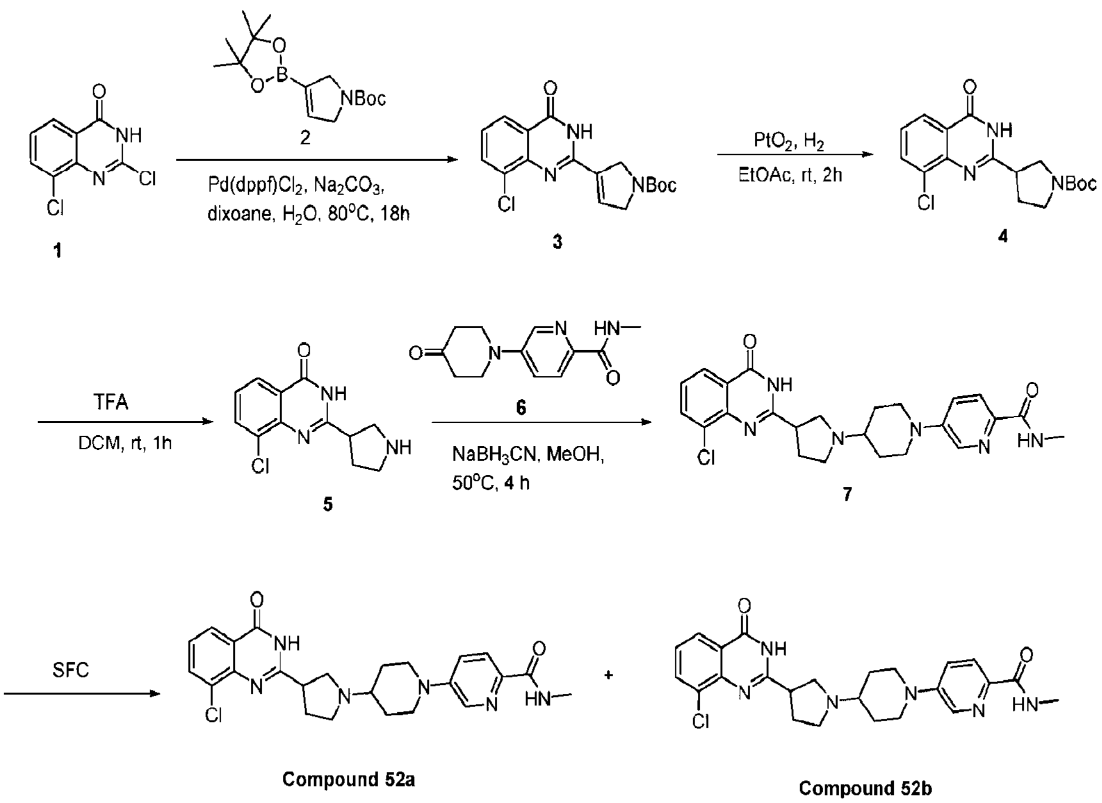


1, 3, 4, 5, 7

Compound 52a + Compound 52b

***Preparation of tert-butyl 3-(8-chloro-4-oxo-3,4-dihydroquinazolin-2-yl)-2,5-dihydro-1H-pyrrole-1-carboxylate (3)***

To a solution of 2,8-dichloroquinazolin-4(3H)-one **1** (500 mg, 2.35 mmol) in 1,4-dioxanc/$H_2O$=5:1 (60 mL) was added tert-butyl 3-(4,4,5,5-tetramethyl-1,3,2-dioxaborolan-2-yl)-2,5-dihydro-1H-pyrrole-1-carboxylate **2** (900 mg, 3.00 mmol), Pd(dppf)$Cl_2$ (170 mg, 0.23 mmol) and $Na_2CO_3$ (740 mg, 6.98 mmol) under $N_2$. The reaction mixture was stirred at 80 °C for 18 h. After cooling to room temperature, the reaction mixture was concentrated under reduced pressure. The residue was purified by flash chromatography (eluting with DCM/MeOH = 100 : 0 to 91 : 9) to give tert-butyl 3-(8-chloro-4-oxo-3,4-dihydroquinazolin-2-yl)-2,5-dihydro-1H-pyrrole-1-carboxylate **3** (400 mg, 40% yield ) as a white solid.

LCMS (ESI) calcd for C17H18ClN3O3 [M + H] $^+$ m/z 348.10, found 348.17.

***Preparation of tert-butyl 3-(8-chloro-4-oxo-3,4-dihydroquinazolin-2-yl)pyrrolidine-1-carboxylate (4)***

To a solution of tert-butyl 3-(8-chloro-4-oxo-3,4-dihydroquinazolin-2-yl)-2,5-dihydro-1H-pyrrole-1-carboxylate **3** (400 mg, 1.15 mmol) in EtOAc (50 mL) was added $PtO_2$ (80 mg, 0.35 mmol) under $H_2$. The reaction mixture stirred for 2h at

room temperature. The mixture was filtered through a Celite pad, and the filtrate was concentrated to give crude product of tert-butyl 3-(8-chloro-4-oxo-3,4-dihydroquinazolin-2-yl)pyrrolidine-1-carboxylate 4 (200 mg, 50 % yield ) as a white solid.

LCMS (ESI) calcd for Cl7H20ClN3O3 [M + H] + m/z 350.12, found 350.18.

***Preparation of 8-chloro-2-(pyrrolidin-3-yl)quinazolin-4(3H)-one (5)***

Tert-butyl 3-(8-chloro-4-oxo-3,4-dihydroquinazolin-2-yl)pyrrolidine-1-carboxylate **4** (200 mg, 0.57 mmol) was added to HCl in dioxane (4 M, 5 mL), stirred for 1 h at room temperature, the reaction mixture was concentrated under reduced pressure to give product of 8-chloro-2-(pyrrolidin-3-yl)quinazolin-4(3H)-one **5** (130 mg, 82% yield) as a white solid .

LCMS (ESI) calcd for C12H12ClN3O [M + H] + m/z 250.07, found 250.12.

***Preparation of 5-(4-(3-(8-chloro-4-oxo-3,4-dihydroquinazolin-2-yl)pyrrolidin-1-yl)piperidin-1-yl)-N-methylpicolinamide (7)***

To a solution of 8-chloro-2-(pyrrolidin-3-yl)quinazolin-4(3H)-one **5** (80 mg, 0.32 mmol) in MeOH (10 mL) was added N-methyl-5-(4-oxopiperidin-1-yl)picolinamide **6** (50 mg, 0.21 mmol), then three drops of acetic acid and $NaBH_3CN$ (15 mg, 0.24 mmol) were added at room temperature. The reaction mixture stirred at 50 °C for 4 h. After cooling to room temperature, the reaction mixture was concentrated under reduced pressure. The residue was purified by flash chromatography (eluting with DCM/MeOH = 100 : 0 to 92 : 8) to give 5-(4-(3-(8-chloro-4-oxo-3,4-dihydroquinazolin-2-yl)pyrrolidin-1-yl)piperidin-1-yl)-N-methylpicolinamide 7 (40 mg, 40% yield ) as a white solid.

LCMS (ESI) calcd for C24H27ClN6O2 [M + H] + m/z 467.19, found 467.28.

***Preparation of 5-(4-(3-(8-chloro-4-oxo-3,4-dihydroquinazolin-2-yl)pyrrolidin-1-yl)piperidin-1-yl)-N-methylpicolinamide (Compound 52a and Compound 52b)***

5-(4-(3-(8-chloro-4-oxo-3,4-dihydroquinazolin-2-yl)pyrrolidin-1-yl)piperidin-1-yl)-N-methylpicolinamide **7** was separated by SFC (Column: DAICEL AS-H 4.6mmI.D.*250mmL 5μm; Mobile phase: CO2/IPA[0.1%NH3(7M Solution in MeOH)]=60/40) and concentrated under reduced pressure to afford the first fraction as **Compound 52a** (14.4 mg, 97% purity, ee%: 100, white solid) and the second fraction as **Compound 52b** (15.4 mg, 99% purity, ee%: 100, white solid)

**Compound 52a**

$^1$H NMR (400 MHz, DMSO) δ 8.42 (d, *J* = 4.8 Hz, 1 H), 8.27 (d, *J* = 2.8 Hz, 1 H), 8.05 (d, *J* = 8.0 Hz, 1 H), 7.94 (d, *J* = 8.0 Hz, 1 H), 7.82 (d, *J* = 8.8 Hz, 1 H), 7.48-7.38 (m, 2 H), 3.83 (d, *J* = 12.8 Hz, 2 H), 3.40-3.32 (m, 1 H), 3.06 (t, *J* = 9.2 Hz, 1 H), 2.95 (dd, *J* = 22.0, 12.0 Hz, 3 H), 2.80-2.68 (m, 5 H), 2.38 (t, *J* = 10.4 Hz, 1 H), 2.24-2.15 (m, 2 H), 1.96 (d, *J* = 11.2 Hz, 2 H), 1.53 (dd, *J* = 22.8, 10.8 Hz, 2 H).

LCMS (ESI) calcd for C24H27ClN6O2 [M + H] + m/z 467.19, found 467.28.

**Compound 52b**

$^1$H NMR (400 MHz, DMSO) δ 8.49 (d, *J* = 4.8 Hz, 1 H), 8.34 (d, *J* = 2.4 Hz, 1 H), 8.11 (d, *J* = 7.6 Hz, 1 H), 8.01 (d, *J* = 7.6 Hz, 1 H), 7.89 (d, *J* = 8.8 Hz, 1 H), 7.55-7.45 (m, 2 H), 3.90 (d, *J* = 12.4 Hz, 2 H), 3.45-3.39 (m, 1 H), 3.13 (t, *J* = 8.8 Hz, 1 H), 3.01 (dd, *J* = 22.8, 11.6 Hz, 3 H), 2.87-2.77 (m, 5 H), 2.43 (d, *J* = 10.0 Hz, 1 H), 2.26 (d, *J* = 6.4 Hz, 2 H), 2.02 (d, *J* = 11.2 Hz, 2 H), 1.59 (d, *J* = 12.0 Hz, 2 H).

LCMS (ESI) calcd for C24H27ClN6O2 [M + H] + m/z 467.19, found 467.28.

***Example 11 - Synthesis of Compound 54***

**Compound 54 synthesis**

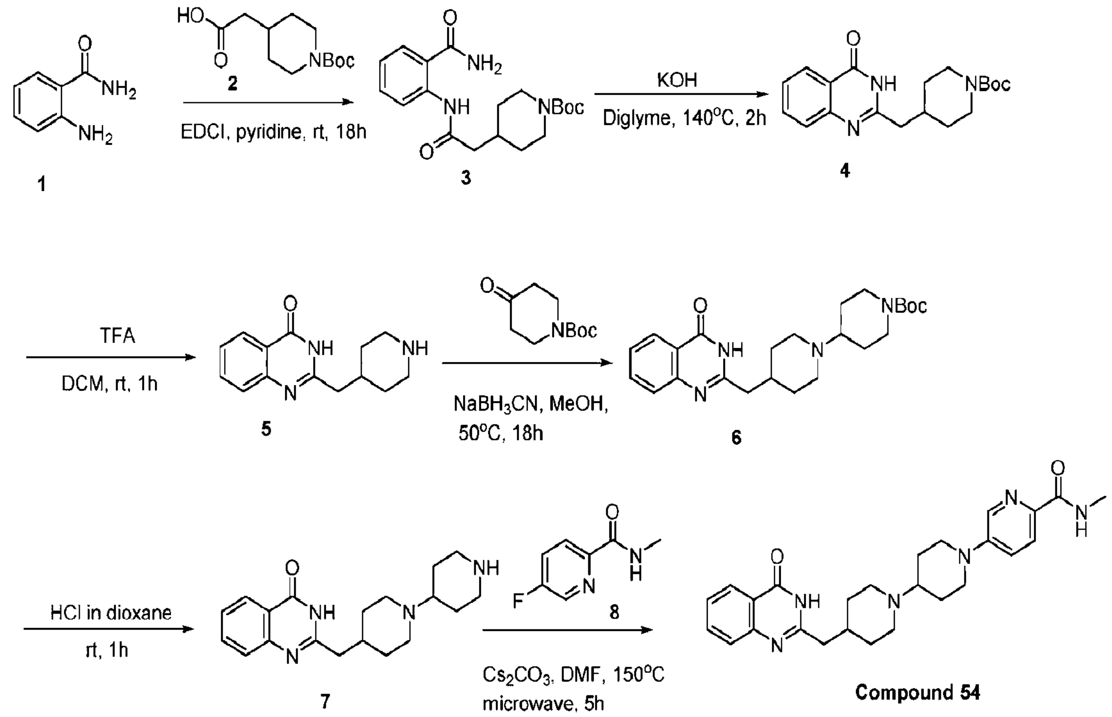


1 3 4 5 6 7 Compound 54

***Preparation of tert-butyl 4-{[(2-carbamoylphenyl)carbamoyl]methyl}piperidine-1-carboxylate (3)***

To a solution of 2-aminobenzamide **1** (2.00 g, 14.70 mmol) and {1-[(tert-butoxy)carbonyl]piperidin-4-yl}acetic acid **2** (3.58 g, 14.70 mmol) in pyridine (50 mL) was added EDCI (2. 82 g, 14.70 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over $Na_2SO4$, concentrated under reduced pressure to give tert-butyl 4-{[(2-carbamoylphenyl)carbamoyl]methyl}piperidine-1-carboxylate 3 (5.76 g, 92% yield) as a yellow oil .

LCMS (ESI) calcd for C19H27N3O4 $[M + H]^+$ m/z 362.20, found 262.30.

***Preparation of tert-butyl 4-[(4-oxo-3H-quinazolin-2-yl)methyl]piperidine-1-carboxylate (4)***

To the solution of tert-butyl 4-{[(2-carbamoylphenyl)carbamoyl]methyl}piperidine-1-carboxylate 3 (5.76 g, 15.90 mmol) in Diglyme (40 mL) was added KOH (1.16 g, 20.67 mmol), heated to 140 °C stirred for 2 h, cooled to 0 °C, ice-water (50 mL) added then adjusted pH < 7 using 1M HCl solution, precipitate formed, filtered, the filter cake washed with ice-water (50 mL*3), then dried to give product of tert-butyl 4-[(4-oxo-3H-quinazolin-2-yl)methyl]piperidine-1-carboxylate **4** (4.20 g, 73% yield) as a white solid .

LCMS (ESI) calcd for C19H25N3O3 $[M + H]^+$ m/z 344.19, found 344.25.

***Preparation of 2-(piperidin-4-ylmethyl)-3H-quinazolin-4-one (5)***

To a solution of tert-butyl 4-[(4-oxo-3H-quinazolin-2-yl)methyl]piperidine-1-carboxylate **4** (4.20 g, 10.00 mmol) in DCM (13 mL) was added TFA (13 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-4-ylmethyl)-3H-quinazolin-4-one **5** (2.32 g, 90% yield) as a white solid .

LCMS (ESI) calcd for C14H17N3O $[M + H]^+$ m/z 244.14, found 244.25.

***Preparation of tert-butyl 4-{4-[(4-oxo-3H-quinazolin-2-yl)methyl]piperidin-1-yl}piperidine-1-carboxylate (6)***

To a solution of 2-(piperidin-4-ylmethyl)-3H-quinazolin-4-one 5 (500 mg, 2.06 mmol) in MeOH (50 mL) was added tert-butyl 4-oxopiperidine-1-carboxylate (532 mg, 2.67 mmol), then two drops of acetic acid and $NaBH_3CN$ (504 mg, 8.01 mmol) were added at room temperature. The reaction mixture stirred at 50 °C for 18 h. After cooling to room temperature, the reaction mixture was concentrated under reduced pressure. The residue was purified by flash chromatography (eluting

with DCM/MeOH = 100 : 0 to 91 : 9) to give tert-butyl 4-{4-[(4-oxo-3H-quinazolin-2-yl)methyl]piperidin-1-yl}piperidine-1-carboxylate **6** (244 mg, 25% yield ) as a white solid.

LCMS (ESI) calcd for C24H34N4O3 [M + H] + m/z 427.26, found 427.40.

***Preparation of tert-butyl 4-[(4-oxo-3H-quinazolin-2-yl)methyl]piperidine-1-carboxylate (7)***

Tert-butyl 4-{4-[(4-oxo-3H-quinazolin-2-yl)methyl]piperidin-1-yl}piperidine-1-carboxylate 6 (244 mg, 0.57 mmol) was added to HCl in dioxane (4 M, 8 mL), stirred for 1 h at room temperature, the reaction mixture was concentrated under reduced pressure to give 2-{[1-(piperidin-4-yl)piperidin-4-yl]methyl}-3H-quinazolin-4-one **7** (187 mg, 90% yield ) as a white solid.

LCMS (ESI) calcd for C19H26N4O [M + H] + m/z 327.21, found 327.44.

***Preparation of N-methyl-5-(4-{4-[(4-oxo-3H-quinazolin-2-yl)methyl]piperidin-1-yl}piperidin-1-yl)pyridine-2-carboxamide (Compound 54)***

To a solution of 2-{[1-(piperidin-4-yl)piperidin-4-yl]methyl}-3H-quinazolin-4-one 7 (200 mg, 0.61 mmol) in DMF (16 mL) were added $Cs_2CO_3$ (1.20 g, 3.68 mmol) and 5-fluoro-N-methylpyridine-2-carboxamide 5 (208 mg, 1.35 mmol). The mixture was stirred for 5 h under 150 °C using microwave. The reaction mixture was concentrated under reduced pressure. The residue was purified by flash chromatography (eluting with DCM/MeOH = 100 : 0 to 91 : 9) to give N-methyl-5-(4-{4-[(4-oxo-3H-quinazolin-2-yl)methyl]piperidin-1-yl}piperidin-1-yl)pyridine-2-carboxamide **Compound 54** (20.11 mg, 99% purity, 7% yield) as a white solid.

$^1$H NMR (400 MHz, DMSO) δ 12.16 (s, 1 H), 8.37 (d, *J* = 4.8 Hz, 1 H), 8.25 (d, *J* = 2.8 Hz, 1 H), 8.07 (d, *J* = 8.0 Hz, 1 H), 7.83-7.74 (m, 2 H), 7.59 (d, *J* = 8.0 Hz, 1 H), 7.45 (t, *J* = 7.6 Hz, 1 H), 7.37 (dd, *J* = 8.8, 2.8 Hz, 1 H), 3.93 (d, *J* = 12.4 Hz, 2 H), 2.90-2.74 (m, 8 H), 2.14 (t, *J* = 10.8 Hz, 2 H), 1.81 (d, *J* = 11.6 Hz, 3 H), 1.64 (d, *J* = 10.8 Hz, 2 H), 1.49 (d, *J* = 8.8 Hz, 2 H), 1.23 (d, *J* = 9.2 Hz, 3 H).

LCMS (ESI) calcd for C24H28N6O2 [M + H] + m/z 461.26, found 461.40.

***Example 12 - Synthesis of Compound 136rac, 136a and 136b***

**Synthesis of Compound *136rac,* 136a and 136b**

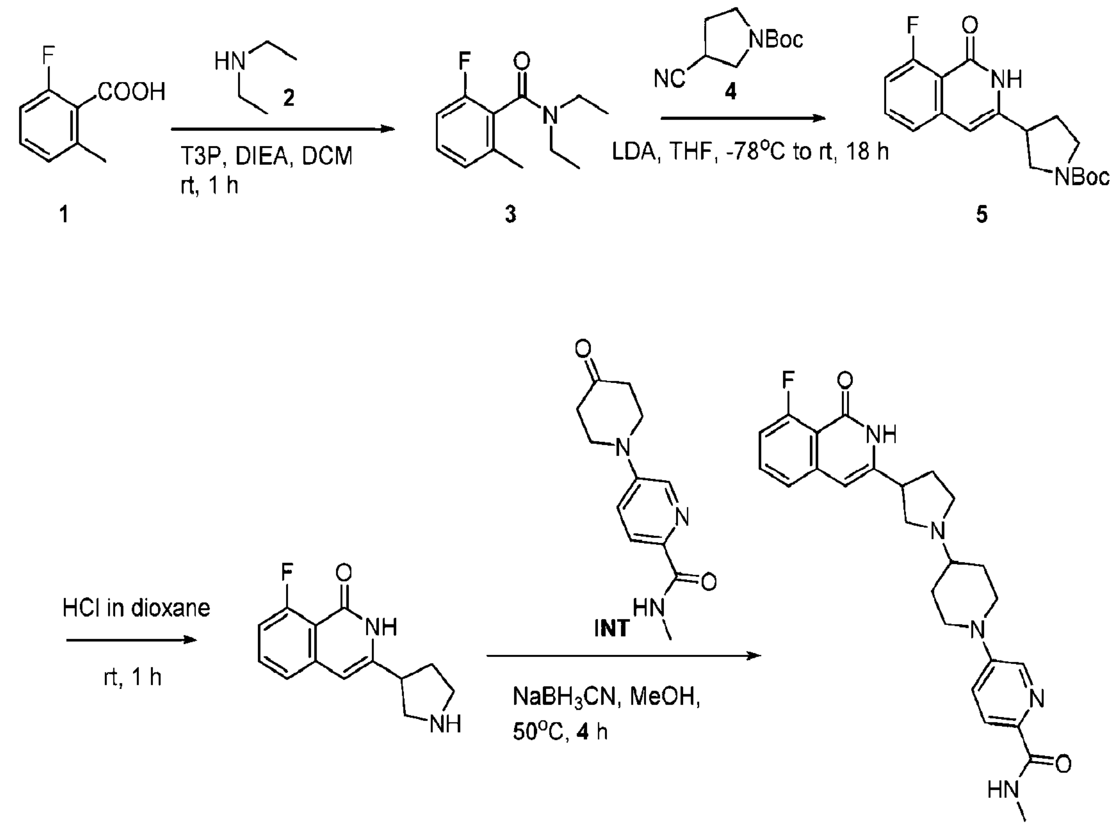


1 3 5

6 136rac

***Preparation of N,N-diethyl-2-fluoro-6-methylbenzamide (3)***

To a solution of 2-fluoro-6-methylbenzoic acid 1 (1.00 g, 6.50 mmol) and diethylamine 2 (0.57 g, 7.80 mmol) in DCM (50 mL) was added tritolylphosphine (50% in EtOAc) (3.10 g, 9.75 mmol) and DIEA (4.20 g, 32.50 mmol) at room temperature. The mixture was stirred for 1 h at room temperature. The reaction mixture was concentrated under reduced pressure. The residue was purified by flash chromatography (eluting with PE/EtOAC = 100 : 0 to 90 : 10) to give N,N-diethyl-2-fluoro-6-methylbenzamide 3 (0.90 g, 63% yield ) as a white solid.

LCMS (ESI) calcd for C12H16FNO $[M + H]^+$ m/z 210.12, found 210.09.

***Preparation of 3-hydroxy-1-(1-methylphenyl)pyrrolidine-3-carboxylic acid (5)***

A dry three-necked round-bottomed flask with an addition funnel at -78°C under inert atmosphere was charged with anhydrous THF (30 ml). A solution of LDA (2.5 M in THF, 0.9 ml) was added dropwise. The internal temperature was maintained below -70°C during the entire addition process. After 0.5 h at -78°C a solution of N,N-diethyl-2-fluoro-6-methylbenzamide 3 (300 mg, 1.43 mmol) in anhydrous THF (10 ml) was added dropwise and the mixture was stirred for 1 h, then tert-butyl 3-cyanopyrrolidine-1-carboxylate **4** (366 mg, 1.86 mmol) was added at -78°C. The mixture was stirred for 18 h at room temperature. The reaction mixture was concentrated under reduced pressure. The residue was purified by flash chromatography (eluting with PE/EtOAC = 100 : 0 to 80 : 20) to give 3-hydroxy-1-(1-methylphenyl)pyrrolidine-3-carboxylic acid 5 (160 mg, 32% yield ) as a white solid.

LCMS (ESI) calcd for Cl8H21FN2O3 $[M + H]^+$ m/z 333.15, found 333.25.

***Preparation of 8-fluoro-3-(pyrrolidin-3-yl)-2H-isoquinolin-1-one (6)***

3-Hydroxy-1-(1-methylphenyl)pyrrolidine-3-carboxylic acid **5** (160 mg, 0.48 mmol) was added to HCl-dioxane (4M, 10 mL). The mixture was stirred for 1 h at room temperature. The reaction mixture was concentrated under reduced pressure to give 8-fluoro-3-(pyrrolidin-3-yl)-2H-isoquinolin-1-one **6** (120 mg, 97% yield) as a white solid.

LCMS (ESI) calcd for Cl3H13FN2O $[M + H]^+$ m/z 233.10, found 233.12.

***Preparation of 5-{4-[3-(8-fluoro-1-oxo-2H-isoquinolin-3-yl)pyrrolidin-1-yl]piperidin-1-yl}-N-methylpyridine-2-carboxamide (Compound 136rac)***

To a solution of 8-fluoro-3-(pyrrolidin-3-yl)-2H-isoquinolin-1-one **6** (120 mg, 0.52 mmol) in MeOH (15 mL) was added N-methyl-5-(4-oxopiperidin-1-yl)pyridine-2-carboxamide **INT** (100 mg, 0.55 mmol), then two drops of acetic acid and $NaBH_3CN$ (30 mg, 0.46 mmol) were added at room temperature. The reaction mixture stirred at 50 °C for 4 h. After cooling to room temperature, the reaction mixture was concentrated under reduced pressure. The residue was purified by flash chromatography (eluting with DCM/MeOH = 100 : 0 to 92 : 8) to give 5-{4-[3-(8-fluoro-1-oxo-2H-isoquinolin-3-yl) pyrrolidin-1-yllpiperidin-1-yl}-N-methylpyridine-2-carboxamide **Compound 136rac** (65 mg, 25% yield, 99% purity) as a yellow solid.

**Compound 136rac**

$^1$H NMR (400 MHz, DMSO) δ 11.10 (s, 1 H), 8.41-8.38 (m, 1 H), 8.28 (d, *J* = 2.8 Hz, 1 H), 7.83 (d, *J* = 8.8 Hz, 1 H), 7.62-7.58 (m, 1 H), 7.45-7.36 (m, 2 H), 7.10 (dd, *J* = 11.6, 8.0 Hz, 1 H), 6.46 (d, *J* = 1.6 Hz, 1 H), 3.84-3.80 (m, 2 H), 3.23-3.16 (m, 1 H), 3.00-2.92 (m, 3 H), 2.84-2.65 (m, 6 H), 2.38-2.33 (m, 1 H), 2.24-2.18 (m, 1 H), 1.97-1.97 (m, 2 H), 1.84-1.76 (m, 1 H), 1.60-1.49 (m, 2 H).

LCMS (ESI) calcd for C25H28FN5O2 [M + H] $^+$ m/z 450.22, found 450.30.

***Preparation of 5-{4-[3-(8-fluoro-1-oxo-2H-isoquinolin-3-yl)pyrrolidin-1-yl]piperidin-1-yl}-N-methylpyridine-2-carboxamide (Compound 136a and Compound 136b)***

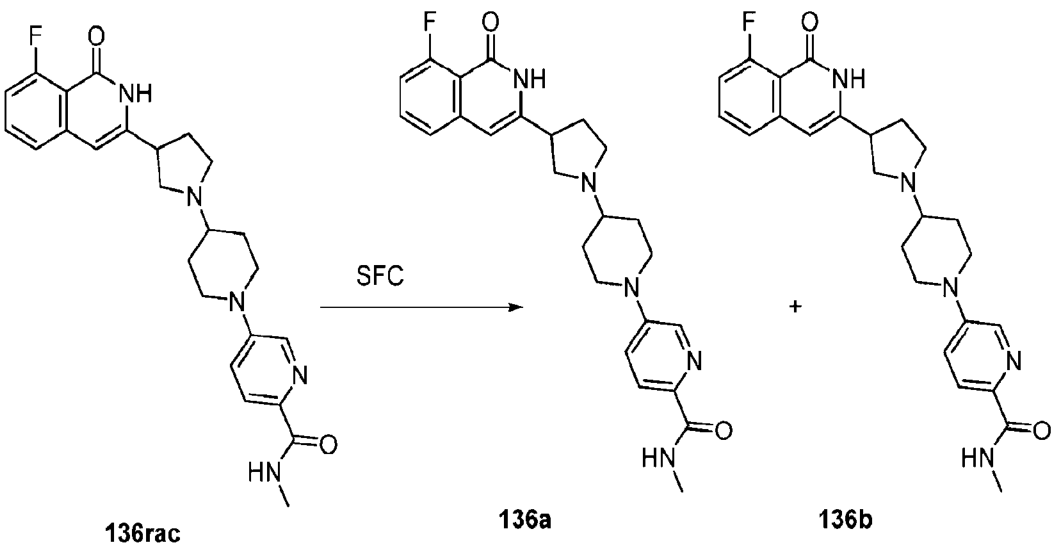


**136rac** **136a** **136b**

5-{4-[3-(8-fluoro-1-oxo-2H-isoquinolin-3-yl)pyrrolidin-1-yl]piperidin-1-yl}- N-methylpyridine-2-carboxamide 136rac was separated by SFC (Column: Daicel CHIRALPAK OJ-H 250 mm × 20 mm I.D., 5 μmm; Mobile phase: $CO_2$/MeOH[0.1%($NH_3$)] = 70/30) and concentrated under reduced pressure to afford the first fraction as Compound 136a (12.8 mg, 99% purity, ee%: 100, white solid) and the second fraction as **Compound 136b** (13.8 mg, 99% purity, ee%: 97, white solid)

**Compound 136a**

$^1$H NMR (400 MHz, DMSO) δ 11.10 (s, 1 H), 8.41 (q, *J* = 4.4 Hz, 1 H), 8.28 (d, *J* = 2.8 Hz, 1 H), 7.83 (d, *J* = 8.8 Hz, 1 H), 7.62-7.58 (m, 1 H), 7.42-7.36 (m, 2 H), 7.10 (dd, *J* = 12.0, 8.0 Hz, 1 H), 6.46 (s, 1 H), 3.84-3.80 (m, 2 H), 3.22-3.15 (m, 1 H), 3.00-2.90 (m, 3 H), 2.81-2.63 (m, 6 H), 2.36-2.31 (m, 1 H), 2.23-2.18 (m, 1 H), 1.97-1.93 (m, 2 H), 1.87-1.79 (m, 1 H), 1.56-1.52 (m, 2 H).

LCMS (ESI) calcd for C25H28FN5O2 [M + H] $^+$ m/z 450.22, found 450.20.

**Compound 136b**

$^1H$ NMR (400 MHz, DMSO) δ 11.10 (s, 1 H), 8.40 (q, *J* = 4.8 Hz, 1 H), 8.28 (d, *J* = 2.8 Hz, 1 H), 7.82 (d, *J* = 8.8 Hz, 1 H), 7.61-7.58 (m, 1 H), 7.42-7.36 (m, 2 H), 7.10 (dd, *J* = 11.6, 8.0 Hz, 1 H), 6.46 (s, 1 H), 3.84-3.80 (m, 2 H), 3.22-3.15 (m, 1 H), 3.00-2.90 (m, 3 H), 2.81-2.63 (m, 6 H), 2.36-2.31 (m, 1 H), 2.25-2.18 (m, 1 H), 1.97-1.94 (m, 2 H), 1.87-1.79 (m, 1H), 1.56-1.52 (m, 2 H).

LCMS (ESI) calcd for C25H28FN5O2 [M + H] + m/z 450.22, found 450.25.

***Example 13 - Synthesis of Compounds 151rac, 151a and 151b***

**Synthesis of Compounds 151rac, 151a and 151b**

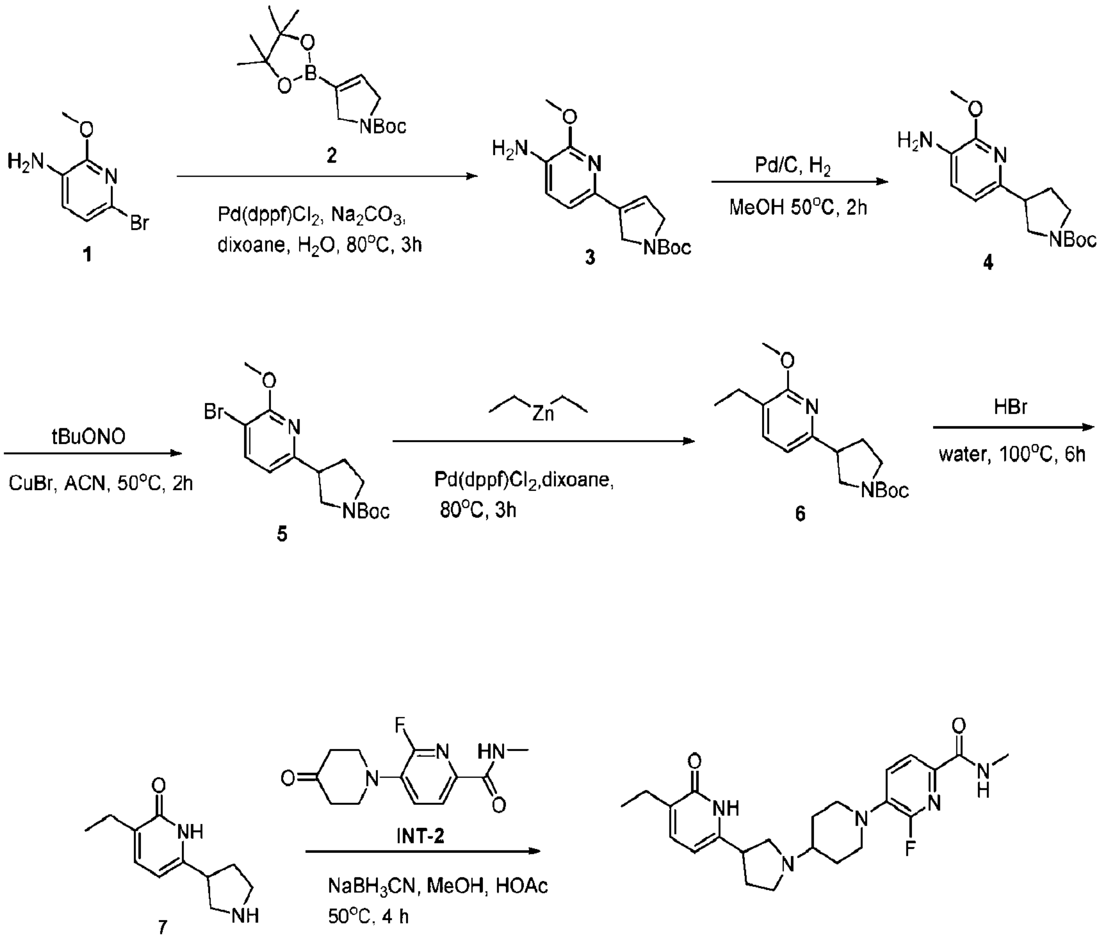


**151rac**

**Preparation of tert-butyl 3-(5-amino-6-methoxypyridin-2-yl)-2,5-dihydropyrrole-1-carboxylate (3)**

To a solution of 6-bromo-2-methoxypyridin-3-amine 1 (1.38 g, 6.80 mmol) in 1,4-dioxane/$H_2O$=4:1 (20 mL) was added tert-butyl 3-(4,4,5,5-tetramethyl-1,3,2-dioxaborolan-2-yl)-2,5-dihydropyrrole-1-carboxylate 2 (2.01 g, 6.80 mmol), then $Pd(dppf)Cl_2$ (0.50 g, 0.68 mmol) and $Na_2CO_3$ (2.23 g, 21.08 mmol) were added at room temperature. The reaction mixture was stirred under nitrogen at 80 °C for 3 h under $N_2$. After cooling to room temperature, the reaction mixture was concentrated under reduced pressure. The residue was purified by flash chromatography (eluting with PE/EA = 100 : 0 to 85 : 15) to give tert-butyl 3-(5-amino-6-methoxypyridin-2-yl)-2,5-dihydropyrrole-1-carboxylate 3 (1.50 g, 72% yield ) as a white solid.

LCMS (ESI) calcd for C15H21N3O3 [M + H] + m/z 292.16, found 292.19.

**Preparation of tert-butyl 3-(5-amino-6-methoxypyridin-2-yl) pyrrolidine-1-carboxylate (4)**

To a solution of tert-butyl 3-(5-amino-6-methoxypyridin-2-yl)-2,5-dihydropyrrole-1-carboxylate 3 (1.50 g, 5.10 mmol) in MeOH (20 mL) was added Pd/C (0.54 g, 5.10 mmol) at room temperature. The reaction mixture was stirred under hydrogen at 50 °C for 2 h. After cooling to room temperature, the reaction mixture was filtered, the solution was concentrated under reduced pressure to give product of tert-butyl 3-(5-amino-6-methoxypyridin-2-yl)pyrrolidine-1-carboxylate **4** (1.20 g, 76% yield ) as a white solid.

LCMS (ESI) calcd for C15H23N3O3 [M + H] + m/z 294.17, found 294.18.

**Preparation of tert-butyl 3-(5-bromo-6-methoxypyridin-2-yl)pyrrolidine-1-carboxylate (5)**

To a solution of tert-butyl 3-(5-amino-6-methoxypyridin-2-yl)pyrrolidine-1-carboxylate **4** (1.40 g, 4.80 mmol) in ACN (20 mL) was added tert-Butyl nitrite (1.14 g, 11.04 mmol). Then CuBr (1.38 g, 9.60 mmol) was added at room temperature. The reaction mixture was stirred at 50 °C for 2 h. After cooling to room temperature, the reaction mixture was concentrated under reduced pressure. The residue was purified by flash chromatography (eluting with PE/EA = 100 : 0 to 85 : 15) to give tert-butyl 3-(5-bromo-6-methoxypyridin-2-yl)pyrrolidine-1-carboxylate 5 (0.65 g, 35% yield ) as a white solid.

LCMS (ESI) calcd for C15H21BrN2O3 [M + H] + m/z 357.07, found 357.11.

**Preparation of tert-butyl 3-(5-ethyl-6-methoxypyridin-2-yl)pyrrolidine-1-carboxylate (6)**

To a solution of tert-butyl 3-(5-bromo-6-methoxypyridin-2-yl)pyrrolidine-1-carboxylate 5 (300 mg, 0.84 mmol) in 1,4-dioxane (15 mL) was added $Pd(dppf)Cl_2$ (61 mg, 0.08 mmol). Then $Et_2Zn$ (5 mL, 0.14mml) was added at room temperature. The reaction mixture under nitrogen was stirred at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-ethyl-6-methoxypyridin-2-yl)pyrrolidine-1-carboxylate 6 (120 mg, 44% yield ) as a white solid.

LCMS (ESI) calcd for C17H26N2O3 [M + H] + m/z 307.19, found 307.25.

**Preparation of 3-ethyl-6-(pyrrolidin-3-yl)-1H-pyridin-2-one (7)**

Tert-butyl 3-(5-ethyl-6-methoxypyridin-2-yl)pyrrolidine-1-carboxylate **6** (120 mg, 0.39 mmol) was added to HBr in water (48%, 15 mL). The reaction mixture was stirred at 100 °C for 6 h. After cooling to room temperature, the reaction mixture was concentrated under reduced pressure to give 3-ethyl-6-(pyrrolidin-3-yl)-1H-pyridin-2-one 7 (70 mg, 88% yield) as a white solid.

LCMS (ESI) calcd for Cl1H16N2O [M + H] + m/z 193.13, found 193.14.

**Preparation of 5-(4-(3-(5-ethyl-6-oxo-1,6-dihydropyridin-2-yl)pyrrolidin-1-yl)piperidin-1-yl)-6-fluoro-N-methylpicolinamide (151rac)**

To a solution of 6-fluoro-N-methyl-5-(4-oxopiperidin-1-yl)picolinamide **INT-2** (100 mg, 0.40 mmol) in MeOH (40 mL) was added 3-ethyl-6-(pyrrolidin-3-yl)-1H-pyridin-2-one 7 (115 mg, 0.60 mmol), then two drops of acetic acid and $NaBH_3CN$ (38 mg, 0.61 mmol) were added at room temperature. The reaction mixture stirred at 50 °C for 12 h. After cooling to room temperature, the reaction mixture was concentrated under reduced pressure. The residue was purified by flash chromatography (eluting with DCM/MeOH = 100 : 0 to 90 : 10) to afford 5-(4-(3-(5-ethyl-6-oxo-1,6-dihydropyridin-2-yl) pyrrolidin-1-yl)piperidin-1-yl)-6-fluoro-N-methylpicolinamide **151rac** (40 mg, 95% purity, 22.34% yield ) as a white solid.

LCMS (ESI) calcd for $C_{23}H_{30}FN_5O_2$ [M + H] + m/z 428.24, found 428.10.

**Chiral resolution of 5-(4-(3-(5-ethyl-6-oxo-1,6-dihydropyridin-2-yl)pyrrolidin-1-yl)piperidin-1-yl)-6-fluoro-N-methylpicolinamide (Compound 151a and Compound 151b)**

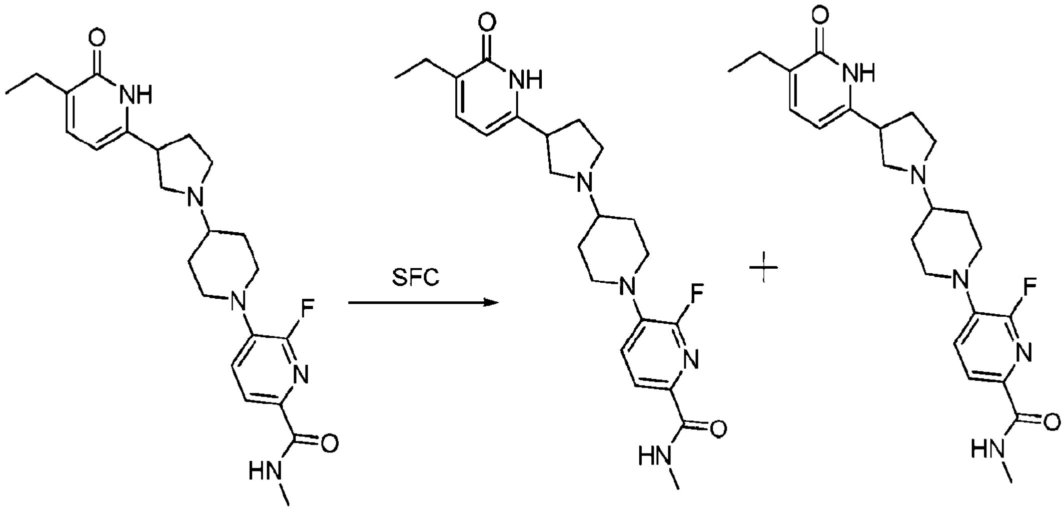


151rac 151a 151b

Compound **151** was separated by SFC (Column: Daicel Chiralpak AD-H SFC 250 mm × 20 mm I.D., 5 μmm; Mobile phase: $CO_2$/MeOH[0.1%($NH_3$)] = 80/20) and concentrated under reduced pressure to afford the first fraction as **Compound 151a** (15.8 mg, 92% purity, ee%: 100, white solid) and the second fraction as **Compound 151b** (15.9 mg, 95% purity, ee%: 100, white solid)

**Compound 151a**

$^1$H NMR (400 MHz, DMSO-$d_6$, ppm) $\delta$: 11.28 (s, 1 H), 8.44-8.37 (m, 1 H), 7.83 (dd, $J$ = 8.0, 1.2 Hz, 1 H), 7.61-7.52 (m, 1 H), 7.17 (d, $J$ = 7.2 Hz, 1 H), 6.03 (d, $J$ = 6.4 Hz, 1 H), 3.56-3.43 (m, 2 H), 3.17-3.09 (m, 1 H), 2.90-2.74 (m, 7 H), 2.68-2.60 (m, 2 H), 2.35-2.17 (m, 4 H), 1.96 (d, $J$ = 12.0 Hz, 2 H), 1.79-1.70 (m, 1 H), 1.63-1.52 (m, 2 H), 1.06 (t, $J$ = 7.2 Hz, 3 H).

LCMS (ESI) calcd for $C_{23}H_{30}FN_5O_2$ [M + H]$^+$ m/z 428.24, found 428.10.

**Compound 151b**

$^1$H NMR (400 MHz, DMSO-$d_6$, ppm) $\delta$: 11.28 (s, 1 H), 8.45-8.35 (m, 1 H), 7.83 (dd, $J$ = 8.0, 1.2 Hz, 1 H), 7.64-7.51 (m, 1 H), 7.17 (d, $J$ = 6.8 Hz, 1 H), 6.03 (d, $J$ = 6.8 Hz, 1 H), 3.58-3.41 (m, 2 H), 3.17-3.09 (m, 1 H), 2.93-2.74 (m, 7 H), 2.68-2.60 (m, 2 H), 2.35-2.16 (m, 4 H), 2.01-1.92 (m, 2 H), 1.79-1.70 (m, 1 H), 1.64-1.52 (m, 2 H), 1.06 (t, $J$ = 7.2 Hz, 3 H).

LCMS (ESI) calcd for $C_{23}H_{30}FN_5O_2$ [M + H]$^+$ m/z 428.24, found 428.20.

Synthesis of INT-2

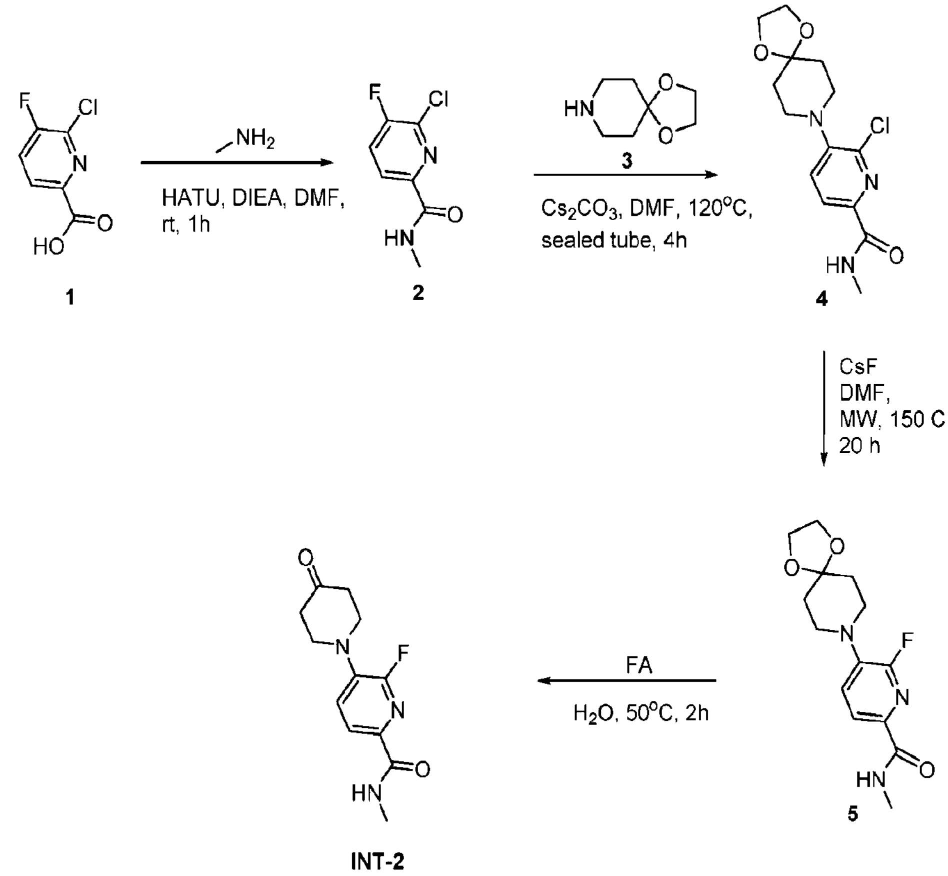


### *Preparation of 6-chloro-5-fluoro-N-methylpicolinamide (2)*

To a solution of 6-chloro-5-fluoropicolinic acid 1 (2.00 g, 11.40 mmol) in DMF (50 mL) was added methanamine (420 mg, 13.68 mmol), DIEA (4.42 g, 34.20 mmol), HATU (6.50 g, 17.10 mmol) at room temperature successively. The mixture was kept stirring at room temperature for 1 h. The resulting mixture was diluted with water and extracted with EtOAc (200 mL x 3). The combined organic layer was dried over $Na_2SO_4$ and concentrated under reduced pressure. The residue was purified by flash chromatography (eluting with PE/EtOAc = 100 : 0 to 70 : 30) to afford 6-chloro-5-fluoro-N-methylpicolinamide 2 (2.00 g, 88 % yield ) as a white solid. LCMS (ESI) calcd for $C_7H_6ClFN_2O$ [M + H] $^+$ m/z 189.02, found 188.90.

### *Preparation of 6-chloro-N-methyl-5-(1,4-dioxa-8-azaspiro[4.5]decan-8-yl)picolinamide (4)*

To a solution 6-chloro-5-fluoro-N-methylpicolinamide 2 (2.00 g, 10.60 mmol) in DMF (20 mL) was added $Cs_2CO_3$ (6.91 g, 21.20 mmol) and 1,4-dioxa-8-azaspiro[4.5]decane 3 (3.04 g, 21.20 mmol). The mixture was stirred for 4 h under 120 °C using a sealed tube. The reaction mixture was concentrated under reduced pressure. The residue was purified by flash chromatography (eluting with PE/EtOAC = 100 : 0 to 50 : 50) to afford 6-chloro-N-methyl-5-(1,4-dioxa-8-azaspiro[4.5]decan-8-yl)picolinamide **4** (1.60 g, 42 % yield ) as a white solid. LCMS (ESI) calcd for $C_{14}H_{18}ClN_3O_3$ [M + H] $^+$ m/z 312.10, found 311.95.

### *Preparation of 6-fluoro-N-methyl-5-(1,4-dioxa-8-azaspiro[4.5]decan-8-yl)picolinamide (5)*

To a solution 6-chloro-N-methyl-5-(1,4-dioxa-8-azaspiro[4.5]decan-8-yl)picolinamide 4 (300 mg, 0.96 mmol) in DMF (20 mL) was added CsF (293 mg, 1.93 mmol). The mixture was stirred for 20 h under 150 °C using microwave. The reaction mixture was concentrated under reduced pressure. The residue was purified by flash chromatography (eluting with PE/EtOAC = 100 : 0 to 30: 70) to 6-fluoro-N-methyl-5-(1,4-dioxa-8-azaspiro[4.5]decan-8-yl)picolinamide 5 (200 mg, 69% yield ) as a yellow solid.

LCMS (ESI) calcd for $C_{14}H_{18}FN_3O_3$ [M + H] $^+$ m/z 296.13, found 295.95.

***Preparation of 6-fluoro-N-methyl-5-(4-oxopiperidin-1-yl)picolinamide (INT-2)***

To the solution of 6-fluoro-N-methyl-5-(1,4-dioxa-8-azaspiro[4.5]decan-8-yl)picolinamide **5** (200 mg, 0.68 mmol) in $H_2O$ (3 mL) was added formic acid (2 mL), stirred at 50 °C for 1 h, The aqueous solution was adjusted to pH 7-8 with aqueous $NaHCO_3$ solution. The mixture was diluted with water (50 mL) and extracted with EtOAc (100 mL × 3). The combined organic layer was washed with brine (100 mL × 2), dried over $Na_2SO_4$, concentrated to give crude product of 6-fluoro-N-methyl-5-(4-oxopiperidin-1-yl)picolinamide **INT-2** (100 mg, 47% yield) as a yellow solid .

LCMS (ESI) calcd for $C_{12}H_{14}FN_3O_2$ [M + H] $^+$ m/z 252.11, found 251.90.

***<u>Example 14 - Synthesis of Compounds 154rac, 154a and 154b</u>***

**Synthesis of Compounds 154rac, 154a and 154b**

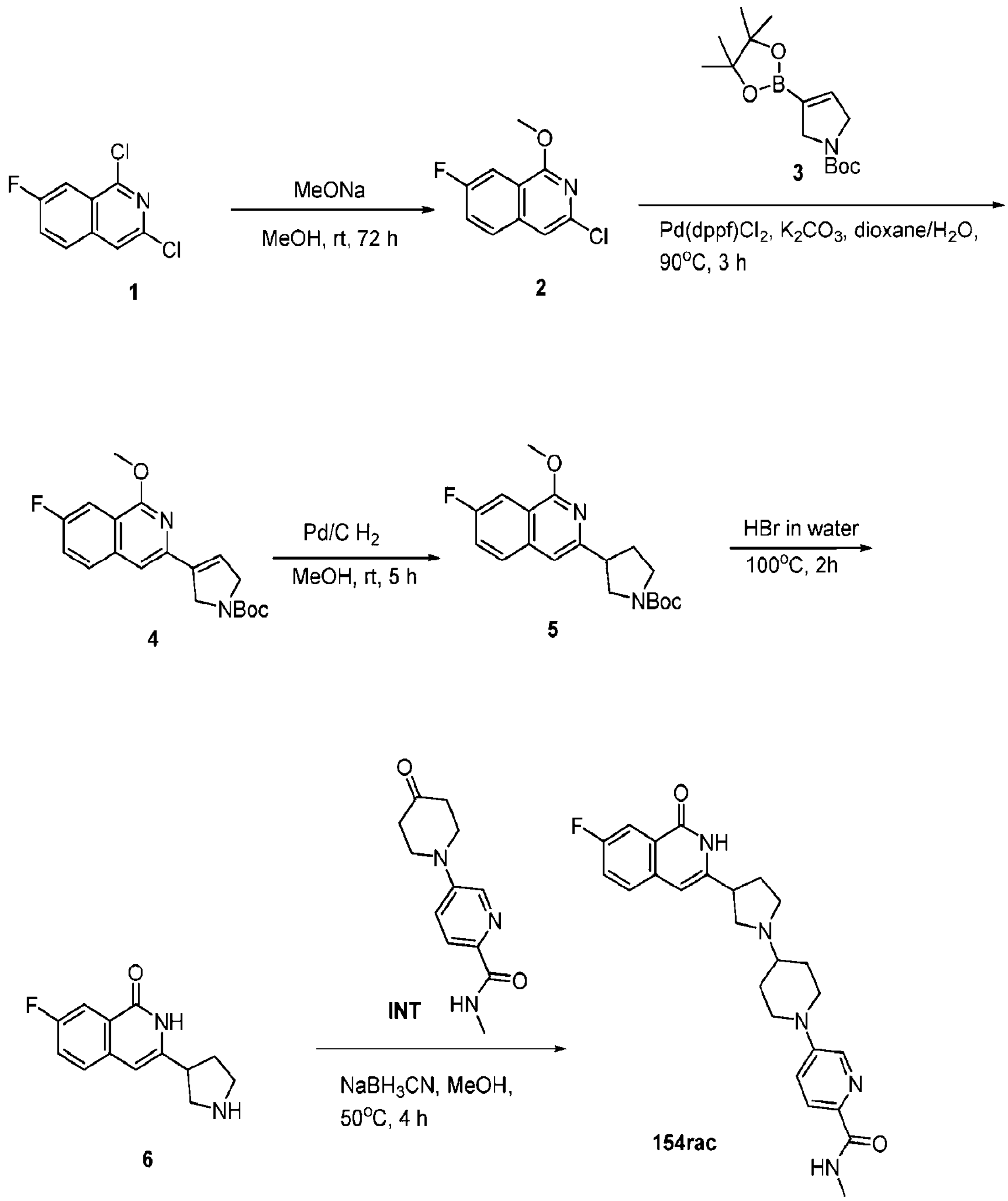


1 2 3 4 5 6 154rac

***Preparation of 3-chloro-7-fluoro-1-methoxyisoquinoline (2)***

To a solution of 1,3-dichloro-7-fluoroisoquinoline 1 (500 mg, 2.32 mmol) in MeOH (50 mL), was added MeONa (150 mg, 2.78 mmol) at room temperature. The mixture was stirred for 72 h at room temperature. The reaction mixture was concentrated under reduced pressure. The residue was purified by flash chromatography (eluting with PE/EtOAC = 100 : 0 to 98 : 2) to give 3-chloro-7-fluoro-1-methoxyisoquinoline 2 (160 mg, 33% yield ) as a white solid.
LCMS (ESI) calcd for C10H7ClFNO [M + H] $^+$ m/z 212.02, found 211.90.

***Preparation of tert-butyl 3-(7-fluoro-1-methoxyisoquinolin-3-yl)-2,5-dihydro-1H-pyrrole-1-carboxylate (4)***

To a solution of 3-chloro-7-fluoro-1-methoxyisoquinoline **2** (160 mg, 0.76 mmol) in dioxane:$H_2O$=5:1 (20 mL) was added tert-butyl 3-(4,4,5,5-tetramethyl-1,3,2-dioxaborolan-2-yl)-2,5-dihydro-1H-pyrrole-1-carboxylate 3 (270 mg, 0.92 mmol), Pd(dppf)$Cl_2$ (50 mg, 0.07 mmol) and $K_2CO_3$ (250 mg, 1.81 mmol) at room temperature. The reaction mixture was stirred under $N_2$ at 90 °C for 3 h. After cooling to room temperature, the reaction mixture was concentrated under reduced pressure. The residue was purified by flash chromatography (eluting with PE/EtOAc = 100 : 0 to 95 : 5) to afford tert-butyl 3-(7-fluoro-1-methoxyisoquinolin-3-yl)-2,5-dihydro-1H-pyrrole-1-carboxylate **4** (160 mg, 61% yield ) as a white solid.
LCMS (ESI) calcd for C19H21FN2O3 [M + H] $^+$ m/z 345.15, found 345.11.

***Preparation of tert-butyl 3-(7-fluoro-1-methoxyisoquinolin-3-yl)pyrrolidine-1-carboxylate (5)***

To a solution of tert-butyl 3-(7-fluoro-1-methoxyisoquinolin-3-yl)-2,5-dihydro-1H-pyrrole-1-carboxylate 4 (160 g, 0.46 mmol) in MeOH (20 mL) was added Pd/C (35 mg, 0.29 mmol) under $H_2$. The reaction mixture was stirred at room temperature for 5 h. The mixture was filtered through a Celite pad and the filtrate was concentrated to give tert-butyl 3-(7-fluoro-1-methoxyisoquinolin-3-yl)pyrrolidine-1-carboxylate **5** (140 mg, 88 % yield ) as a white solid. LCMS (ESI) calcd for C19H23FN2O3 [M + H] $^+$ m/z 347.17, found 347.18.

***Preparation of 7-fluoro-3-(pyrrolidin-3-yl)isoquinolin-1(2H)-one (6)***

Tert-butyl 3-(7-fluoro-1-methoxyisoquinolin-3-yl)pyrrolidine-1-carboxylate **5** (140 mg, 0.40 mmol) was added to HBr in water (48 %, 3 mL). The mixture was stirred at 100 °C for 2 h. The reaction mixture was concentrated under reduced pressure to give 7-fluoro-3-(pyrrolidin-3-yl)isoquinolin-1(2H)-one **6** (90 mg, 97% yield) as a white solid.
LCMS (ESI) calcd for C13H13FN2O [M + H] $^+$ m/z 233.10, found 232.95.

***Preparation of 5-(4-(3-(7-fluoro-1-oxo-1,2-dihydroisoquinolin-3-yl)pyrrolidin-1-yl)piperidin-1-yl)-N-methylpicolinamide (Compound 154rac)***

To a solution of 7-fluoro-3-(pyrrolidin-3-yl)isoquinolin-1(2H)-one **6** (90 mg, 0.39 mmol) in MeOH (15 mL) was added N-methyl-5-(4-oxopiperidin-1-yl)picolinamide **(INT,** 100 mg, 0.43 mmol), then two drops of acetic acid and $NaBH_3CN$ (25 mg, 0.40 mmol) were added at room temperature. The reaction mixture stirred at 50 °C for 4 h. After cooling to room temperature, the residue was purified by prep-HPLC (columns: Gemini 5 um C18 150 $\times$ 21.2 mm, mobile phase: ACN - $H_2O$ (0.1% FA), gradient: 10 - 25) to give 5-(4-(3-(7-fluoro-1-oxo-1,2-dihydroisoquinolin-3-yl)pyrrolidin-1-yl) piperidin-1-yl)-N-methylpicolinamide **154rac** (35 mg, 99% purity, 20% yield) as a white solid.

**Compound 154rac**

1H NMR (400 MHz, DMSO) $\delta$ 11.35 (s, 1 H), 8.39 (q, *J* = 4.8 Hz, 1 H), 8.30 (d, *J* = 2.8 Hz, 1 H), 7.87-7.76 (m, 2 H), 7.70 (dd, *J* = 8.8, 5.2 Hz, 1 H), 7.59 (td, *J* = 8.8, 2.8 Hz, 1 H), 7.43 (dd, *J* = 8.8, 2.8 Hz, 1 H), 6.57 (s, 1 H), 4.02-3.85 (m, 2 H), 3.31-2.71 (m, 11 H), 2.40-2.25 (m, 1 H), 2.13-1.88 (m, 3 H), 1.68-1.52 (m, 2 H).
LCMS (ESI) calcd for C25H28FN5O2 [M + H] $^+$ m/z 450.22, found 450.23.

***Preparation of 5-(4-(3-(7-fluoro-1-oxo-1,2-dihydroisoquinolin-3-yl)pyrrolidin-1-yl)piperidin-1-yl)-N-methylpicolinamide (154a and 154b)***

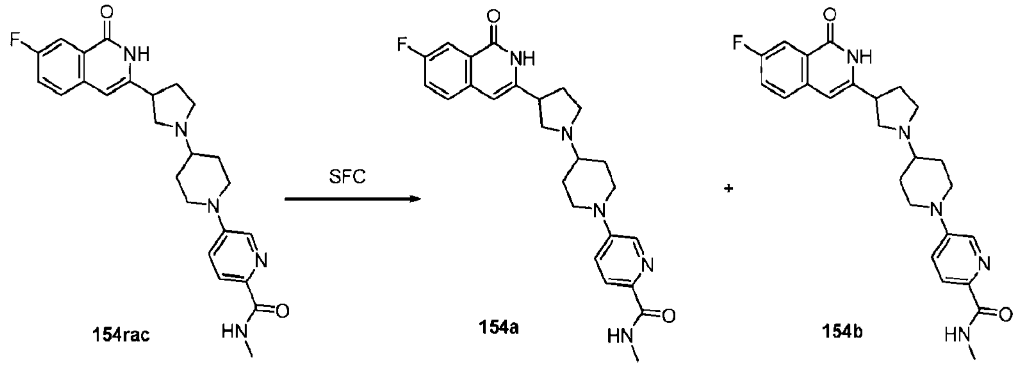


154rac 154a 154b

5-(4-(3-(7-fluoro-1-oxo-1,2-dihydroisoquinolin-3-yl)pyrrolidin-1-yl)piperidin-1-yl)-N-methylpicolinamide **154rac** was separated by SFC (Column: DAICEL OJ-H 4.6mmI.D.*250mmL 5μm; Mobile phase: CO2/MeOH[0.1%NH3(7M Solution in MeOH)]=75/25) and concentrated under reduced pressure to afford the first fraction as **154a** (10 mg, 99% purity, ec%: 100, white solid) and the second fraction as **154b** (10 mg, 97% purity, ee%: 100, white solid)

**Compound 154a**

1H NMR (400 MHz, DMSO) δ 11.25 (s, 1 H), 8.39 (d, *J* = 4.8 Hz, 1 H), 8.28 (d, *J* = 2.8 Hz, 1 H), 7.82 (d, *J* = 8.4 Hz, 1 H), 7.77 (dd, *J* = 9.2, 2.8 Hz, 1 H), 7.68 (dd, *J* = 8.8, 5.6 Hz, 1 H), 7.56 (td, *J* = 8.8, 2.8 Hz, 1 H), 7.40 (dd, *J* = 8.8, 2.8 Hz, 1 H), 6.51 (s, 1 H), 3.87-3.77 (m, 2 H), 3.25-3.17 (m, 1 H), 3.03-2.91 (m, 3 H), 2.85-2.75 (m, 4 H), 2.74-2.66 (m, 2 H), 2.35-2.32 (m, 1 H), 2.28-2.17 (m, 1 H), 2.01-1.90 (m, 2 H), 1.88-1.78 (m, 1 H), 1.60-1.47 (m, 2 H). LCMS (ESI) calcd for C25H28FN5O2 [M + H] $^+$ m/z 450.22, found 450.20.

**Compound 154b**

1H NMR (400 MHz, DMSO) δ 11.25 (s, 1 H), 8.39 (d, *J* = 4.8 Hz, 1 H), 8.28 (d, *J* = 2.8 Hz, 1 H), 7.82 (d, *J* = 8.8 Hz, 1 H), 7.77 (dd, *J* = 9.2, 2.8 Hz, 1 H), 7.68 (dd, *J* = 8.8, 5.2 Hz, 1 H), 7.56 (td, *J* = 8.8, 2.8 Hz, 1 H), 7.40 (dd, *J* = 8.8, 2.8 Hz, 1 H), 6.51 (s, 1 H), 3.88-3.78 (m, 2 H), 3.26-3.16 (m, 1 H), 3.03-2.91 (m, 3 H), 2.83-2.78 (m, 4 H), 2.74-2.66 (m, 2 H), 2.35-2.31 (m, 1 H), 2.28-2.17 (m, 1 H), 2.00-1.92 (m, 2 H), 1.89-1.79 (m, 1 H), 1.61-1.47 (m, 2 H).
LCMS (ESI) calcd for C25H28FN5O2 [M + H] $^+$ m/z 450.22, found 450.20.

***Example 15 - Synthesis of Compounds 162rac, 162a and 162b***

**Synthesis of Compounds 162rac, 162a and 162b**

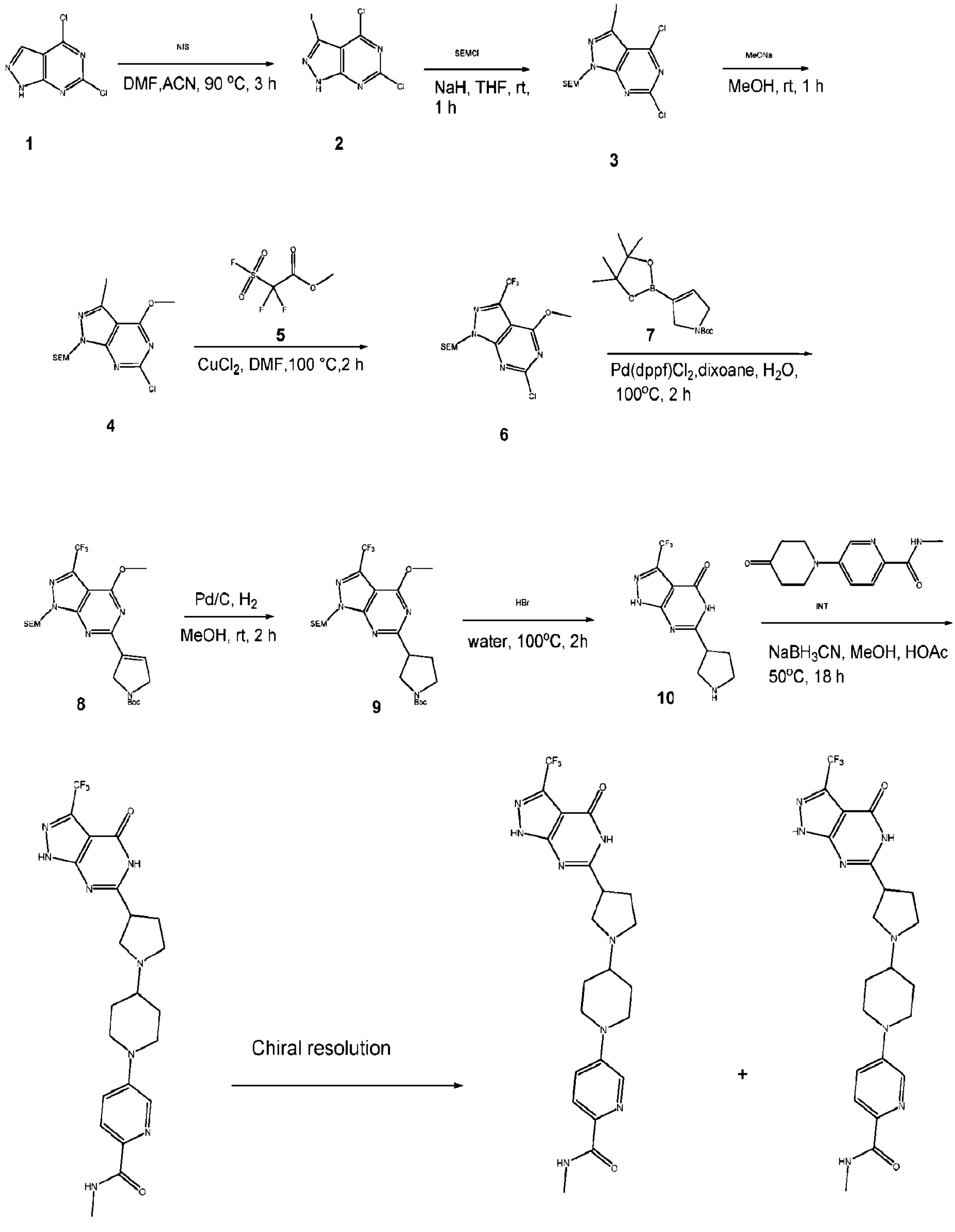


162rac 162a 162b

***Preparation of 4,6-dichloro-3-iodo-1H-pyrazolo[3,4-d]pyrimidine (2)***

To a solution of 4,6-dichloro-1H-pyrazolo[3,4-d]pyrimidine 1 (5 g, 26.50 mmol) in ACN/DMF = 1:1 (100 mL) was added NIS (8.9 g, 39.75 mmol). The resulting mixture was stirred at 90 °C for 3 h. The residue was purified by flash chromatography (eluting with PE/EtOAc = 100: 0 to 72: 28) to give 4,6-dichloro-3-iodo-1H-pyrazolo[3,4-d]pyrimidine 2 (2.9 g, 35% yield ) as white solid.

LCMS (ESI) calcd for C5HCl2IN4 [M + H] $^{+}$ m/z 314.86, found 314.85.

***Preparation of 4,6-dichloro-3-iodo-1-((2-(trimethylsilyl)ethoxy)methyl)-1H-pyrazolo[3,4-d]pyrimidine (3)***

To a solution of 4,6-dichloro-3-iodo-1H-pyrazolo[3,4-d]pyrimidinee 2 (2.9 g, 9.24 mmol) in THF (40 mL) was added NaH (290 mg, 11.96 mmol) at 0 °C, stirred 10 min, then added SEMCl (2.3 g, 13.80 mmol). The resulting mixture was stirred at room temperature for 1 h. The reaction mixture was concentrated under reduced pressure to give 4,6-dichloro-3-iodo-1-((2-(trimethylsilyl)ethoxy)methyl)-1H-pyrazolo[3,4-d]pyrimidine **3** (3.6 g, 88% yield ) as white solid.
LCMS (ESI) calcd for C11H15Cl2IN4OSi [M + H] $^+$ m/z 444.94, found 444.85.

***Preparation of 6-chloro-3-iodo-4-methoxy-1-((2-(trimethylsilyl)ethoxy)methyl)-1H-pyrazolo[3,4-d]pyrimidine (4)***

To a solution of 4,6-dichloro-3-iodo-1-((2-(trimethylsilyl)ethoxy)methyl)-1H-pyrazolo[3,4-d]pyrimidine **3** (1.5 g, 3.39 mmol) in MeOH (45 mL) was added MeONa (183 mg, 3.39 mmol). The mixture was stirred at room temperature for 1 h. The residue was purified by flash chromatography (eluting with PE/EtOAc = 100: 0 to 95: 5) to give 6-chloro-3-iodo-4-methoxy-1-((2-(trimethylsilyl)ethoxy)methyl)-1H-pyrazolo[3,4-d]pyrimidine 4 (282 mg, 19% yield) as a white solid.
LCMS (ESI) calcd for C12H18ClIN4O2Si [M + H] $^+$ m/z 440.99 found 441.01.

***Preparation of 6-chloro-4-methoxy-3-(trifluoromethyl)-1-((2-(trimethylsilyl)ethoxy)methyl)-1H-pyrazolo[3,4-d] pyrimidine (6)***

To a solution of 6-chloro-3-iodo-4-methoxy-1-((2-(trimethylsilyl)ethoxy)methyl)-1H-pyrazolo[3,4-d]pyrimidine 4 (282 mg, 0.64 mmol) and methyl 2,2-difluoro-2-(fluorosulfonyl)acetate **5** (491 mg, 2.56 mmol) in DMF (15 mL) was added $CuCl_2$ (131 mg, 0.98 mmol) under $N_2$. The reaction mixture was stirred at 100 °C for 2 h. The residue was purified by flash chromatography (eluting with PE/EtOAc = 100: 0 to 96: 4) to 6-chloro-4-methoxy-3-(trifluoromethyl)-1-((2-(trimethylsilyl) ethoxy)methyl)-1H-pyrazolo[3,4-d]pyrimidine **6** (220 mg, 90% yield ) as a white solid.
LCMS (ESI) calcd for C13H18ClF3N4O2Si [M + H] + m/z 383.08, found 383.08.

***Preparation of tert-butyl 3-(4-methoxy-3-(trifluoromethyl)-1-((2-(trimethylsilyl)ethoxy)methyl)-1H-pyrazolo[3,4-d]pyrimidin-6-yl)-2,5-dihydro-1H-pyrrole-****1-carboxylate (8)*

To a solution of tert-butyl 3-(4-methoxy-5-(trifluoromethyl)-7-((2-(trimethylsilyl)ethoxy)methyl)-7H-pyrrolo[2,3-d] pyrimidin-2-yl)-2,5-dihydro-1H-pyrrole-1-carboxylate **6** (200 mg, 0.52 mmol), tert-butyl 3-(4,4,5,5-tetramethyl-1,3,2-dioxaborolan-2-yl)-2,5-dihydro-1H-pyrrole-1-carboxylate **7** (154 mg, 0.52 mmol) and Pd(dppf)$Cl_2$ (23 mg, 0.031 mmol) in 1,4-dioxane/$H_2O$=10:1 (11 mL) was added $Na_2CO_3$ (165 mg, 1.56 mmol) under $N_2$. The reaction mixture was stirred at 100 °C for 2 h. The residue was purified by flash chromatography (eluting with PE/EtOAc = 100: 0 to 93: 7) to give tert-butyl 3-(4-methoxy-3-(trifluoromethyl)-1-((2-(trimethylsilyl)ethoxy)methyl)-1H-pyrazolo[3,4-d]pyrimidin-6-yl)-2,5-dihydro-1H-pyrrole-1-carboxylate **8** (280 mg, 94% yield ) as an orange oil.
LCMS (ESI) calcd for C27H32F3N5O4Si [M + H] + m/z 516.22, found 516.23.

***Preparation of tert-butyl 3-(4-methoxy-3-(trifluoromethyl)-1-((2-(trimethylsilyl)ethoxy)methyl)-1H-pyrazolo[3,4-d]pyrimidin-6-yl)pyrrolidine-1-carboxylate (9)***

To a solution of tert-butyl 3-(4-methoxy-5-(trifluoromethyl)-7-((2-(trimethylsilyl)ethoxy)methyl)-7H-pyrrolo[2,3-d] pyrimidin-2-yl)pyrrolidine-1-carboxylate **8** (280 mg, 0.54 mmol) in MeOH (15 mL) was added Pd/C (114 mg, 1.08 mmol) under $H_2$. The reaction mixture was stirred at room temperature for 2 h. Then the mixture was filtered through celite and concentrated under vacuum to give tert-butyl 3-(4-methoxy-3-(trifluoromethyl)-1-((2-(trimethylsilyl)ethoxy)methyl)- 1H-pyrazolo[3,4-d]pyrimidin-6-yl)pyrrolidine-1-carboxylate **9** (158 mg, 56% yield ) as a white solid.
LCMS (ESI) calcd for C22H34F3N5O4Si [M + H] + m/z 518.23, found 518.25.

***Preparation of 6-(pyrrolidin-3-yl)-3-(trifluoromethyl)-1,5-dihydro-4H-pyrazolo[3,4-d]pyrimidin-4-one (10)***

Tert-butyl 3-(4-methoxy-3-(trifluoromethyl)-1-((2-(trimethylsilyl)ethoxy)methyl)-1H-pyrazolo[3,4-d]pyrimidin-6-yl)pyrrolidine-1-carboxylate **9** (158 mg, 0.31 mmol) was added to HBr water (5 mL). The reaction mixture was stirred at 100 °C for 2 h. Then the mixture was concentrated under vacuum to give 6-(pyrrolidin-3-yl)-3-(trifluoromethyl)-1,5-dihydro-4H-pyrazolo[3,4-d]pyrimidin-4-one **10** (80 mg, 96% yield ) as a white solid.
LCMS (ESI) calcd for C10H10F3N5O [M + H] + m/z 274.08, found 274.00.

***Preparation of N-methyl-5-(4-(3-(4-oxo-3-(trifluoromethyl)-4,5-dihydro-1H-pyrazolo[3,4-d]pyrimidin-6-yl)pyrrolidin-1-yl)piperidin-1-yl)picolinamide (162rac)***

To a solution of 6-(pyrrolidin-3-yl)-3-(trifluoromethyl)-1,5-dihydro-4H-pyrazolo[3,4-d]pyrimidin-4-one **10** (80 mg, 0.29 mmol), N-methyl-5-(4-oxopiperidin-1-yl)picolinamide **INT** (90 mg, 0.29 mmol) and sodium triacetoxyborohydride (92 mg, 0.44 mmol) in McOH (10 mL) was added two drops of HOAc and the solution was stirred for 0.5 h at 50 °C. Then was added NaBH3CN (22 mg, 0.35 mmol). The mixture was stirred for 18 h at 50 °C. The residue was purified by flash chromatography (eluting with DCM/MeOH = 100 : 0 to 90 : 10) to give product of N-methyl-5-(4-(3-(4-oxo-3-(trifluoromethyl)-4,5-dihydro-1H-pyrazolo[3,4-d]pyrimidin-6-yl)pyrrolidin-1-yl)piperidin-1-yl)picolinamide **162rac** (69 mg, 48% yield ) as white solid.

LCMS (ESI) calcd for C22H25F3N8O2 [M + H] + m/z 491.21, found 491.25.

***Chiral resolution of N-methyl-5-(4-(3-(4-oxo-3-(trifluoromethyl)-4,5-dihydro-1H-pyrazolo[3,4-d]pyrimidin-6-yl) pyrrolidin-1-yl)piperidin-1-yl)picolinamide (162a and 162b)***

Compound **162rac** was separated by SFC (Column: DAICEL OJ-H 4.6 mmI.D.*250 mmL, 5 μm; Mobile phase: $CO_2$/MeOH[0.1% $NH_3$ (7M Solution in MeOH)] = 70/30) and concentrated under reduced pressure to afford the first fraction as **162a** (39 mg, 93% purity, ee%: 100, white solid) and the second fraction as **162b** (31 mg, 99% purity, ee%: 100, white solid)

**Compound 162a**

1H NMR (400 MHz, DMSO) δ 14.37 (s, 1 H), 12.27 (s, 1 H), 8.39 (q, *J* = 4.4 Hz, 1 H), 8.27 (d, *J* = 2.8 Hz, 1 H), 7.82 (d, *J* = 8.8 Hz, 1 H), 7.40 (dd, *J* = 8.4, 2.8 Hz, 1 H), 3.83 (d, *J* = 12.8 Hz, 2 H), 3.38-3.35 (m, 1 H), 3.06 (t, *J* = 8.4 Hz, 1 H), 2.98-2.88 (m, 2 H), 2.83-2.73 (m, 5 H), 2.68-2.61 (m, 1 H), 2.39-2.29 (m, 1 H), 2.21-2.09 (m, 2 H), 1.93 (d, *J* = 11.6 Hz, 2 H), 1.55-1.43 (m, 2 H).

LCMS (ESI) calcd for C22H25F3N8O2 [M + H] + m/z 491.21, found 491.26.

**Compound 162b**

1H NMR (400 MHz, ) δ 14.41 (s, 1 H), 12.41 (s, 1 H), 8.39 (q, *J* = 4.8 Hz, 1 H), 8.28 (d, *J* = 2.0 Hz, 1 H), 7.82 (d, *J* = 8.8 Hz, 1 H), 7.41 (dd, *J* = 8.8, 2.0 Hz, 1 H), 3.86 (s, 1 H), 3.30-3.27 (m, 1 H), 3.16-3.03 (m, 1 H), 2.96-2.58 (m, 8 H), 2.34-2.10 (m, 3 H), 2.03-1.88 (m, 2 H), 1.63-1.44 (m, 2 H).

LCMS (ESI) calcd for C22H25F3N8O2 [M + H] + m/z 491.21, found 491.25.

***<u>Example 16 - Synthesis of Compounds 184rac, 184a and 184b</u>***

**Synthesis of Compounds 184rac, 184a and 184b**

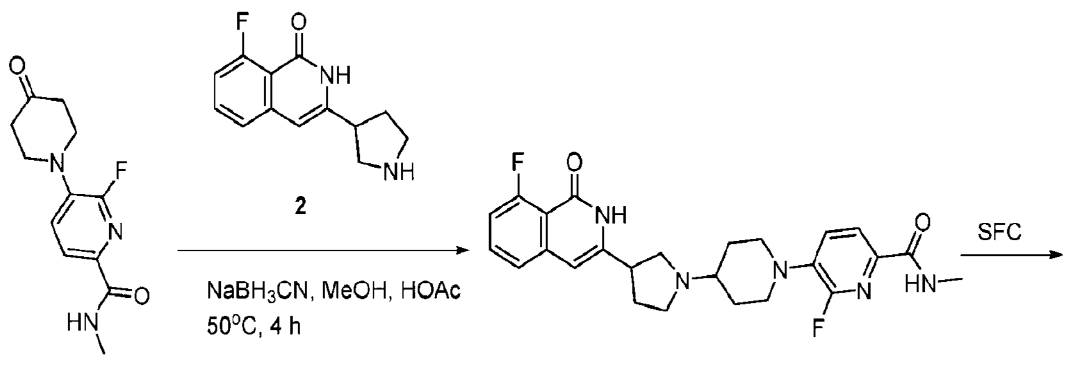


INT-1

184rac

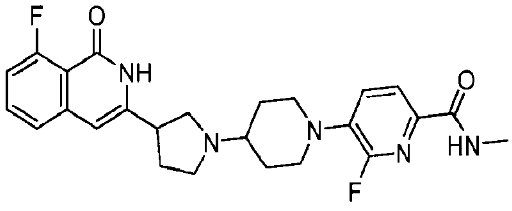
+
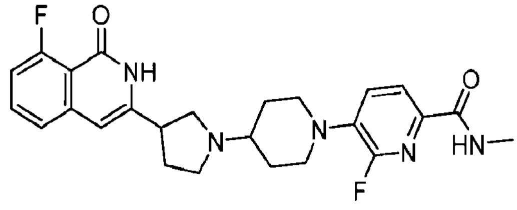

**184a** **184b**

***Preparation of 6-fluoro-5-{4-[3-(8-fluoro-1-oxo-2H-isoquinolin-3-yl)pyrrolidin-1-yl]piperidin-1-yl}-N-methylpyridine-2-carboxamide (184rac)***

To a solution of 6-fluoro-N-methyl-5-(4-oxopiperidin-1-yl)pyridine-2-carboxamide **INT-1** (92 mg, 0.40 mmol) in MeOH (10 mL) was added N-methyl-5-(4-oxopiperidin-1-yl)pyridine-2-carboxamide **2** (intermediate **6** in synthesis of compound **136**) (100 mg, 0.40 mmol). Then two drops of acetic acid and $NaBH_3CN$ (25 mg, 0.26 mmol) was added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 92 : 8) to give product of 6-fluoro-5-{4-[3-(8-fluoro-1-oxo-2H-isoquinolin-3-yl)pyrrolidin-1-yl]piperidin-1-yl}-N-methylpyridine-2-carboxamide **184rac** (60 mg, 27% yield ) as a colorless oil.

LCMS (ESI) calcd for C25H27F2N5O2 $[M + H]^+$ m/z 468.21, found 468.26.

***Preparation of 6-fluoro-5-{4-[3-(8-fluoro-1-oxo-2H-isoquinolin-3-yl)pyrrolidin-1-yl]piperidin-1-yl]-N-methylpyriditle-2-carboxamide (184a and 184b)***

6-chloro-5-{4-[3-(8-fluoro-1-oxo-2H-isoquinolin-3-yl)pyrrolidin-1-yl]piperidin-1-yl }-N-methylpyridine-2-carboxamide **184rac** was separated by SFC (Column: Daicel CHIRALPAK OJ-H 250 mm × 20 mm I.D., 5 μmm; Mobile phase: $CO_2$/MeOH[0.1%($NH_3$)] = 80/20) and concentrated under reduced pressure to afford the first fraction as **184a** (24.5 mg, 98% purity, cc%: 100, white solid) and the second fraction as **184b** (24.4 mg, 99% purity, ce%: 97, white solid)

**Compound 184a**

$^1$H NMR (400 MHz, DMSO) δ 11.10 (s, 1 H), 8.41 (q, *J* = 5.2 Hz, 1 H), 7.85 (d, *J* = 7.2 Hz, 1 H), 7.64-7.56 (m, 2 H), 7.39 (d, *J* = 7.6 Hz, 1 H), 7.10 (dd, *J* = 12.0, 8.0 Hz, 1 H), 6.47 (s, 1 H), 3.52-3.51 (m, 2 H), 3.22-3.16 (m, 1 H), 2.91-2.73 (m, 8 H), 2.67-2.63 (m, 1 H), 2.30-2.22 (m, 2 H), 2.00-1.97 (m, 2 H), 1.86-1.79 (m, 1 H), 1.68-1.53 (m, 2 H).

LCMS (ESI) calcd for C25H27F2N5O2 $[M + H]^+$ m/z 468.21, found 468.22.

**Compound 184b**

$^1$H NMR (400 MHz, DMSO) δ 11.10 (s, 1 H), 8.41 (q, *J* = 4.8 Hz, 1 H), 7.85 (dd, *J* = 8.0, 1.2 Hz, 1 H), 7.64-7.56 (m, 2 H), 7.39 (d, *J* = 7.6 Hz, 1 H), 7.11 (dd, *J* = 11.6, 8.0 Hz, 1 H), 6.46 (s, 1 H), 3.53-3.49 (m, 2 H), 3.21-2.87 (m, 1 H), 2.93-2.71 (m, 8 H), 2.68-2.63 (m, 1 H), 2.28-2.19 (m, 2 H), 2.00-1.97 (m, 2 H), 1.88-1.79 (m, 1 H), 1.64-1.54 (m, 2 H).

LCMS (ESI) calcd for C25H27F2N5O2 $[M + H]^+$ m/z 468.21, found 468.18.

***<u>Example 17 - Synthesis of compound 192</u>***

**Synthesis of compound 192**

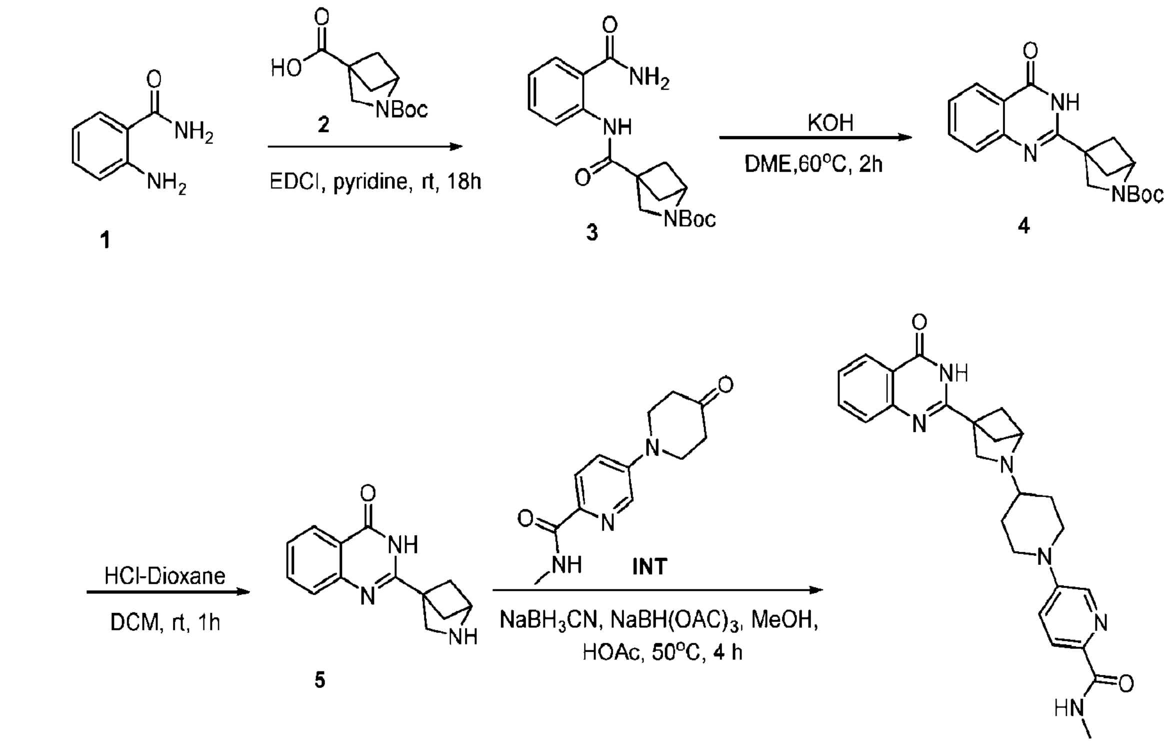


1 3 4 5

192

***Preparation of tert-butyl 4-((2-carbamoylphenyl)carbamoyl)-2-azabicyclo[2.1.1]hexane-2-carboxylate (3)***

To a solution of 2-aminobenzamide 1 (360 mg, 2.64 mmol) and 2-(tert-butoxycarbonyl)-2-azabicyclo[2.1.1] hexane-4-carboxylic acid **2** (500 mg, 2.20 mmol) in pyridine (30 mL) was added EDCI (422 mg, 2.20 mmol). Then the mixture was stirred for 18 h at room temperature. The reaction mixture was quenched with water, the aqueous layer was extracted with EtOAc (200 mL x 3). The combined organic layers was washed by 1M HCl solution and brine, dried over $Na_2SO_4$, concentrated under reduced pressure to give product of tert-butyl 4-((2-carbamoylphenyl)carbamoyl)-2-azabicyclo[2.1.1]hexane-2-carboxylate **3** (600 mg, 90% yield) as a white solid .

LCMS (ESI) calcd for $C_{18}H_{23}N_3O_4$ [M + Na] $^+$ m/z 368.17, found 368.05.

***Preparation of tert-butyl 4-(4-oxo-3,4-dihydroquinazolin-2-yl)-2-azabicyclo[2.1.1]hexane-2-carboxylate (4)***

To the solution of tert-butyl 4-((2-carbamoylphenyl)carbamoyl)-2-azabicyclo[2.1.1]hexane-2-carboxylate **3** (600 mg, 1.74 mmol) in DME (60 mL) was added KOH (195 mg, 3.47 mmol), heated to 60 °C stirred for 2 h, cooled to 25 °C, the reaction mixture was concentrated under reduced pressure. The residue was purified by flash chromatography (eluting with DCM/MeOH = 100 : 0 to 90 : 10) to give tert-butyl 4-(4-oxo-3,4-dihydroquinazolin-2-yl)-2-azabicyclo[2.1.1]hexane-2-carboxylate **4** (400 mg, 63% yield ) as a white solid..

LCMS (ESI) calcd for $C_{18}H_{21}N_3O_3$ [M + H] $^+$ m/z 328.16, found 328.05.

***Preparation of 2-(2-azabicyclo[2.1.1]hexan-4-yl)quinazolin-4(3H)-one (5)***

Tert-butyl 4-(4-oxo-3,4-dihydroquinazolin-2-yl)-2-azabicyclo[2.1.1]hexane-2-carboxylate **4** (400 mg, 0.56 mmol) was added to HCl in dioxane (4M, 10 mL). The mixture was stirred for 1 h at room temperature. The reaction mixture was concentrated under reduced pressure to give product of 2-(2-azabicyclo[2.1.1]hexan-4-yl)quinazolin-4(3H)-one **5** (320 mg, 93% yield ) as a white solid.

LCMS (ESI) calcd for $C_{13}H_{13}N_3O$ [M + H] $^+$ m/z 228.11, found 228.02.

***Preparation of N-methyl-5-(4-(4-(4-oxo-3,4-dihydroquinazolin-2-yl)-2-azabicyclo[2.1.1]hexan-2-yl)piperidin-1-yl)picolinamide (compound 192)***

To a solution of 2-(2-azabicyclo[2.1.1]hexan-4-yl)quinazolin-4(3H)-one **5** (80 mg, 0.35 mmol) in MeOH (40 mL)

was added N-methyl-5-(4-oxopiperidin-1-yl)picolinamide (**INT,** 98 mg, 0.42 mmol), then two drops of acetic acid and $NaBH(OAC)_3$ (149 mg, 0.70 mmol) were added at room temperature. The reaction mixture stirred at 50 °C for 1 h. Then was added $NaBH_3CN$ (11.06 mg, 0.176 mmol). The reaction mixture stirred at 50 °C for 4 h. After cooling to room temperature, the reaction mixture was concentrated under reduced pressure. The residue was purified by C18 column (mobile phase: ACN - $H_2O$ (0.1% FA), gradient: 10 - 95) to give N-methyl-5-(4-(4-(4-oxo-3,4-dihydroquinazolin-2-yl)-2-azabicyclo[2.1.1]hexan-2-yl)piperidin-1-yl)picolinamide **192** (25.6 mg, 99 % purity, 16% yield) as a white solid.

**Compound 192**

$^1$H NMR (400 MHz, DMSO-$d_6$, ppm) δ: 12.17 (s, 1 H), 8.43-8.36 (m, 1 H), 8.29 (d, $J$ = 2.8 Hz, 1 H), 8.17 (s, 1 H), 8.10 (dd, $J$ = 8.0, 1.2 Hz, 1 H), 7.86-7.76 (m, 2 H), 7.62 (d, $J$ = 7.8 Hz, 1 H), 7.52-7.47 (m, 1 H), 7.42 (dd, $J$ = 8.8, 2.9 Hz, 1 H), 3.93-3.86 (m, 2 H), 3.76 (s, 1 H), 3.12 (s, 2 H), 2.97-2.90 (m, 2 H), 2.78 (d, $J$ = 4.8 Hz, 3 H), 2.69-2.62 (m, 1 H), 2.19-2.14 (m, 2 H), 2.02-1.90 (m, 4 H), 1.54-1.44 (m, 2 H).

LCMS (ESI) calcd for $C_{25}H_{28}N_6O_2$ $[M + H]^+$ m/z 445.23, found 445.15.

***Example 18 - Synthesis of compound 194a and 194b***

**Synthesis of compounds 194a and 194b**

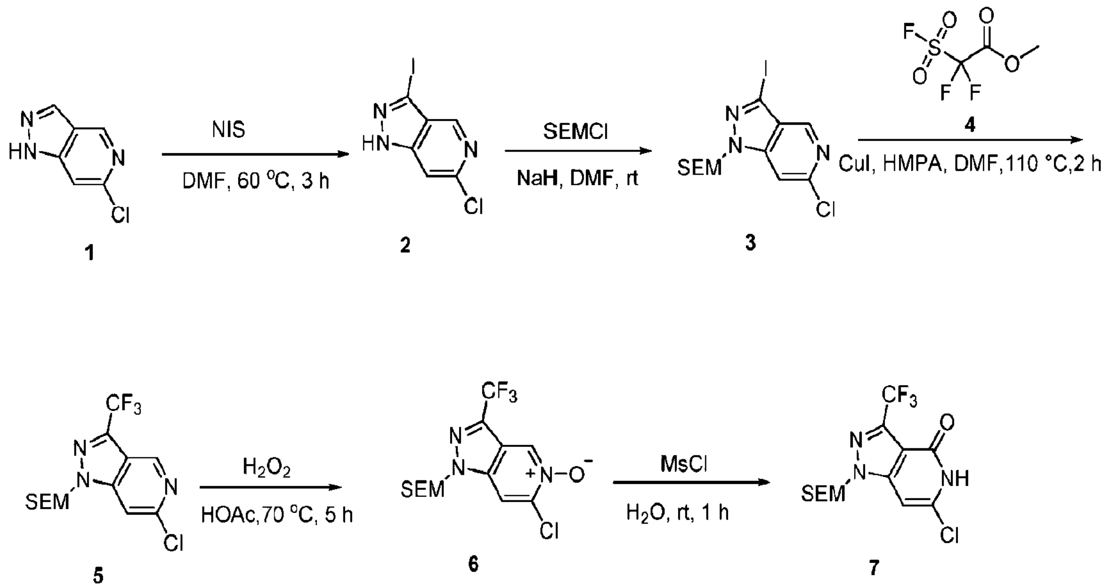


1 2 3 5 6 7

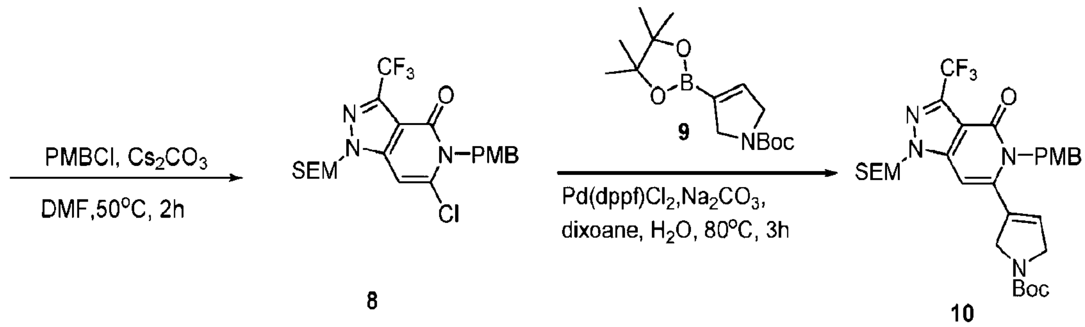


8

10

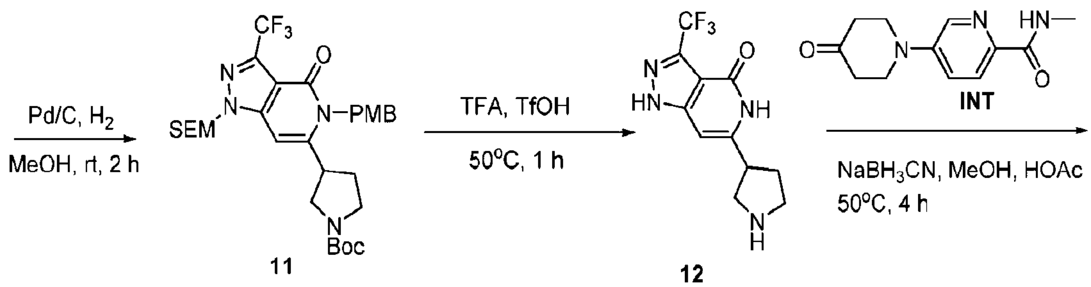


11

12

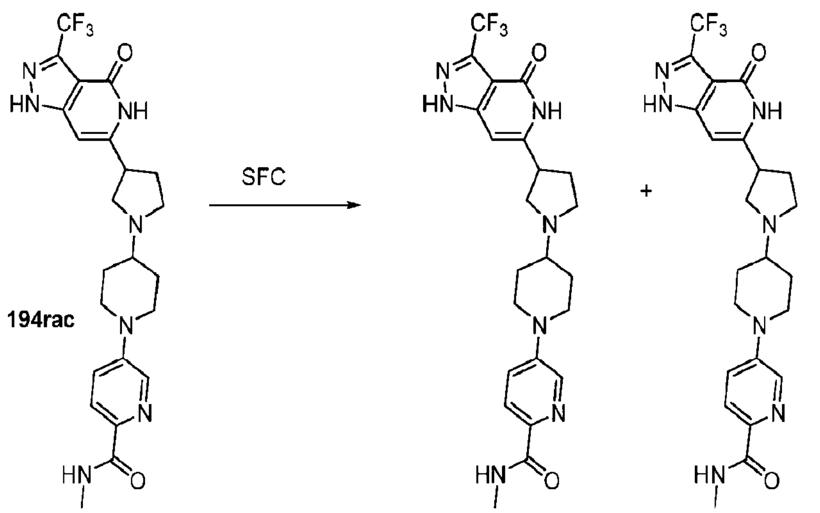


194rac

+

194a

194b

***Preparation of 6-chloro-3-iodo-1H-pyrazolo[4,3-c] pyridine** (2)*

To a solution of 6-chloro-1H-pyrazolo[4,3-c]pyridine **1** (10.00 g, 0.06 mol) in DMF (200 mL) was added NIS (8.44 g, 0.08 mol). The mixture was stirred for 3 h at 60 °C. The reaction mixture was quenched with water, the aqueous layer was extracted with EtOAc (50 mL x 3). The combined organic layers were concentrated under reduced pressure. The residue was purified by flash chromatography (eluting with PE/ EtOAc = 100 : 0 to 60 : 40) to give product of 6-chloro-3-iodo-1H-pyrazolo[4,3-c]pyridine 2 (10.5 g, 64% yield ) as a yellow soild.

LCMS (ESI) calcd for C6H3ClIN3 [M + H] $^+$ m/z 279.91, found 280.00.

***Preparation of 6-chloro-3-iodo-1-((2-(trimethylsilyl)ethoxy)methyl)-1H-pyrazolo[4,3-c]pyridine (3)***

A suspension of 6-chloro-3-iodo-1H-pyrazolo[4,3-c]pyridine **2** (10.50 g, 0.04 mol) and NaH (2.70 g, 0.11 mol) in DMF (200 mL) was stirred for 30 min at 0 °C, then SEMC1 (12.30 g, 0.08 mol) was added. The mixture was stirred for 3 h at room temperature. The reaction mixture was quenched with water, the aqueous layer was extracted with EtOAc (50 mL x 3). The combined organic layer was concentrated under reduced pressure. The residue was purified by flash chromatography (eluting with PE/ EtOAc = 100 : 0 to 70 : 30) to give product of 6-chloro-3-iodo-1-((2-(trimethylsilyl)ethoxy) methyl)-1H-pyrazolo[4,3-c]pyridine 3 (9.90 g, 61 % yield ) as a yellow solid.

LCMS (ESI) calcd for C12H17ClIN30Si [M + H] $^+$ m/z 409.99, found 410.05.

***Preparation of 6-chloro-3-(trifluoromethyl)-1-((2-(trimethylsilyl)ethoxy)methyl)-1H-pyrazolo[4,3-c]pyridine (5)***

To a solution of 6-chloro-3-iodo-1-((2-(trimethylsilyl)ethoxy)methyl)-1H-pyrazolo[4,3-c]pyridine 3 (9.90 g, 0.02 mol) and methyl 2,2-difluoro-2-(fluorosulfonyl)acetate **4** (23.04 g, 0.12 mol) in DMF (80 mL) were added CuI (9.15 g, 0.04 mmol) and HMPA (21.35g, 0.12 mmol) at room temperature. The mixture was stirred for 2 h at 110 °C. The reaction mixture was quenched with water, the aqueous layer was extracted with EtOAc (50 mL x 3). The combined organic layer was concentrated under reduced pressure. The residue was purified by flash chromatography (eluting with PE/ EtOAc = 100 : 0 to 80 : 20) to give product of 6-chloro-3-(trifluoromethyl)-1-((2-(trimethylsilyl)ethoxy)methyl)-1H-pyrazolo[4,3-c]pyridinc **5** (3.30 g, 38% yield ) as a yellow solid.

LCMS (ESI) calcd for for C13H17ClF3N3OSi [M + H] $^+$ m/z 352.08, found 352.20.

***Preparation of 6-chloro-3-(trifluoromethyl)-1-((2-(trimethylsilyl)ethoxy)methyl)-1H-pyrazolo[4,3-c]pyridine 5-oxide (6)***

To a solution of 6-chloro-3-(trifluoromethyl)-1-((2-(trimethylsilyl)ethoxy)methyl)-1H-pyrazolo[4,3-c]pyridine **5** (3.30 g, 0.01 mmol) in HOAc (20 mL) was added $H_2O_2$ (20 mL). The reaction mixture stirred at 70 °C for 5 h. After cooling to room temperature, the reaction mixture was quenched with water, the aqueous layer was extracted with EtOAc (30 mL x 3). The combined organic layer was concentrated under reduced pressure. The residue was purified by flash chromatography (eluting with PE/ EtOAc = 100 : 0 to 55 : 45) to give product of 6-chloro-3-(trifluoromethyl)-1-((2-(trimethylsilyl)ethoxy)methyl)-1H-pyrazolo[4,3-c]pyridine 5-oxide **6** (1.60 g, 48% yield ) as an yellow oil.

LCMS (ESI) calcd for C13H17ClF3N3O2Si [M + H] $^+$ m/z 368.07, found 367.97.

***Preparation of 6-chloro-3-(trifluoromethyl)-1-((2-(trimethylsilyl)ethoxy)methyl)-1,5-dihydro-4H-pyrazolo[4,3-c] pyridin-4-one (7)***

To a solution of 6-chloro-3-(trifluoromethyl)-1-((2-(trimethylsilyl)ethoxy)methyl)-1H-pyrazolo[4,3-c]pyridine 5-oxide **6** (800 mg, 2.16 mmol) in $H_2O$ (15 mL) and MsCl (1.00 g, 8.62 mmol). The reaction mixture was stirred at room temperature for 1 h. The reaction mixture was quenched with water, the aqueous layer was extracted with EtOAc (20 mL x 3). The combined organic layer was concentrated under reduced pressure. The residue was purified by flash chromatography (eluting with PEI EtOAc = 100 : 0 to 85 : 15) to give product of 6-chloro-3-(trifluoromethyl)-1-((2-(trimethylsilyl) ethoxy)methyl)-1,5-dihydro-4H-pyrazolo[4,3-c]pyridin-4-one **7** (400 mg, 48% yield ) as an yellow oil.

LCMS (ESI) calcd for Cl3H17ClF3N3O2Si [M + H] $^+$ m/z 368.07, found 368.01.

***Preparation of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one (8)***

To a solution of 6-chloro-3-(trifluoromethyl)-1-((2-(trimethylsilyl)ethoxy)methyl)-1,5-dihydro-4H-pyrazolo[4,3-c] pyridin-4-one **7** (400 mg, 1.07 mmol) and $Cs_2CO_3$ (1.25 g, 3.85 mmol) in DMF (15 mL) was added PMBCl (0.84 g, 5.35 mmol) at room temperature. The reaction mixture was stirred at 50 °C for 2 h. After cooling to room temperature, the combined organic layer was concentrated under reduced pressure. The residue was purified by flash chromatography (eluting with PE/ EtOAc = 100 : 0 to 85 : 15) to give product of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one **8** (200 mg, 32% yield ) as a yellow oil.

LCMS (ESI) calcd for C21H25ClF3N3O3Si [M + H] $^+$ m/z 488.13, found 488.15.

***Preparation of tert-butyl 3-(5-(4-methoxybenzyl)-4-oxo-3-(trifluoromethyl)-1-((2-(trimethylsilyl)ethoxy) methyl)-4,5-dihydro-1H-pyrazolo[4,3-c]pyridin-6-yl)-2,5-dihydro-1H-pyrrole-1-carboxylate (10)***

To a solution of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one **8** (200 mg, 0.48 mmol) and tert-butyl 3-(4,4,5,5-tetramethyl-1,3,2-dioxaborolan-2-yl)-2,5-dihydropyrrole-1-carboxylate **9** (150 mg, 0.50 mmol) in Dioxan:$H_2O$=5:1 (15 mL) was added Pd(dppf)$Cl_2$ (50 mg, 0.07 mmol) and $Na_2CO_3$ (70 mg, 0.06 mmol) at room temperature. The reaction mixture stirred under nitrogen at 80 °C for 3 h. After cooling to room temperature, the reaction mixture was concentrated under reduced pressure and purified by flash chromatography (eluting with PE/ EtOAc = 100 : 0 to 80 : 20) to give product of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)-2,5-dihydro-1H-pyrrole-1-carboxylate **10** (140 mg, 60% yield ) as a yellow oil. LCMS (ESI) calcd for C30H39F3N4O5Si [M + H] $^+$ m/z 621.26, found 621.30.

***Preparation of tert-butyl 3-(5-(4-methoxybenzyl)-4-oxo-3-(trifluoromethyl)-1-((2-(trimethylsilyl)ethoxy) methyl)-4,5-dihydro-1H-pyrazolo[4,3-c]pyridin-6-yl)pyrrolidine-1-carboxylate (11)***

To a solution of tert-butyl 3-(5-(4-methoxybenzyl)-4-oxo-3-(trifluoromethyl)-1-((2-(trimethylsilyl)ethoxy) methyl)-4,5-dihydro-1H-pyrazolo[4,3-c]pyridin-6-yl)-2,5-dihydro-1H-pyrrole-1-carboxylate **10** (140 mg, 0.30 mmol) in MeOH (15 mL) was added Pd/C (60 mg, 0.57 mmol). The reaction mixture stirred under hydrogen at room temperature for 2 h. The reaction mixture was filtered, the solution was concentrated under reduced pressure to give product of tert-butyl 3-(5-(4-methoxybenzyl)-4-oxo-3-(trifluoromethyl)-1-((2-(trimethylsilyl)ethoxy)methyl)-4,5-dihydro-1H-pyrazolo [4,3-c]pyridin-6-yl)pyrrolidine-1-carboxylate **11** (120 mg, 80% yield ) as a colourless oil.

LCMS (ESI) calcd for C30H41F3N405Si [M + H] $^+$ m/z 623.28, found 623.30.

***Preparation of 6-(pyrrolidin-3-yl)-3-(trifluoromethyl)-1,5-dihydro-4H-pyrazolo[4,3-c]pyridin-4-one (12)***

To a solution of tert-butyl 3-(5-(4-methoxybenzyl)-4-oxo-3-(trifluoromethyl)-1-((2-(trimethylsilyl)ethoxy) methyl)-4,5-dihydro-1H-pyrazolo[4,3-c]pyridin-6-yl)pyrrolidine-1-carboxylate **11** (120 mg, 0.19 mmol) in TFA (5 mL) was added two drops of TfOH. The reaction mixture was stirred at 50 °C for 1 h. The reaction mixture was concentrated under reduced pressure to give product of 6-(pyrrolidin-3-yl)-3-(trifluoromethyl)-1,5-dihydro-4H-pyrazolo[4,3-c]pyridin-4-one **12** (50 mg, 90% yield ) as a yellow oil.

LCMS (ESI) calcd for C11H11F3N4O [M + H] $^+$ m/z 273.09, found 273.15.

***Preparation of N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide** (racemic mixture of **194a and 194b)***

To a solution of 6-(pyrrolidin-3-yl)-3-(trifluoromethyl)-1,5-dihydro-4H-pyrazolo[4,3-c]pyridin-4-one **12** (50 mg, 0.18 mmol) in MeOH (10 mL) was added N-methyl-5-(4-oxopiperidin-1-yl)pyridine-2-carboxamide **INT** (43 mg, 0.18 mmol). Then two drops of acetic acid and $NaBH_3CN$ (12 mg, 0.18 mmol) were added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 92 : 8) to give N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide a racemic mixture of compounds **194a** and **194b** (30 mg, 32% yield ) as a white solid.

LCMS (ESI) calcd for C23H26F3N7O2 [M + H] $^+$ m/z 490.21, found 490.17.

***Chiral separation of N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide (compounds 194a and 194b)***

A racemic mixture of N-methyl-5-(4-(3-(4-oxo-3-(trifluoromethyl)-4,5-dihydro-1H-pyrazolo[4,3-c]pyridin-6-yl) pyrrolidin-1-yl)piperidin-1-yl)picolinamide **(194rac)** was separated by SFC (Column: Daicel CHIRALPAK OJ-H 250 mm × 20 mm I.D., 5 μmm; Mobile phase: $CO_2$/MeOH[0.1%($NH_3$)] = 70/30) and concentrated under reduced pressure to afford the first fraction as **194a** (9.9 mg, 98% purity, ee%: 100, white solid) and the second fraction as **194b** (9.4 mg, 100% purity, ee%: 98, white solid)

**Compound 194a**

$^1$H NMR (400 MHz, DMSO) δ 13.82 (s, 1 H), 11.10 (s, 1 H), 8.39 (q, *J* = 4.4 Hz, 1 H), 8.28 (d, *J* = 2.8 Hz, 1 H), 7.83 (d, *J* = 8.8 Hz, 1 H), 7.41 (dd, *J* = 8.8, 2.8 Hz,1 H), 6.39 (s, 1 H), 3.84-3.81 (m, 2 H), 3.29-3.25 (m, 1 H), 2.98 (t, *J* = 11.2 Hz, 2 H), 2.89-2.85 (m, 1 H), 2.81-2.78 (m, 5 H), 2.60-2.56 (m, 1 H), 2.39-2.30 (m, 1 H),2.30-2.17 (m, 1 H), 1.97 (d, *J* = 12.0 Hz, 2 H), 1.83-1.77 (m, 1 H), 1.56-1.51 (m, 2 H).

LCMS (ESI) calcd for C23H26F3N7O2 [M + H] $^+$ m/z 490.21, found 490.20.

**Compound 194b**

$^1H$ NMR (400 MHz, DMSO) δ 13.81 (s, 1 H), 11.11 (s, 1 H), 8.39 (q, *J* = 4.8 Hz, 1 H), 8.28 (d, *J* = 2.8 Hz, 1 H), 7.83 (d, *J* = 8.8 Hz, 1 H), 7.40 (dd, *J* = 8.8, 2.8 Hz,1 H), 6.40 (s, 1 H), 3.91-3.81 (m, 2 H), 3.27-3.23 (m, 1 H), 2.98 (t, *J* = 11.6 Hz, 2 H), 2.90-2.86 (m, 1 H), 2.80-2.77 (m, 5 H), 2.60-2.54 (m, 1 H), 2.36-2.33 (m, 1 H),2.25-2.19 (m, 1 H), 1.97 (d, *J* = 11.2 Hz, 2 H), 1.86-1.75 (m, 1 H), 1.58-1.48 (m, 2 H).

LCMS (ESI) calcd for C23H26F3N7O2 [M + H] + m/z 490.21, found 490.16.

***Example 19 - Synthesis of compounds 208a and 208b***

**Synthesis of compounds 208a and 208b**

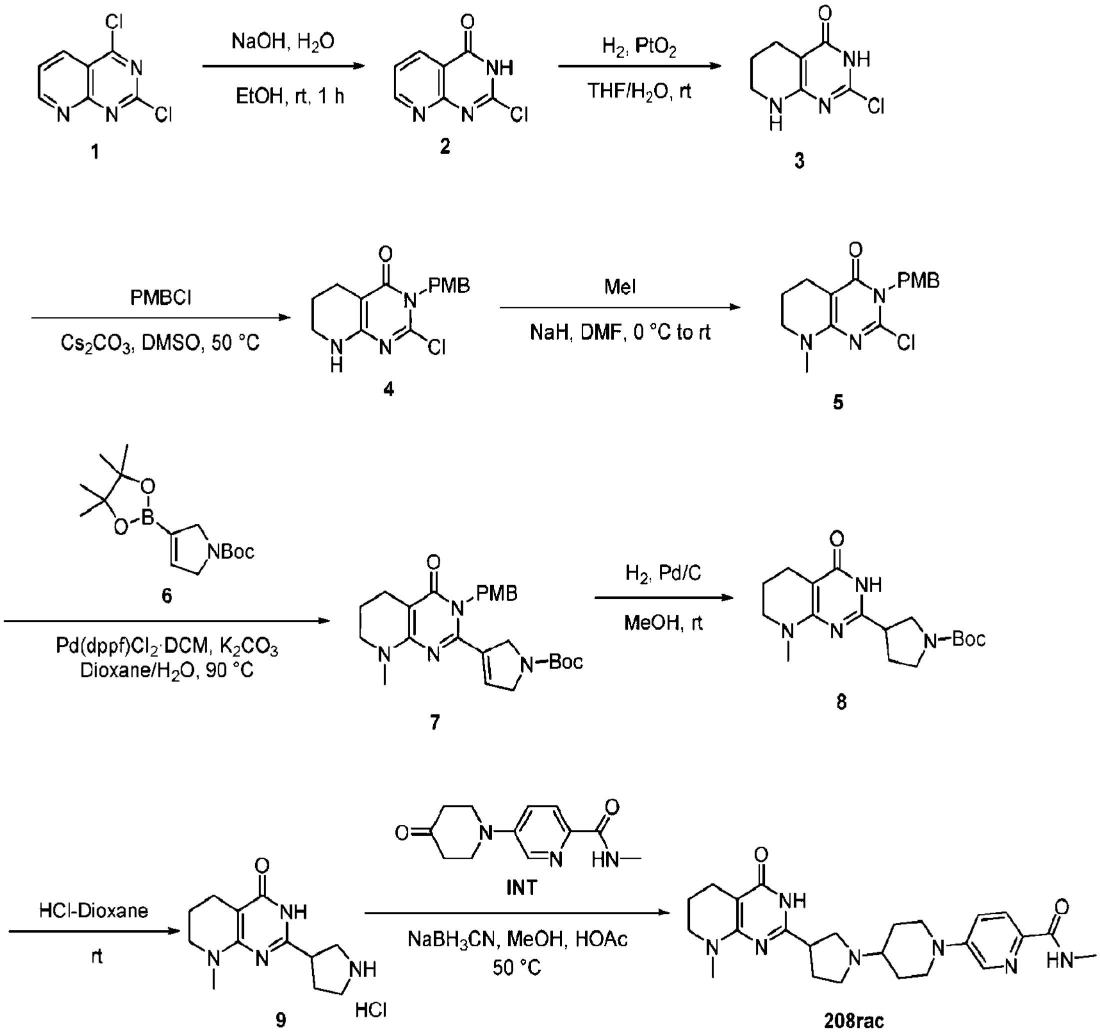


***Preparation of 2-chloropyrido[2,3-d]pyrimidin-4(3H)-one (2)***

To a solution of 2,4-dichloropyrido[2,3-d] pyrimidine 1 (1.5 g, 0.0075 mol) in EtOH (30 mL) at room temperature was added NaOH (0.66 g, 0.017 mol) in $H_2O$ (16.5 mL) at rt. The reaction mixture was stirred at rt for 1 h. The reaction solution was quenched with water, then adjusted pH to 4-5 with 1 M HCl. The solution was filtered, and the filter cake was collected and dried *in vacuo* pressure to give 2-chloropyrido[2,3-d]pyrimidin-4(3H)-one **2** (1.1 g, 90% purity, 73% yield) as yellow solid.

LCMS (ESI) calcd for $C_7H_4ClN_3O$ [M + H] + m/z 182.00, found 181.90.

***Preparation of 2-chloro-5,6,7,8-tetrahydropyrido[2,3-d]pyrimidin-4(3H)-one (3)***

To a solution of 2-chloropyrido[2,3-d] pyrimidin-4(3H)-one **2** (1.1 g, 0.0061 mol) in THF/$H_2O$ = 4:1 (50 mL) was added $PtO_2$ (0.14 g). Then the mixture was stirred under hydrogen for 18 h at rt. The mixture was filtered through a celite pad, and the filtrate was concentrated under reduced pressure to give 2-chloro-5,6,7,8-tetrahydropyrido[2,3-d] pyrimidin-4(3H)-one **3** (1.1 g, 95% purity, 91% yield) as a yellow solid.

LCMS (ESI) calcd for $C_7H_8ClN_3O$ [M + H] $^+$ m/z 186.04, found 185.95.

***Preparation of 2-chloro-3-(4-methoxybenzyl)-5,6,7,8-tetrahydropyrido[2,3-d]pyrimidin-4(3H)-one (4)***

To a solution of 2-chloro-5,6,7,8-tetrahydropyrido[2,3-d] pyrimidin-4(3H)-one **3** (0.9 g, 4.85 mmol) in DMSO (150 mL) at room temperature were added $Cs_2CO_3$ (4.7 g, 14.5 mmol) and PMBCl (1.52 g, 9.7 mmol). The reaction mixture was stirred at 50 °C for 1 h. The reaction solution was quenched with water and extracted with EtOAc (100 mL × 3). The combined organic phase was washed three times with brine and concentrated under reduced pressure. The residue was purified by flash chromatography (eluting with PE/EtOAc = 100 : 0 to 50: 50) to give 2-chloro-3-(4-methoxybenzyl)-5,6,7,8-tetrahydropyrido[2,3-d]pyrimidin-4(3H)-one **4** (1.23 g, 90% purity, 74% yield) as white solid.

LCMS (ESI) calcd for $C_{13}H_{16}ClN_3O_2$ [M + H] $^+$ m/z 306.09, found 305.80.

***Preparation of 2-chloro-3-(4-methoxybenzyl)-8-methyl-5,6,7,8-tetrahydropyrido[2,3-d]pyrimidin-4(3H)-one (5)***

To a solution of 2-chloro-3-(4-methoxybenzyl)-5,6,7,8-tetrahydropyrido[2,3-d]pyrimidin-4(3H)-one **4** (1.2 g, 0.004 mol) in DMF (50 mL) at 0 °C were added MeI (3.05 g, 0.02 mol) and NaH (60% in mineral oil, 0.32 g, 0.008 mol). The reaction mixture was stirred at rt for 2 h. The reaction solution was quenched with water and extracted with EtOAc (100 mL × 3). The combined organic phase was washed three times with brine and concentrated under reduced pressure. The residue was purified by flash chromatography (eluting with PE/EtOAc = 100 : 0 to 50: 50) to give 2-chloro-3-(4-methoxybenzyl)-8-methyl-5,6,7,8-tetrahydropyrido[2,3-d]pyrimidin-4(3H)-one **5** (450 mg, 90% purity, 33% yield) as yellow oil.

LCMS (ESI) calcd for $C_{16}H_{18}ClN_3O_2$ [M + H] $^+$ m/z 320.11, found 320.00.

***Preparation of tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate (7)***

To a solution of 2-chloro-3-(4-methoxybenzyl)-8-methyl-5,6,7,8-tetrahydropyrido[2,3-d]pyrimidin-4(3H)-one **5** (450 mg, 1.41 mmol) in dioxane/$H_2O$ = 5:1 (30 mL) at room temperature were added tert-butyl 3-(4,4,5,5-tetramethyl-1,3,2-dioxaborolan-2-yl)-2,5-dihydro-1H-pyrrole-1-carboxylate **6** (500 mg, 1.69 mmol), Pd(dppf)$Cl_2$·DCM (115 mg, 0.14 mmol) and $K_2CO_3$ (587 mg, 4.22 mmol). The reaction mixture was stirred at 90 °C for 3 h. The reaction solution was quenched with water and extracted with EtOAc (50 mL × 3). The combined organic phase was concentrated under reduced pressure. The residue was purified by flash chromatography (eluting with PE/EtOAc = 100 : 0 to 50: 50) to give tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate **7** (550 mg, 90% purity, 77% yield) as white solid. LCMS (ESI) calcd for $C_{23}H_{32}N_4O_4$ [M + H] $^+$ m/z 453.24, found 453.20.

***Preparation of tert-butyl 3-(8-methyl-4-oxo-3,4,5,6,7,8-hexahydropyrido[2,3-d]pyrimidin-2-yl)pyrrolidine-1-carboxylate (8)***

To a solution of tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate **7** (550 mg, 1.2 mmol) in McOH (15 mL) at room temperature were added Pd/C (55 mg). The reaction mixture was stirred at rt for 18 h under a hydrogen atmosphere. The reaction solution was filtered, and the filter cake was washed with MeOH (5 mL × 2). The filtrate was concentrated under reduced pressure to give tert-butyl 3-(8-methyl-4-oxo-3,4,5,6,7,8-hexahydropyrido[2,3-d]pyrimidin-2-yl)pyrrolidine-1-carboxylate **8** (400 mg, 90% purity, 88% yield) as yellow oil. LCMS (ESI) calcd for $C_{17}H_{26}N_4 0_3$ [M + H] $^+$ m/z 335.20, found 335.30.

***Preparation of 8-methyl-2-(pyrrolidin-3-yl)-5,6,7,8-tetrahydropyrido[2,3-d]pyrimidin-4(3H)-one hydrochloride (9)***

A solution of tert-butyl 3-(8-methyl-4-oxo-3,4,5,6,7,8-hexahydropyrido[2,3-d]pyrimidin-2-yl)pyrrolidine-1-carboxylate **8** (200 mg, 0.60 mmol) in HCl-dioxanc (4 M, 10 mL) was stirred at room temperature for 2 h under $N_2$ atmosphere. The precipitate formed was washed with DCM (5 mL × 3), collected and dried under reduced pressure to obtain 8-methyl-2-(pyrrolidin-3-yl)-5,6,7,8-tetrahydropyrido[2,3-d]pyrimidin-4(3H)-one hydrochloride **9** (110 mg, 90% purity, 70% yield) as white solid.

LCMS (ESI) calcd for $C_{12}H_{18}N_4O$ $[M + H]^+$ m/z 235.15, found 235.00.

***Preparation of N-methyl-5-(4-(3-(8-methyl-4-oxo-3,4,5,6,7,8-hexahydropyrido[2,3-d]pyrimidin-2-yl)pyrrolidin-1-yl)piperidin-1-yl)picolinamide (208a and 208b)***

To a solution of 8-methyl-2-(pyrrolidin-3-yl)-5,6,7,8-tetrahydropyrido[2,3-d]pyrimidin-4(3H)-one hydrochloride **9** (110 mg, 0.46 mmol) in MeOH (1 mL) was added TEA (1 mL) at rt and stirred for 5 mins, then the reaction was concentrated to dryness under reduced pressure. The residue was dissolved in MeOH (15 mL) and AcOH (0.01 mL) at room temperature. N-methyl-5-(4-oxopiperidin-1-yl)picolinamide **INT** (131 mg, 0.56 mmol) and $NaBH_3CN$ (88 mg, 1.40 mmol) were added. The reaction mixture was stirred at 50 °C for 1 h. The reaction solution was concentrated under reduced pressure and the residue was purified by flash chromatography (eluting with DCM/MeOH = 100 : 0 to 90: 10) to give a racemic mixture of N-methyl-5-(4-(3-(8-methyl-4-oxo-3,4,5,6,7,8-hexahydropyiido[2,3-d]pyrimidin-2-yl)pyrolidin-1-yl)piperidin-1-yl)picolinamide **208rac** (120 mg, 99% purity, 55 % yield) as a white solid.

LCMS (ESI) calcd for $C_{24}H_{33}N_7O_2$ $[M + H]^+$ m/z 452.27, found 452.20.

***Chiral resolution of N-methyl-5-(4-(3-(8-methyl-4-oxo-3,4,5,6,7,8-hexahydropyrido[2,3-d]pyrimidin-2-yl)pyrrolidin-1-yl)piperidin-1-yl)picolinamide (208rac)***

A racemic mixture of compounds **208a** and **208b** was separated by SFC (Column: Daicel Chiralpak IH SFC, 250 mm ×20 mm I.D., 5 μmm; Mobile phase: $CO_2$/MeOH [0.1% $NH_3$] = 70/30) and concentrated under reduced pressure to afford the first fraction as **208a** (52.3 mg, 99% purity, 100% ee, white solid) and the second fraction as **208b** (54.2 mg, 100% purity, 98% ee, white solid).

**Compound 208a**

$^1$H NMR (400 MHz, DMSO-$d_6$, ppm) δ: 11.37 (s, 1 H), 8.43-8.34 (m, 1 H), 8.26 (d, *J* = 2.8 Hz, 1 H), 7.81 (d, *J* = 8.8 Hz, 1 H), 7.39 (dd, *J* = 8.8, 3.2 Hz, 1 H), 3.82-3.77(m, 2 H), 3.23-3.20 (m, 2 H), 3.13-3.09 (m, 1 H), 3.03 (s, 3 H), 2.99-2.92 (m, 3 H), 2.78 (d, *J* = 4.8 Hz, 3 H), 2.68-2.66 (m, 1 H), 2.59-2.57 (m, 1 H), 2.35-2.26 (m, 4 H), 2.07-2.00 (m, 2 H), 1.94-1.88 (m, 2 H), 1.78-1.72 (m, 2 H), 1.53-1.44 (m, 2 H).

LCMS (ESI) calcd for $C_{24}H_{33}N_7O_2$ $[M + H]^+$ m/z 452.27, found 452.30.

**Compound 208b**

$^1$H NMR (400 MHz, DMSO-$d_6$, ppm) δ: 11.35 (s, 1 H), 8.41-8.33 (m, 1 H), 8.26 (d, *J* = 2.8 Hz, 1 H), 7.81 (d, *J* = 8.8 Hz, 1 H), 7.39 (dd, *J* = 8.8, 2.8 Hz, 1 H), 3.85-3.75 (m, 2 H), 3.24-3.19 (m, 2 H), 3.14-3.07 (m, 1 H), 3.03 (s, 3 H), 3.00-2.88 (m, 3 H), 2.77 (d, *J* = 4.8 Hz, 3 H), 2.67-2.62 (m, 1 H), 2.61-2.56 (m, 1 H), 2.35-2.21 (m, 4 H), 2.09-1.99 (m, 2 H), 1.96-1.87 (m, 2 H), 1.79-1.70 (m, 2 H), 1.55-1.43 (m, 2 H).

LCMS (ESI) calcd for $C_{24}H_{33}N_7O_2$ $[M + H]^+$ m/z 452.27, found 452.30.

***Example 20 - Synthesis of INT-3***

Synthesis **of INT-3**

***Preparation of tert-butyl 3-(4-fluoro-1-oxo-1,2-dihydroisoquinolin-3-yl)pyrrolidine-1-carboxylate (INT-3)***

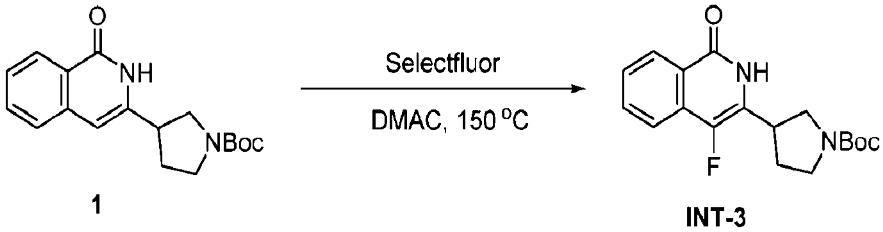


1

INT-3

To a solution of tert-butyl 3-(1-oxo-1,2-dihydroisoquinolin-3-yl)pyrrolidine-1-carboxylate **1** (500 mg, 1.59 mmol) in DMAC (20 mL) was added Selectfluor (563 mg, 1.59 mmol) at rt. The mixture was stirred at 150 °C for 10 min. After cooling to rt, the reaction mixture was added into cold water and then extracted with EtOAc (100 mL × 3). The combined organic layer was washed with brine, dried over $Na_2SO_4$ and concentrated under reduced pressure. The residue was purified by flash silica chromatography (eluting with PE/EtOAc = 100 : 0 to 50 : 50) to give tert-butyl 3-(4-fluoro-1-oxo-1,2-dihydroisoquinolin-3-yl)pyrrolidine-1-carboxylate **INT-3** (100 mg, 60% purity, 11% yield) as yellow oil.

LCMS (ESI) calcd for $C_{18}H_{21}FN_2O_3$ [M + H] $^+$ m/z 333.15, found 332.90.

***Example 21 - synthesis of compound 259***

**Synthesis of compound 259**

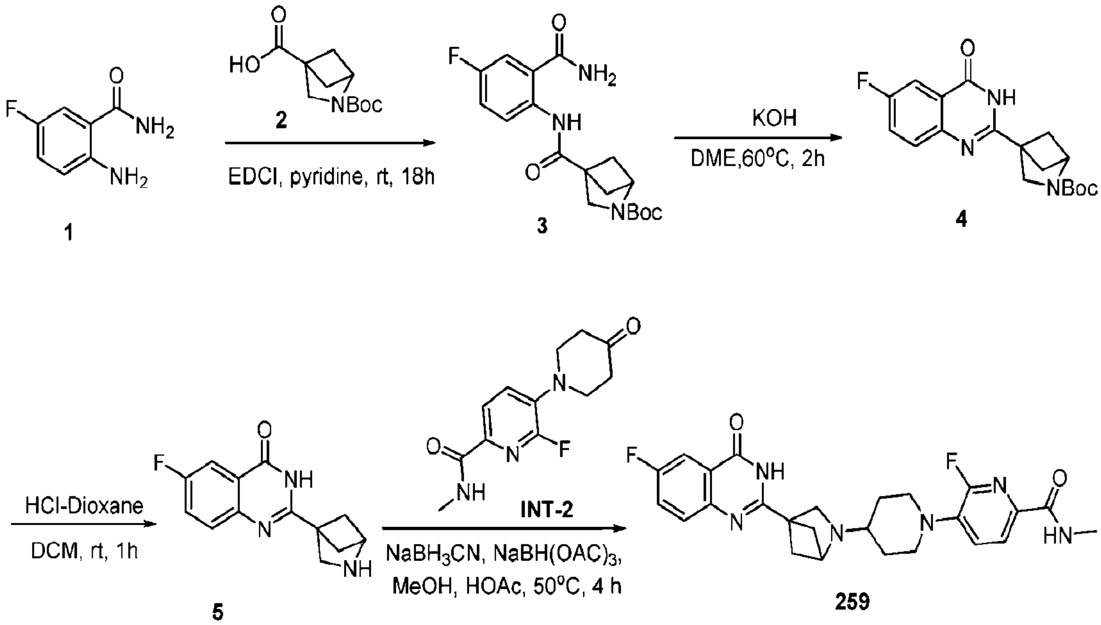


***Preparation of tert-butyl 4-((2-carbamoyl-4-fluorophenyl)carbamoyl)-2-azabicyclo[2.1.1]hexane-2-carboxylate (3)***

To a solution of 2-amino-5-fluorobenzamide **1** (30 mg, 2.21 mmol) and 2-(tert-butoxycarbonyl)-2-azabicyclo [2.1.1]hexane-4-carboxylic acid **2** (520 mg, 2.20 mmol) in pyridine (20 mL) was added EDCI (423 mg, 2.21 mmol), then the mixture was stirred for 18 h at room temperature. The reaction mixture was quenched with water, the aqueous layer was extracted with EtOAc (150 mL x 3). The combined organic layers were washed with 1M HCl solution and brine, dried over $Na_2SO_4$, concentrated under reduced pressure to give product of tert-butyl 4-((2-carbamoyl-4-fluorophenyl)carbamoyl)-2-azabicyclo[2.1.1]hexane-2-carboxylate 3 (500 mg, 56% yield) as a white solid .

LCMS (ESI) calcd for $C_{18}H_{22}FN_3O_4$ [M + Na] $^+$ m/z 386.16, found 385.95.

***Preparation of tert-butyl 4-(6-fluoro-4-oxo-3,4-dihydroquinazolin-2-yl)-2-azabicyclo[2.1.1]hexane-2-carboxylate (4)***

To the solution of *tert*-butyl 4-((2-carbamoyl-4-fluorophenyl)carbamoyl)-2-azabicyclo[2.1.1]hexane-2-carboxylate **3** (500 mg, 1.38 mmol) in DME (50 mL) was added KOH (154 mg, 2.75 mmol). The solution was stirred at 60 °C for 2 h, cooled to room temperature, then the reaction mixture was concentrated under reduced pressure. The residue was purified by flash chromatography (eluting with DCM/MeOH = 100 : 0 to 90 : 10) to give tert-butyl 4-(6-[luoro-4-oxo-3.4-dihydroquinazolin-2-yl)-2-azabicyclo[2.1.]1.1]hexane-2-carboxylate **4** (400 mg, 76% yield ) as a white solid.

LCMS (ESI) calcd for $C_{18}H_{20}FN_3O_3$ [M + H] $^+$ m/z 346.15, found 345.85.

***Preparation of 2-(2-azabicyclo[2.1.1]hexan-4-yl)-6-fluoroquinazolin-4(3H)-one (5)***

To a solution of tert-butyl 4-(6-fluoro-4-oxo-3,4-dihydroquinazolin-2-yl)-2-azabicyclo[2.1.1]hexane-2-carboxylate **4** (400 mg, 1.16 mmol) in DCM (10 mL) was added HCl in dioxane (4M, 20 mL). The mixture was stirred for 1 h at room temperature. The reaction mixture was concentrated under reduced pressure to give product of 2-(2-azabicyclo[2.1.1]hexan-4-yl)-6-fluoroquinazolin-4(3H)-one **5** (200 mg, 63% yield ) as a white solid.

LCMS (ESI) calcd for $C_{15}H_{17}N_3O$ [M + H] $^+$ m/z 246.10, found 245.95.

***Preparation of 6-fluoro-5-(4-(4-(6-fluoro-4-oxo-3,4-dihydroquinazolin-2-yl)-2-azabicyclo[2.1.1]hexan-2-yl)piperidin-1-yl)-N-methylpicolinamide (Compound 259)***

To a solution of 2-(2-azabicyclo[2.1.1]hexan-4-yl)-6-fluoroquinazolin-4(3H)-one **5** (60 mg, 0.24 mmol) in MeOH (20 mL) was added 6-fluoro-N-methyl-5-(4-oxopiperidin-1-yl)picolinamide **INT-2** (61 mg, 0.24 mmol), then two drops of acetic acid and $NaBH(OAC)_3$ (104 mg, 0.49 mmol) were added at room temperature. After 1 h, $NaBH_3CN$ (8 mg, 0.13 mmol) was added. The reaction mixture stirred at 50 °C for 4 h. After cooling to room temperature, the reaction mixture was concentrated under reduced pressure. The residue was purified by $C_{18}$ column (mobile phase: ACN - $H_2O$ (0.1% FA), gradient: 10 - 95) to give 6-fluoro-5-(4-(4-(6-fluoro-4-oxo-3,4-dihydroquinazolin-2-yl)-2-azabicyclo[2.1.1]hexan-2-yl)piperidin-1-yl)-N-methylpicolinamide **259** (45.5 mg, 99 % purity, 15% yield) as a white solid.

$^1H$ NMR (400 MHz, DMSO-$d_6$, ppm) δ: 12.29 (s, 1 H), 8.40 (q, 1 H), 7.84 (dd, *J* = 8.0, 1.2 Hz, 1 H), 7.77 (dd, *J* = 8.4, 2.8 Hz, 1 H), 7.72-7.65 (m, 2 H), 7.62-7.56 (m, 1 H), 3.70 (s, 1 H), 3.59-3.53 (m, 2 H), 3.05 (s, 2 H), 2.86 (t, *J* = 11.2 Hz, 2 H), 2.77 (d, *J* = 4.8 Hz, 3 H), 2.57-2.52 (m, 1 H), 2.15-2.09 (m, 2 H), 1.99 (d, *J* = 10.8 Hz, 2 H), 1.92-1.86 (m, 2 H), 1.58-1.47 (m, 2 H).

LCMS (ESI) calcd for $C_{25}H_{26}F_2N_eO_2$ [M + H] $^+$m/z 481.21, found 481.05.

***Example 22 - synthesis of compound 299***

**Synthesis of compound 299**

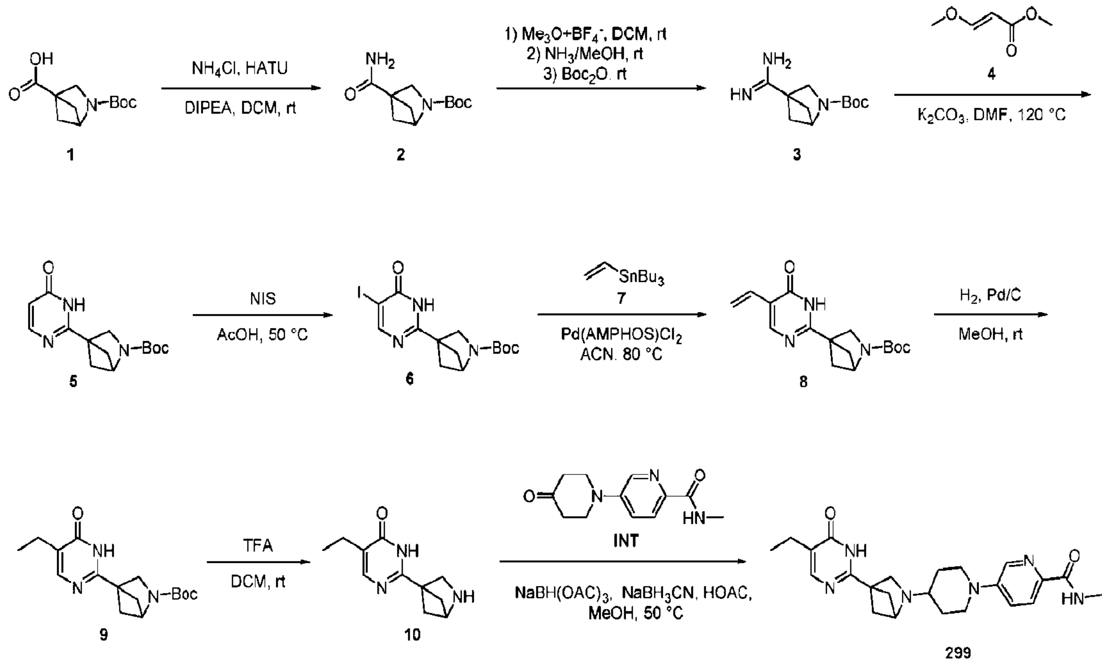


***Preparation of tert-butyl ((1-(6-(methylcarbamoyl)pyridin-3-yl)piperidin-4-yl)methyl)carbamate (2)***

To a solution of 2-(tert-butoxycarbonyl)-2-azabicyclo[2.1.1]hexane-4-carboxylic acid **1** (500 mg, 2.19 mmol) in DCM (20 mL) were added $NH_4Cl$ (234 mg, 4.38 mmol), DIPEA (1415 mg, 10.95 mmol) and HATU (1249 mg, 3.28 mmol) at room temperature successively. The mixture was kept stirring at room temperature for 1 h. The resulting mixture was

diluted with water and extracted with DCM (50 mL × 3). The combined organic layers were dried over $Na_2SO_4$ and concentrated under reduced pressure. The residue was purified by flash chromatography (eluting with MeOH/DCM, 3% to 5%) to give tert-butyl 4-carbamoyl-2-azabicyclo[2.1.1]hexane-2-carboxylate 2 (400 mg, 90% purity, 72% yield) as a white solid.

LCMS (ESI) calcd for $C_{11}H_{18}N_2O_3$ [M - 56 + H] + m/z 171.13, found 171.10.

***Preparation of tert-butyl 4-carbamimidoyl-2-azabicyclo[2.1.1]hexane-2-carboxylate (3)***

Tert-butyl 4-carbamoyl-2-azabicyclo[2.1.1]hexane-2-carboxylate **2** (400 mg, 1.76 mmol) was added to a suspension of $Me_3O^+BF_4^-$ (312 mg, 2.1 mmol) in anhydrous DCM (10 mL). The mixture was stirred under argon atmosphere at rt for 2 h. Solvent was then removed to provide crude imidate salt. This crude was dissolved in $NH_3$/MeOH (7 M, 10 mL) and stirred at rt for 16 h. Then $Boc_2O$ (1152 mg, 5.28 mmol) was added. The mixture was stirred at rt for 10 min and then concentrated to give crude tert-butyl 4-carbamimidoyl-2-azabicyclo[2.1.1]hexane-2-carboxylate **3** (500 mg, 50% purity, 62% yield) as a white solid.

LCMS (ESI) calcd for $C_{11}H1_9N_3O_2$ [M + H] + m/z 226.15, found 226.05.

***Preparation of tert-butyl 4-(6-oxo-1,6-dihydropyrimidin-2-yl)-2-azabicyclo[2.1.1]hexane-2-carboxylate (5)***

To a solution of tert-butyl 4-carbamimidoyl-2-azabicyclo[2.1.1]hexane-2-carboxylate **3** (500 mg, 2.21 mmol) in DMF (20 mL) were added $K_2CO_3$ (916 mg, 6.63 mmol) and methyl (E)-3-methoxyacrylate **4** (770 mg, 6.63 mmol) in one portion. The reaction mixture was heated with stirring at 120 °C for 2 h. The reaction mixture was concentrated and purified by silica gel column chromatography (eluting with EtOAc/PE, 50% to 100%) to obtain tert-butyl 4-(6-oxo-1,6-dihydropyrimidin-2-yl)-2-azabicyclo[2.1.1]hexane-2-carboxylate 5 (200 mg, 90% purity, 29% yield) as a white solid.

LCMS (ESI) calcd for $C_{14}H_{19}N_3O_3$ [M + H] + m/z 278.15, found 277.95.

***Preparation of tert-butyl 4-(5-iodo-6-oxo-1,6-dihydropyrimidin-2-yl)-2-azabicyclo[2.1.1]hexane-2-carboxylate (6)***

To a solution of tert-butyl 4-(6-oxo-1,6-dihydropyrimidin-2-yl)-2-azabicyclo[2.1.1]hexane-2-carboxylate **5** (215 mg, 0.77 mmol) in AcOH (5 mL) was added NIS (260 mg, 1.16 mmol) in one portion. The reaction mixture was heated with stirring at 50 °C for 2 h. The reaction mixture was concentrated and purified by silica gel column chromatography (eluting with EtOAc/PE, 50% to 70%) to obtain tert-butyl 4-(5-iodo-6-oxo-1,6-dihydropyrimidin-2-yl)-2-azabicyclo[2.1.1]hexane-2-carboxylate 6 (180 mg, 90% purity, 51% yield) as a white solid.

LCMS (ESI) calcd for $Ci_4HisIN_3O_3$ [M + H] + m/z 404.04, found 403.85.

***Preparation of tert-butyl 4-(6-oxo-5-vinyl-1,6-dihydropyrimidin-2-yl)-2-azabicyclo[2.1.1]hexane-2-carboxylate (8)***

A solution of tert-butyl 4-(5-iodo-6-oxo-1,6-dihydropyrimidin-2-yl)-2-azabicyclo[2.1.1]hexane-2-carboxylate **6** (180 mg, 0.44 mmol), tributyl(vinyl)stannane **7** (282 mg, 0.89 mmol) and Pd(AMPHOS)$Cl_2$ (32 mg, 0.04 mmol) in ACN (10 mL) was purged with $N_2$ for 2 min and then stirred at 80 °C for 1 h. After cooling to rt, the reaction mixture was concentrated and purified by flash chromatography (eluting with EtOAc/PE, 50% to 70%) to give tert-butyl 4-(6-oxo-5-vinyl-1,6-dihydropyrimidin-2-yl)-2-azabicyclo[2.1.1]hexane-2-carboxylate **8** (120 mg, 90% purity, 79% yield) as a white solid.

LCMS (ESI) calcd for $C_{16}H_{21}N_3O_3$ [M + H] + m/z 304.16, found 304.20.

***Preparation of tert-butyl 4-(5-ethyl-6-oxo-1,6-dihydropyrimidin-2-yl)-2-azabicyclo[2.1.1]hexane-2-carboxylate (9)***

To a solution of give tert-butyl 4-(6-oxo-5-vinyl-1,6-dihydropyrimidin-2-yl)-2-azabicyclo[2.1.1]hexane-2-carboxylate **8** (120 mg, 0.40 mmol) in MeOH (5 mL) was added 10% Pd/C (12 mg). The mixture was evacuated and backfilled with nitrogen three times and then charged with hydrogen. The resulting mixture was stirred at room temperature for 16 hours. Then the mixture was filtered through celite and concentrated under vacuum to give crude tert-butyl 4-(5-ethyl-6-oxo-1,6-dihydropyrimidin-2-yl)-2-azabicyclo[2.1.1]hexane-2-carboxylate **9** (120 mg, 90% purity, 89% yield) as a white solid which was used directly in the next step without further purification.

LCMS (ESI) calcd for $C_{16}H_{23}N_3O_3$ [M + H] + m/z 306.18, found 306.00.

***Preparation of 2-(2-azabicyclo[2.1.1]hexan-4-yl)-5-ethylpyrimidin-4(3H)-one (10)***

To a solution of tert-butyl 4-(5-ethyl-6-oxo-1,6-dihydropyrimidin-2-yl)-2-*azabicyclo[2.1.1]hexane-2-carboxylate* **9** (120 mg, 0.39 mmol) in DCM (4 mL) was added TFA (1 mL) dropwise at 0 °C. The reaction solution was stirred at room temperature for 1 hour then concentrated under vacuum. The resulted mixture quenched with $Et_3N$ and concentrated under vacuum to afford 2-(2-azabicyclo[2.1.1]hexan-4-yl)-5-ethylpyrimidin-4(3H)-one **10** (120 mg, 50% purity, 74% yield).

LCMS (ESI) calcd for $C_{11}H_{15}N_3O$ $[M + H]^+$ m/z 206.12, found 206.00.

***Preparation of 5-(4-(4-(5-ethyl-6-oxo-1,6-dihydropyrimidin-2-yl)-2-azabicyclo[2.1.1]hexan-2-yl)piperidin-1-yl)-N-methylpicolinamide (299)***

To a solution of 2-(2-azabicyclo[2.1.1]hexan-4-yl)-5-ethylpyrimidin-4(3H)-one **10** (40 mg, 0.19 mmol) in MeOH (5 mL) was added N-methyl-5-(4-oxopiperidin-1-yl)picolinamide **INT** (68 mg, 0.29 mmol), then two drops of acetic acid and $NaBH(OAc)_3$ (82 mg, 0.39 mmol) were added at room temperature. After 1 h, $NaBH_3CN$ (12 mg, 0.19 mmol) was added. The reaction mixture stirred at 50 °C for 1 h. After cooling to room temperature, the reaction mixture was concentrated under reduced pressure. The residue was purified by prep-HPLC (Gemini 5 um C18 150 × 21.2 mm, eluting with 10% to 40% ACN/$H_2O$ containing 0.1% $NH_3 \cdot H_2O$) and prep-TLC (MeOH/DCM, 1/20) to give 5-(4-(4-(5-ethyl-6-oxo-1,6-dihydropyrimidin-2-yl)-2-azabicyclo[2.1.1]hexan-2-yl)piperidin-1-yl)-N-methylpicolinamide **299** (3.2 mg, 99% purity, 3% yield ) as a white solid.

$^1H$ NMR (400 MHz, DMSO-$d_6$, ppm) $\delta$: 12.30 (s, 1 H), 8.39 (q, *J* = 4.8 Hz, 1 H), 8.27 (d, *J* = 2.8 Hz, 1 H), 7.81 (d, *J* = 8.8 Hz, 1 H), 7.72 (s, 1 H), 7.40 (dd, *J* = 8.8, 2.8 Hz, 1 H), 3.94-3.81 (m, 2 H), 3.64 (s, 1 H), 3.00-2.85 (m, 4 H), 2.78 (d, *J* = 4.8 Hz, 3 H), 2.57-2.52 (m, 1 H), 2.40-2.27 (m, 2 H), 2.07-2.01 (m, 2 H), 1.96-1.91 (m, 2 H), 1.87-1.72 (m, 2 H), 1.50-1.36 (m, 2 H), 1.07 (t, *J* = 7.6 Hz, 3 H).
LCMS (ESI) calcd for $C_{23}H_{30}N_6O_2$ $[M + H]^+$ m/z 423.25, found 423.15

***<u>Example 23 - synthesis of compounds 309a and 309b</u>***

**Synthesis of compounds 309a and 309b**

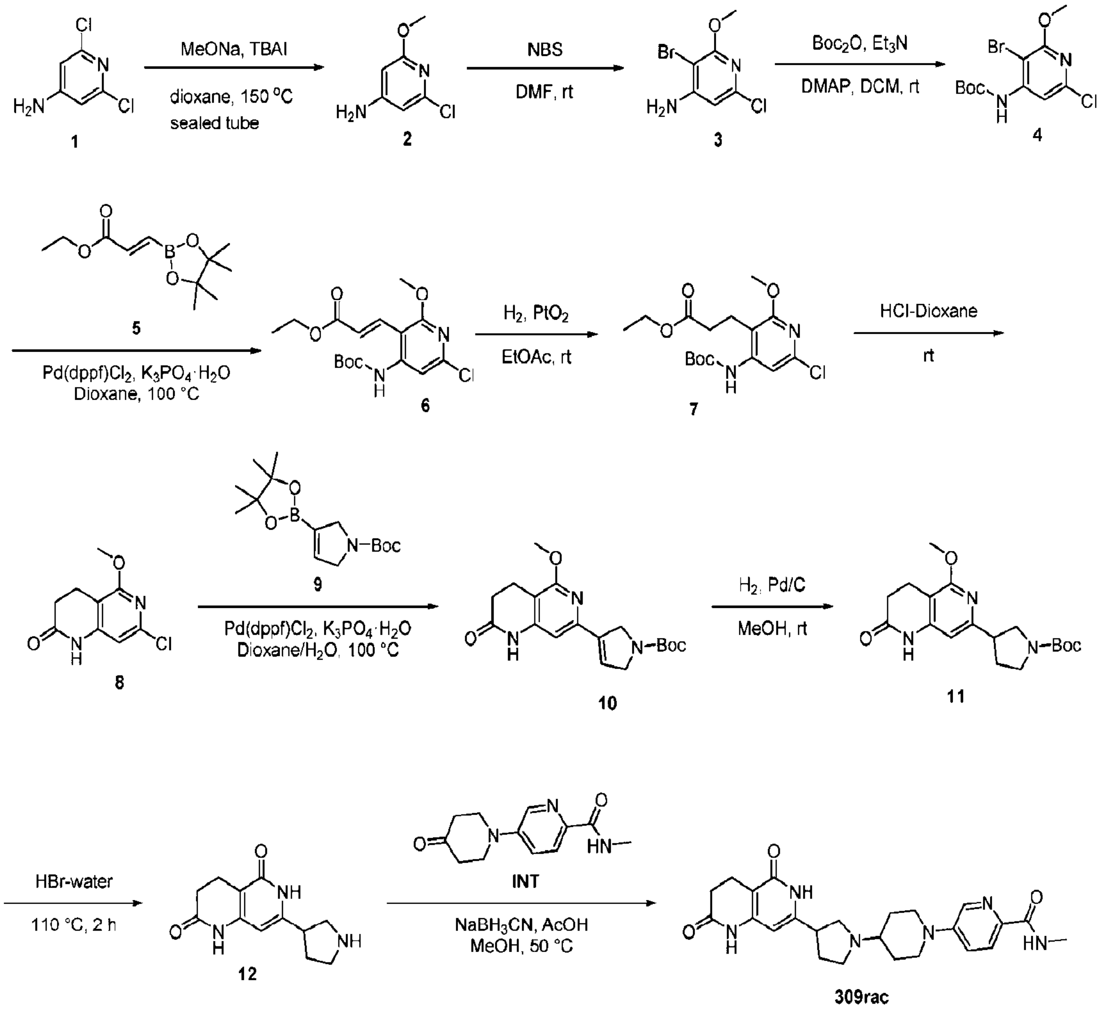


### *Preparation of 2-chloro-6-methoxypyridin-4-amine (2)*

To a solution of 2,6-dichloropyridin-4-amine **1** (1.0 g, 6.1 mmol) in dioxane (15 mL) was added MeONa (3.3 g, 61.0 mmol) and TBAI (230 mg, 0.6 mmol) at rt. The reaction mixture was stirred at 150 °C for 15 h in a sealed tube. The reaction solution was cooled to rt and concentrated under reduced pressure. The residue was purified by flash column chromatography (eluting with PE/EtOAc = 100 : 0 to 95 : 5) to afford 2-chloro-6-methoxypyridin-4-amine 2 (880 mg, 90% purity, 81% yield) as a white solid.

LCMS (ESI) calcd for $C_6H_7ClN_2O$ [M + H] $^+$ m/z 159.02, found 158.93.

### *Preparation of 3-bromo-6-chloro-2-methoxypyridin-4-amine (3)*

To a solution of 2-chloro-6-methoxypyridin-4-amine **2** (880 mg, 5.5 mmol) in DMF (40 mL) was added NBS (1.2 g, 6.7 mmol) at rt. The reaction mixture was stirred at rt for 1 h. The reaction mixture was poured into water. The aqueous layer was extracted with EtOAc (100 mL × 3). The combined organic layers were washed with brine (100 mL × 3), dried over $Na_2SO_4$ and concentrated under reduced pressure. The residue was purified by flash column chromatography (eluting with PE/EtOAc = 100 : 0 to 70 : 30) to afford 3-bromo-6-chloro-2-methoxypyridin-4-amine 3 (950 mg, 90% purity, 64% yield) as a white solid.

LCMS (ESI) calcd for $C_6H_6BrClN_2O$ [M + H] $^+$ m/z 236.94, found 236.87.

### *Preparation of tert-butyl (3-bromo-6-chloro-2-methoxypyridin-4-yl)carbamate (4)*

To a solution of 3-bromo-6-chloro-2-methoxypyridin-4-amine **3** (950 mg, 4.0 mmol) in DCM (30 mL) was added $Boc_2O$ (1.31 g, 6.0 mmol), $Et_3N$ (810 mg, 8.0 mmol) and DMAP (244 mg, 2.0 mmol) successively at rt. The reaction mixture was stirred at rt for 1 h. The reaction solution was concentrated under reduced pressure. The residue was purified by flash

column chromatography (eluting with PE/EtOAc = 100 : 0 to 80 : 20) to afford a mixture of tert-butyl (3-bromo-6-chloro-2-methoxypyridin-4-yl)carbamate 4 and tert-butyl (3-bromo-6-chloro-2-methoxypyridin-4-yl)(tert-butoxycarbonyl)carbamate **4a** (750 mg, 90% purity, 49% yield) as a white solid.

LCMS (ESI) calcd for $C_{11}H_{14}BrClN_2O_3$ [M + H] $^+$ m/z 336.99, found 336.92.

***Preparation of ethyl (E)-3-(4-((tert-butoxycarbonyl)amino)-6-chloro-2-methoxypyridin-3-yl)acrylate (6)***

To a solution of the mixture of tert-butyl (3-bromo-6-chloro-2-methoxypyridin-4-yl)carbamate **4** and tert-butyl (3-bromo-6-chloro-2-methoxypyridin-4-yl)(tert-butoxycarbonyl)carbamate **4a** (750 mg, 2.2 mmol) in dioxane/$H_2O$ (25 mL, 10:1) was added ethyl (E)-3-(4,4,5,5-tetramethyl-1,3,2-dioxaborolan-2-yl)acrylate **5** (751 mg, 3.3 mmol), Pd(dppf)$Cl_2$·DCM (181 mg, 0.22 mmol) and $K_3PO_4$·$H_2O$ (1.5 g, 6.6 mmol) successively at rt. The reaction mixture was stirred at 100 °C for 6 h under $N_2$. The reaction solution was cooled to rt and concentrated under reduced pressure. The residue was purified by flash column chromatography (eluting with PE/EtOAc = 100 : 0 to 70 : 30) to afford a mixture of ethyl (E)-3-(4-((tert-butoxycarbonyl)amino)-6-chloro-2-methoxypyridin-3-yl)acrylate **6** and ethyl (E)-3-(4-(bis(tert-butoxycarbonyl)amino)-6-chloro-2-methoxypyridin-3-yl)acrylate **6a** (410 mg, 90% purity, 46% yield) as a white solid.

LCMS (ESI) calcd for $C_{16}H_{21}ClN_2O_5$ [M + H] $^+$ m/z 357.11, found 357.00.

***Preparation of ethyl 3-(4-((tert-butoxycarbonyl)amino)-6-chloro-2-methoxypyridin-3-yl)propanoate (7)***

A solution of the mixture of ethyl (E)-3-(4-((tert-butoxycarbonyl)amino)-6-chloro-2-methoxypyridin-3-yl)acrylate **6** and ethyl (E)-3-(4-(bis(tert-butoxycarbonyl)amino)-6-chloro-2-methoxypyridin-3-yl)acrylate **6a** (410 mg, 0.9 mmol), and $PtO_2$ (50 mg) in EtOAc (10 mL) was stirred at rt for 1 h under $H_2$ atmosphere. The resulting solution was filtered through diatomaceous earth and the filter cake was washed with DCM (20 mL). The filtrate was concentrated under reduced pressure to give a mixture of ethyl 3-(4-((tert-butoxycarbonyl)amino)-6-chloro-2-methoxypyridin-3-yl)propanoate **7** and ethyl 3-(4-(bis(tert-butoxycarbonyl)amino)-6-chloro-2-methoxypyridin-3-yl)propanoate **7a** (210 mg, 90% purity, 45% yield) as a colorless oil.

LCMS (ESI) calcd for $C_{16}H_{23}ClN_2O$ [M + H] $^+$ m/z 359.13, found 359.08.

***Preparation of 7-chloro-5-methoxy-3,4-dihydro-1,6-naphthyridin-2(1H)-one (8)***

A solution of the mixture of ethyl 3-(4-((tert-butoxycarbonyl)amino)-6-chloro-2-methoxypyridin-3-yl)propanoate **7** and ethyl 3-(4-(bis(tert-butoxycarbonyl)amino)-6-chloro-2-methoxypyridin-3-yl)propanoate **7a** (210 mg, 0.6 mmol) in HCl-dioxane (10 mL, 4 M) was stirred at rt for 2 h. The reaction mixture was concentrated under reduced pressure to give 7-chloro-5-methoxy-3,4-dihydro-1,6-naphlhyridin-2(1H)-one **8** (110 mg, 90% purity, 79% yield) as a white solid.

LCMS (ESI) calcd for $C_9H_9ClN_2o_2$ [M + H] $^+$ m/z 213.04, found 212.92.

***Preparation of tert-butyl 3-(5-methoxy-2-oxo-1,2,3,4-tetrahydro-1,6-naphthyridin-7-yl)-2,5-dihydro-1H-pyrrole-1-carboxylate (10)***

To a solution of 7-chloro-5-methoxy-3,4-dihydro-1,6-naphthyridin-2(1H)-one **8** (110 mg, 0.5 mmol) in dioxane/-$H_2O$ (10 mL, 10:1) was added tert-butyl 3-(4,4,5,5-tetramethyl-1,3,2-dioxaborolan-2-yl)-2,5-dihydro-1H-pyrrole-1-carboxylate **9** (184 mg, 0.6 mmol), Pd(dppf)$Cl_2$ (42 mg, 0.06 mmol) and $K_3PO_4$· $H_2O$ (357 mg, 1.6 mmol) successively at rt. The reaction mixture was stirred at 100 °C for 6 h under $N_2$ atmosphere. The reaction solution was cooled to rt and concentrated under reduced pressure. The residue was purified by flash column chromatography (eluting with PE/EtOAc = 100 : 0 to 20 : 80) to afford tert-butyl 3-(5-methoxy-2-oxo-1,2,3,4-tetrahydro-1,6-naphthyridin-7-yl)-2,5-dihydro-1H-pyrrole-1-carboxylate **10** (150 mg, 90% purity, 75% yield) as a white solid.

LCMS (ESI) calcd for $C_{18}H_23N_3O_4$ [M + H] $^+$ m/z 346.17, found 346.05.

***Preparation of tert-butyl 3-(5-methoxy-2-oxo-1,2,3,4-tetrahydro-1,6-naphthyridin-7-yl)pyrrolidine-1-carboxylate (11)***

A solution of tert-butyl 3-(5-methoxy-2-oxo-1,2,3,4-tetrahydro-1,6-naphthyridin-7-yl)-2,5-dihydro-1H-pyrrole-1-carboxylate **10** (150 mg, 0.4 mmol) and Pd/C (20 mg) in MeOH (10 mL) was stirred at rt for 2 h under $H_2$ atmosphere. The resulting solution was filtered through diatomaceous earth and the filter cake was washed with DCM (20 mL × 3). The filtrate was concentrated under reduced pressure to give tert-butyl 3-(5-methoxy-2-oxo-1,2,3,4-tetrahydro-1,6-naphthyridin-7-yl)pyrrolidine-1-carboxylate **11** (140 mg, 90% purity, 83% yield) as a colorless oil.

LCMS (ESI) calcd for $C_{18}H_{25}N_3O_4$ [M + H] $^+$ m/z 348.18, found 347.95.

***Preparation of 7-(pyrrolidin-3-yl)-4,6-dihydro-1,6-naphthyridine-2,5(1H,3H)-dione (12)***

A solution of tert-butyl 3-(5-methoxy-2-oxo-1,2,3,4-tetrahydro-1,6-naphthyridin-7-yl)pyrrolidine-1-carboxylate **11** (120 mg, 0.34 mmol) in HBr (48% in $H_2O$, 5 mL) was stirred at 110 °C for 2 h. The reaction solution was cooled to rt and concentrated under reduced pressure. The residue was dissolved in MeOH (5 mL) and $Et_3N$ (1 mL) was added dropwise. The reaction mixture was stirred at rt for 5 min. The reaction solution was concentrated under reduced pressure to give 7-(pyrrolidin-3-yl)-4,6-dihydro-1,6-naphthyridine-2,5(1H,3H)-dione **12** (65 mg, 90% purity, 72% yield) as a brown solid.
LCMS (ESI) calcd for $C_{12}H_{15}N_3O_2$ [M + H] $^+$ m/z 234.12, found 234.06.

***Preparation of 5-(4-(3-(2,5-dioxo-1,2,3,4,5,6-hexahydro-1,6-naphthyridin-7-yl)pyrrolidin-1-yl)piperidin-1-yl)-N-methylpicolinamide (racemic mixture of compounds 309a and 309b)***

To a solution of 7-(pyrrolidin-3-yl)-4,6-dihydro-1,6-naphthyridine-2,5(1H,3H)-dione **12** (65 mg, 0.3 mmol) in MeOH (10 mL) was added N-methyl-5-(4-oxopiperidin-1-yl)picolinamide **INT** (78 mg, 0.3 mmol), AcOH (2 drops) and $NaBH_3CN$ (35 mg, 0.6 mmol) and at rt. The reaction mixture was stirred at 50 ° C for 1 h. The reaction mixture was quenched with water (2 mL) and the reaction solution was concentrated under reduced pressure. The residue was purified by flash column chromatography (eluting with DCM/MeOH = 100 : 0 to 90 : 10) to afford 5-(4-(3-(2,5-dioxo-1,2,3,4,5,6-hexahydro-1,6-naphthyridin-7-yl)pyrrolidin-1-yl)piperidin-1-yl)-N-methylpicolinamide a racemic mixture of compounds **309a** and **309b** (35 mg, 98% purity, 27% yield )as a white solid.

***Chiral resolution of 5-(4-(3-(2,5-dioxo-1,2,3,4,5,6-hexahydro-1,6-naphthyridin-7-yl)pyrrolidin-1-yl)piperidin-1-yl)-N-methylpicolinamide (racemic mixture of compounds 309a and 309b)***

A racemic mixture of compounds 309a and 309b was separated by SFC (Column: Daicel Chiralpak IH SFC; 20 mm I.D. × 250 mm, 5 μmm; Mobile phase: $CO_2$/MeOH [0.1% $NH_3$ (7 M Solution in MeOH)]=60/40) and concentrated under reduced pressure to afford the first fraction as **309a** (4.8 mg, 95% purity, 100% ee, white solid) and the second fraction as **309b** (8.7 mg, 95% purity, 100% ee, white solid).

**Compound 309a**

$^1H$ NMR (400 MHz, DMSO-$d_6$, ppm) δ: 11.08 (s, 1 H), 10.04 (s, 1 H), 8.43-8.34 (m, 1 H), 8.27 (d, *J* = 2.8 Hz, 1 H), 7.81 (d, *J* = 8.8 Hz, 1 H), 7.48-7.36 (m, 1 H), 5.83 (s, 1 H), 3.89-3.76 (m, 2 H), 3.14-3.07 (m, 1 H), 3.01-2.91 (m, 2 H), 2.84-2.72 (m, 5 H), 2.60-2.56 (m, 3 H), 2.43-2.41 (m, 3 H), 2.24-2.10 (m, 2 H), 1.96-1.88 (m, 2 H), 1.72-1.62 (m, 1 H), 1.55-1.46 (m, 2 H).
LCMS (ESI) calcd for $C_{24}H_3ON_6O_3$ [M + H]$^+$ m/z 451.24, found 451.40.

**Compound 309b**

$^1H$ NMR (400 MHz, DMSO-$d_6$, ppm) δ: 11.07 (s, 1 H), 10.04 (s, 1 H), 8.46-8.32 (m, 1 H), 8.27 (d, *J* = 2.8 Hz, 1 H), 7.81 (d, *J* = 8.8 Hz, 1 H), 7.40 (dd, *J* = 8.8, 2.8 Hz, 1 H), 5.83 (s, 1 H), 3.87-3.76 (m, 2 H), 3.14-3.04 (m, 1 H), 3.02-2.91 (m, 2 H), 2.84-2.75 (m, 5 H), 2.58-2.54 (m, 3 H), 2.44-2.37 (m, 3 H), 2.30-2.24 (m, 1 H), 2.23-2.12 (m, 1 H), 1.97-1.90 (m, 2 H), 1.75-1.63 (m, 1 H), 1.59-1.44 (m, 2 H).
LCMS (ESI) calcd for $C_{24}H_3ON_6O_3$ [M + H] $^+$ m/z 451.24, found 451.20.

***Example 24 = synthesis of compound 324rac***

**Synthesis of compound 324rac**

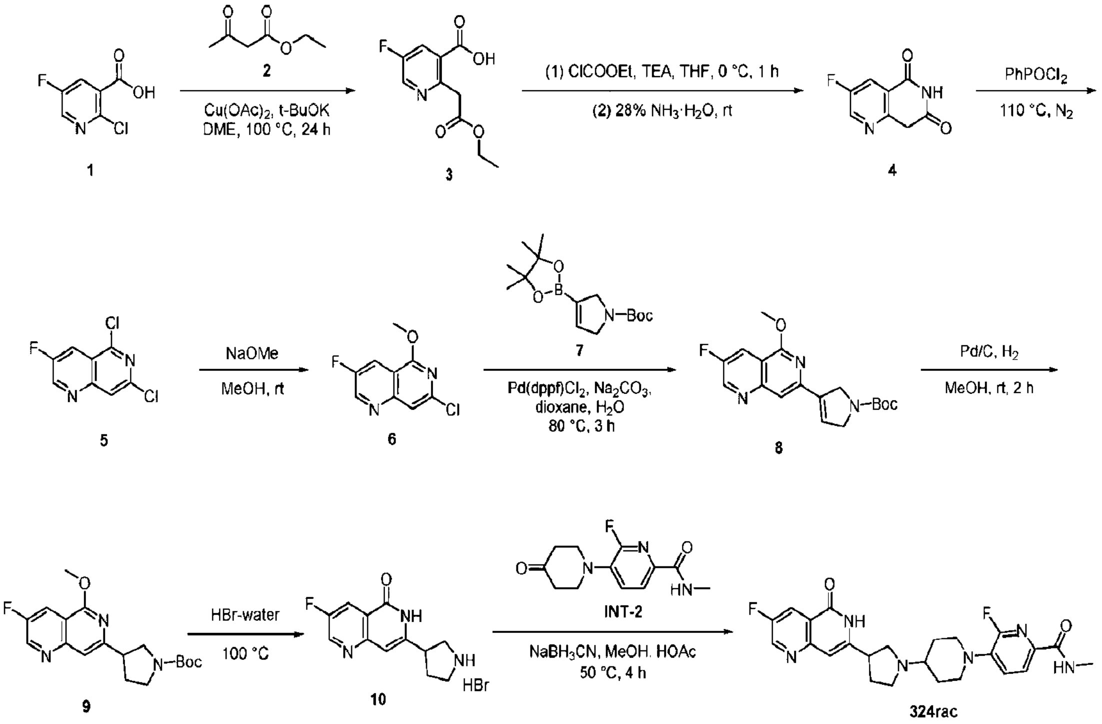


***Preparation of 2-(2-ethoxy-2-oxoethyl)-5-fluoropyridine-3-carboxylic acid (3)***

To a solution of ethyl 3-oxobutanoate 2 (4.57 g, 0.0351 mol) in DME (250 mL) was added t-BuOK (5.25 g, 0.0468 mol). The mixture was stirred at room temperature for 1 hour. $Cu(OAc)_2$ (1.70 g, 0.00936 mol) and 2-chloro-5-fluoropyridine-3-carboxylic acid 1 (4.1 g, 0.0234 mol) were added and the resulting mixture was stirred at 100 °C for 24 hours under nitrogen atmosphere. The mixture was diluted with water, acidified with 1 M HCl to pH = 2 and extracted with ethyl acetate. The combined organic layers were dried over sodium sulfate, filtered, and concentrated under reduced pressure. The residue was purified by column chromatography on silica (DCM/MeOH =100: 0 to 90: 10) to afford 2-(2-ethoxy-2-oxoethyl)-5-fluoropyridine-3-carboxylic acid 3 (2.4 g, 90% purity, 40% yield) as a white solid.

LCMS (ESI) calcd for $C_{10}H_{10}FNO_4$ [M + H] $^+$ m/z 228.06, found 228.00.

***Preparation of 3-fluoro-6,8-dihydro-1,6-naphthyridine-5,7-dione (4)***

To a solution of 2-(2-ethoxy-2-oxoethyl)-5-fluoropyridine-3-carboxylic acid **3** (2400 mg, 10.56 mmol) and TEA (2137 mg, 21.12 mmol) in THF (40 mL) was added ethyl carbonochloridate (2281 mg, 21.12 mmol) dropwise at 0 °C under $N_2$ atmosphere. The mixture was stirred at this temperature for 1 hour before $NH_3{\cdot}H_2O$ (28%, 1285 mg) was added dropwise and the resulting mixture was stirred at room temperature for 1 hour. The mixture was diluted with water and extracted with ethyl acetate. The combined organic layers were washed with brine, dried over sodium sulfate, filtered and concentrated. The residue was purified by Biotage Isolera One ($C_{18}$ column, eluting with 5% to 90% $MeCN/H_2O$ containing 0.1% FA) to afford 3-fluoro-6,8-dihydro-1,6-naphthyridine-5,7-dione **4** (505 mg, 90% purity, 23% yield) as a white solid.

LCMS (ESI) calcd for $C_8H_5FN_2O_2$ [M + H] $^+$ m/z 181.03, found 180.90.

***Preparation of 5,7-dichloro-3-fluoro-1,6-naphthyridine (5)***

A solution of 3-fluoro-6,8-dihydro-1,6-naphthyridine-5,7-dione **4** (300 mg, 1.66 mmol) in $PhPOCl_2$ (3 mL) was stirred at 110 °C for 12 hours under nitrogen atmosphere. The mixture was cooled and adjusted pH to 9 ~ 10 with aqueous KOH (5% wt) and diluted with ethyl acetate. The mixture was washed with brine, dried over anhydrous sodium sulfate, filtered, and concentrated under reduced pressure. The residue was purified by silica gel column chromatography (PE/EtOAc = 100 : 0 to 50 : 50) to afford 5,7-dichloro-3-fluoro-1,6-naphthyridine **5** (125 mg, 90% purity, 31% yield) as a white solid.

LCMS (ESI) calcd for $CsH_3C_2FN_2$ [M + H] $^+$ m/z 216.97, found 216.90.

***Preparation of 7-chloro-3-fluoro-5-methoxy-1,6-naphthyridine (6)***

To a solution of 5,7-dichloro-3-fluoro-1,6-naphthyridine **5** (195 mg, 0.8985 mmol) in MeOH (20 mL) was added MeONa (48 mg, 0.8985 mmol), then stirred for 2 h at room temperature. The reaction mixture was separated with EtOAc (20 mL × 3) and brine. The combined organic phase was concentrated under reduced pressure. The residue was purified by flash chromatography (eluting with PE/EtOAc = 100 : 0 to 95 : 5) to give 7-chloro-3-fluoro-5-methoxy-1,6-naphthyridine **6** (135 mg, 90% purity, 63% yield) as white solid.

LCMS (ESI) calcd for $C_9H_6ClFN_2O$ [M + H] $^+$ m/z 213.02, found 212.90.

***Preparation of tert-butyl 3-(3-fluoro-5-methoxy-1,6-naphthyridin-7-yl)-2,5-dihydro-1H-pyrrole-1-carboxylate (8)***

A mixture of 7-chloro-3-fluoro-5-methoxy-1,6-naphthyridine **6** (100 mg, 0.470 mmol), tert-butyl 3-(4,4,5,5-tetramethyl-1,3,2-dioxaborolan-2-yl)-2,5-dihydro- 1H-pyrrole-1-carboxylate **7** (167 mg, 0.564 mmol), Pd(dppf)$Cl_2$ (38 mg, 0.047 mmol) and $Na_2CO_3$ (306 mg, 0.941 mmol) in dioxane and $H_2O$ (3:1, 2 mL) was degassed with $N_2$ and heated at 80 °C for 3 h under $N_2$ atmosphere. The reaction mixture was concentrated and purified by flash silica chromatography to obtain tert-butyl 3-(3-fluoro-5-methoxy-1,6-naphthyridin-7-yl)-2,5-dihydro-1H-pyrrole-1-carboxylate 8 (100 mg, 90% purity, 24% yield) as a colorless oil.

LCMS (ESI) calcd for $C_{18}H_{20}FN_3O_3$ [M + H] $^+$ m/z 346.15, found 346.15.

***Preparation of tert-butyl 3-(3-fluoro-5-methoxy-1,6-naphthyridin-7-yl)pyrrolidine-1-carboxylate (9)***

A mixture of 3-(3-fluoro-5-methoxy-1,6-naphthyridin-7-yl)-2,5-dihydro-1H-pyrrole-1-carboxylate **8** (100 mg, 0.289 mmol) and Pd/C (31 mg) in MeOH (2 mL) was degassed with $H_2$ and stirred at rt for 2 h under $H_2$ atmosphere. The reaction mixture was filtered. The filtrate was concentrated under reduced pressure to obtain tert-butyl 3-(3-fluoro-5-methoxy-1,6-naphthyridin-7-yl)pyrrolidine-1-carboxylate **9** (100 mg, 60% purity, 59% yield) as a yellow oil.

LCMS (ESI) calcd for $C_{18}H_{22}FN_3O_3$ [M + H] $^+$ m/z 348.16, found 348.05.

***Preparation of 3-fluoro-7-(pyrrolidin-3-yl)-1,6-naphthyridin-5(6H)-one hydrobromide (10)***

A solution of tert-butyl 3-(3-fluoro-5-methoxy-1,6-naphthyridin-7-yl)pyrrolidine-1-carboxylate **9** (100 mg, 0.287 mmol) in HBr-water (48%, 10 mL) was heated at 100°C for 2 h. The mixture was concentrated under reduced pressure to obtain 3-fluoro-7-(pyrrolidin-3-yl)-1,6-naphthyridin-5(6H)-one hydrobromide **10** (80 mg, 90% purity, 79% yield) as a white solid. LCMS (ESI) calcd for $C_{12}H_{12}FN_3O$ [M + H] $^+$ m/z 234.10, found 233.95.

***Preparation of 6-fluoro-5-(4-(3-(3-fluoro-5-oxo-5,6-dihydro-1,6-naphthyridin-7-yl)pyrrolidin-1-yl)piperidin-1-yl)-N-methylpicolinamide (compound 324rac)***

To a solution of 3-fluoro-7-(pyrrolidin-3-yl)-6H-1,6-naphthyridin-5-one hydrobromide **10** (40 mg, 0.127 mmol) in MeOH (10 mL) was added 6-fluoro-N-methyl-5-(4-oxopiperidin-1-yl)pyridine-2-carboxamide **INT-2** ( 32 mg, 0.127 mmol), then two drops of acetic acid and $NaBH_3CN$ (40 mg, 0.636 mmol) were added at room temperature. The reaction mixture stirred at 50 °C for 4 h. After cooling to room temperature, the reaction mixture was concentrated under reduced pressure. The residue was purified by flash chromatography to give 6-fluoro-5-(4-(3-(3-fluoro-5-oxo-5,6-dihydro-1,6-naphthyridin-7-yl)pyrrolidin-1-yl)piperidin-1-yl)-N-methylpicolinamide racemic **324rac** (13.2 mg, 95% purity, 21% yield ) as a white solid.

$^1$H NMR (400 MHz, DMSO-$d_6$ ppm) δ 11.56 (s, 1 H), 8.92 (d, *J* = 2.8 Hz, 1 H), 8.42-8.35 (m, 1 H), 8.23-8.17 (m, 1 H), 8.16 (s, 1 H), 7.84 (d, *J* = 8.0 Hz, 1 H), 7.62-7.54 (m, 1 H), 6.61 (s, 1 H), 3.54-3.49 (m, 2 H), 3.31-3.27 (m, 1 H), 2.97-2.84 (m, 4 H), 2.83-2.79 (m, 1 H), 2.77 (d, J = 4.8 Hz, 3 H), 2.70-2.64 (m, 1 H), 2.38-2.31 (m, 1 H), 2.30-2.23 (m, 1 H), 1.99 (d, *J* = 12.4 Hz, 2 H), 1.92-1.81 (m, 1 H), 1.67-1.55 (m, 2 H).

LCMS (ESI) calcd for $C_{24}H_{26}F_2N_6O_2$ [M + H] $^+$ m/z 469.21, found 469.10.

***<u>Example 25 - Synthesis of compounds 337a and 337b</u>***

**Synthesis of compounds 337a and 337b**

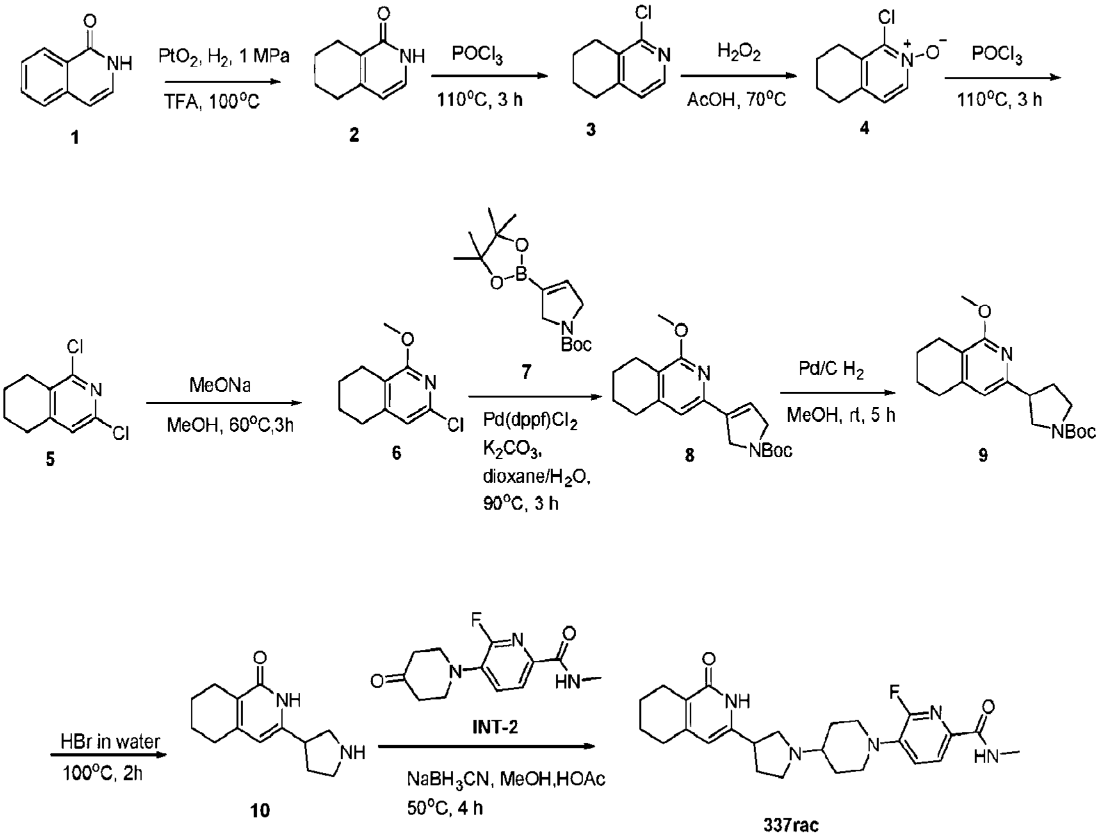


***Preparation of 5,6,7,8-tetrahydroisoquinolin-1(2H)-one (2)***

2H-isoquinolin-1-one (8 g, 0.05 mol) and $PtO_2$ (0.9 g, 0.003 mol) were added to TFA (80 mL), stirred under $H_2$ (1Mpa) at 100 °C for 12h. The precipitate was collected by filtration, extracted with EtOAc (400 mL x 3). The combine organic phases were washed with brine, dried over sodium sulfate, concentrated, and purified by silica gel column chromatography (eluent: PE/EtOAC =100: 0 to 80 :20) to give 5,6,7,8-tetrahydroisoquinolin-1(2H)-one **2** (7.00 g, 77% yield) as a black oil.
LCMS (ESI) calcd for $C_9H_{11}NO$ [M + H] $^+$ m/z 150.08, found 150.00

***Preparation of 1-chloro-5,6,7,8-tetrahydroisoquinoline (3)***

A solution of sodium 5,6,7,8-tetrahydro-2H-isoquinolin-1-one **2** (7 g, 0.047 mmol) in $POCl_3$ (100 mL) was stirred at 110 °C for 3 h. After completion of the reaction, the mixture was cooled to room temperature, taken into ice-water, neutralized with $NH_3.H_2O$ under cooling, extracted with EtOAc (300 mLx3). The combined organic layer was washed with brine, dried over anhydrous $Na_2SO_4$ and concentrated. The reaction mixture was concentrated under reduced pressure. The residue was purified by flash column chromatography (eluting with PE/EtOAc = 100 : 0 to 80 : 20) to afford 1-chloro-5,6,7,8-tetrahydroisoquinoline **3** (5 g, 90% purity, 57% yield) as a colorless oil.

LCMS (ESI) calcd for $C_9H_{10}ClN$ [M + H] $^+$ m/z 168.05, found 168.00.

***Preparation of 1-chloro-5,6,7,8-tetrahydroisoquinoline 2-oxide (4)***

1-chloro-5,6,7,8-tetrahydroisoquinoline **3** (5 g, 0.029 mol) was added to $AcOH/H_2O_2$ (100 mL). The reaction mixture was stirred at 70 °C for 2 h. The precipitate was collected by filtration, extracted with EtOAc (200 mL x 3). The combine organic phases were washed with brine, dried over sodium sulfate, concentrated, and purified by silica gel column chromatography (eluent: DCM/MeOH =100: 0 to 90 :10) to give 1-chloro-5,6,7,8-tetrahydroisoquinoline 2-oxide **4** (2.00 g, 73% yield) as a yellow oil.

LCMS (ESI) calcd for $C_9H_{10}ClNO$ [M + H] $^+$ m/z 184.05, found 184.00.

***Preparation of 1,3-dichloro-5,6,7,8-tetrahydroisoquinoline (5)***

1-chloro-5,6,7,8-tetrahydroisoquinoline 2-oxide **4** (2.00 g, 0.019 mmol) was added to $POCl_3$ (50 mL) slowly at room temperature. The mixture was heated to 110 °C stirred for 3 h. The resulting mixture was diluted with ice water (200 mL) and extracted with EtOAc (400 mL x 3). The combined organic phases were washed with brine, dried over sodium sulfate, concentrated, and purified by silica gel column chromatography (eluent: PE/EtOAc =100: 0 to 70 :30) to give 1,3-dichloro-5,6,7,8-tetrahydroisoquinoline **5** (370 mg, 15% yield) as a yellow oil.

LCMS (ESI) calcd for $C_9H_9Cl_2N$ [M + H] $^+$ m/z 202.01, found 201.95.

***Preparation of 3-chloro-1-methoxy-5,6,7,8-tetrahydroisoquinoline (6)***

To a solution of 1,3-dichloro-5,6,7,8-tetrahydroisoquinoline **5** (350 mg, 1.73 mmol) in MeOH (15 mL) was added MeONa (280 mg, 5.20 mmol), the mixture was heated to 60 °C with stirring for 3h. The precipitate was collected by filtration, extracted with EtOAc (100 mL x 3). The combined organic phases were washed with brine, dried over sodium sulfate, concentrated, and purified by silica gel column chromatography (eluent: PE/EtOAC =100: 0 to 90 :10) to afford 3-chloro-1-methoxy-5,6,7,8-tetrahydroisoquinoline **6** (250 mg, 66% yield) as a white solid.

LCMS (ESI) calcd for $C_{10}H_{12}ClNO$ [M + H] $^+$ m/z 198.06, found 197.95.

***Preparation of tert-butyl 3-(1-methoxy-5,6,7,8-tetrahydroisoquinolin-3-yl)-2,5-dihydro-1H-pyrrole-1-carboxylate (8)***

To a solution of 3-chloro-1-methoxy-5,6,7,8-tetrahydroisoquinoline **6** (240 mg, 1.21 mmol) in dioxane/$H_2O$=10:1 (60 mL) were added tert-butyl 3-(4,4,5,5-tetramethyl-1,3,2-dioxaborolan-2-yl)-2,5-dihydro-1 H-pyrrole-1-carboxylate 7 (394 mg, 1.33 mmol), Pd(dppf)$Cl_2$ (88 mg, 0.12 mmol) and $Na_2CO_3$ (503 mg, 3.64 mmol) successively at room temperature. The reaction mixture was stirred at 90 °C for 3 h under $N_2$. The mixture was diluted with water (50 mL) and extracted with EtOAc (100 mL × 3). The combined organic layer was washed with brine (100 mL × 2), dried over $Na_2SO_4$, filtered and concentrated in vacuo to obtain the crude product, which was purified by flash column chromatography (PE/EtOAc = 100: 0 to 85: 15) to afford tert-butyl 3-(1-methoxy-5,6,7,8-tetrahydroisoquinolin-3-yl)-2,5-dihydro-1H-pyrrole-1-carboxylate **8** (140 mg, 31% yield) as a yellow solid.

LCMS (ESI) calcd for $C_{19}H_{26}N_2O_3$ [M + H] $^+$ m/z 331.19, found 331.15.

***Preparation of tert-butyl 3-(1-methoxy-5,6,7,8-tetrahydroisoquinolin-3-yl)pyrrolidine-1-carboxylate (9)***

To a solution of tert-butyl 3-(1-methoxy-5,6,7,8-tetrahydroisoquinolin-3-yl)-2,5-dihydro-1H-pyrrole-1-carboxylate **8** (130 mg, 0.39 mmol) in MeOH (50 mL) was added Pd/C (30 mg). The mixture was evacuated and backfilled with hydrogen three times and then charged with hydrogen. The resulting mixture was stirred at room temperature for 5 h. Then the mixture was filtered through celite and concentrated under vacuum to give crude product, which was purified by flash column chromatography (PE/EtOAc = 100: 0 to 50: 50) to afford tert-butyl 3-(1-methoxy-5,6,7,8-tetrahydroisoquinolin-3-yl)pyrrolidine-1-carboxylate **9** (130 mg, 89% yield) as a yellow solid.

LCMS (ESI) calcd for $C_{19}H_{28}N_2O_3$ [M + H] $^+$ m/z 333.21, found 333.10.

***Preparation of 3-(pyrrolidin-3-yl)-5,6,7,8-tetrahydroisoquinolin-1(2H)-one (10)***

Tert-butyl 3-(1-methoxy-5,6,7,8-tetrahydroisoquinolin-3-yl)pyrrolidine-1-carboxylate 9 (115 mg, 0.71 mmol) was added to HBr in water (10 mL). The mixture was stirred for 2 h at 100°C. The reaction mixture was concentrated under reduced pressure to give product of 3-(pyrrolidin-3-yl)-5,6,7,8-tetrahydroisoquinolin-1(2H)-one **10** (80 mg, 95% yield) as a white solid.

LCMS (ESI) calcd for $C_{13}H_{18}N_2O$ [M + H] $^+$ m/z 219.14, found 219.05.

***Preparation of 6-fluoro-N-methyl-5-(4-(3-(1-oxo-1,2,5,6,7,8-hexahydroisoquinolin-3-ylpyrrolidin-1-yl)piperidin-1-yl)picolinamide (racemic mixture of compounds 337a and 337b)***

To a solution of 3-(pyrrolidin-3-yl)-5,6,7,8-tetrahydroisoquinolin-1(2H)-one **10** (60 mg, 0.27 mmol), 6-fluoro-N-methyl-5-(4-oxopiperidin-1-yl)pyridine-2-carboxamide **INT-2** (69 mg, 0.27 mmol) and AcOH (two drops) in MeOH (5 mL) was added sodium triacetoxyborohydride (146 mg, 0.69 mmol) and then stirred at 50 °C for 1 h, then $NaBH_3CN$ (21 mg, 0.33 mmol) was added and stirred for 4 h. After cooling to room temperature, the reaction mixture was concentrated under reduced pressure. The residue was purified by flash chromatography (eluting with DCM/MeOH = 100: 0 to 90: 10) to give 6-fluoro-N-methyl-5-(4-(3-(1-oxo-1,2,5,6,7,8-hexahydroisoquinolin-3-yl)pyrrolidin-1-yl)piperidin-1-yl)picolinamide, a racemic mixture of compounds **337a** and **337b** (22 mg, 18 % yield ) as a white solid.

LCMS (ESI) calcd for $C_{25}H_{32}FN_5O_2$ [M + H] $^+$ m/z 454.25, found 454.15.

***Chiral resolution of 6-fluoro-N-methyl-5-(4-(3-(1-oxo-1,2,5,6,7,8-hexahydroisoquinolin-3-yl)pyrrolidin-1-yl)piperidin-1-yl)picolinamide (racemic mixture of compounds 337a and 337b)***

A racemic mixture of compounds **337a** and **337b** was separated by SFC (Column: DAICEL OD-H 4.6mmI.D.*250mmL 5 μ m; Mobile phase: CO2/MeOH [0.1%NH3(7M Solution in MeOH)]=70/30) and concentrated under reduced pressure to afford the first fraction as **337a** (7 mg, 95% purity, ee%: 100, white solid) and the second fraction as **337b** (5 mg, 92% purity, ee%: 100, white solid).

**Compound 337a**

$^1$H NMR (400 MHz, DMSO) δ 11.03 (s, 1H), 8.40 (d, *J* = 4.8 Hz, 1H), 7.83 (d, *J* = 7.8 Hz, 1H), 7.57 (dd, *J* = 10.6, 8.4 Hz, 1H), 5.83 (s, 1H), 3.55-3.44 (m, 2H), 3.14-3.04 (m, 1H), 2.86 (t, *J* = 11.6 Hz, 3H), 2.75 (t, *J* = 6.4 Hz, 4H), 2.63 (dd, *J* = 14.8, 8.0 Hz, 2H), 2.44 (s, 2H), 2.27 (s, 3H), 2.21-2.10 (m, 1H), 1.95 (d, *J* = 13.2 Hz, 2H), 1.73 (dd, *J* = 12.8, 8.4 Hz, 1H), 1.58 (dd, *J* = 22.4, 12.0 Hz, 6H).

LCMS (ESI) calcd for $C_{25}H_{32}FN_5O_2$ [M + H] $^+$ m/z 454.25, found 454.20.

**Compound 337b**

$^1$H NMR (400 MHz, DMSO) δ 11.03 (s, 1H), 8.40 (d, *J* = 4.4 Hz, 1H), 7.83 (d, *J* = 8.0 Hz, 1H), 7.70-7.45 (m, 1H), 5.83 (s, 1H), 3.49 (d, *J* = 11.2 Hz, 2H), 3.08 (d, *J* = 6.4 Hz, 1H), 2.85 (t, *J* = 11.2 Hz, 3H), 2.76 (d, *J* = 4.4 Hz, 4H), 2.69-2.57 (m, 2H), 2.44 (s, 2H), 2.27 (s, 3H), 2.20-2.10 (m, 1H), 1.95 (d, *J* = 12.4 Hz, 2H), 1.80-1.68 (m, 1H), 1.68-1.51 (m, 6H).

LCMS (ESI) calcd for $C_{25}H_{32}FN_5O_2$ [M + H] $^+$ m/z 454.25, found 454.15.

***Example 26 - Synthesis of compounds 343a and 343b***

**Synthesis *of* compounds 343a and 343b**

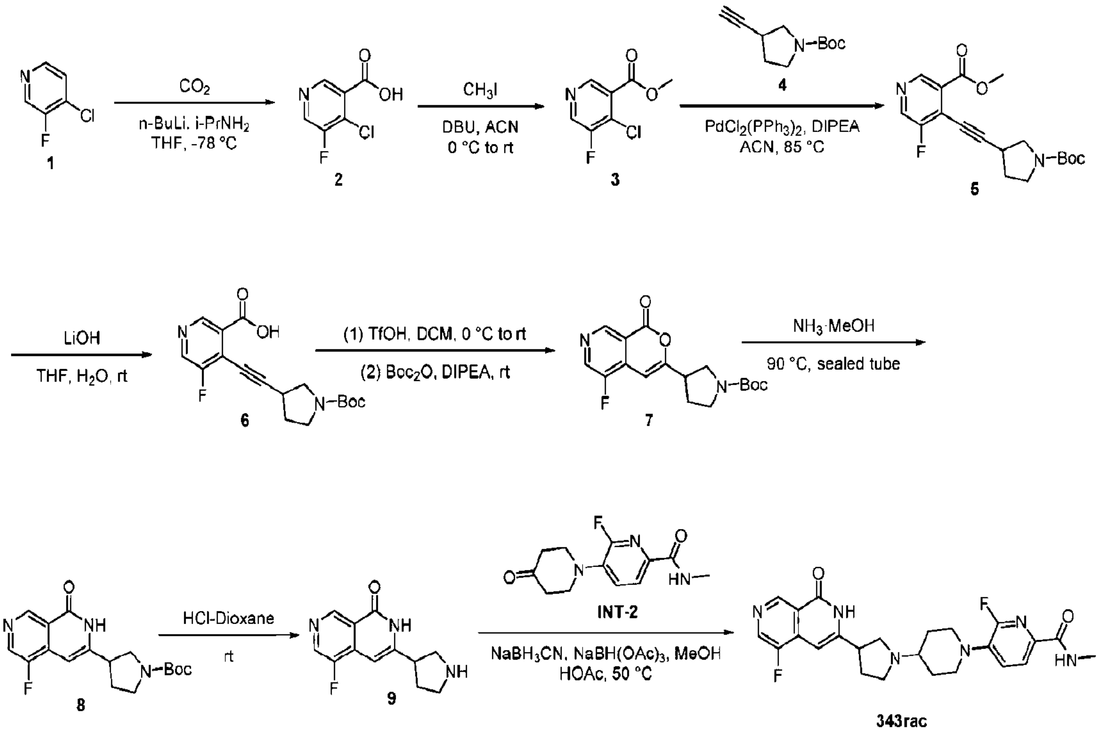

***Preparation of 4-chloro-5-fluoronicotinic acid (2)***

N-BuLi (2.4 M in hexanes, 10 mL, 23.9 mmol) was added to THF (100 mL) and cooled to -78 °C. To this solution was added i-$PrNH_2$ (2.54 g, 25.0 mmol) slowly followed by 4-chloro-3-fluoropyridine **1** (3 g, 22.8 mmol). After stirring for 2 h at -78 °C, crushed dry ice was added in one portion. The reaction mixture was kept stirring until the reaction mixture warmed to room temperature. The reaction was quenched by addition of aqueous ammonium chloride solution and acidified to pH = 2 using conc. HCl and extracted with ethyl acetate (3 x 200 mL). The combined organic layers were washed with brine (10 mL), dried over $Na_2SO_4$ and concentrated under reduced pressure to afford 4-chloro-5-fluoronicotinic acid **2** (2.2 g, 70% purity, 36% yield) as a yellow solid.

LCMS (ESI) calcd for $C_6H_3ClFNO_2$ [M + H] $^+$ m/z 175.99, found 175.80.

***Preparation of methyl 4-chloro-5-fluoronicotinate (3)***

To a solution of 4-chloro-5-fluoronicotinic acid **2** (2.2 g, 11.6 mmol) in ACN (100 mL) were added DBU (5.29 g, 34.8 mmol) and $CH_3I$ (8.2 g, 58.0 mmol) dropwise at 0 °C. The mixture was stirred at rt for 5 hours. The resulting mixture was diluted with water (300 mL) and extracted with EtOAc (100 mL x 3). The combined organic phases were washed with brine, dried over sodium sulfate, concentrated, and purified by silica gel column chromatography (eluting with EtOAc/PE, 20% to 50%) to give methyl 4-chloro-5-fluoronicotinate **3** (1.5 g, 90% purity, 63% yield) as a white solid.

LCMS (ESI) calcd for $C_7H_5ClFNO_2$ [M + H] $^+$ m/z 190.00, found 189.95.

***Preparation of methyl 4-((1-(tert-butoxycarbonyl)pyrrolidin-3-yl)ethynyl)-5-fluoronicotinate (5)***

A mixture of methyl 4-chloro-5-fluoronicotinate **3** (150 mg, 0.79 mmol), tert-butyl 3-ethynylpyrrolidine-1-carboxylate **4** (233 mg, 1.19 mmol), $PdCl_2(PPh_3)_2$ (55 mg, 0.079 mmol) and DIPEA (511 mg, 3.96 mmol) in ACN (15 mL) was heated at 85 °C for 5 hours under an atmosphere of $N_2$. After cooling to ambient temperature, the mixture was filtered through celite, and the filtrate was concentrated under vacuum. The residue was diluted with water and extracted with EtOAc. The combined organic phases were washed with water and brine, dried over sodium sulfate, concentrated under reduced pressure, and purified by flash chromatography (eluting with EtOAc/PE, 20% to 40%) to obtain methyl 4-((1-(tert-butoxycarbonyl)pyrrolidin-3-yl)ethynyl)-5-fluoronicotinate **5** (150 mg, 90% purity, 48% yield) as a yellow solid.

LCMS (ESI) calcd for $C_{18}H_{21}FN_2O_4$ [M + H] $^+$ m/z 349.15, found 348.95.

***Preparation of 4-((1-(tert-butoxycarbonyl)pyrrolidin-3-yl)ethyttyl)-5-fluoronicotinic acid (6)***

To a solution of methyl 4-((1-(tert-butoxycarbonyl)pyrrolidin-3-yl)ethynyl)-5-fluoronicotinate **5** (150 mg, 0.43 mmol) in THF/$H_2O$ (3/1, 10 mL) was added LiOH (31 mg, 1.29 mmol). The mixture was stirred at room temperature for 2 h. Then the organic solvent was removed under reduced pressure. The aqueous solution was adjusted to pH 3-4 with 1 M HCl and extracted with DCM (50 mL × 3). The combined organic layer was washed with brine, dried over $Na_2SO_4$ and concentrated under reduced pressure to give 4-((1-(tert-butoxycarbonyl)pyrrolidin-3-yl)ethynyl)-5-fluoronicotinic acid **6** (120 mg, 90% purity, 75% yield) as a yellow oil.

LCMS (ESI) calcd for $C_{17}H_{19}FN_2O_4$ [M + H] $^+$ m/z 335.14, found 335.00.

***Preparation of tert-butyl 3-(5-fluoro-1-oxo-1H-pyrano[3,4-c]pyridin-3-yl)pyrrolidine-1-carboxylate (7)***

To a solution of 4-((1-(tert-butoxycarbonyl)pyrrolidin-3-yl)ethynyl)-5-fluoronicotinic acid **6** (120 mg, 0.36 mmol) in DCM (10 mL) was added TfOH (161 mg, 1.07 mmol) dropwise at 0 °C. The mixture was stirred at rt for 16 hours. The reaction was quenched with DIPEA (231 mg, 1.79 mmol), then $Boc_2O$ (156 mg, 0.716 mmol) was added. After stirring at rt for 1 h, the resulting mixture were concentrated under reduced pressure and the residue was purified by silica gel column chromatography (eluting with EtOAc/PE, 20% to 40%) to give tert-butyl 3-(5-fluoro-1-oxo-1H-pyrano[3,4-c]pyridin-3-yl)pyrrolidine-1-carboxylate **7** (70 mg, 90% purity, 52% yield) as a white solid.

LCMS (ESI) calcd for $C_{17}H_{19}FN_2O_4$ [M -t-Bu + H] $^+$ m/z 279.14, found 278.85.

***Preparation of tert-butyl 3-(5-fluoro-1-oxo-1,2-dihydro-2,7-naphthyridin-3-yl)pyrrolidine-1-carboxylate (8)***

A solution of tert-butyl 3-(5-fluoro-1-oxo-1H-pyrano[3,4-c]pyridin-3-yl)pyrrolidine-1-carboxylate **7** (70 mg, 0.21 mmol) in $NH_3$-MeOH (7 M, 5 mL) was stirred at 90 °C for 1 h in a sealed tube. Then the reaction was concentrated under reduced pressure to provide tert-butyl 3-(5-fluoro-1-oxo-1,2-dihydro-2,7-naphthyridin-3-yl)pyrrolidine-1-carboxylate **8** (70 mg, 90% purity, 90% yield) as a yellow solid.

LCMS (ESI) calcd for $C_{17}H_{20}FN_3O_3$ [M + H] $^+$ m/z 334.15, found 333.95.

***Preparation of 5-fluoro-3-(pyrrolidin-3-yl)-2,7-naphthyridin-1(2H)-one (9)***

A mixture of tert-butyl 3-(5-fluoro-1-oxo-1,2-dihydro-2,7-naphthyridin-3-yl)pyrrolidine-1-carboxylate **8** (70 mg, 0.44 mmol) in HCl dioxane solution (4 M, 5 mL) was stirred at room temperature for 1 hours. The mixture was concentrated under reduced pressure then quenched with $Et_3N$. The resulting solution was concentrated under vacuum to give 5-fluoro-3-(pyrrolidin-3-yl)-2,7-naphthyridin-1(2H)-one **9** (50 mg, 90% purity, 92% yield) as a yellow oil.

LCMS (ESI) calcd for $C_{12}H_{12}FN_3O$ [M + H] $^+$ m/z 234.10, found 234.05.

***Preparation of 6-fluoro-5-(4-(3-(5-fluoro-1-oxo-1,2-dihydro-2,7-naphthyridin-3-yl)pyrrolidin-1-yl)piperidin-1-yl)-N-methylpicolinamide (racemic mixture of compounds 343a and 343b)***

To a solution of 5-fluoro-3-(pyrrolidin-3-yl)-2,7-naphthyridin-1(2H)-one **9** (70 mg, 0.30 mmol) in MeOH (10 mL) was added 6-fluoro-N-methyl-5-(4-oxopiperidin-1-yl)picolinamide **INT-2** (113 mg, 0.45 mmol), then two drops of acetic acid, $NaBH_3CN$ (19 mg, 0.30 mmol) and $NaBH(OAc)_3$ (191 mg, 0.90 mmol) were added at room temperature. The reaction mixture stirred at 50 °C for 1 h. After cooling to room temperature, the reaction mixture was concentrated under reduced pressure. The residue was purified by flash chromatography (eluting with DCM/MeOH = 100 : 0 to 90 : 10) to give 6-fluoro-5-(4-(3-(5-fluoro-1-oxo-1,2-dihydro-2,7-naphthyridin-3-yl)pyrrolidin-1-yl)piperidin-1-yl)-N-methylpicolinamide, a racemic mixture of compounds **343a** and **343b** (50 mg, 95% purity, 33% yield) as a white solid.

***Chiral resolution of 6-fluoro-5-(4-(3-(5-fluoro-1-oxo-1,2-dihydro-2,7-naphthyridin-3-yl)pyrrolidin-1-yl)piperidin-1-yl)-N-methylpicolinamide (racemic mixture of compounds 343a and 343b)***

A racemic mixture of compounds 343a and 343b was separated by SFC (Column: Daicel Chiralpak IH 20 mm I.D. × 250 mm, 5 μm; Mobile phase: $CO_2$/MeOH [0.1% ($NH_3$)] = 70/30) and concentrated under reduced pressure to afford the first fraction as **343a** (19.7 mg, 99% purity, 100% ce, white solid) and the second fraction as **343b** (24.7 mg, 98% purity, 90% ec. white solid)

**Compound 343a**

$^1$H NMR (400 MHz, DMSO-$d_6$, ppm) δ: 11.74 (s, 1 H), 9.09 (s, 1 H), 8.68 (d, *J* = 2.0 Hz, 1 H), 8.40 (d, *J* = 4.8 Hz, 1 H), 7.87-7.80 (m, 1 H), 7.58 (dd, *J* = 10.6, 8.2 Hz, 1 H), 6.54 (s, 1 H), 3.58-3.47 (m, 2 H), 3.29-3.16 (m, 1 H), 2.91-2.83 (m, 5 H), 2.77 (d, *J* = 4.8 Hz, 3H), 2.72-2.58 (m, 1 H), 2.47-2.23 (m, 2 H), 2.10-1.96 (m, 2 H), 1.95-1.81 (m, 1 H), 1.77-1.50 (m, 2 H). LCMS (ESI) calcd for $C_{24}H_{26}F_2N_6O_2$ [M + H] $^+$ m/z 469.21, found 469.07.

**Compound 343a**

$^1$H NMR (400 MHz, DMSO-$d_6$, ppm) δ: 11.77 (s, 1 H), 9.09 (s, 1 H), 8.68 (d, *J* = 2.0 Hz, 1 H), 8.45-8.31 (m, 1 H), 7.89-7.76 (m, 1 H), 7.58 (dd, *J* = 10.6, 8.2 Hz, 1 H), 6.55 (s, 1 H), 3.54-3.46 (m, 2 H), 3.29-3.23 (m, 1 H), 2.94-2.82 (m, 5 H), 2.77 (d, *J* = 4.8 Hz, 3 H), 2.69-2.58 (m, 1 H), 2.36-2.25 (m, 2 H), 2.02-1.94 (m, 2 H), 1.91-1.78 (m, 1 H), 1.67-1.56 (m, 2 H).

LCMS (ESI) calcd for $C_{24}H_{26}F_2N_6O_2$ [M + H] $^+$ m/z 469.21, found 469.10.

***<u>Example 27 - Synthesis of compound 361</u>***

**Synthesis of compound 361**

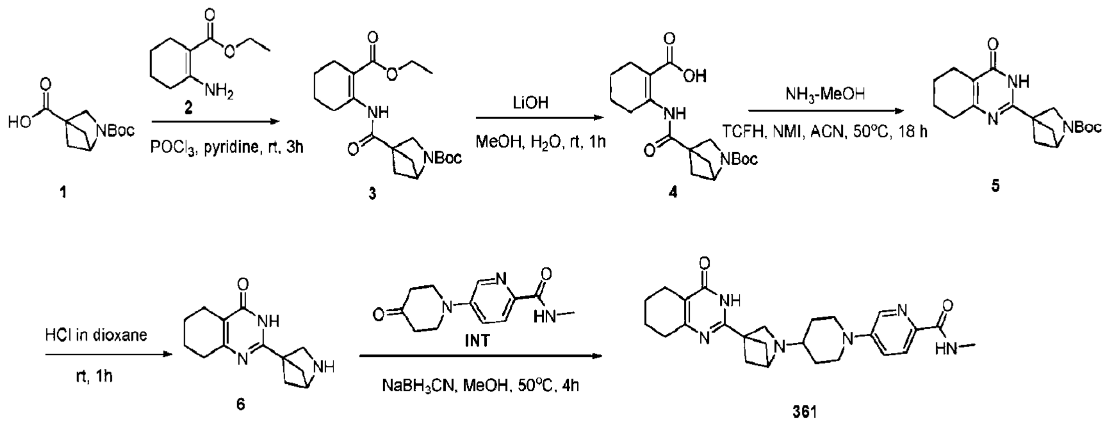


***Preparation of tert-butyl 4-((2-(ethoxycarbonyl)cyclohex-1-en-1-yl)carbamoyl)-2-azabicyclo[2.1.1]hexane-2-carboxylate (3)***

To a solution of 2-(tert-butoxycarbonyl)-2-azabicyclo[2.1.1]hexane-4-carboxylic acid **1** (110 mg, 0.48 mmol) in pyridine (10 mL) was added ethyl 2-aminocyclohex-1-ene-1-carboxylate **2** (160 mg, 0.95 mmol) at room temperature, then added 10 drops of $POCl_3$. The reaction mixture stirred at room temperature for 3 h. After cooling to room temperature, the reaction mixture was poured into water, The aqueous was extracted with organic solvent (50 mL) 3 times. The combined organic layers were washed with brine (50 mL) and dried over $Na_2SO_4$, then concentrated under reduced pressure. The residue was purified by flash chromatography (eluting with PE/EtOAc = 100: 0 to 85: 15) to give tert-butyl 4-((2-(ethoxycarbonyl)cyclohex-1-en-1-yl)carbamoyl)-2-azabicyclo[2.1.1]hexane-2-carboxylate 3 (100 mg, 55% yield ) as a white solid.

LCMS (ESI) calcd for C20H30N2O5 [M - 56 + H] $^+$ m/z 323.22, found 323.05.

***Preparation of 2-(2-(tert-butoxycarbonyl)-2-azabicyclo[2.1.1]hexane-4-carboxamido)cyclohex-1-ene-1-carboxylic acid (4)***

To a solution of tert-butyl 4-((2-(ethoxycarbonyl)cyclohex-1-en-1-yl)carbamoyl)-2-azabicyclo[2.1.1]hexane-2-carboxylate **3** (100 mg, 0.26 mmol) in MeOH : H2O=1 : 1 (10 mL) was added LiOH (30 mg, 1.25 mmol).The reaction mixture stirred at room temperature for 1 h. The reaction mixture was concentrated under reduced pressure to give 2-(2-(tert-butoxycarbonyl)-2-azabicyclo[2.1.1]hexane-4-carboxamido)cyclohex-1-ene-1-carboxylic acid 4 (80 mg, 88 % yield ) as a white solid.

LCMS (ESI) calcd for C18H26N2O5 [M - 56 + H] $^+$ m/z 295.18, found 295.05.

***Preparation of tert-butyl 4-(4-oxo-3,4,5,6,7,8-hexahydroquinazoliti-2-yl)-2-azabicyclo[2.1.1]hexane-2-carboxylate (5)***

To a solution of 2-(2-(tert-butoxycarbonyl)-2-azabicyclo[2.1.1]hexane-4-carboxamido)cyclohex-1-ene-1-carboxylic acid **4** (80 mg, 0.23 mmol) in ACN (10 mL) was added N,N,N',N'-tetramethylchloroformamidinium hexafluorophosphate / TCFH (150 mg, 0.54 mmol) and N-methyl imidazole / NMI (50 mg, 0.61 mmol) at room temperature. After 10 mins, excess of $NH_3$-MeOH (7 M, 5 mL) was added at 50 °C. The mixture was stirred at 50 °C for 18 h. The residue was concentrated under reduced pressure, then purified by flash chromatography (eluting with DCM/MeOH = 100: 0 to 96: 4) to give tert-butyl 4-(4-oxo-3,4,5,6,7,8-hexahydroquinazolin-2-yl)-2-azabicyclo[2.1.1]hexane-2-carboxylate 5 (60 mg, 79% yield ) as a white solid.

LCMS (ESI) calcd for C18H25N3O3 [M + H] $^+$ m/z 332.19, found 332.10.

***Preparation of 2-(2-azabicyclo[2.1.1]hexan-4-yl)-5,6,7,8-tetrahydroquinazolin-4(3H)-one (6)***

Tert-butyl 4-(4-oxo-3,4,5,6,7,8-hexahydroquinazolin-2-yl)-2-azabicyclo[2.1.1 ]hexane-2-carboxylate **5** (60 mg, 0.18 mmol) was added to HCl in dioxane (4 M, 3 mL), stirred for 1 h at room temperature, the reaction mixture was concentrated under reduced pressure to give 2-(2-azabicyclo[2.1.1]hexan-4-yl)-5,6,7,8-tetrahydroquinazolin-4(3H)-one **6** (40 mg, 96 % yield ) as a white solid.

LCMS (ESI) calcd for C13H17N3O [M + H] $^+$ m/z 232.14, found 232.05.

***Preparation of N-methyl-5-(4-(4-(4-oxo-3,4,5,6,7,8-hexahydroquinazolin-2-yl)-2-azabicyclo[2.1.1]hexan-2-yl)piperidin-1-yl)picolinamide (compound 361)***

To a solution of 2-(2-azabicyclo[2.1.1]hexan-4-yl)-5,6,7,8-tetrahydroquinazolin-4(3H)-one **6** (40 mg, 0.17 mmol) in MeOH (10 mL) was added N-methyl-5-(4-oxopiperidin-1-yl)picolinamide **INT** (40 mg, 0.17 mmol) and 3 drops of HOAc, then added $NaBH_3CN$ (10 mg, 0.16 mmol). The reaction mixture stirred at 50 °C for 4 h. After cooling to room temperature, the reaction mixture was concentrated under reduced pressure. The residue was purified by prep-HPLC (columns: Gemini 5 um C18 150 × 21.2 mm, mobile phase: ACN - $H_2O$ (0.1% FA), gradient: 10 - 25) to give N-methyl-5-(4-(4-(4-oxo-3,4,5,6,7,8-hexahydroquinazolin-2-yl)-2-azabicyclo[2.1.1]hexan-2-yl)piperidin-1-yl)picolinamide **361** (30 mg, 97% purity, 38% yield) as a white solid.

**Compound 361**

$^1$H NMR (400 MHz, DMSO) δ 12.11 (s, 1 H), 8.38 (q, *J* = 4.8 Hz, 1 H), 8.28 (d, *J* = 2.8 Hz, 1 H), 7.82 (d, *J* = 8.8 Hz, 1 H), 7.40 (dd, *J* = 8.8, 2.8 Hz, 1 H), 3.87 (d, *J* = 12.8 Hz, 2 H), 3.69 (s, 1 H), 2.98 (s, 2 H), 2.91 (t, *J* = 11.2 Hz, 2 H), 2.78 (d, *J* = 4.8 Hz, 3 H), 2.61-2.54 (m, 1 H), 2.48-2.44 (m, 2 H), 2.31 (t, *J* = 6.0 Hz, 2 H), 2.09-2.02 (m, 2 H), 1.99-1.92 (m, 2 H), 1.84-1.78 (m, 2 H), 1.72-1.59 (m, 4 H), 1.51-1.41 (m, 2 H).

LCMS (ESI) calcd for C25H32N602 [M + H] $^+$ m/z 449.26, found 449.20.

***Example 28 - Synthesis of compound 375***

**Synthesis of compound 375**

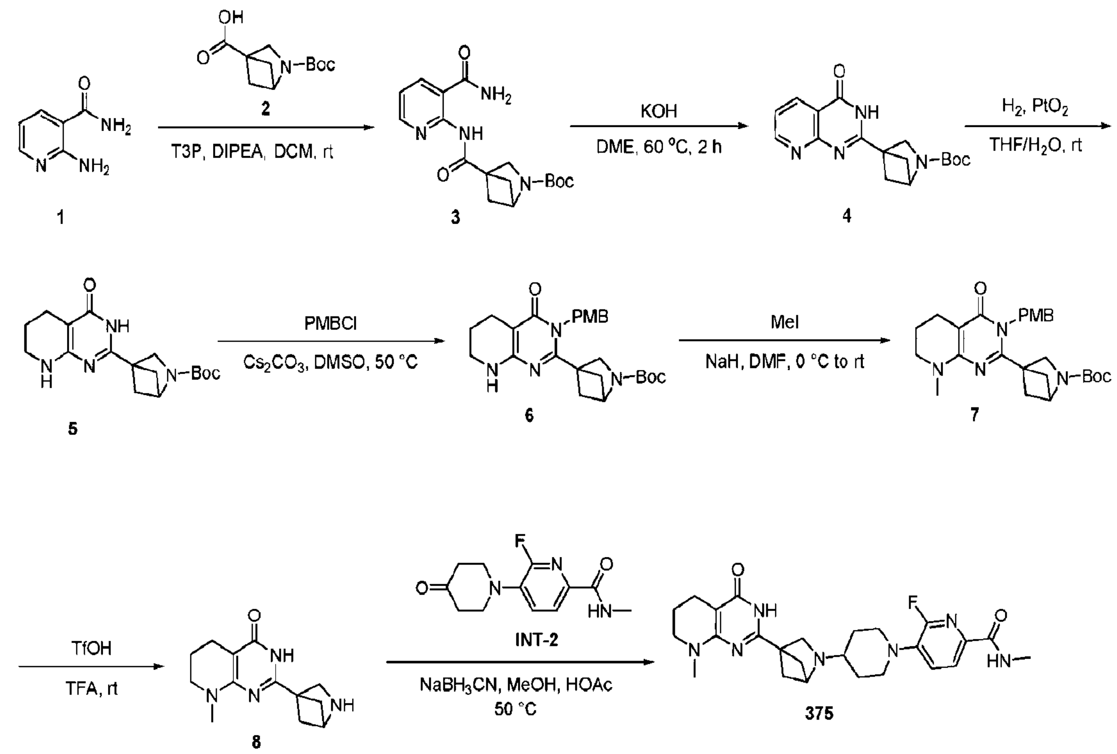


***Preparation of tert-butyl 4-((3-carbamoylpyridin-2-yl)carbamoyl)-2-azabicyclo[2.1.1]hexane-2-carboxylate (3)***

To a solution of 2-(tert-butoxycarbonyl)-2-azabicyclo[2.1.1]hexane-4-carboxylic acid **2** (500 mg, 2.19 mmol) in DCM (15 mL) were added 2-aminonicotinamide **1** (330 mg, 2.41 mmol), T3P (50% wt in EtOAc, 4.18 g, 6.57 mmol) and DIPEA (850 mg, 6.57 mmol) at room temperature successively. The mixture was kept stirring at room temperature for 1 h. The reaction solution was concentrated under reduced pressure and the residue was purified by flash chromatography (eluting with DCM/ MeOH = 100 : 0 to 90 : 10) to give tert-butyl 4-((3-carbamoylpyridin-2-yl)carbamoyl)-2-azabicyclo[2.1.1] hexane-2-carboxylate 3 (330 mg, 90% purity, 39% yield) as white solid.

LCMS (ESI) calcd for $C_{17}H_{22}N_4O_4$ [M + H] $^+$ m/z 347.16, found 346.85.

***Preparation of tert-butyl 4-(4-oxo-3,4-dihydropyrido[2,3-d]pyrimidin-2-yl)-2-azabicyclo[2.1.1]hexane-2-carboxylate (4)***

To a solution of tert-butyl 4-((3-carbamoylpyridin-2-yl)carbamoyl)-2-azabicyclo[2.1.1]hexane-2-carboxylate **3** (330 mg, 0.95 mmol) in DME (20 mL) at rt was added KOH (160 mg, 2.85 mmol). The reaction mixture was stirred at 60 °C for 6 h. The reaction solution was filtered to remove KOH. The filtrate was diluted with water and extracted with EtOAc (10 mL × 3). The water phase was purified by silica gel $C_{18}$ column (eluting with 10% to 50% MeCN/$H_2O$, containing 0.1% formic acid) to give tert-butyl 4-(4-oxo-3,4-dihydropyrido[2,3-d]pyrimidin-2-yl)-2-azabicyclo[2.1.1]hexane-2-carboxylate **4** (220 mg, 80% purity, 56% yield) as white solid.

LCMS (ESI) calcd for $C_{17}H_{20}N_4O_3$ [M + H]$^+$ m/z 329.15, found 329.00.

***Preparation of tert-butyl 4-(4-oxo-3,4,5,6,7,8-hexahydropyrido[2,3-d]pyrimidin-2-yl)-2-azabicyclo[2.1.1]hexane-2-carboxylate (5)***

To a solution of tert-butyl 4-(4-oxo-3,4-dihydropyrido[2,3-d]pyrimidin-2-yl)-2-*azabicyclo[2.1.1]hexane-2-carboxylate* **4** (200 mg, 0.607 mmol) in THF/$H_2O$ = 4:1 (20 mL) was added $PtO_2$ (41 mg). Then the mixture was stirred under hydrogen for 18 h at rt. The mixture was filtered through a celite pad, and the filtrate was concentrated under reduced pressure to give tert-butyl 4-(4-oxo-3,4,5,6,7,8-hexahydropyrido[2,3-d]pyrimidin-2-yl)-2-*azabicyclo[2.1.1]hexane-2-carboxylate* **5** (200 mg, 90% purity, 88% yield) as a yellow solid. LCMS (ESI) calcd for $C_{17}H_{24}N_4O_3$ [M + H]$^+$ m/z 333.18, found 333.25.

***Preparation of tert-butyl 4-(3-(4-methoxybenzyl)-4-oxo-3,4,5,6,7,8-hexahydropyrido[2,3-d]pyrimidin-2-yl)-2-azabicyclo[2.1.1]hexane-2-carboxylate (6)***

To a solution of tert-butyl 4-(4-oxo-3,4,5,6,7,8-hexahydropyrido[2,3-d]pyrimidin-2-yl)-2-azabicyclo[2.1.1]hexane-2-carboxylate **5** (200 mg, 0.6 mmol) in DMSO (10 mL) at room temperature were added $Cs_2CO_3$ (586 mg, 1.8 mmol) and PMBCl (188 mg, 1.2 mmol). The reaction mixture was stirred at 50 °C for 1 h. The reaction solution was quenched with water and extracted with EtOAc (30 mL × 3). The combined organic phase was washed three times with brine and concentrated under reduced pressure. The residue was purified by flash chromatography (eluting with PE/EtOAc = 100 : 0 to 50: 50) to give tert-butyl 4-(3-(4-methoxybenzyl)-4-oxo-3,4,5,6,7,8-hexahydropyrido[2,3-d]pyrimidin-2-yl)-2-azabicyclo[2.1.1]hexane-2-carboxylate **6** (150 mg, 90% purity, 49% yield) as white solid. LCMS (ESI) calcd for $C_{25}H_{32}N_4O_4$ [M + H]$^+$ m/z 453.24, found 453.05.

***Preparation of tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate (7)***

To a solution of methyl tert-butyl 4-(3-(4-methoxybenzyl)-4-oxo-3,4,5,6,7,8-hexahydropyrido[2,3-d]pyrimidin-2-yl)-2-azabicyclo[2.1.1]hexane-2-carboxylate **6** (50 mg, 0.11 mmol) in DMF (10 mL) at 0 °C were added NaH (7 mg, 0.17 mmol, 60% wt) and MeI (24 mg, 0.17 mmol). The reaction mixture was stirred at rt for 1 h. The reaction solution was quenched with water and extracted with EtOAc (50 mL × 3). The organic phase was concentrated under reduced pressure and the residue was purified by flash chromatography (eluting with PE/EtOAc = 100 : 0 to 70: 30) to give tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate **7** (30 mg, 90% purity, 52% yield) as white solid.

LCMS (ESI) calcd for $C_{26}H_{34}N_4O_4$ [M + H]$^+$ m/z 467.26, found 467.30.

***Preparation of 2-(2-azabicyclo[2.1.1]hexan-4-yl)-8-methyl-5,6,7,8-tetrahydropyrido/2,3-d]pyrimidin-4(3H)-one (8)***

To a solution of tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate **7** (30 mg, 0.064 mmol) in TFA (5 mL)was added TfOH (0.2 mL). The reaction was stirred at rt for 0.5 h. The reaction solution was adjusted to pH 8 with saturated aqueous $NaHCO_3$ at 0 °C. The basified solution was extracted with DCM (10 mL × 3). The combined organic layer was washed with brine, dried over $Na_2SO_4$ and concentrated under reduced pressure to give 2-(2-azabicyclo[2.1.1]hexan-4-yl)-8-methyl-5,6,7,8-tetrahydropyrido[2,3-d]pyrimidin-4(3H)-one 8 (25 mg, 60% purity, 94% yield) as a yellow oil.

LCMS (ESI) calcd for $C_{13}H_{18}N_4O$ [M + H] $^+$ m/z 247.15, found 247.25.

***Preparation of 6-fluoro-N-methyl-5-(4-(4-(8-methyl-4-oxo-3,4,5,6,7,8-hexahydropyrido[2,3-d]pyrimidin-2-yl)-2-azabicyclo[2.1.1]hexan-2-yl)piperidin-1-yl)picolinamide (compound 375)***

To a solution of 2-(2-azabicyclo[2.1.1]hexan-4-yl)-8-methyl-5,6,7,8-tetrahydropyrido[2,3-d]pyrimidin-4(3H)-one **8** (25 mg, 0.10 mmol) in MeOH (15 mL) and AcOH (0.01 mL) at room temperature was added 6-fluoro-N-methyl-5-(4-oxopiperidin-1-yl)picolinamide **INT-2** (31 mg, 0.12 mmol) and $NaBH_3CN$ (10 mg, 0.15 mmol). The reaction mixture was stirred at 50 °C for 1 h. The reaction solution was concentrated under reduced pressure and the residue was purified by flash chromatography (eluting with DCM/MeOH = 100 : 0 to 90: 10) and prep-HPLC (Gemini 5um $C_{18}$ 150 × 21.2mm, mobile phase: ACN - $H_2O$ (0.05% $NH_3$), gradient: 25 - 95) to give 6-fluoro-N-methyl-5-(4-(4-(8-methyl-4-oxo-3,4,5,6,7,8-hexahydropyrido[2,3-d]pyrimidin-2-yl)-2-azabicyclo[2.1.1]hexan-2-yl)piperidin-1-yl)picolinamide **375** (5.0 mg, 97% purity, 10 % yield) as white solid.

$^1H$ NMR (400 MHz, DMSO-$d_6$, ppm) δ: 11.32 (s, 1 H), 8.43-8.35 (m, 1 H), 7.83 (d, $J$ = 8.0 Hz, 1 H), 7.63-7.53 (m, 1 H), 3.61 (s, 1 H), 3.57-3.50 (m, 2 H), 3.25-3.18 (m, 2 H), 3.03 (s, 3 H), 2.91 (s, 2 H), 2.84 (t, $J$ = 11.2 Hz, 2 H), 2.76 (d, $J$ = 4.8 Hz, 3 H), 2.47-2.42 (m, 1 H), 2.37-2.31 (m, 2 H), 2.03-1.90 (m, 4 H), 1.80-1.71 (m, 4 H), 1.55-1.42 (m, 2 H).

LCMS (ESI) calcd for $C_{25}H_{32}FN_7O_2$ [M + H] $^+$ m/z 482.26, found 482.15.

***Example* 29 - Synthesis of compound 393rac**

**Synthesis of compound 393rac**

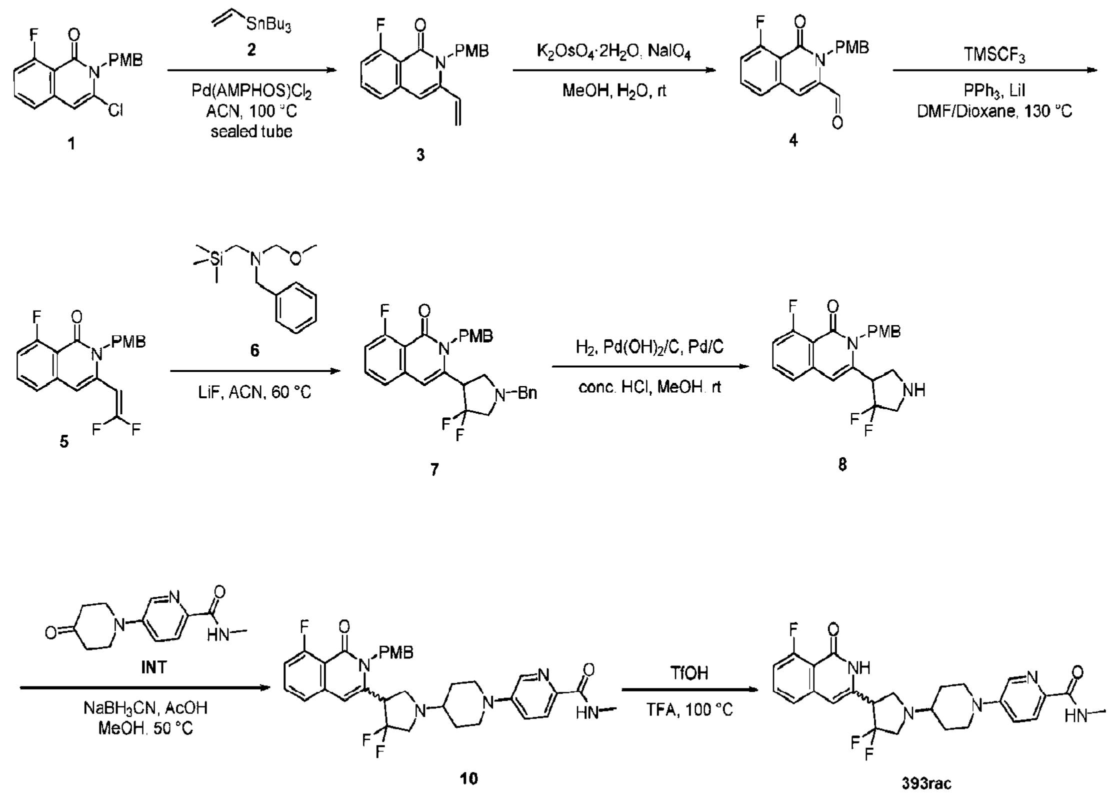


***Preparation of 8-fluoro-2-(4-methoxybenzyl)-3-vinylisoquinolin-1 (2H)- (3)***

To a solution of 3-chloro-8-fluoro-2-(4-methoxybenzyl)isoquinolin-1(2H)-one 1 (2000 mg, 6.294 mmol) and Pd(AMPHOS)$Cl_2$ (445 mg, 0.6294 mmol) in ACN (30 mL) was added tributyl(vinyl)stannane 2 (1995 mg, 6.294 mmol). The mixture was heated at 100 °C in sealed tube for 5 h. After cooling to room temperature, the reaction mixture was concentrated under reduced pressure. The residue was purified by flash chromatography (eluting with EtOAc/PE, 0 to 15%) to give 8-fluoro-2-(4-methoxybenzyl)-3-vinylisoquinolin-1(2H)-one 3 (1500 mg, 90% purity, 69% yield) as a white solid.

LCMS (ESI) calcd for $C_{19}H_{16}FNO_2$ [M + H] $^+$ m/z 310.12, found 309.90.

***Preparation of 8-fluoro-2-(4-methoxybenzyl)-1-oxo-1,2-dihydroisoquinoline-3-carbaldehyde (4)***

To a solution of 8-fluoro-2-(4-methoxybenzyl)-3-vinylisoquinolin-1(2H)-one **3** (1500 mg, 4.85 mmol) in MeOH/-$H_2O$ (3:1, 80 mL) was added $NaIO_4$ (4150 mg, 19.40 mmol) and $K_2OsO_4 \cdot 2H_2O$ (178 mg, 0.48 mmol). The reaction stirred for 1 h at room temperature. The reaction mixture was diluted with water and extracted with EtOAc (200 mL x 3). The combined organic layers were washed by brine, dried over $Na_2SO_4$ and concentrated under reduced pressure. The residue was purified by flash chromatography on silica gel (eluting with EtOAc/PE, 0 to 25%) to give 8-fluoro-2-(4-methoxybenzyl)-1-oxo-1,2-dihydroisoquinoline-3-carbaldehyde **4** (430 mg, 90% purity, 25% yield) as a yellow solid.

LCMS (ESI) calcd for $C_{18}H_{14}FNO_3$ [M + H] $^+$ m/z 312.10, found 311.90.

***Preparation of 3-(2,2-difluorovinyl)-8-fluoro-2-(4-methoxybenzyl)isoquinolin-1(2H)-one*** (5)

To a solution of 8-fluoro-2-(4-methoxybenzyl)-1-oxo-1,2-dihydroisoquinoline-3-carbaldehyde **4** (430 mg, 1.38 mmol), $PPh_3$ (724 mg, 2.76 mmol) and LiI (369 mg, 2.76 mmol) in DMF/Dioxane (8%, 10 mL) was added $TMSCF_3$ (491 mg, 3.45 mmol). The mixture was heated at 130 °C in a sealed tube for 3 h. After cooling to room temperature, the reaction mixture was concentrated under reduced pressure. The residue was purified by flash chromatography (eluting with EtOAc/PE, 0 to 15%) to give 3-(2,2-difluorovinyl)-8-fluoro-2-(4-methoxybenzyl)isoquinolin-1(2H)-one **5** (200 mg, 60% purity, 25% yield) as a white solid.

LCMS (ESI) calcd for $C_{19}H_{14}F_3NO_2$ [M + H] $^+$ m/z 346.10, found 346.00.

***Preparation of 3-(1-benzyl-4,4-difluoropyrrolidin-3-yl)-8-fluoro-2-(4-*****methoxybenzyl)isoquinolin-1(2H)-one** *(7)*

To a solution of 3-(2,2-difluorovinyl)-8-fluoro-2-(4-methoxybenzyl)isoquinolin-1(2H)-one **5** (260 mg, 0.75 mmol) and LiF (39 mg, 1.51 mmol) in ACN (50 mL) was added N-benzyl-1-methoxy-N-((trimethylsilyl)methyl)methanamine **6** (357 mg, 1.51 mmol). The mixture was kept stirring at 60 °C for 16 h. The resulting mixture was diluted with water and extracted with DCM (30 mL × 3). The combined organic layer was dried over $Na_2SO_4$ and concentrated under reduced pressure. The residue was purified by flash chromatography (eluting with EtOAc/PE, 0 to 15%) to give 3-(1-benzyl-4,4-difluoropyrrolidin-3-yl)-8-fluoro-2-(4-methoxybenzyl)isoquinolin-1(2H)-one **7** (25 mg, 90% purity, 6% yield) as a white solid.

LCMS (ESI) calcd for $C_{28}H_{23}F_3N_2O_2$ [M + H] $^+$ m/z 479.19, found 479.20.

***Preparation of 3-(4,4-difluoropyrrolidin-3-yl)-8-fluoro-2-(4-methoxybenzyl)isoquinolin-1(2H)-one (7)***

To a solution of 3-(1-benzyl-4,4-difluoropyrrolidin-3-yl)-8-fluoro-2-(4-methoxybenzyl)isoquinolin-1(2H)-one **7** (130 mg, 0.271 mmol) in MeOH (5 mL) was added one drop of conc. HCl, 10% Pd/C (20 mg) and 10% $Pd(OH)_2/C$ (20 mg). The mixture was evacuated and backfilled with hydrogen three times and then charged with hydrogen. The resulting mixture was stirred at room temperature for 16 hours. Then the mixture was filtered through celite and concentrated under vacuum to give crude 3-(4,4-difluoropyrrolidin-3-yl)-8-fluoro-2-(4-methoxybenzyl)isoquinolin-1(2H)-one **8** (100 mg, 50% purity, 47% yield) as a white solid which was used directly in next step without further purification.

LCMS (ESI) calcd for $C_{21}H_{19}F_3N_2O_2$ [M + H] $^+$ m/z 389.14, found 389.00.

***Preparation of 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide (10)***

To a solution of 3-(4,4-difluoropyrrolidin-3-yl)-8-fluoro-2-(4-methoxybenzyl)isoquinolin-1(2H)-one **8** (100 mg, 0.257 mmol) in MeOH (10 mL) was added N-methyl-5-(4-oxopiperidin-1-yl)picolinamide **INT** (90 mg, 0.386 mmol), then two drops of acetic acid and $NaBH_3CN$ (24 mg, 0.386 mmol) were added at room temperature. The reaction mixture stirred at 50 °C for 1 h. After cooling to room temperature, the reaction mixture was concentrated under reduced pressure. The residue was purified by flash chromatography (eluting with DCM/MeOH = 100 : 0 to 90 : 10) to give 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide **10** (80 mg, 50% purity, 25% yield) as a white solid.

LCMS (ESI) calcd for $C_{33}H_{34}F_3N_5O_3$ [M + H] $^+$ m/z 606.26, found 606.20.

***Preparation of 5-(4-(3,3-difluoro-4-(8-fluoro-1-oxo-1,2-dihydroisoquinolin-3-yl)pyrrolidin-1-yl)piperidin-1-yl)-N-methylpicolinamide (compound 393rac)***

To a solution of 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide **10** (80 mg, 0.13 mmol) in TFA (5 mL) was added TfOH (1 mL) dropwise at rt. The reaction mixture was stirred at 100 °C for 5 min. The pH of the resulting mixture was adjusted to around 8.0 by progressively adding saturated $NaHCO_3$ solution, and then extracted with DCM (50 mL × 3). The combined organic layers were dried over $Na_2SO_4$ and concentrated under reduced pressure. The residue was purified by prep-HPLC (Gemini 5 um C18 150 × 21.2 mm, eluting with 35% to 40% ACN/$H_2O$ containing 0.1% FA) to give 5-(4-(3,3-difluoro-4-(8-fluoro-1-oxo-1,2-dihydroisoquinolin-3-yl)pyrrolidin-1-yl)piperidin-1-yl)-N-methylpicolinamide **393rac** (6.2 mg, 93% purity, 9% yield) as a brown solid. $^1H$ NMR (400 MHz, $CD_3OD$_SPE, ppm) δ: 8.31 (d, *J* = 2.8 Hz, 1 H), 7.95 (d, *J* = 8.8 Hz, 1 H), 7.72-7.65 (m, 1 H), 7.57-7.47 (m, 1 H), 7.43 (d, *J* = 8.0 Hz, 1 H), 7.17 (dd, *J* = 11.8, 8.2 Hz, 1 H), 6.76-6.68 (m, 1 H), 4.10-3.94 (m, 2 H), 3.85-3.50 (m, 5 H), 3.28-3.25 (m, 1 H), 3.07 (t, *J* = 12.4 Hz, 2 H), 2.96-2.90 (m, 3 H), 2.24-2.09 (m, 2 H), 1.84-1.66 (m, 2 H).

LCMS (ESI) calcd for $C_{25}H_{26}F_3N_5O_2$ $[M + H]^+$ m/z 486.21, found 486.40.

## ***Assays***

Exemplary compounds of the invention were prepared and tested to determine their effect as PARP1 and PARP2 inhibitors. Typical assays are described below.

### ***PARP1 biochemical dissociation-enhanced lanthanide fluorescence immunoassay (DELFIA assay)***

Optiplate HB 384-well plates were coated with anti-FLAG antibody, supplied as a 4 mg/ml solution, using a $Na_2CO_3/HCO_3$ coating buffer at pH 9.6, overnight at 4°C, in order to achieve a final immobilisation per well of 0.3 μg. Wells were then washed 3 x 5 min in coating wash buffer (PBS/0.05 % Tween (v/v)), and blocked with 2 % BSA (w/v) in coating wash buffer overnight at 4°C. Prior to assay, wells were washed 3 x 5 min in coating wash buffer. For the assay 20 μl of 2.5 nM recombinant full length human N-terminally FLAG-tagged PARP1 was added to each well of the 384-well plate for 30 min at room temperature followed by addition of 50 nL of compound solution in DMSO using pintool technology. Following incubation for 30 min at room temperature, 5 μl of 10 μM biotin-NAD* and 10 nM activation DNA (sequence shown below) in solution in 20 mM HEPES (pH 7.5), 100 mM NaCl, 2 mM DTT, 0.1 % BSA (w/v), 0.02 % Tween (v/v) assay buffer. Auto-PARylation proceeded for 2 h at room temperature prior to the addition of 5 μl of 12 mM $NAD^+$ quenching solution. After 30 min at room temperature, assay solution was removed and following washing 5 times for 3 min, 100 μl of a 1:1000 dilution of DELFIA Eu-N1 Streptavidin reagent was added. Plates were then incubated for 30 min at room temperature. Reaction mixture was removed and plates washed 5 times for 3 min prior to the addition of 25 μl DELFIA enhancement solution. Following incubation for 30 min at room temperature, fluorescence was measured on a Pherastar FS (Ex337 nm, Em620 nm; integration start 60 μs; integration time 400 μs).

Typically compounds were tested from 20 μM at 3-fold dilution intervals in 12-point concentration-response curves to determine $IC_{50}$ values. Data was analysed using ActivityBase software and replicate values for the low (without enzyme, 0.2% DMSO) and high (0.2 % DMSO) % controls were averaged and the data obtained from the test compounds expressed as a % of 100 % using the below formulae:

$$\% \text{ value} = 100\text{-}(100{*}((\text{high control - unknown}) / (\text{high control - low control}))$$

% data was fitted to a non-linear regression equation (log inhibitor vs response-variable slope 4-parameters) to obtain $IC_{50}$ values.

The $IC_{50}$ values for a variety of test compounds are shown in Table 1.

**Activation DNA sequence:**

*Duplex Sequences*

5'-ACCCTGCTGTGGGC/ideoxyU/GGAGAACAAGGTGAT-3' (SEQ ID NO:1)
5'-ATCACCTTGTTCTCCAHGCCCACAGCAGGGT-3' (SEQ ID NO:2)

```
    5'-ACCCTGCTGTGGGCGGAGAACAAGGTGAT-3' (SEQ ID NO:3)
          |       || |     |||||||||||||||
  3'-TGGGACGACACCCGHACCTCTTGTTCCACTA-5'
```

***PARP1 probe displacement homogeneous time-resolved fluorescence assay (HTRF assay)***

10 nM full length N-terminally FLAG-tagged PARP1 was incubated with 2 nM Anti-FLAG Tb-cryptate antibody and PARP1/2 Cy5 fluorescent dye-labelled binding probe (10-fold probe $K_d$ = 270 nM) in 20 mM HEPES (pH 7.5), 100 mM NaCl, 2 mM DTT, 0.1 % BSA (w/v), 0.02 % Tween (v/v) assay buffer for 40 min at room temperature. A Cy5-labelled binding probe is shown below and described in Papeo, G. et al. J. Biomol. Screen. 2014; 19:1212-1219. 6 μl of this reaction mixture was then transferred to each well of a black non-binding surface 384-well plate and 35 nl of compound solution in DMSO was then added using pintool technology. Following incubation for 1 h at room temperature, fluorescence was measured on a Pherastar FS (Ex 337 nm, Em620 nm, em665 nm; integration start 60μs; integration time 400μs) using the HTRF module.

Typically compounds were tested from 58.5 μM at factor 3 dilution intervals in 12-point concentration-response curves to determine $IC_{50}$ values. Data was analysed using ActivityBase software and replicate values for the low (without enzyme but with probe and Tb-cryptate antibody, 0.6% DMSO) and high (0.6 % DMSO) % controls were averaged and the data obtained from the test compounds expressed as a % of 100 % using the below formulae:

$$\%activity = 100*(value - low\ control) / (high\ control - low\ control)$$

%activity data was fitted to a non-linear regression equation to obtain IC50 values
$K_d$ values were calculated using Cheng-Prussoff formula:

$$IC_{50} = (1+ ([probe\ concentration]/[Km_{probe}]))*K_d$$

Therefore $K_d = IC_{50} / (1+[[probe\ concentration]/[Km_{probe}]))$; using probe at 10 x $K_m$, this equated to $K_d = IC_{50}/11$

***PARP2 probe displacement homogeneous time-resolved fluorescence assay (HTRF assay)***

This assay was performed under identical conditions as for PARP1, except that N-terminally FLAG-tagged PARP2 (amino acids 1-583) was used instead of PARP1, and PARP1/2 binding probe was used at 10-fold probe $K_d$ = 540 nM. Data analysis was performed identical as for PARP1.

Cy5 probe structure:

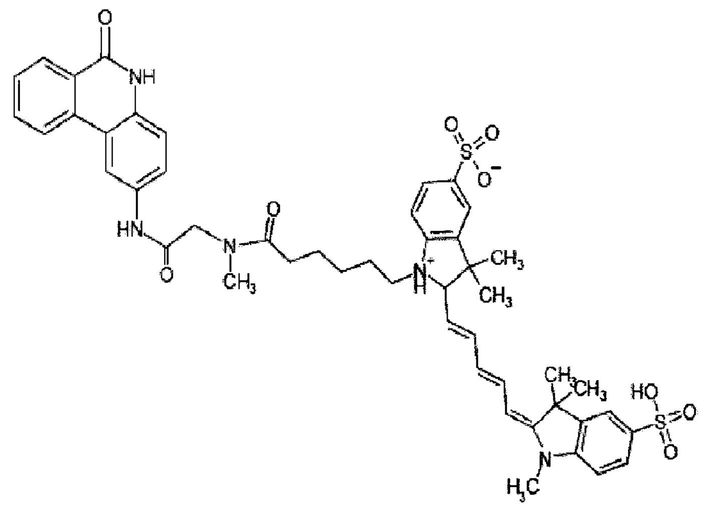

***NanoBRET cellular target occupancy assay***

NanoBRET assays were employed to demonstrate cellular target engagement and selectivity at PARP1 and PARP2. These assays are based on bioluminescence resonance energy transfer (BRET) between a Nano-luc-tagged protein (eg PARP1 or PARP2) and a fluorescent group on a high affinity $NAD^+$ competitive binding probe. Such cellular

probe displacement assays can be utilised to measure inhibitor affinities and selectivity ratios at PARP1 and 2.

Frozen HEK293 cells transiently transfected with either PARP1-NanoLuc® fusion or PARP2-NanoLuc® fusion constructs (Promega) were thawed and dispensed as a suspension in 384-well microplates each at a density of 1750 cells per well. NanoBRET™ TE PARP Tracer-01 was then added to final concentrations of 11 and 2 nM for PARP1 and PARP2 assays, respectively. Compounds were added from 25 μM at factor 3 dilution intervals in 12-point concentration-response curves and plates were incubated for 2 hours at 37°C. BRET ratios were then measured using a NanoBRET module (LUM 610-LP 450-80) and PHERAstar FS or FSX reader following addition of NanoBRET™ Nano-Glo® Substrate and Extracellular NanoLuc® Inhibitor according to manufacturer's instructions. Kd values were calculated using Cheng-Prussoff formula:

$$IC50 = (1 + ([\text{tracer concentration}]/[Km_{\text{tracer}}])) * Kd$$

Binned potency, affinity and selectivity data for a variety of test compounds are shown in Table 1 where DELFIA and Probe Displacement HTRF assays were used. Binned potency, affinity and selectivity data for a subset of test compounds where the NanoBRET assay was used are shown in Table 2.

**TABLE 1**

| *Results of Parp 1/2 assays for selected compounds (DELFIA and Probe Displacement HTRF)* | | | | |
|---|---|---|---|---|
| **Compound** | **PARP1 DELFIA** | **PARP1 HTRF** | **PARP2 HTRF** | **Selectivity** |
| 1 | ++ | NT | NT | NT |
| 2 | ++++ | ++++ | + | +++ |
| 3a | ++ | NT | NT | NT |
| 3b | ++ | NT | NT | NT |
| 4a | ++ | ++ | ++ | - |
| 4b | ++ | NT | NT | NT |
| 5 | + | NT | NT | NT |
| 6a | ++++ | ++++ | - | +++ |
| 6b | ++ | ++ | + | + |
| 7a | +++ | ++++ | ++ | +++ |
| 7b | ++ | NT | NT | NT |
| 8 | ++++ | ++++ | - | +++ |
| 9a | +++ | +++ | + | +++ |
| 9b | +++ | +++ | ++ | - |
| 10a | +++ | ++++ | ++ | +++ |
| 10b | ++++ | ++++ | ++ | +++ |
| 11a | + | ++ | - | - |
| 11b | ++ | ++ | + | - |
| 12 | ++ | NT | NT | NT |
| 13a | + | NT | NT | NT |
| 13b | + | NT | NT | NT |
| 14a | + | NT | NT | NT |
| 14b | +++ | +++ | - | +++ |
| 15 | ++ | NT | NT | NT |
| 16a | ++ | NT | NT | NT |
| 16b | ++ | NT | NT | NT |

(continued)

| Results of Parp 1/2 assays for selected compounds (DELFIA and Probe Displacement HTRF) | | | | |
|---|---|---|---|---|
| Compound | PARP1 DELFIA | PARP1 HTRF | PARP2 HTRF | Selectivity |
| 17 | +++ | +++ | - | +++ |
| 18 | ++ | NT | NT | NT |
| 19 | + | NT | NT | NT |
| 20 | +++ | +++ | - | ++ |
| 21 | + | NT | NT | NT |
| 22 | + | NT | NT | NT |
| 23a | ++ | NT | NT | NT |
| 23b | + | + | - | - |
| 24a | + | NT | NT | NT |
| 24b | + | NT | NT | NT |
| 25 | ++ | NT | NT | NT |
| 26rac | +++ | +++ | ++ | + |
| 27 | +++ | +++ | + | + |
| 28 | ++ | NT | NT | NT |
| 29 | + | NT | NT | NT |
| 30 | ++ | NT | NT | NT |
| 31a | + | NT | NT | NT |
| 31b | +++ | +++ | - | +++ |
| 32a | +++ | +++ | + | + |
| 32b | ++ | NT | NT | NT |
| 33a | - | NT | NT | NT |
| 33b | +++ | +++ | - | ++ |
| 34a | +++ | +++ | ++ | - |
| 34b | ++++ | ++++ | + | +++ |
| 35a | ++ | NT | NT | NT |
| 35b | +++ | +++ | - | +++ |
| 36 | ++++ | ++++ | - | +++ |
| 37rac | ++ | NT | NT | NT |
| 38a | + | + | + | - |
| 38b | + | + | - | - |
| 39rac | ++ | ++ | - | + |
| 40 | ++++ | ++++ | ++ | ++ |
| 41 | ++ | ++ | - | + |
| 42rac | ++++ | ++++ | + | +++ |
| 43a | ++ | +++ | ++ | - |
| 43b | ++++ | ++++ | ++ | + |
| 44a | ++++ | ++++ | - | +++ |
| 44b | +++ | +++ | + | + |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 45 | ++++ | ++++ | ++ | +++ |
| 46a | ++++ | +++ | + | +++ |
| 46b | ++ | ++ | ++ | - |
| 47 | ++ | ++ | - | - |
| 48 | ++++ | ++++ | ++ | +++ |
| 49rac | ++++ | ++++ | ++ | +++ |
| 50a | +++ | +++ | + | + |
| 50b | +++ | +++ | - | +++ |
| 51a | ++ | ++ | - | + |
| 51b | + | + | + | - |
| 52a | +++ | +++ | ++ | - |
| 52b | ++++ | ++++ | ++ | +++ |
| 53a | ++++ | ++++ | + | +++ |
| 53b | ++ | ++ | - | + |
| 54 | ++++ | ++++ | ++ | +++ |
| 55a | ++++ | ++++ | + | +++ |
| 55b | +++ | +++ | ++ | - |
| 10rac | ++++ | ++++ | ++ | +++ |
| 56a | ++ | +++ | - | ++ |
| 56b | + | ++ | - | - |
| 57a | ++++ | +++ | - | +++ |
| 57b | ++ | ++ | - | + |
| 58a | ++++ | ++++ | - | +++ |
| 58b | +++ | +++ | - | ++ |
| 59 | + | ++ | - | - |
| 60a | ++++ | ++++ | - | +++ |
| 60b | ++ | +++ | - | ++ |
| 61a | ++ | +++ | + | + |
| 61b | ++++ | ++++ | + | +++ |
| 62a | +++ | +++ | + | ++ |
| 62b | ++ | ++ | + | - |
| 63 | ++++ | +++ | + | ++ |
| 64a | ++++ | ++++ | ++ | + |
| 64b | ++++ | ++++ | ++ | +++ |
| 65a | ++++ | ++++ | ++ | +++ |
| 65b | ++ | ++ | + | + |
| 66a | + | + | - | - |
| 66b | + | + | - | - |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 67a | ++ | ++ | + | + |
| 67b | ++ | ++ | + | + |
| 68a | ++++ | ++++ | ++ | +++ |
| 68b | +++ | ++++ | ++ | + |
| 69a | ++++ | +++ | ++ | ++ |
| 69b | +++ | +++ | ++ | - |
| 70a | +++ | ++ | + | + |
| 70b | +++ | ++ | ++ | - |
| 71a | + | + | - | - |
| 71b | +++ | +++ | - | +++ |
| 72a | +++ | +++ | + | +++ |
| 72b | ++++ | ++++ | ++ | +++ |
| 73a | ++ | ++ | - | - |
| 73b | +++ | +++ | - | ++ |
| 74a | ++ | ++ | + | + |
| 74b | +++ | +++ | + | ++ |
| 75 | NT | + | - | - |
| 76a | NT | +++ | ++ | - |
| 76b | NT | +++ | ++++ | - |
| 77a | NT | ++ | - | + |
| 77b | NT | +++ | - | +++ |
| 78a | NT | ++++ | + | +++ |
| 78b | NT | +++ | ++ | + |
| 79a | NT | +++ | ++ | + |
| 79b | NT | +++ | ++ | + |
| 80a | NT | ++++ | ++ | +++ |
| 80b | NT | ++ | ++ | - |
| 81a | NT | ++ | - | + |
| 81b | NT | +++ | - | +++ |
| 82a | NT | +++ | ++ | - |
| 82b | NT | +++ | - | ++ |
| 83a | NT | ++ | + | - |
| 83b | NT | ++ | - | + |
| 84a | NT | + | - | - |
| 84b | NT | +++ | - | ++ |
| 85a | NT | +++ | ++ | + |
| 85b | ++++ | ++++ | +++ | ++ |
| 86a | +++ | +++ | ++ | + |

(continued)

| *Results of Parp 1/2 assays for selected compounds (DELFIA and Probe Displacement HTRF)* | | | | |
|---|---|---|---|---|
| **Compound** | **PARP1 DELFIA** | **PARP1 HTRF** | **PARP2 HTRF** | **Selectivity** |
| 86b | ++++ | ++++ | ++++ | + |
| 87a | NT | ++ | + | + |
| 87b | NT | ++++ | + | +++ |
| 88a | NT | ++ | ++ | - |
| 88b | NT | ++++ | + | +++ |
| 89a | NT | - | - | - |
| 89b | NT | ++ | - | + |
| 90a | NT | ++ | - | + |
| 90b | NT | ++ | + | + |
| 91a | ++++ | ++++ | ++ | +++ |
| 91b | ++++ | ++++ | ++ | +++ |
| 92a | ++ | ++ | - | + |
| 92b | +++ | ++++ | + | +++ |
| 93a | ++ | ++ | - | + |
| 93b | + | + | - | - |
| 94a | +++ | +++ | + | + |
| 94b | ++++ | ++++ | - | +++ |
| 95rac | ++ | + | + | - |
| 96a | +++ | +++ | + | + |
| 96b | ++ | ++ | ++ | - |
| 97a | - | - | - | - |
| 97b | ++ | ++ | - | - |
| 98a | +++ | +++ | - | +++ |
| 98b | ++ | ++ | - | + |
| 99a | + | + | - | - |
| 99b | +++ | ++ | ++ | - |
| 100a | ++++ | ++++ | ++ | +++ |
| 100b | +++ | +++ | + | +++ |
| 101a | +++ | +++ | ++ | + |
| 101b | ++++ | ++++ | ++ | +++ |
| 102rac | ++++ | ++++ | + | +++ |
| 103rac | ++++ | ++++ | + | +++ |
| 104rac | ++++ | ++++ | + | +++ |
| 105rac | ++ | ++ | + | - |
| 106a | ++ | ++ | - | + |
| 106b | + | - | - | - |
| 107a | +++ | +++ | + | + |
| 107b | ++ | ++ | ++ | - |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 108a | ++ | ++ | - | - |
| 108b | ++ | ++ | - | + |
| 109a | +++ | ++++ | - | +++ |
| 109b | +++ | ++ | + | + |
| 110a | ++++ | ++++ | - | +++ |
| 110b | ++ | +++ | ++ | - |
| 111a | +++ | +++ | ++ | + |
| 111b | ++++ | ++++ | ++ | +++ |
| 112a | ++ | ++ | + | - |
| 112b | +++ | +++ | - | +++ |
| 113a | ++ | ++ | ++ | - |
| 113b | ++ | ++ | - | + |
| 114a | ++++ | ++++ | - | +++ |
| 114b | ++ | ++ | + | + |
| 115a | ++ | ++ | + | + |
| 115b | +++ | +++ | + | +++ |
| 116a | +++ | +++ | - | +++ |
| 116b | ++ | ++ | + | - |
| 117a | +++ | +++ | ++ | + |
| 117b | ++++ | ++++ | ++ | +++ |
| 118a | ++++ | ++++ | - | +++ |
| 118b | +++ | +++ | - | ++ |
| 119rac | ++++ | ++++ | + | +++ |
| 120rac | ++ | ++ | - | + |
| 121a | +++ | +++ | ++ | + |
| 121b | ++++ | ++++ | ++ | +++ |
| 122a | ++++ | +++ | - | +++ |
| 122b | +++ | ++ | + | - |
| 123a | +++ | +++ | ++ | + |
| 123b | ++++ | ++++ | + | +++ |
| 124rac | ++++ | ++++ | ++ | +++ |
| 125a | ++++ | ++++ | + | +++ |
| 125b | ++++ | ++++ | + | +++ |
| 126a | ++++ | ++++ | + | +++ |
| 126b | ++ | ++ | - | + |
| 127rac | +++ | +++ | - | +++ |
| 128a | +++ | +++ | + | +++ |
| 128b | ++++ | ++++ | +++ | ++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 129a | ++++ | ++++ | + | +++ |
| 129b | ++++ | ++++ | +++ | +++ |
| 130a | ++++ | ++++ | ++ | + |
| 130b | ++++ | ++++ | ++ | +++ |
| 131a | +++ | ++ | + | + |
| 131b | ++++ | ++++ | - | +++ |
| 132rac | +++ | +++ | - | +++ |
| 133a | ++++ | ++++ | + | +++ |
| 133b | +++ | +++ | - | +++ |
| 134rac | ++++ | ++++ | - | +++ |
| 135rac | ++++ | ++++ | +++ | + |
| 136rac | ++++ | ++++ | +++ | +++ |
| 137a | ++++ | ++++ | ++ | +++ |
| 137b | ++++ | ++++ | +++ | + |
| 138rac | +++ | +++ | + | ++ |
| 139a | ++++ | ++++ | + | +++ |
| 139b | +++ | +++ | + | +++ |
| 140a | ++ | +++ | - | ++ |
| 140b | +++ | +++ | + | +++ |
| 141rac | ++++ | ++++ | + | +++ |
| 142rac | ++++ | ++++ | + | +++ |
| 143rac | +++ | ++++ | + | +++ |
| 144rac | ++++ | ++++ | + | +++ |
| 136a | ++++ | ++++ | ++ | +++ |
| 136b | ++++ | ++++ | +++ | +++ |
| 145a | ++++ | ++++ | + | +++ |
| 145b | ++++ | ++++ | + | +++ |
| 146a | ++ | ++ | + | - |
| 146b | +++ | +++ | - | +++ |
| 147a | + | + | - | - |
| 147b | + | + | - | - |
| 148a | + | - | - | - |
| 148b | + | + | - | - |
| 149a | ++ | ++ | - | + |
| 149b | + | + | - | - |
| 150rac | ++++ | ++++ | ++++ | - |
| 151a | ++++ | ++++ | ++ | +++ |
| 151b | ++++ | ++++ | - | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 152a | ++++ | ++++ | + | +++ |
| 152b | +++ | +++ | - | ++ |
| 153rac | ++++ | ++++ | ++ | ++ |
| 154rac | ++++ | ++++ | +++ | + |
| 155a | +++ | +++ | ++ | + |
| 155b | ++++ | ++++ | ++ | +++ |
| 156rac | +++ | +++ | - | ++ |
| 157rac | ++++ | ++++ | + | +++ |
| 158a | ++++ | ++++ | + | +++ |
| 158b | +++ | +++ | + | +++ |
| 159a | - | - | - | - |
| 159b | ++ | ++ | - | + |
| 160rac | ++++ | ++++ | + | +++ |
| 161a | ++ | ++ | - | - |
| 161b | ++++ | ++++ | + | +++ |
| 162a | +++ | +++ | ++ | - |
| 162b | ++++ | ++++ | +++ | ++ |
| 163rac | ++++ | ++++ | ++ | +++ |
| 164a | ++++ | ++++ | + | +++ |
| 164b | +++ | +++ | - | +++ |
| 165a | ++++ | ++++ | ++ | +++ |
| 165b | +++ | +++ | + | +++ |
| 154a | ++++ | ++++ | ++ | +++ |
| 154b | ++++ | ++++ | +++ | + |
| 166rac | ++++ | ++++ | + | +++ |
| 167a | +++ | +++ | - | ++ |
| 167b | ++++ | ++++ | ++ | +++ |
| 168 | ++++ | ++++ | - | +++ |
| 169a | + | - | - | - |
| 169b | ++++ | +++ | - | +++ |
| 170rac | ++++ | ++++ | ++++ | - |
| 171rac | ++++ | ++++ | ++ | +++ |
| 172a | ++++ | ++++ | + | +++ |
| 172b | ++++ | ++++ | +++ | +++ |
| 173 | ++ | + | - | - |
| 174rac | ++++ | ++++ | ++ | +++ |
| 174a | ++++ | ++++ | ++ | +++ |
| 174b | ++++ | ++++ | +++ | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 175rac | ++++ | ++++ | ++ | +++ |
| 176rac | NT | ++ | - | + |
| 177rac | NT | ++++ | - | +++ |
| 178a | NT | ++ | + | - |
| 178b | NT | ++++ | + | +++ |
| 179 | NT | +++ | - | +++ |
| 180a | ++++ | ++++ | + | +++ |
| 180b | ++++ | ++++ | +++ | ++ |
| 181a | +++ | +++ | ++ | + |
| 181b | ++++ | ++++ | ++ | +++ |
| 182a | +++ | +++ | ++ | + |
| 182b | ++++ | ++++ | ++ | +++ |
| 183a | ++++ | ++++ | + | +++ |
| 183b | ++++ | ++++ | ++ | +++ |
| 184a | ++++ | ++++ | + | +++ |
| 184b | ++++ | ++++ | ++ | +++ |
| 176a | ++ | ++ | - | + |
| 176b | +++ | ++ | - | ++ |
| 170a | ++++ | ++++ | ++++ | - |
| 170b | ++++ | ++++ | ++++ | - |
| 185a | ++++ | ++++ | ++ | +++ |
| 185b | +++ | ++ | + | + |
| 186a | ++++ | ++++ | ++ | +++ |
| 186b | ++++ | ++++ | +++ | + |
| 187a | +++ | +++ | ++ | - |
| 187b | ++++ | ++++ | ++++ | - |
| 188a | ++ | ++ | - | + |
| 188b | ++++ | ++++ | ++ | +++ |
| 189a | +++ | +++ | ++ | - |
| 189b | ++++ | ++++ | +++ | ++ |
| 190a | ++ | ++ | + | + |
| 190b | ++++ | ++++ | +++ | ++ |
| 191rac | ++ | ++ | - | + |
| 192 | ++++ | ++++ | + | +++ |
| 193 | +++ | +++ | + | +++ |
| 194a | +++ | +++ | + | + |
| 194b | ++++ | ++++ | +++ | + |
| 195rac | ++++ | ++++ | ++ | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 196a | ++++ | ++++ | ++ | +++ |
| 1966 | ++++ | ++++ | + | +++ |
| 197a | ++++ | ++++ | ++ | +++ |
| 197b | +++ | +++ | + | +++ |
| 198rac | ++++ | ++++ | +++ | + |
| 199a | ++++ | ++++ | ++ | +++ |
| 199b | ++ | ++ | - | + |
| 200rac | +++ | +++ | - | +++ |
| 201a | ++ | ++ | - | + |
| 201b | ++++ | ++++ | + | +++ |
| 202a | +++ | +++ | ++ | + |
| 202b | ++++ | ++++ | +++ | + |
| 203rac | ++++ | ++++ | - | +++ |
| 204rac | ++++ | ++++ | - | +++ |
| 205a | ++ | ++ | - | + |
| 205b | +++ | +++ | + | + |
| 206a | ++++ | ++++ | ++ | +++ |
| 206b | ++++ | ++++ | + | +++ |
| 207a | ++++ | ++++ | - | +++ |
| 207b | ++ | ++ | - | - |
| 208a | ++++ | +++ | ++ | + |
| 208b | ++++ | ++++ | +++ | ++ |
| 209a | ++++ | ++++ | ++ | +++ |
| 209b | +++ | +++ | + | +++ |
| 210a | ++ | ++ | - | + |
| 210b | ++++ | ++++ | + | +++ |
| 211rac | ++++ | ++++ | ++ | +++ |
| 212a | ++ | ++ | - | + |
| 212b | - | - | - | - |
| 213a | +++ | +++ | - | +++ |
| 213b | ++++ | ++++ | ++ | +++ |
| 214a | +++ | +++ | ++ | ++ |
| 214b | ++++ | ++++ | ++ | +++ |
| 215rac | +++ | +++ | ++ | - |
| 216rac | ++ | ++ | - | - |
| 217a | ++ | ++ | - | - |
| 217b | ++++ | ++++ | ++ | ++ |
| 218a | +++ | +++ | - | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 218b | ++++ | ++++ | ++ | +++ |
| 219a | ++++ | ++++ | +++ | ++ |
| 219b | ++++ | ++++ | ++ | +++ |
| 220a | ++ | ++ | + | - |
| 220b | +++ | +++ | + | + |
| 221a | +++ | +++ | ++ | + |
| 221b | ++++ | ++++ | +++ | ++ |
| 222a | ++++ | ++++ | ++ | + |
| 222b | ++++ | ++++ | +++ | +++ |
| 223a | ++++ | ++++ | ++ | +++ |
| 223b | ++++ | ++++ | ++ | +++ |
| 224a | ++ | + | + | - |
| 224b | ++ | ++ | + | - |
| 225rac | ++++ | ++++ | + | +++ |
| 226rac | +++ | +++ | - | ++ |
| 227 | ++++ | ++++ | ++ | +++ |
| 228 | ++++ | ++++ | + | +++ |
| 229rac | +++ | ++++ | ++ | +++ |
| 230rac | ++++ | ++++ | + | +++ |
| 231rac | ++++ | ++++ | + | +++ |
| 232a | +++ | +++ | ++ | - |
| 232b | ++++ | ++++ | ++ | +++ |
| 233rac | ++++ | ++++ | ++ | +++ |
| 234rac | ++++ | ++++ | ++ | +++ |
| 235rac | ++++ | ++++ | +++ | + |
| 236a | +++ | +++ | ++ | + |
| 236b | ++++ | ++++ | +++ | + |
| 237rac | ++++ | ++++ | +++ | ++ |
| 238 | ++++ | ++++ | ++ | +++ |
| 239a | ++++ | ++++ | - | +++ |
| 239b | ++++ | ++++ | + | +++ |
| 240a | +++ | +++ | - | ++ |
| 240b | + | + | - | - |
| 241rac | ++++ | ++++ | ++ | +++ |
| 242rac | ++++ | ++++ | ++ | ++ |
| 243rac | +++ | +++ | - | ++ |
| 244rac | ++++ | ++++ | ++++ | - |
| 245a | ++++ | ++++ | ++ | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 245b | +++ | +++ | - | +++ |
| 246a | ++ | ++ | - | + |
| 246b | ++++ | ++++ | + | +++ |
| 247rac | ++++ | ++++ | +++ | + |
| 248rac | ++++ | ++++ | ++ | +++ |
| 249a | + | + | - | - |
| 249b | ++ | ++ | - | + |
| 250a | ++ | ++ | + | - |
| 250b | +++ | +++ | ++ | + |
| 251a | ++ | ++ | + | - |
| 251b | +++ | +++ | ++ | + |
| 252a | ++ | ++ | + | - |
| 252b | +++ | +++ | ++ | + |
| 253rac | +++ | +++ | - | + |
| 254rac | +++ | ++ | + | + |
| 255rac | ++++ | ++++ | ++ | ++ |
| 256rac | ++++ | ++++ | + | +++ |
| 257rac | ++++ | ++++ | + | +++ |
| 258rac | ++++ | ++++ | ++ | +++ |
| 259 | ++++ | ++++ | ++ | +++ |
| 260 | ++++ | ++++ | + | +++ |
| 261a | ++ | + | - | - |
| 261b | ++++ | ++++ | + | +++ |
| 262a | ++ | + | + | - |
| 262b | ++ | ++ | + | + |
| 263rac | +++ | ++ | ++ | - |
| 264rac | ++++ | ++++ | ++ | +++ |
| 265a | ++++ | ++++ | + | +++ |
| 265b | ++++ | ++++ | ++ | +++ |
| 266a | ++++ | ++++ | ++ | +++ |
| 266b | +++ | +++ | - | +++ |
| 267a | +++ | +++ | ++ | + |
| 267b | ++++ | ++++ | +++ | +++ |
| 268rac | ++++ | ++++ | ++ | +++ |
| 269a | ++++ | ++++ | + | +++ |
| 269b | ++ | ++ | - | + |
| 270a | +++ | +++ | +++ | - |
| 270b | ++++ | ++++ | +++ | + |

(continued)

| *Results of Parp 1/2 assays for selected compounds (DELFIA and Probe Displacement HTRF)* | | | | |
|---|---|---|---|---|
| **Compound** | **PARP1 DELFIA** | **PARP1 HTRF** | **PARP2 HTRF** | **Selectivity** |
| 271rac | ++++ | ++++ | ++ | +++ |
| 272a | - | - | - | - |
| 272b | ++ | ++ | - | + |
| 273a | ++ | ++ | + | - |
| 274a | ++ | ++ | + | - |
| 274b | +++ | ++ | + | + |
| 275rac | ++++ | ++++ | ++ | + |
| 276rac | ++++ | ++++ | ++ | +++ |
| 277a | ++++ | ++++ | + | +++ |
| 277b | +++ | +++ | - | ++ |
| 278rac | +++ | +++ | - | +++ |
| 279a | ++ | ++ | - | + |
| 279b | + | + | - | - |
| 280a | +++ | +++ | + | ++ |
| 280b | ++++ | ++++ | +++ | ++ |
| 281a | ++++ | ++++ | +++ | + |
| 281b | ++ | ++ | ++ | - |
| 282rac | +++ | +++ | ++ | + |
| 283a | ++ | ++ | - | + |
| 283b | ++++ | ++++ | + | +++ |
| 284 | ++++ | ++++ | - | +++ |
| 285a | ++++ | ++++ | ++ | +++ |
| 285b | ++++ | ++++ | - | +++ |
| 286rac | +++ | +++ | - | +++ |
| 287a | +++ | +++ | - | ++ |
| 287b | + | + | - | - |
| 288rac | +++ | +++ | + | +++ |
| 273b | +++ | +++ | ++ | + |
| 289a | ++ | ++ | + | - |
| 289b | ++++ | +++ | ++ | + |
| 290rac | +++ | ++ | + | + |
| 291a | +++ | ++ | + | + |
| 291b | ++ | ++ | + | - |
| 292a | +++ | ++ | + | - |
| 292b | ++++ | ++++ | ++ | +++ |
| 293a | - | - | - | - |
| 293b | ++ | ++ | + | - |
| 294rac | ++ | ++ | - | - |

(continued)

| *Results of Parp 1/2 assays for selected compounds (DELFIA and Probe Displacement HTRF)* | | | | |
|---|---|---|---|---|
| **Compound** | **PARP1 DELFIA** | **PARP1 HTRF** | **PARP2 HTRF** | **Selectivity** |
| 295 | ++++ | ++++ | - | +++ |
| 296rac | ++++ | ++++ | ++ | + |
| 297rac | +++ | +++ | ++ | - |
| 298a | + | + | - | - |
| 298b | +++ | +++ | + | +++ |
| 299 | ++++ | ++++ | - | +++ |
| 300 | ++++ | ++++ | + | +++ |
| 301rac | ++ | ++ | + | - |
| 302 | ++++ | ++++ | - | +++ |
| 303 | ++++ | ++++ | - | +++ |
| 304 | ++++ | +++ | - | +++ |
| 305 | ++++ | ++++ | - | +++ |
| 306a | ++ | ++ | + | + |
| 306b | ++++ | ++++ | ++ | +++ |
| 307rac | +++ | +++ | ++ | - |
| 308a | ++ | ++ | + | + |
| 308b | ++++ | ++++ | ++ | +++ |
| 309a | ++++ | ++++ | - | +++ |
| 309b | +++ | +++ | - | ++ |
| 310 | +++ | ++++ | - | +++ |
| 311 | ++++ | ++++ | + | +++ |
| 312 | ++++ | ++++ | ++ | +++ |
| 313a | +++ | +++ | - | +++ |
| 313b | ++++ | ++++ | + | +++ |
| 314a | ++++ | ++++ | ++ | + |
| 314b | ++++ | ++++ | ++ | +++ |
| 315rac | ++++ | ++++ | ++ | +++ |
| 316a | ++++ | ++++ | ++ | +++ |
| 316b | +++ | +++ | ++ | + |
| 317a | +++ | ++ | + | - |
| 317b | ++++ | ++++ | ++ | +++ |
| 318rac | +++ | +++ | ++ | + |
| 319rac | + | + | + | - |
| 320rac | ++ | ++ | + | - |
| 321rac | ++ | ++ | ++ | - |
| 322a | ++++ | ++++ | - | +++ |
| 322b | ++++ | ++++ | ++ | +++ |
| 323a | ++++ | ++++ | - | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 323b | ++++ | ++++ | +++ | +++ |
| 324rac | ++++ | ++++ | ++ | +++ |
| 325rac | ++++ | ++++ | + | +++ |
| 326a | ++ | ++ | + | + |
| 326b | +++ | ++++ | + | +++ |
| 327a | ++ | ++ | - | + |
| 327b | ++ | ++ | - | - |
| 328a | ++ | ++ | - | + |
| 328b | +++ | +++ | - | +++ |
| 329rac | ++++ | ++++ | ++ | +++ |
| 330 | ++++ | ++++ | +++ | + |
| 331a | ++++ | ++++ | ++ | +++ |
| 331b | +++ | +++ | + | +++ |
| 332a | ++ | ++ | + | + |
| 332b | ++++ | ++++ | + | +++ |
| 333b | +++ | +++ | + | +++ |
| 334rac | ++++ | +++ | + | ++ |
| 335rac | ++ | ++ | - | + |
| 336rac | ++++ | ++++ | +++ | ++ |
| 337a | ++++ | ++++ | ++ | +++ |
| 337b | ++++ | ++++ | ++ | +++ |
| 338a | ++++ | ++++ | ++ | +++ |
| 338b | +++ | +++ | + | ++ |
| 339a | ++++ | ++++ | +++ | + |
| 339b | ++++ | ++++ | ++ | +++ |
| 340a | ++++ | ++++ | ++ | +++ |
| 340b | +++ | +++ | ++ | + |
| 341a | ++++ | ++++ | ++ | +++ |
| 341b | +++ | +++ | ++ | + |
| 342a | ++ | ++ | ++ | - |
| 342b | ++++ | ++++ | +++ | + |
| 333a | ++++ | ++++ | ++ | +++ |
| 343a | ++++ | ++++ | ++ | +++ |
| 343b | ++++ | ++++ | + | +++ |
| 344a | ++++ | ++++ | - | +++ |
| 344b | ++++ | ++++ | + | +++ |
| 345a | ++++ | ++++ | ++ | +++ |
| 345b | +++ | +++ | + | + |

(continued)

| Results of Parp 1/2 assays for selected compounds (DELFIA and Probe Displacement HTRF) | | | | |
|---|---|---|---|---|
| Compound | PARP1 DELFIA | PARP1 HTRF | PARP2 HTRF | Selectivity |
| 346a | ++++ | ++++ | + | +++ |
| 346b | ++ | ++ | - | + |
| 347a | ++++ | ++++ | ++ | +++ |
| 347b | +++ | +++ | + | +++ |
| 348a | +++ | +++ | ++ | - |
| 348b | ++ | ++ | ++ | - |
| 349a | ++++ | ++++ | - | +++ |
| 349b | ++++ | ++++ | ++ | +++ |
| 350a | ++++ | ++++ | ++ | +++ |
| 350b | ++++ | ++++ | +++ | ++ |
| 351a | ++++ | ++++ | + | +++ |
| 351b | ++++ | ++++ | ++ | +++ |
| 352a | ++++ | ++++ | +++ | +++ |
| 352b | ++++ | ++++ | ++ | ++ |
| 353a | ++++ | ++++ | ++ | +++ |
| 353b | +++ | +++ | + | +++ |
| 354a | ++++ | ++++ | +++ | + |
| 354b | ++++ | ++++ | ++ | +++ |
| 355a | ++ | ++ | + | - |
| 355b | ++++ | ++++ | ++ | +++ |
| 356a | ++++ | ++++ | + | +++ |
| 356b | ++ | ++ | + | + |
| 357a | ++++ | ++++ | ++ | +++ |
| 357b | +++ | ++++ | + | +++ |
| 358rac | ++++ | ++++ | ++ | +++ |
| 359a | +++ | +++ | + | ++ |
| 359b | +++ | ++ | - | + |
| 360rac | ++++ | ++++ | ++ | +++ |
| 361 | ++++ | ++++ | ++ | +++ |
| 362rac | ++++ | ++++ | + | +++ |
| 363a | ++++ | ++++ | + | +++ |
| 363b | ++++ | ++++ | + | +++ |
| 364a | ++ | ++ | - | + |
| 364b | ++++ | ++++ | ++ | ++ |
| 365rac | ++++ | ++++ | ++ | +++ |
| 315a | ++++ | ++++ | ++ | +++ |
| 315b | ++++ | ++++ | ++ | +++ |
| 366a | +++ | +++ | - | +++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 366b | ++++ | ++++ | ++ | ++ |
| 367a | ++++ | ++++ | ++ | +++ |
| 367b | ++++ | ++++ | + | +++ |
| 368 | ++++ | ++++ | - | +++ |
| 369 | ++++ | ++++ | + | +++ |
| 370a | ++++ | ++++ | ++ | +++ |
| 370b | ++++ | ++++ | +++ | ++ |
| 371a | ++++ | ++++ | +++ | +++ |
| 371b | +++ | +++ | + | ++ |
| 372a | ++++ | ++++ | ++ | + |
| 372b | ++++ | ++++ | +++ | +++ |
| 324a | ++++ | ++++ | - | +++ |
| 324b | ++++ | ++++ | ++ | +++ |
| 373 | ++++ | ++++ | + | +++ |
| 374a | +++ | +++ | ++ | + |
| 374b | ++++ | ++++ | +++ | ++ |
| 375 | ++++ | ++++ | ++ | + |
| 376 | ++++ | ++++ | ++ | +++ |
| 377a | +++ | +++ | - | +++ |
| 377b | ++++ | ++++ | + | +++ |
| 378a | ++++ | ++++ | ++ | +++ |
| 378b | ++++ | ++++ | + | +++ |
| 379 | ++++ | ++++ | ++ | +++ |
| 380 | ++++ | ++++ | - | +++ |
| 381 | ++++ | ++++ | + | +++ |
| 382a | ++ | ++ | - | + |
| 382b | ++++ | ++++ | ++ | +++ |
| 383 | ++++ | ++++ | ++ | + |
| 384 | ++++ | ++++ | ++ | + |
| 385a | ++++ | ++++ | + | +++ |
| 385b | ++++ | ++++ | +++ | +++ |
| 386a | ++++ | ++++ | ++ | +++ |
| 386b | ++++ | ++++ | + | +++ |
| 387 | ++++ | ++++ | ++ | +++ |
| 388 | ++++ | ++++ | ++ | +++ |
| 389 | ++++ | ++++ | ++ | +++ |
| 390a | ++++ | ++++ | ++ | +++ |
| 390b | ++++ | ++++ | +++ | ++ |

(continued)

| Results of Parp 1/2 assays for selected compounds (DELFIA and Probe Displacement HTRF) | | | | |
|---|---|---|---|---|
| **Compound** | **PARP1 DELFIA** | **PARP1 HTRF** | **PARP2 HTRF** | **Selectivity** |
| 391rac | ++++ | ++++ | + | +++ |
| 392rac | ++ | ++ | - | + |
| 393rac | ++++ | ++++ | + | +++ |
| 394 | ++++ | ++++ | +++ | + |
| 395a | ++++ | ++++ | ++ | + |
| 395b | ++++ | ++++ | +++ | ++ |
| 396a | ++++ | ++++ | ++ | +++ |
| 396b | ++++ | ++++ | ++ | +++ |
| 397a | ++++ | ++++ | ++ | +++ |
| 397b | ++++ | ++++ | +++ | +++ |
| 398a | ++++ | ++++ | + | +++ |
| 398b | ++++ | ++++ | ++ | +++ |
| 399a | ++++ | ++++ | ++ | +++ |
| 399b | ++++ | ++++ | +++ | + |
| 400rac | ++++ | ++++ | ++ | +++ |
| 401a | ++++ | ++++ | +++ | +++ |
| 401b | ++++ | ++++ | +++ | + |
| 402a | ++++ | ++++ | ++ | +++ |
| 402b | ++++ | ++++ | +++ | +++ |
| 403rac | ++++ | ++++ | +++ | +++ |
| 404a | +++ | +++ | + | +++ |
| 404b | ++++ | ++++ | + | +++ |
| 405rac | ++++ | ++++ | ++ | +++ |
| 415rac | +++ | ++++ | - | +++ |
| 406rac | ++++ | ++++ | - | +++ |
| 407a | +++ | ++++ | ++ | ++ |
| 407b | ++++ | ++++ | ++++ | + |
| 408a | ++++ | ++++ | ++ | +++ |
| 408b | +++ | +++ | + | + |
| 409a | ++++ | ++++ | + | +++ |
| 409b | ++++ | ++++ | ++ | +++ |
| 410a | ++++ | ++++ | ++ | +++ |
| 410b | ++++ | ++++ | ++ | +++ |
| 411 | ++++ | ++++ | ++ | +++ |
| 412rac | ++ | ++ | - | + |
| 413rac | ++++ | ++++ | ++ | +++ |
| 414rac | +++ | +++ | ++ | ++ |

**TABLE 2**

| Results of Parp 1/2 assays for selected compounds (NanoBRET) | | | |
|---|---|---|---|
| **Compound** | **PARP1 NanoBRET** | **PARP2 NanoBRET** | **Selectivity** |
| 10b | ++++ | + | +++ |
| 64b | ++++ | ++ | +++ |
| 68a | ++++ | + | +++ |
| 72b | ++++ | + | +++ |
| 86b | ++++ | +++ | + |
| 129b | ++++ | ++ | +++ |
| 137b | ++++ | ++ | +++ |
| 144rac | ++++ | - | +++ |
| 136b | ++++ | ++ | +++ |
| 150rac | +++ | ++ | + |
| 151a | ++++ | - | +++ |
| 157rac | ++++ | - | +++ |
| 154b | ++++ | ++ | +++ |
| 170rac | ++++ | +++ | + |
| 174rac | ++++ | + | +++ |
| 174a | ++++ | + | +++ |
| 174b | ++++ | ++ | +++ |
| 175rac | ++++ | + | +++ |
| 180b | ++++ | ++ | +++ |
| 183b | ++++ | + | +++ |
| 184b | ++++ | ++ | +++ |
| 186b | ++++ | +++ | +++ |
| 192 | ++++ | + | +++ |
| 196a | ++++ | ++ | +++ |
| 197a | ++++ | ++ | +++ |
| 198rac | ++++ | ++ | + |
| 223b | ++++ | ++ | +++ |
| 228 | ++++ | - | +++ |
| 239b | ++++ | + | +++ |
| 255rac | ++++ | + | +++ |
| 258rac | ++++ | ++ | +++ |
| 259 | ++++ | + | +++ |
| 260 | ++++ | - | +++ |
| 285a | ++++ | ++ | +++ |
| 295 | +++ | - | ++ |
| 299 | +++ | - | +++ |
| 300 | ++++ | - | +++ |
| 302 | +++ | - | +++ |

(continued)

| Results of Parp 1/2 assays for selected compounds (NanoBRET) | | | |
|---|---|---|---|
| **Compound** | **PARP1 NanoBRET** | **PARP2 NanoBRET** | **Selectivity** |
| 312 | ++ | + | ++ |
| 313b | ++++ | + | +++ |
| 314b | ++++ | ++ | +++ |
| 315rac | ++++ | ++ | +++ |
| 316a | ++++ | ++ | +++ |
| 322b | ++++ | ++ | +++ |
| 323a | ++++ | - | +++ |
| 323b | ++++ | ++ | +++ |
| 324rac | ++++ | + | +++ |
| 329rac | ++++ | + | +++ |
| 330 | +++ | ++ | + |
| 337a | ++++ | + | +++ |
| 337b | ++++ | ++ | +++ |
| 339a | ++++ | +++ | + |
| 343a | ++++ | + | +++ |
| 344b | ++++ | - | +++ |
| 350b | ++++ | +++ | +++ |
| 352a | ++++ | ++ | +++ |
| 353a | ++++ | ++ | +++ |
| 354a | ++++ | +++ | ++ |
| 354b | ++++ | ++ | +++ |
| 357a | ++++ | + | +++ |

**Key**

DELFIA, Probe Displacement HTRF and NanoBRET assay categories:

- indicates $IC_{50}$ or $K_d$ value above 10 μM

+ indicates $IC_{50}$ or $K_d$ value above 1 μM up to 10 μM

++ indicates $IC_{50}$ or $K_d$ value above 100 nM up to 1 μM

+++ indicates $IC_{50}$ or $K_d$ value above 10 nM up to 100 nM

++++ indicates $IC_{50}$ or $K_d$ value of 10 nM or less

Selectivity categories:

- indicates a value of less than 10

+ indicates a value of 10 to less than 50

++ indicates a value of 50 to less than 100

+++ indicate a value of at least 100

The selectivity values relate to the selectivity preference of PARP1 over PARP2. They are calculated from the ratio of $K_d$ values for PARP1 and PARP2 inhibition as $K_d$ (PARP2) / $K_d$ (PARP1).

The present disclosure provides the following Clauses:

Clause 1. A PARP1 inhibitor compound for use in medicine, which compound comprises the following structure:

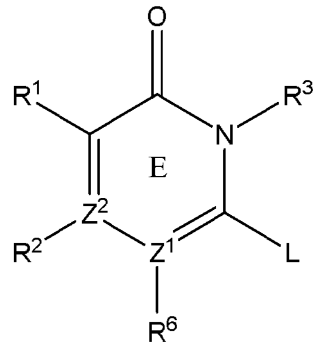

wherein $R^1$ is selected from H and a substituted or unsubstituted organic group; $R^2$ may be present or absent and is independently selected from H and a substituted or unsubstituted organic group; $R^1$ and $R^2$ may together form a ring; $R^3$ is independently selected from H and a substituted or unsubstituted organic group; $R^6$ may be present or absent and is independently selected from H and a substituted or unsubstituted organic group; $Z^1$ and $Z^2$ are independently selected from C and N; and L comprises a group having the following structure:

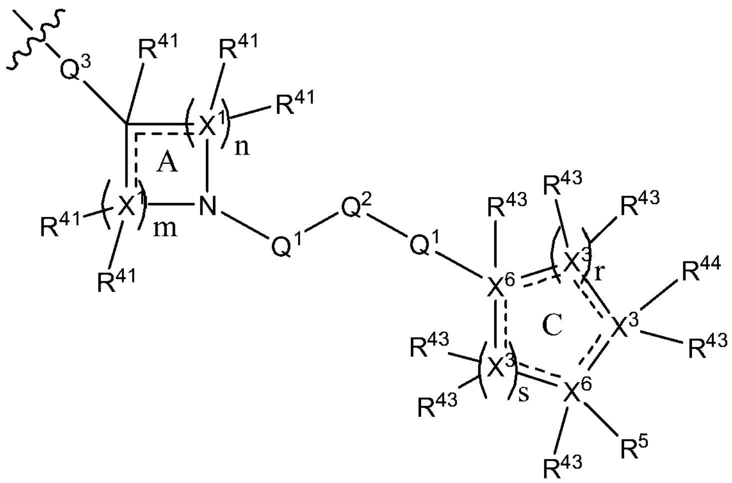

wherein, n is a number independently selected from 0, 1, 2, 3, 4, 5 and 6; m is a number independently selected from 0, 1, 2, 3, 4, 5 and 6; and m + n is a number selected from 2, 3, 4, 5, and 6; preferably wherein both n and m are at least 1;

wherein, r is a number independently selected from 0, 1, 2, 3, 4, and 5; s is a number independently selected from 0, 1, 2, 3, 4, and 5; and r + s is a number selected from 1, 2, 3, 4, and 5; preferably wherein both r and s are at least 1;

and wherein, each $X^1$ may be the same or different and is independently selected from C, N, O and S; each $X^3$ may be the same or different and is independently selected from C, N, O and S; each $X^6$ may be the same or different and is independently selected from C and N; each $R^{41}$ may be the same or different, and may be present or absent, and is selected from H and a substituted or unsubstituted organic group; each $R^{43}$ may be the same or different, and may be present or absent, and is selected from H and a substituted or unsubstituted organic group; and $R^{44}$ may be present or absent, and is selected from H and a substituted or unsubstituted organic group;

and wherein, the dotted lines indicate that: the ring A may comprise single bonds, or a combination of both single and double bonds, and may be aliphatic or aromatic; and independently the ring C may comprise single bonds, or a combination of both single and double bonds, and may be aliphatic or aromatic;

and wherein, $R^5$ is selected from H and a substituted or unsubstituted organic group;

and wherein each $Q^1$ may be the same or different, and may be present or absent, and comprises a group independently selected from the following structures:

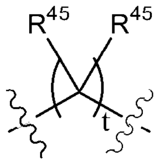
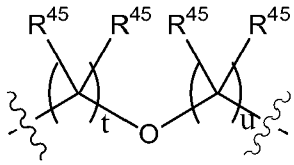
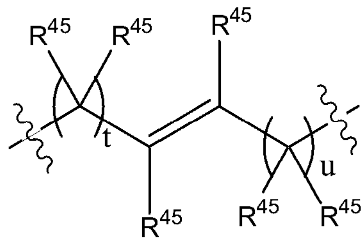

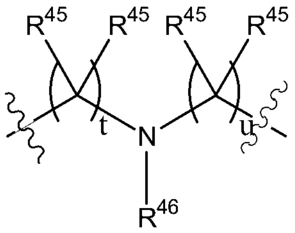
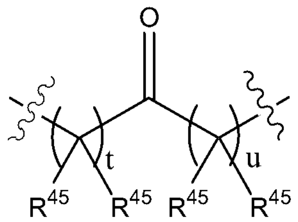

wherein t is independently a number selected from 0, 1, 2, 3, 4 and 5; u is independently a number selected from 0, 1, 2, 3, 4 and 5; and t + u is a number selected from 0, 1, 2, 3, 4, 5 and 6 (preferably a number selected from 0, 1, 2, and 3); each $R^{45}$ may be the same or different and is independently selected from H and a substituted or unsubstituted organic group; and $R^{46}$ is selected from H and a substituted or unsubstituted organic group;
and wherein $Q^2$ may be present or absent and is a group having the following structure:

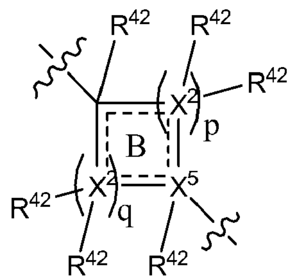

wherein, p is a number independently selected from 0, 1, 2, 3, 4, 5 and 6; q is a number independently selected from 0, 1, 2, 3, 4, 5 and 6; and p + q is a number selected from 2, 3, 4, 5, and 6;
and wherein, each $X^2$ may be the same or different and is independently selected from C, N, 0 and S; $X^5$ is independently selected from C and N; each $R^{42}$ may be the same or different, and may be present or absent, and is selected from H and a substituted or unsubstituted organic group;
and wherein, the dotted lines indicate that: the ring B may comprise single bonds, or a combination of both single and double bonds, and may be aliphatic or aromatic;
and wherein $Q^3$ may be present or absent and comprises a group independently selected from the following structures:

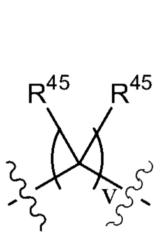
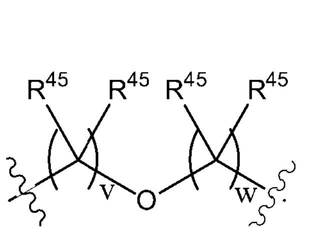
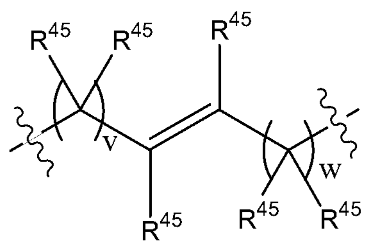

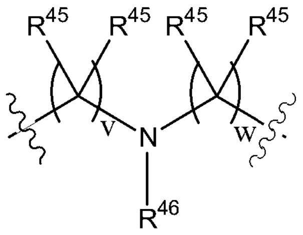

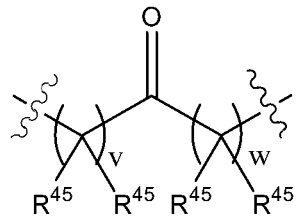

wherein v is independently a number selected from 0, 1, 2, 3, 4 and 5; w is independently a number selected from 0, 1, 2, 3, 4 and 5; and v + w is a number selected from 0, 1, 2, 3, 4, 5 and 6 (preferably a number selected from 0, 1, and 2).

Clause 2. A compound according to Clause 1, wherein $Q^3$ is absent or is a group independently selected from the following structures:

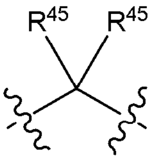

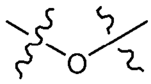

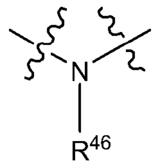

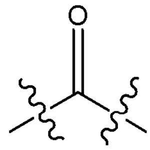

wherein each $R^{45}$ may be the same or different and is independently selected from H and a substituted or unsubstituted organic group; and $R^{46}$ is selected from H and a substituted or unsubstituted organic group.

Clause 3. A compound according to any preceding Clause, wherein $R^{41}$, $R^{42}$, $R^{43}$, $R^{44}$ and $R^{45}$ are each independently as defined on pages 21 to 25 of the description, i.e. selected from H and a group selected from the following groups: deuterium; - a halogen; - a nitrile group; - a substituted or unsubstituted linear or branched $C_1$-$C_6$ alkyl group; - a substituted or unsubstituted linear or branched $C_1$-$C_6$ alkyl-aryl group; - a substituted or unsubstituted linear or branched $C_1$-$C_6$ halogenated alkyl group; - $-NH_2$ or a substituted or unsubstituted linear or branched primary secondary or tertiary $C_1$-$C_6$ amine group; - a substituted or unsubstituted amino-aryl group; - a substituted or unsubstituted cyclic amine or amido group; - a substituted or unsubstituted cyclic $C_3$-$C_8$ alkyl group; - an -OH group or a substituted or unsubstituted linear or branched $C_1$-$C_6$ alcohol group; - a substituted or unsubstituted linear or branched carbonyl group; - a substituted or unsubstituted linear or branched $C_1$-$C_6$ carboxylic acid ester group; - a substituted or unsubstituted linear or branched $C_1$-$C_6$ amide group; - a substituted or unsubstituted linear or branched $C_1$-$C_7$ amino carbonyl group; - a substituted or unsubstituted linear or branched $C_1$-$C_7$ alkoxy or aryloxy group; - a substituted or unsubstituted linear or branched aminoalkoxy group; - a substituted or unsubstituted sulphonyl group; - a substituted or unsubstituted aminosulphonyl group; - a substituted or unsubstituted aromatic group;

- a saturated or unsaturated, substituted or unsubstituted, heterocyclic group including an aromatic heterocyclic group and/or a non-aromatic heterocyclic group;
- where there are two R groups attached to the same atom, they may together form a group which is double bonded to that atom, (such as a carbonyl group (=O) or an alkene group ($=C(R')_2$) wherein each R' group is the same or different and is H or an organic group, preferably H or a straight or branched $C_1$-$C_6$ alkyl group);
- wherein, a pair of $R^{41}$ groups attached to different atoms may together form a ring with ring A atoms, and/or a pair of $R^{42}$ groups attached to different atoms may together form a ring with ring B atoms, optionally wherein each of the pair of $R^{41}$ groups and/or pair of $R^{42}$ groups independently comprises $(X^7)_{1\text{ or }2}$, wherein each $X^7$ may be the same or different and is independently selected from C, N, O and S; and wherein each $X^7$ is independently unsubstituted or (i) independently substituted with H or an organic group selected from a $C_1$-$C_6$ alkyl group, a $C_1$-$C_6$ halogenated alkyl group, a halogen such as F, or hydroxyl, when $X^7$ is C; and (ii) independently substituted with H or an organic group selected from a $C_1$-$C_6$ alkyl group, a $C_1$-$C_6$ halogenated alkyl group, or a $C_1$-$C_6$ amide group, when $X^7$ is N; and
- wherein groups $R^5$ and $R^{44}$ may together form a ring with atoms $X^6$ and $X^3$ of ring C to which they are attached, optionally wherein the $R^5$ and $R^{44}$ groups together comprise $(X^8)_{3,\ 4\text{ or }5}$, wherein each $X^8$ may be the same or different and is independently selected from C, N, O and S; and wherein each $X^8$ is independently unsubstituted or (i) independently substituted with H or an organic group selected from a $C_1$-$C_6$ alkyl group, a $C_1$-$C_6$ halogenated alkyl group, a halogen such as F, or hydroxyl, when $X^8$ is C; and (ii) independently substituted with H or an organic group selected from a $C_1$-$C_6$ alkyl group, a $C_1$-$C_6$ halogenated alkyl group, or a $C_1$-$C_6$ amide group, when $X^8$ is N.

Clause 4. A compound according to Clause 3, wherein $R^{41}$, $R^{42}$, $R^{43}$ and $R^{44}$ are each independently selected from H, deuterium, a halogen (such as -F, -Cl, -Br, and -I, preferably F or Cl), a nitrile group, a substituted or unsubstituted $C_1$-$C_6$ alkyl group, a substituted or unsubstituted linear or branched $C_1$-$C_6$ halogenated alkyl group (preferably $CF_3$ or $CHF_2$), a cyclopropyl group, an -OH group or a substituted or unsubstituted linear or branched $C_1$-$C_6$ alcohol group, a substituted or unsubstituted linear or branched $C_1$-$C_7$ amino carbonyl group (such as -NH-CO-Me), an -$NH_2$ group or a substituted or unsubstituted $C_1$-$C_6$ amino group and a substituted or unsubstituted $C_1$-$C_6$ alkoxy group; wherein, when a pair of $R^{41}$ groups attached to different atoms together forms a ring with ring A atoms, and/or a pair of $R^{42}$ groups attached to different atoms together forms a ring with ring B atoms, each of the pair of $R^{41}$ groups and/or pair of $R^{42}$ groups independently comprises -$CH_2$- or -$CH_2CH_2$-; and wherein when groups $R^5$ and $R^{44}$ together form a ring with atoms of ring C, $R^5$ and $R^{44}$ together comprise - CH=CH-CH=CH- or -NH-CO-NH-.

Clause 5. A compound according to Clause 3 or Clause 4, wherein $R^{45}$ is selected from H, a halogen (such as -F, -Cl, -Br, and -I, preferably -F), a substituted or unsubstituted $C_1$-$C_6$ alkyl group, a substituted or unsubstituted linear or branched $C_1$-$C_6$ halogenated alkyl group (preferably $CF_3$), an -$NH_2$ group or a substituted or unsubstituted $C_1$-$C_6$ amino group, an -OH group or a substituted or unsubstituted linear or branched $C_1$-$C_6$ alcohol group and a substituted or unsubstituted $C_1$-$C_6$ alkoxy group.

Clause 6. A compound according to any preceding Clause, wherein $R^{46}$ is as defined on pages 26 to 28 of the description, i.e. is selected from H and a group selected from the following groups:

- a substituted or unsubstituted linear or branched $C_1$-$C_6$ alkyl group; - a substituted or unsubstituted linear or branched $C_1$-$C_6$ alkyl-aryl group; - a substituted or unsubstituted linear or branched $C_1$-$C_6$ halogenated alkyl group; - a substituted or unsubstituted cyclic amine or amido group; - a substituted or unsubstituted cyclic $C_3$-$C_8$ alkyl group; - a substituted or unsubstituted linear or branched $C_2$-$C_6$ alcohol group; - a substituted or unsubstituted linear or branched $C_2$-$C_6$ carboxylic acid group; - a substituted or unsubstituted linear or branched carbonyl group; - a substituted or unsubstituted linear or branched $C_1$-$C_6$ carboxylic acid ester group; - a substituted or unsubstituted linear or branched $C_1$-$C_6$ amide group; - a substituted or unsubstituted sulphonyl group; - a substituted or unsubstituted aromatic group; and - a substituted or unsubstituted saturated or unsaturated, substituted or unsubstituted, heterocyclic group including an aromatic heterocyclic group and/or a non-aromatic heterocyclic group.

Clause 7. A compound according to Clause 6, wherein $R^{46}$ is selected from H, a substituted or unsubstituted $C_1$-$C_6$ alkyl group or a substituted or unsubstituted linear or branched $C_1$-$C_6$ halogenated alkyl group.

Clause 8. A compound according to any preceding Clause, wherein ring A of the group L comprises any of the following structures:

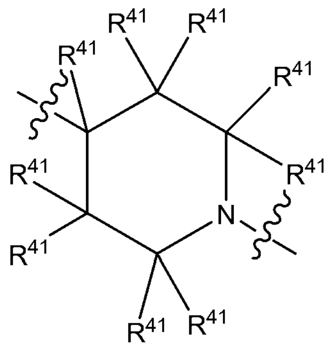
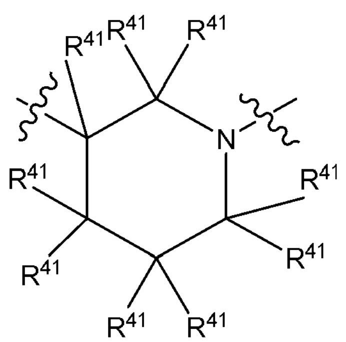
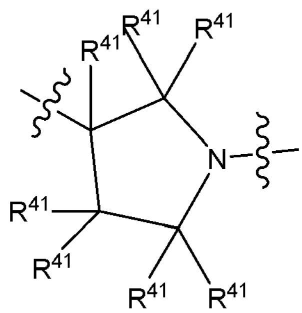

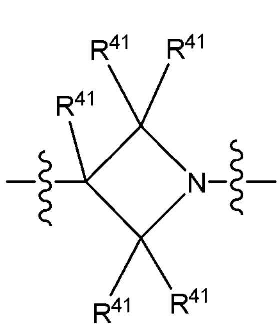
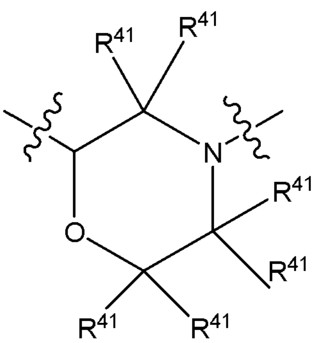
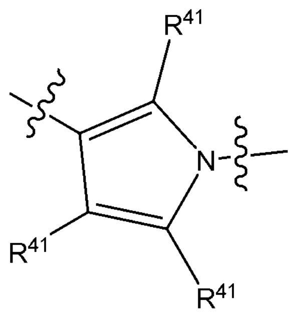
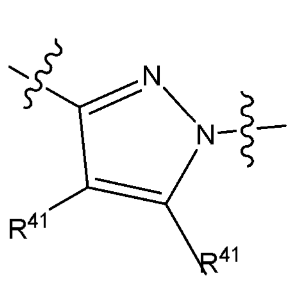

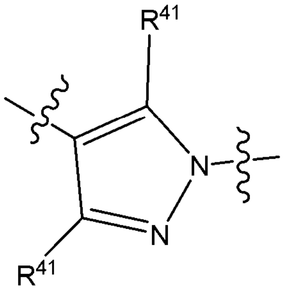
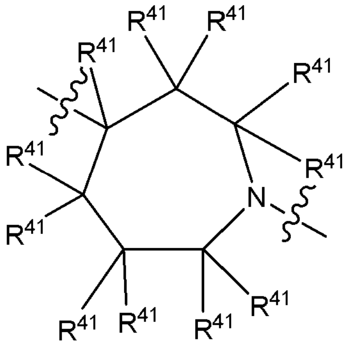
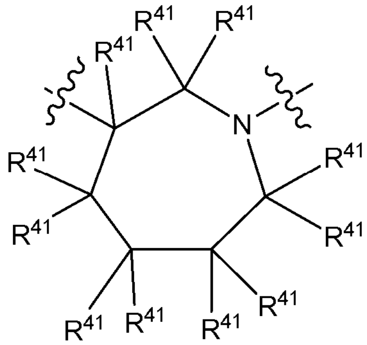

wherein $R^{41}$ is as defined in any preceding Clause.

Clause 9. A compound according to Clause 8, wherein ring A of the group L comprises any of the following structures:

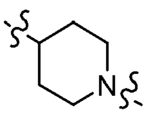
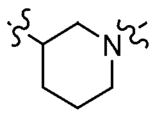
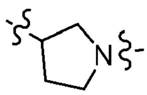
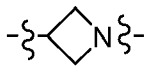
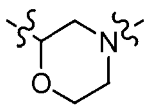
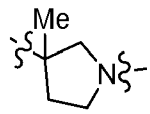
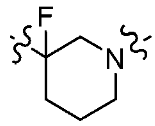
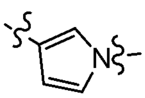
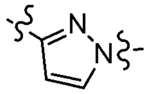
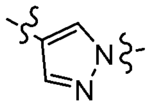
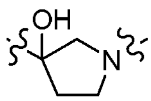
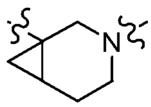
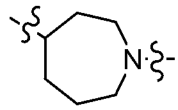
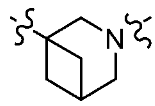
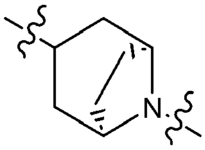
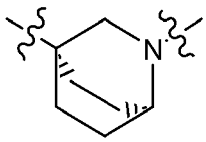
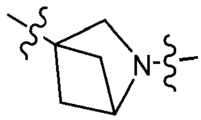
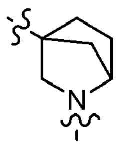
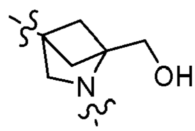
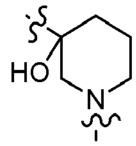
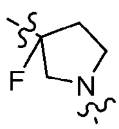
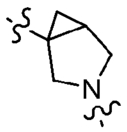
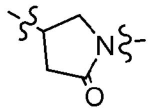
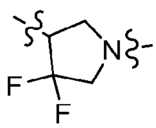 .

Clause 10. A compound according to any preceding Clause, wherein ring C of the group L comprises any of the following structures:

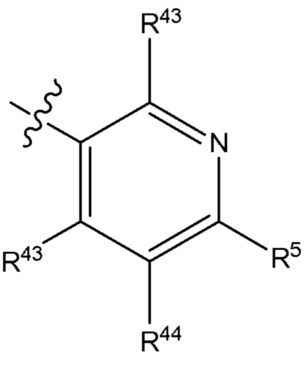
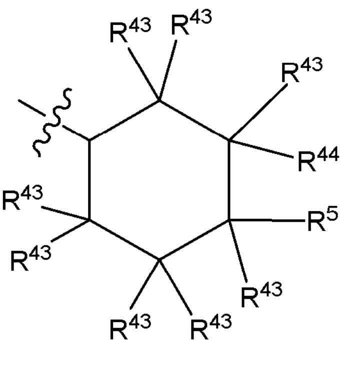
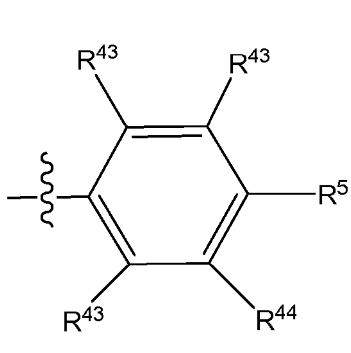

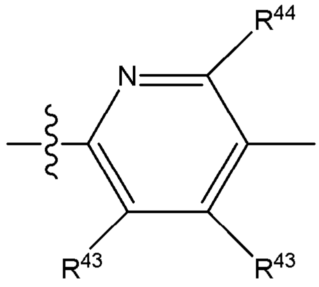
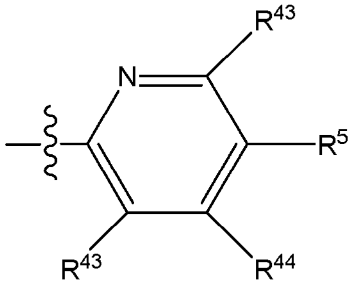
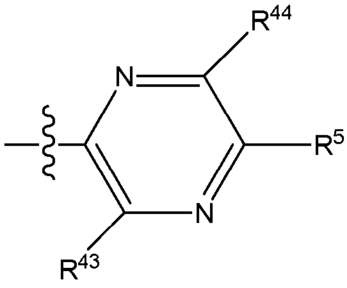

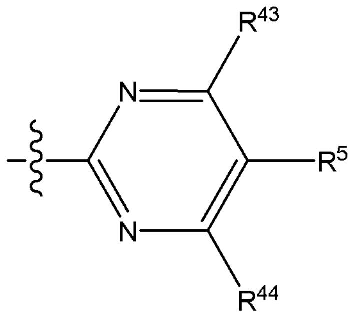
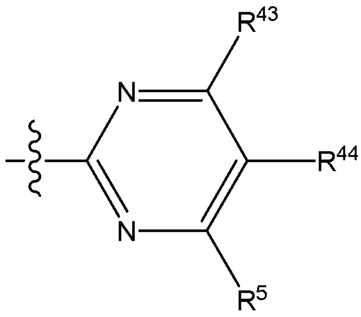
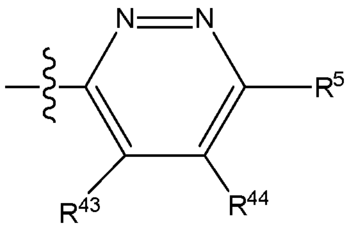

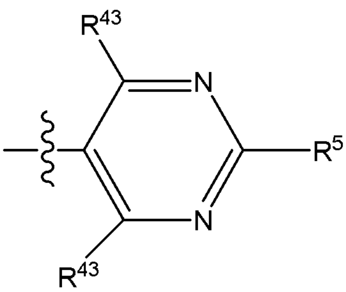
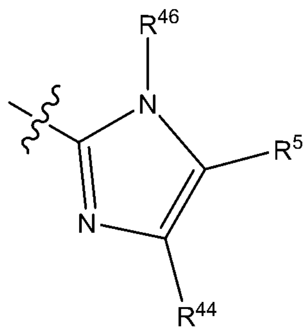
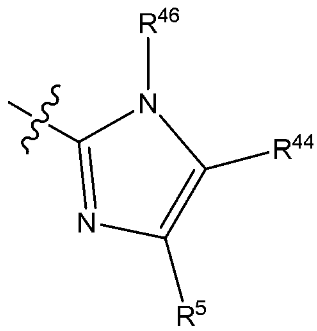

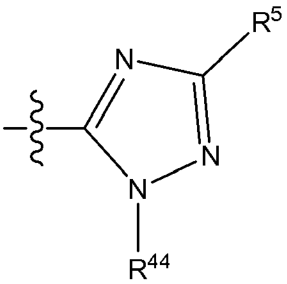
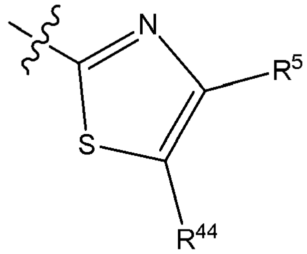
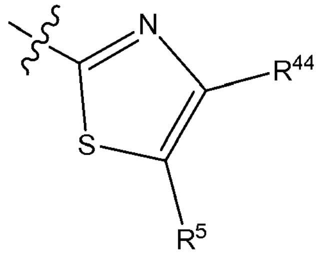

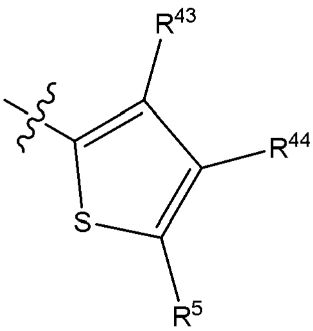
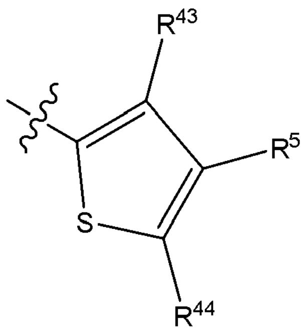
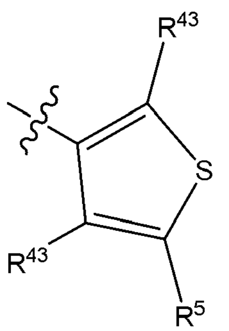

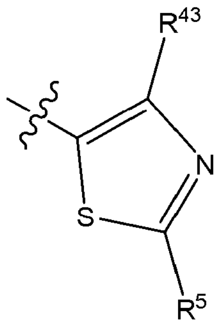

wherein $R^5$, $R^{43}$, $R^{44}$ and $R^{46}$ are as defined in any preceding Clause.

Clause 11. A compound according to Clause 10, wherein ring C of the group L comprises any of the following structures:

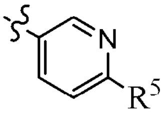 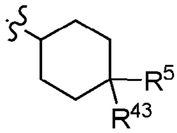 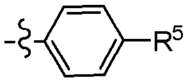 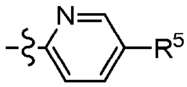

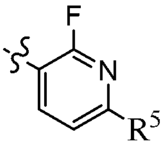 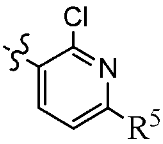 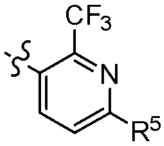 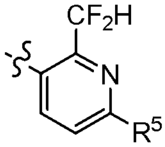 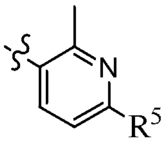

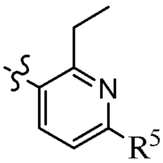 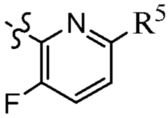 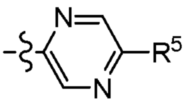 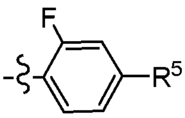 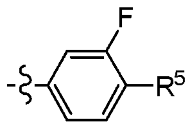

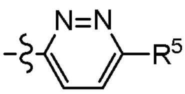 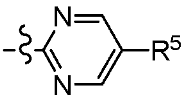 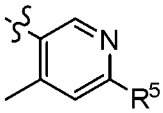 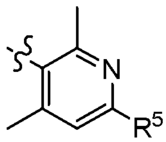

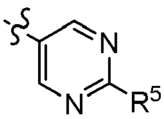 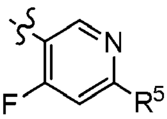 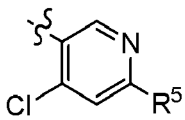 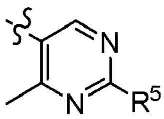

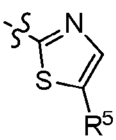 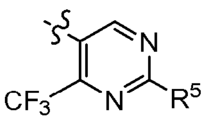 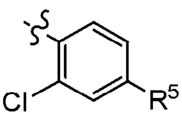 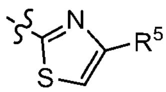 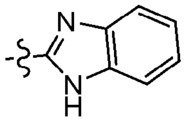

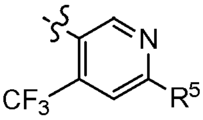 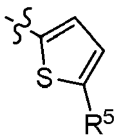 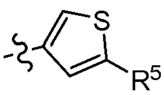 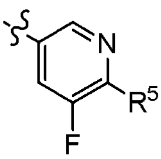 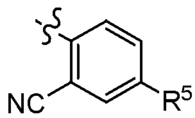

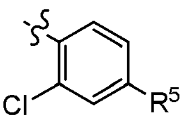 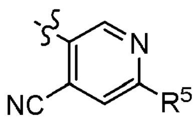 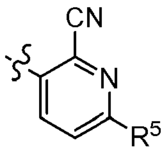 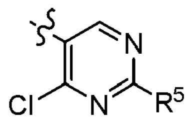 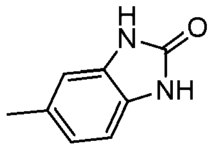

wherein $R^5$ and $R^{43}$ are as defined in Clause 10.

Clause 12. A compound according to any preceding Clause, wherein $R^5$ is a substituted or unsubstituted organic group.

Clause 13. A compound according to any of Clauses 1 to 11, wherein the group $R^5$ is selected from H, -F, -Cl, -Br, -I, -CN, $-CONR^{51}R^{51}$, $-NR^{51}COR^{52}$, $-SO_2NR^{51}R^{51}$, $-NR^{51}SO_2R^{53}$, $-O-CR^{52}R^{52}R^{52}$, $-CR^{52}R^{52}NR^{51}R^{51}$ and any of the following structures:

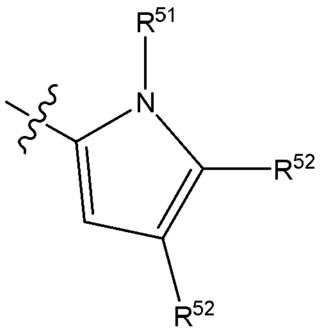 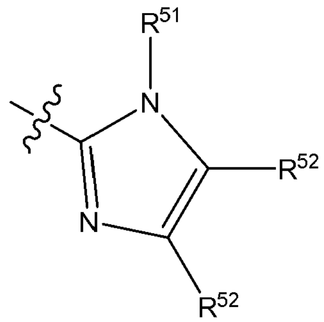 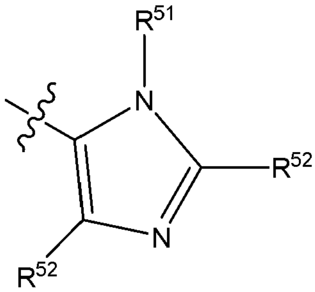

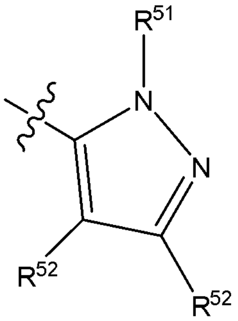 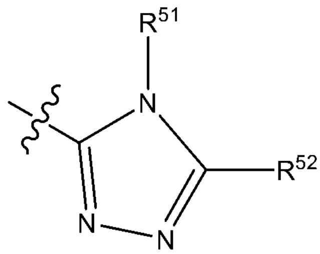 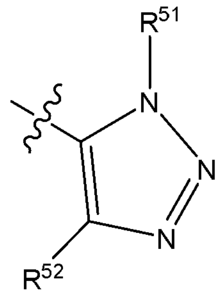

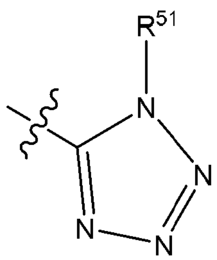 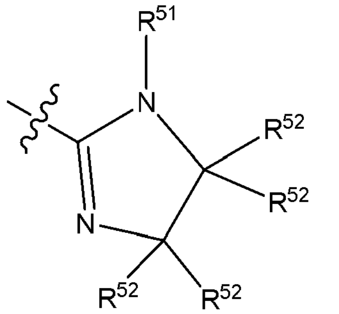

wherein, each of $R^{51}$, $R^{52}$ and $R^{53}$ may be the same or different and are independently selected from H and a substituted or unsubstituted organic group.

Clause 14. A compound according to Clause 13, wherein the group $R^5$ is selected from -F, -Cl, - CN, $-CONH_2$, -CONHMe, -CONHEt, $-CONMe_2$, -CONHCOMe, $-CONHCH_2-CH_2OMe$, $-CONH-CH_2-CH_2F$, $-CONH-CH_2-CF_3$, $-CONH-CH_2-CHF_2$, $-OCHF_2$, -NHCOMe, $-NHSO_2Me$, $-SO_2NHMe$, - $CONHSO_2Me$,

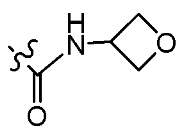 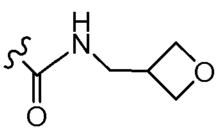 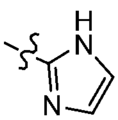 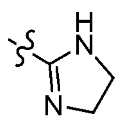 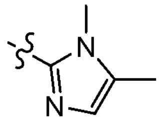 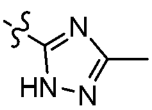

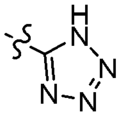 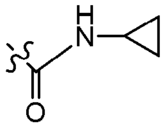 and 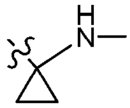 .

Clause 15. A compound according to any preceding Clause, wherein $Q^2$ is present and each $Q^1$ is independently absent or is a group independently selected from the following structures:

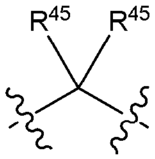 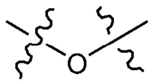 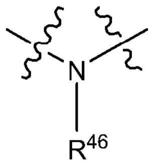 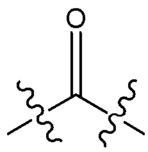

wherein each $R^{45}$ may be the same or different and is independently selected from H and a substituted or unsubstituted organic group; and $R^{46}$ is selected from H and a substituted or unsubstituted organic group.
Clause 16. A compound according to Clause 15, wherein group $Q^2$ of the group L is present in which $X^5$ is N.
Clause 17. A compound according to Clause 16, wherein $Q^2$ of the group L comprises any of the following structures:

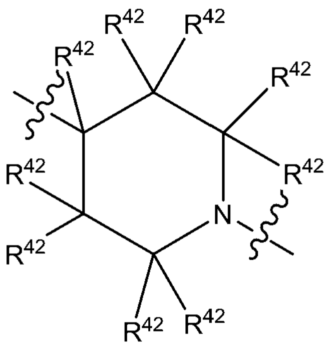 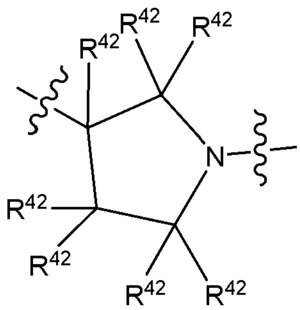 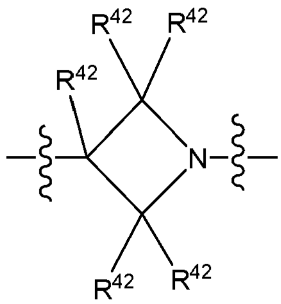

wherein $R^{42}$ is as defined in any preceding Clause.
Clause 18. A compound according to Clause 17, wherein $Q^2$ of the group L comprises any of the following structures:

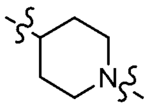 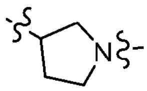 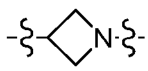 .

Clause 19. A compound according to any of Clauses 15 to 18, wherein the group L comprises a group having the following structure:

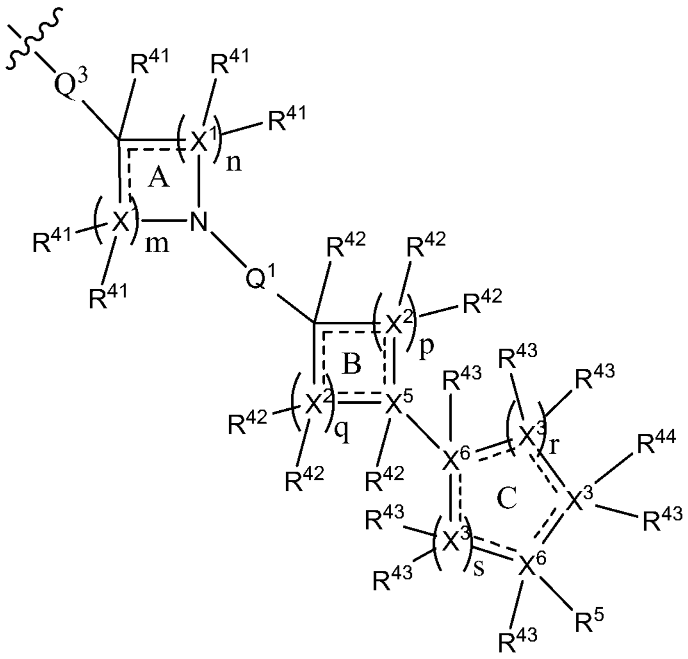

wherein $Q^1$ is absent or is a group as defined in Clause 3, $Q^3$ is absent or is a group as defined in Clause 2, and n, m, p, q, r, s, $X^1$, $X^2$, $X^3$, $X^5$, $X^6$, $R^{41}$, $R^{42}$, $R^{43}$, $R^{44}$, $R^5$, and rings A, B, and C are as defined in any preceding Clause.

Clause 20. A compound according to Clause 19, wherein the group L comprises a group having the following structure:

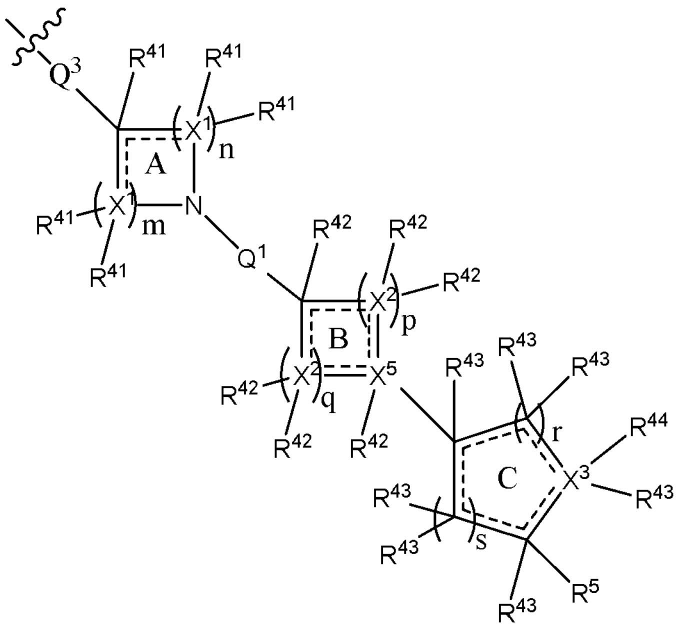

wherein n, m, p, q, r, s, $X^1$, $X^2$, $X^3$, $X^5$, $R^{41}$, $R^{42}$, $R^{43}$, $R^{44}$, $R^5$, $Q^1$, $Q^3$ and rings A, B, and C are as defined in Clause 19.

Clause 21. A compound according to Clause 20, wherein the group L comprises a group having the following structure:

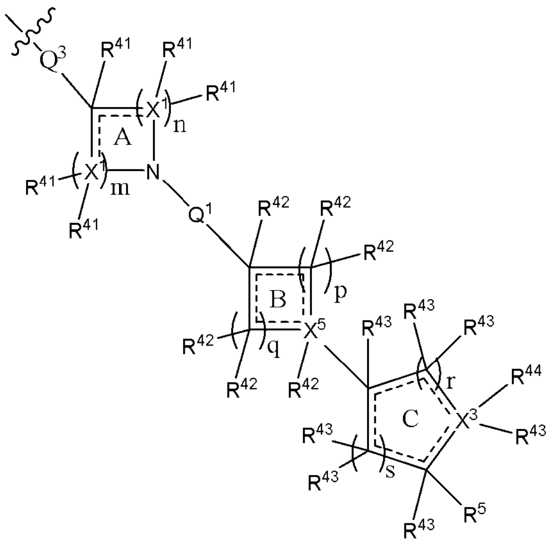

wherein n, m, p, q, r, s, $X^1$, $X^3$, $X^5$, $R^{41}$, $R^{42}$, $R^{43}$, $R^{44}$, $R^5$, $Q^1$, $Q^3$ and rings A, B, and C are as defined in Clause 20.

Clause 22. A compound according to Clause 21, wherein the group L comprises a group having the following structure:

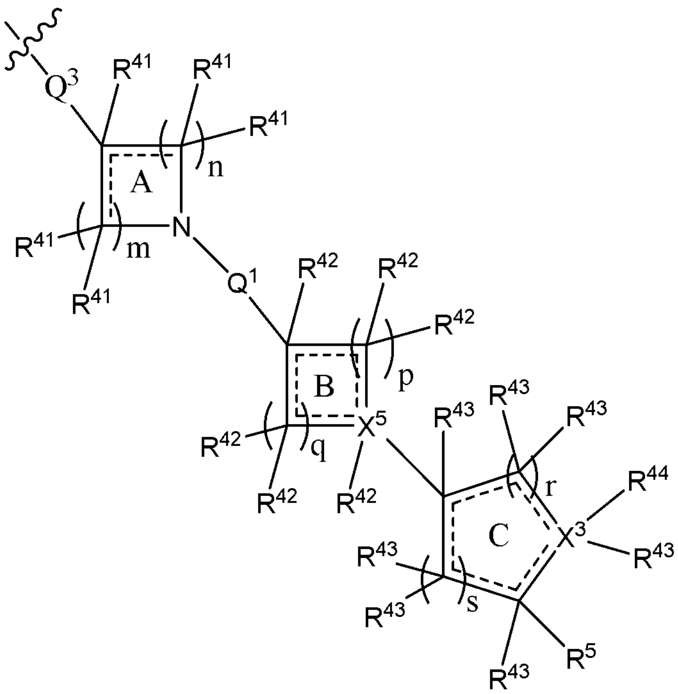

wherein n, m, p, q, r, s, $X^3$, $X^5$, $R^{41}$, $R^{42}$, $R^{43}$, $R^{44}$, $R^5$, and rings A, B, and C are as defined in Clause 21.

Clause 23. A compound according to Clause 22, wherein the group L comprises a group having the following structure:

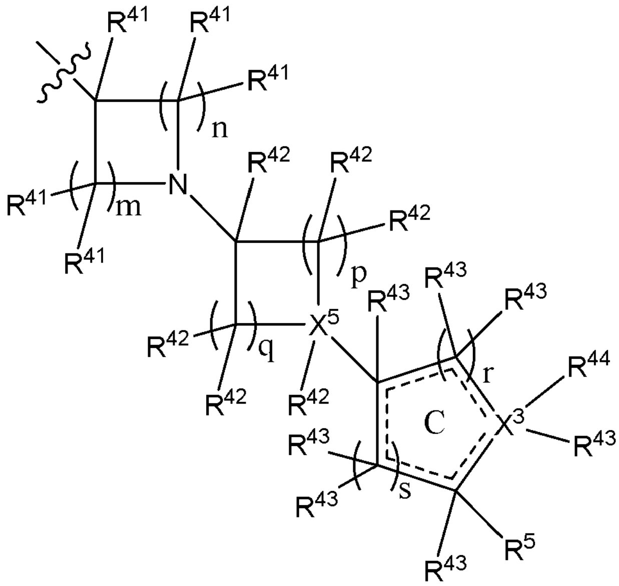

wherein n, m, p, q, r, s, $X^3$, $X^5$, $R^{41}$, $R^{42}$, $R^{43}$, $R^{44}$, $R^5$, and ring C, are as defined in Clause 22. Clause 24. A compound according to Clause 19, wherein the group L comprises a group having the following structure:

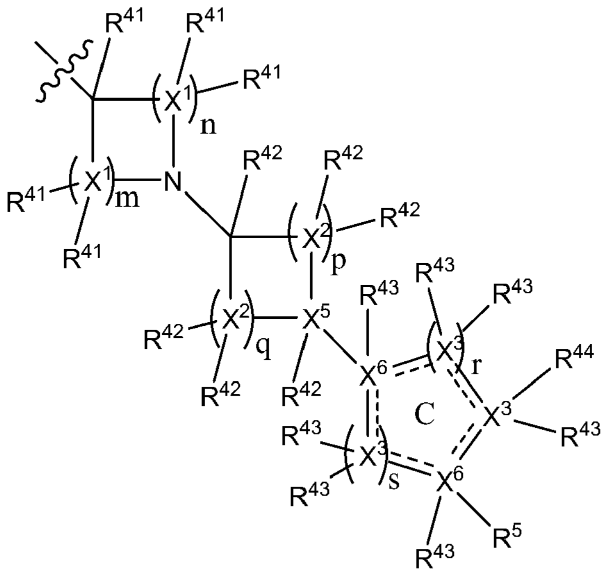

wherein n, m, p, q, r, s, $X^1$, $X^2$, $X^3$, $X^5$, $X^6$, $R^{41}$, $R^{42}$, $R^{43}$, $R^{44}$, $R^5$, and ring C, are as defined in Clause 19.
Clause 25. A compound according to Clause 24, wherein the group L comprises a group selected from the following structures:

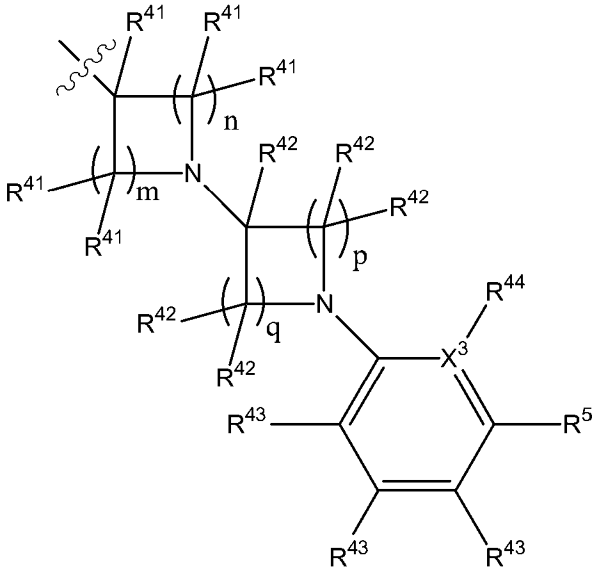

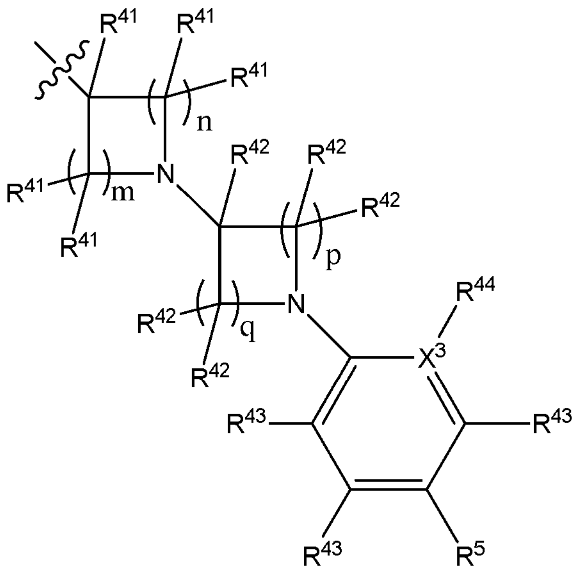

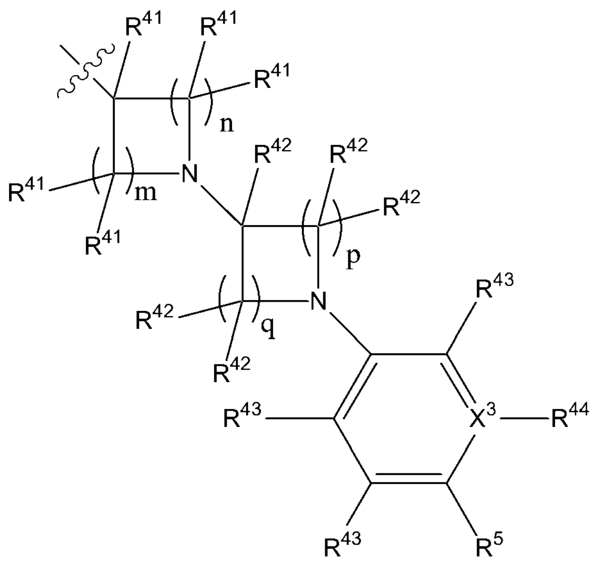

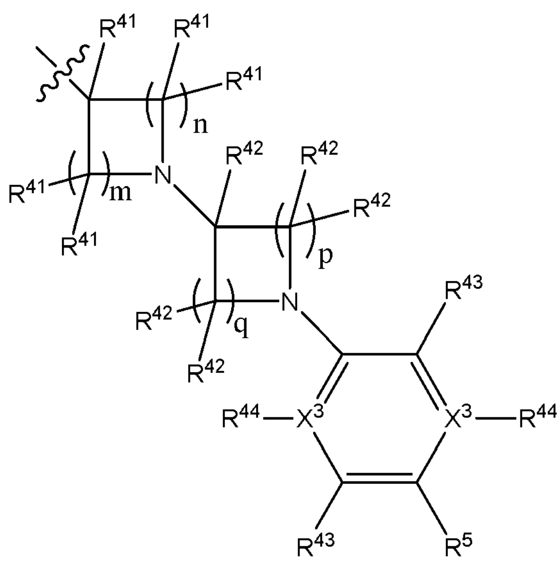

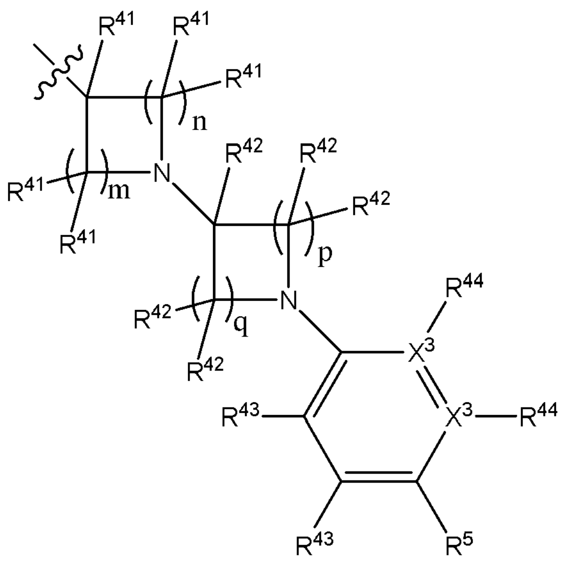

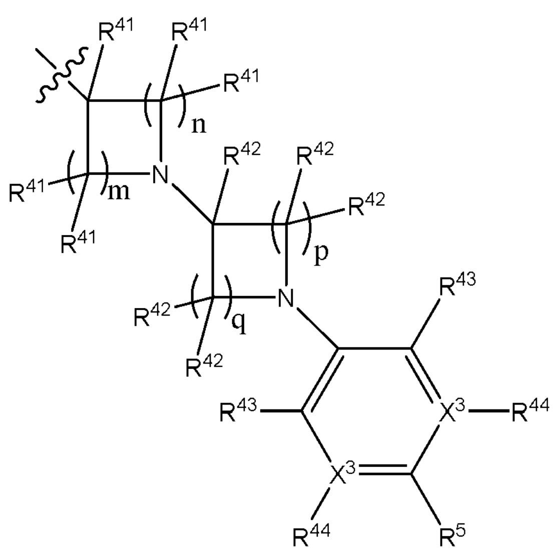

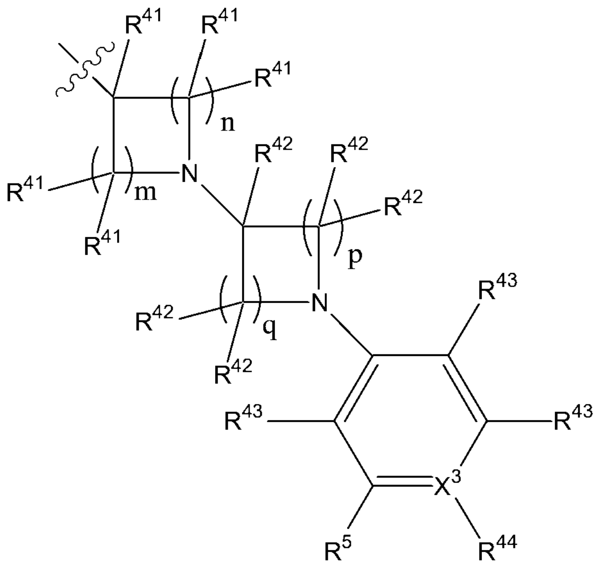

wherein n, m, p, q, $X^3$, $R^{41}$, $R^{42}$, $R^{43}$, $R^{44}$, and $R^5$, are as defined in Clause 24.

Clause 26. A compound according to any of Clauses 15 to 25, wherein m is selected from 1 or 2, n is selected from 2 or 3, p is selected from 1, 2 or 3 and q is selected from 1 or 2.

Clause 27. A compound according to Clause 25 or Clause 26, wherein the group L comprises a group having any the following structures:

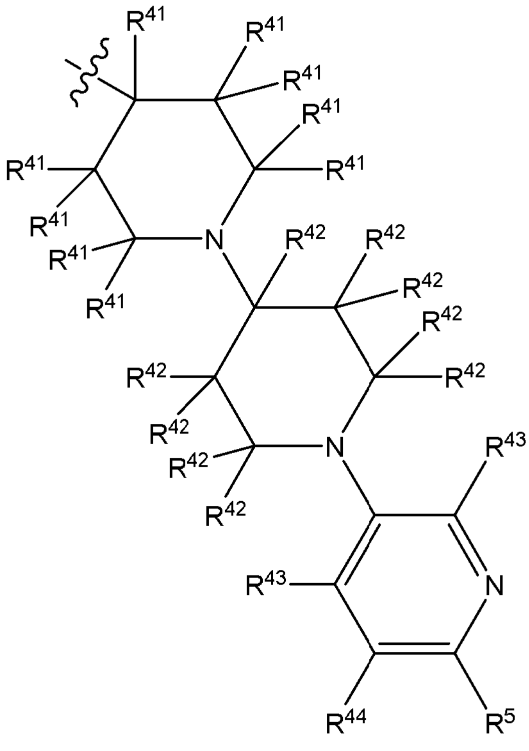

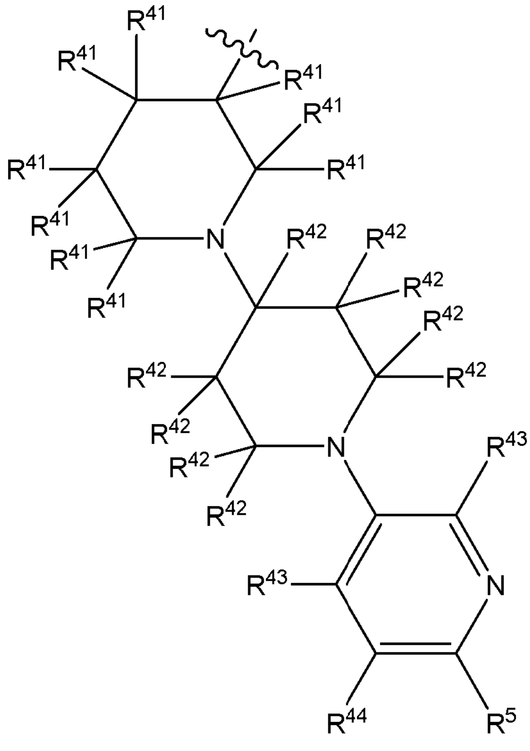

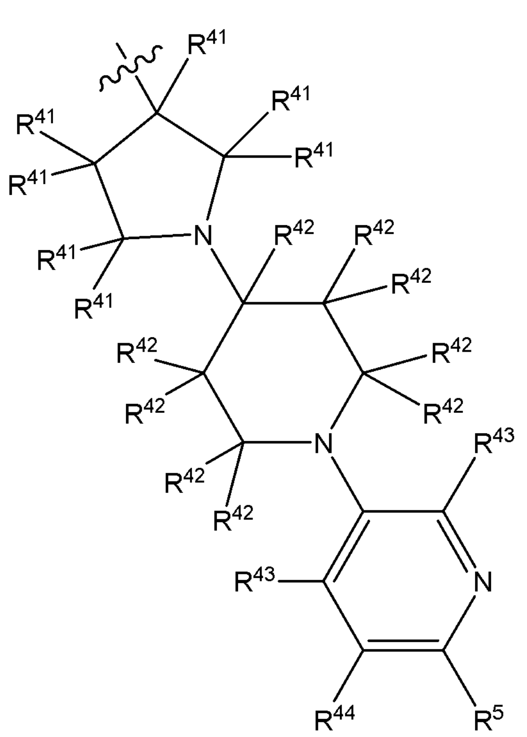

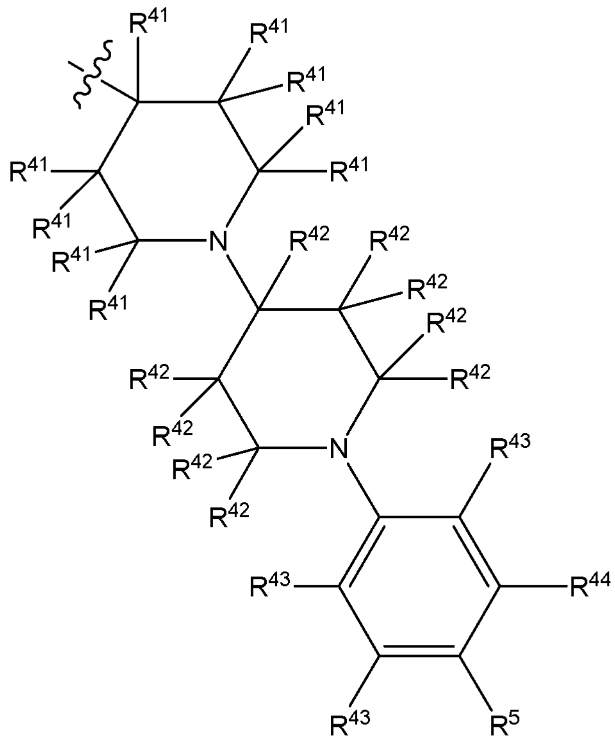

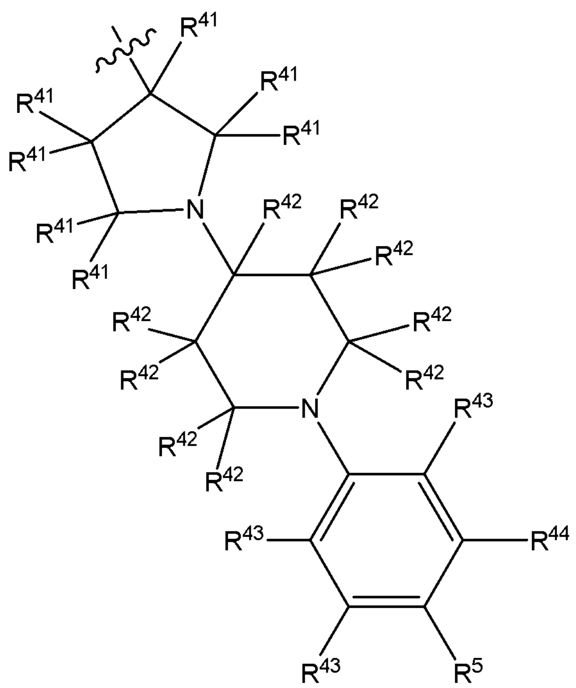

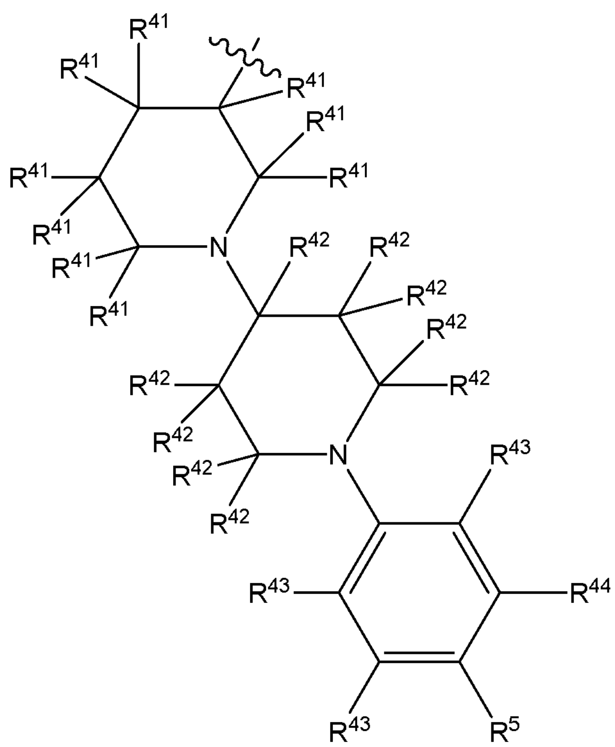

wherein, $R^{41}$, $R^{42}$, $R^{43}$, $R^{44}$ and $R^5$ are as defined in Clause 25.

Clause 28. A compound according to any of Clauses 1 to 14, wherein group $Q^2$ of the group L is absent and only one $Q^1$ group is present, and wherein the $Q^1$ group separates ring A from ring C by 3 or 4 atoms in the linear direction.

Clause 29. A compound according to Clause 28, wherein the $Q^1$ group comprises any of the following structures:

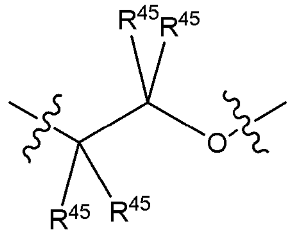
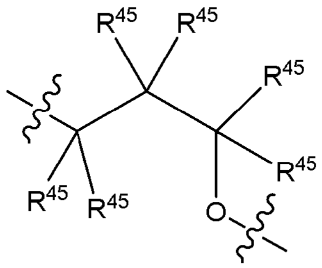
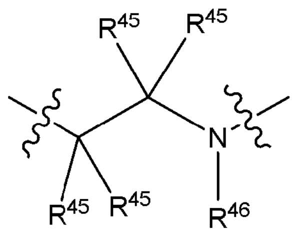

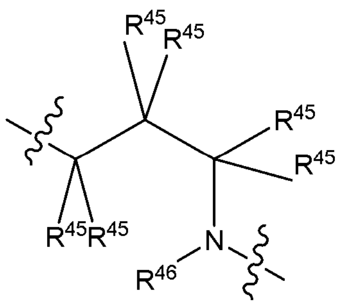

wherein $R^{45}$ and $R^{46}$ are as defined in any preceding Clause.

Clause 30. A compound according to Clause 29, wherein the $Q^1$ group comprises any of the following structures:

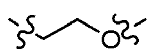
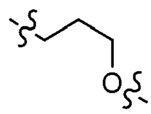
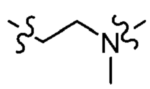
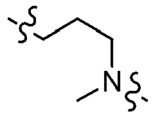

Clause 31. A compound according to any of Clauses 1 to 11, or 13 to 14, wherein the group L comprises a group having any the following structures:

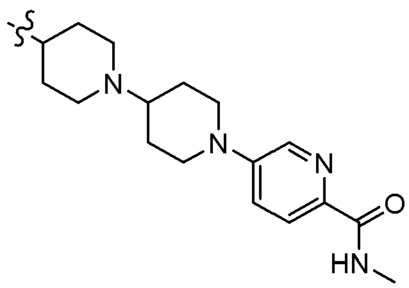
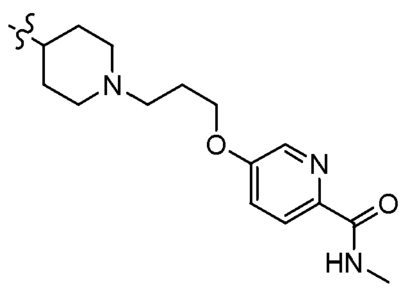

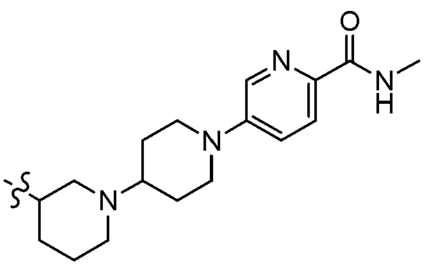
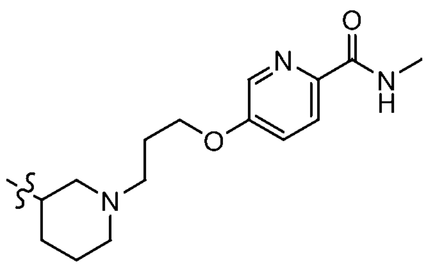

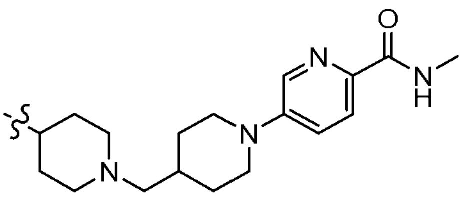 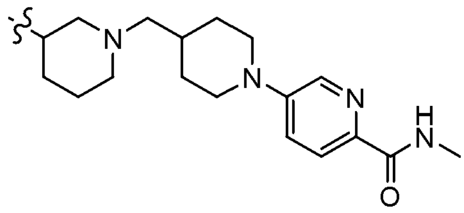

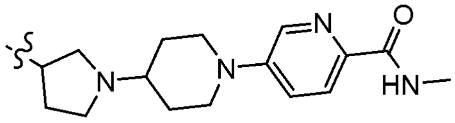 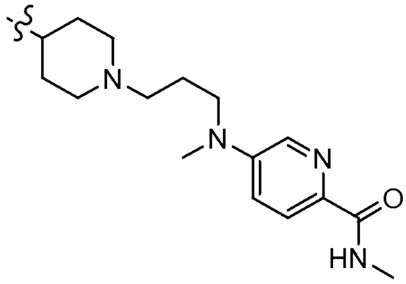

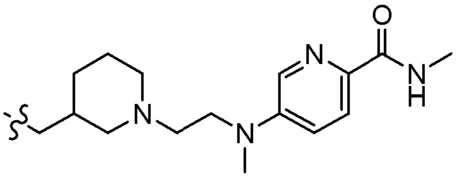 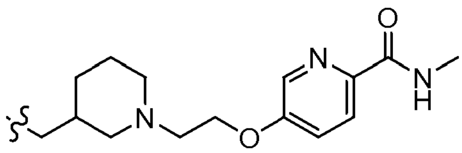

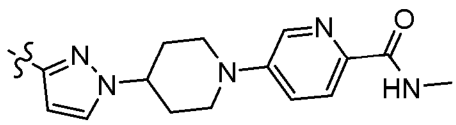 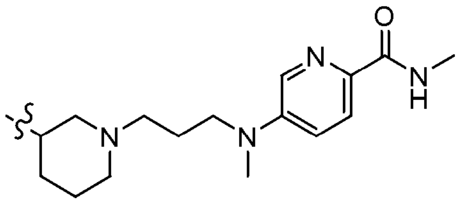

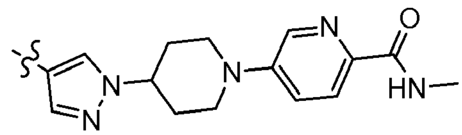 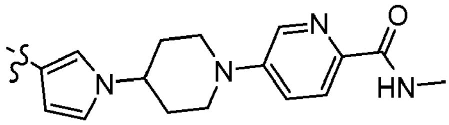

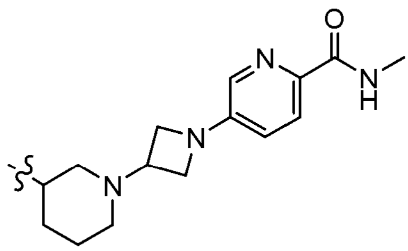 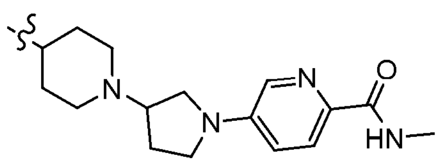

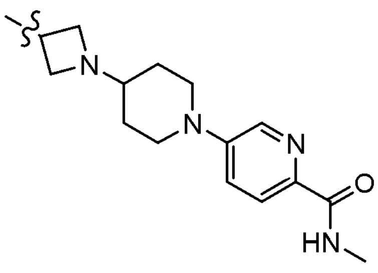 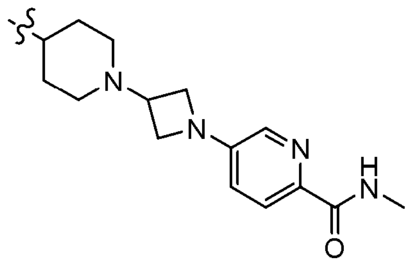 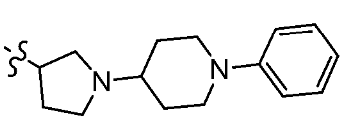

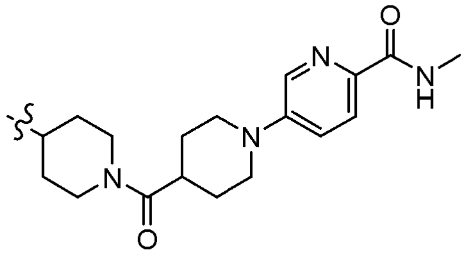

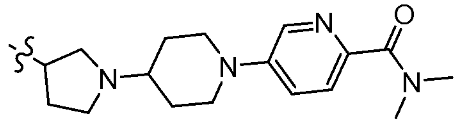

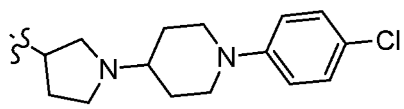

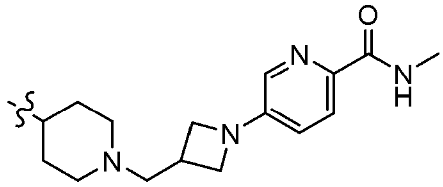

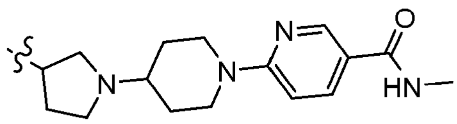

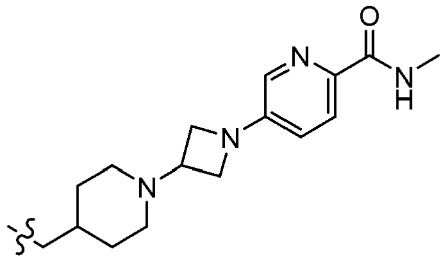

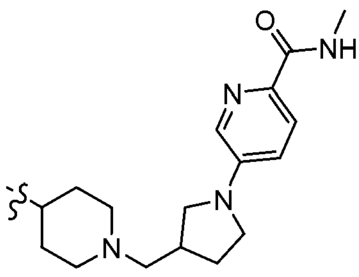

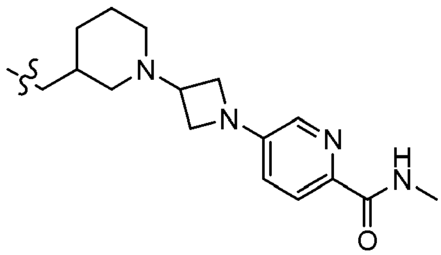

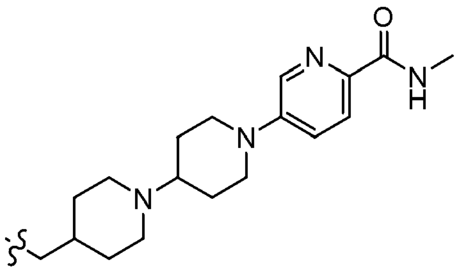

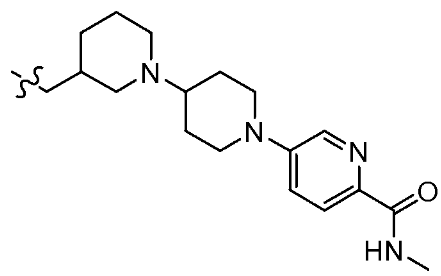

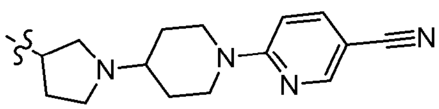

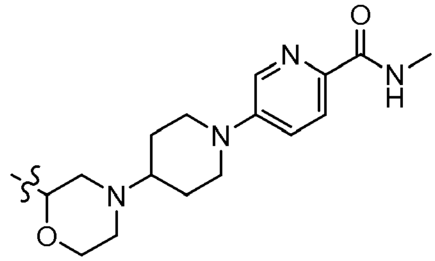

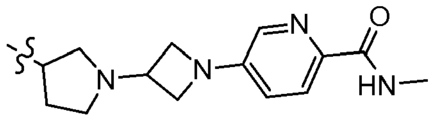

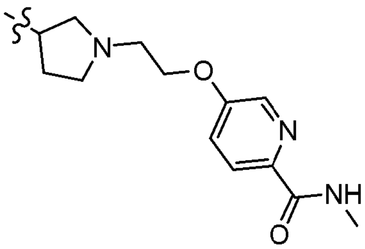

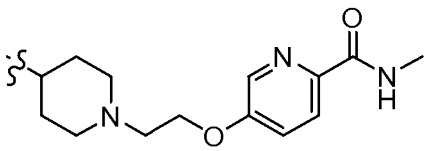

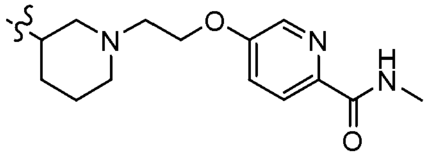

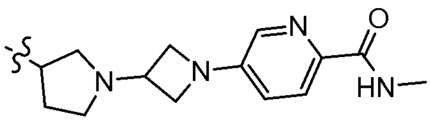

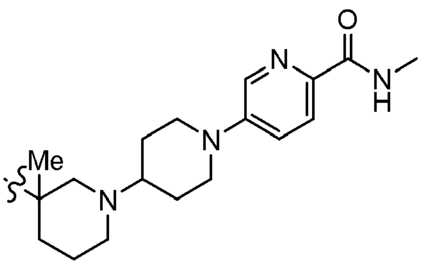

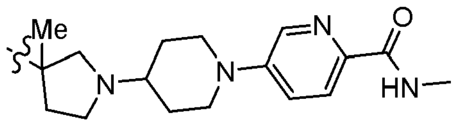

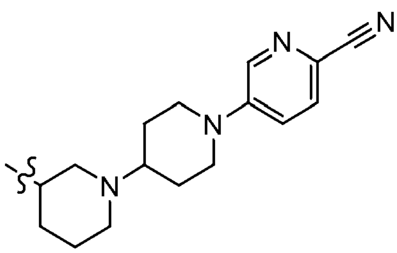

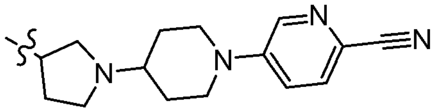

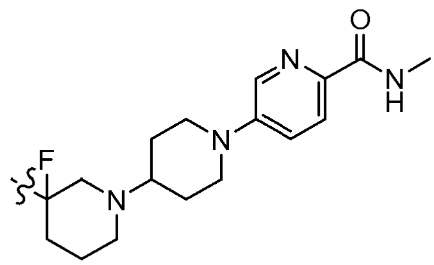

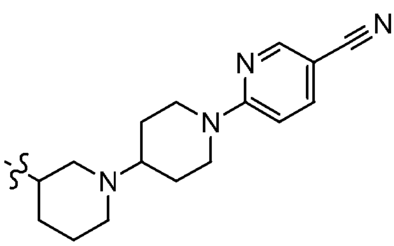

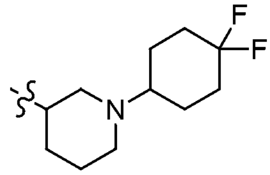

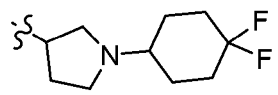

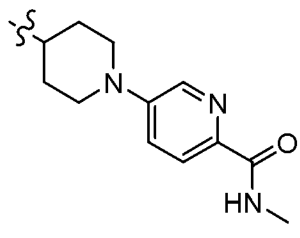

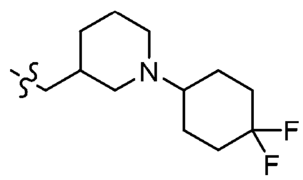

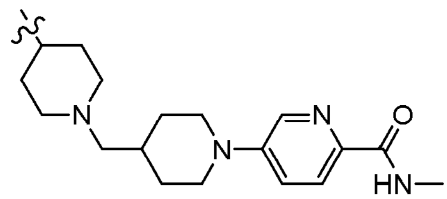

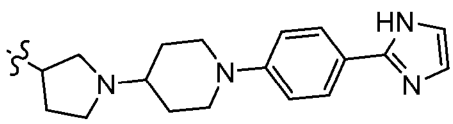

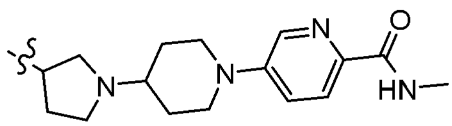

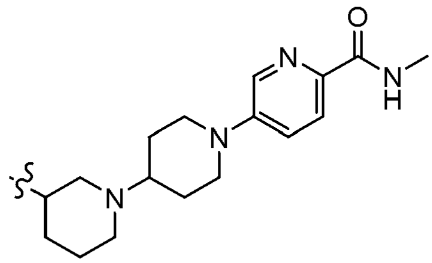

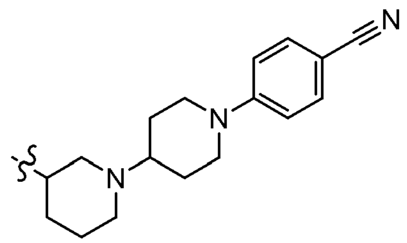

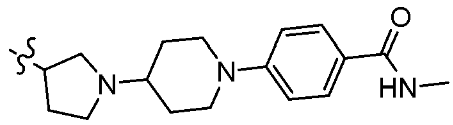

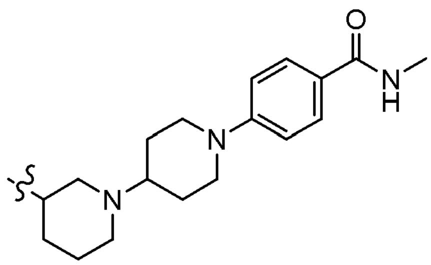

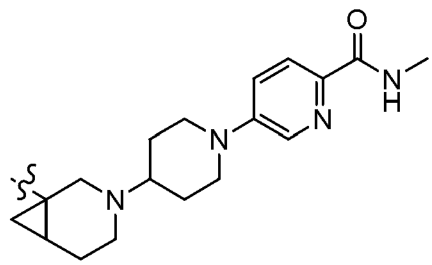

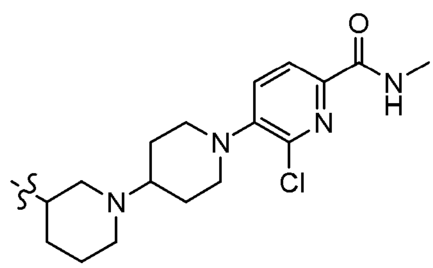

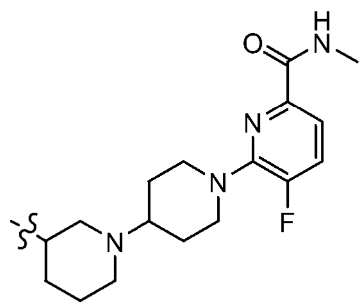

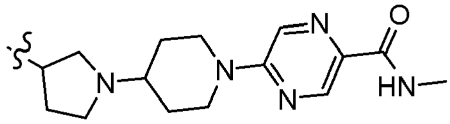

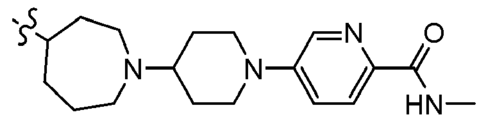

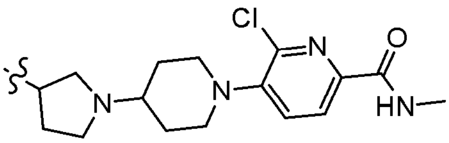

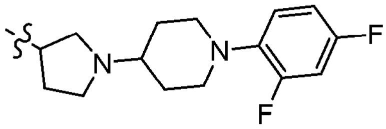

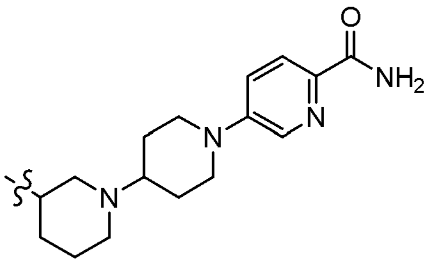

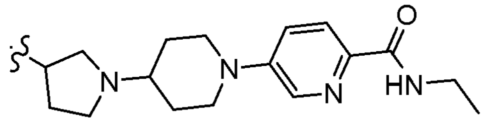

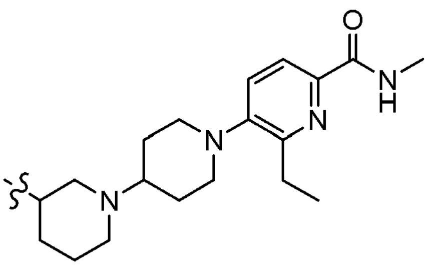

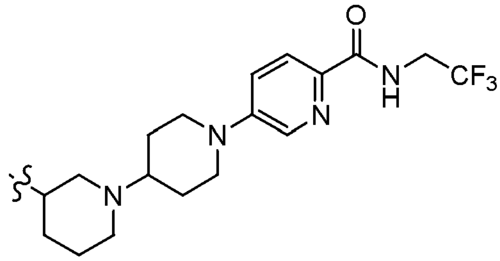

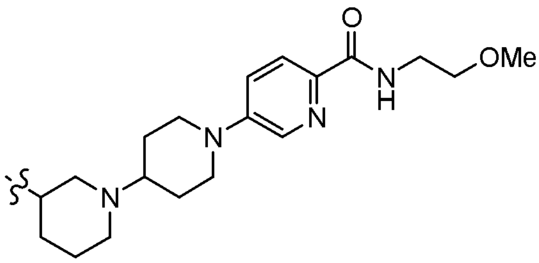

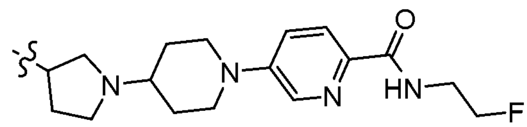

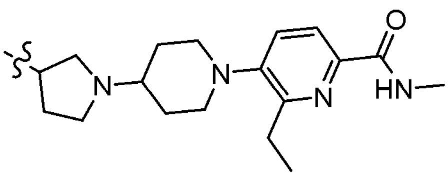

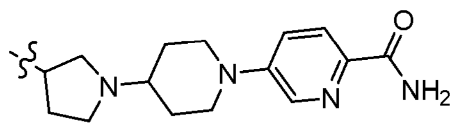

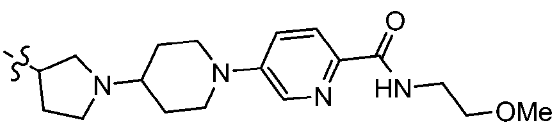

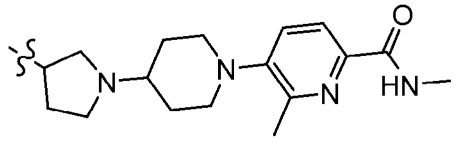

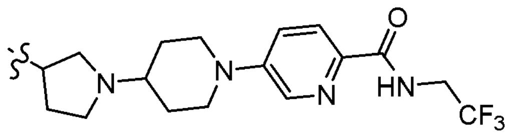

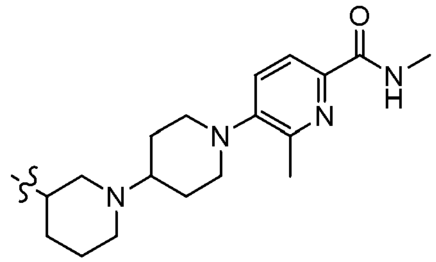

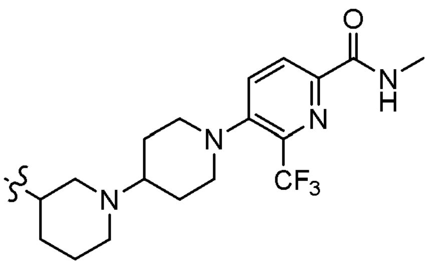

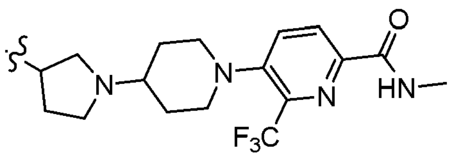

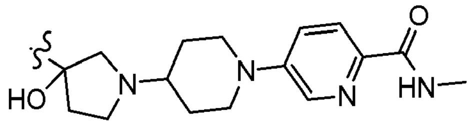

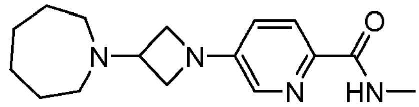

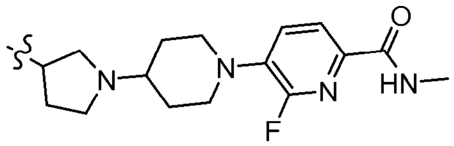

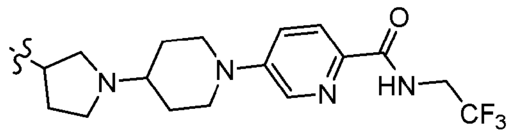

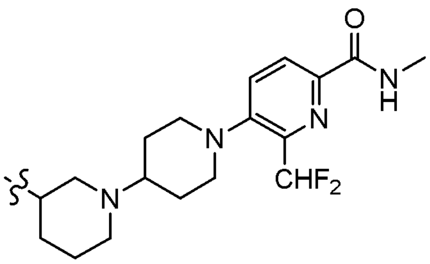

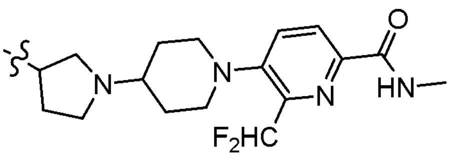

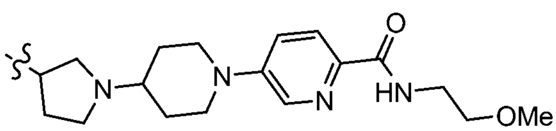

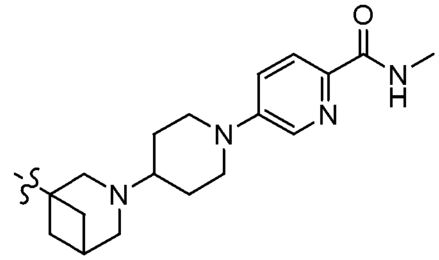

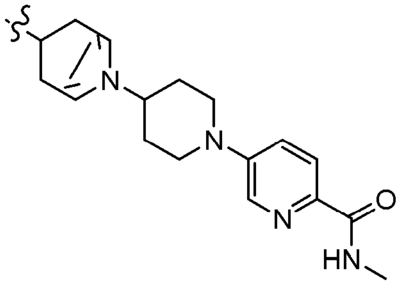

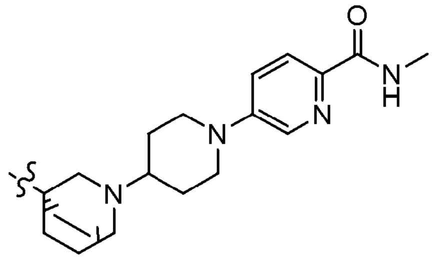

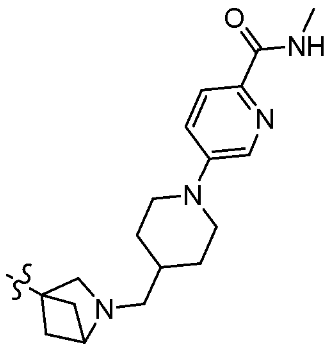

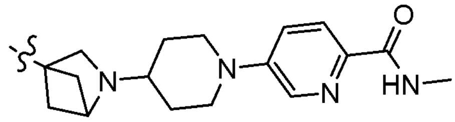

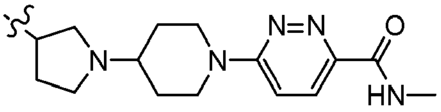

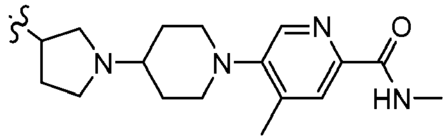

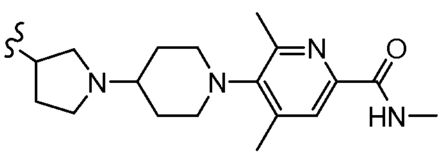

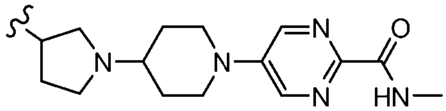

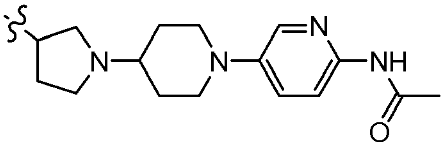

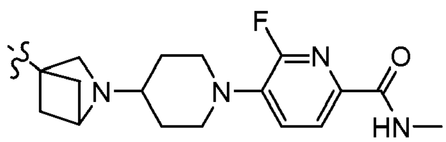

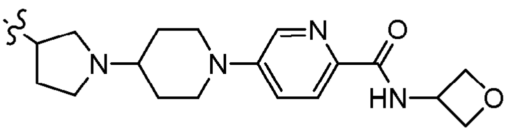

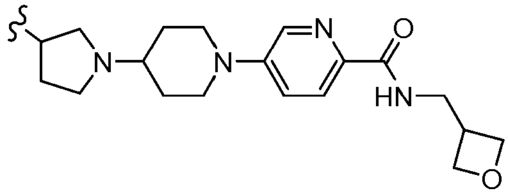

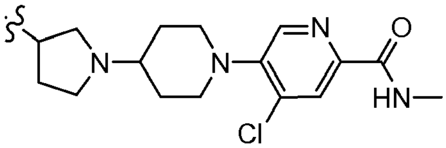

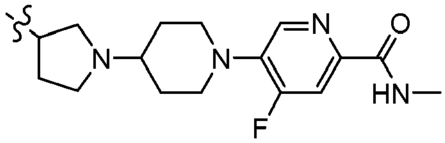

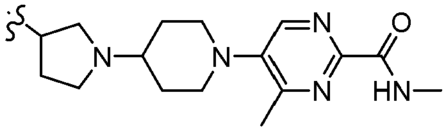

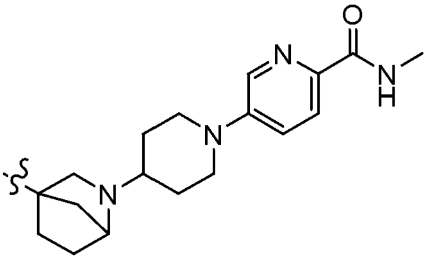

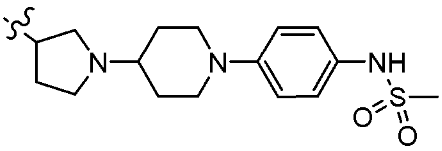

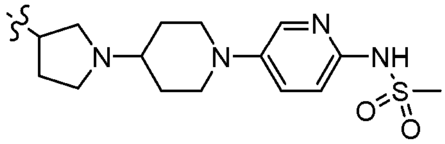

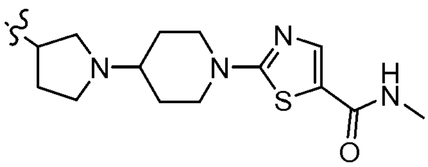

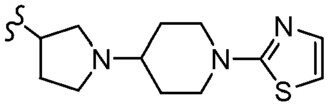

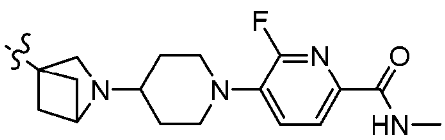

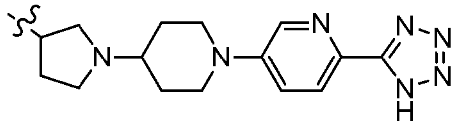

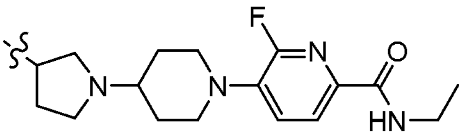

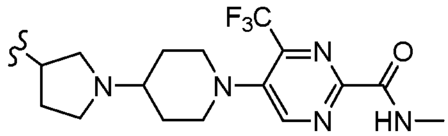

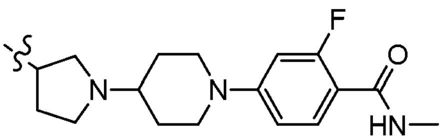

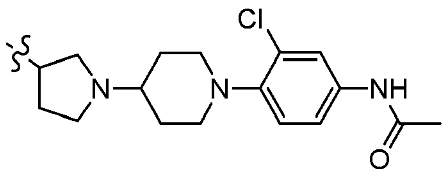

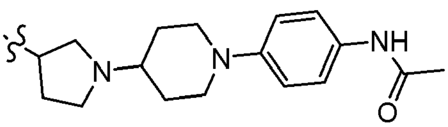

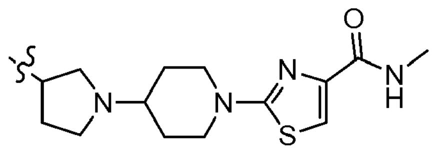

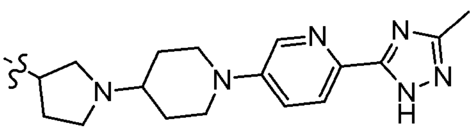

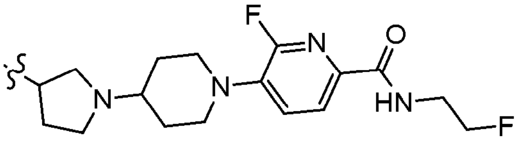

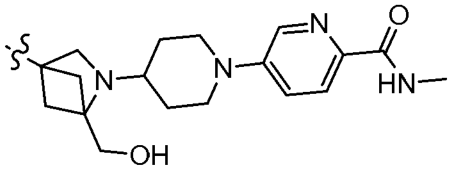

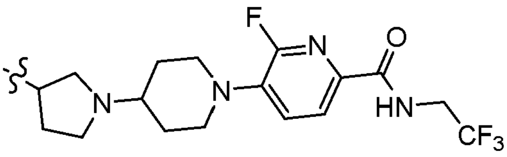

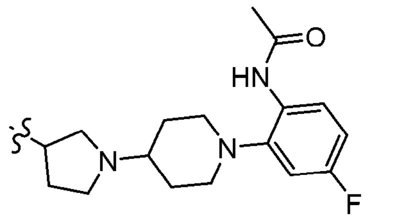

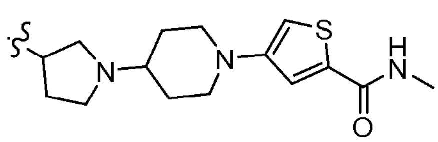

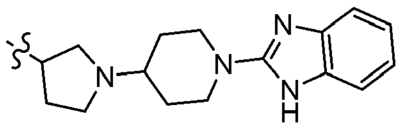

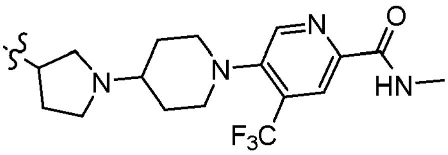

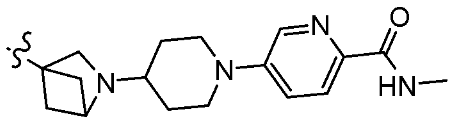

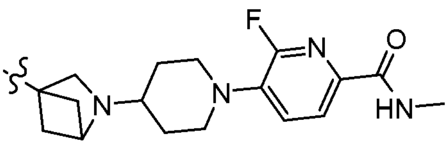

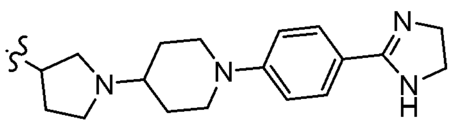

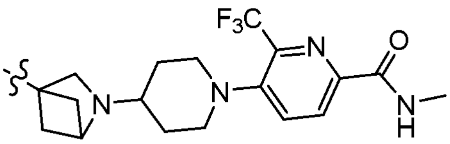

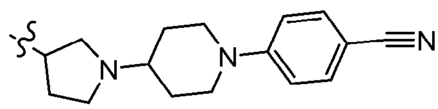

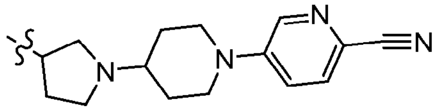

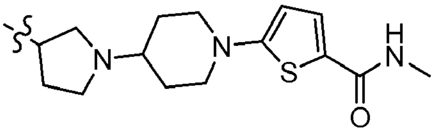

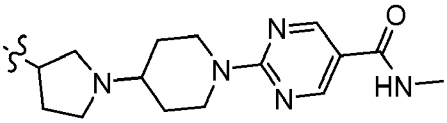

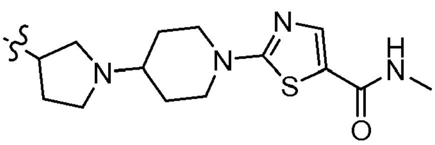

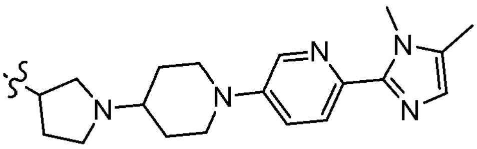

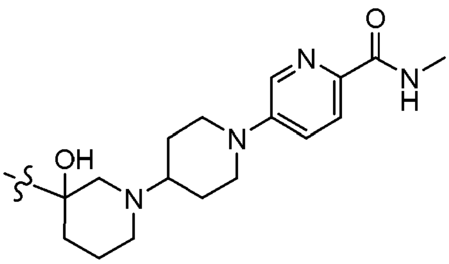

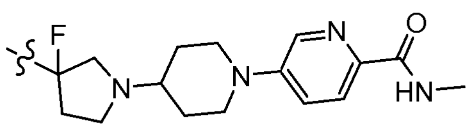

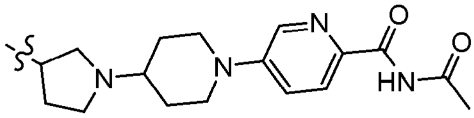
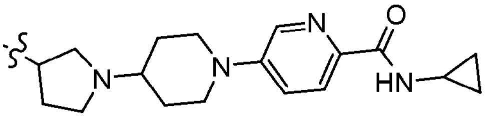
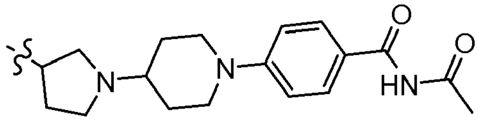
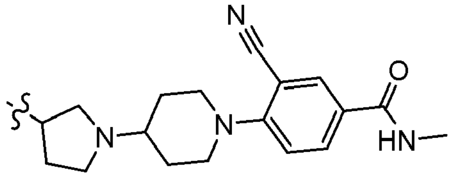
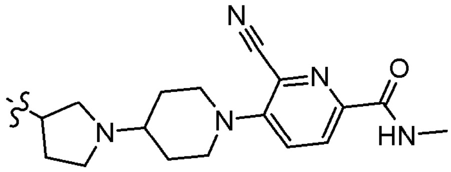
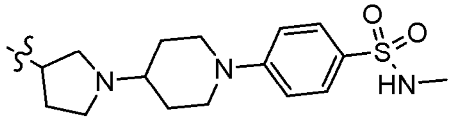
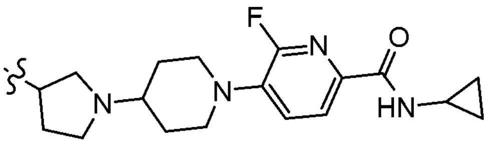
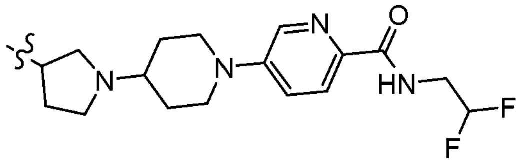
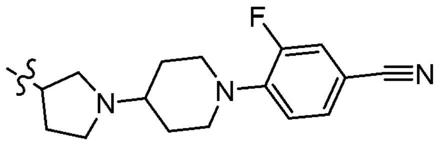
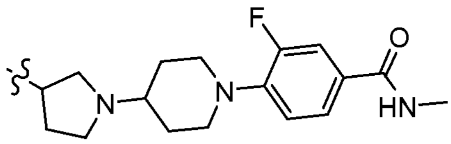
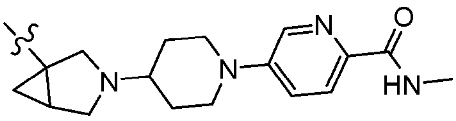
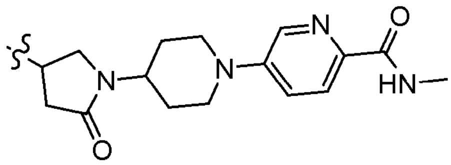
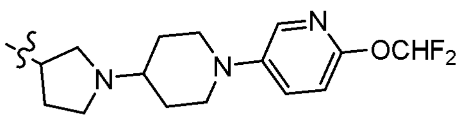
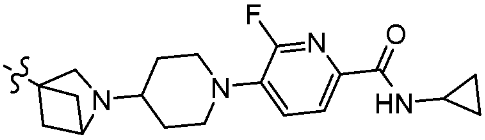
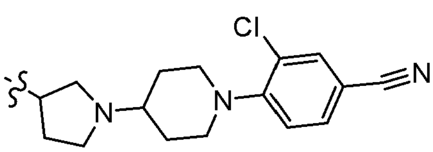
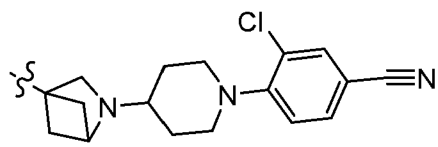
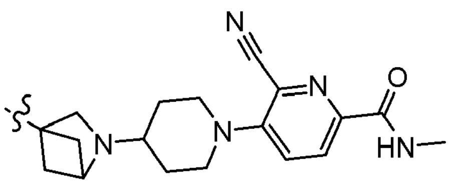
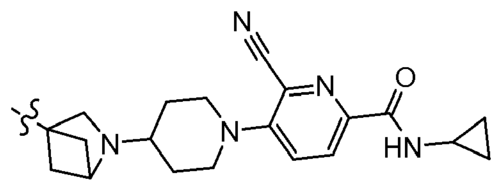

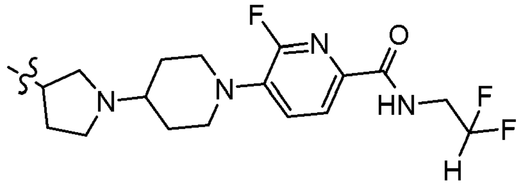

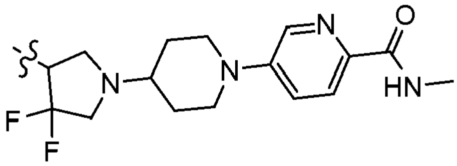

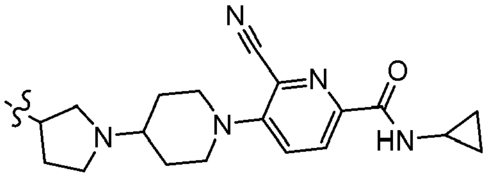

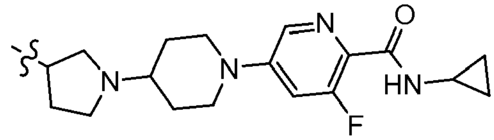

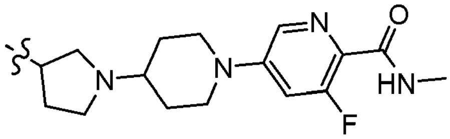

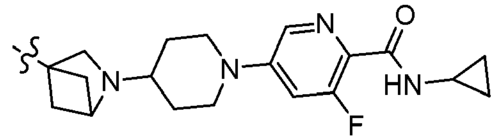

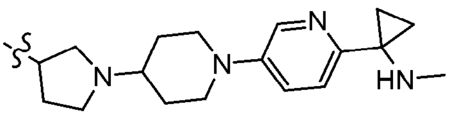

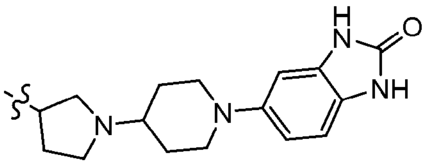

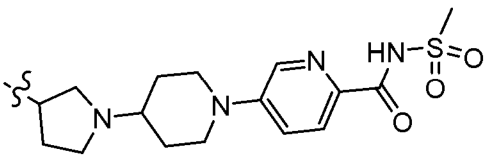

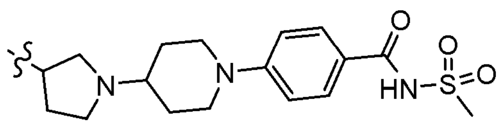

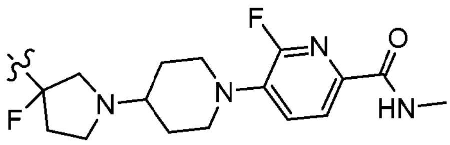

.

Clause 32. A compound according to any preceding Clause, which compound comprises any of the following structures:

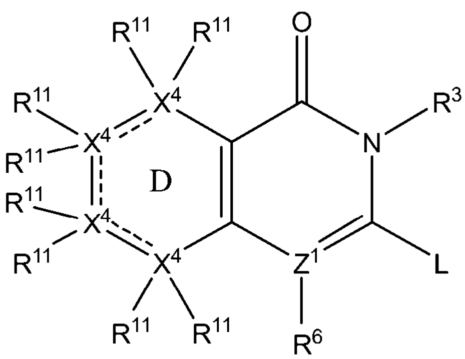

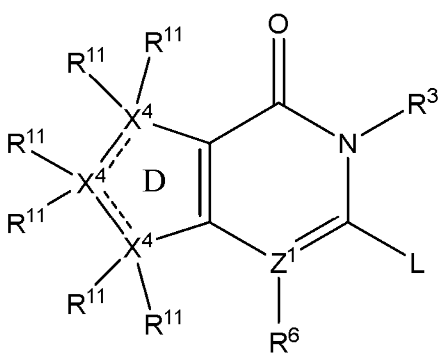

wherein, the dotted lines indicate that the ring D may comprise single bonds, or a combination of both single and double bonds, and may be aliphatic or aromatic; each $X^4$ may be the same or different and is independently selected from C, N, O and S; each $R^{11}$ may be the same or different and is independently selected from H and a substituted or unsubstituted organic group; and wherein $Z^1$, $R^3$, $R^6$, and L are as defined in any preceding Clause.

Clause 33. A compound according to Clause 32, which compound comprises any of the following structures:

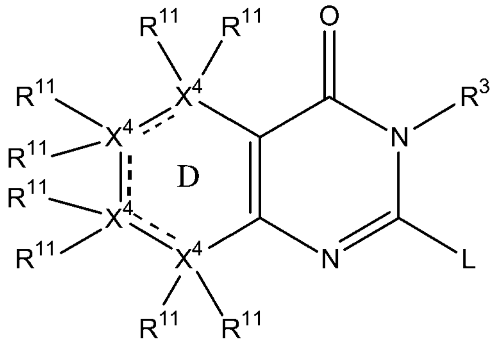

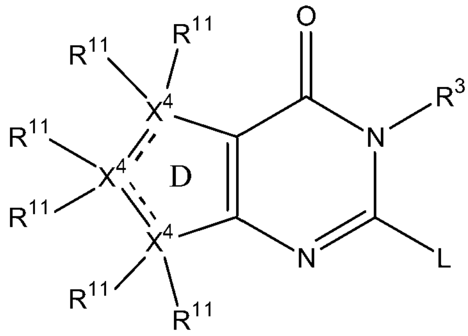

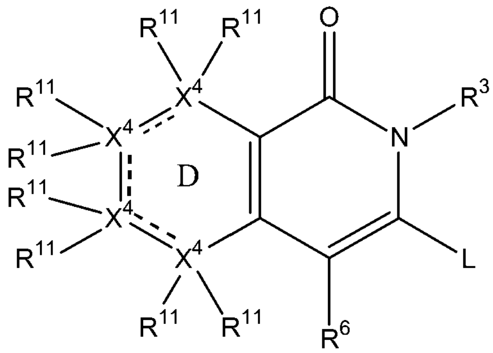

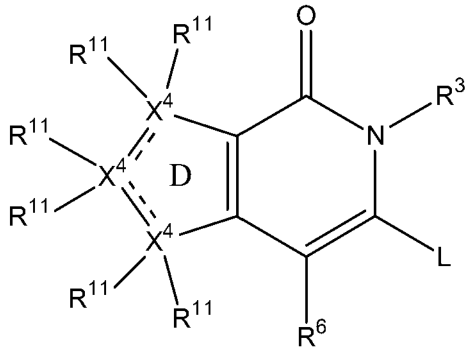

wherein the ring D, $X^4$, $R^{11}$, $R^3$, $R^6$, and L are as defined in Clause 32, preferably wherein at least one $X^4$ is C.
Clause 34. A compound according to Clause 33, which compound comprises any of the following structures:

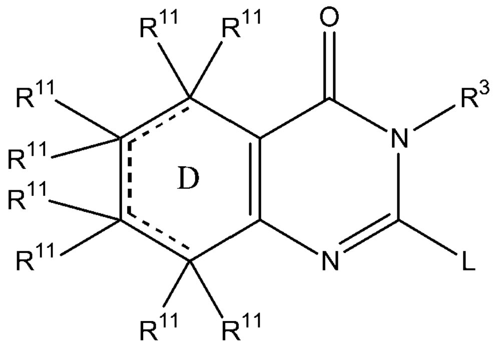

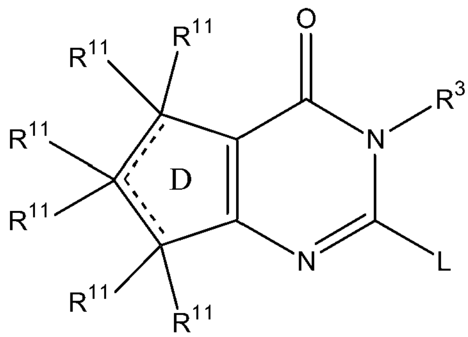

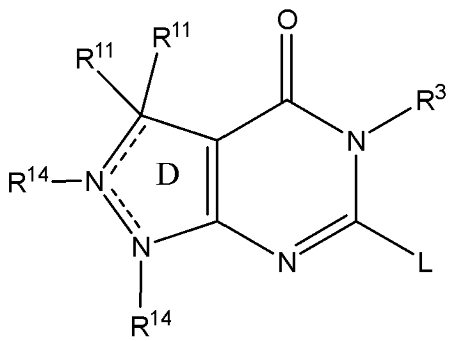

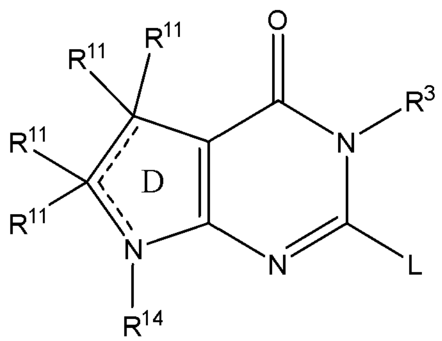

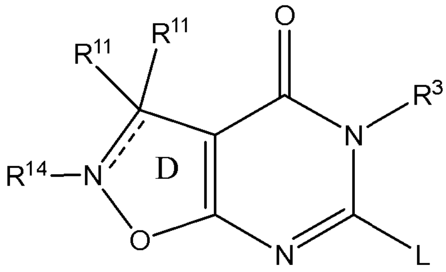

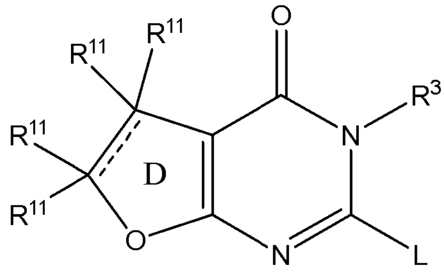

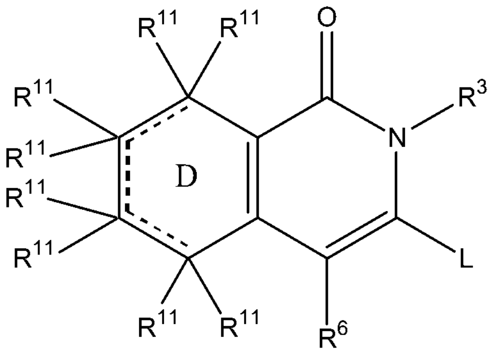

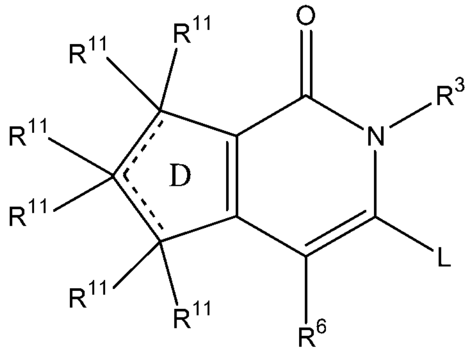

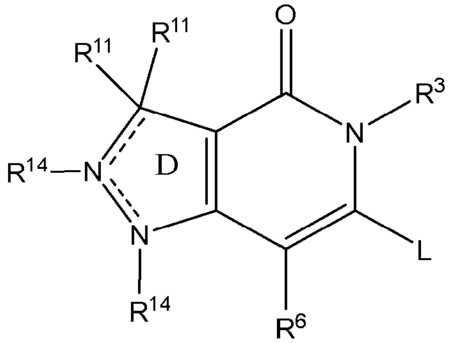

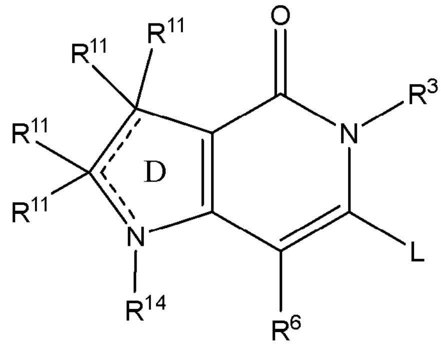

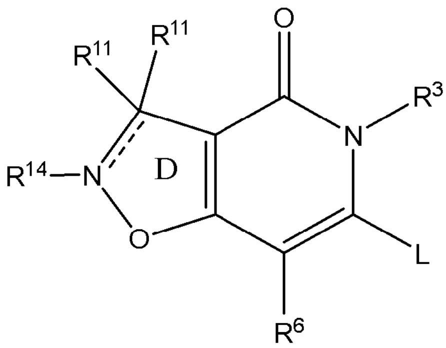

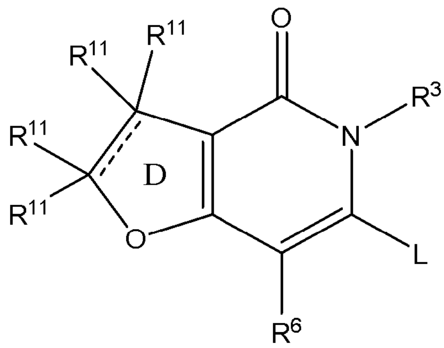

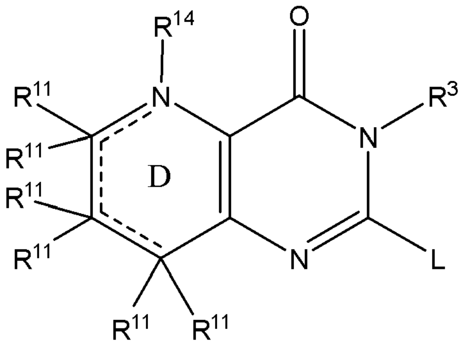

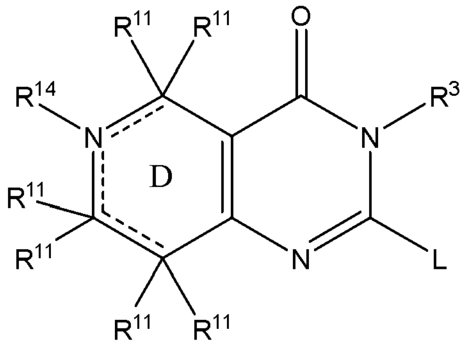

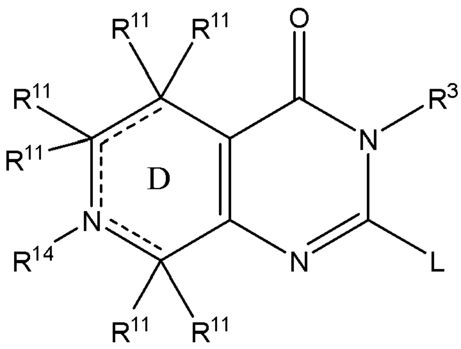

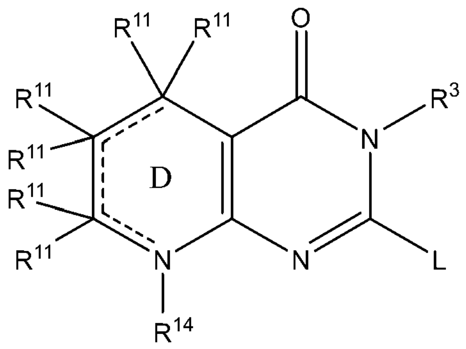

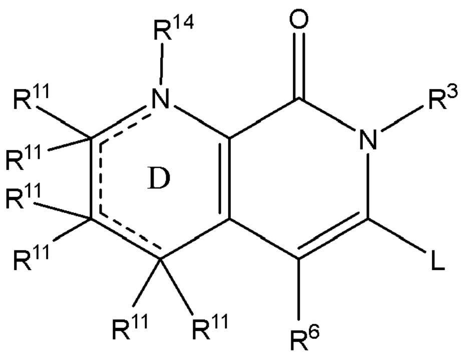

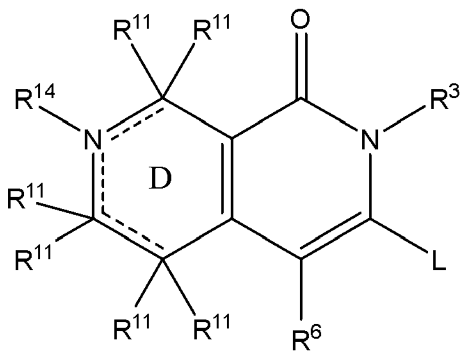

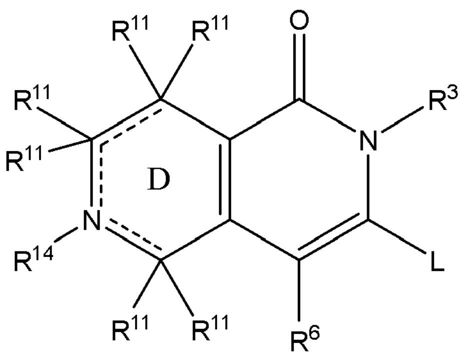

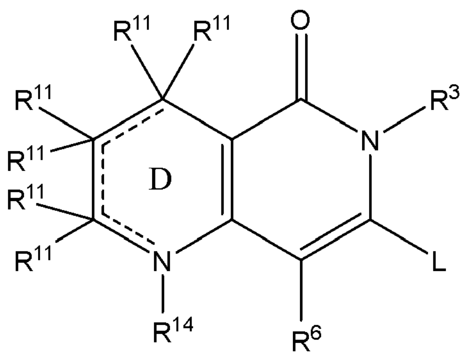

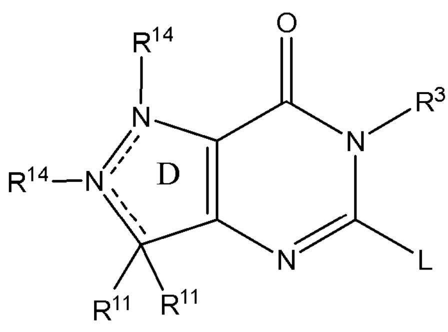

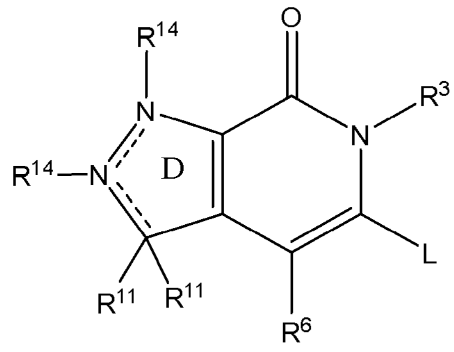

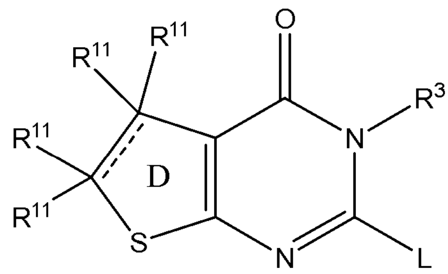

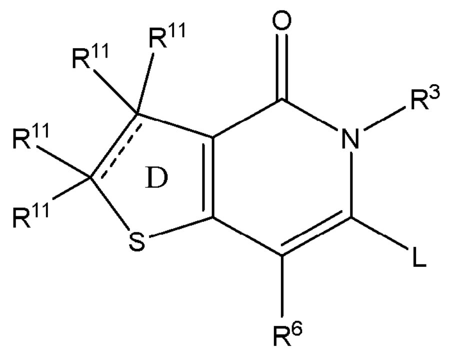

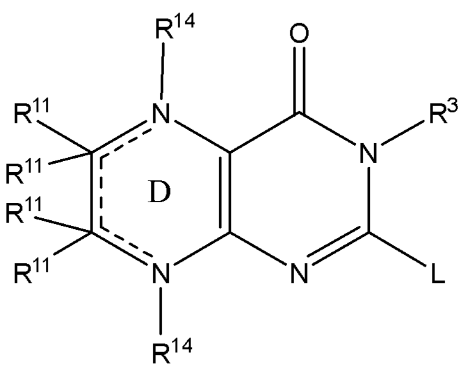

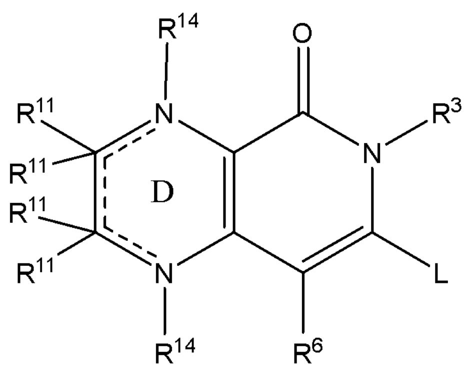

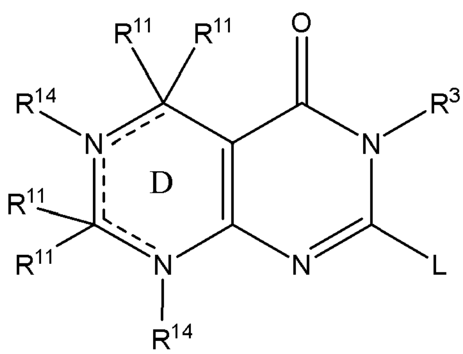

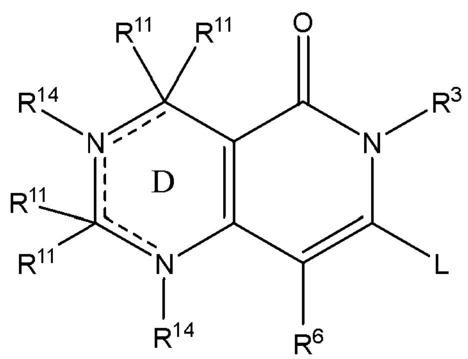

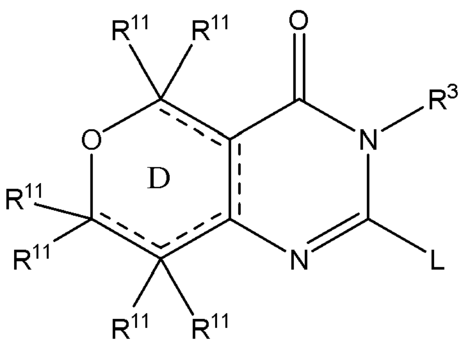

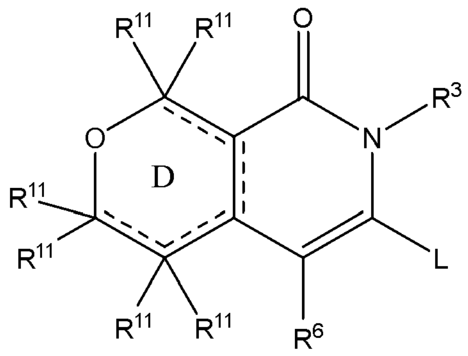

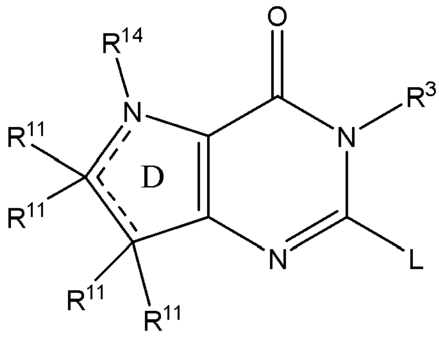

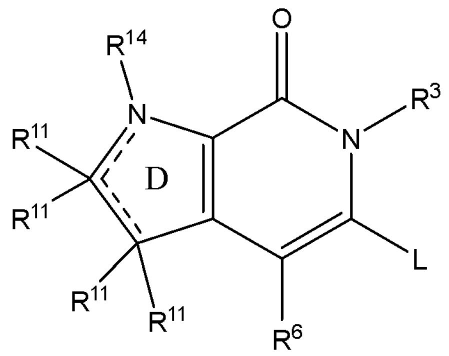

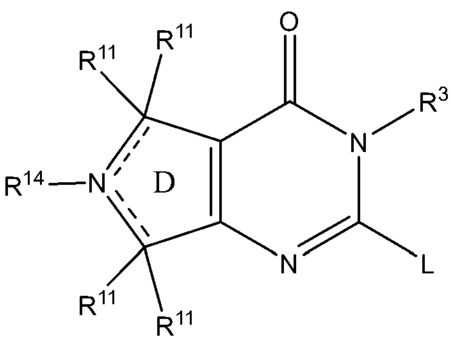

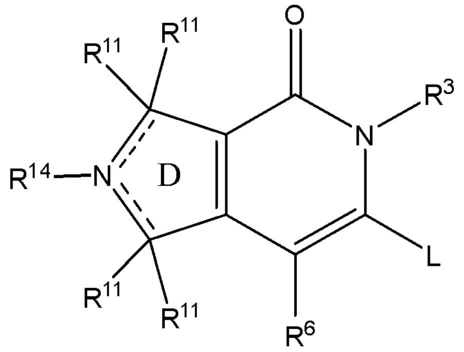

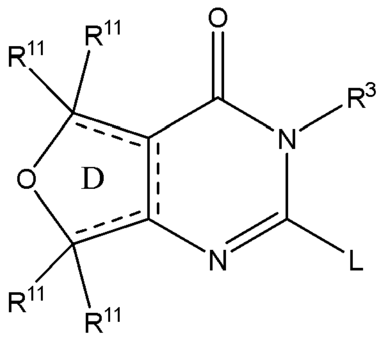

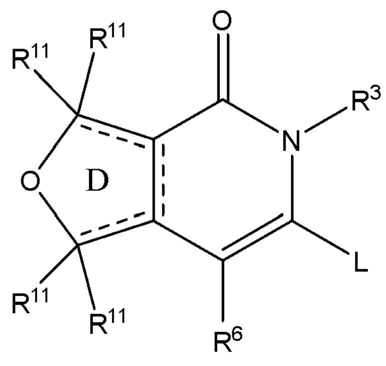

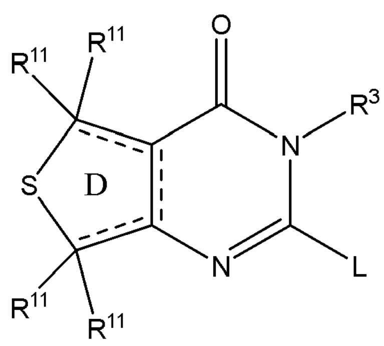

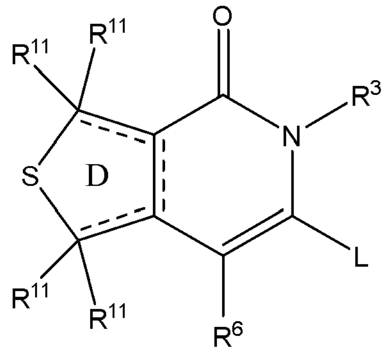

herein $R^{14}$ may be present or absent and if present is selected from H, $C_1$ to $C_3$ alkyl or $C_1$ to $C_3$ fluoroalkyl; and $R^{11}$, ring D, $R^3$, $R^6$, and L are as defined in Clause 33.

Clause 35. A compound according to Clause 34, which compound comprises any of the following structures:

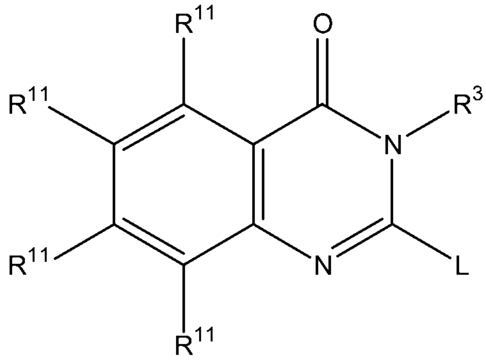

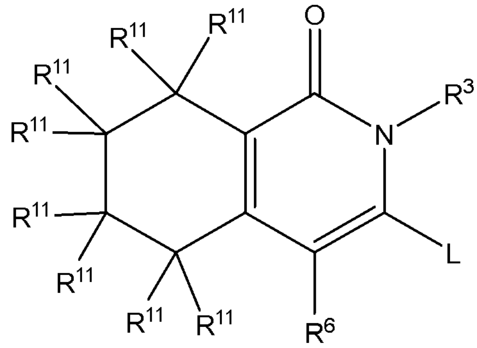

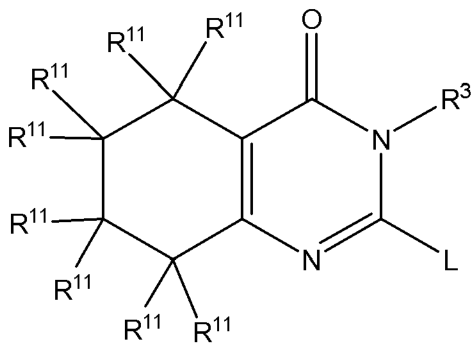

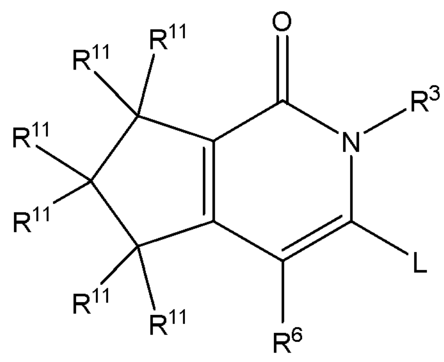

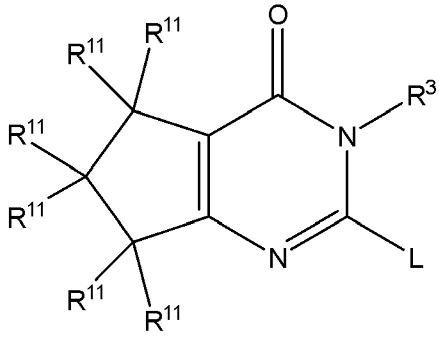

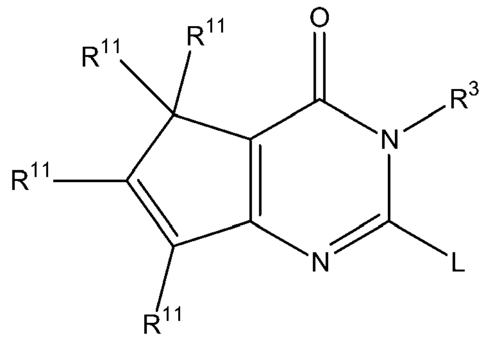

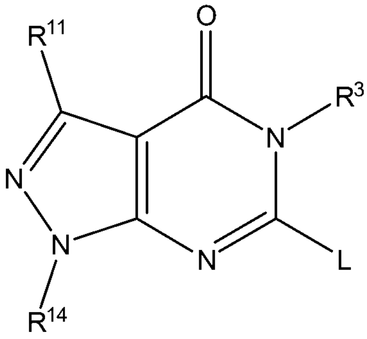

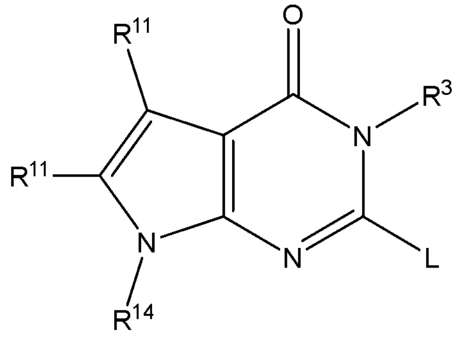

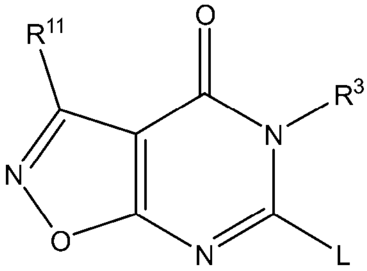

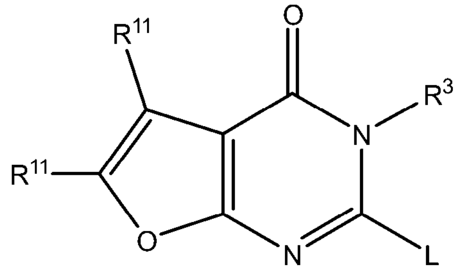

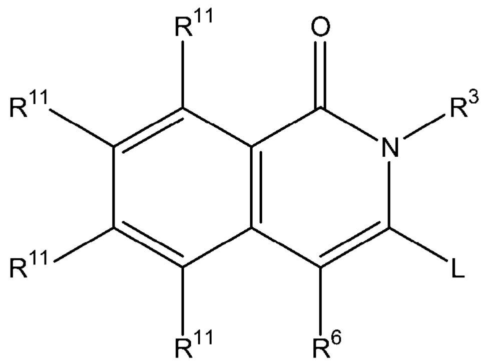

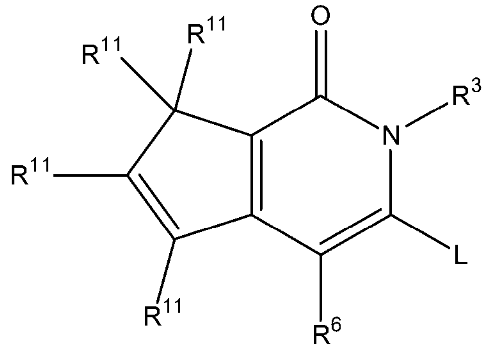

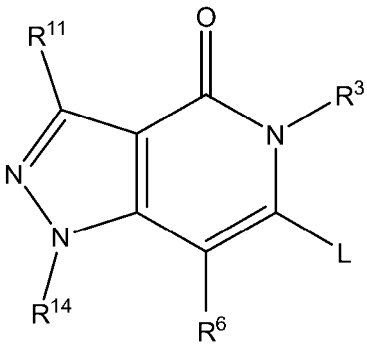

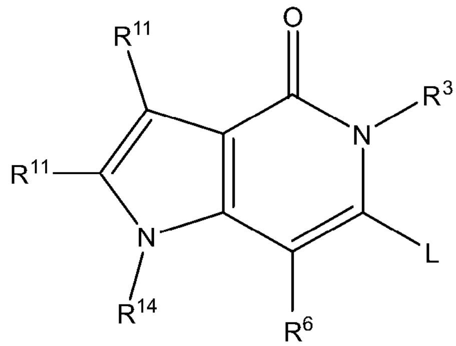

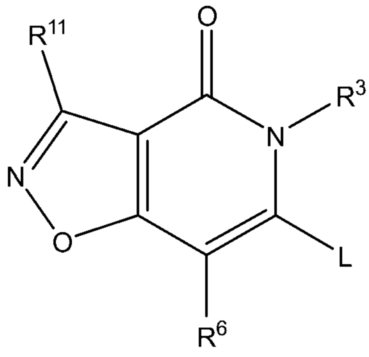

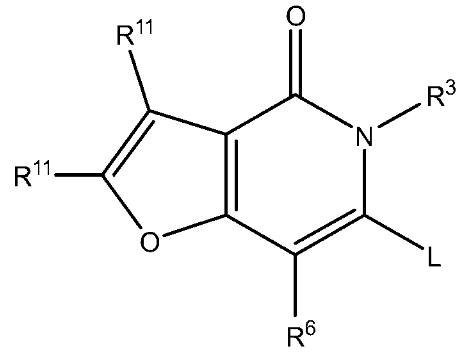

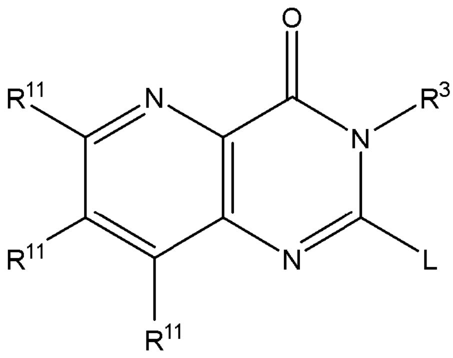

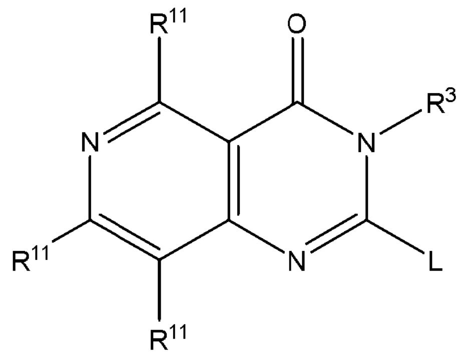

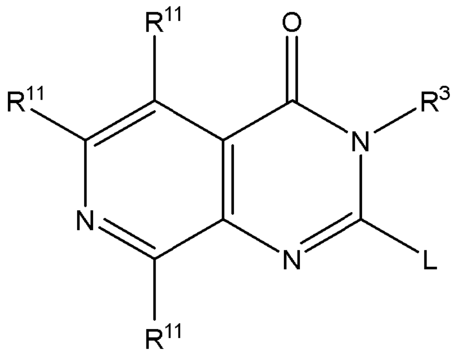

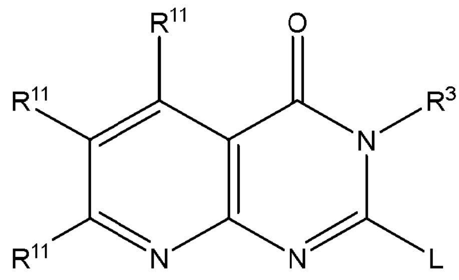

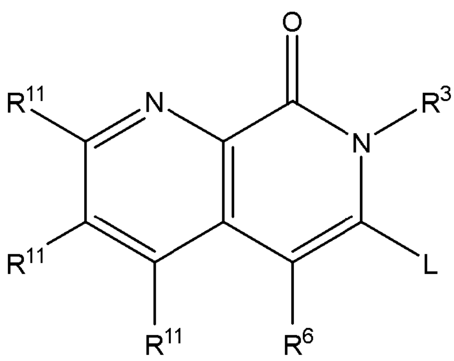

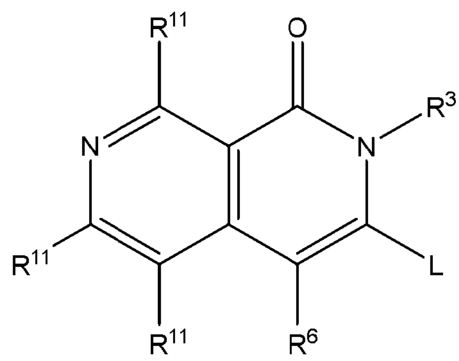

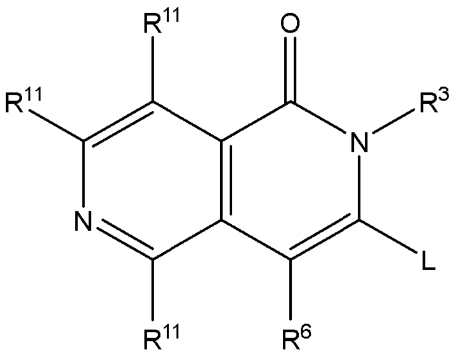

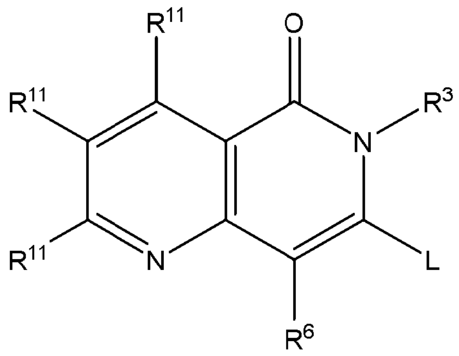

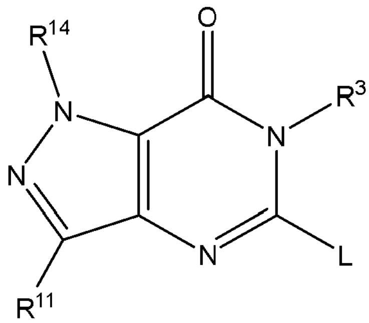

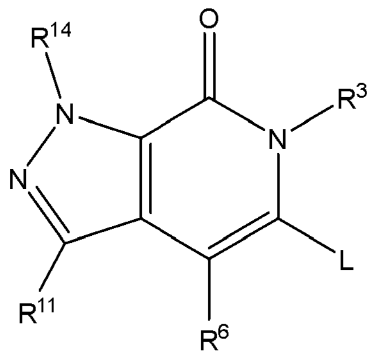

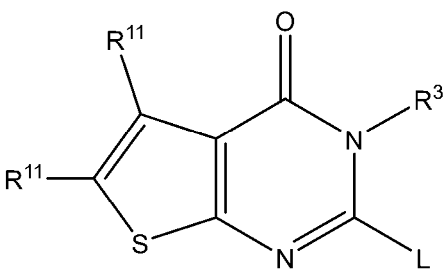

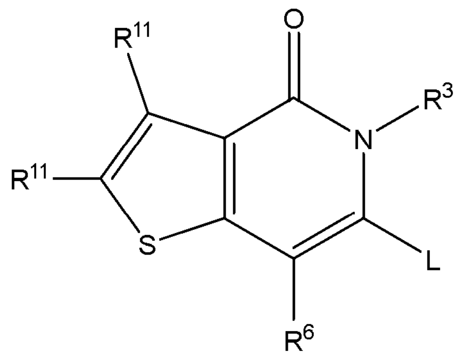

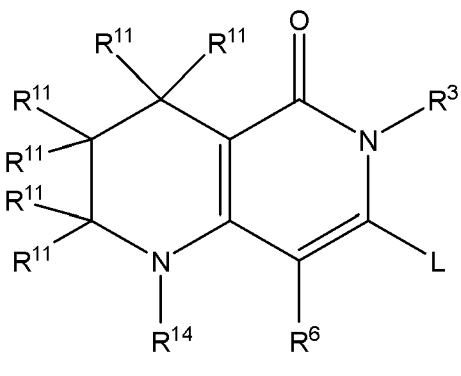

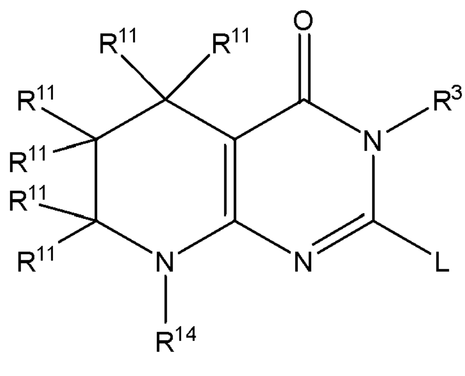

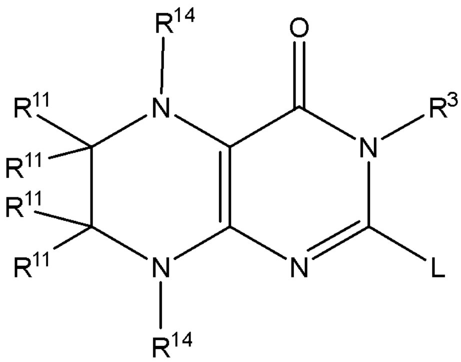

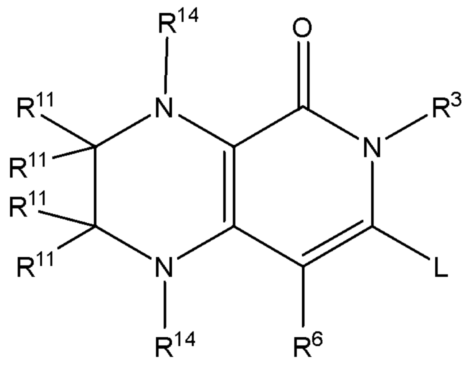

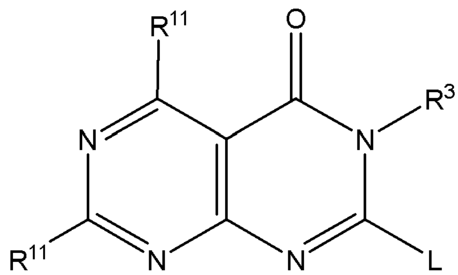

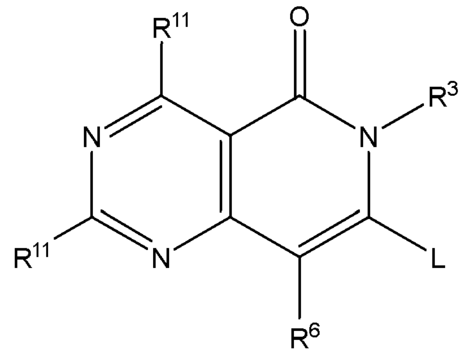

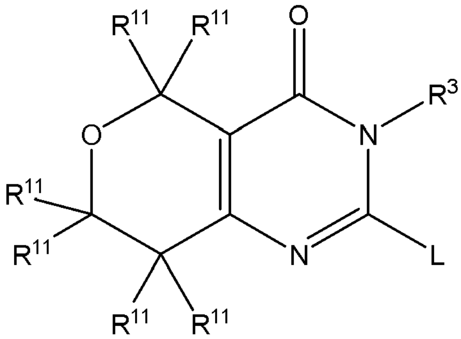

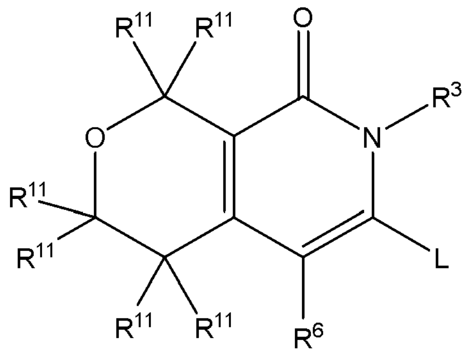

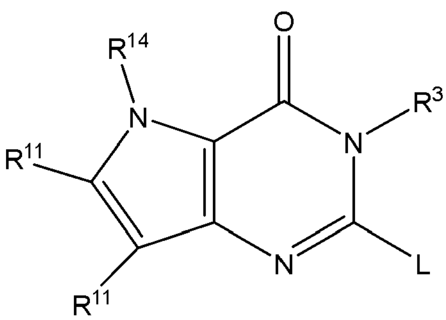

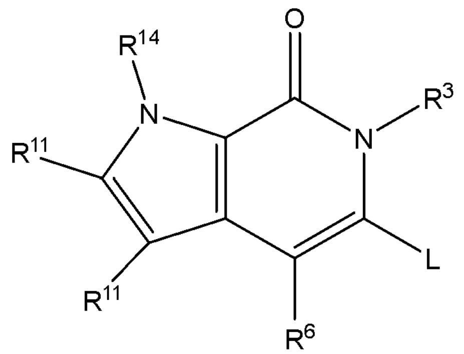

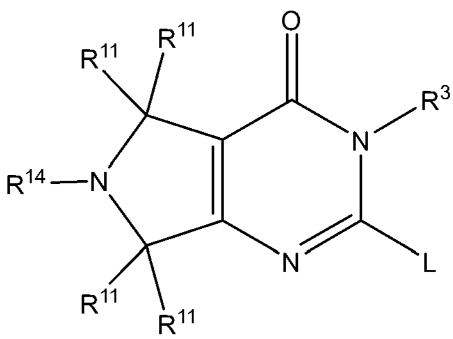

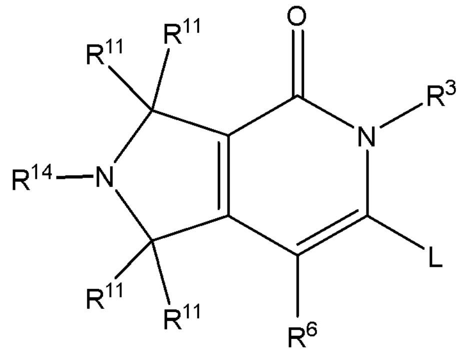

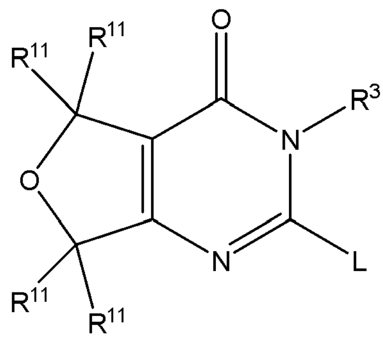

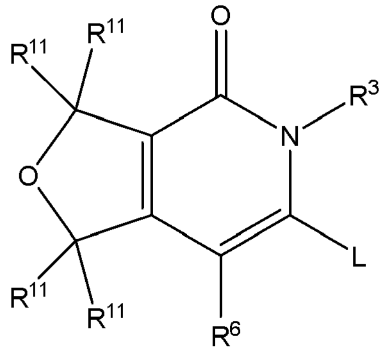

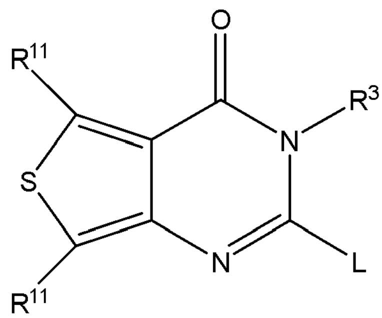

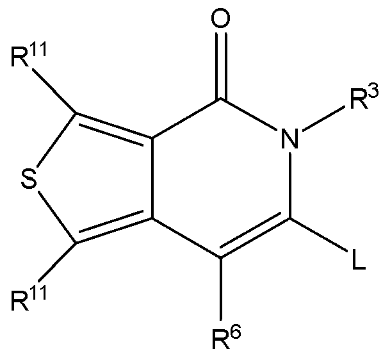

wherein $R^3$, $R^{11}$, $R^{14}$, $R^6$, and L are as defined in Clause 34.

Clause 36. A compound according to any of Clauses 32 to 35, wherein each $R^{11}$ is independently selected from H, a halogen, a nitrile group, a linear or branched $C_1$-$C_3$ alkyl group, a linear or branched $C_1$-$C_3$ halogenated alkyl group (preferably fluoroalkyl), an -OH group, a linear or branched $C_1$-$C_3$ alcohol group, a halogenated (preferably fluoro-) linear or branched $C_1$-$C_3$ alcohol group, $-NH_2$, a linear or branched primary secondary or tertiary $C_1$-$C_3$ amine group, a halogenated (preferably fluoro-) linear or branched primary, secondary or tertiary $C_1$-$C_3$ amine group, a linear or branched $C_1$-$C_3$ alkoxy group, a linear or branched $C_1$-$C_3$ halogenated alkoxy group (preferably fluoroalkoxy), and/or a pair of $R^{11}$ groups attached to the same atom forming =O; and independently, and/or when a pair of $R^{11}$ groups attached to different atoms together forms a ring with ring D atoms, the pair of $R^{11}$ groups comprises $-CH_2CH_2CH_2-$.

Clause 37. A compound according to Clause 36, wherein each $R^{11}$ is independently selected from H, Cl, F, $CHF_2$, $CF_3$, $CH_3$, OH, $CH_3O$, and $NH_2$, and/or a pair of $R^{11}$ groups attached to the same atom forming =O; and independently, when a pair of $R^{11}$ groups attached to different atoms together forms a ring with ring D atoms, the pair of $R^{11}$ groups comprises $-CH_2CH_2CH_2-$.

Clause 38. A compound according to Clause 37, which compound comprises any of the following structures:

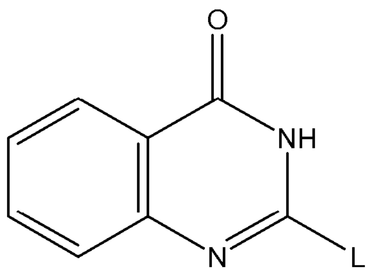
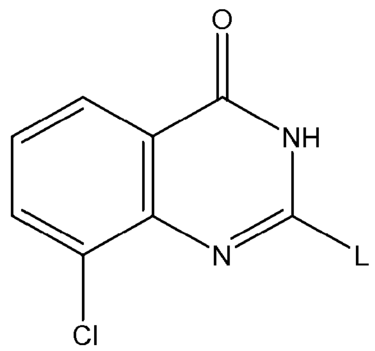
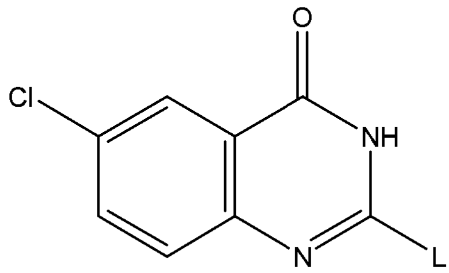
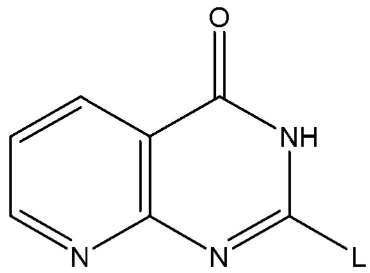
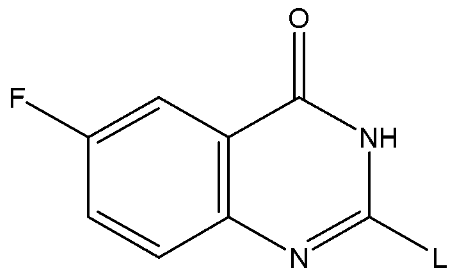
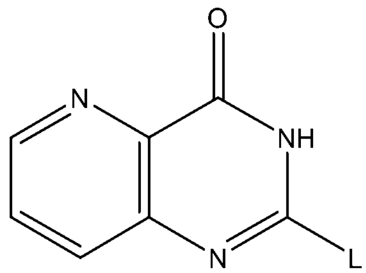
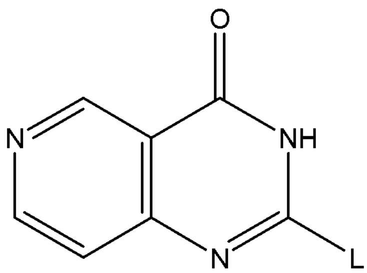
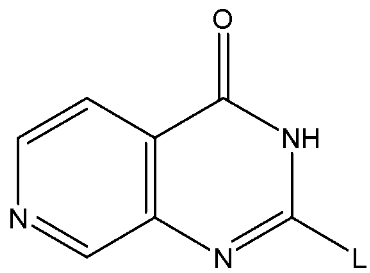
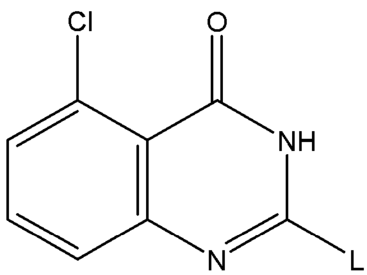
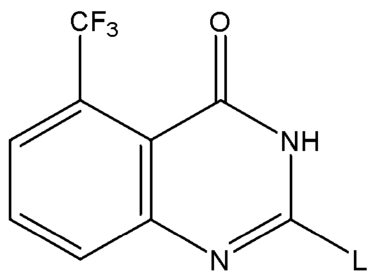

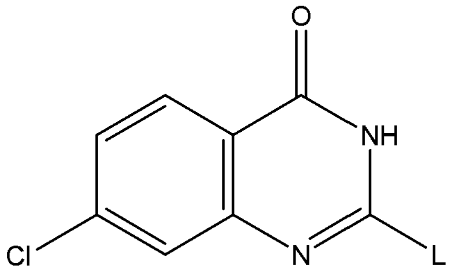

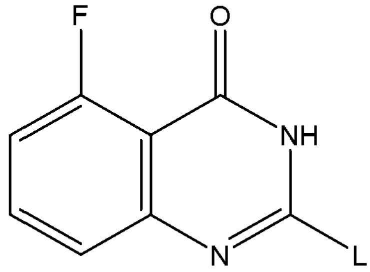

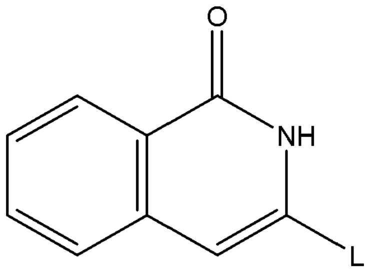

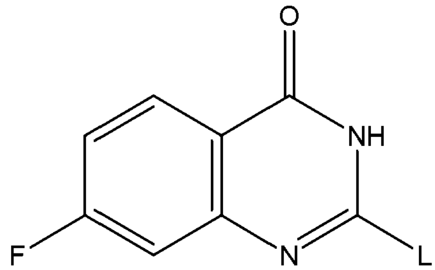

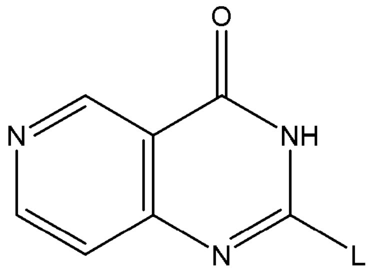

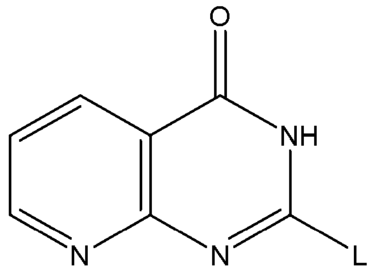

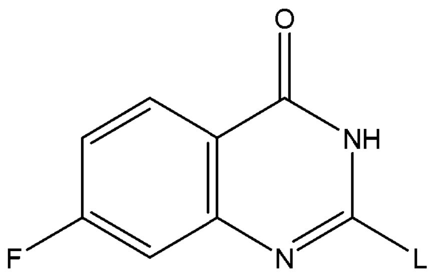

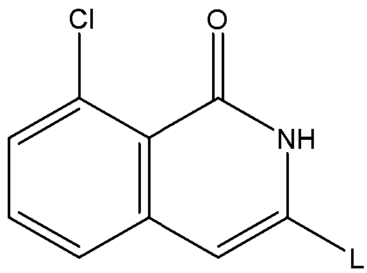

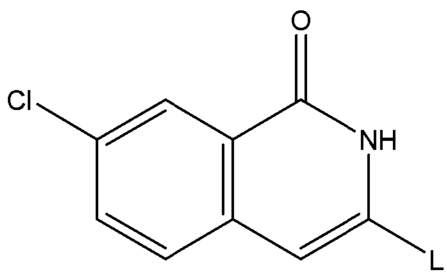

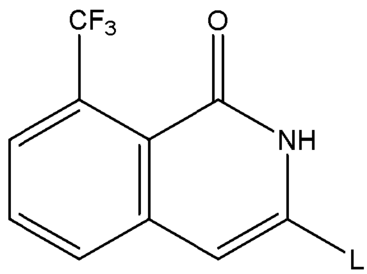

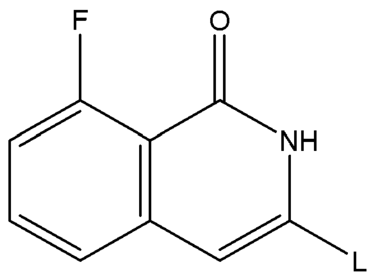
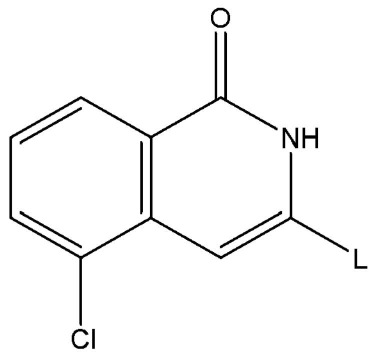
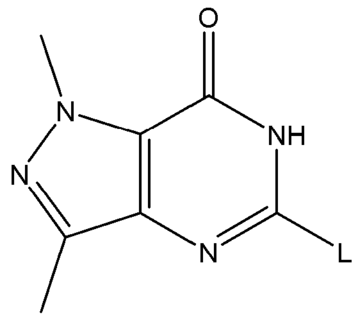
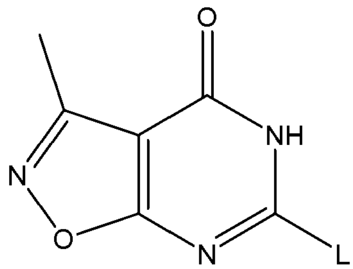
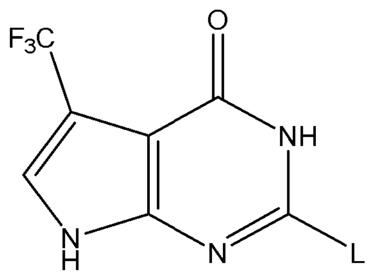
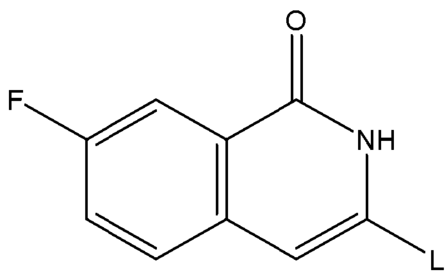
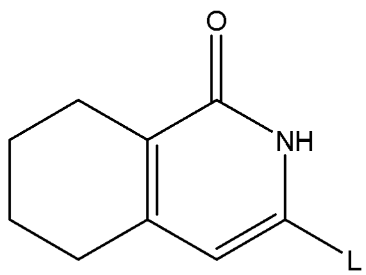
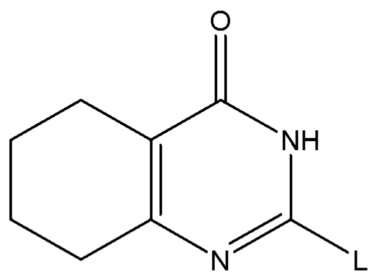
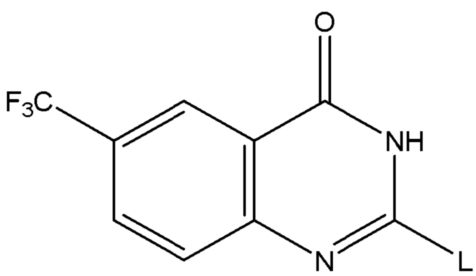
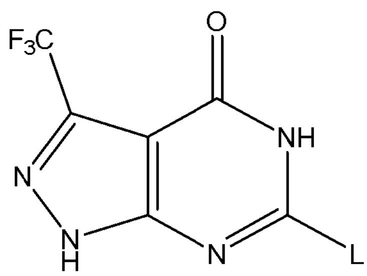

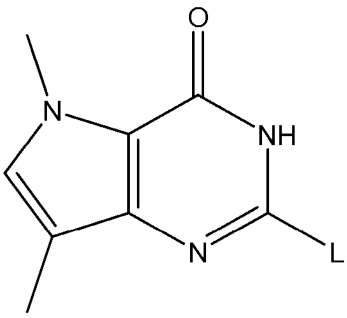

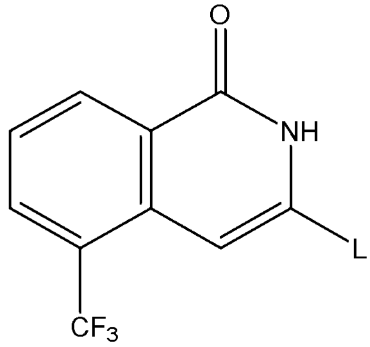

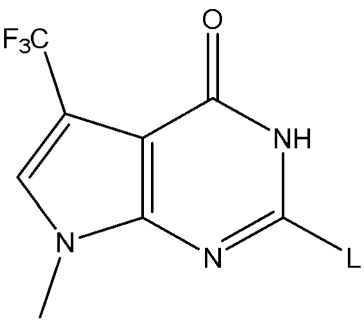

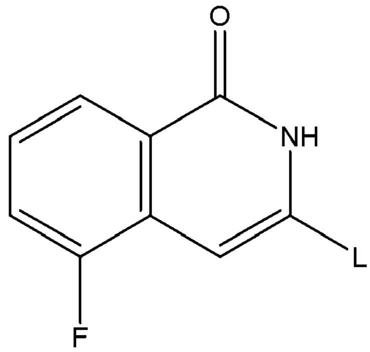

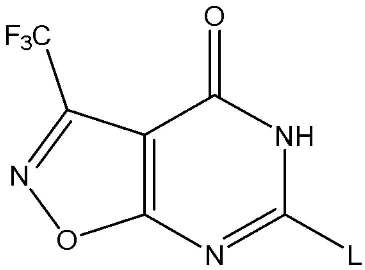

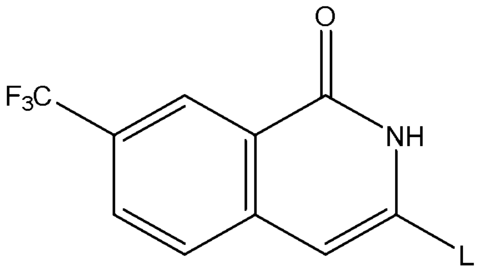

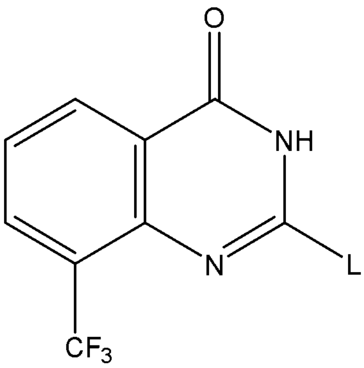

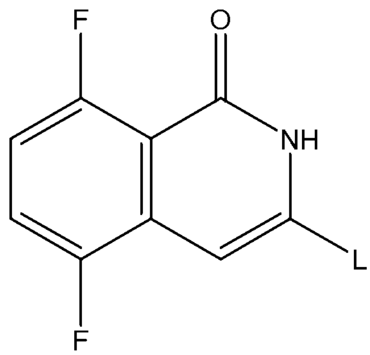

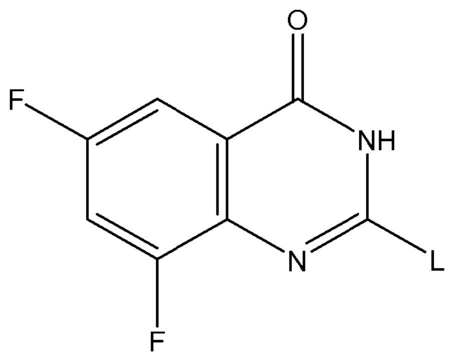
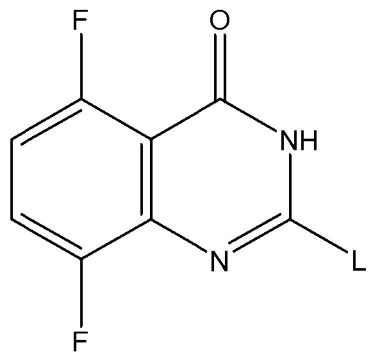
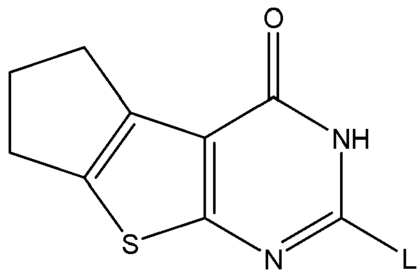
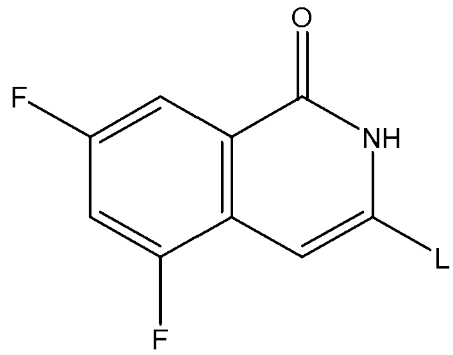
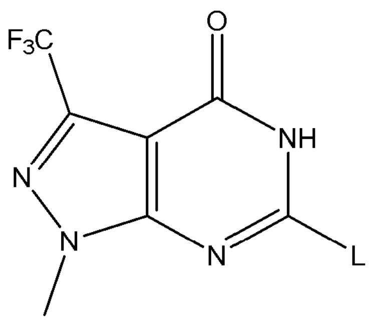
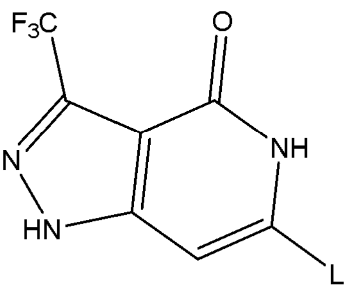
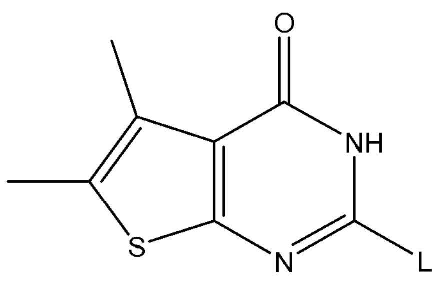
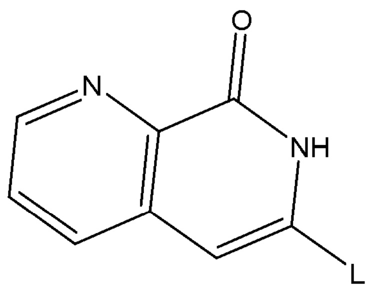
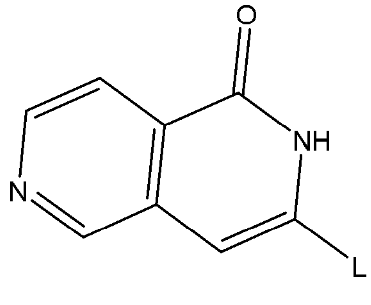
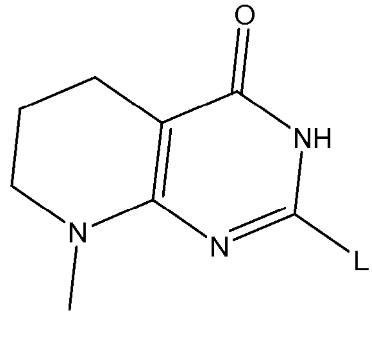

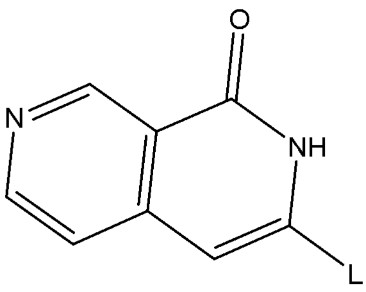

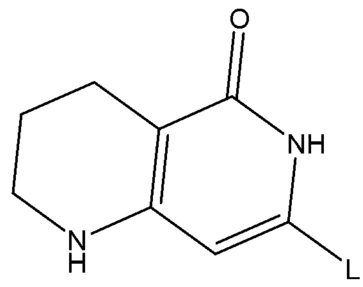

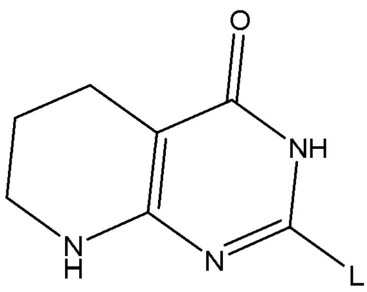

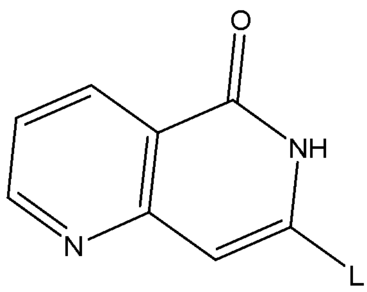

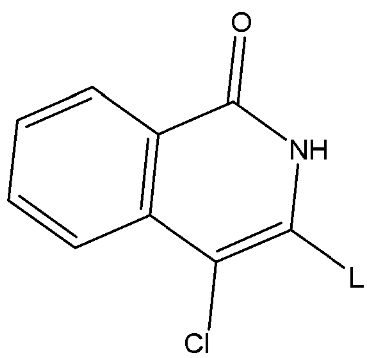

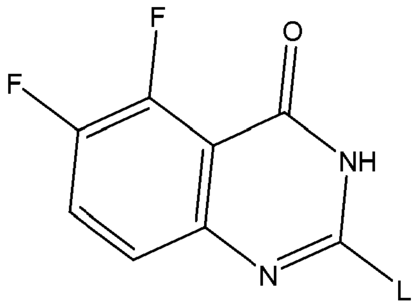

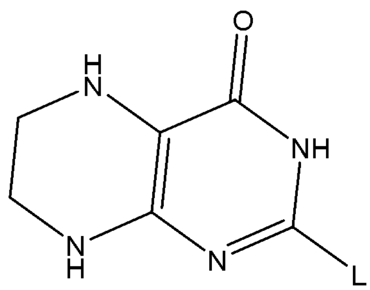

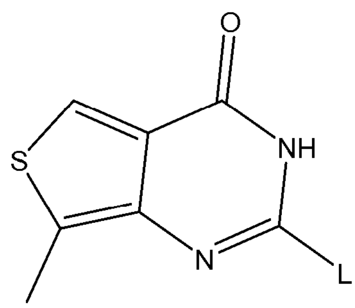

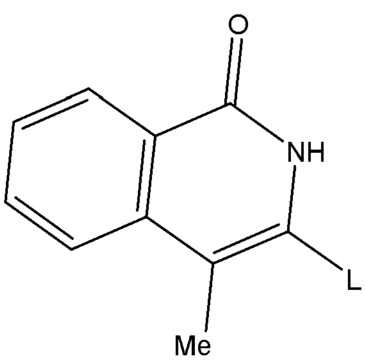

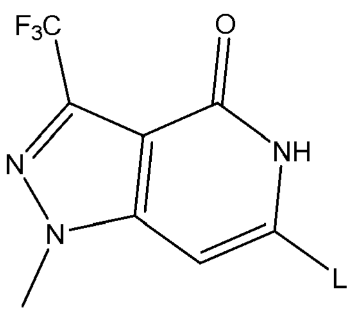

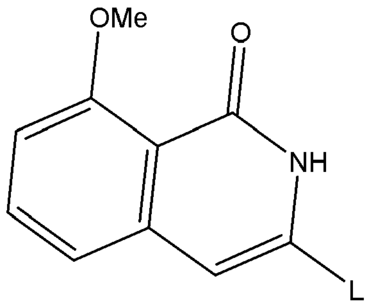
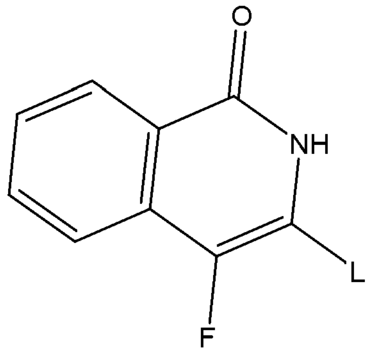
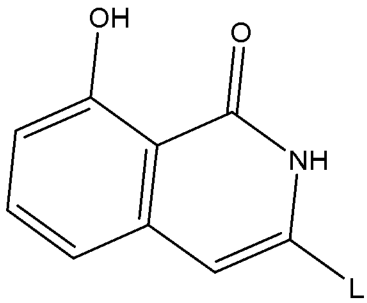
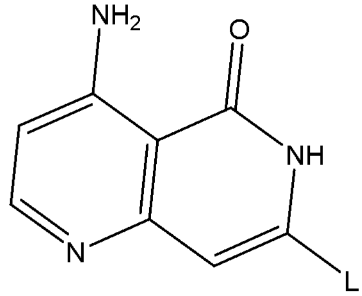
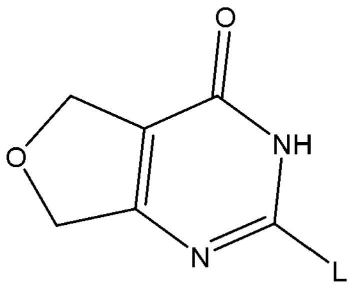
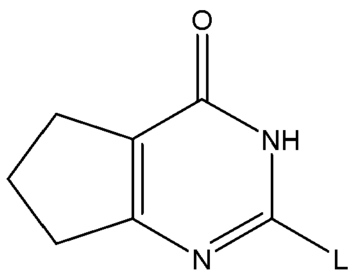
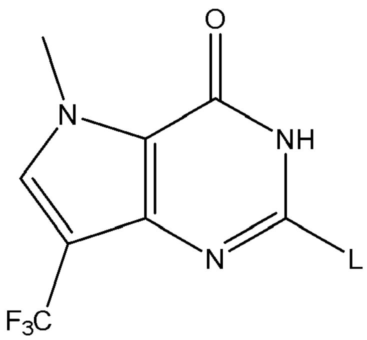
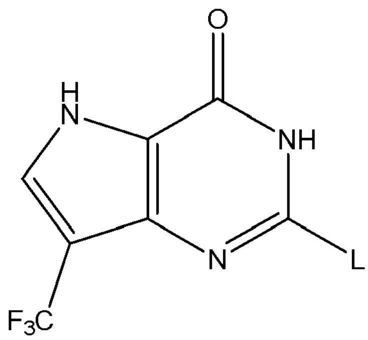
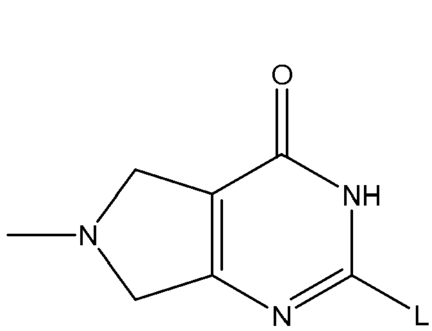
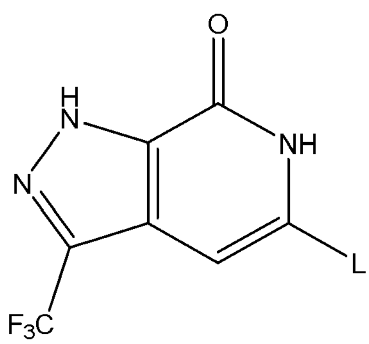

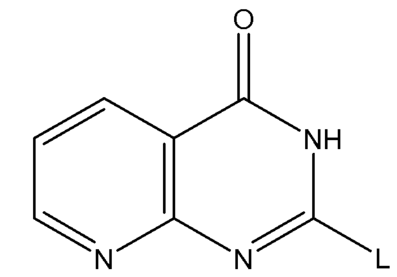

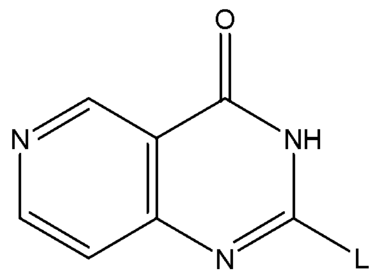

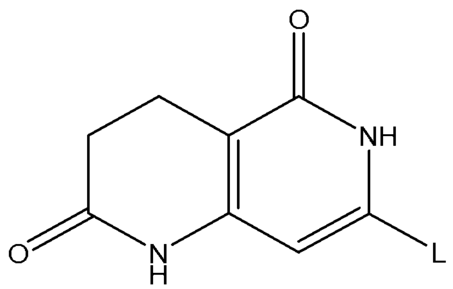

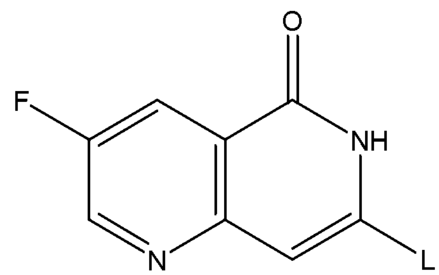

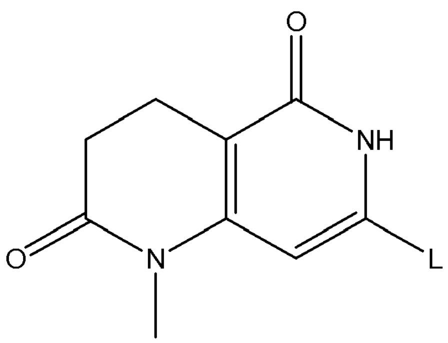

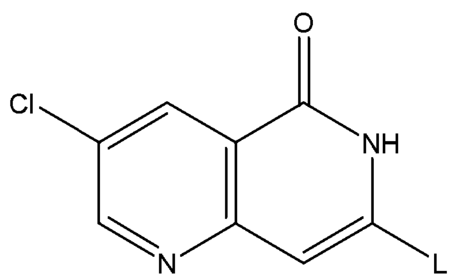

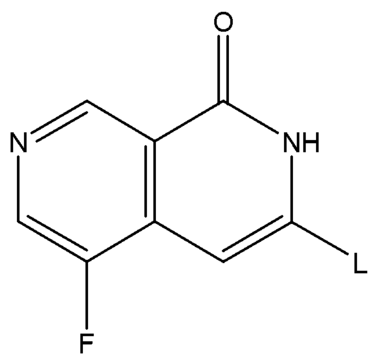

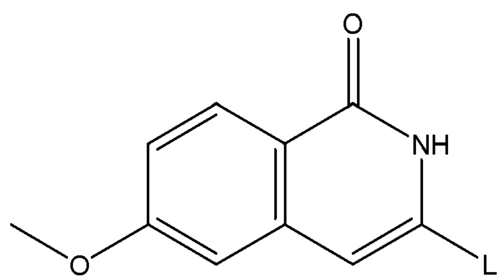

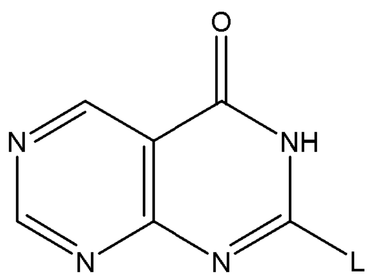

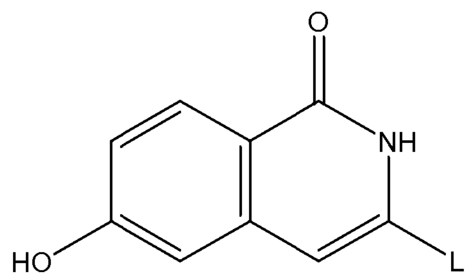

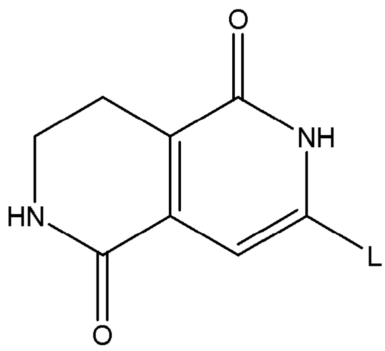

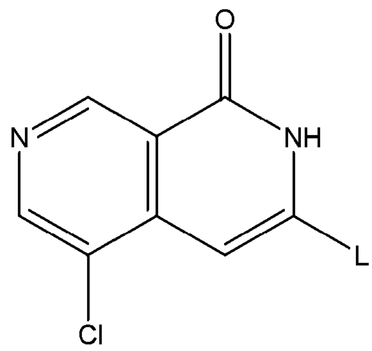

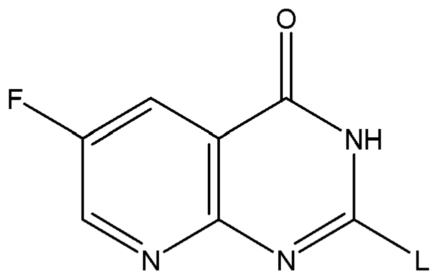

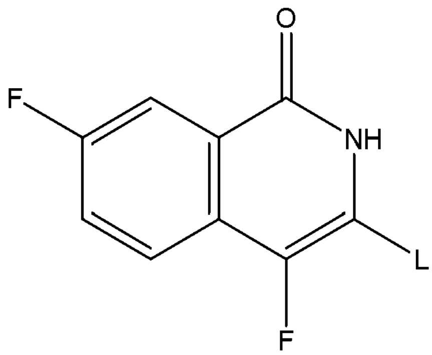

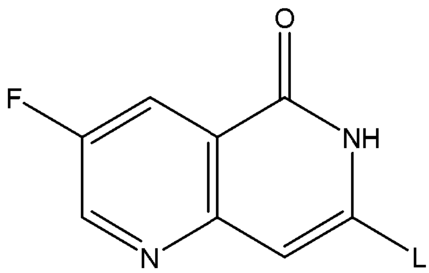

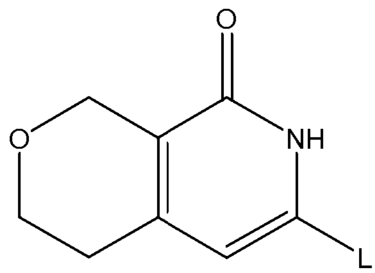

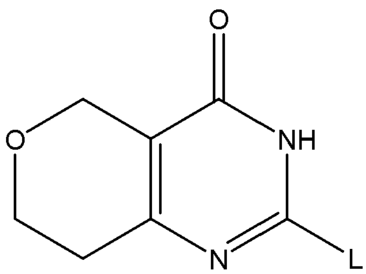

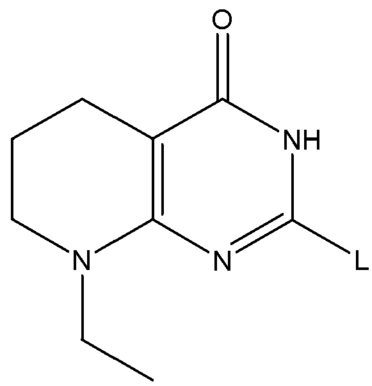

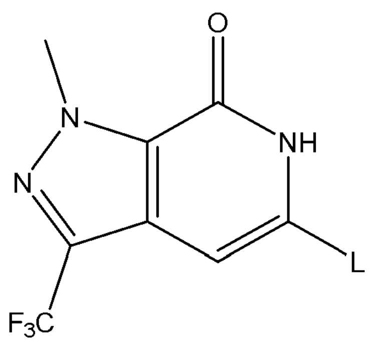

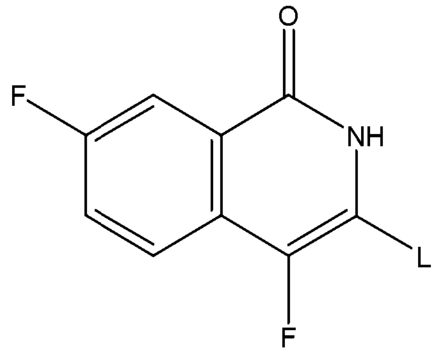

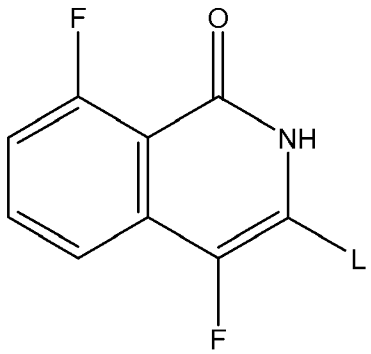

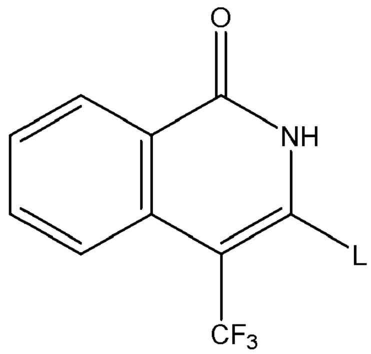

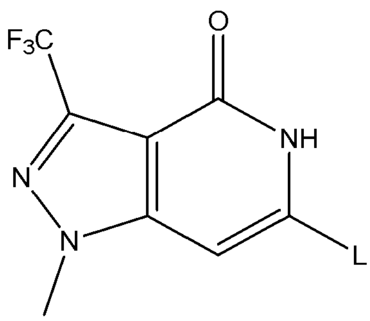

Clause 39. A compound according to any of Clauses 1 to 31, which compound comprises any of the following structures:

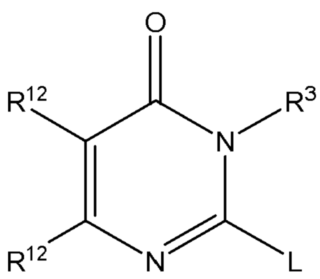

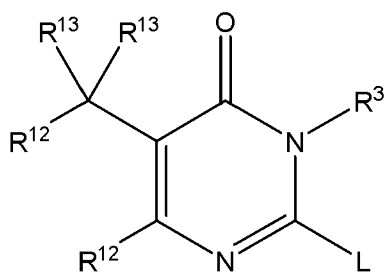

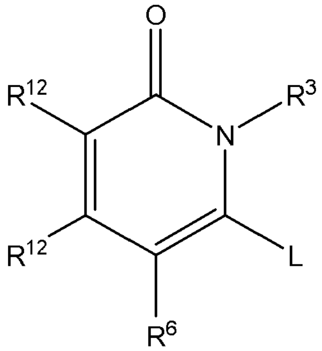
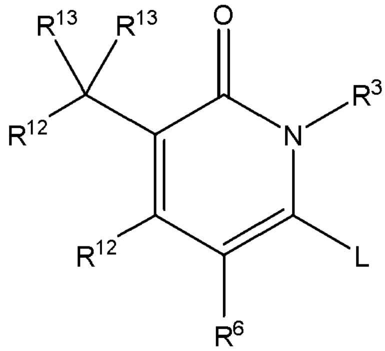

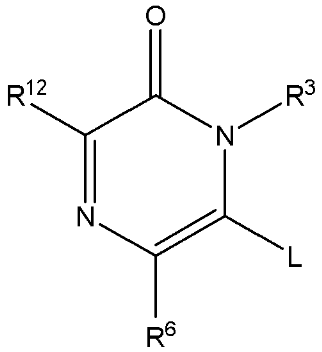
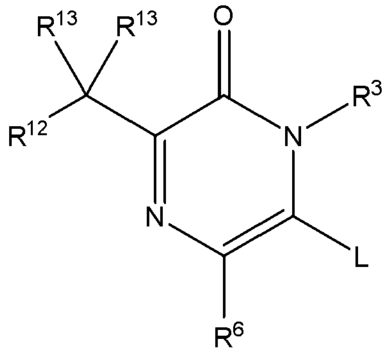

wherein each $R^{12}$ is independently selected from H and a substituted or unsubstituted organic group, preferably a lower ($C_1$ to $C_6$) alkyl, alkoxy or haloalkyl group, a substituted or unsubstituted $C_3$ to $C_6$ cycloalkyl or heterocyclic group, and a halogen group, wherein at least one $R^{12}$ is not H; and wherein each $R^{13}$ may be the same or different and is independently selected from H and a substituted or unsubstituted organic group; and wherein $R^3$, $R^6$, and L are as defined in any preceding Clause.

Clause 40. A compound according to Clause 39, wherein at least one $R^{12}$ is selected from $-CH_3$, $-CH_2CH_3$, $-CH_2CH_2CH_3$, $-CH_2F$, $-CHF_2$, $-CF_3$, -F, -Cl, $-CH_2CF_3$, $-CH_2CH_2F$, $-CH_2CH_2OH$ methoxy, methoxymethyl, methoxyethyl, isopropyl, cyclopropyl or cyclopropylmethyl.

Clause 41. A compound according to Clause 39 or Clause 40, wherein $R^{13}$ is selected from H, F, $C_1$ to $C_3$ alkyl or $C_1$ to $C_3$ fluoroalkyl.

Clause 42. A compound according to any of Clauses 39 to 41, which compound comprises any of the following structures:

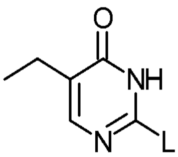
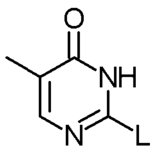
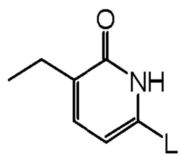
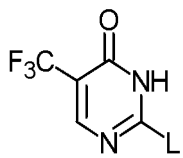

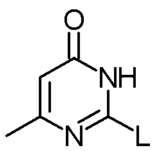
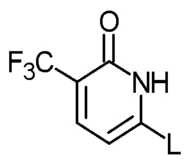
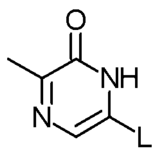
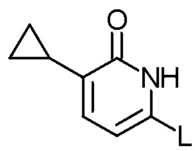

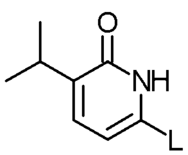
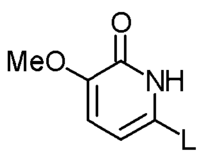
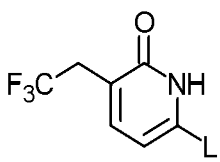
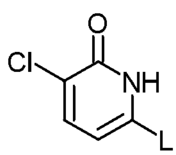

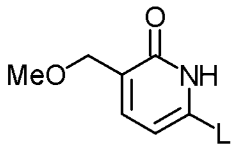
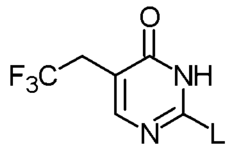
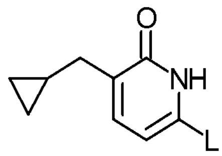
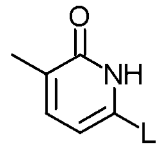
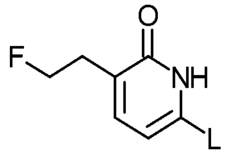
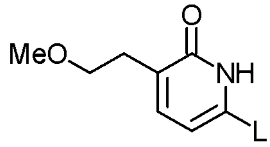
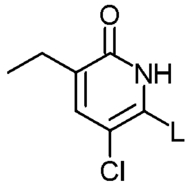
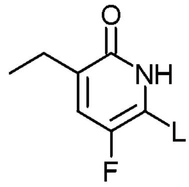
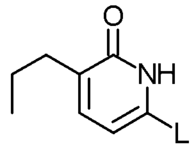
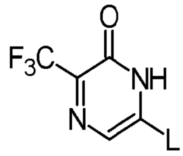
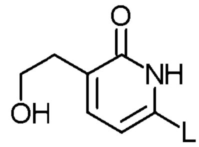
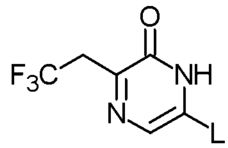
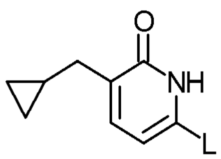
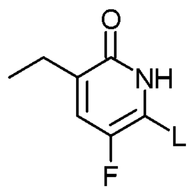
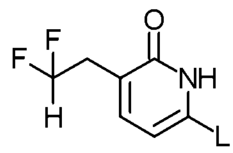

Clause 43. A compound according to any of Clauses 32 to 37 or 39 to 41, wherein $R^3$ is H.

Clause 44. A compound according to any of Clauses 32 to 37 or 39 to 41, wherein $R^6$ is selected from H, halogen, $C_1$ to $C_3$ alkyl, $C_1$ to $C_3$ haloalkyl, $C_1$ to $C_3$ alcohol or $C_1$ to $C_3$ aminooalkyl.

Clause 45. A compound for use in medicine according to any preceding Clause, which compound comprises a formula selected from one of compounds 1 to 415 as defined in the description.

Clause 46. A compound for use in medicine according to any preceding Clause, which compound comprises: - an isolated enantiomer, or - a mixture of two or more enantiomers, or - a mixture of two or more diastereomers, and/or epimers, or - a racemic mixture, or - a tautomer of the compound.

Clause 47. A compound according to any preceding Clause which is selective for PARP1 over PARP2.

Clause 48. A compound for use in treating a cancer, which compound is a compound as defined in any preceding Clause.

Clause 49. A compound according to Clause 48, wherein the cancer is a cancer selected from: a solid or liquid tumour including cancer of the eye, brain (such as gliomas, glioblastomas, medullablastomas, craniopharyngioma, ependymoma, and astrocytoma), spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer, an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes (PHTS) (such as Lhermitte-Duclos disease, Cowden syndrome, Proteus syndrome, and Proteus-like syndrome), leukaemias and lymphomas (such as acute lymphoblastic leukaemia, chronic lymphocytic leukaemia, acute myelogenous leukaemia, chronic myelogenous leukaemia, hairy cell leukaemia, T-cell prolymphocytic leukemia (T-PLL), large granular lymphocytic leukemia, adult T-cell leukemia, juvenile myelomonocytic leukaemia, Hodgkin lymphoma, non-Hodgkin lymphoma, mantle lymphoma, follicular lymphoma, primary effusion lymphoma, AIDS-related lymphoma, diffuse B cell lymphoma, Burkitt lymphoma, cutaneous T-cell lymphoma, nasopharyngeal and gastrointestinal cancers.

Clause 50. A compound according to Clause 48 or Clause 49, wherein the cancer is deficient in a DNA damage response repair pathway, such as Homologous Recombination dependent DNA Double Strand Break DNA repair activity.

Clause 51. A compound according to any of Clauses 48 to 50, wherein the cancer is deficient in BRCA1 and/or BRCA2 function.

Clause 52. A pharmaceutical composition comprising a compound as defined in any of Clauses 1 to 47.

Clause 53. A pharmaceutical composition according to Clause 52, further comprising a pharmaceutically acceptable additive and/or excipient, and/or wherein the compound is in the form of a pharmaceutically acceptable salt, hydrate, acid, ester, or other alternative form of the compound.

Clause 54. A pharmaceutical composition according to Clause 52 or Clause 53, which composition is for treating a cancer as defined in any of Clauses 48 to 51.

Clause 55. A pharmaceutical composition according to Clause 54 for treating a cancer, further comprising a further agent for treating cancer; preferably wherein the further agent for treating cancer is selected from anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, immunotherapeutic agents, hormone-deprivation therapy, proapoptotic agents and cell cycle signalling inhibitors.

Clause 56. A pharmaceutical composition according to Clause 55 for treating a cancer, wherein the further agent is an immunotherapeutic agent selected from an anti-tumour vaccine, an oncolytic virus, an immune stimulatory antibody such as anti-CTLA4, anti-PD1, anti-PDL-1, anti-OX40, anti-41BB, anti-CD27, anti-CD40, anti-LAG3, anti-TIM3, and anti-GITR, a pattern recognition receptor agonist such as a STING, TLR-9 or RIG-I Helicase agonist, an IDO or TDO inhibitor, a novel adjuvant, a peptide, a cytokine, a chimeric antigen receptor T cell therapy (CAR-T), a small molecule immune modulator, tumour microenvironment modulators, and anti-angiogenic agents.

Clause 57. A pharmaceutical kit for treating a cancer, which pharmaceutical kit comprises:

(a) a compound as defined in any of Clauses 1 to 47; and

(b) a further agent for treating cancer; preferably wherein the further agent for treating cancer is selected from anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, hormone-deprivation therapy, immunotherapeutic agents (such as selected from an anti-tumour vaccine, an oncolytic virus, an immune stimulatory antibody such as anti-CTLA4, anti-PDl, anti-PDL-1, anti-OX40, anti-41BB, anti-CD27, anti-CD40, anti-LAG3, anti-TIM3, and anti-GITR, a pattern recognition receptor agonist such as a STING, TLR-9 or RIG-I Helicase agonist, an IDO or TDO inhibitor, a novel adjuvant, a peptide, a cytokine, a chimeric antigen receptor T cell therapy (CAR-T), a small molecule immune modulator, tumour microenvironment modulators, and anti-angiogenic agents), proapoptotic agents and cell cycle signalling inhibitors;

wherein the compound and the further agent are suitable for administration simultaneously, sequentially or separately.

Clause 58. A method of treating a disease and/or a condition and/or a disorder, which method comprises administering to a patient a compound or a composition or a kit as defined in any preceding Clause.

Clause 59. A method according to Clause 58, wherein the disease or condition or disorder is a disease or condition or disorder as defined in any of Clauses 48 to 51.

Clause 60. A method according to Clause 59 for treating a cancer, which method comprises administering to a patient a compound or a composition as defined in any of Clauses 1 to 50 and a further agent for treating a cancer as defined in Clause 55 or Clause 56; preferably wherein the compound or composition and the further agent are administered simultaneously, sequentially or separately.

Clause 61. A method according to any of Clauses 58 to 60, wherein the patient is an animal, preferably a mammal, including canines, equines and felines, and more preferably a human.

Clause 62. A compound, which is selected from any of the compounds defined in Clause 45.

Clause 63. A compound according to Clause 62, which compound comprises:

- an isolated enantiomer, or
- a mixture of two or more enantiomers, or
- a mixture of two or more diastereomers, and/or epimers, or
- a racemic mixture, or
- a tautomer of the compound.

Clause 64. A method of synthesis of a PARP1 inhibitor compound as defined in any of Clauses 1 to 47, which method comprises conducting a reaction between (i) a first reactant comprising ring E bearing a portion of substituent group L and (ii) a second reactant comprising the remainder of substituent group L, so as to form the PARP1 inhibitor compound.

Clause 65. A method according to Clause 64, wherein the first reactant comprises ring E and ring A, and the second reactant comprises a Q1 or Q2 precursor bearing a reactive group, which method comprises joining the N atom of ring A to the Q1 or Q2 precursor.

Clause 66. A method according to Clause 65, wherein the reactive group of the Q1 or Q2 precursor comprises a carbonyl group, an alkyl halide or an alkyl sulfonate.

Clause 67. A method according to any of Clauses 64 to 66, wherein the reaction comprises alkylation, reductive amination or amide formation so as to form group L.

Clause 68. A method according to Clause 64, wherein the first reactant comprises ring E, ring A, and at least one of Q1 and Q2, and the second reactant comprises a ring C derivative bearing a leaving group such as a halide or sulfonate.

Clause 69. A method according to Clause 68, wherein the reaction comprises a nucleophilic substitution reaction, such as a nucleophilic aromatic substitution reaction, so as to form group L.

## Claims

**1.** A PARP1 inhibitor compound for use in medicine, which compound has any of the following structures:

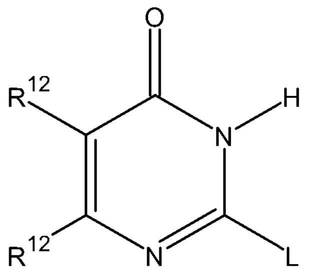
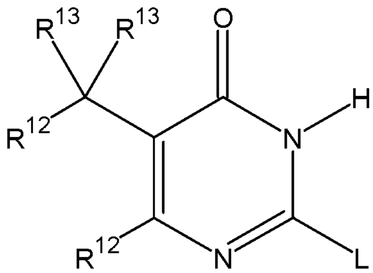
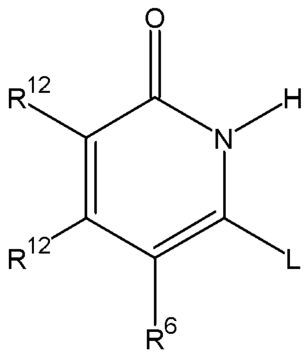
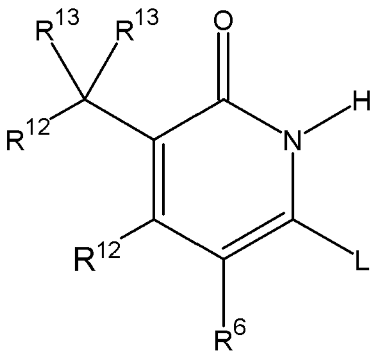
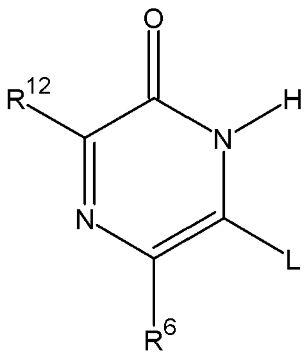
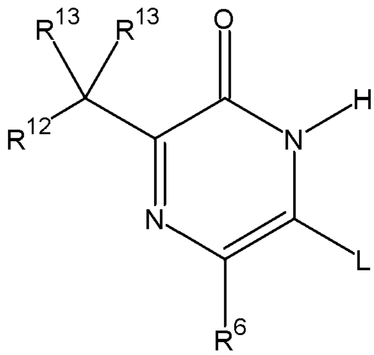

wherein:

each $R^{12}$ is independently selected from H, $-CH_3$, $-CH_2CH_3$, $-CH_2CH_2CH_3$, $-CH_2F$, $-CHF_2$, $-CF_3$, -F, -Cl, $-CH_2CF_3$, $-CH_2CH_2F$, $-CH_2CH_2OH$, methoxy, methoxymethyl, methoxyethyl, isopropyl, cyclopropyl, and cyclopropylmethyl; with the proviso that at least one $R^{12}$ is not H;

each $R^{13}$ is independently selected from H, F, $C_1$ to $C_3$ alkyl, and $C_1$ to $C_3$ fluoroalkyl;

$R^6$ is selected from H, halogen, $C_1$ to $C_3$ alkyl, $C_1$ to $C_3$ haloalkyl, $C_1$ to $C_3$ alcohol, and $C_1$ to $C_3$ aminooalkyl; and L has the following structure:

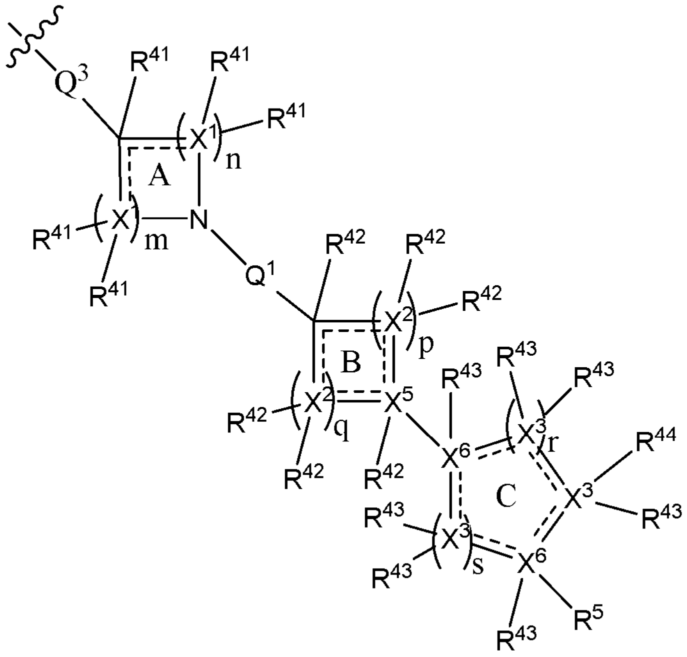

wherein:

n is 0, 1, 2, 3, 4, 5 or 6; m is 0, 1, 2, 3, 4, 5 or 6; and m + n is 2, 3, 4, 5, or 6;
p is 0, 1, 2, 3, 4, 5 or 6; q is 0, 1, 2, 3, 4, 5 or 6; and p + q is 2, 3, 4, 5, or 6;
r is 0, 1, 2, 3, 4, or 5; s is 0, 1, 2, 3, 4, or 5; and r + s is 1, 2, 3, 4, or 5;
each $X^1$ is independently selected from C, N, O and S;
each $X^2$ is independently selected from C, N, O and S;
each $X^3$ is independently selected from C, N, O and S;
$X^5$ is selected from C and N;
each $X^6$ is independently selected from C and N;
$Q^1$ and $Q^3$ are each independently absent or selected from:

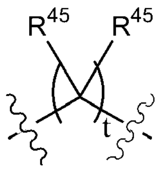

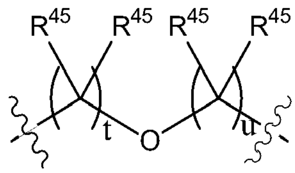

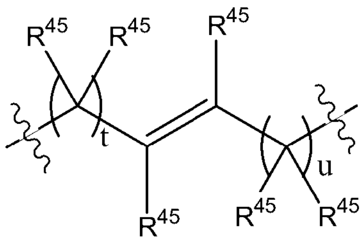

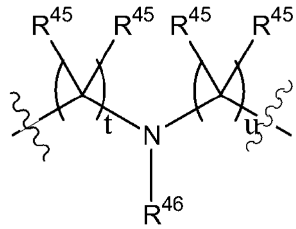

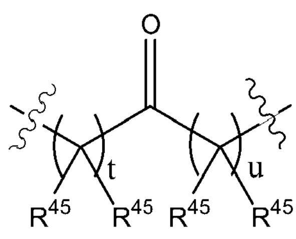

wherein t is 0, 1, 2, 3, 4 or 5; u is 0, 1, 2, 3, 4 or 5; t + u is 0, 1, 2, 3, 4, 5 or 6; and when $Q^1$ and $Q^3$ are both present, values of each t and each u are selected independently;

the dotted lines indicate that the rings A, B, and C each independently comprise single bonds or a combination of both single and double bonds, and are aliphatic or aromatic;

each $R^{41}$, $R^{42}$, $R^{43}$, and $R^{44}$ is independently absent or selected from H, deuterium, a halogen, a nitrile group, a $C_1$-$C_6$ alkyl group, a linear or branched $C_1$-$C_6$ halogenated alkyl group, a cyclopropyl group, an -OH group, a linear or

branched $C_1$-$C_6$ alcohol group, a linear or branched $C_1$-$C_7$ amino carbonyl group, an $-NH_2$ group, a $C_1$-$C_6$ amino group, and a $C_1$-$C_6$ alkoxy group; and

when a pair of $R^{41}$ groups attached to different atoms together forms a ring with ring A atoms, and/or a pair of $R^{42}$ groups attached to different atoms together forms a ring with ring B atoms, each of the pair of $R^{41}$ groups and/or pair of $R^{42}$ groups independently comprises $-CH_2-$ or $-CH_2CH_2-$;

each $R^{45}$ is independently selected from H, a halogen, a $C_1$-$C_6$ alkyl group, a linear or branched $C_1$-$C_6$ halogenated alkyl group, an $-NH_2$ group, a $C_1$-$C_6$ amino group, an -OH group, a linear or branched $C_1$-$C_6$ alcohol group, and a substituted or unsubstituted $C_1$-$C_6$ alkoxy group;

$R^{46}$ is selected from H, a $C_1$-$C_6$ alkyl group, and a linear or branched $C_1$-$C_6$ halogenated alkyl group; and

$R^5$ is selected from -F, -Cl, -CN, $-CONH_2$, -CONHMe, -CONHEt, $-CONMe_2$, -CONHCOMe, $-CONHCH_2-CH_2OMe$, $-CONH-CH_2-CH_2F$, $-CONH-CH_2-CF_3$, $-CONH-CH_2-CHF_2$, $-OCHF_2$, -NHCOMe, $-NHSO_2Me$,

$-SO_2NHMe$, $-CONHSO_2Me$,

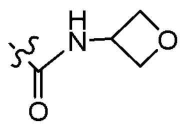 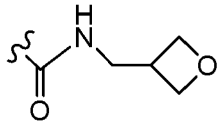 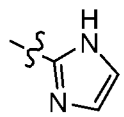

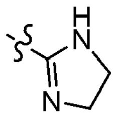 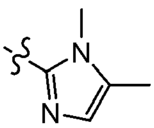 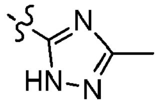 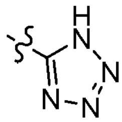 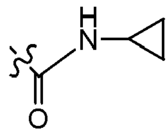 and 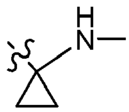 .

2. A compound for use according to claim 1, wherein $Q^3$ is absent or is a group independently selected from the following structures:

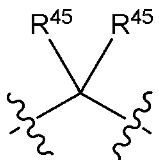 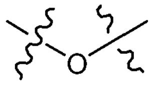 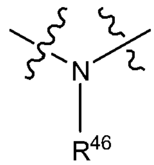 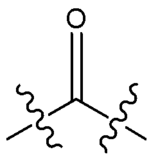 .

3. A compound for use according to claim 1 or claim 2, wherein ring A of the group L is selected from:

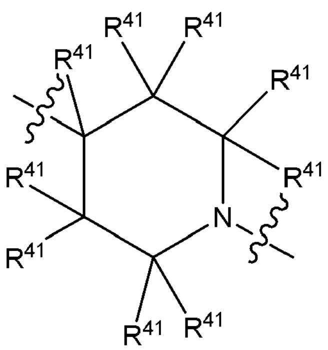 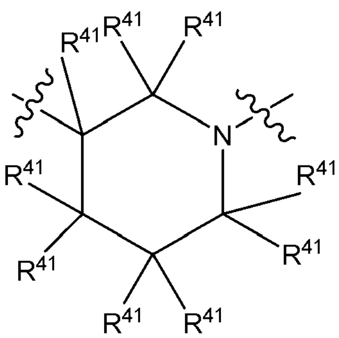 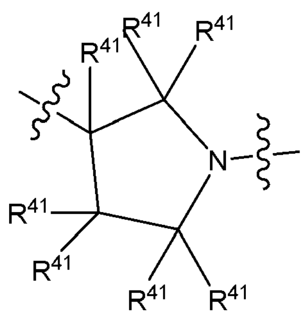

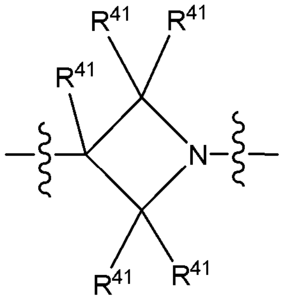
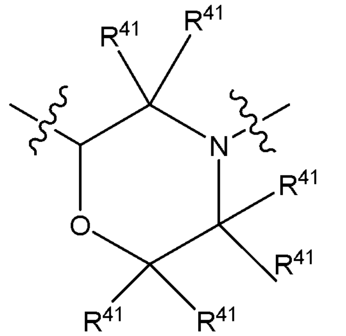
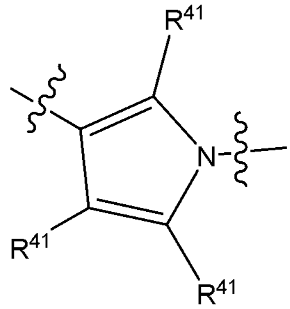

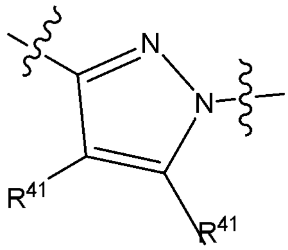
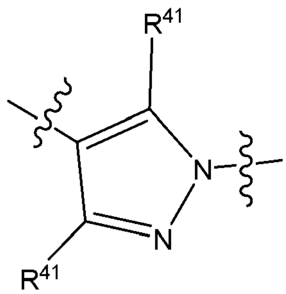
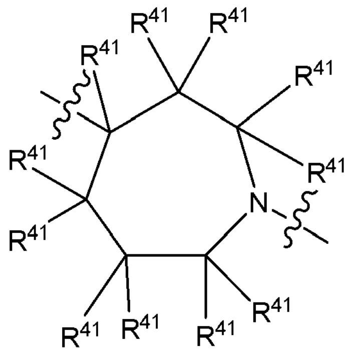

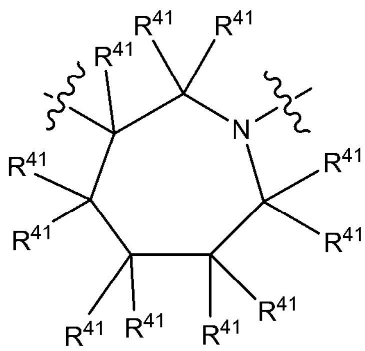

optionally wherein ring A of the group L is selected from:

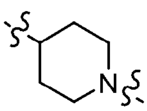
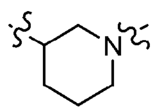
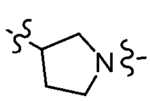
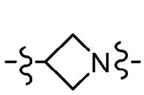
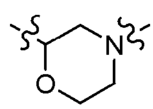
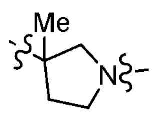
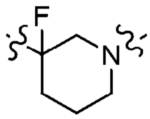

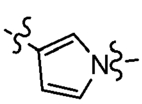
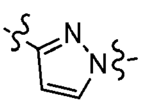
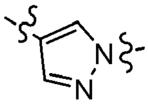
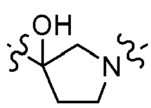
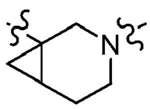
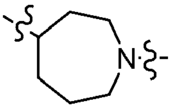
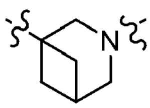

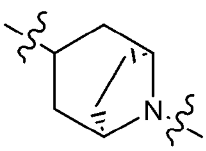
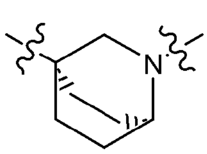
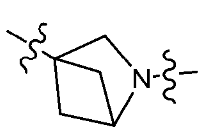
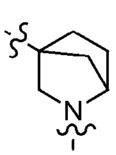
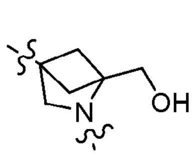
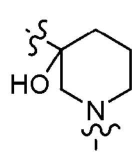

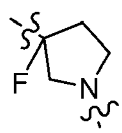 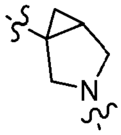 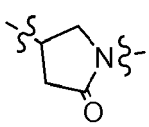 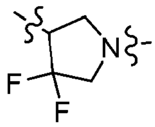 .

**4.** A compound for use according to any preceding claim, wherein ring C of the group L is selected from:

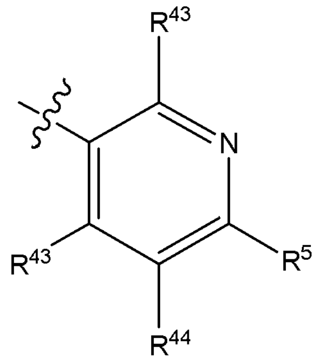 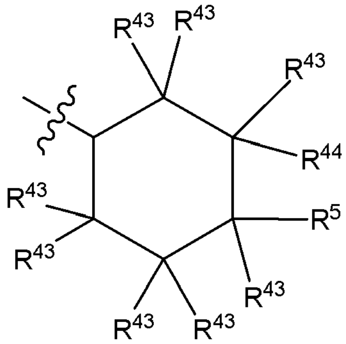 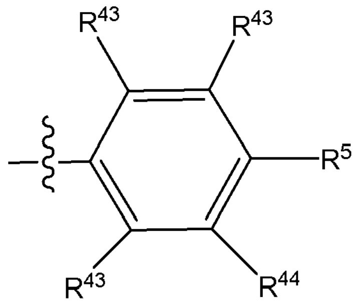

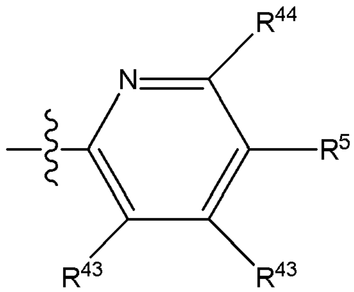 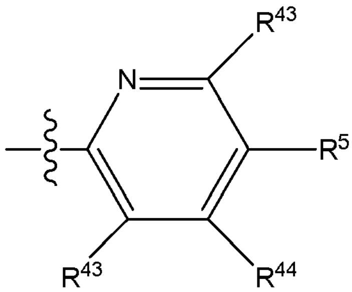 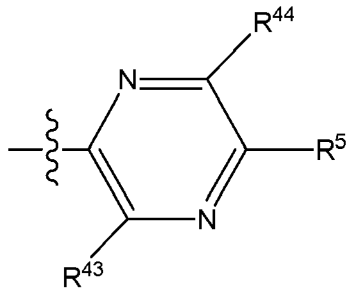

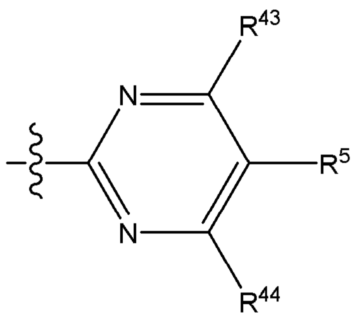 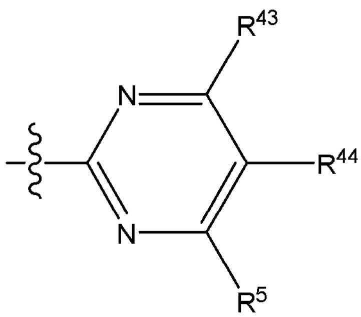 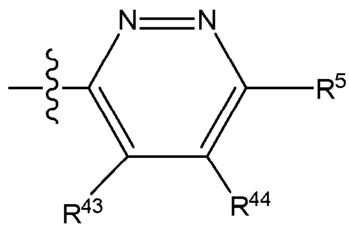

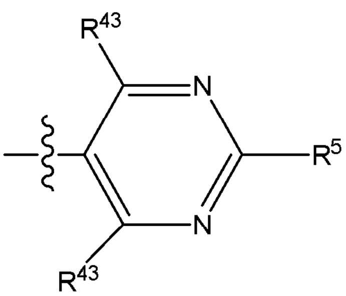 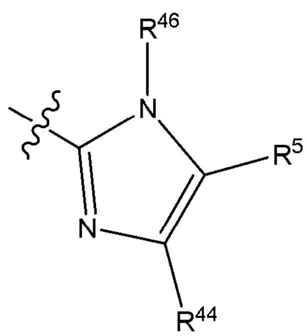 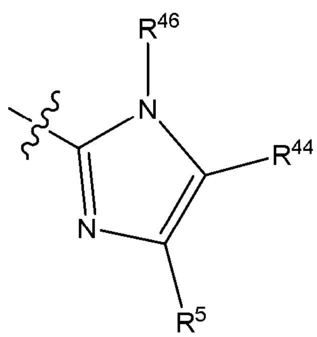

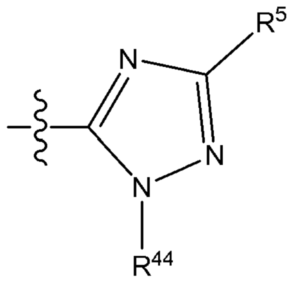 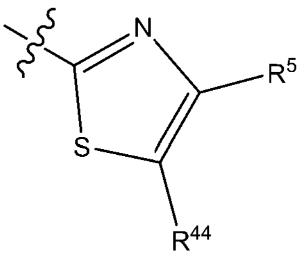 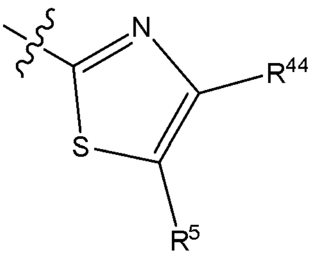

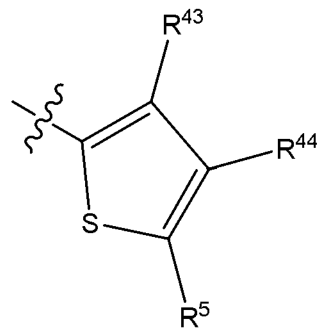 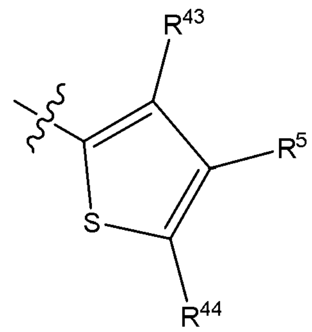 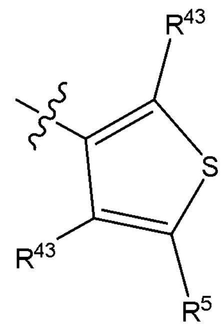

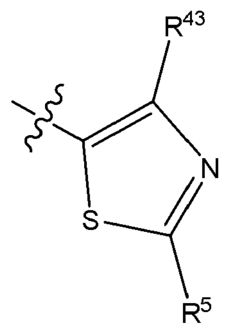

optionally wherein ring C of the group L comprises any of the following structures:

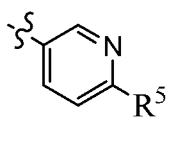 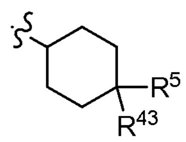 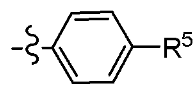 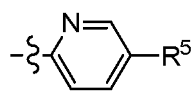 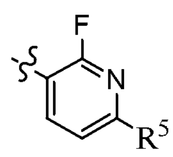

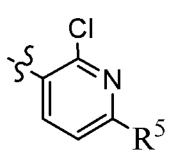 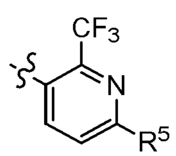 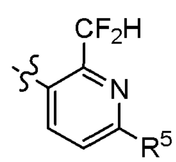 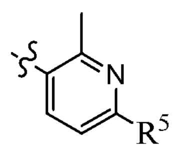 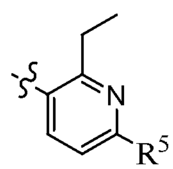

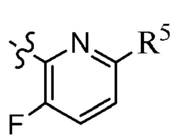 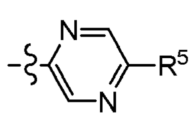 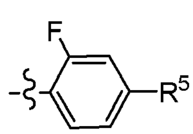 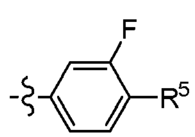 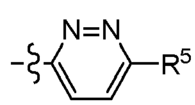

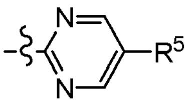 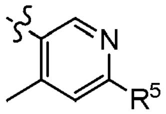 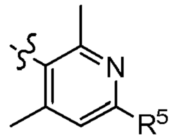 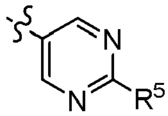 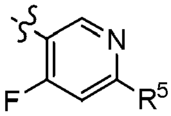

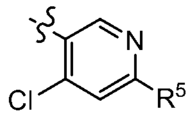 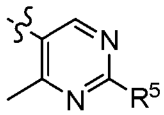 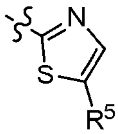 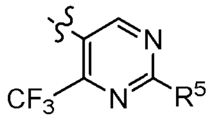 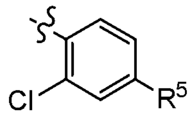

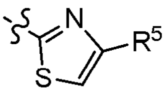 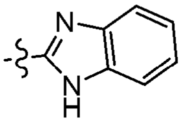 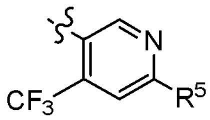 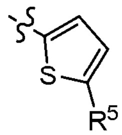 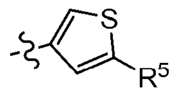

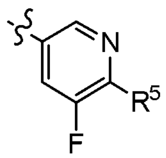 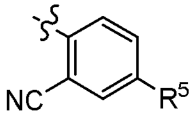 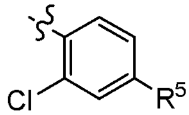 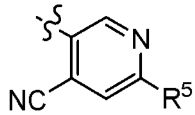 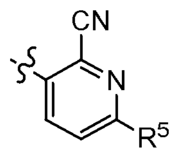

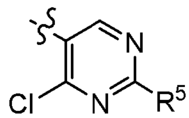 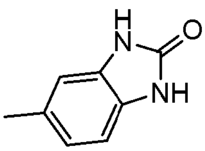

**5.** A compound for use according to any preceding claim, wherein $Q^1$ is absent or selected from:

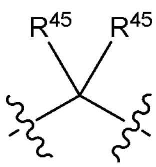 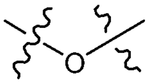 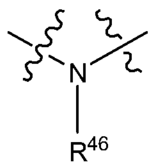 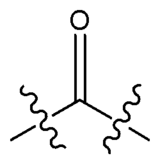

optionally wherein $X^5$ is N; and
further optionally wherein ring B of the group L is selected from:

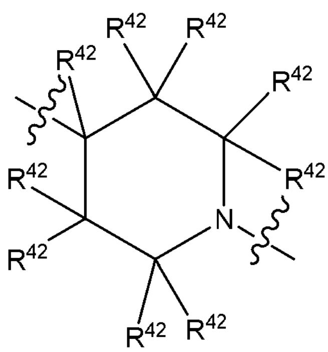 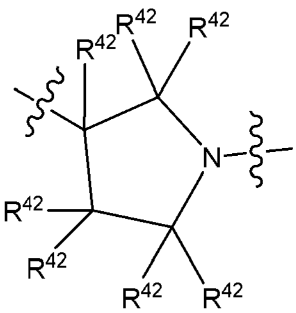 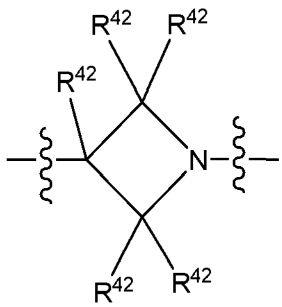

**6.** A compound for use according to claim 5, wherein ring B of the group L has any of the following structures:

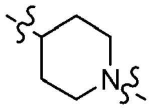
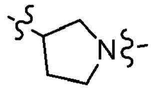
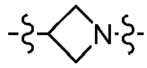

7. A compound for use according to any preceding claim, wherein the group L has the following structure:

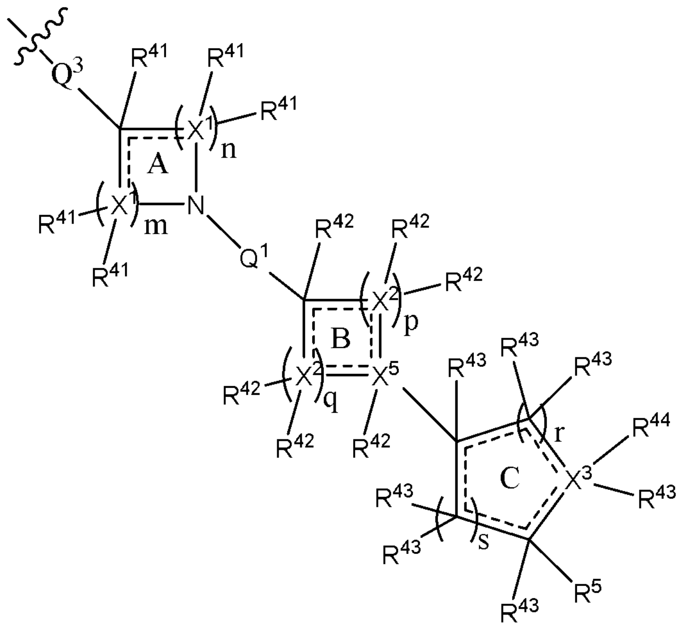

optionally wherein each $X^2$ is C;
further optionally wherein each $X^l$ is C; and
still further optionally wherein the bonds in rings A and B are single bonds.

8. A compound for use according to any of claims 1 to 6, wherein the group L has the following structure:

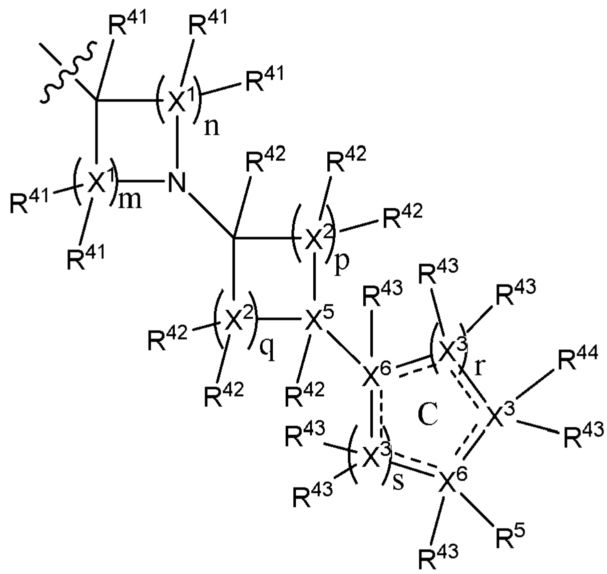

optionally wherein group L has a structure selected from:

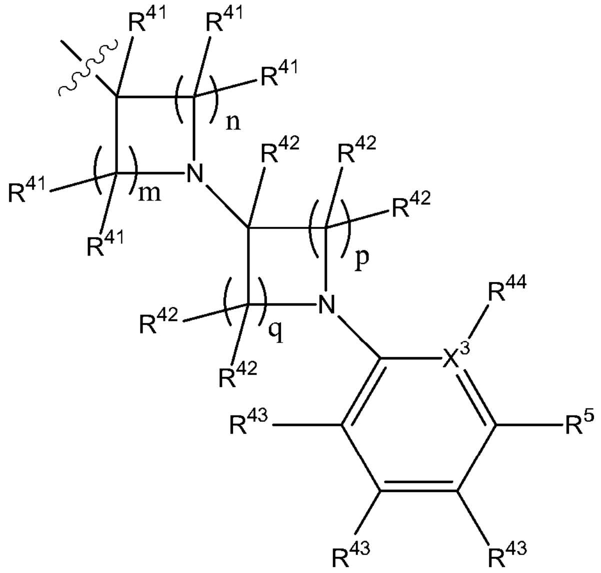

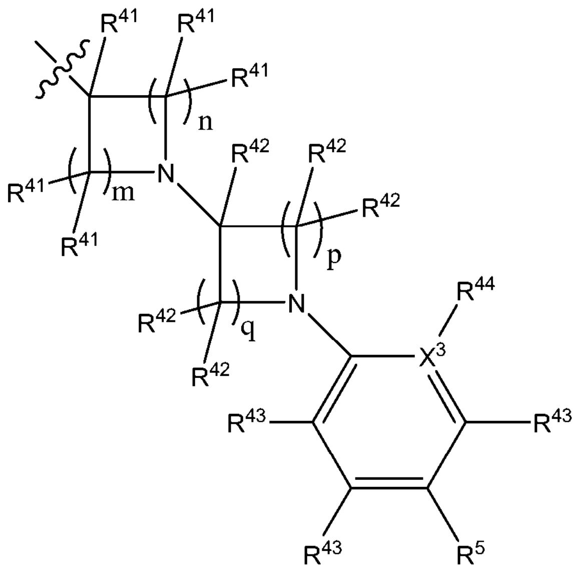

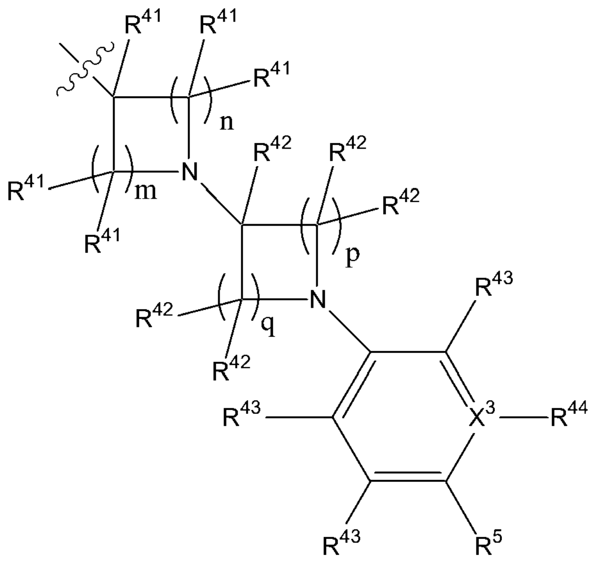

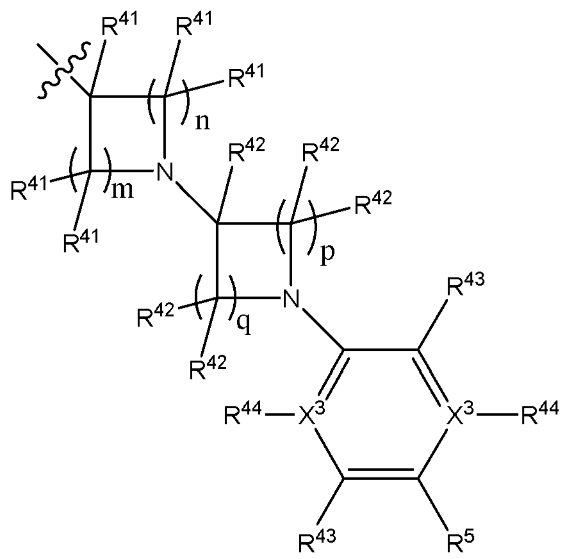

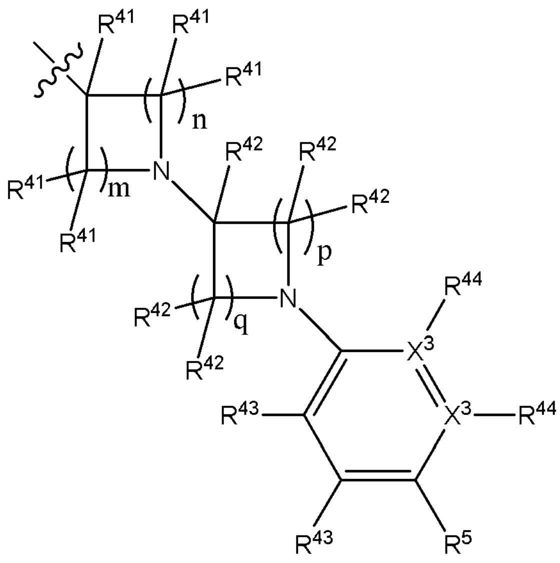

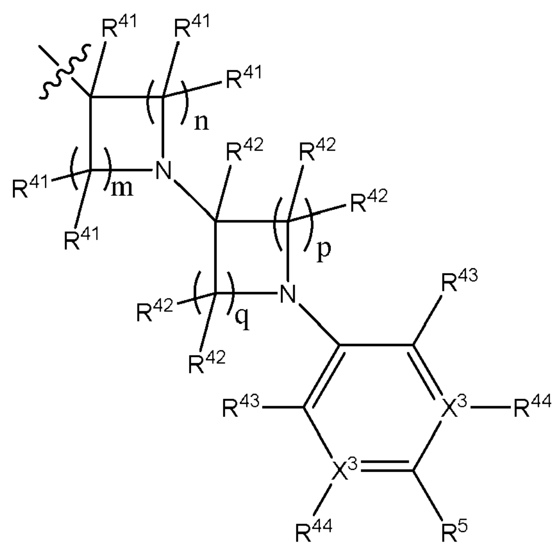

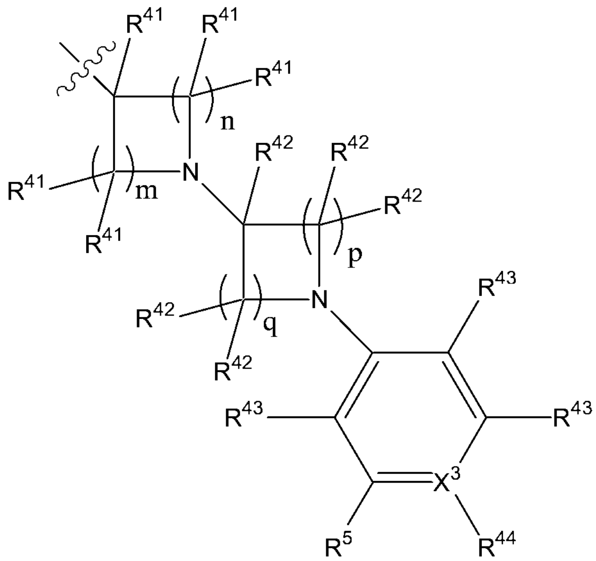

.

9. A compound for use according to any preceding claim, wherein m is selected from 1 or 2, n is selected from 2 or 3, p is selected from 1, 2 or 3 and q is selected from 1 or 2;
optionally wherein the group L has any the following structures:

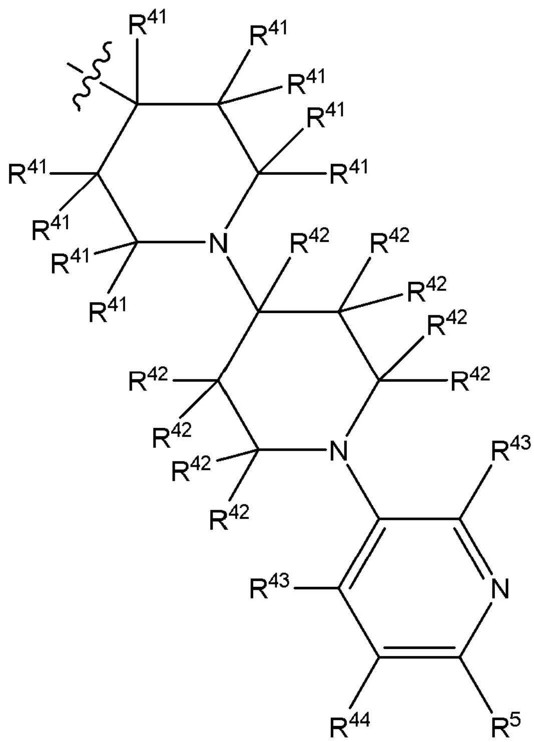

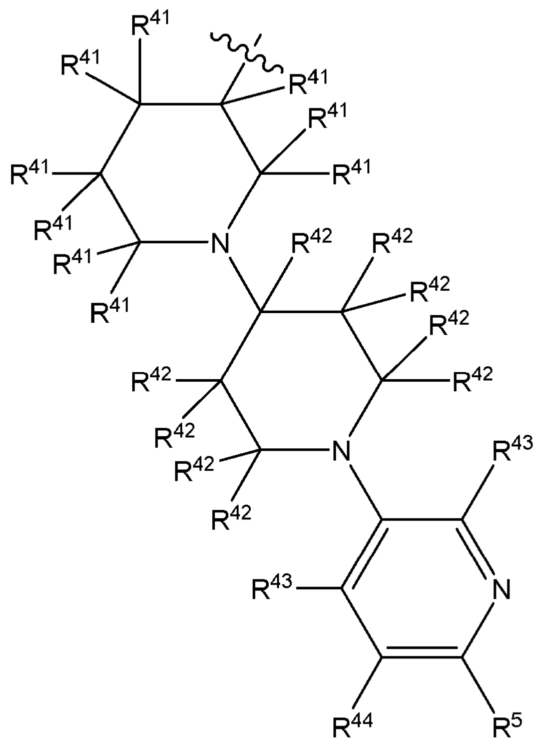

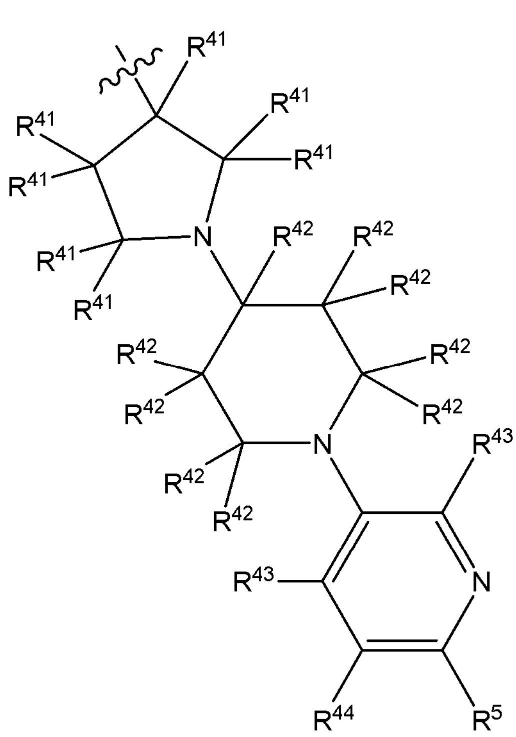
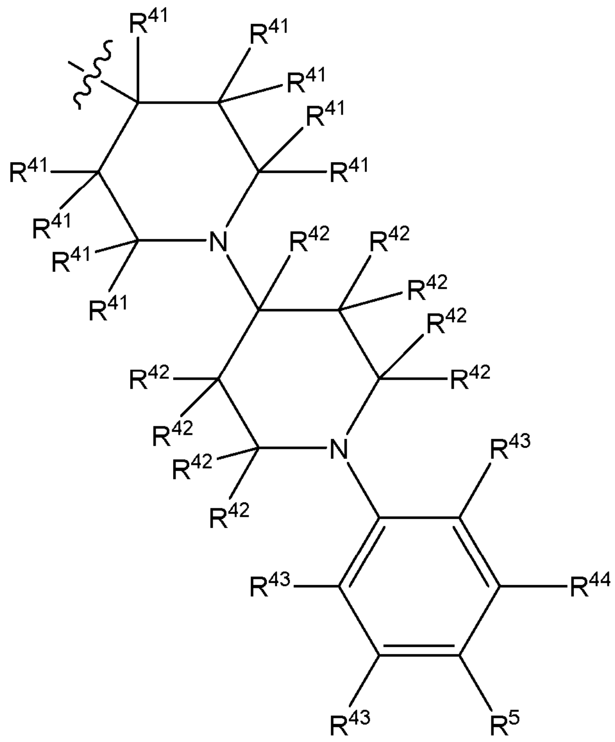
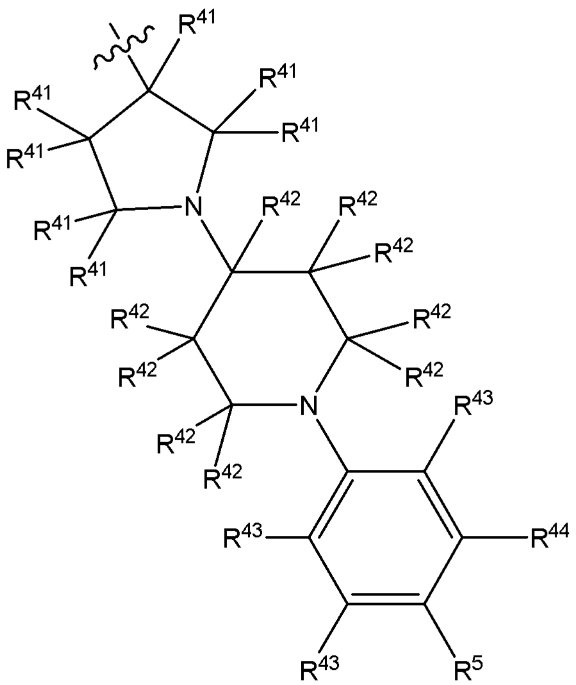
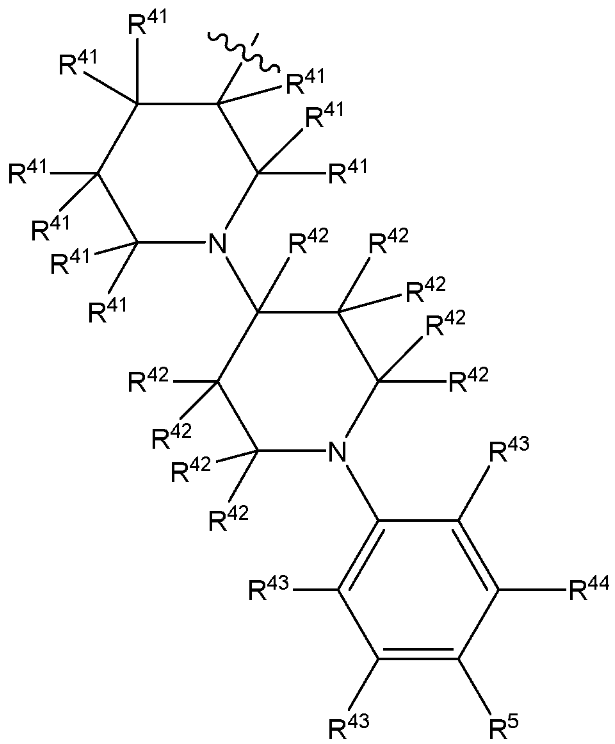
.

**10.** A compound for use according to any preceding claim, wherein the group L has any the following structures:

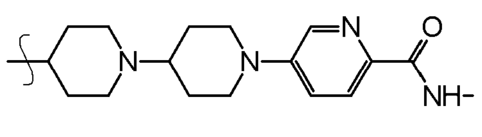
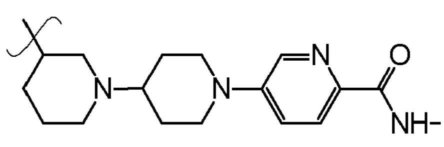

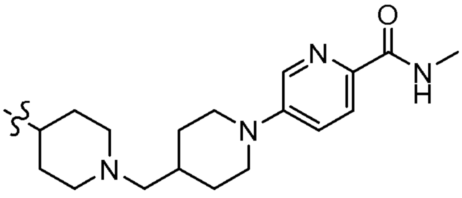

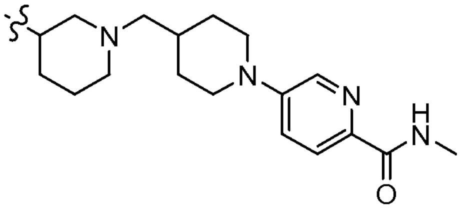

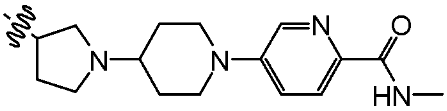

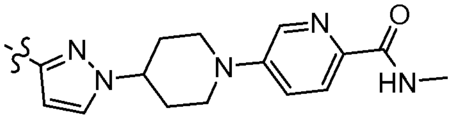

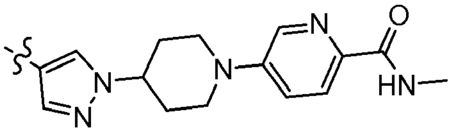

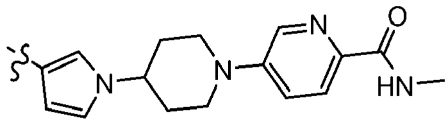

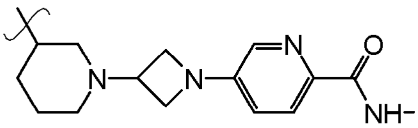

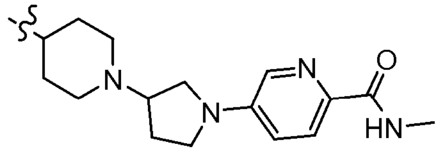

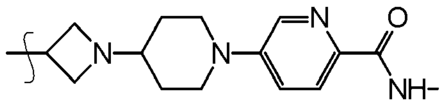

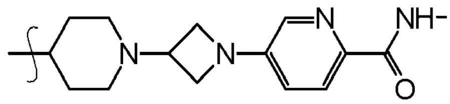

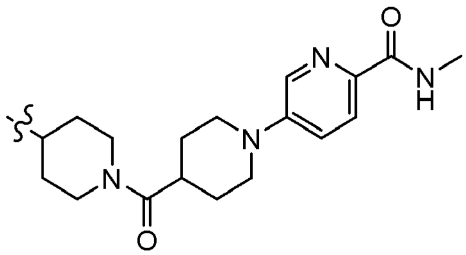

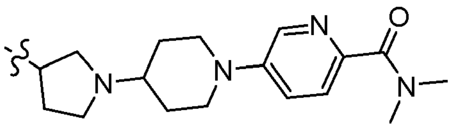

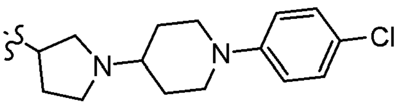

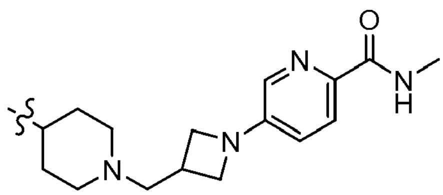

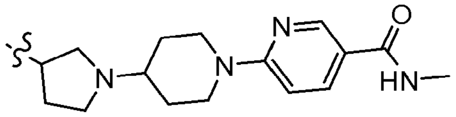

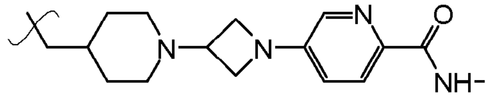

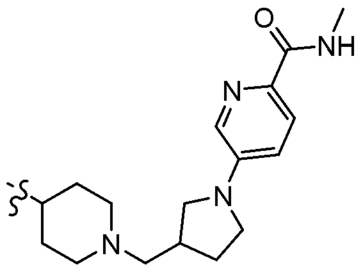

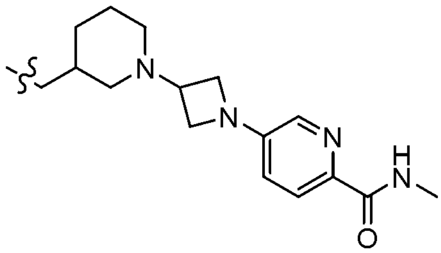

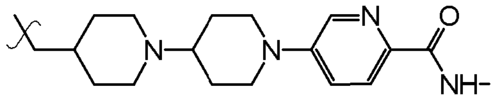

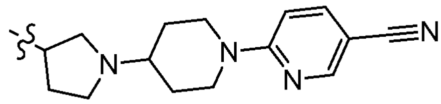

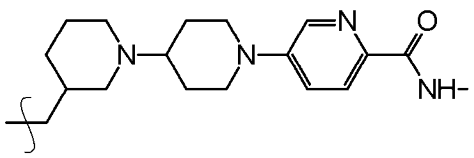

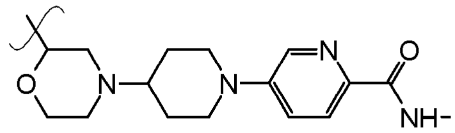

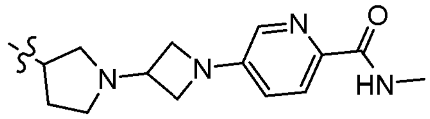

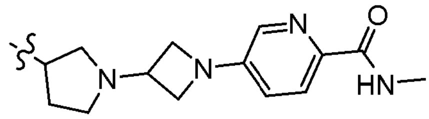

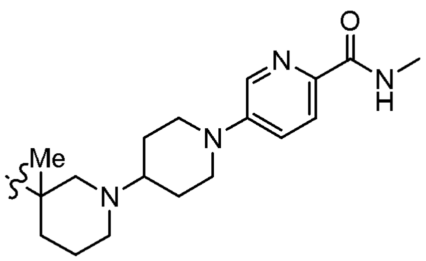

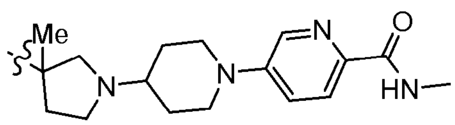

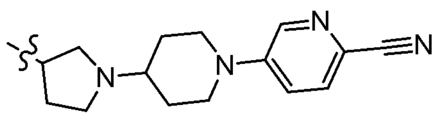

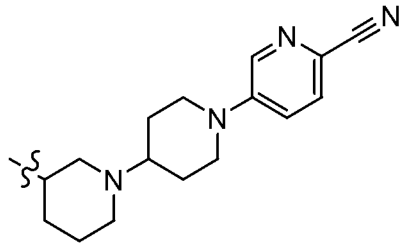

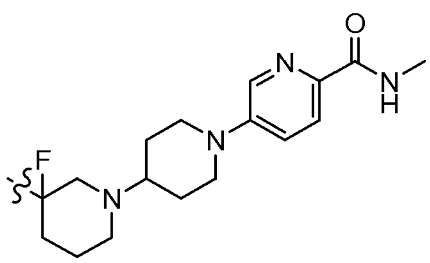

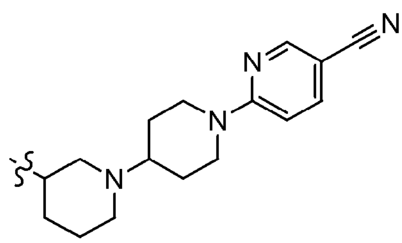

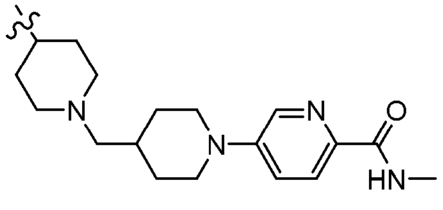

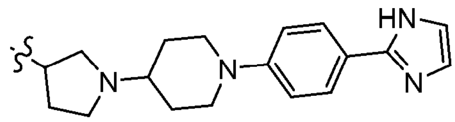

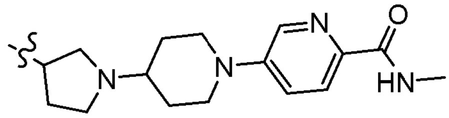

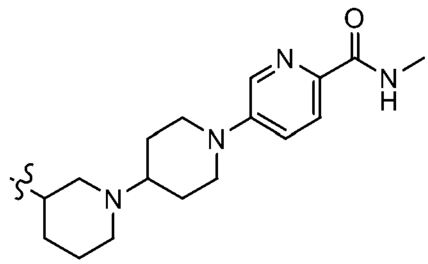

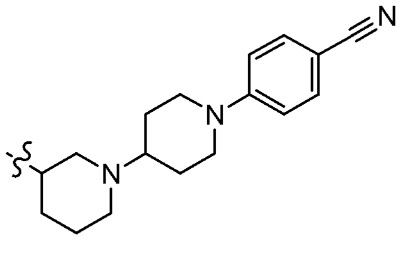

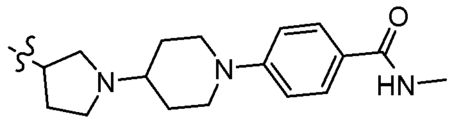

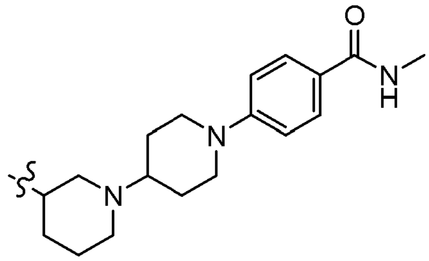

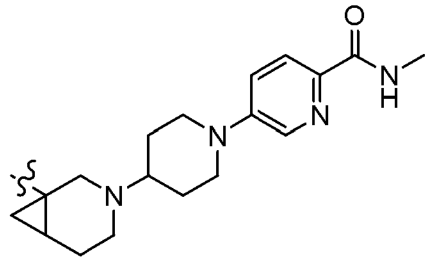

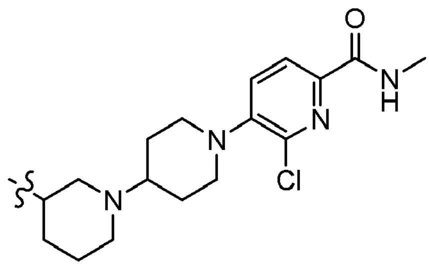

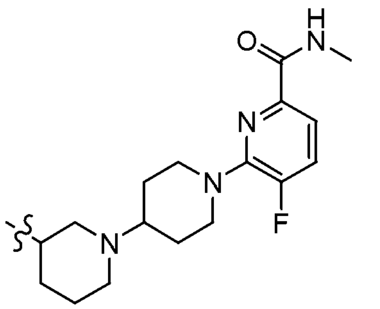

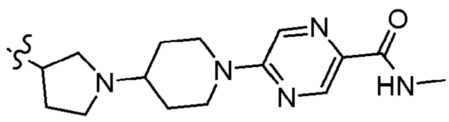

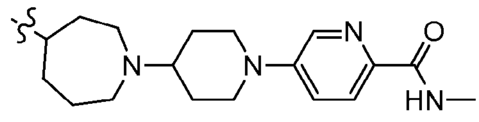

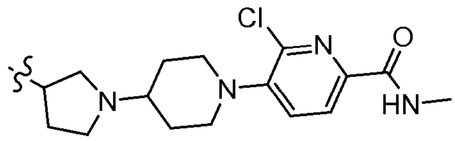

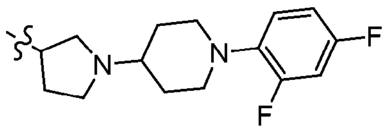

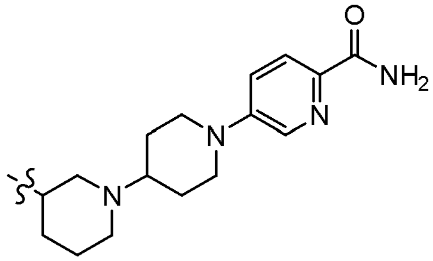

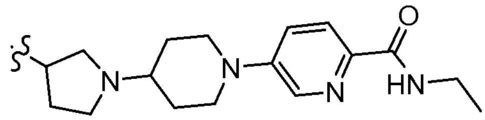

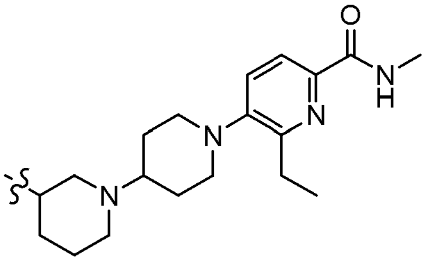

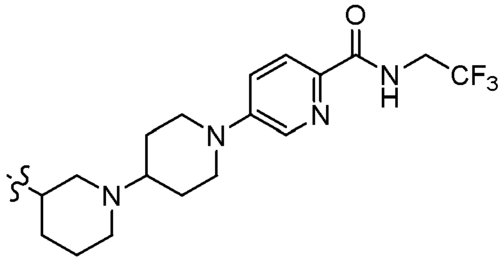

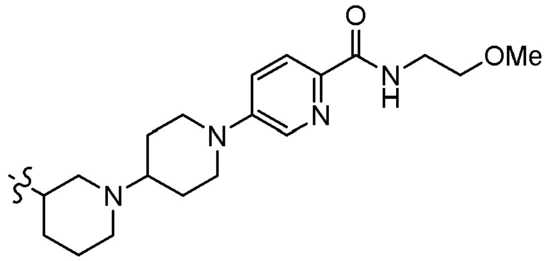

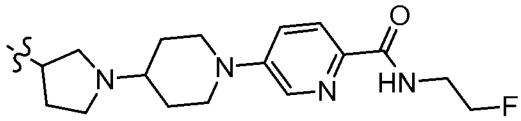

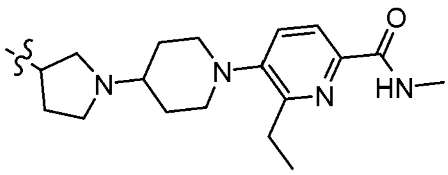

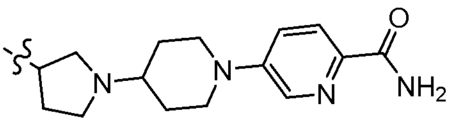

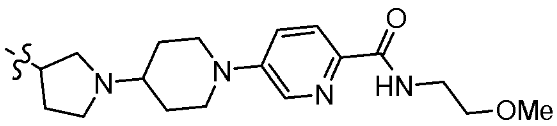

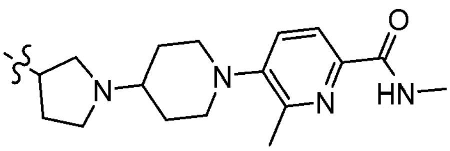

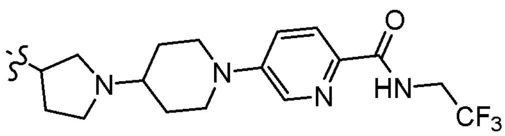

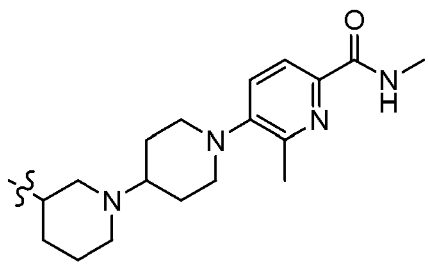

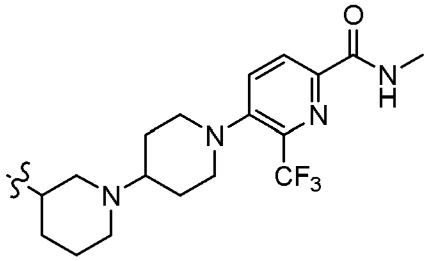

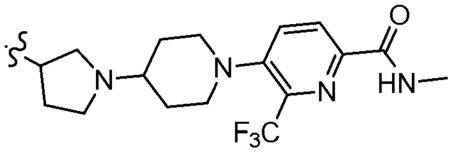

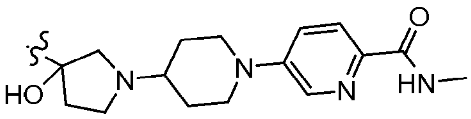

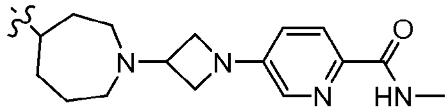

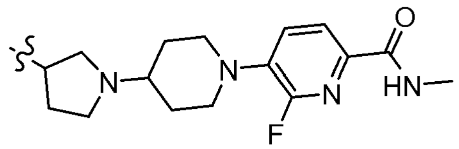

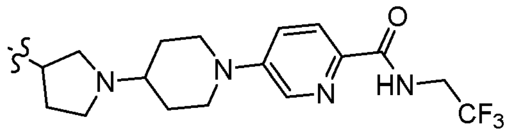

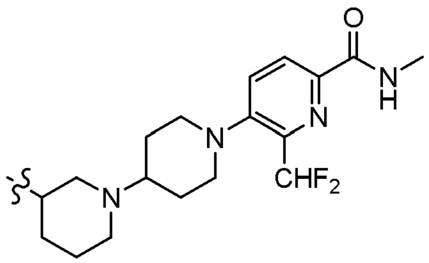

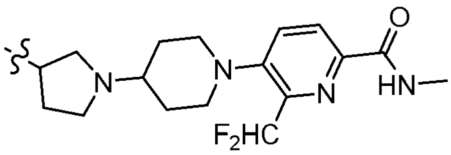

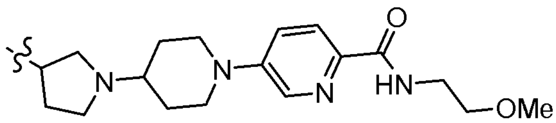

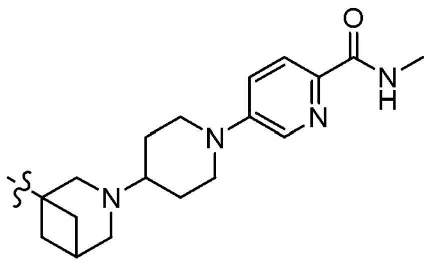

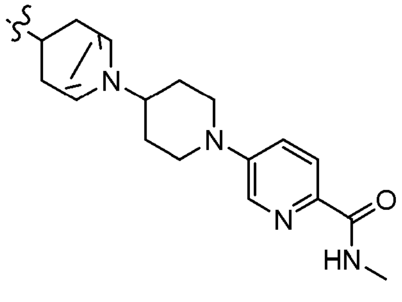

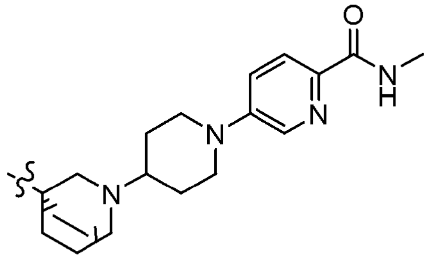

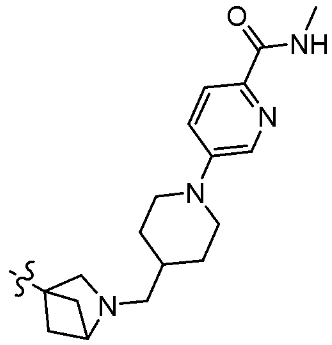
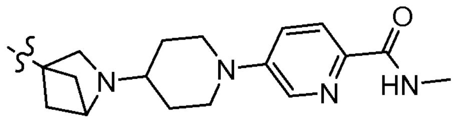
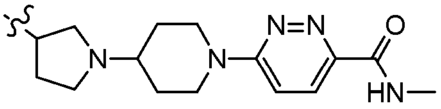
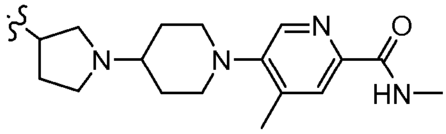
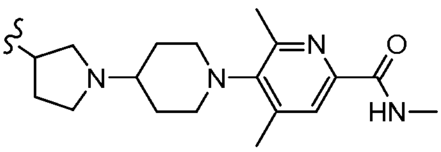
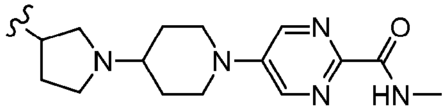
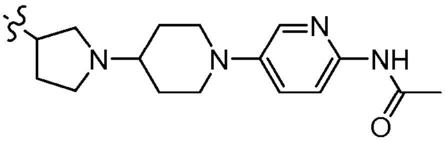
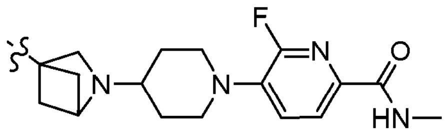
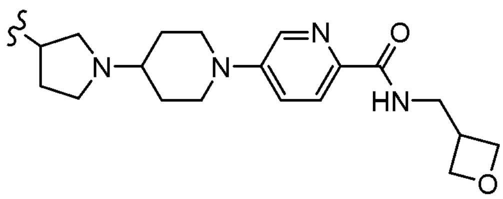
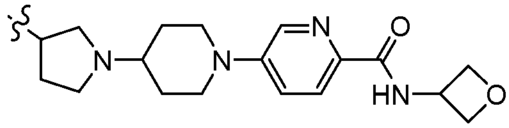
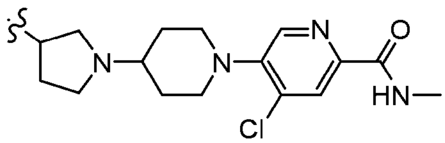
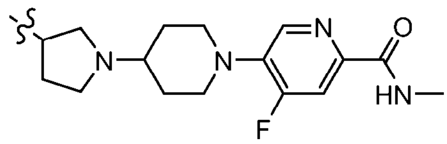
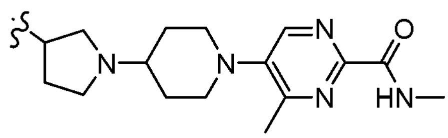
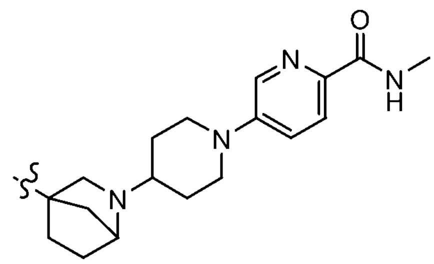
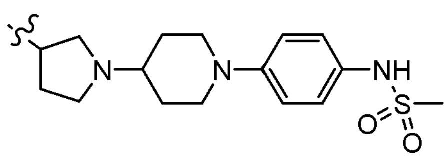
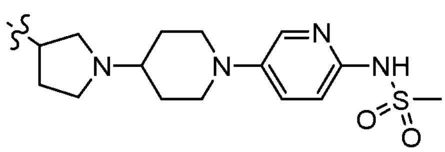

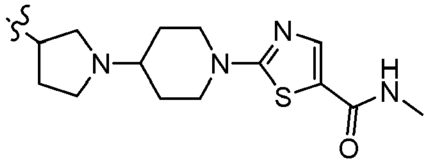
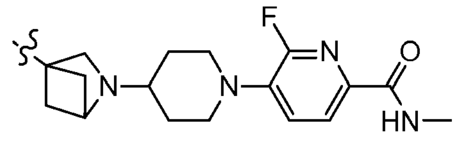
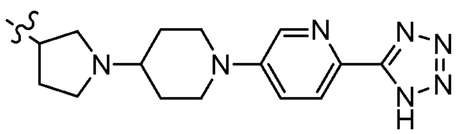
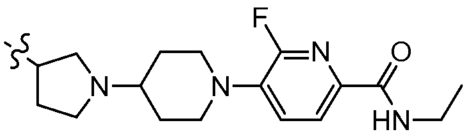
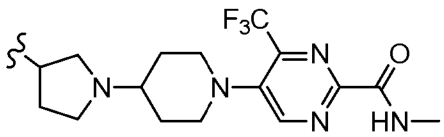
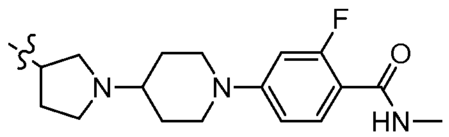
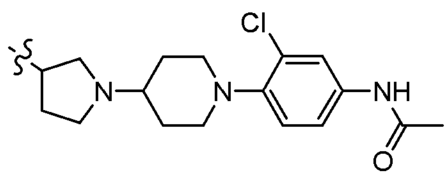
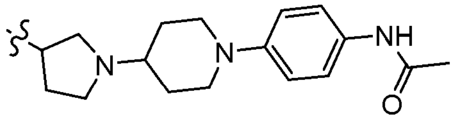
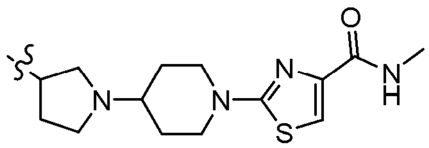
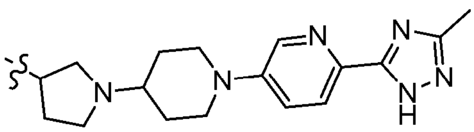
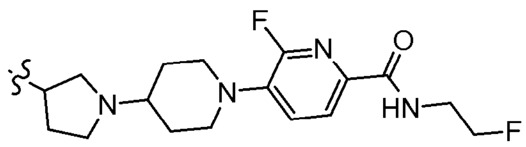
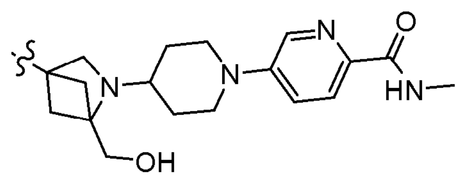
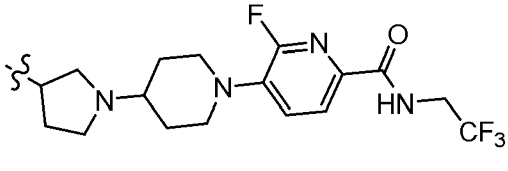
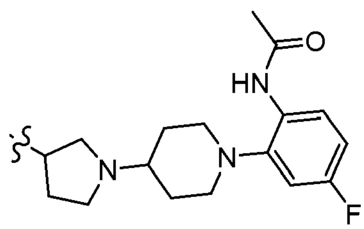
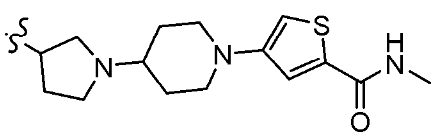
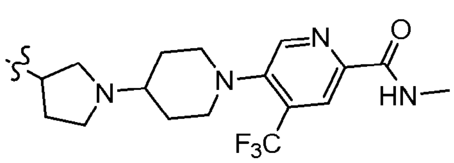
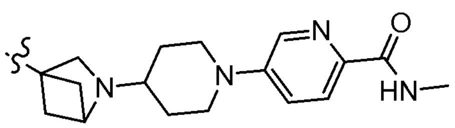
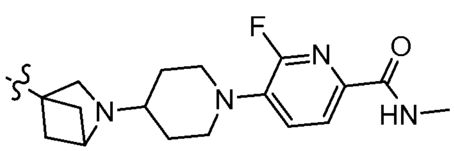

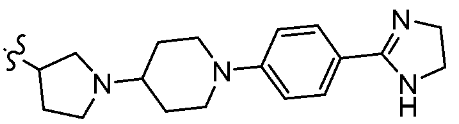

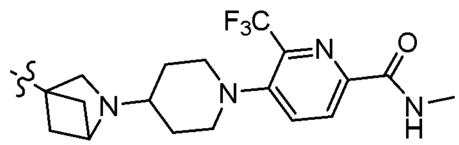

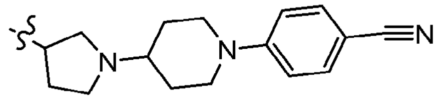

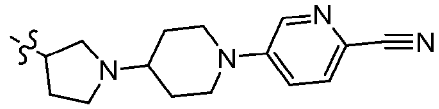

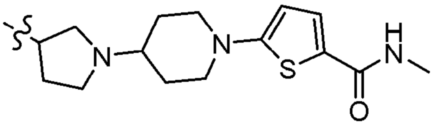

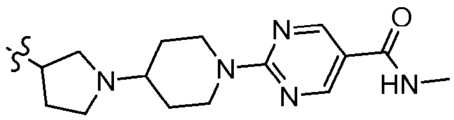

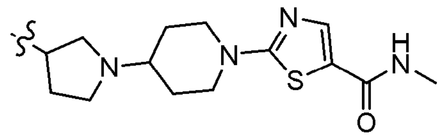

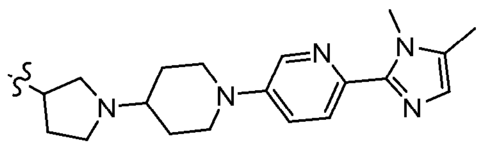

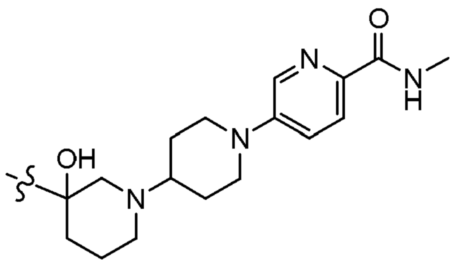

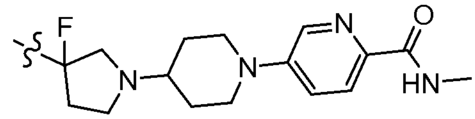

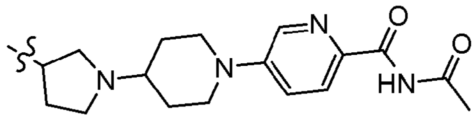

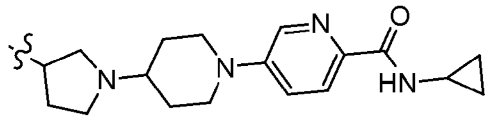

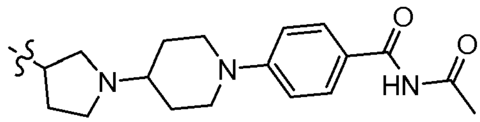

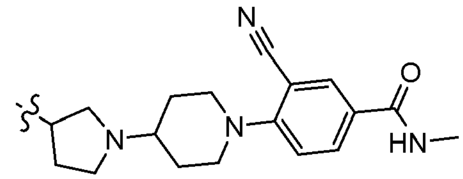

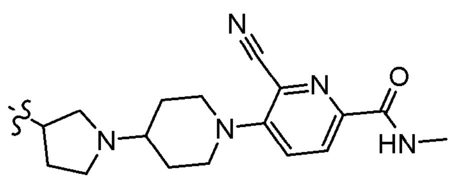

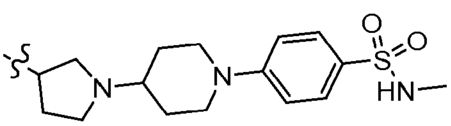

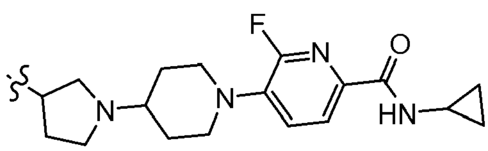

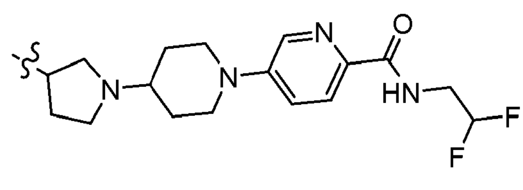

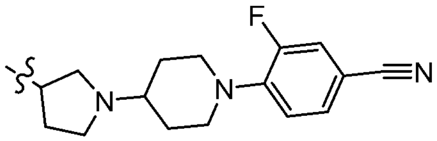
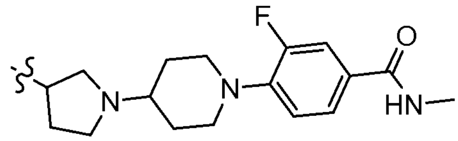
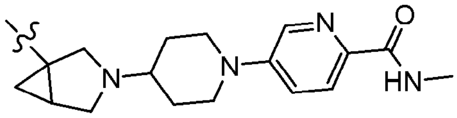
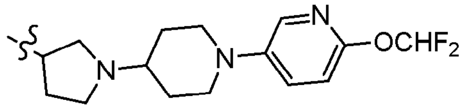
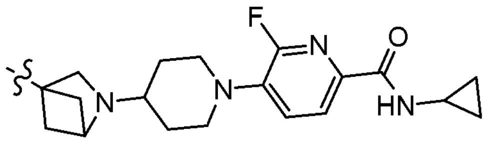
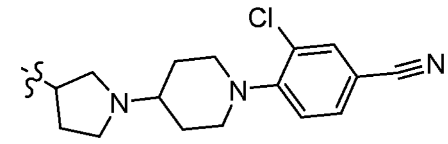
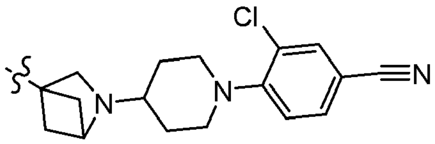
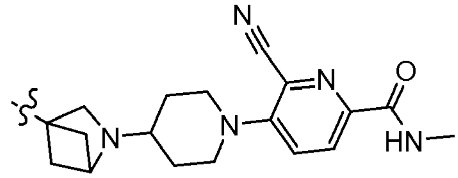
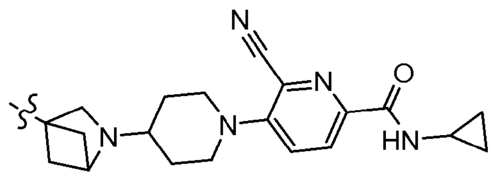
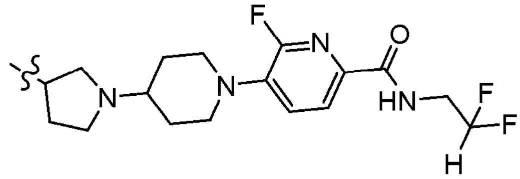
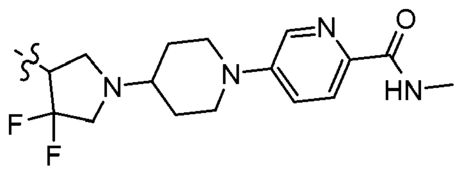
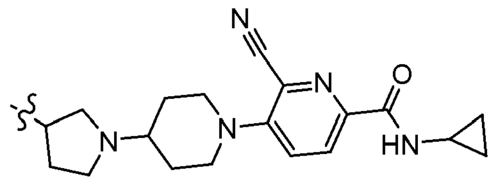
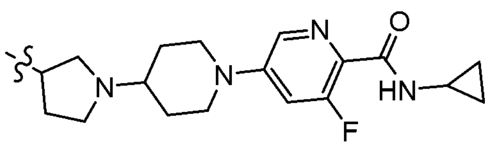
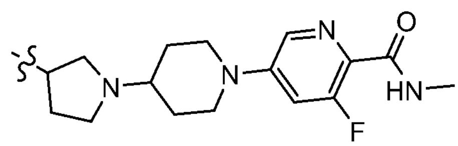
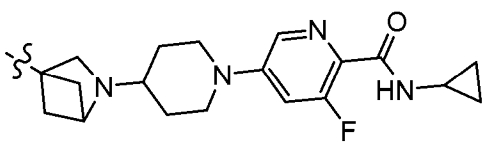
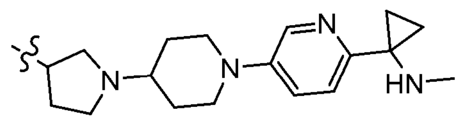
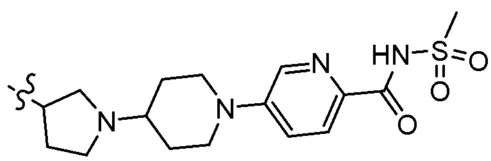
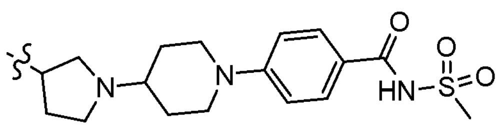

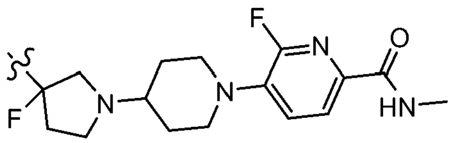

11. A compound for use according to any preceding claim, having any of the following structures:

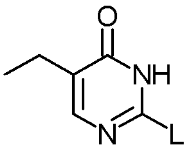 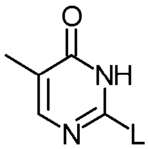 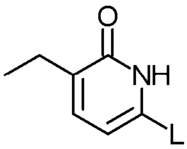 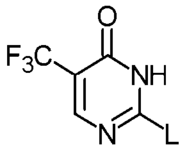 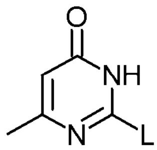

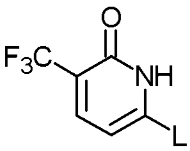 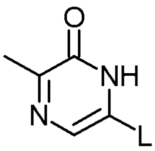 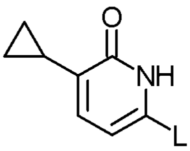 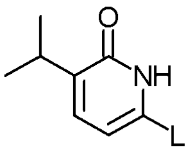 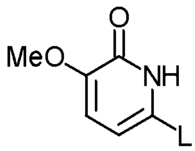

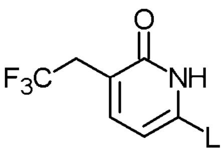 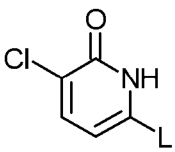 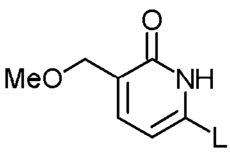 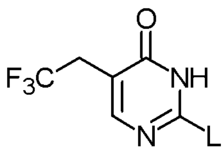 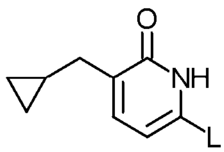

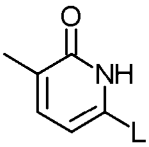 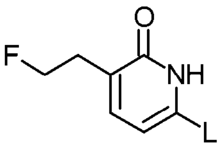 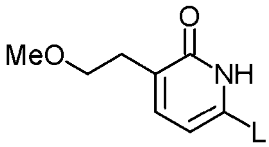 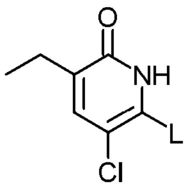 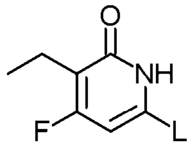

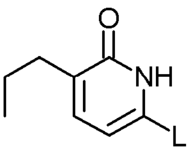 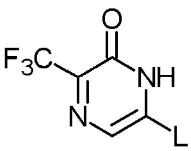 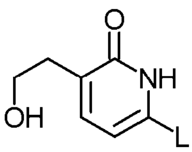 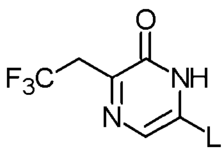 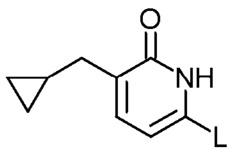

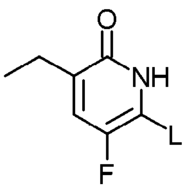 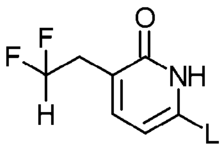

12. A compound for use according to any preceding claim, having a formula selected from:

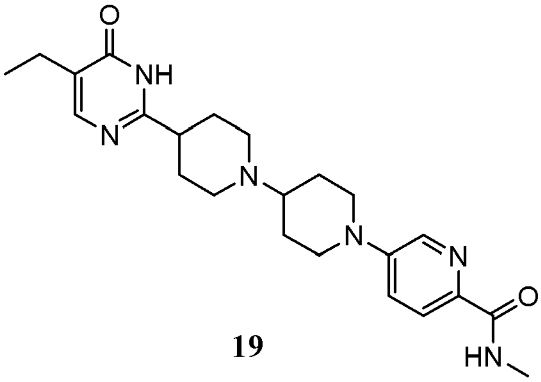

**19**

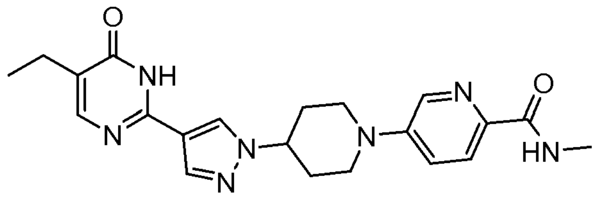

**20**

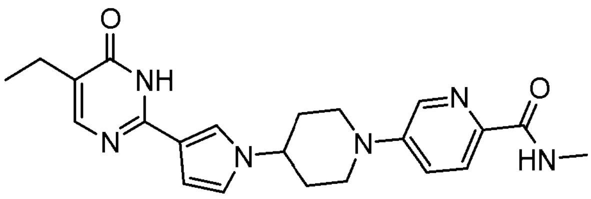

**22**

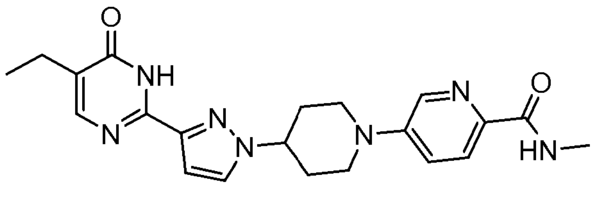

**25**

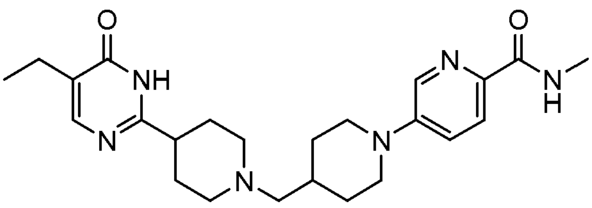

**29**

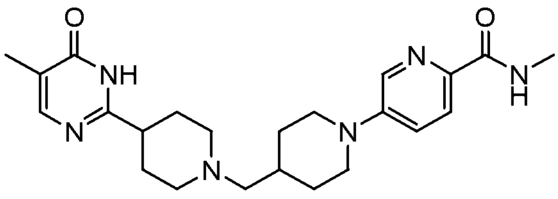

**30**

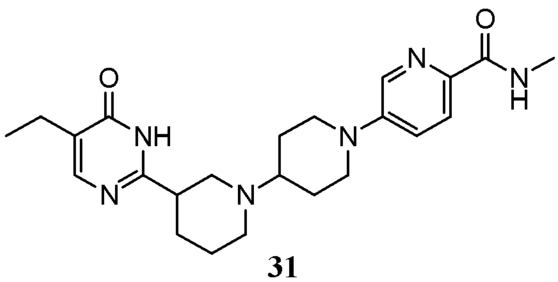

**31**

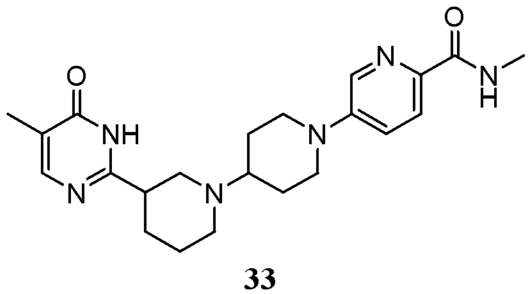

**33**

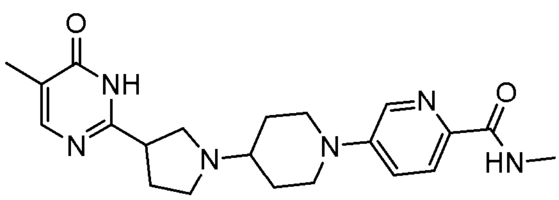

**35**

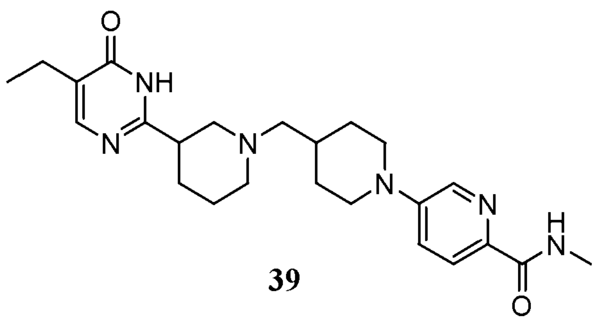

**39**

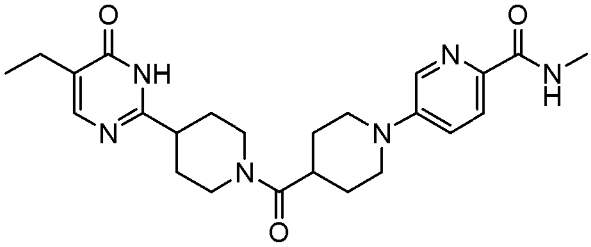

**41**

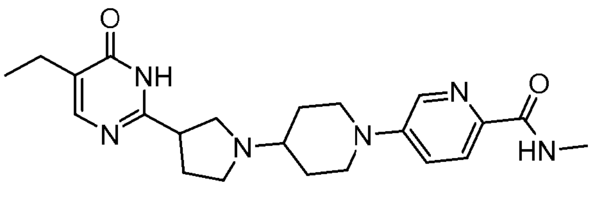

**42**

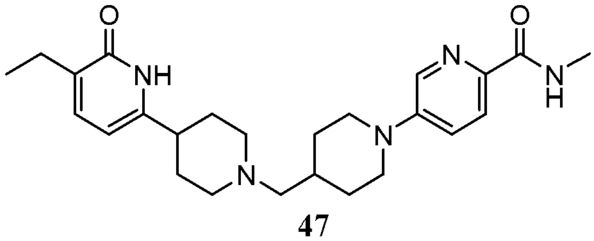

**47**

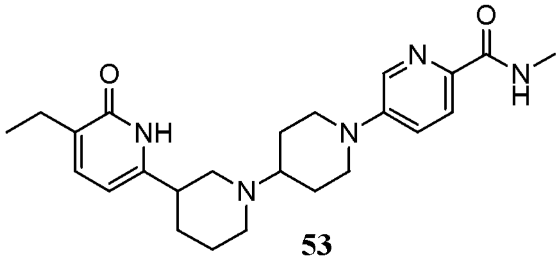

**53**

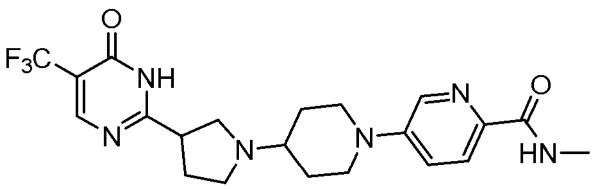

**56**

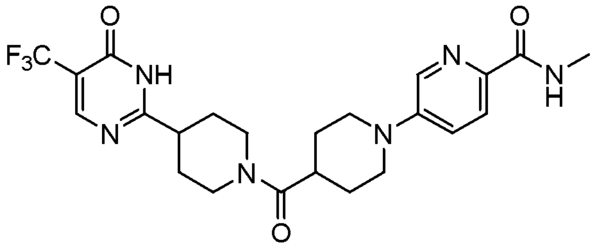

**59**

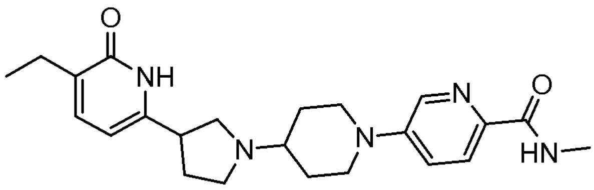

**72**

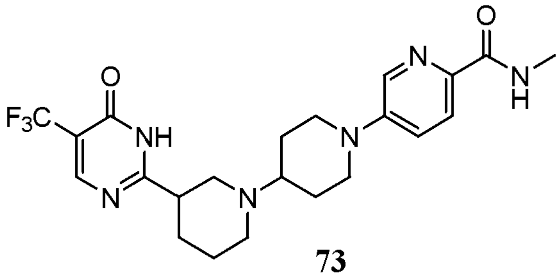

**73**

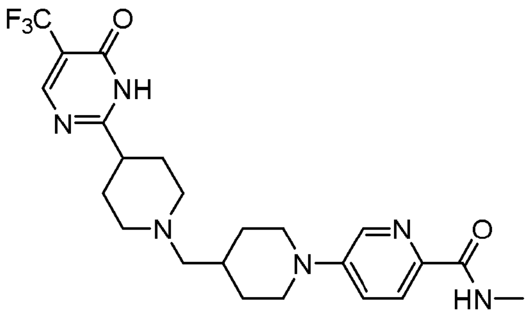

**75**

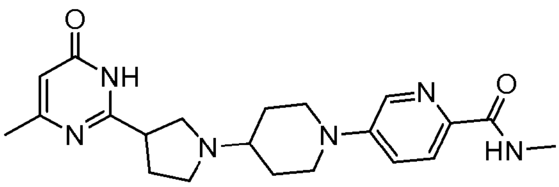

**97**

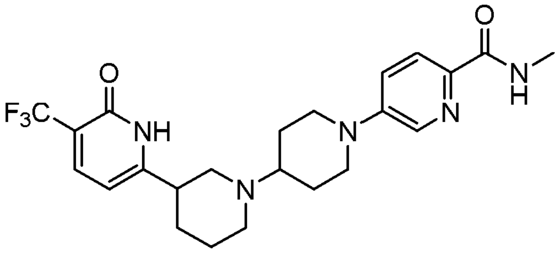

**119**

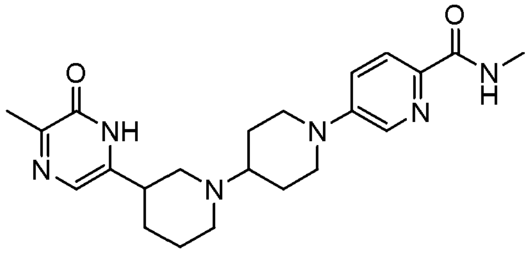

**120**

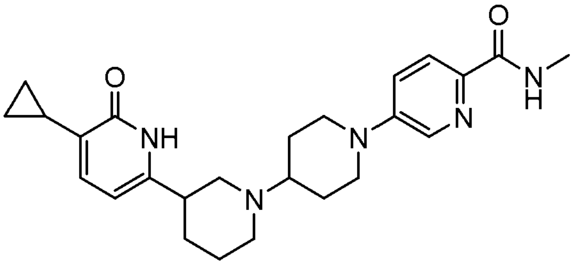

**126**

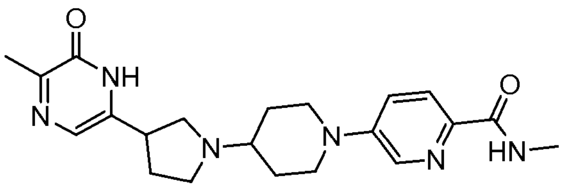

**127**

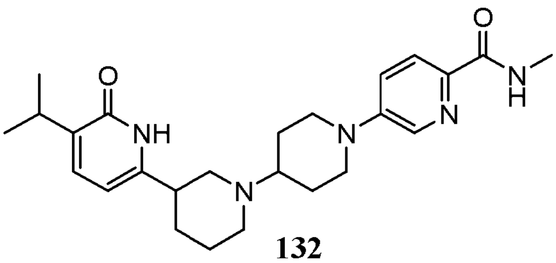

**132**

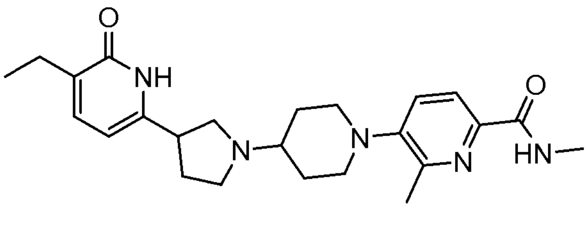

**133**

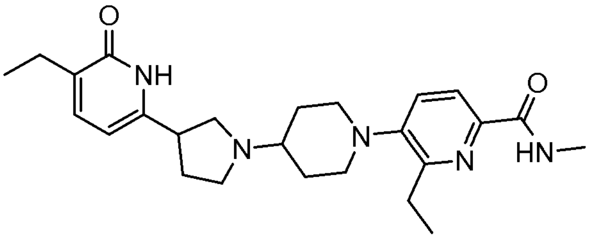

**134**

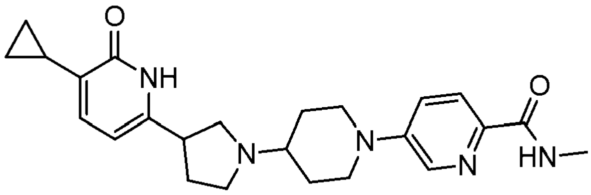

**141**

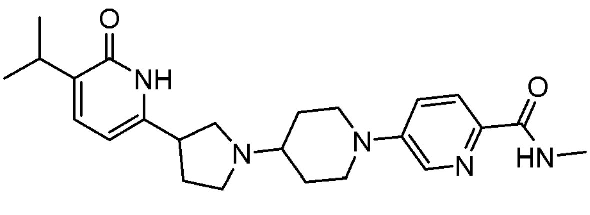

**142**

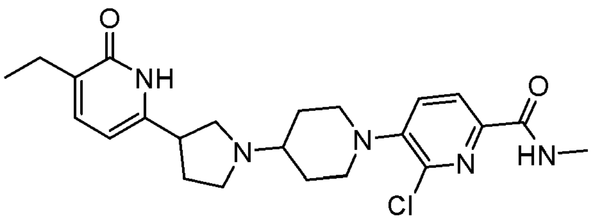

**144**

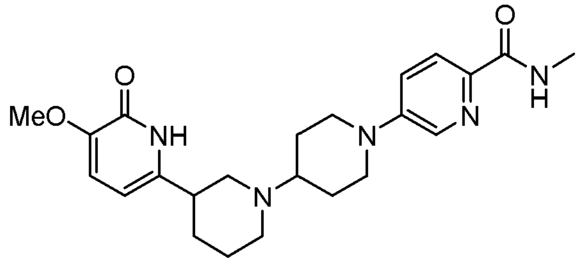

**147**

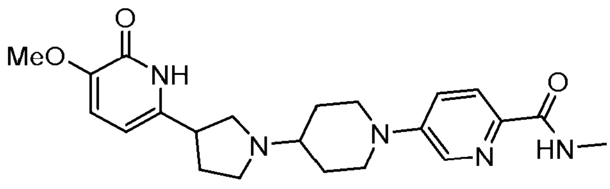

**148**

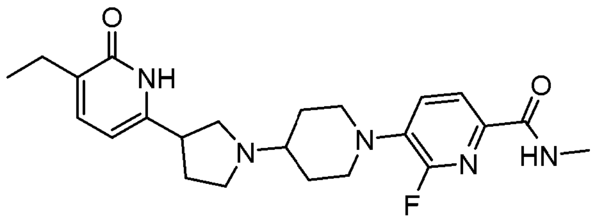

**151**

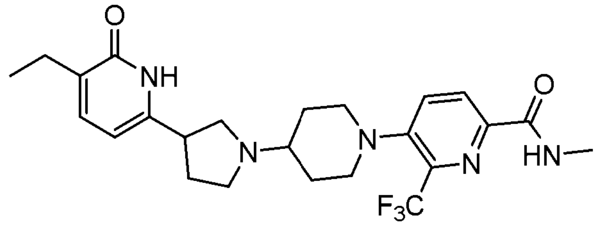

**152**

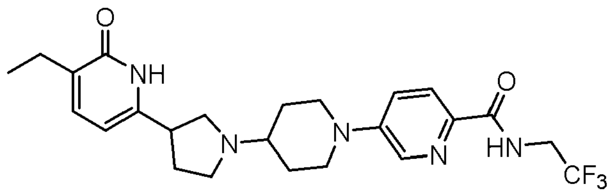

**153**

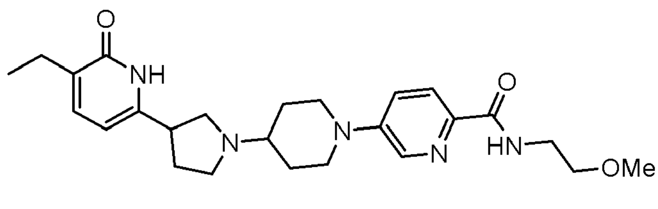

**160**

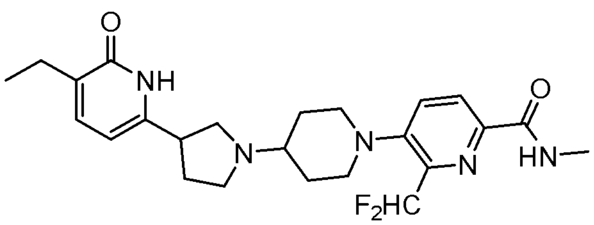

**164**

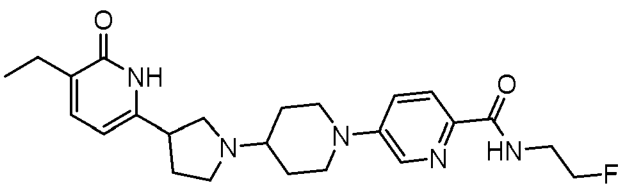

**165**

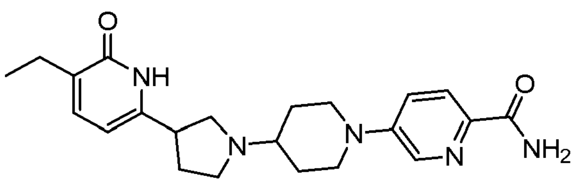

**166**

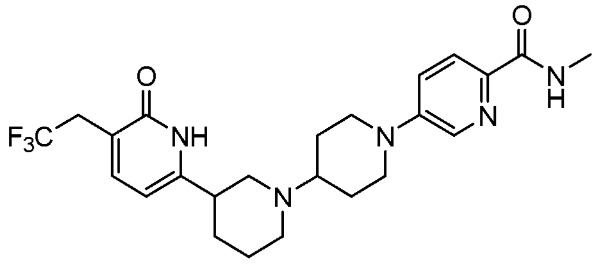

**169**

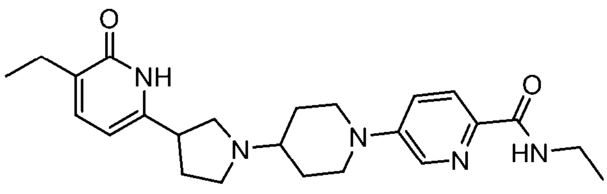

**171**

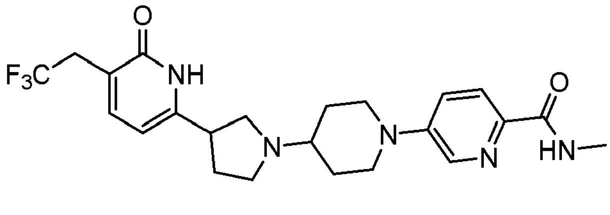

**188**

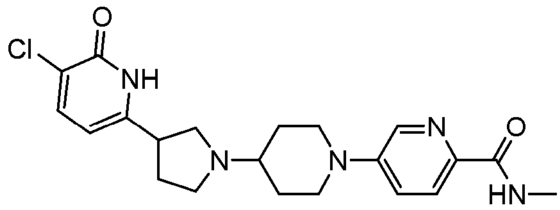

**201**

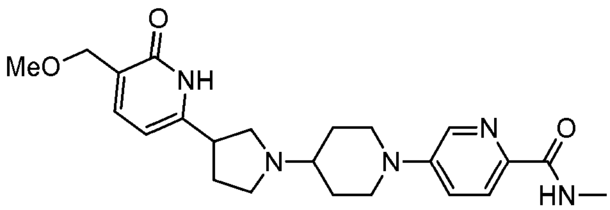

**207**

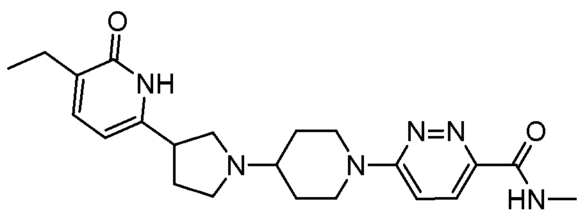

**210**

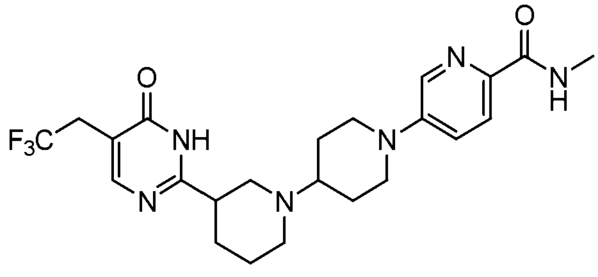

**212**

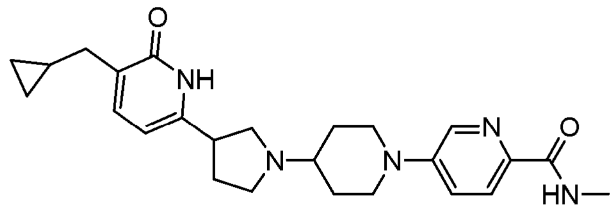

**213**

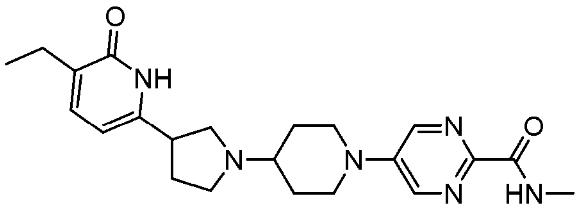

**216**

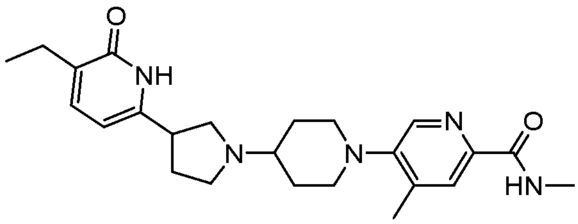

**217**

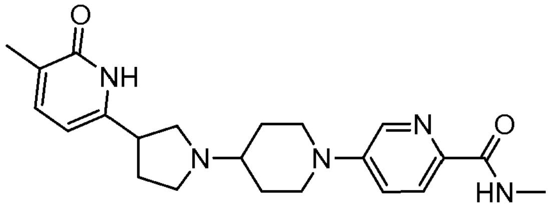

**225**

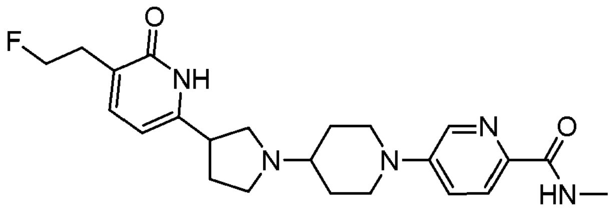

**231**

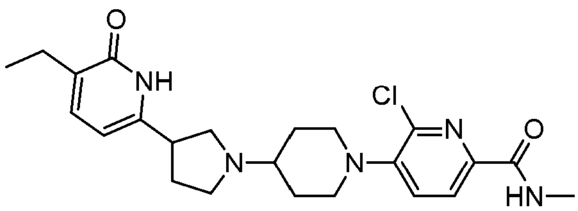

**239**

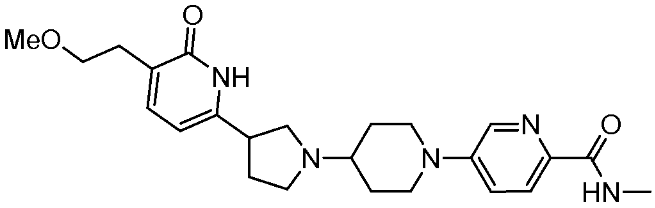

240

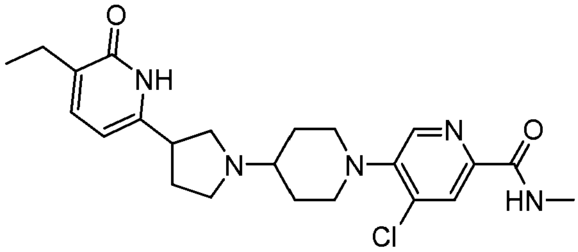

242

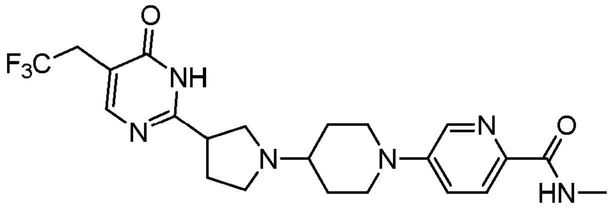

243

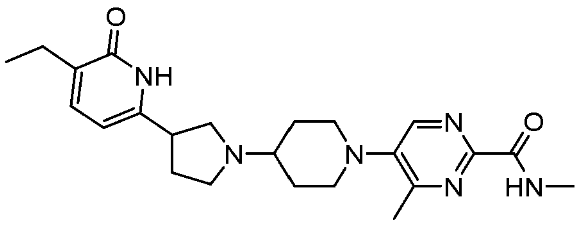

246

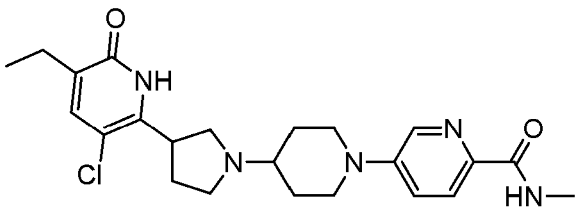

255

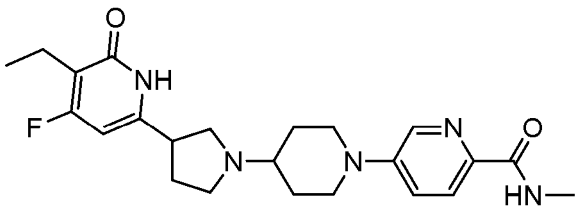

256

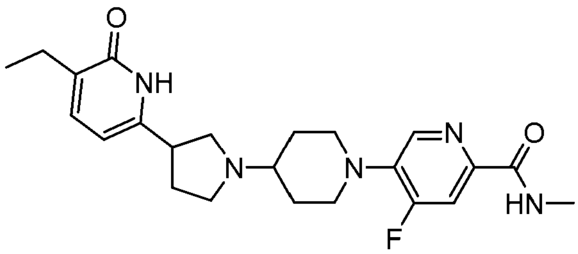

257

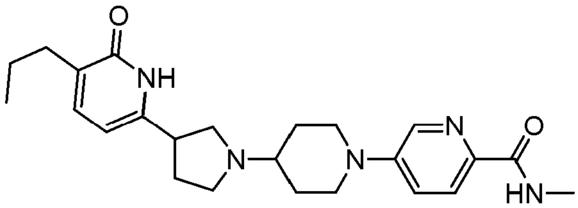

261

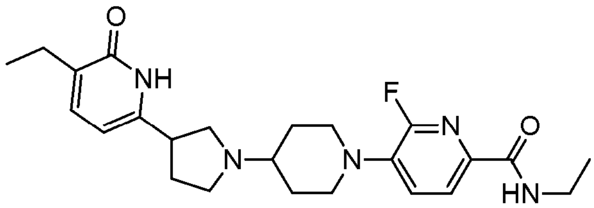

264

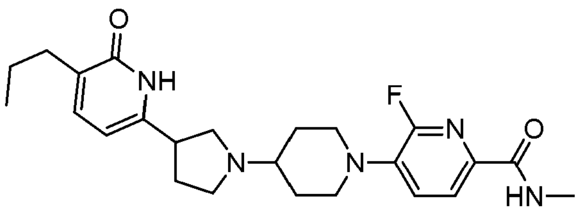

269

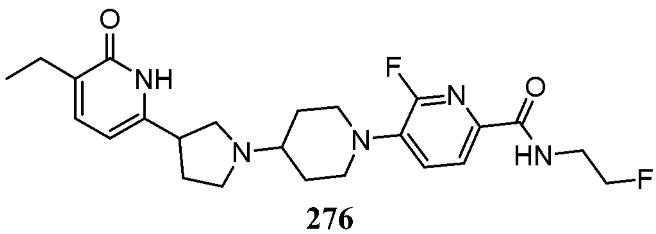

276

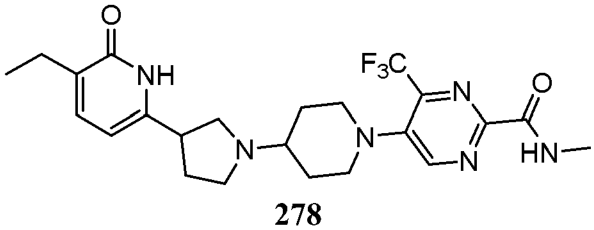

278

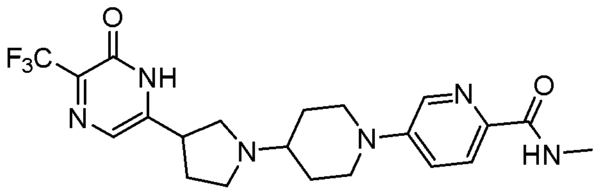

**279**

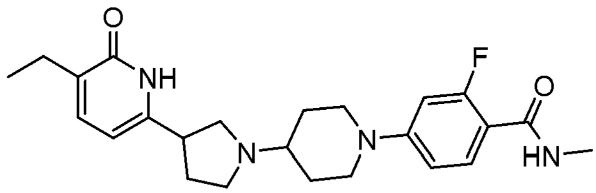

**282**

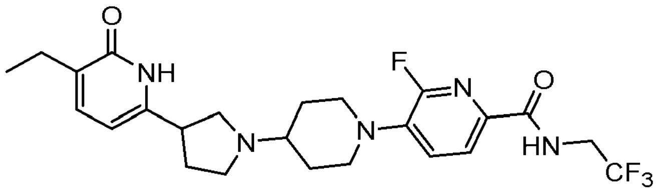

**285**

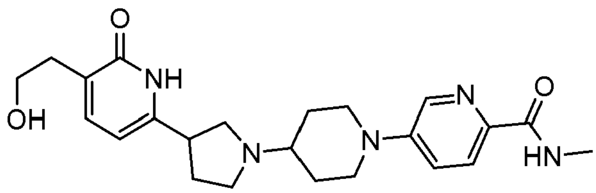

**286**

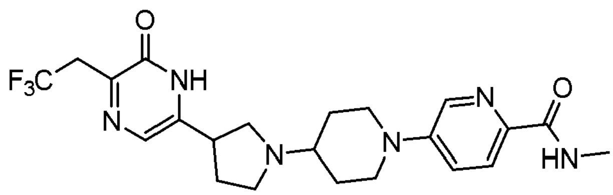

**287**

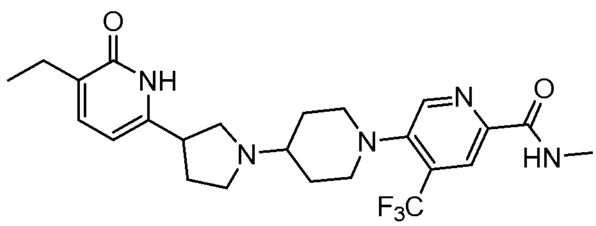

**298**

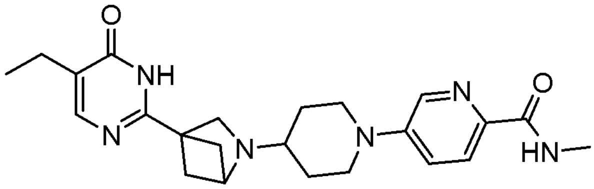

**299**

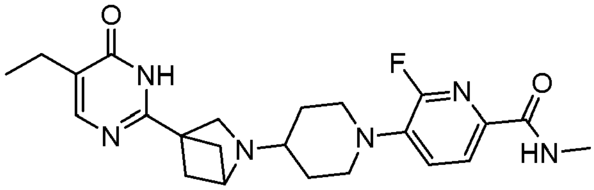

**300**

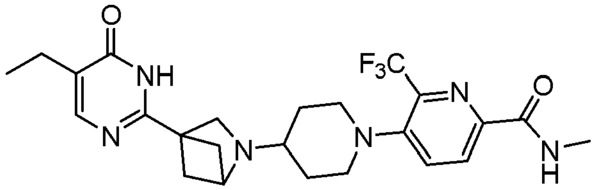

**310**

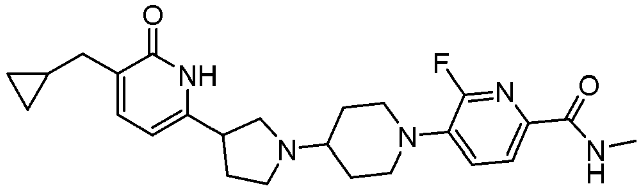

**313**

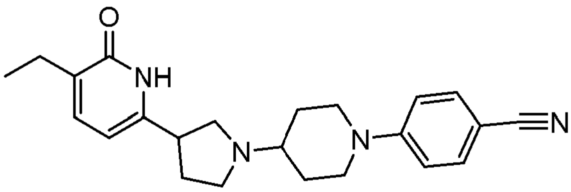

**317**

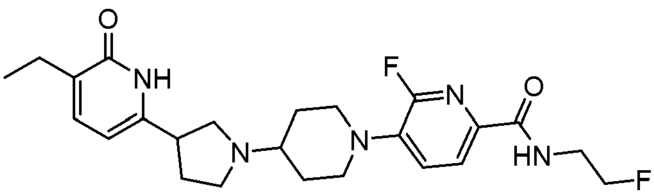

**322**

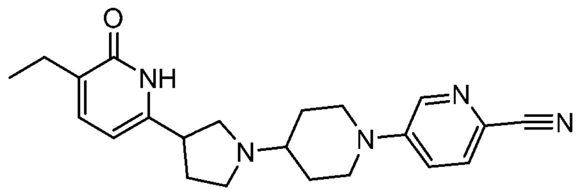

326

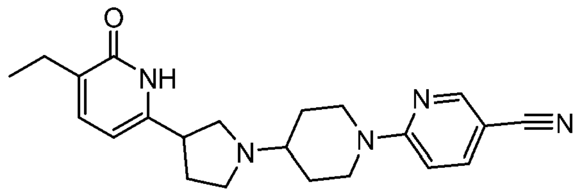

332

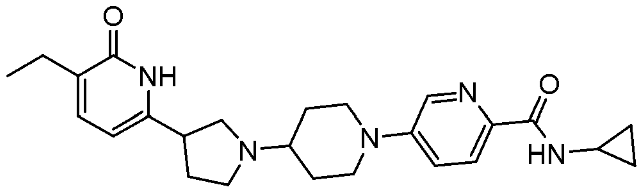

338

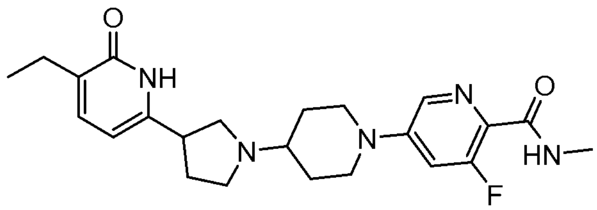

345

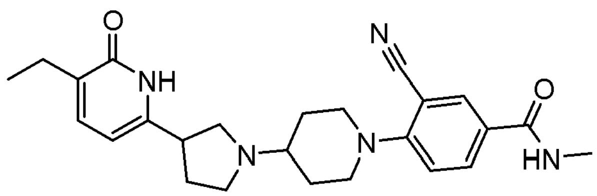

346

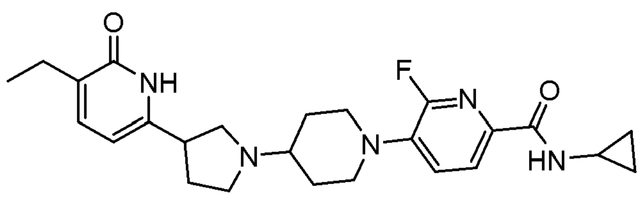

349

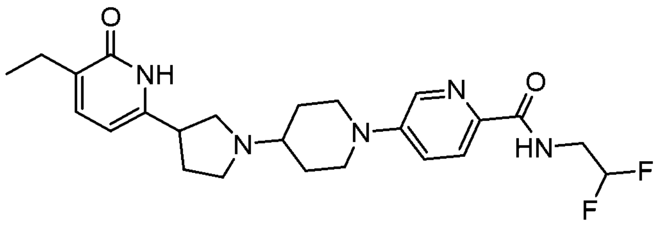

353

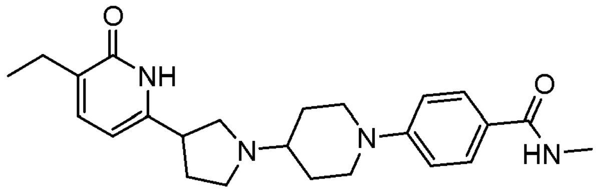

355

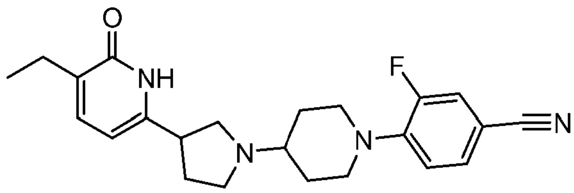

356

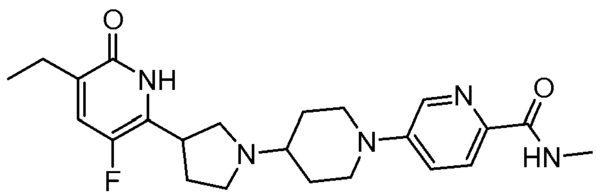

360

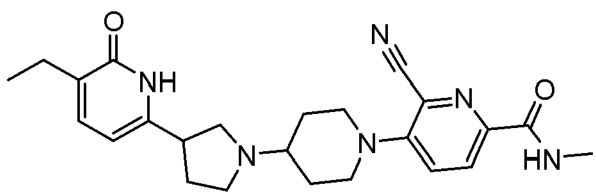

363

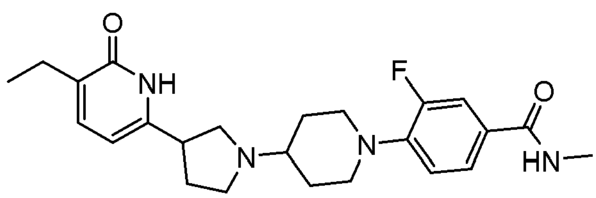

364

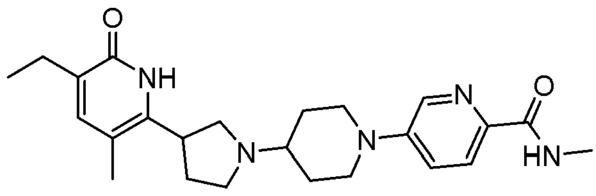

365

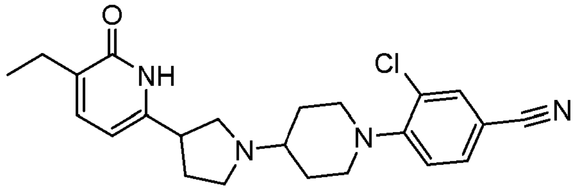

377

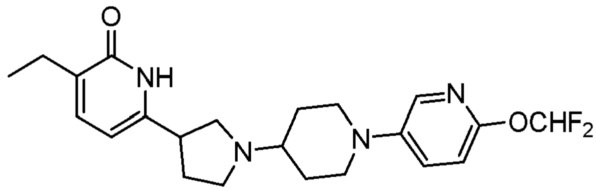

382

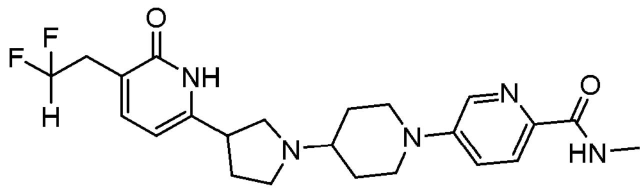

391

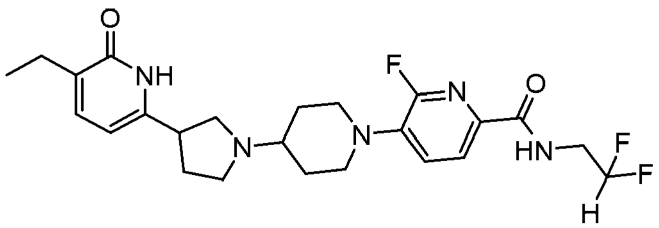

398

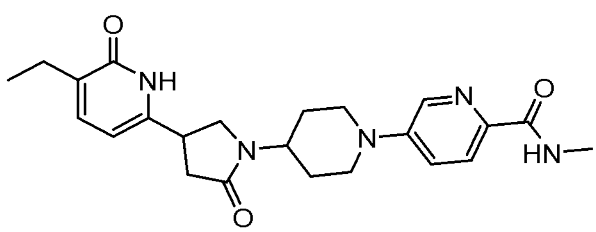

406

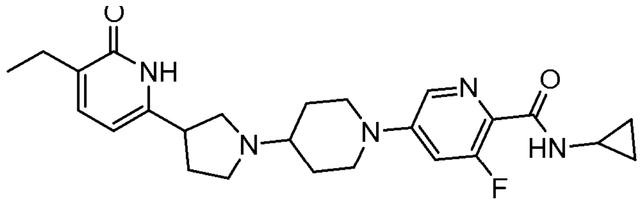

**408**

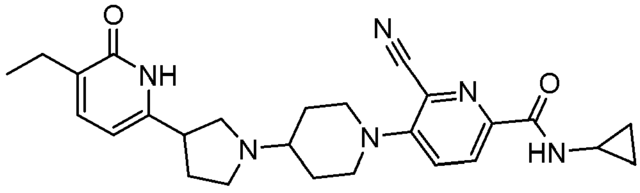

**409**

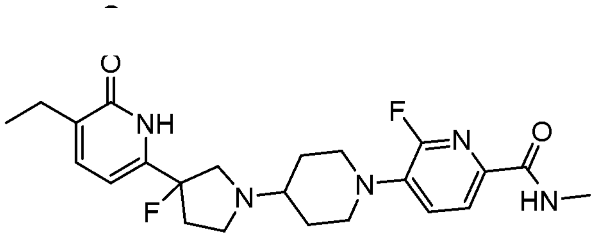

**415**

**13.** A compound for use according to any preceding claim, which is for use in treating a cancer.

**14.** A pharmaceutical composition comprising:

a compound as defined in any of claims 1 to 12; and
a pharmaceutically acceptable additive and/or excipient.

**15.** A pharmaceutical kit for treating a cancer, which pharmaceutical kit comprises:

(a) a compound as defined in any of claims 1 to 12; and
(b) a further agent for treating cancer;

wherein the compound and the further agent are suitable for administration simultaneously, sequentially or separately; and
optionally wherein the further agent for treating cancer is selected from anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, hormone-deprivation therapy, immunotherapeutic agents, proapoptotic agents and cell cycle signalling inhibitors.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KAMALETDINOVA, T. et al.** *Cell*, 2019, vol. 8, 1625
- **NGOI, YL. et al.** *Cancer J*, 2021, vol. 27, 521-528
- **FARMER, H. et al.** *Nature*, 2005, vol. 434, 917-921
- **LORD, CJ.** ; **ASHWORTH, A**. *Science*, 2017, vol. 355, 1152-1158
- **FONG, PC. et al.** *N. Engl. J. Med.*, 2009, vol. 361, 123-134
- **ZHU, Y. et al.** *Mol. Cancer.*, 2019, vol. 18, 152
- **SEN, T. et al.** *Cancer Discov.*, 2019, vol. 9, 646-661
- **PANTELIDOU, C. et al.** *bioRxiv*, 2021
- **CHABANON, RM et al.** *Nat. Rev. Cancer.*, 2021, vol. 21, 701-717
- **MORGAN, SM. et al.** *Nat. Commun.*, 2022, vol. 13, 187
- **BJOMEVIK, K. et al.** *Science*, 2021, vol. 375, 296-301
- **LAFARGUE, CJ. et al.** *Lancet Oncol.*, 2019, vol. 20, c15-c28
- **FARRÉS, J. et al.** *Blood.*, 2013, vol. 122, 44-54
- **RONSON, G E. et al.** *Nat. Commun.*, 2018, vol. 9, 746
- **JOHANNES, JW. et al.** *J. Med. Chem.*, 2021, vol. 64, 14498-14512
- **ILLUZZI, G. et al.** *Clin. Cancer Res.*, 2022, CCR-22-0301
- **HUGHES-DAVIES, L. et al.** *Cell*, 2003, vol. 115, 523-535
- **JASIN, M. et al.** *Oncogene*, 2002, vol. 21, 8981-93
- **RADICE, PJ. et al.** *Exp. Clin. Cancer. Res.*, 2002, vol. 21, 9-12
- **CHAPPNIS, PO** ; **FOULKES WO**. *Cancer Treat Res.*, 2002, vol. 107, 29-59
- **PAPEO, G. et al.** *J. Biomol. Screen*, 2014, vol. 19, 1212-1219